# EUROPEAN PATENT SPECIFICATION

(11) **EP 1 274 865 B1**
(45) Date of publication and mention of the grant of the patent: **14.02.2007**
(21) Application number: 01953936.0
(22) Date of filing: 06.04.2001
(51) Int. Cl.: C12Q 1/68

(54) **DIAGNOSIS OF DISEASES ASSOCIATED WITH APOPTOSIS BY MEANS OF ASSESSING THE METHYLATION STATUS OF GENES ASSOCIATED WITH APOPTOSIS**
DIAGNOSE VON MIT APOPTOSE ASSOZIIERTEN ERKRANKUNGEN MITTELS ERMITTLUNG DES METHYLIERUNGSZUSTANDES VON APOPTOSE-ASSOZIERTEN GENEN
DIAGNOSTIC DE MALADIES ASSOCIEES A L'APOPTOSE EN EVALUANT L'ETAT DE METHYLATION DE GENES ASSOCIES A L'APOPTOSE

(30) Priority: 06.04.2000 DE 10019058; 07.04.2000 DE 10019173; 30.06.2000 DE 10032529; 01.09.2000 DE 10043826
(43) Date of publication of application: 15.01.2003
(73) Proprietor: Epigenomics AG, 10178 Berlin (DE)
(72) Inventor: OLEK, Alexander, 10115 Berlin (DE); PIEPENBROCK, Christian, 10115 Berlin (DE); BERLIN, Kurt, 14532 Stahnsdorf (DE)
(74) Representative: Krauss, Jan
(86) International application number: PCT/EP2001/003969
(87) International publication number: WO 2001/077164

(56) References cited:
- WO-A-01/68911
- WO-A-01/68912
- WO-A-01/77376
- WO-A-99/28498
- WO-A-99/29898
- US-A- 5 744 305
- DATABASE GENBANK [Online] NCBI; 6 March 1995 (1995-03-06) ITHO N ET AL: "Human Fas antigen (fas) mRNA, complete cds" retrieved from HTTP://WWW.NCBI.NLM.NIH.GOV/ Database accession no. M67454 XP002187215 & ITHO N ET AL.: "The polypetide encoded by the cDNA for human cell surface antigen FAS can mediate apoptosis." CELL, vol. 66, no. 2, 1991, pages 233-243,
- ALLAN LA ET AL.: "The p21WAFi1/CIP1 promoter is methylated in Rat- cells: stable restoration of p53-dependent p21WAF1/CIP1 expression after transfection of a genomic clone containing the p21WAF1/CIP1 gene." MOLECULAR AND CELLULAR BIOLOGY, vol. 20, no. 4, February 2000 (2000-02), pages 1291-1298, XP001026537
- SALVATORE P ET AL.: "High resolution analysis of the galectin-1 gene promoter region in expressing and nonexpressing tissues." FEBS LETTERS, vol. 421, no. 2, 1998, pages 152-158, XP004261737
- DATABASE EMBL [Online] EBI; 11 December 1993 (1993-12-11) DEISS LP ET AL.: "DAP-kinase" retrieved from HTTP://WWW.EBI.AC.UK/CGI-BIN/EMBLFETCH Database accession no. X76104 XP002187216 -& DEISS LP ET AL: "Identification of a novel serine/threonine kinase and a novel 15-kD protein as potential mediators of the gamma interferon-induced cell death." GENES & DEVELOPMENT, vol. 9, 1995, pages 15-30, XP002102392
- KATZENELLENBOGEN RA ET AL.: "Hypermethylation of the DAP-Kinase CpG island is a common alteration in B-cell malignancies." NEOPLASIA, vol. 93, no. 12, June 1999 (1999-06), pages 4347-4353, XP002187214
- UEKI T ET AL.: "Hypermethylation of multiple genes in pancreatic adenocarcinoma." CANCER RESAERCH, vol. 60, no. 7, 1 April 2000 (2000-04-01), pages 1835-1839, XP001026187

## Description

### Field of the Invention

The levels of observation that have been well studied by the methodological developments of recent years in molecular biology, are the genes themselves, the translation of these genes into RNA, and the resulting proteins. The question of which gene is switched on at which point in the course of the development of an individual, and how the activation and inhibition of specific genes in specific cells and tissues are controlled is correlatable to the degree and character of the methylation of the genes or of the genome. In this respect, pathogenic conditions may manifest themselves in a changed methylation pattern of individual genes or of the genome.

The present invention relates to nucleic acids, and to a method for the diagnosis of diseases which have a connection with the genetic and/or epigenetic parameters of genes associated with apoptosis and, in particular, with the methylation status thereof.

### Prior Art

During embryogenesis, tissue turnover or metamorphosis, multi cellular eukaryotic organisms retain the ability to eliminate unwanted cells. This form of programmed cell death is termed 'apoptosis'. Apoptosis takes place in response to a variety of stimuli that trigger biochemical pathways that result in a characteristic set of processes leading to cell death.

The stimuli that trigger apoptosis can include the levels of essential growth factors, treatment with glucocorticoids, irradiation, and activation of certain receptors. These trigger a variety of biochemical pathways. The 'classical' pathway comprises of a ligand-receptor interaction which triggers the activation of a protease. This leads to the release of cytochrome C from mitochondria. This in turn activates a series of proteases, whose actions culminate in the destruction of cellular structures.

For example, a common pathway involves activation of caspase-8 by oligomerization at an activated surface receptor. Caspase-8 cleaves Bid, which triggers release of cytochrome c from mitochondria. The cytochrome c causes Apaf-1 to oligomerize with caspase-9. The activated caspase-9 cleaves procaspase-3, whose two subunits then form the active protease. This cleaves various targets that lead to cell death. Cell death by apoptosis is characterised by processes in which the cell becomes more compact, blebbing occurs at the membranes, chromatin becomes condensed, and DNA is fragmented.

The correct control of apoptosis is probably essential to all higher organisms. For example, in the model organism C. Elegans 131 of the 1090 cells die at defined points of the lifecycle of the organism. In vertebrates, the importance of apoptosis has been proved using knockout mouse models. In humans, apoptosis pathways have been implicated in a variety of diseases including neurodegenerative diseases, ageing and cancer:
- HIV infection; Badley et. al. "Mechanisms of HIV-associated lymphocyte apoptosis" Blood,Vol. 96; 2951-2964 (2000).
- Bloom Syndrome; Bischof et. al. 'Selective cleavage of BLM, the Bloom syndrome protein, during apoptotic cell death.' J Biol Chem 2001 Jan 11.
- Cardiomyopathy; Narula et. al. "Apoptosis in heart failure: release of cytochrome c from mitochondria and activation of caspase-3 in human cardiomyopathy" Proc Natl Acad Sci U S A.;96:8144-8149 (1999).
- Familial Alzheimers; Simian et. al. Presenilin-1 P264L Knock-In Mutation: Differential Effects on Aβ Production, Amyloid Deposition, and Neuronal Vulnerability The Journal of Neuroscience, 20(23):8717-8726 (1999).
- Aging; Schindowski et. al. 'Age-related changes of apoptotic cell death in human lymphocytes.' Neurobiol Aging 2000 Sep-Oct;21(5):661-70.
- Herpes simplex virus infection; Perng et. al. "Virus-Induced Neuronal Apoptosis Blocked by the Herpes Simplex Virus Latency-Associated. Transcript" Science 287: 1500-1503 (2000).
- Renal ischemia; Yuexian et. al. "Downregulation of the calpain inhibitor protein calpastatin by caspases during renal ischemia-reperfusion" Am. J. Physiol. 279: 509-517.
- Amyotrophic lateral sclerosis; Li et. al. "Functional Role of Caspase-1 and Caspase-3 in an ALS Transgenic Mouse Model" Science 288 (5464); 335-339 (2000).
- Breast cancer; Sierra et. al. 'Bcl-2 with loss of apoptosis allows accumulation of genetic alterations: a pathway to metastatic progression in human breast cancer.' Int J Cancer. 2000 Mar 20;89(2): 142-7.

The complexities of the pathways leading to apoptosis allow for many mechanisms by which it can be diverted. In addition to genomic mutations, the epigenetic control of genes has been implicated in disruptions to apoptosis pathways. The epigenetic parameter that has been best characterised, DNA methylation, has been implicated as a key factor in the resistance of tumors to chemotherapy. It has been shown (Soengas et al "Inactivation of the apoptosis effector Apaf-1 in malignant melanoma" Nature 409; 207-211;2001) that in malignant melanomas disruptions in the apoptosis pathway could be attributed to silencing of the Apaf-1 gene.

The identification of methylation of apoptosis genes as a factor in tumor malignancy opens up the possibility of creating alternative methods of treatment. Methylation based therapies could have considerable advantages over current methods of treatment such as chemotherapy, surgery and radiotherapy. They may even, as demonstrated by Soengas et al, provide a means of treating tumors resistant to conventional therapies. In addition to the development of methylation specific therapies, experiments with Min mice have shown that inhibition of DNA methylation can suppress tumor initiation (Laird et. al. 'Suppression of intestinal neoplasia by DNA hypomethylation' Cell 81; 197-205 1995). Furthermore, DNA methylation analysis may provide novel means for tumor diagnosis as suggested by Rosas et. al. 'Promoter hypermethylation patterns of p16, O6-methylguanine-DNA-methyltransferase, and death-associated protein kinase in tumors and saliva of head and neck cancer patients.' Cancer Res 2001 Feb 1;61(3):939-42.

5-methylcytosine is the most frequent covalent base modification in the DNA of eukaryotic cells. It plays a role, for example, in the regulation of the transcription, in genetic imprinting, and in tumorigenesis. Therefore, the identification of 5-methylcytosine as a component of genetic information is of considerable interest. However, 5-methylcytosine positions cannot be identified by sequencing since 5-methylcytosine has the same base pairing behavior as cytosine. Moreover, the epigenetic information carried by 5-methylcytosine is completely lost during PCR amplification.

A relatively new and currently the most frequently used method for analyzing DNA for 5-methylcytosine is based upon the specific reaction of bisulfite with cytosine which, upon subsequent alkaline hydrolysis, is converted to uracil which corresponds to thymidine in its base pairing behavior. However, 5-methylcytosine remains unmodified under these conditions. Consequently, the original DNA is converted in such a manner that methylcytosine, which originally could not be distinguished from cytosine by its hybridization behavior, can now be detected as the only remaining cytosine using "normal" molecular biological techniques, for example, by amplification and hybridization or sequencing. All of these techniques are based on base pairing which can now be fully exploited. In terms of sensitivity, the prior art is defined by a method which encloses the DNA to be analyzed in an agarose matrix, thus preventing the diffusion and renaturation of the DNA (bisulfite only reacts with single-stranded DNA), and which replaces all precipitation and purification steps with fast dialysis (Olek A, Oswald J, Walter J. A modified and improved method for bisulphite based cytosine methylation analysis. Nucleic Acids Res. 1996 Dec 15;24(24):5064-6). Using this method, it is possible to analyze individual cells, which illustrates the potential of the method. However, currently only individual regions of a length of up to approximately 3000 base pairs are analyzed, a global analysis of cells for thousands of possible methylation events is not possible. However, this method cannot reliably analyze very small fragments from small sample quantities either. These are lost through the matrix in spite of the diffusion protection.

An overview of the further known methods of detecting 5-methylcytosine may be gathered from the following review article: Rein, T., DePamphilis, M. L., Zorbas, H., Nucleic Acids Res. 1998, 26, 2255.

Katzenellenbogen et al, "Hypermethylation of the DAP-kinase CpG island is a common alteration in B-cell malignancies" Blood Vol. 93 No. 12; 4347-53 disclose MSP based analysis of the first 525 bp region from the transcription start site. Promoter methylation was observed in B-cell malignancies.

To date, barring few exceptions (e.g., Zeschnigk M, Lich C, Buiting K, Doerfler W, Horsthemke B. A single-tube PCR test for the diagnosis of Angelman and Prader-Willi syndrome based on allelic methylation differences at the SNRPN locus. Eur J Hum Genet. 1997 Mar-Apr;5(2):94-8) the bisulfite technique is only used in research. Always, however, short, specific fragments of a known gene are amplified subsequent to a bisulfite treatment and either completely sequenced (Olek A, Walter J. The pre-implantation ontogeny of the H19 methylation imprint. Nat Genet. 1997 Nov;17(3):275-6) or individual cytosine positions are detected by a primer extension reaction (Gonzalgo ML, Jones PA. Rapid quantitation of methylation differences at specific sites using methylation-sensitive single nucleotide primer extension (Ms-SNuPE). Nucleic Acids Res. 1997 Jun 15;25(12):2529-31, WO 95/00669) or by enzymatic digestion (Xiong Z, Laird PW. COBRA: a sensitive and quantitative DNA methylation assay. Nucleic Acids Res. 1997 Jun 15;25(12):2532-4). In addition, detection by hybridization has also been described (Olek et al., WO 99/28498).

Further publications dealing with the use of the bisulfite technique for methylation detection in individual genes are: Grigg G, Clark S. Sequencing 5-methylcytosine residues in genomic DNA. Bioessays. 1994 Jun;16(6):431-6, 431; Zeschnigk M, Schmitz B, Dittrich B, Buiting K, Horsthemke B, Doerfler W. Imprinted segments in the human genome: different DNA methylation patterns in the Prader-Willi/Angelman syndrome region as determined by the genomic sequencing method. Hum Mol Genet. 1997 Mar;6(3):387-95; Feil R, Charlton J, Bird AP, Walter J, Reik W. Methylation analysis on individual chromosomes: improved protocol for bisulphite genomic sequencing. Nucleic Acids Res. 1994 Feb 25;22(4):695-6; Martin V, Ribieras S, Song-Wang X, Rio MC, Dante R. Genomic sequencing indicates a correlation between DNA hypomethylation in the 5' region of the pS2 gene and its expression in human breast cancer cell lines. Gene. 1995 May 19;157(1-2):261-4; WO 97/46705, WO 95/15373 and WO 97/45560.

An overview of the Prior Art in oligomer array manufacturing can be gathered from a special edition of Nature Genetics (Nature Genetics Supplement, Volume 21, January 1999), published in January 1999, and from the literature cited therein.

Fluorescently labeled probes are often used for the scanning of immobilized DNA arrays. The simple attachment of Cy3 and Cy5 dyes to the 5'-OH of the specific probe are particularly suitable for fluorescence labels. The detection of the fluorescence of the hybridized probes may be carried out, for example via a confocal microscope. Cy3 and Cy5 dyes, besides many others, are commercially available.

Matrix Assisted Laser Desorption Ionization Mass Spectrometry (MALDI-TOF) is a very efficient development for the analysis of biomolecules (Karas M, Hillenkamp F. Laser desorption ionization of proteins with molecular masses exceeding 10,000 daltons. Anal Chem. 1988 Oct 15;60(20):2299-301). An analyte is embedded in a light-absorbing matrix. The matrix is evaporated by a short laser pulse thus transporting the analyte molecule into the vapor phase in an unfragmented manner. The analyte is ionized by collisions with matrix molecules. An applied voltage accelerates the ions into a field-free flight tube. Due to their different masses, the ions are accelerated at different rates. Smaller ions reach the detector sooner than bigger ones.

MALDI-TOF spectrometry is excellently suited to the analysis of peptides and proteins. The analysis of nucleic acids is somewhat more difficult (Gut I G, Beck S. DNA and Matrix Assisted Laser Desorption Ionization Mass Spectrometry. Current Innovations and Future Trends. 1995, 1; 147-57). The sensitivity to nucleic acids is approximately 100 times worse than to peptides and decreases disproportionally with increasing fragment size. For nucleic acids having a multiply negatively charged backbone, the ionization process via the matrix is considerably less efficient. In MALDI-TOF spectrometry, the selection of the matrix plays an eminently important role. For the desorption of peptides, several very efficient matrixes have been found which produce a very fine crystallization. There are now several responsive matrixes for DNA, however, the difference in sensitivity has not been reduced. The difference in sensitivity can be reduced by chemically modifying the DNA in such a manner that it becomes more similar to a peptide. Phosphorothioate nucleic acids in which the usual phosphates of the backbone are substituted with thiophosphates can be converted into a charge-neutral DNA using simple alkylation chemistry (Gut IG, Beck S. A procedure for selective DNA alkylation and detection by mass spectrometry. Nucleic Acids Res. 1995 Apr 25;23(8):1367-73). The coupling of a charge tag to this modified DNA results in an increase in sensitivity to the same level as that found for peptides. A further advantage of charge tagging is the increased stability of the analysis against impurities which make the detection of unmodified substrates considerably more difficult.

Genomic DNA is obtained from DNA of cell, tissue or other test samples using standard methods. This standard methodology is found in references such as Fritsch and Maniatis eds., Molecular Cloning: A Laboratory Manual, 1989.

### Description

The object of the present invention is to provide an amplificate of DNA of genes associated with apoptosis, as well as a method which is suitable for the diagnosis of genetic and epigenetic parameters of genes associated with apoptosis. The present invention is based on the discovery that genetic and epigenetic parameters and, in particular, the cytosine methylation pattern of genes associated with apoptosis are particularly suitable for the diagnosis of diseases associated with apoptosis.

This objective is achieved according to the present invention by a nucleic acid amplificate as claimed in claim 14. In the table, after the listed gene designations, the respective data bank numbers (accession numbers) are specified which define the appertaining gene sequences as unique. GenBank was used as the underlying data bank, which is located at the National Institute of Health, internet address www.ncbi.nlm.nih.gov.

The chemically modified nucleic acid could heretofore not be connected with the ascertainment of genetic and epigenetic parameters.

The present invention further describes an oligonucleotide or oligomer for detecting the cytosine methylation state in chemically pretreated DNA, containing at least one base sequence having a length of at least 13 nucleotides which hybridizes to a chemically pretreated DNA of genes associated with apoptosis according to Seq. ID No. 73 through Seq. ID No. 74 and sequences complementary thereto. The oligomer probes as described constitute important and effective tools which, for the first time, make it possible to ascertain the genetic and epigenetic parameters of genes associated with apoptosis. The base sequence of the oligomers preferably contains at least one CpG dinucleotide. The probes may also exist in the form of a PNA (peptide nucleic acid) which has particularly preferred pairing properties. Particularly preferred are oligonucleotides as described in which the cytosine of the CpG dinucleotide is the 5th - 9th nucleotide from the 5'-end of the 13-mer; in the case of PNA-oligomers, it is preferred for the cytosine of the CpG dinucleotide to be the 4th - 6th nucleotide from the 5'-end of the 9-mer.

The oligomers as described are normally used in so called "sets" which contain at least one oligomer for each of the CpG dinucleotides of the sequences of Seq. ID No. 73 through Seq. ID No. 74 and sequences complementary thereto. Preferred is a set which contains at least one oligomer for each of the CpG dinucleotides from one of Seq. ID No. 73 through Seq. ID No. 74 and sequences complementary thereto.

Moreover, the present invention describes a set of at least two oligonucleotides which can be used as so-called "primer oligonucleotides" for amplifying DNA sequences of one of Seq. ID No. 73 through Seq. ID No. 74 and sequences complementary thereto.

In the case of the sets of oligonucleotides as described, it is preferred that at least one oligonucleotide is bound to a solid phase.

The present invention moreover describes a set of at least 10 n (oligonucleotides and/or PNA-oligomers) used for detecting the cytosine methylation state in chemically pretreated genomic DNA (Seq. ID No. 73 through Seq. ID No. 74 and sequences complementary thereto. These probes enable diagnosis of genetic and epigenetic parameters of genes associated with apoptosis. The set of oligomers may also be used for detecting single nucleotide polymorphisms (SNPs) in the chemically pretreated DNA of genes associated with apoptosis according to one of Seq. ID No. 73 through Seq. ID No. 74 and sequences complementary thereto.

According to the present invention, it is preferred that an arrangement of different oligonucleotides and/or PNA-oligomers (a so-called "array") made available by the present invention is present in a manner that it is likewise bound to a solid phase. This array of different oligonucleotide- and/or PNA-oligomer sequences can be characterized in that it is arranged on the solid phase in the form of a rectangular or hexagonal lattice. The solid phase surface is preferably composed of silicon, glass, polystyrene, aluminum, steel, iron, copper, nickel, silver, or gold. However, nitrocellulose as well as plastics such as nylon which can exist in the form of pellets or also as resin matrices are possible as well.

Therefore, the further described is a method for manufacturing an array fixed to a carrier material for analysis in connection with diseases associated with apoptosis in which method at least one oligomer as described is coupled to a solid phase. Methods for manufacturing such arrays are known, for example, from US Patent 5,744,305 by means of solid-phase chemistry and photolabile protecting groups.

A further subject matter of the present invention relates to the use of a DNA chip for the analysis of diseases associated with apoptosis according to claim 17. DNA chips are known, for example, for US Patent 5,837,832.

Moreover, also described is a kit which may be composed, for example, of a bisulfite-containing reagent, a set of primer oligonucleotides containing at least two oligonucleotides whose sequences in each case correspond or are complementary to an 18 base long segment of the base sequences specified in the appendix (Seq. ID No. 73 through Seq. ID No. 74 and sequences complementary thereto, oligonucleotides and/or PNA-oligomers as well as instructions for carrying out and evaluating the described method. However, a kit along the lines as described can also contain only part of the aforementioned components.

The present invention also makes available a method for ascertaining genetic and/or epigenetic parameters suitable for the diagnosis of diseases associated with apoptosis by analyzing cytosine methylations and single nucleotide polymorphisms, including the following steps:

In the first step of the method, a genomic DNA sample is chemically treated in such a manner that cytosine bases which are unmethylated at the 5'-position are converted to uracil, thymine, or another base which is dissimilar to cytosine in terms of hybridization behavior. This will be understood as 'chemical pretreatment' hereinafter.

The genomic DNA to be analyzed is preferably obtained form usual sources of DNA such as cells or cell components, for example, cell lines, biopsies, blood, sputum, stool, urine, cerebral-spinal fluid, tissue embedded in paraffin such as tissue from eyes, intestine, kidney, brain, heart, prostate, lung, breast or liver, histologic object slides, or combinations thereof.

The above described treatment of genomic DNA is carried out with bisulfite (hydrogen sulfite, disulfite) and subsequent alkaline hydrolysis which results in a conversion of non-methylated cytosine nucleobases to uracil or to another base which is dissimilar to cytosine in terms of base pairing behavior.

Fragments of the chemically pretreated DNA are amplified, using a set of at least two primer oligonucleotides as described, and a preferably heat-stable polymerase. Because of statistical and practical considerations, preferably more than ten different fragments having a length of 100 - 2000 base pairs are amplified. The amplification of several DNA segments can be carried out simultaneously in one and the same reaction vessel. Usually, the amplification is carried out by means of a polymerase chain reaction (PCR).

The set of primer oligonucleotides preferably includes at least two olignonucleotides whose sequences are each reverse complementary or identical to an at least 18 base-pair long segment of the base sequences specified in the appendix (Seq. ID No. 73 through Seq. ID No. 74 and sequences complementary thereto). The primer oligonucleotides are preferably characterized in that they do not contain any CpG dinucleotides.

According to the present invention, it is preferred that at least one primer oligonucleotide is bonded to a solid phase during amplification. The different oligonucleotide and/or PNA-oligomer sequences can be arranged on a plane solid phase in the form of a rectangular or hexagonal lattice, the solid phase surface preferably being composed of silicon, glass, polystyrene, aluminum, steel, iron, copper, nickel, silver, or gold, it being possible for other materials such as nitrocellulose or plastics to be used as well.

The fragments obtained by means of the amplification can carry a directly or indirectly detectable label. Preferred are labels in the form of fluorescence labels, radionuclides, or detachable molecule fragments having a typical mass which can be detected in a mass spectrometer, it being preferred that the fragments that are produced have a single positive or negative net charge for better detectability in the mass spectrometer. The detection may be carried out and visualized by means of matrix assisted laser desorption/ionization mass spectrometry (MALDI) or using electron spray mass spectrometry (ESI).

The amplificates obtained in the second step of the method are subsequently hybridized to an array or a set of at least two oligonucleotides and/or PNA probes. In this context, the hybridization takes place in the manner described in the following. The set of probes used during the hybridization is preferably composed of at least 10 oligonucleotides or PNA-oligomers. In the process, the amplificates serve as probes which hybridize to oligonucleotides previously bonded to a solid phase. The non-hybridized fragments are subsequently removed. Said oligonucleotides contain at least one base sequence having a length of 13 nucleotides which is reverse complementary or identical to a segment of the base sequences specified in the appendix, the segment containing at least one CpG dinucleotide. The cytosine of the CpG dinucleotide is the 5th to 9th nucleotide from the 5'-end of the 13-mer. One oligonucleotide exists for each CpG dinucleotide. Said PNA-oligomers contain at least one base sequence having a length of 9 nucleotides which is reverse complementary or identical to a segment of the base sequences specified in the appendix, the segment containing at least one CpG dinucleotide. The cytosine of the CpG dinucleotide is the 4th to 6th nucleotide seen from the 5'-end of the 9-mer. One oligonucleotide exists for each CpG dinucleotide.

In the fourth step of the method, the non-hybridized amplificates are removed.

In the final step of the method, the hybridized amplificates are detected. In this context, it is preferred that labels attached to the amplificates are identifiable at each position of the solid phase at which an oligonucleotide sequence is located.

According to the present invention, it is preferred that the labels of the amplificates are fluorescence labels, radionuclides, or detachable molecule fragments having a typical mass which can be detected in a mass spectrometer. The mass spectrometer is preferred for the detection of the amplificates, fragments of the amplificates or of probes which are complementary to the amplificates, it being possible for the detection to be carried out and visualized by means of matrix assisted laser desorption/ionization mass spectrometry (MALDI) or using electron spray mass spectrometry (ESI).

The produced fragments may have a single positive or negative net charge for better detectability in the mass spectrometer. The aforementioned method is preferably used for ascertaining genetic and/or epigenetic parameters of genes associated with apoptosis.

The oligomers as described or arrays thereof as well as a kit as described are intended to be used for the diagnosis of diseases associated with apoptosis by analyzing methylation patterns of genes associated with apoptosis. According to the present invention, the method is preferably used for the diagnosis of important genetic and/or epigenetic parameters within genes associated with apoptosis.

The method according to the present invention is used, for example, for the diagnosis of solid tumors and cancers.

The nucleic acids as described of Seq. ID No. 73 through Seq. ID No. 74 and sequences complementary thereto can be used for the diagnosis of genetic and/or epigenetic parameters of genes associated with apoptosis.

Also described is a method for manufacturing a diagnostic agent and/or therapeutic agent for the diagnosis of diseases associated with apoptosis by analyzing methylation patterns of genes associated with apoptosis, the diagnostic agent and/or therapeutic agent being characterized in that at least one nucleic acid as described is used for manufacturing it, possibly together with suitable additives and auxiliary agents.

The present disclosure further relates to a diagnostic agent and/or therapeutic agent for diseases associated with apoptosis by analyzing methylation patterns of genes associated with apoptosis, the diagnostic agent and/or therapeutic agent containing at least one nucleic acid as described, possibly together with suitable additives and auxiliary agents.

The present invention moreover relates to the diagnosis of events which are disadvantageous to patients or individuals in which important genetic and/or epigenetic parameters within genes associated with apoptosis said parameters obtained by means of the present invention may be compared to another set of genetic and/or epigenetic parameters, the differences serving as the basis for a diagnosis and/or prognosis of events which are disadvantageous to patients or individuals.

In the context of the present invention the term "hybridization" is to be understood as a bond of an oligonucleotide to a completely complementary sequence along the lines of the Watson-Crick base pairings in the sample DNA, forming a duplex structure. To be understood by "stringent hybridization conditions" are those conditions in which a hybridization is carried out at 60°C in 2.5 x SSC buffer, followed by several washing steps at 37°C in a low buffer concentration, and remains stable.

The term "functional variants" denotes all DNA sequences which are complementary to a DNA sequence, and which hybridize to the reference sequence under stringent conditions and have an activity similar to the corresponding polypeptide.

In the context of the present invention, "genetic parameters" are mutations and polymorphisms of genes associated with apoptosis and sequences further required for their regulation. To be designated as mutations are, in particular, insertions, deletions, point mutations, inversions and polymorphisms and, particularly preferred, SNPs (single nucleotide polymorphisms).

In the context of the present invention, "epigenetic parameters" are, in particular, cytosine methylations and further chemical modifications of DNA bases of genes associated with apoptosis and sequences further required for their regulation. Further epigenetic parameters include, for example, the acetylation of histones which, however, cannot be directly analyzed using the described method but which, in turn, correlates with the DNA methylation.

In the following, the present invention will be explained in greater detail on the basis of the sequences and examples with reference to the accompanying figure without being limited thereto.

### Figure 1

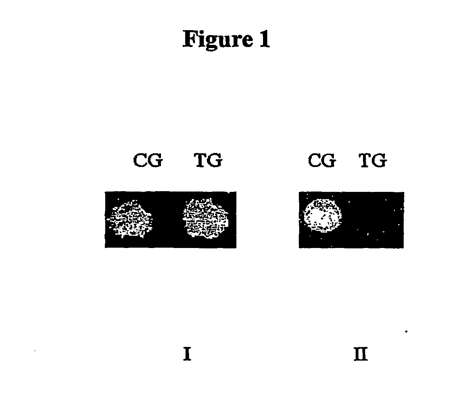

Figure 1 shows the hybridisation of fluorescent labelled amplificates to a surface bound olignonucleotide. Sample I being from healthy tissue and sample II being from pilocytic astrocytoma (tumor) tissue. Flourescence at a spot shows hybridisation of the amplificate to the olignonucleotide. Hybridisation to a CG olignonucleotide denotes methylation at the cytosine position being analysed, hybridisation to a TG olignonucleotide denotes no methylation at the cytosine position being analysed.

### Sequence ID Nos. 1 to 74

Sequences having odd sequence numbers (e.g., Seq. ID No. 1, 3, 5, ...) exhibit in each case sequences of the chemically pretreated genomic DNAs of different genes associated with apoptosis. Sequences having even sequence numbers (e.g., Seq. ID No. 2, 4, 6, ...) exhibit in each case the sequences of the chemically pretreated genomic DNAs of genes associated with apoptosis which are complementary to the preceding sequences (e.g., the complementary sequence to Seq. ID No.1 is Seq. ID No.2, the complementary sequence to Seq. ID No.3 is Seq. ID No.4, etc.)

### Sequence ID Nos. 75 to 78

Sequence ID Nos. 75 to 78 show the sequences of oligonucleotides used in Example 1.

The following example relates to a fragment of a gene associated with apoptosis, in this case, death-associated protein 1 (DAPK1) in which a specific CG-position is analyzed for its methylation status.

### Example 1:Methylation analysis in the gene DAPK1 associated with apoptosis.

The following example relates to a fragment of the gene DAPK1 in which a specific CG-position is to be analyzed for methylation.

In the first step, a genomic sequence is treated using bisulfite (hydrogen sulfite, disulfite) in such a manner that all cytosines which are not methylated at the 5-position of the base are modified in such a manner that a different base is substituted with regard to the base pairing behavior while the cytosines methylated at the 5-position remain unchanged.

If bisulfite solution is used for the reaction, then an addition takes place at the non-methylated cytosine bases. Moreover, a denaturating reagent or solvent as well as a radical interceptor must be present. A subsequent alkaline hydrolysis then gives rise to the conversion of non-methylated cytosine nucleobases to uracil. The chemically converted DNA (sequence ID Nos. 73 and 74) is then used for the detection of methylated cytosines. In the second method step, the treated DNA sample is diluted with water or an aqueous solution. Preferably, the DNA is subsequently desulfonated (10-30 min, 90-100 °C) at an alkaline pH value. In the third step of the method, the DNA sample is amplified in a polymerase chain reaction, preferably using a heat-resistant DNA polymerase. In the present case, cytosines of the gene DAPK1 are analyzed. To this end, a defined fragment having a length of 465 bp is amplified with the specific primer oligonucleotides ATTAATATTATGTAAAGTGA (Sequence ID No. 75) and CTTACAACCATTCACCCACA (Sequence ID No. 76). This amplificate serves as a sample which hybridizes to an oligonucleotide previously bonded to a solid phase, forming a duplex structure, for example GTTATATCGTGGAGGATA (Sequence ID No. 77), the cytosine to be detected being located at position 135 of the amplificate. The detection of the hybridization product is based on Cy3 and Cy5 flourescently labeled primer oligonucleotides which have been used for the amplification. A hybridization reaction of the amplified DNA with the oligonucleotide takes place only if a methylated cytosine was present at this location in the bisulfite-treated DNA. Thus, the methylation status of the specific cytosine to be analyzed is inferred from the hybridization product.

In order to verify the methylation status of the position, a sample of the amplificate is further hybridized to another oligonucleotide previously bonded to a solid phase. Said olignonucleotide is identical to the oligonucleotide previously used to analyze the methylation status of the sample, with the exception of the position in question. At the position to be analysed said oligonucleotide comprises a thymine base as opposed to a cytosine base i.e GTTATATTGTGGAGGATA (Sequence ID No. 78). Therefore, the hybridisation reaction only takes place if an unmethylated cytosine was present at the position to be analysed. The procedure was carried out on cell samples from 2 patients, sample I being from normal healthy tissue and sample II being from a pilocytic astrocytoma tumor sample.

From the results (see Figure 1) it can be seen that the sample I contained a mixture of both methylated and unmethylated cells at position of the amplificate whereas sample II contained only methylated cells at position 135 of the amplificate.

### Example 2: Diagnosis of diseases associated with apoptosis

In order to relate the methylation patterns to one of the diseases associated with apoptosis, it is initially required to analyze the DNA methylation patterns of a group of diseased and of a group of healthy patients. These analyses are carried out, for example, analogously to Example 1. The results obtained in this manner are stored in a database and the CpG dinucleotides which are methylated differently between the two groups are identified. This can be carried out by determining individual CpG methylation rates as can be done, for example, in a relatively imprecise manner, by sequencing or else, in a very precise manner, by a methylation-sensitive "primer extension reaction". It is also possible for the entire methylation status to be analyzed simultaneously, and for the patterns to be compared, for example, by clustering analyses which can be carried out, for example, by a computer.

Subsequently, it is possible to allocate the examined patients to a specific therapy group and to treat these patients selectively with an individualized therapy.

Example 2 can be carried out, for example, for the following diseases:
Solid tumors and cancers.

| Gene | Database entry No. (GenBank, intemet address www.ncbi.nlm.nih.gov) |
|---|---|
| TNFRSF6 | (NM_000043), |
| TNFRSF6 | (NM_000043), |
| TNFRSF6 | (NM_000043), |
| BCL2 | (NM_000633), |
| BCL2L1 | (NM_001191), |
| BCL2L2 | (NM_004050), |
| BNIP3 | (NM_004052), |
| CASP3 | (NM_004346), |
| CASP7 | (NM_001227), |
| PDCD1 | (NM_005018), |
| SFRP5 | (NM_003015), |
| STK17A | (NM_004760), |
| REQ | (NM_006268) |

## Claims

1. A method for ascertaining genetic and/or epigenetic parameters suitable for the diagnosis of existing diseases or the predisposition to specific diseases by analyzing cytosine methylations, **characterized in that** the following steps are carried out:
a) in a genomic DNA sample, cytosine bases which are unmethylated at the 5-position are converted, by chemical treatment by means of a solution of a bisulfite, hydrogen sulfite or disulfite, to uracil or another base which is dissimilar to cytosine in terms of hybridization behaviour;
b) fragments of said chemically pretreated genomic DNA sample are amplified using a set of at least two primer oligonucleotides and a polymerase, wherein the oligomers comprise in each case at least one base sequence having a length of at least 9 nucleotides which hybridizes to or is identical to a chemically pretreated DNA of a gene associated with apoptosis according to one of the Seq ID Nos 73 or 74 and sequences complementary thereto,
c) the amplificates are hybridized to a set of at least two oligonucleotides and/or PNA probes which in each case comprise a base sequence having a length of at least 9 nucleotides which hybridizes to or is identical to a chemically pretreated DNA of a gene associated with apoptosis according to one of the Seq ID Nos 73 or 74 or sequences complementary thereto or else to an array of these oligonucleotides and/or PNA probes; and
d) the hybridized amplificates are subsequently detected.

2. Method according to claim 1, wherein the amplification of the chemically pretreated genomic DNA using sets of primer oligonucleotides and a polymerase is performed in a way that the amplificates carry a detectable label.

3. Method according to claim 1 or 2, further comprising the step of hybridizing the amplificates to a set of oligomers (oligonucleotides and/or PNA probes) or to an array, wherein the base sequence of the oligomers includes at least one CpG dinucleotide.

4. Method according to claim 3, **characterized in that** the cytosine of the CpG dinucleotide is located in the middle third of the oligomer.

5. Method according to any of claims 1 to 4, wherein a set of oligomers is used, comprising oligomers for detecting the methylation state of all CpG dinucleotides within one of the sequences according to Seq. ID Nos. 73 or 74, and sequences complementary thereto.

6. Method according to any of claims 1 to 5, **characterized in that** more than ten different fragments having a length of 100 - 2000 base pairs are amplified.

7. Method according to any of claims 1 to 6, **characterized in that** the amplification of several DNA segments is carried out in one reaction vessel.

8. Method according to any of claims 1 to 7, **characterized in that** the polymerase is a heat-resistant DNA polymerase and wherein the amplification is preferred carried out by means of the polymerase chain reaction (PCR).

9. Method according to any of claims 2 to 7, **characterized in that** the labels of the amplificates are selected from the group of fluorescence labels, radionuclides, and detachable molecule fragments having a typical mass which are detected in a mass spectrometer.

10. Method according to claim 9, **characterized in that** the produced fragments have a single positive or negative net charge for better detectability in a mass spectrometer.

11. Method according to claim 9 or 10, **characterized in that** the amplificates or fragments of the amplificates are detected in a mass spectrometer, wherein the detection preferably is carried out and visualized by means of matrix assisted laser desorption/ionization mass spectrometry (MALDI) or using electron spray mass spectrometry (ESI).

12. Method according to any of claims 1 to 11, **characterized in that** the genomic DNA is obtained from cells or cellular components which contain DNA, sources of DNA comprising, for example, cell lines, biopsies, blood, sputum, stool, urine, cerebral-spinal fluid, tissue embedded in paraffin such as tissue from eyes, intestine, kidney, brain, heart, prostate, lung, breast or liver, histologic object slides, and all possible combinations thereof.

13. Method according to any of claims 1 to 12, further comprising the step of performing a diagnosis of a disease selected from solid tumours and cancers.

14. A nucleic acid amplificate, produced by
a) chemical conversion of cytosine bases which are unmethylated at the 5-position into uracil or another base which is dissimilar to cytosine in terms of hybridization behaviour in a genomic DNA-sample by means of a solution of a bisulfite, hydrogen sulfite or disulfite and subsequent alkaline hydrolysis, and
b) PCR-amplification of fragments from said pretreated genomic DNA using sets of at least two primer oligonucleotides and a polymerase, wherein the primer oligonucleotides comprise a base sequence having a length of at least 18 nucleotides which is reverse complementary to or is identical to a chemically pretreated DNA of a gene associated with apoptosis according to one of the Seq ID Nos 73 or 74 and sequences complementary thereto, wherein the fragments have a length of 100 - 2000 basepairs.

15. The nucleic acid according to Claim 14 that carries a detectable marker.

16. The nucleic acid according to Claim 14 or 15 for use in the diagnosis of diseases.

17. Use of an arrangement of a set of at least two different oligomers (array) which in each case comprise a base sequence having a length of at least 9 nucleotides which is identical to a chemically pretreated DNA of a gene associated with apoptosis according to one of the Seq ID Nos 73 or 74 or sequences complementary thereto fixed to a carrier material for analyzing diseases associated with the methylation state of the CpG dinucleotides of one of the Seq. ID No. 73 or Seq. ID No. 74 or sequences complementary thereto.

18. Use of an array according to claim 17 in a method for the diagnosis of solid tumours and cancers according to claim 13.

## Patentansprüche

1. Verfahren zur Ermittlung von genetischen und/oder epigenetischen Parametern, die für die Diagnose von bestehenden Erkrankungen oder der Prädisposition für spezifische Erkrankungen geeignet sind, durch analysieren von Cytosinmethylierungen, **dadurch gekennzeichnet, dass** die folgenden Schritte durchgeführt werden:
a) in einer genomischen DNA Probe werden Cytosinbasen, die an der 5-Position nicht methyliert sind, durch chemische Behandlung mittels einer Lösung eines Bisulfits, Hydrogensulfits oder Disulfits, zu Uracil oder einer anderen Base, die hinsichtlich ihres Hybridisierungsverhaltens von Cytosin unähnlich ist umgewandelt;
b) Fragmente der chemisch vorbehandelten genomischen DNA Probe werden unter der Verwendung eines Sets von mindestens zwei Primeroligonukleotiden und einer Polymerase amplifiziert, wobei die Oligomere in jedem Fall mindestens eine Basensequenz mit einer Länge von mindestens 9 Nukleotiden umfassen, die an eine chemisch vorbehandelte DNA eines Gens, das mit Apoptose assoziiert ist, nach einer der Seq ID Nrn. 73 oder 74 und dazu komplementären Sequenzen, hybridisiert oder dazu identisch ist,
c) die Amplifikate werden an ein Set von mindestens zwei Oligonukleotiden und/oder PNA Sonden hybridisiert, die in jedem Fall eine Basensequenz umfassen, die eine Länge von mindestens 9 Nukleotiden aufweisen, die an eine chemisch vorbehandelte DNA eines Gens, das mit Apoptose assoziiert ist nach einer der Seq ID Nrn. 73 oder 74 und dazu komplementären Sequenzen oder andererseits an eine Anordnung dieser Oligonukleotide und/oder PNA Sonden hybridisiert oder dazu identisch ist; und
d) die hybridisierten Amplifikate werden anschließend nachgewiesen.

2. Verfahren nach Anspruch 1, wobei die Amplifikation der chemisch vorbehandelten genomischen DNA unter der Verwendung von Sets von Primeroligonukleotiden und einer Polymerase auf eine Weise durchgeführt wird, dass die Amplifikate einen nachweisbaren Marker tragen.

3. Verfahren nach Anspruch 1 oder 2, weiter umfassend den Schritt von hybridisieren der Amplifikate an ein Set von Oligomeren (Oligonukleotide und/oder PNA Sonden) oder an eine Anordnung, wobei die Basensequenz der Oligomere mindestens ein CpG Dinukleotid einschließt.

4. Verfahren nach Anspruch 3, **dadurch gekennzeichnet, dass** das Cytosin des CpG Dinukleotids im mittleren Drittel des Oligomers lokalisiert ist.

5. Verfahren nach einem der Ansprüche 1 bis 4, wobei ein Set an Oligomeren verwendet wird, umfassend Oligomere zum Nachweis des Methylierungszustands aller CpG Dinukleotide innerhalb einer der Sequenzen nach Seq. ID Nrn. 73 oder 74, und dazu komplementären Sequenzen.

6. Verfahren nach einem der Ansprüche 1 bis 5, **dadurch gekennzeichnet, dass** mehr als zehn verschiedene Fragmente mit einer Länge von 100 - 2000 Basenpaaren amplifiziert werden.

7. Verfahren nach einem der Ansprüche 1 bis 6, **dadurch gekennzeichnet, dass** die Amplifikation von mehreren DNA Segmenten in einem Reaktionsgefäß durchgeführt wird.

8. Verfahren nach einem der Ansprüche 1 bis 7, **dadurch gekennzeichnet, dass** die Polymerase eine Hitze-resistente DNA Polymerase ist, und wobei die Amplifikation bevorzugt mittels der Polymerase-Kettenreaktion (PCR) durchgeführt wird.

9. Verfahren nach einem der Ansprüche 2 bis 7, **dadurch gekennzeichnet, dass** die Marker der Amplifikate ausgewählt sind aus der Gruppe von Fluoreszenmarkern, Radionukliden und ablösbaren Molekülfragmenten, die eine typische Masse aufweisen, die in einem Massenspektrometer nachgewiesen wird.

10. Verfahren nach Anspruch 9, **dadurch gekennzeichnet, dass** die hergestellten Fragmente eine einzelne positive oder negative Nettoladung für eine bessere Nachweisbarkeit in einem Massenspektrometer aufweisen.

11. Verfahren nach Anspruch 9 oder 10, **dadurch gekennzeichnet, dass** die Amplifikate oder Fragmente der Amplifikate in einem Massenspektrometer nachgewiesen werden, wobei der Nachweis bevorzugt mittels Matrix-unterstützter Laserdesorptions-/Ionisierungs-Massenspektrometrie (MALDI) oder unter der Verwendung von Elektronenspray-Massenspektrometrie (ESI) durchgeführt und visualisiert wird.

12. Verfahren nach einem der Ansprüche 1 bis 11, **dadurch gekennzeichnet, dass** die genomische DNA aus Zellen oder zellulären Komponenten, die DNA enthalten, erhalten wird, wobei Quellen an DNA zum Beispiel umfassen, Zelllinien, Biopsien, Blut, Sputum, Stuhl, Urin, cerebrospinale Flüssigkeit, Gewebe eingebettet in Paraffin, wie zum Beispiel Gewebe von Augen, Darm, Niere, Hirn, Herz, Prostata, Lunge, Brust oder Leber, histologische Objektträger und alle mögliche Kombinationen davon.

13. Verfahren nach einem der Ansprüche 1 bis 12, weiter umfassend den Schritt von Durchführen einer Diagnose einer Erkrankung ausgewählt aus soliden Tumoren und Krebs.

14. Nukleinsäureamplifikat, hergestellt durch
a) chemische Umwandlung von Cytosinbasen, die an der 5-Position nicht methyliert sind, in Uracil oder eine andere Base, die im Hinblick auf ihr Hybridisierungsverhalten von Cytosine unähnlich ist, in einer genomischen DNA-Probe mittels einer Lösung eines Bisulfits, Hydrogensulfits oder Disulfits und anschließender alkalischer Hydrolyse, und
b) PCR-Amplifikation von Fragmenten aus der vorbehandelten genomischen DNA unter der Verwendung von mindestens zwei Primeroligonukleotiden und einer Polymerase, wobei die Primeroligonukleotide eine Basensequenz umfassen, die eine Länge von mindestens 18 Nukleotiden aufweist, die revers komplementär oder identisch ist zu einer chemisch vorbehandelten DNA eines Gens, das mit Apoptose assoziiert ist nach einer der Seq ID Nrn. 73 oder 74 und dazu komplementärer Sequenzen, wobei die Fragmente eine Länge von 100 - 2000 Basenpaaren aufweisen.

15. Nukleinsäure nach Anspruch 14, die einen nachweisbaren Marker trägt.

16. Nukleinsäure nach Anspruch 14 oder 15 zur Verwendung in der Diagnose von Erkrankungen.

17. Verwendung einer Anordnung eines Sets von mindestens zwei verschiedenen Oligomeren (Array), die in jedem Fall eine Basensequenz umfassen, die eine Länge von mindestens 9 Nukleotiden aufweist, die identisch ist zu einer chemisch vorbehandelten DNA eines Gens, das mit Apoptose assoziiert ist nach einer der Seq ID Nrn. 73 oder 74 oder dazu komplementären Sequenzen, fixiert an ein Trägermaterial zur Analyse von Erkrankungen, die mit dem Methylierungszustand der CpG Dinukleotide einer der Seq. ID Nr. 73 oder Seq. ID Nr. 74 oder dazu komplementärer Sequenzen assoziiert sind.

18. Verwendung einer Anordnung nach Anspruch 17 in einem Verfahren zur Diagnose von soliden Tumoren und Krebs nach Anspruch 13.

## Revendications

1. Procédé pour assurer des paramètres génétiques et/ou épigénétiques, approprié au diagnostic de maladies existantes ou d'une prédisposition à des maladies définies par analyse de méthylations de cytosine, **caractérisé en ce que** les étapes suivantes sont exécutées:
a) dans un échantillon d'ADN génomique des bases cytosine non méthylées en position 5 sont converties, par traitement chimique à l'aide d'une solution d'un bisulfite, sulfite d'hydrogène ou disulfite, en uracile ou en une autre base qui concernant le comportement d'une hybridation est dissemblable de la cytosine ;
b) des fragments de l'échantillon ADN génomique chimiquement prétraité sont amplifiés en recourant à un ensemble d'au moins deux oligonucléotides primaires et une polymérase, les oligomères comprenant en tout cas au moins une séquence de base longue d'au moins 9 nucléotides, hybridée sur ou identique à un ADN chimiquement prétraité d'un gêne associé à apoptosis suivant une des séquences ID NO 73 ou ID NO 74 et séquences complémentaires,
c) les amplificats sont hybridés sur un ensemble d'au moins deux oligonucléotides et/ou PNA échantillons comprenant en tout cas une séquence de base longue d'au moins 9 nucléotides, hybridée sur ou identique à un ADN chimiquement prétraité d'un gêne associé à apoptosis suivant une des séquences ID NO 73 ou ID NO 74 ou des séquences complémentaires, ou sur un groupement de ces oligonucléotides et/ou des PNA échantillons; et
d) les amplificats hybridés sont ensuite détectés.

2. Procédé conforme à la revendication 1, l'amplification de l'échantillon ADN génomique chimiquement prétraité en recourant à un ensemble d'oligonucléotides primaires et une polymérase étant exécutée de façon que les amplificats portent une étiquette détectable.

3. Procédé conforme à la revendication 1 ou 2, comprenant en outre une étape d'hybridation des amplificats sur un ensemble d'oligomères (oligonucléotides et/ou PNA échantillons) ou sur un groupement dont la séquence de base des oligomères comprend au moins un dinucléotide CpG.

4. Procédé conforme à la revendication 3, **caractérisé en ce que** le cytosine du dinucléotide CpG est localisé au tiers central de l'oligomère.

5. Procédé conforme aux revendications 1 à 4, un ensemble d'oligomères étant utilisé, comprenant des oligomères pour détecter l'état de méthylation de tous les dinucléotides CpG compris dans une des séquences suivant les séquences ID NO 73 ou ID NO 74 et séquences complémentaires.

6. Procédé conforme aux revendications 1 à 5, **caractérisé en ce que** plus de dix fragments différents d'une longueur de 100 à 2000 paires de bases sont amplifiés.

7. Procédé conforme aux revendications 1 à 6, **caractérisé en ce que** l'amplification de plusieurs segments d'ADN est exécuté dans un seul récipient de réaction.

8. Procédé conforme aux revendications 1 à 7, **caractérisé en ce que** la polymérase est une polymérase d'ADN résistante à la chaleur et l'amplification étant de préférence exécutée à l'aide de la réaction en chaîne par polymérase (PCR).

9. Procédé conforme aux revendications 2 à 7, **caractérisé en ce que** les étiquettes des amplificats sont sélectionnées du groupe des étiquettes fluorescentes, des radionucléides et des fragments de molécule séparables ayant une masse typique détectable par un spectromètre de masse.

10. Procédé conforme à la revendication 9, **caractérisé en ce que** les fragments produits possèdent une seule charge nette positive ou négative pour une meilleure détectabilité par un spectromètre de masse.

11. Procédé conforme à la revendication 9 ou 10, **caractérisé en ce que** les amplificats ou fragments des amplificats sont détectés par un spectromètre de masse, la détection étant de préférence exécutée et visualisée à l'aide de la spectrométrie de masse désorption/ionisation par laser depuis une matrice MALDI ou en utilisant la spectrométrie de masse de spray d'électrons (ESI).

12. Procédé conforme aux revendications 1 à 11, **caractérisé en ce que** l'ADN génomique est obtenu de cellules ou de composants de cellules comportant de l'ADN, sources d'ADN comprenant par exemple des lignées cellulaires, des biopsies, du sang, du crachat, des selles, de l'urine, du fluide venant de la moelle épinière, du tissu encadré en paraffine comme par exemple du tissu des yeux, des intestines, du rein, du cerveau, du coeur, de la prostate, du poumon, de la mammaire ou de la foie, des porte-objets histologiques, et de toute combinaison possible des mêmes.

13. Procédé conforme aux revendications 1 à 12, comprenant en outre l'étape de diagnose d'une maladie choisie parmi les tumeurs solides et cancers.

14. Un amplificat d'acide nucléique produit par
a) la conversion chimique, à l'aide d'une solution d'un bisulfite, sulfite d'hydrogène ou disulfite, de bases cytosine non méthylées en position 5 en uracile ou en une autre base qui concernant le comportement d'une hybridation est dissemblable de la cytosine, dans un échantillon d'ADN génomique, suivie d'une hydrolyse alcaline, et
b) amplification par PCR des fragments du susnommé ADN génomique chimiquement prétraité en recourant à des ensembles d'au moins deux oligonucléotides primaires et une polymérase, les oligonucléotides primaires comprenant une séquence de base longue d'au moins 18 nucléotides qui est inversement complémentaire à ou identique à un ADN chimiquement prétraité d'un gêne associé à apoptosis suivant une des séquences ID NO 73 ou ID NO 74 et séquences complémentaires, dont les fragments sont longues de 100 à 2000 paires de base.

15. L'acide nucléique conforme à la revendication 14 portant une étiquette détectable.

16. L'acide nucléique conforme à la revendication 14 ou 15 pour utilisation dans le diagnostic de maladies.

17. Utilisation d'un groupement d'un ensemble d'au moins deux oligomères différents qui en tout cas comprennent une séquence de base longue d'au moins 9 nucléotides qui est identique à un ADN chimiquement prétraité d'un gêne associé à apoptosis suivant une des séquences ID NO 73 ou ID NO 74 ou séquences complémentaires, attaché à un matériau porteur pour l'analyse de maladies associées à l'état de méthylation des dinucléotides CpG d'une des séquences ID NO 73 ou ID NO 74 ou séquences complémentaires.

18. Utilisation d'un groupement conforme à la revendication 17 dans un procédé pour le diagnostic de tumeurs solides et cancers conforme à la revendication 13.


### Sequence listing

<110> Epigenomics AG
   <120> Diagnosis of Diseases Associated with Apoptosis
<160> 78
<210> 1
   <211> 6536
   <212> DNA
   <213> Artificial Sequence
<220>
   <223> chemically treated genomic DNA (Homo sapiens)
<400> 1
   ggcgtatatt tgtaatttta gttattcggg aggttgaggt aggagaattg tttgaatttg 60
   ggaggtggag gtggaggttg cggtgagttg agattgcgtt attgtatttt agtttgggta 120
   ataagagtaa aattttattt tttaaaataa aataaaataa aataaaataa aataaaataa 180
   aaaagtaatt aaaaaattta ttgaagtttt tataagaaat aaaattttta taatggaaat 240
   tttagttgat taaattttta tgtaattaaa taagtatgag tgataggttt tgtgttttgt 300
   ttagtaattt aataattttt taattttttg aaagtagaat tattttaatt tttatttgaa 360
   aaatttttat aagagtttag aaaagagtta tgtaaattga tatttaagtt atatgggttt 420
   gggtaattta tttttagtgt ttattgttaa gatgatatag gatatattag gttttttttt 480
   ttttgtaggg ttttattttg ttatttaggt tgagtgttgt ggtgtaatta aagtttattg 540
   aaattttaaa tttcggggtt taagtaattt tttaatttta attttttaag tagttgggat 600
   tataggtatg cgttattatg tttggttaat tttttgtatt ttttgtagag atagggtttt 660
   attatgttgt ttaggttggt tttgaatttt tgagtttaag attcgtttaa ttcggttttt 720
   taaagtgttg ggattatagg tgggagttat tatatttggt ttattaggtt tttatttagt 780
   agagagaaga tattaaagta atatattttt aagtttttta tgtattattt tttttttttt 840
   tttatatagg aaatggtttg aagataatga tatagtgtga ttttttgaaa ttttttttgg 900
   gtagtggaag ggtttagatt tttttttttt aatttaatta gatgaagagt gagagtaagt 960
   agtatttaag agtatataga taggtatata tatgtatttg tattttaatt attagggtcg 1020
   tgttagtacg gaaagtattt aataaagatt tgatagatcg atttttagat gttgaattac 1080
   gtgttttagt ggtaaagatt ttaatggttt tgttaaaatt ttagtgagtt tttaagtatt 1140
   ttaagatgtt aaatatttgt tttttatttg gaaaatgttt ataaatgttt tatggggata 1200
   tatttttatt ttaaaattta ttatgaataa atattttgta ataaatagta attaaagtat 1260
   atttgtttta atttttgtag taaaagattt tataagagta attaagaggt aattatttaa 1320
   ataataatta tagaatttgt ttataaaaaa tatatataat atatatattt tagagagata 1380
   gggttttgtt ttgtttttta ggttggagta tagtggtata attatgggtt attgtagttt 1440
   tgatttttta gatttaagtt atttttttat tttagttttt taagtatttg ggattatagc 1500
   gaatgttatt atatttggtt aatatttaaa aatttttcgt agatacggtt ttattatgtt 1560
   ttttaggttg gttttaaatt tttgatttta agtaattttt tcgttttagt tttttaaagt 1620
   gttgggatta taggtatgaa ttattgggtt tggtttttaa aattattttt gatatagtat 1680
   tgtgttatta ttttgtaaat atagtttaaa ttaaattttt ttaatgaatg taatatattt 1740
   attataatgt aatataggaa ttattaatag taaatttttg atgtgaggga aattttattt 1800
   ttataaagtt tagtttttta ttaaagttta ttttaggata gtaattaatt tttttaaaat 1860
   ataaaaggtt atttatttat ttaggcgaag tttcgttttg ttatttaggt tggagtgtag 1920
   tagtagattt tggtttattg taatttttat tttttaggtt taagtgattt ttttgtttta 1980
   atttttgagt agttgggatt atattaggtt tagttaattt ttgtgttttt ggtagagata 2040
   gggttttatt atgttggata ggttggtttt aaatttttga ttttaagtga ttcgtttgtt 2100
   tcggtttttt aaagtgttag gattataggc gtaagttatt gtatttggtt taaaaaggtt 2160
   ttttttttaa tttatttaaa ggaggttaag tattttaagg tataagagtc gtttatttaa 2220
   aatagtaaat ttagtatatt tatttttttt agattaaaat taatatattt cgtattagta 2280
   aaataaataa tgttatttgt tttttttttt tttttttttg agaaggagtt ttgttttatt 2340
   ttgtaggttg gagtgtagtg gtacgatttc ggtttattgt aattttcgtt ttttgggttt 2400
   aagtgatttt tttgttttag ttttttgagt agttgggatt ataggagtat gttattatgt 2460
   ttggtttttt tgtattttta gtagatatag ggttttgtta tgttggttag gttggtttta 2520
   aatttttgat tttaggtgat ttatttattt tggtttttta aagtgttggg agtataggta 2580
   tgagttattg tcggtaggtt gataatttta ttgtttttga agagggcgtt taattatttt 2640
   attgtgataa aattttgttt gtttgttttt tgagatggag tttcgttttg ttgtttaggt 2700
   tggagtgtaa tggtacgatt ttagtttatt gtaattttta tttttttatt tcgagtgatt 2760
   tttttgtttt agttttttga atagttggga ttgtaggttt gtgttattaa gtttggttaa 2820
   tttttgtatt ttttgtagag atagggtttt attatgttgg ttagtttggt tttgaatttt 2880
   tgattttatg atttgtttat ttcggttttt taaagtgttg ggattatagg tgtgagttat 2940
   cgcgtttagt cgtaataaat tttttaattt tgaaaaatat attatttggg atgagttatt 3000
   agtaaagtat aattttatat gtagtagtat aaaaaatgtt tagatgttta gggttttttt 3060
   taaaatttta ttattttgga agattaataa tagttttatt aaataatttt atatatgaat      3120
   gaaaaatgga gaaaaatttt attaagatgt taattttatt aaaatgaaat gggttatttt      3180
   tttttttcgt tagaaaaaaa aaggttggga aaataaaatt tataagttgg tttttttttt      3240
   tttttttttt ttacgagttg gttttaaggt tatttttaat atttattttt tttaatgata      3300
   ttaatatatc gttatttatt tgattttaaa attgtaattg gttagtttta agtgaatata      3360
   agtgttatgt ttttgtagtt ttattatatt taatttgtaa tttatttaat aaaagatttt      3420
   aaatttataa tgtatgtacg gtgattaaaa tatgagtatt tttttaagtt ttgttattat      3480
   aacgtttgtt tttttgggag tataatgata tggttttttt tttttttttt ttttgagacg      3540
   gagtttcgtt cggtcgttta ggttggagtg tagtgatgcg attttggttt atagtaattt      3600
   tcgtttttcg ggtttaagcg atttttttgt tttagttttc ggaggaattg ggattatagg      3660
   cgttcgttat tacgttcggt tgattattcg tatttttggt agagatgggg ttttattatg      3720
   ttggttaggt tagtttcgaa tttttgattt taagtgattt atttgtttcg gtttttaaat      3780
   ataatgattt gttttaaggt tgtaagtatt tttattaggt atttatgggg tgagtagggt      3840
   ggggtagatg tgtttcggaa tggggtatag cggataggaa gtaacggttt ataataacgt      3900
   gaacgttttt gacgtgtagc gggagtttta gaaagcgtgg tcgaatttgt agtttttaat      3960
   aaatggttcg tttttaaatt ttagttttaa ttttttttga gcgatagggg tatgttatta      4020
   gtacgggggg aaatgagata taagaaacgt tcgaaaggga aaaataaggt tgggttgttt      4080
   tttttttttg gggttaagat agaggtatag aaggtttagg tttgggaggt tggattattt      4140
   tcggttggat gttgagtcgg gtgggagttt aaatagtagg gggtttatag gaggtcggtt      4200
   taggtagttt cgcgtttatt tattaggtta ggtttaggta cgtttttagc gatagtaggt      4260
   ttaggtacgt tcgggggttt gtttagtttt cgttttcgtt gtttcgggtt acgggggttt      4320
   tttcgttttc gtttcgtttt tcgggtttcg tagtaggggt ttttttgggt ttcgattttt      4380
   ttcggttttt tattttagat atgtttggag attttaggac gataagttta gggtagtttt      4440
   tttattaggg ggagtaggac gtggtcgttt tggcgttcgt gggaattttg ggcggtgatc      4500
   gcgttttttt atagatttgg tatcgtttag agtttagttt tttttttttt ttcggtattt      4560
   tcgttgtttt attgagtttt ttagttgtta gttttatagt ttttttagga gaggtgggcg      4620
   gcgattttaa tttatagcgt tttttattgc gatattgttt taaattcgag gaaatttaaa      4680
   ttttcgggcg cgcgtaggtc gacgggattc gaggaggagg ggtaggacga aggggtcgcg      4740
   cgcgttacgt cgggcgcgtc gtcgttgttc gagttcggta ttggtgggaa cgcggcgcgt      4800
   ttttgaggtt tagttacgtt tcgttcgcgg ggtaggcggg tatttttacg cgcgcgggta      4860
   tgagtcgtgg taggagtgcg cggcggtagc ggtggtcgtt ttttggggtt tggggtgtgt      4920
   ttatttgtat aagcgggcgc gcgtcgttcg ggttgggtgg attcggcggg atttgattgt      4980
   ttcgttgttt agaggcggag aagaagaggt agcgagtgga cgtgattgtt ttatttcggg      5040
   taaaagggat agaattagag gtggggagtt tgggtagtcg gcgattcgcg aagatttgag      5100
   gtgtcgtagc ggtattcgga gtagcgtcgg gtttttttcg gggtgtagtc gtcgtcgggg      5160
   gaagggcgtt ataggtcggg aagatttttt ttttttgtgt ttagtagtgg ggtttatcgg      5220
   agggcggttc gtgggtcggg ttttatcgcg gcgtttcggg attgtggggt taggttgcgt      5280
   tgggtggacg tttatttcgt taattttcgg aggtttttgg gggttttcgt gcgtttcggg      5340
   gttgtagaga tttaggggag gcgtttgtga ggttcggatt tgtttcgggg cgaagggtat      5400
   gtggcgagat agagttttgt atttttaatt ttcggtggaa aatttttgtt gtcgtttttt      5460
   tttatcggtt tggagttttt tagttttgtt tcggtagtgt cgtttttttt attaagattt      5520
   aggcgtaaag gtttgggtaa gttgattttt tcgttttttt tttcgagtta ggttttttgg      5580
   aggtttggta gttgatttta gggattgaga atttgaggat atggaatatt tgataattat      5640
   tttgtttttg tattaggaag agaaattttg taatgaggaa gtagtaaatt tggggttttc      5700
   gtggataaac gtgcgttgtt ttttgtttta cgtttatgaa gagttttggt tttttatttt      5760
   gttgtgggtt ataaaaggtt ttgagattta gtttagataa gttgtgtagt tttaaaacgt      5820
   atttaagatt tgtatatttt agtttaatag gattaggtga aattggaaat tagttttaga      5880
   aaattagagt gttttatttg tttagttatt ggatattatt taaaaaattt atttattttt      5940
   gtttttttat atcgaatttt attattataa attaaggaat attttatgtt taagaggata      6000
   aaaacgttat taatatttta aaaaatgaga gttttgattc gaattaataa ttttttatta      6060
   gttttgtgtt tttgttgggt attttgtaag ttttgataag tataggtaaa tgttttacgt      6120
   tcgtttttgt ttttttaagt gttatatttg gtgattttaa aaatagttat tgaagtttga      6180
   taattattgt ttttttttgt gagttttaag ttatttgtgt tttgtttttg tatttcgggt      6240
   atttatattt ttattttttt tttttttttt ttttttgagt agattgtgaa ttattttagg      6300
   taaatgattt aatttttttt gtagttttgg tgtttgatat atagggaggg gtataaatgt      6360
   gtattgaatg aatgaattga taaattcgtt tagatttttt tattttatat attgtgtaaa      6420
   gttttgttaa tgttttgaaa atttgtagat attgtgaatt gggttttatg tttatggaaa      6480
   aatttgtatt atatttgaaa taaagttgtt agaaacgtta agtattttat tataaa          6536
<210> 2
   <211> 6536
   <212> DNA
   <213> Artificial Sequence
<220>
   <223> chemically treated genomic DNA (Homo sapiens)
<400> 2
   tttatagtaa aatatttgac gtttttagta attttatttt aaatataatg taaatttttt     60
   tatgggtata aaatttagtt tataatattt gtaagttttt aaaatattaa tagaatttta    120
   tataatgtat agagtgaaag agtttaaacg ggtttattaa tttatttatt taatatatat    180
   ttgtgttttt ttttatatgt tagatattaa gattatagaa aagattgagt tatttgtttg    240
   aaataattta tagtttattt aaagaagaaa aagagagaga gagtgggaat gtgaatgttc    300
   gaagtgtaaa aatagagtat aagtaatttg gagtttatag aagggggtaa taattattaa    360
   gttttaatga ttgtttttga agttattaaa tgtagtattt ggaaaaatag ggacggacgt    420
   agggtatttg tttatgtttg ttaaggttta taaaatgttt agtaggggta taaaattgat    480
   aaaaagttat tgattcgaat taaaattttt attttttaaa atgttaataa cgtttttgtt    540
   tttttaaata taaaatgttt tttaatttat agtaatgaaa ttcgatgtga aaaaataaga    600
   atgaatgaat tttttaggtg atgtttaata gttaagtaaa taagatattt tggttttttg    660
   aaattaattt ttaattttat ttagttttgt taaattaaaa tatgtaaatt ttaaatacgt    720
   tttaaaattg tataatttat ttgaattaag ttttaaaatt ttttgtaatt tataataaaa    780
   tggaaagtta aaatttttta tggacgtaaa atagaaaata gcgtacgttt atttacgaaa    840
   attttaagtt tattattttt ttattataga attttttttt ttggtatagg agtaggatgg    900
   ttattaaata ttttatgttt ttaaattttt agtttttgaa attagttatt aagtttttaa    960
   agaatttggt tcggggagaa aagcgaggag gttaatttat ttaagttttt gcgtttaggt   1020
   tttagtgggg agggcggtat tgtcgggata agattgggag attttaggtc ggtggaggga   1080
   aacggtaata ggagtttttt atcgggaatt aggggtgtag ggttttgttt cgttatatat   1140
   ttttcgtttc ggggtaggtt cgggttttat aggcgttttt tttggatttt tgtagtttcg   1200
   gggcgtacga agatttttag ggattttcga aggttggcga ggtgggcgtt tatttaacgt   1260
   agtttgattt tatagtttcg gagcgtcgcg gtgaggttcg gtttacgggt cgtttttcgg   1320
   tggattttat tattggatat aaagggagga ggttttttcg gtttgtggcg tttttttttc   1380
   gacggcggtt gtatttcgga gggagttcgg cgttatttcg gatgtcgttg cggtatttta   1440
   agttttcgcg ggtcgtcgat tgtttagatt ttttattttt ggttttattt tttttgttcg   1500
   ggatagagta gttacgttta ttcgttattt tttttttttc gtttttggat agcggagtag   1560
   ttaaatttcg tcggatttat ttagttcgga cggcgcgcgt tcgtttatgt aaataaatat   1620
   attttaagtt ttaaggggcg gttatcgttg tcgtcgcgta tttttgttac ggtttatgtt   1680
   cgcgcgcgta gaagtgttcg tttatttcgc ggacggggcg tggttaagtt ttagggacgc   1740
   gtcgcgtttt tattaatgtc ggattcgggt agcggcggcg cgttcgacgt ggcgcgcgcg   1800
   attttttcgt tttgtttttt tttttcgggt ttcgtcggtt tgcgcgcgtt cgggagtttg   1860
   aattttttcg gatttggagt aatatcgtag tggaaggcgt tgtgggttga ggtcgtcgtt   1920
   tatttttttt aggggaatta tggagttggt agttgaaaga tttagtgaag taacgaggat   1980
   gtcggggaga gggaaggggt tgggttttgg gcggtgttaa gtttgtgagg gggcgcggtt   2040
   atcgtttagg gtttttacga acgttaaggc ggttacgttt tgtttttttt ggtgaagaag   2100
   ttgttttggg tttgtcgttt tagggttttt agatatgttt gaggtgaaga gtcggaagaa   2160
   gtcggggttt aagggagttt ttgttgcgga gttcgggaag cggagcgagg gcgggaagat   2220
   tttcgtggtt cggagtagcg gaggcggggg ttgggtagat tttcgaacgt gtttgagttt   2280
   gttgtcgttg gggacgtgtt tgggtttggt ttggtaagtg gacgcgaggt tgtttgggtc   2340
   ggttttttgt gggttttttg ttgtttgggt ttttattcgg tttaatattt agtcggaggt   2400
   gatttaattt tttagatttg ggttttttgt gtttttgttt tgattttaaa ggaaggaaat   2460
   agtttagttt tgtttttttt tttcgggcgt tttttgtatt ttattttttt tcgtgttggt   2520
   agtatgtttt tatcgtttag aaggagttga ggttaaggtt tgggaacgag ttatttgtta   2580
   agagttgtag attcggttac gttttttgaa gttttcgtta tacgttagaa gcgtttacgt   2640
   tgttataaat cgttattttt tgttcgttgt gttttatttc gggatatatt tgttttattt   2700
   tatttatttt ataaatattt aatggaaata tttgtaattt taaaatagat tattatgttt   2760
   ggagatcgag gtaggtggat tatttgaggt taggagttcg agattagttt ggttaatatg   2820
   gtgaaatttt atttttatta aaaatacgaa taattagtcg ggcgtggtgg cgggcgtttg   2880
   tagttttagt tttttcggag gttgaggtag gagaatcgtt tgaattcggg aggcggaggt   2940
   tgttatgagt taagatcgta ttattgtatt ttagtttggg cgatcgagcg agatttcgtt   3000
   ttaaaaaaaa aaaaaaaaga aaattatatt attatgtttt taaaggagta aacgttatag   3060
   tagtagagtt tggaggagtg tttatatttt gattatcgtg tatgtattat gaatttaagg   3120
   ttttttatta agtaagttat agattgaata tggtaaagtt ataaaaatat agtatttgtg   3180
   tttatttaaa attaattagt tgtagtttta aaattaggta aatggcgata tattggtgtt   3240
   attaggaaaa atgaatatta gaaataattt taaaattaat tcgtaaaaaa agaaaaaaga   3300
   aaaaaattaa tttgtgaatt ttattttttt agtttttttt tttttggcgg gaggagggaa   3360
   tagtttattt tattttaata agattggtat tttaatagaa ttttttttta ttttttattt   3420
   atgtgtaaag ttatttagta agattgttgt taatttttta gagtgataga attttagaag   3480
   agattttgga tatttagata ttttttatgt tattatatat ggaattatat tttattggtg   3540
   atttatttta aatagtgtat tttttagagt taaaagattt gttacggttg ggcgcggtgg   3600
   tttatatttg taattttagt attttgggag gtcgaggtgg gtagattatg aggttaggag   3660
   tttaagatta ggttggttaa tatggtgaaa ttttgttttt ataaaaaata taaaaattag   3720
   ttaggtttgg tggtataaat ttgtaatttt agttatttag gaggttgagg taggagaatt   3780
   attcgaaatg gggaggtgga ggttgtagtg agttgggatc gtgttattgt attttagttt   3840
   gggtaataga gcgagatttt attttaaaaa ataaataaat aaaattttgt tataataaag   3900
   tgattaaacg ttttttttaa aaataataga attgttagtt tgtcggtagt ggtttatgtt   3960
   tgtattttta gtattttggg aggttaaggt gggtggatta tttgaggtta ggagtttgag   4020
   attagtttgg ttaatatagt aaaattttgt gtttattaaa aatataaaaa agttaggtat   4080
   ggtggtatgt ttttgtaatt ttagttattt aggaggttga ggtaggaaaa ttatttgaat   4140
   ttaagaggcg gaggttgtag tgagtcgaaa tcgtgttatt gtattttagt ttgtaggatg     4200
   gagtaaaatt tttttttaaa aaaaaaaaaa aaaaaagtag atagtattat ttattttatt     4260
   aatacggaat atgttaattt tgatttgaaa ggaataaatg tattgaattt attgttttaa     4320
   ataaacggtt tttatgtttt aaaatgtttg atttttttta agtaaattga aaaaaaaatt     4380
   tttttgggtt aggtgtagtg gtttgcgttt gtaattttag tattttggga ggtcgaggta     4440
   ggcgaattat ttgaggttag gaatttgaga ttagtttgtt taatatggtg aaattttatt     4500
   tttattaaaa atataaaaat tagttgggtt tggtgtaatt ttagttattt agaggttgag     4560
   gtaggagaat tatttgaatt tgggaggtgg aggttgtagt gagttaagat ttgttattgt     4620
   attttagttt ggataataga gcgagatttc gtttaaataa ataagtaatt ttttgtgttt     4680
   tgggaaagtt agttattatt ttgggataag ttttaataga aaattaaatt ttatgggaat     4740
   aaaatttttt ttatattagg aatttgttgt tagtgatttt tgtattatat tgtaatgaat     4800
   gtattatatt tattaggaag atttaatttg aattatgttt gtaaaatgat aatataatgt     4860
   tatattaaaa ataattttgg gggttagatt tagtggttta tgtttataat tttaatattt     4920
   tgggaaattg aggcgagaga attgtttgag gttaggagtt tgaaattagt ttgggaaata     4980
   tagtgagatc gtgtttacga aaaattttta aatattagtt aagtgtggtg gtattcgttg     5040
   tagttttagg tatttgggag gttgaggtga gaagatagtt tgagtttggg aagttaaagt     5100
   tgtagtaatt tatgattata ttattgtgtt ttagtttgag agatagagta agattttgtt     5160
   tttttaaaat gtatgtatta tatgtgtttt ttatgggtag attttataat tattatttaa     5220
   atagttgttt tttagttgtt tttgtgaaat tttttgttgt aaaaattgaa gtaaatgtat     5280
   tttggttgtt gtttattgta aaatatttat ttatagtaaa ttttgaaata aaaatatatt     5340
   tttataagat atttgtaaat attttttaga taaaaagtaa gtatttggta ttttaaaata     5400
   tttaaaagtt tattagaatt ttgataaagt tattgagatt tttattatta aaatacgtaa     5460
   tttagtattt aaaagtcggt ttattaaatt tttattaaat gtttttcgta ttgatacggt     5520
   tttggtggtt aaaatataag tgtatatgtg tgtttgtttg tgtattttta aatattgttt     5580
   atttttattt tttatttgat taagttggag ggaaaaaatt tgaatttttt tattgtttaa     5640
   gaaggatttt aaggaattat attatattat tgtttttaga ttatttttta tgtggaaaga     5700
   aaagagaaat aatatataag aaatttaaaa gtgtgttgtt ttaatatttt ttttttgtta     5760
   aataaaggtt tgataggtta ggtgtggtgg tttttatttg taattttagt attttgggag     5820
   gtcgagttgg gcggattttg agtttaggag tttaagatta gtttgggtaa tatggtaaaa     5880
   ttttgttttt ataaaaaata taaaaaatta gttaggtatg gtggcgtatg tttgtagttt     5940
   tagttatttg gggggttgag gttggaggat tgtttgagtt tcggaatttg aggttttagt     6000
   gagttttgat tgtattatag tatttagttt gggtgataaa gtgagatttt gtaaaaaaaa     6060
   aaaggtttga tatattttat gttattttga taataaatat tggagataag ttgtttaaat     6120
   ttatatggtt tgagtgttag tttgtatagt ttttttttaa atttttatag aaatttttta     6180
   ggtaagaatt gaggtgattt tatttttaga aagttaaaaa gttgttaaat tattaaataa     6240
   gatataaaat ttgttattta tgtttattta attatataga aatttaatta gttggaattt     6300
   ttattgtgaa aattttgttt tttatggaaa ttttaataga ttttttaatt gtttttttgt     6360
   tttgttttgt tttgttttgt tttgttttgt tttgagagat ggagttttgt ttttgttgtt     6420
   taggttggag tgtagtggcg taattttagt ttatcgtaat ttttattttt attttttagg     6480
   tttaagtaat tttttgtttt tagtttttcg agtagttggg attataggtg tgcgtt         6536
<210> 3
   <211> 6681
   <212> DNA
   <213> Artificial sequence
<220>
   <223> chemically treated genomic DNA (Homo sapiens)
<400> 3
   gagaagttta gggaatggga tatttttaag aggaagtgtg ttttagtgtt agatgtaaat      60
   atttttattg gttttagtaa aaaagttatt ggtgttatta atatcgaaag tagttttaag     120
   tgatgttgat ttaatgagta aatgggagag ggaaaatgga gatattaatt atatatatat     180
   gtttagtttg tttggttgtg aagagaaggg gtgatatagg gtagttgtta gagtggaaag     240
   tgtggttgaa agaggatttt tttttttttt ttttgagata gagttttatt ttgttattta     300
   ggttggagtg tagtggtgta gtttcggttt attgtaattt ttatttttcg ggtttaagcg     360
   atttttttgt tttagttttt tgagtagttg ggattatagg tgtatgttat tatattcggt     420
   taattttttg tatttttttt agtagagacg gggttttatc gtgttagtta ggatggtttt     480
   agttttttga tttcgtgatt tattcgtttt agttttttga agtgttggga ttataggtat     540
   gagttattat attcggtcga aagagaattt taaaaatttt ttttatttga aagttgagat     600
   agatttaagt atggttatgt gaaaggaaaa aggttagtcg agagtgatag gaatgagagt     660
   tgaagtaatg aggggatggg attaagaata agatttttga ggtagtagga tagtgtagga     720
   ttttataagg gggtaagttt ttaattgaga tagtaagtag gttgtgagtt aaaacggttt     780
   ggatatgaga acgtgagaag gtttttgttt aatgatgttt tttttttttg agaaatagaa     840
   ggtaaagtaa ttgattgaaa atgagatgga gtgtagtgga gaaatttgag gaaggtgttg     900
   aaggtttaga agaggtgtat agggtatggg ttagggagtt gagtttattg ttaagggatg     960
   gtagaggagt tttaagtgat gttggtttta gtttgtaggg ttgtatttag gtgttggaaa    1020
   tgattaggat agaggttgaa gtgatttagg gttgatattt tggtatattt gtgtatattt    1080
   attgtatagt tgttgtttaa atttttataa atatatgttt tgttggagtg tagtggtgtg    1140
   attttggttt attgtaattt ttatttttcg ggtttaagtg atttttttgt tttagttttt    1200
   tgagtagttg gaaatatagg tgcgtgttat tacgttcgat ggtttttttt tttggatttt    1260
   tagtagagat agggttttat tatgttggtt agtttggttt cgaatttttg attttaagtt    1320
   atttgttttt tttggttttt taaagtgttg ggattatagg cgtgagttat tacgtttgat    1380
   ttaaagtttt tttaggataa gatttgtgta aggggtttgt aaatttaaat ggaggtgata    1440
   taaatgagaa aaatttttag tgtttttttt tttttttttt tttttttttt tgagataggg    1500
   ttttgttttg ttatttagtt tggagtgtag ttatgtagtt ttatattgta agttttgttt    1560
   tatgggttta agtgaatttt ttgttttagt tttttaagta gttgggatta taggtaggtg    1620
   ttattatgtt tggttaattt ttgagttttt ttgtagagat ggtgttttgt taagttattt    1680
   agtttgaggt tggttttaaa tatttgggtt taagtaattt atttatttta gttttttaaa    1740
   gtgttgggat tataggagtg agttatggta tgaggttttg tggggtgttt tttttaaatg    1800
   aaagtatatt ttgtttacgt atttgatatg aaggaatatt tttttttttt ataaagataa    1860
   aaattatttt atttttttta aaatatatgt tttttttttt tattttttat ttttgttatt    1920
   tttgatggat atatgtgtta tattgatttt atttttttat aattttgttg taagaaaaat    1980
   aatagtgtta gtttaatgat aaatagtaat agtttgttat tgttagattg ttattgttag    2040
   tggagattat tagaatagtt agttttagtg ttagggaatt aaagagaata tgtttttttt    2100
   ttaaagggta tagttgttgt ttagttttag ttgattgttg ttttgtagga ttataggttt    2160
   agtgttgtta gattttttga tgttttaaga gaagtttgga atttagaatg tgatgggaag    2220
   ttttttatat ttaaatatgt tggtaattaa tggtaagatt taaaaatatt gtggtttaag    2280
   aaaaaaatgg atttggaaat tggattaaat ttaaatgagg tatgtagatt aatttatagt    2340
   atggtataat gtgaattttt tggttttttt aattgtattg taattaggta agatgttagt    2400
   tttggggaag ttaagtgtag agtatgtaga aattattatt tttgtaagtt ttttttaagt    2460
   ataaataaat tttaaaataa aaataaaaat ttagtaaaga attataatgt aattttatgt    2520
   aagttaaata taatatgttt tttagtttga aatttttggg ttttatttta ttttatttta    2580
   tttttgagat agagttttgt tgtgttattt aggttggagt gtagtggtat tatttcggtt    2640
   tattgtaatt tttatttttt aggtttatat gatttttttg ttttagtttt cggagtagtt    2700
   gggattatag gcgcgtatta ttatatttag ttaatttttg tatttttagt agagatgggg    2760
   gttttattat tttggttagg ttggttttga atttttgatt ttaagtgatt tatttgtttt    2820
   ggttttttaa aatgttggga ttataggcgt gagttattgt attaggtaga ggtttttgtt    2880
   ttttattttt ttttggtttt tatagtgttt agtaaagtat ttgatatatg gtaaacgatt    2940
   agtaattatt agtattttat tttggagaaa atgatttttt aaaaagttat tgtgttttat    3000
   ttatgagtcg tttgagtttt aaaattgttt ttttgtttgt ttttgaatag gtttataaag    3060
   gaggaaaacg atttttttta gatttttttt ttagtttttt ttataaatta aaatatttta    3120
   aaatggagat ttaaaatttt taaagggatt tagtttaatt tcgggaggta gttttgtgta    3180
   tgggtaaata aattaagtat taattggtgt tttattattt aaagaatgtt aattttataa    3240
   atatgatcga gttatataag gtatattata atgagtttga ttttgaattt gatttgtgga    3300
   aataaaggaa aagtgatttt agttggggta tattgttaaa gtattttttt tagagttggt    3360
   taggtagttt tttattggta tattttttta ttatgtagaa tagaaatagt atttgtgttt    3420
   gggaaagatt ttaaaatgag tgatagttat ttggaataaa gagttaataa ttaatttatt    3480
   gtaaattaaa gaaatatgta gatgaaagtt ttgatatatt aaaatatttt tatagtgata    3540
   aagaaaaatt aagaataaag ttttttgata tgtgtaataa atttagagga agtaaaaaga    3600
   taaatgtgat gattggttaa gaaattattt agttatttat aaggttattg atattttaaa    3660
   cgtttaaaag tttgtttaaa tgggttgtta tcgttgagaa tgatgaggat gagaatgatg    3720
   gttgaaggtt atattttagg aaatgaagaa atttagaaaa ttaatataaa gatagtgatg    3780
   aatataaaga agatttttat aataatgtgt aaaatttttg gttagggaaa ggaatattga    3840
   agttagatat aattatttat ttttgaggga aaataattgt tggtaatgag atgtgatgtt    3900
   ttttttgttt tttggaaata aagtattgaa gtttgtagtt gaaaagttta acgtttgtga    3960
   gatttaggaa attatgtttg taaatttttg gtaaaaaaaa aaaaaaaaaa aaaaaaaagt    4020
   tatagtgatt tgtttattgg ttattgttag tattatcgat ttagaatttt tttattaatg    4080
   gttagtaaat tataattgag aaattttgaa ttttgataag gtttttgttg ttgaaatggt    4140
   aaatttatta ttttttttgt tatgataaat tttggtttaa ggtatgttat ttatgaaata    4200
   atttttgatt aaaattaaat tgatgtaatt tgattattta ttttagttta tagatggtat    4260
   ttggtaattt ttgattgttt taaaaaataa atttattatt agagtagatt tgatgttggt    4320
   tttagaaata tttagaaaaa taaaagttta aaaatgtttt taggaggtga taagttgaat    4380
   aattttataa tgttagtttt ttgaggggga taaaaaattt aaaatttttg aaaggtttta    4440
   ttttatagtt atatttaaat tattttaaga aaatttttaa taaagggaat gaaatatata    4500
   ttatgatttt gtttttttaa aagtaatttg aatatagtaa tgaagtttag ttttgttatt    4560
   ggtagtttgg gtagagtttt tttttgtagt atttgttgtt tattataatt atagaggata    4620
   tttttatgtt ttagttaagt atattattag aataaaaaaa tttaatattg agttgtttta    4680
   atagtatgaa attgagttta aaagattaaa tgaataaata tattaatttt tgattattta    4740
   ttttaaatag aatatttaat tgtgtaagat ttaatagtat tattatattt aagtaattat    4800
   atttttgatg atttatggga aataattatt atttaattaa tattgaaatt aggttttaag    4860
   atgtgttagt tagttttgtt attagtaaat ttttttattt ggagagaaat tttagattgt    4920
   tttgtttttt ttattagaag gattgtagaa agaaaaaaat gattaattgg agaaaaattg    4980
   gggatatatt atattttatt gaatttaaaa tgtttttagt tgtaaatttt attattattt    5040
   tacgtatttt taagaaataa aagtgttttt aattaaaata tgatgttatt aattatgaaa    5100
   tattttttga taatagaagt tttaaaatag ttattttaga attagtgaaa tatggtaatg    5160
   tattattttt tttttttgag ttaggttttg tgtttttttt ttttggttat taaattttat    5220
   aatttttaaa aagtaaaata aatatatttt gaatattttt gttgtgaaat atttgatagt     5280
   agagtttttt attatgaaaa gaagttttat gagttatata ttatattttt gggttgattg     5340
   aatgttattg aaatatttta gtagtttgga gaagttgatt tatttgtgga gatgtttgtt     5400
   attaaatggt attttgatgg tttaatatat attatttttt tgtgaagggt tttaattttt     5460
   aatatagttt attttgtagt attatgttta tattgtatgt ataaagatta tataaaggtg     5520
   taattgtgta tttttttttt aaaatgtatt agtataggat ttagaatttt tatgttgaaa     5580
   ttttaaatgt atagaaataa aaataataaa aaatttttta ttttggtttt ttagtttagt      5640
   attaaaattg ataaaagtaa agttatgtat aaaattattt ttttagagaa aggttagttt     5700
   tttttttttt ttatttattt tattatgaat atagtagaaa atagtatatt tttattaaat     5760
   ttgatgaaaa gcgttaatac gtttgaattg aaatacgatt tgttatgtga attgtatcga     5820
   atgtttacgt attttatttt ttttgttggg gtttttgttt tagaaaggag ttttgttcgt     5880
   gttggttttt attatattgg tgtgaatgat aaggttaaat gtttttgttg tggtttgatg     5940
   ttggataatt ggaaaagagg agatagtttt attgaaaagt ataaaaagtt gtattttagt     6000
   tgtagattcg tttagagttt aaatttcgtt aataatttgg aagttatttt ttagtttatt     6060
   tttttttttt tagtaataaa ttttatatat ttattatttt cgggtataga aaatagtgga     6120
   tattttcgtg gtttttattt aaatttttta ttaaattttg taaattttag agtaaattaa      6180
   gatttttttg ttttgatgag aagtttttat tattgtgtaa tgaataacga aaatgttaga      6240
   ttatttattt tttagatatg gttattgatt tttttgtcgt taatagattt ggtaaaagta     6300
   ggtttttatt atataggatt tggagataga gtggtttgtt ttgtttgtgg tggaaaattg      6360
   agtaattggg aatcgaagga taatgttatg ttagaatatt tgagatattt ttttaaatgt     6420
   ttatttatag aaaattagtt ttaagatatt ttaagatata tagtttttaa tttgagtatg     6480
   tagatatatg tagttcgttt taaaatattt tttaattggt tttttagtgt tttagttaat     6540
   tttgagtagt ttgtaagtgc gggtttttat tatgtgggta agaaattgaa tttgttaatt     6600
   aaaaaaaata tattttattt tatatatttt agtgggtaaa ttatgtgtat ttataagttt     6660
   tggtttaaaa ttaattttag g                                               6681
<210> 4
   <211> 6681
   <212> DNA
   <213> Artificial Sequence
<220>
   <223> chemically treated genomic DNA (Homo sapiens)
<400> 4
   tttaaaatta attttagatt aaaatttatg aatatatata atttgtttat taaaatgtat      60
   aaaatgaaat atattttttt taattagtag atttagtttt ttatttatat aataaaaatt     120
   cgtatttgta agttgtttag gattaattag aatattagag ggttagttaa agaatgtttt     180
   aaagcgggtt gtatgtgttt gtatgtttag attagaaatt gtgtattttg aagtgttttg     240
   aagttgattt tttataaatg ggtatttggg aaaatgtttt aggtgttttg atatagtatt     300
   atttttcggt ttttaattgt ttaatttttt attataggta aagtaagtta ttttgttttt     360
   aggttttatg tagtaaaagt ttgtttttgt tagatttgtt ggcgatagaa aagttaatgg     420
   ttatgtttga aaagtaagta atttggtatt ttcgttattt attgtatagt ggtaggaatt     480
   ttttattaag gtagaaaaat tttgatttgt tttggagttt ataggatttg atggagagtt     540
   tgaataagag ttacggaaat atttattgtt ttttgtattc ggaagtaatg agtgtgtgga     600
   atttgttatt gaagaaggaa aagtaggttg agaggtagtt tttaagttgt taacggaatt     660
   tagattttga acgaatttgt agttaggata taatttttta tgttttttag taggattgtt       720
   tttttttttt tagttattta gtattaggtt ataatagaag tatttgattt tgttatttat      780
   attagtgtaa tagaaattag tacgagtaag attttttttt gagataggaa ttttagtagg      840
   aaaagtggaa tacgtagata ttcggtatag tttatatgat aagtcgtatt ttagtttaaa     900
   cgtgttggcg ttttttatta aatttgataa gaatatgttg ttttttatta tgtttataat     960
   gaaatgaatg gggaagaaaa gggattagtt tttttttagg gaggtagttt tgtgtatggt    1020
   tttgttttta ttagttttaa tattaggttg aaaagttaaa atgaaaaatt ttttattatt    1080
   tttattttta tgtatttaga gttttaatat ggagatttta aattttatat tgatatattt    1140
   taaggaagaa atatataatt gtatttttgt ataattttta tatatataat gtaaatatga    1200
   tgttatagag taagttgtgt tgaaaattaa aattttttat agaagagtga tgtgtattaa      1260
   gttattagga tgttatttaa tggtaggtat ttttataggt aggttaattt ttttaggtta    1320
   ttagaatgtt ttagtggtat ttaattaatt taaagatgta atgtgtgatt tatgaagttt    1380
   ttttttatgg tggaaagttt tgttgttaag tgttttatag taaaaatatt tagaatatgt     1440
   ttattttgtt ttttggaaat tgtgaaattt agtggttagg aaaaaaaagt ataagattta    1500
   atttaaagga ggaaaataat atattattat attttattga ttttaagatg gttattttaa    1560
   aatttttgtt attaagaagt attttataat taatgatatt atattttaat tagaagtatt    1620
   tttatttttt agaggtacgt aaaataatgg taagatttat aattgaagat attttgaatt    1680
   tagtgaaata tgatatattt ttaatttttt tttaattagt tatttttttt tttttataat     1740
   ttttttaata aggagaataa aataatttaa aatttttttt taaataaaga gatttattag     1800
   taataggatt ggttaatata ttttaaaatt tggttttaat attaattaaa taatagttat    1860
   tttttataga ttattaggaa tatgattgtt taagtataat gatattatta gattttatat    1920
   aattaaatat tttatttaaa ataaataatt agagattaat gtgtttgttt atttggtttt    1980
   ttggatttag ttttatgttg ttgaagtaat ttaatgttaa gtttttttat tttaatagta    2040
   tatttggtta gaatatggaa atgttttttg taattatggt agataatagg tgttgtaaaa    2100
   agagattttg tttaaattat taataataaa attgaatttt attgttatat ttaggttatt    2160
   tttggaaaaa tagaattatg atatatattt tatttttttt gttaaaaatt tttttaagat    2220
   aatttagata tggttgtaaa ataagatttt ttaaagattt taaatttttt gtttttttta    2280
   aagaattaat attgtagagt tatttaattt attatttttt gaaaatattt ttgaattttt    2340
   gtttttttaa atgtttttga agttaatatt aaatttattt tgatagtgga tttatttttt    2400
   aaaatagtta aaagttatta gatgttattt gtaggttaag atggataatt aaattgtatt    2460
   aatttagttt tggttagaaa ttattttatg gatagtatat tttgaattag aatttattat    2520
   gataaaaaaa ataataaatt tattatttta atagtagaga ttttgttaaa atttagaatt    2580
   ttttaattat gatttattag ttattagtaa agaggttttg agtcgataat attagtaatg    2640
   attaataagt aagttattgt ggtttttttt tttttttttt ttttttttta ttaggggttt    2700
   gtaagtatgg ttttttggat tttatagacg ttgggttttt taattgtaaa ttttaatgtt    2760
   ttgtttttag ggggtaggag aaatattata ttttattatt aataattatt tttttttaaa    2820
   ggtaagtaat tgtatttaat tttaatattt ttttttttgg ttaaaaattt tatatattgt    2880
   tataaaaatt ttttttgtat ttattattgt ttttatatta attttttaag tttttttatt    2940
   ttttaaaatg taatttttaa ttattatttt tatttttatt atttttagcg gtaatagttt    3000
   atttaaataa atttttggac gtttaaaata ttagtggttt tgtaaataat tggataattt    3060
   tttgattaat tattatattt atttttttat tttttttaaa tttgttgtat atattaaaaa    3120
   gttttgtttt tgattttttt ttgttattgt agaagtattt taatgtgtta aaatttttat    3180
   ttgtatgttt ttttaatttg tagtggattg attattagtt ttttgtttta aataattgtt    3240
   atttatttta aaattttttt taaatatagg tattattttt attttatata atgggagaat    3300
   gtgttagtag gagattgttt ggttaatttt gaaaaaaatg ttttaataat atgttttagt    3360
   tagaattatt ttttttttat ttttataaat taaatttaaa attaaattta ttatggtata    3420
   ttttatataa ttcgattatg tttataaaat tagtattttt tggatagtaa aatattagtt    3480
   aatatttaat ttgtttattt atgtataaaa ttatttttcg agattagatt aagttttttt    3540
   aaggatttta ggtttttatt ttgagatgtt ttgatttata gaagaaattg aaaaaaaaat    3600
   ttagaagaag tcgttttttt tttttgtaaa tttgtttaaa aataaataaa aagatagttt    3660
   taaaatttaa acgatttatg gatggaatat aatgattttt taaaaaatta ttttttttaa    3720
   aatagagtat tagtaattat tgatcgttta ttatgtatta ggtgttttat taggtattgt    3780
   agaggttaaa aagagataaa aaatagaggt ttttgtttgg tgtagtggtt tacgtttgta    3840
   attttagtat tttgggaggt taagataagt ggattatttg aggttaggag tttaaaatta    3900
   gtttggttaa aatggtgaaa tttttatttt tattaaaaat ataaaaatta gttgggtgtg    3960
   gtggtacgcg tttgtaattt tagttatttc ggaggttgag gtaggagaat tatgtgagtt    4020
   tgggaggtgg aggttgtagt gggtcgaaat agtgttatta tattttagtt tgggtgatat    4080
   agtaagattt tgttttaaaa ataaaataaa ataaaataaa atttagaggt tttaaattgg    4140
   aagatatatt atgtttggtt tatatagaat tgtattatag tttttattta agtttttatt    4200
   tttattttga aatttattta tatttagaga aagtttataa aaataatagt ttttgtatat    4260
   tttgtattta gtttttttaa agttaatatt ttatttaatt atagtgtaat taaagaaatt    4320
   agaaaattta tattgtatta tgttgtagat taatttgtat gttttatttg aatttaattt    4380
   agtttttaaa tttatttttt ttttggatta tagtattttt aaattttatt attaattgtt    4440
   aatatgttta aatgtaagag attttttatt atattttaga ttttaagttt tttttgaaat    4500
   attaaaagat ttagtaatat tgggtttata gttttgtagg gtagtaatta gttaaagttg    4560
   agtagtagtt gtgtttttta gagagggaat atgttttttt tgattttttg atattgggat    4620
   tgattgtttt ggtagttttt attaatagta atagtttagt aataatagat tgttgttatt    4680
   tgttattgaa ttggtattat tgtttttttt atagtagaat tatgagaaag tgaaattaat    4740
   gtaatatatg tgtttattaa aagtaataaa aatgaaaagt agagaaaaag gatatatgtt    4800
   ttgagaaaaa taggataatt tttgtttttg tggaaaggaa ggatattttt ttatattaaa    4860
   tacgtaaata gagtatgttt ttatttaaaa gagatatttt ataaggtttt atgttatggt    4920
   ttatttttgt aattttagta ttttgggagg ttgaggtaga tggattgttt gagtttaggt    4980
   gtttgagatt agttttaaat tgggtgattt ggtaaaatat tatttttata aagaaattta    5040
   aaaattagtt agatatggtg gtatttgttt gtggttttag ttatttgaga ggttgaggta    5100
   ggaggtttat ttgagtttat gaggtagagt ttgtagtgtg agattgtata attgtatttt    5160
   aaattgggtg atagagtaag attttgtttt aaaaaaagag agagagagag aaagagagat    5220
   attgagagtt ttttttattt atattatttt tatttgagtt tgtaaatttt ttatataaat    5280
   tttattttaa agaagtttta ggttaggcgt ggtggtttac gtttgtaatt ttagtatttt    5340
   gggaggttaa ggaaagtaga taatttgaga ttaggagttc gagattaggt tggttaatat    5400
   ggtgaaattt tgtttttatt aaaaatttaa aaaaaaaaat tatcgggcgt ggtggtacgt    5460
   atttgtattt ttagttattt aggagattga agtaggagaa ttatttgaat tcgggaggtg    5520
   aaggttgtag taagttaaga ttatattatt gtattttagt aaggtatata tttgtggaaa    5580
   tttaggtaat aattatataa taaatatata taaatatgtt aagatgttaa ttttagatta    5640
   ttttaatttt tgttttaatt atttttaata tttagatgta attttgtaga ttgggattag    5700
   tattatttaa gatttttttg ttatttttta gtaataggtt tagtttttta gtttatgttt    5760
   tatgtatttt ttttaaattt ttaatatttt ttttaaattt ttttattgta ttttatttta    5820
   tttttagtta attattttgt tttttgtttt ttaaagaaaa gagatattat tgagtaggaa    5880
   tttttttacg tttttatatt taagtcgttt tggtttataa tttatttgtt gttttagtta    5940
   aagatttgtt tttttatggg attttatatt gttttattgt tttagaaatt ttgtttttaa    6000
   ttttattttt ttattgtttt aatttttatt tttgttattt tcgattggtt tttttttttt    6060
   tatataatta tgtttaaatt tattttaatt tttaaatgaa agaaattttt aaaatttttt    6120
   ttcggtcggg tgtggtggtt tatgtttgta attttagtat tttaggaggt tgaggcgagt     6180
   ggattacgag gttaggagat tgagattatt ttggttaata cggtgaaatt tcgtttttat     6240
   taaaaaaaat ataaaaaatt agtcgggtgt ggtggtatgt atttgtagtt ttagttgttt     6300
   aggaggttga ggtaggagaa tcgtttgaat tcgggaggtg gaggttgtag taagtcgaga     6360
   ttgtattatt gtattttagt ttgggtgata gagtgagatt ttgttttaaa aaaaaaaaaa     6420
   aaaaattttt ttttaattat atttttattt ttagtaatta ttttgtatta tttttttttt     6480
   ttatagttaa gtaaattgga tatgtgtgta tggttaatgt ttttattttt ttttttttat     6540
   ttatttatta agttagtatt atttaaaatt gttttcggtg ttaataatat taatggtttt     6600
   tttgttaaaa ttaatggaaa tgtttgtatt tgatattggg atatattttt ttttagaaat     6660
   gttttatttt ttgggttttt t                                               6681
<210> 5
   <211> 7049
   <212> DNA
   <213> Artificial Sequence
<220>
   <223> chemically treated genomic DNA (Homo sapiens)
<400> 5
   ggatttaaag atattttaga aatttttgag gtagtttttt ttattatagg ttagaggttt      60
   aggaggaaag aattatttta ggggttaggt atagggtatt gtttttttgt ttagttttag    120
   gattttattt tttgtatttt ggttatttta gttttattta tggtttatag ggttataggt    180
   atagtttaag ttgttatttt ggaaagtgta aattattgta agttttggta gtttttatat    240
   ggtgttaagg ttgtaggtgt ataaaatgta agagtaaagg aggtttggta atttttattt    300
   agattttaga ggatatatgg aaaagtttag atgtttaggt agaagtttgt agtagggata    360
   gagtttttat agagaaatgt tattagggta gtattgagag gaaatgtggg gtttgagatt    420
   ttatagagtt tttagtagga tattgtttag tggagttgta ggaagagggt tacgaatttt    480
   tagatttgag aatggtaaag ttatagtagt ttgtaagttt agtatgaaaa agttatagga    540
   atttaatttt agtttattag agtagttata aattgtattt tgtaaagtta taagggtaaa    600
   gttgtttaag gttttgggaa tttatatttt gtattagtat gtttaggata taggatatgg    660
   aggtaaggga aattattttg gaatttaaag tttaatgttt attttgttgg gttttagatt    720
   tgtatggggt taattgtttt ttttttttgg ttgatttttt ttttttggaa tagagtgttt    780
   atttaatgtt tttattatta ttgtattttg ggtgtaaata atttgttttg attttatagg    840
   tttgtaggta aaaagaattt aatttgagat ttagatgtga ttttggattt ttgattgaat    900
   tgatgttgga atgaggtaag gttttggggg attgttagaa aggatgaatg tattttataa    960
   tgtgagaagg atatgagatt tgaggggtta gaaggggaat gatatggttt gggtgtttga   1020
   ttttttttaa ttttatgttg aaatgtaatt tttaattttg gaggtggggt taagtgggag   1080
   atattggatt atgggggtag atttttgatg aatggtatta tttttatgat gagtgggttt   1140
   ttattttgtt agtttatttg agatttggtt atttttaaaa gggtatggta tttttttttt   1200
   tttttttttg tttttgtttt ttttatgtga tatgttggat ttcgattgtt ttttaatatg   1260
   attgtaattt ttttgaggtt ttattagaag tagatgttag tattatgttt ttttaaagtt   1320
   tgttaaatta tgagataatt aaattttttt ttattgtaaa ttatttagtt tcggggattt   1380
   ttttatagta atgtaagaat agattaatgt atatgttaag tgaattttta tttagaatag   1440
   attaaaaaaa ttagatatta aaattatttg attaagattt gttttttaag gtggttgaat   1500
   aagaagagtt tcggtttgta gtttttagta tgatcgatgt agaagatggg tgatttttgt   1560
   atttttaatt gaggtatttg gtttatttaa ttgggattgg ttggatagtg ggtgtagttt   1620
   acggagggcg agttaaagta gggtggggta ttgttttatt cgggaagtac gagagattgg   1680
   gggatttttt ttttttagtt aagggaagtt aggatagaat gtatttggaa aaataggata   1740
   ttttcgttta aatattgtat tttttttaag gttttagtaa ttggtagata aggagatttt   1800
   tttttgtgtt tggtttagtg ggttttatgt ttatggagtt ttgtttattg ttagtatagt   1860
   agattgagat taaatagtga ggtggtagtt tggttggggg aggggtattt gttattgttg   1920
   aggtttgagt aggtaaataa attagttaag aagttcgaat tgggtggagt ttattgtaga   1980
   ttagtaaggt ttattgtttt tatagatttt atatttgtgg gtagggtata gttgaataaa   2040
   agatagtaga tagtttttgt agatttaaat gtttttgttt gatagttttg aagagagtgg   2100
   tgattttttt agtatggtgt ttgagttttg agaaagtata gattattttt ttaagtgggt   2160
   ttttgtgtag tttaattggg aggtattttt tagtagggga taatagatat tttatatagg   2220
   tgggtgtttt tttgggatga agtttttaga ggaaggatta gggagtaata tttgttattt   2280
   tttaatattt gttgttttgt agttttcgtt ggtgatattt aggtaaatag ggtgtggagt   2340
   ggatttttag taaattttaa tagatttgta gttgagggat ttgattatta gaaggaaaat   2400
   taatatatag aaaggaatag tattaatatt aataaaaagg atatttatat taaaatttta   2460
   tttgtagatt attaatatta aagattaaag gtaaagggat ggaggaagat ttattatgta   2520
   aatggaaaat aaaaaaagta ggggttgtaa ttttagtttt tgaaataata gattttaaag   2580
   taataaagat taaaagagat aaagaaggtt attatataat ggtaaaggga tgaatttaat   2640
   aagaagagtt aattatttta aatatatatg tatttaatat aggagtattt agatttataa   2700
   agtaaatttt tagagattta taaagagatt tagatttttt atataataat aatgggagat   2760
   tttaatattt tattgttaat attagaaagt tttatgagat agaaagttaa taaggatatt   2820
   taggaagtga atttagtttt gtattaggta gatttaatag atattaatag gattttttat    2880
   tttagattaa tagaatatat atttttttta gtattatatt atatttattg aaaattgatt   2940
   gcgtagttgg aagtaaagta ttttttagta aatgtaaaag aatagaaatt ataaattgtt   3000
   ttttagatta tagtgtaatt aaattagaat ttaggattaa gaaatttatt taaaattatt   3060
   taaattatat ggaaattgaa taatttgttt ttgaatgatt attgggtata taataaaatg   3120
   aaggtagaaa taaagatgtt ttttgaaatt aaagagaata aagatataat atattagaat   3180
   ttttgggata tatttaaagt agtgtgtaga gggaaattta tagtattaaa tgtttataag   3240
   agaaagtagg aaagatttaa aattgatatt ttaatattat agttaaaaga attagagaag   3300
   gaagagaaaa tatatttaaa agttagtaga aggtaagaaa taattaagat tagagtagaa   3360
   ttgaaggaga tagagatata aaaaaatttt taaaaaaatt aatgaattta ggagttggtt   3420
   ttttgaaaat attaataaaa ttgatagatt attagtaaga ttaataaaga agaaaagaga   3480
   gaagaattaa atagatgtaa taaaaaatga taaaggggat attattatcg attttataga   3540
   aatataaatt attattagag aatattataa atatttttat acgaataaat tagaaaattt   3600
   agaagaaatg gataatatat ttttttaaga ttaaattagg aagaattgga atttttgaat   3660
   agattaatat taggttttga aattgaggta ataattaata gtttattaat taaaaaaatt   3720
   taggattaga cggatttata gtcgaatttt attagaggta taaagaggag ttggtagtat   3780
   tttttttgaa attattttaa ttaatagaaa aagaggtaat tttttttaat ttattttatg   3840
   aggttagtat tattttaata ttaaagttag gtagagatat aataaaaaaa gagaattgta   3900
   gattaatatt tttgatgatt attgatttaa aaatttttaa taaaatattg gtaaattgaa   3960
   tttagtagta tattaaaaag tttatttatt acgattaaga tagttttatt tttgggatgt   4020
   aagattggtt taatatatgt aaattaataa atataattta ttatataaat agaattaatg   4080
   ataaaaatta cgattatttg aatagatgta gaaaaggttt tcgataaaat ttaatagttt   4140
   tttatgttaa atatttttaa taaattaggt attgatggaa cgtattttaa aataataaga   4200
   gttatttatg ataaatttat agttaatatt atattgaacg ggtaaaaatt ggtagtattt   4260
   tttttgaaaa ttggtataag atagggatgt ttttttttat cgtttttatt taatatagtg   4320
   ttgaaagttt tggttagggt aattaggtaa gagaaagaaa taaagggtat ttaattagga   4380
   aaagaggaag ttaaattgtt tttgtttgta gatgatatga ttgtatattt tgaaaatttt   4440
   atcgttttag ttttaaattt ttttaagttg ataagtaatt ttagtaaagt tttaggatat   4500
   aaaattaatg tgtaaaaatt ataagtattt ttatatatta gtaatagata gagagttaaa   4560
   ttatgagtga agttttattt ataattgttt taaagtgaat aaaatattta ggaatttaat   4620
   ttataaggta tgtgaaagat ttttttaagg agaattataa attattgttt aatgaaataa   4680
   aagaggatat aaataaatgg aagaatattt tatgtttatg gatagaaaga attaatatta   4740
   tgaaaatggt tatgtttttt aaggtaattt atagatttaa tgttattttt attaagttat   4800
   taatgatttt ttttatagaa ttggaaaaaa ttatttttaa gtttatatgg aattaaaaaa   4860
   gagttcgtat tgttaagata attttaagtt aaagaataaa gaattttatg gagtgtaatt   4920
   ttgtgtatga attatatttt taaaatattg aagagttttt agaaagaagg ttagtagagt   4980
   tgattattga tattttatgt taagtagtat ttttttggta gtataatatt ttgttaggcg   5040
   tttttgataa tattagaaag gataagtttt attttgtgat aaattgatta atgtttataa   5100
   tatgattgat aattatagtt gaatagtttt taaatgatga ataggttatt tagtttttaa   5160
   atgtagtgta aaaagtgtgt tgtggaaatt ttatggttaa ttaagtttat ggagaaaata   5220
   tttttagttg attaagaata atagtggtat ataaagttag gaagaaagtt aatatgatgt   5280
   tgtaggaaat ggaaataaat ataaatgata tttaataaag atagagttta tagtttttga   5340
   attttaagtt aaatttattt gatattaagt attatagtag gtataggttt aataaagttt   5400
   gtgggtattg atttttttta aaagttgtta gttgaagtaa tttagtttat ttaagtaaat   5460
   attatgatga taagttgtgt gaatttagtt tttaaatagt gtgattatat gaaggtttta   5520
   attatttttg tttattggaa taaaatgaga ttttttgggt tgttatgtta aagtgtttat   5580
   agggaaagaa gtttgtatat aattttttat tttgtggtat aattagtaat tggtttgtta   5640
   tttaggtttt atagtttgta attaaatata aataaaaggt ataatttagg tattttatag   5700
   ttgtttagaa ttttgttaat ataaattttt gtgaaaaatt aaggagtttt aatattttta   5760
   gaagtgtatt tattttttag ggttttaagt tagtattatt taagattatg aataaatagt   5820
   atttaggtta tttgaaagag ttattataat tttaaagagt tgtgttttaa gtagtatttt   5880
   ggtaatttag agagatattt attttatttg aatataaatt gagataaatt tagtaaagaa   5940
   agtgtagtaa attttatata agagtttatt attgattttt ttttgtggta aaaattttag   6000
   tttatgtgaa gaaattttat gtgaatgttt tagttattaa atagtattgt tatttattta   6060
   tgtataaaat tgttttttaa agattttttt taggtttttc gtattaaaat attaagagta   6120
   taatggaaga tagtacgatt ttgttagatt ggataaatag taataaataa aaaatgaagt   6180
   atgatttttt ttgtgaattt tatagaatgt ttatatattt aatttttttc gtcggggtgt   6240
   ttgttttaga aaggagtttt gttcgtgttg gtttttatta tattggtgtg aatgataagg   6300
   ttaaatgttt ttgttgtggt ttgatgttgg ataattggaa attaggagat agttttattt   6360
   aaaagtataa atagttatat tttagttgta gttttattta gaatttggtt ttagttagtt   6420
   tgggatttat ttttaagaat acgtttttaa tgagaaatag ttttgtatat ttattatttt   6480
   ttattttgga atatagtagt ttgtttagtg gtttttattt tagttttttt ttaaattttt   6540
   ttaattttag agtagttgaa gatatttttt tatcgaggat taatttttat agttatgtaa   6600
   tgagtattga agaagttaga ttttttattt attatatgtg gttattaatt tttttgttat   6660
   tattagaatt ggtaagagtt ggtttttatt atataggatt tggagatagg gtagtttgtt   6720
   ttgtttgtgg tgggaagttt agtaattggg aattaaagga tgatgttatg ttagaatatc   6780
   ggaggtattt ttttaattgt ttatttttgg aaaatttttt agaaattttg aggtttagta   6840
   ttttaaattt gagtatgtag atatatgtag ttcgaatgag aatatttatg tattggttat   6900
   ttagtgtttt agtttagttt gagtagtttg taagtgttgg tttttattat gtgggtaaga   6960
   agtaaataat tatatatttt attattttat tttaatttat atattagaat atacgtgttt   7020
   ttaatattta gttttttttt ttttgaagg                                        7049
<210> 6
   <211> 7049
   <212> DNA
   <213> Artificial sequence
<220>
   <223> chemically treated genomic DNA (Homo sapiens)
<400> 6
<210> 7
   <211> 7990
   <212> DNA
   <213> Artificial Sequence
<220>
   <223> chemically treated genomic DNA (Homo sapiens)
<400> 7
   gggaggttga gataggagaa tggtttggat ttgggaggcg gaggttgtag tgagttgaga      60
   ttacgttatt ttattttagt ttgggttata agagtaaaat tttgttttaa aaaaaaaaaa     120
   aaaaaaaggg agaaatagaa atttttgtgt cgtatataga tattagggtt ttgtatgtgt     180
   ttttgttttt gtatttttta tttttatatt tttatatttt cgttagatta gttgttaatt       240
   tttttttttt ggtatttttt ggtggttttt tttacgtagt tgattggatt aaatgtatgt     300
   atatatatgt atattgtaaa attttatata ttattattgt agaatttttt aaatttttta     360
   tttaaatatg tattattgtt tgttggtttt ttttgtttta ttttttgttt ttatagtgtt     420
   atttttttaa ttattgttat ttttaagatt tttgattttt tagttttttt atattaaaag    480
   ttttttttga tatttttttt ttttcgtgtt tttttgtttt tttatttatt tagtataaag     540
   tttgaagatg tttttataga gaagttttaa attattttag tatattttat tgtatttata     600
   tttttttatt ttaaaaattt ttttgttatt tttgtttaat atttatattt tatttttttt    660
   tatattttta atttatttgt tttatatata tttgtttttt gtggtattat tttttttatt     720
   tttattttat ttagatttta tattttttta ttttatttta ttttttttgt ttggtgtttt   780
   tttttaaatt taaggttttt gttttttatt agttattttt ttatttttta tttttaaatt    840
   ttggtaattt tggttttttt tttttttaaa aagtttatat tttgttaatg tgtatatatt     900
   gtttatttga gtgtttttaa ataaatattt attttttaaa atagtgaaat ttgaattatt    960
   agtatggaaa taatttttgg gaagataaga tttttattta aaagttttaa attgagattt    1020
   ttttgtttat gttgaatgat ggttttattt aatatttttt gaaatagggt gattatttga   1080
   attatataaa attgtgaaat gagaatattt ttgattttat atttttattt tttttttttt   1140
   ttttgagata gagttttatt ttattattta ggttggagtg tagtggtagt tttggtttat   1200
   tgtatttttt attttttagg tttaggtgat tttcgttttt tggtttttta agtagttggg  1260
   attataggta tgtgttatta tattcggtta atttttgtag tttttggtag agatggggtt   1320
   ttattatgtt ggttaggttg gttttgaatt tttgatttta agtgatttat ttattttagt   1380
   tttttaaagt gttgggatta taggcgtgag ttattgtgtt tggttattta tatttatttt   1440
   gattgatttg aaataaaatt taatatttta gggaaggtaa tttttatgtg ttttgaatta   1500
   tattgagtat taatttttta ggataattat agatttggaa aggtttaatt tagtttttta   1560
   gtttatgttg aagttttttt aagtgatagg aggaatttat aatttataag gtaatttaat   1620
   ttatttggtg ttattatcga ttgttataaa gtttattttt gggttaaaat ttattattta   1680
   tagttgtatt taatgatttt aattttgttt ttaagttata taaaatttgg atttgttttt   1740
   tatatgataa ttttttagat attttaaggt agttagttta ttttttttat tttttttttt  1800
   atagtttttt tattttttga gataaatatt tttcgttatt ttaattattt tttatatggt   1860
   attattttta ttatttcgat tgttttttaa ggatgttaaa ttttgttaat gtttttttta  1920
   aaatgtattt ggaattggaa ataatatttt ttggtagttg aattggattt ggatgttgta   1980
   tatttggata ttaatgtagt ttgaagttgt tgtaagtttt ttagtcgatt tgtaatgttg   2040
   gtttatgatg aatttgtaat taaataaaat tttaaggttt taatttggag gattgttttt   2100
   aagtgatttt tttttaaatt aagttttaat ttttttttag ttatgtaata gatttttttt  2160
   gtttgttttt tgagacgttc gttttgtcgt ttaggttggg gtgtagtggt acgattttag   2220
   tttattgtaa tttttatttt ttgggtttaa gtaatttttt tgttttagtt ttttaagtag   2280
   ttgggattat aggtattcgt tattatgttt ggttaatttt tttttttttt tttttatttt   2340
   tttttagtag agacggggtt tttttatttt ggttaggttg gtttcgaatt tttgatttta   2400
   ggtgatttat tttttttagt tttttagagt gttgggatta taggcgtgag ttattacgtt   2460
   cggttttttt tgagatggag tttcgttttg tcgtttagga tggagtgtag tggtatgatt   2520
   ttggtttatt gtaattttcg tttttcgggt ttaaggtatt tttttgtttt agtttttcga   2580
   gtagttggga ttataggtat ttgttattac gtttggttaa tttttgtatt tttagtagag   2640
   gtgggggttt tattatgttg gttaggttgg tttcgaattt ttgattttaa gttattcggt   2700
   tattttggtt ttttaaagtg ttgagattat aggtatgagt tatcgtgttc ggtttatgta   2760
   ttagatattt tttaaaaatt agatgtaaga ttttatattt atttttatta aattttattt  2820
   tattagttta ttaggtagtt ttggtttttt tttttttttt tttttttttt tttttttttt   2880
   ttttgagata gagttttatt tttgttgttt aggttggagt gtagtggcgc gattttagtt   2940
   tattgtaatt tttgtttttc gggtttaagc gatttttttg ttttagtttt ttaagtagtt   3000
   gggattatag gtatgtttta ttacgtttag ttaattttgt gtttttagta gagatagggt   3060
   ttttttttgt tggttaggtc ggttttaaat tttcgatttt aggtgatttg tttatttcgg   3120
   ttttttaaag tgttgggatt ataggtgtga attattatgt tcggttgttt tttttaagat  3180
   tagttaagtg tagagtgaga aggggggaaa gagtagaata aggagtttga ttagttttgg   3240
   tttttttgaa ttttgatttt tgttatttag tttattgtgt tgatttaata aagggttttg  3300
   aagttaatgt tggaatgttt ttagatatat aggaaatgat aatttattaa aagttatgga   3360
   gattatattt taaaagtttt atgtagatat aatttagttg ttgaggtata tgagttaata   3420
   ttattataaa ttgatatggt taggaaagat tgaagtttat tgttagtttg ttttgtgttg   3480
   aatattattt taaaagagat taaattttaa ggtatattgt gttaatataa aggtaaaaga   3540
   gttggttaat tttttttaag aaattaatta atggtagggc gcggtggttt acgtttgtaa  3600
   ttttagtatt ttggaaggtc gaggcgggta gattatttga ggttaggagt ttaagattag   3660
   tttgattaat ttggagaaat ttcgttttta ttaaaaaata taaaattagt cgggtatggt   3720
   ggcgtatgtt tgtaatttta gttatttgag aggttgaggt aggataattg tttgaatttg   3780
   ggaggcggag gtcgtatgaa ttagtattat attattgtat tttagtttgg gtagtaagag   3840
   taaaatttta ttttaaaaaa aaaaaaaatt aattaatgta cgtataatat agagatagag   3900
   ttattgttat tattttttta ttttgttttt gataagttta tattgagtat gttattaggt   3960
   aatgtgggag attttggaga attaggtttg ttttataatt agggttatta tatattttag 4020
   tttgtttggg gtaattttga tttatgtttg ttgttttagt gtgattatta ttagtgtaat 4080
   tttttatttt gagaagtgtt taggtttgga ggataaatta tttttttaat aattgatatt 4140
   ttttttataa tttaacgggt tttttttagt attttatttg ggttaaaatt attagttgta 4200
   atttggtagt tttaataaga ttgtagtaat atttattttt tatttgaata gattgttatt 4260
   tgattaaggg aagttaatag taaaagtaat tgtagggtat atgtatgtta tgggtaaaaa 4320
   aaaaaaagta atagtaatta aggtttgtag gtatttagaa ttttttttga gttatttttt 4380
   agagggtagt gttatatata tatttgtaat tatttagtta taataaaaaa agggttttta 4440
   tttatgtatg aaaattagaa atattttata tttttaaaga atatattgga attaatatta 4500
   tgattaaaat atattttgtt aagtaaagag atattaaaaa ttaatttatt aatattttga 4560
   atatttttta atttgtaaaa ataattttga tgttttgaat atataatgat ttattataat 4620
   aattatgtat agattttaat aatttgtata ttttatgttt ttatgttttt tttaattaat 4680
   gatttgatat ggttaataat tataatatat tttgtattat agtttatata tttatgtaaa 4740
   ataagtattt aaaaattatt agttttattt tgtttgttta aatattattt tttttaaaaa 4800
   gagaaaataa aaatgttaga ttttatttta tgatttgaat gatgtggtaa tgtcgaattt 4860
   tagtatttag aattagaatg ttttttagcg gtcgtgtagt tatttttatg ttataagtgg 4920
   ataatttgtt agtttttata ataaaagttt gttgtttgtg ttttatattt tggatttttt 4980
   aatataatgt ttttttttta gaaaaggtgg ataagtttta tttttaagag aagatgattt 5040
   ttaatagttt tgaaggattt aaaatttgtg tatttgtaga tattaataag gaagaagaat 5100
   ttgtagaaga gtttaataga ttaaaaattt ttgttaattt tttaagtggt agttttgttt 5160
   tagtattaat attggtacga gtagggtttt tttatattgg tgaaggagat atcgtgcggt 5220
   gttttagttg ttatgtagtt gtagatagat ggtaatatgg agatttagta gttggaagat 5280
   ataggaaagt atttttaaat tgtagattta ttaacggttt ttattttgaa aatagtgtta 5340
   cgtagtttat aaattttggt atttagaatg gttagtataa agttgaaaat tatttgggaa 5400
   gtagagatta ttttgtttta gataggttat ttgagatata tgtagattat tttttgagaa 5460
   ttgggtaggt tgtagatata ttagatatta tatattcgag gaattttgtt atgtatagtg 5520
   aagaagttag attaaagttt ttttagaatt ggttagatta tgtttattta attttaagag 5580
   agttagtaag tgttggattt tattatatag gtattggtga ttaagtgtag tgtttttgtt 5640
   gtggtggaaa attgaaaaat tgggaatttt gtgatcgtgt ttggttagaa tataggcgat 5700
   atttttttaa ttgttttttt gttttgggtc ggaattttaa tattcgaagt gaatttgatg 5760
   ttgtgagttt tgataggaat tttttaaatt taataaattt tttaagaaat ttatttatgg 5820
   tagattatga agtacggatt ttttattttg ggatatggat atatttagtt aataaggagt 5880
   agtttgtaag agttggattt tatgttttag gtaaatttta ttataaaatt aataaatagt 5940
   tttttaagta tgttagggtt tataaaaagt aaatagatgt ttttagtttt ttgaattggt 6000
   aaatatttag gtataatttg gtatgattat atatatattt gtattatttc gtgaattttt 6060
   atgttgaatt tgtaatgtaa ttattgaatt tatgtgaaaa agattattat attatgagtt 6120
   atgttttttt ttatgtaatt gtttttagga agtagataat ttgtaaaaga tggtagatat 6180
   ttttaatagt aaaatgggtt taatagtaat attaattatt atttattgag tatttattat 6240
   aagtgtttaa tatatatatt ttatgtattt tttttaagtt ttataatagt tttgtttgtt 6300
   tgtttttgag atagagtttc gtttttgtta tttaggttgg agtgtaatgg cgtgatttag 6360
   gtttattata atttttattt ttcgggttta agcgattttt ttgttttagt tttttgagta 6420
   gttgggatta taggtatgtg ttattacgtt cggttaattt tgtattttta gtagatatag 6480
   ggtttttttt tgttggttag gttggtttta aatttttgat tttaggtgat ttattcgttt 6540
   cggtttttta aagtgttggg attataggtg tatgtattat gtttggttaa tttttgtatt 6600
   tttagtagag atagggtttt attatggtta ggttggtttc gaatttttga ttttgtgatt 6660
   cgttcgtttt ggttttttaa agtgttggga ttataggtat gagttattgc gtcggttata 6720
   tgagtatttt taaatggatt gtggtagata ttttaggaag ggttattgga gtgattgttt 6780
   ttaaagatat tttaaaaagt tatattatta tatatttgat ttatttattt taagattgaa 6840
   taatgtttat aaaatattgg ttatttcgaa agattttatt tatgtattat tatatagtaa 6900
   ttttgaaggt ttgtttagtg ttatgtagtt ataaatttag agaaatttat aattttatta 6960
   tgtagaattt taatgttatt aatatttgaa atataatttg ttttaaaatt ttatagtttg 7020
   aaatagtaag gataatattg gtggtttaaa taatatatta aaaagaaagt atggtcgggc 7080
   gcggtggttt tcgtttataa ttttagtatt ttgggaggtt gaggtgggtg gattatttga 7140
   agttaggagt ttaagattag tttggttaat atggtgaaat ttcgttttta ttaaaaatat 7200
   aaaaattagt tgggcgtgat ggtatgcgtt tgtagtttta gttatttggg agattgaggt 7260
   aggagaatta tttgaattta gaaggtagag gttgtagtga gtcgtgatcg cgttattgta 7320
   ttttagtttg ggtaataaga gtaaaatttt attttagaaa aaaataataa taattaataa 7380
   ataaataaat aaataaataa ataaataaat gaaaataaag tatgttaggt atagtggttt 7440
   acgtttgtaa ttttaatatt ttggaaggtc gaggcgggtg gattatttga ggttaggagt 7500
   ttaagattag gttggttaat atggtgaaat tttattttta ttaaagatat aaaaaattag 7560
   tcgggtgtgg tggcgtatgt ttgtaatttt agttatttga gaggttgagg taggagaatc 7620
   gtttgaattt aggaggtaaa ggttgtagtg aattaagatt gcgttattgt atttcggttt 7680
   ggggaataga gttagattta gttttaaaaa aagaaagaaa gtaataattt tagtttttaa 7740
   tataaaattt gggtagtttt atttttgttt tttataaaaa tattttatat tgaattagtg 7800
   aatttagtgt gtattttttt tttaaaggat aaaaattata tttgttattt agatgtgaat 7860
   ttgaatgttt tgttttaagt tgtgagattt ttatgttttt tagatgtgtt taatttttag 7920
   gtgttaaatg aatatttttg tgtaagtttt taattgtata aatatatata tttttgtgtg 7980
   ttttcgtagg 7990
<210> 8
   <211> 7990
   <212> DNA
   <213> Artificial Sequence
<220>
   <223> chemically treated genomic DNA (Homo sapiens)
<400> 8
   tttacgaaaa tatataggaa tatatgtatt tgtgtaatta gaagtttata taaaaatatt      60
   tatttaatat ttaaaaatta agtatattta aaaaatatga aaattttata gtttaaaata     120
   agatatttaa atttatattt aagtaataga tatgattttt attttttgag gaagaaatat     180
   atattaaatt tattgattta atgtgaggta tttttataga agataaaaat aaaattgttt     240
   aagttttatg ttaaaaatta aaattgttat tttttttttt tttttgagat tgagtttggt     300
   tttgtttttt agatcggagt gtaatggcgt agttttggtt tattgtaatt tttgtttttt     360
   gagtttaagc gatttttttg ttttagtttt ttaagtagtt gggattatag gtatgcgtta     420
   ttatattcgg ttaatttttt atatttttgg tagagatggg gttttattat gttggttagt     480
   ttgattttga atttttgatt ttaagtgatt tattcgtttc ggttttttaa agtgttggga     540
   ttataggcgt aagttattgt atttggtata ttttattttt atttatttat ttatttattt     600
   atttatttat ttattaatta ttattatttt tttttgagat ggagttttat ttttgttgtt     660
   taggttggag tgtaatggcg cgattacggt ttattgtaat ttttgttttt tgggtttaag     720
   tgattttttt gttttagttt tttaagtagt tgggattata ggcgtatgtt attacgttta     780
   gttaattttt gtatttttag tagagacggg gttttattat gttggttagg ttggttttga     840
   atttttgatt ttaggtgatt tatttatttt agttttttaa agtgttggga ttataggcgg     900
   gagttatcgc gttcggttat attttttttt tagtatgtta tttgggttat taatattgtt     960
   tttattgttt taaattgtga aattttaaaa taagttatgt tttaagtatt agtaatatta    1020
   aagttttata taatgaaatt gtaagttttt ttaagtttat agttatatga tattaaataa    1080
   atttttaaga ttattatatg atggtatata aataaaattt ttcgaaataa ttaatatttt    1140
   ataggtatta tttaatttta aaataaatgg gttaaatata taatggtata attttttaaa    1200
   atatttttag aagtaattat tttaatggtt ttttttgaaa tgtttattat agtttatttg    1260
   aaagtattta tatggtcggc gtagtggttt atgtttgtaa ttttagtatt ttgggaggtt    1320
   aaggcgggcg gattataagg ttaggagttc gagattagtt tggttatggt gaaattttgt    1380
   ttttattaaa agtataaaaa ttagttaggt atggtgtatg tatttgtaat tttagtattt    1440
   tgggaggtcg aggcgggtgg attatttgag gttaggagtt tgagattagt ttgattaata    1500
   gggagagatt ttgtgtttat taaaaatata aaattagtcg ggcgtggtgg tatatgtttg    1560
   taattttagt tatttaggag gttgaggtag gagaatcgtt tgaattcggg aggtggaggt    1620
   tgtggtgagt ttaaattacg ttattgtatt ttagtttgga taataagagc gaaattttgt    1680
   tttaaaaata aataaataaa gttattgtga agtttagaga aaatgtatga agtatatgta    1740
   ttgagtattt atagtaagtg tttaataaat gatggttaat attattatta gatttatttt    1800
   gttattgggg atgtttatta ttttttgtaa gttatttatt ttttaaaaat aattatatga    1860
   agagaagtat gatttataat atggtagttt tttttatata aatttagtag ttatattata    1920
   ggtttaatat aagagtttac ggaataatat agatatatat ataattatgt taagttatat    1980
   ttaaatattt attagtttag ggggttaagg gtatttattt attttttatg agttttggta    2040
   tatttgggaa gttatttatt ggttttataa taaagtttat ttaaagtata aaatttagtt    2100
   tttgtaagtt gttttttgtt aattgagtat atttatgttt taaaagtaaa gattcgtgtt    2160
   ttataatttg ttatggatgg attttttgga agatttgttg aatttgggaa atttttatta    2220
   gaatttatag tattagattt atttcgaata ttaagatttc ggtttaaaat aaagaagtaa    2280
   ttaggaaagt gtcgtttgtg ttttgattag gtacgattat aaggttttta attttttagt    2340
   tttttattat aataaaagta ttgtatttgg ttattaatat ttgtgtagta gagtttagta    2400
   tttgttaatt tttttggggt taggtgagta tagtttggtt agttttgaaa ggattttaat    2460
   ttagtttttt tattatatat ggtagggttt ttcgggtata tggtgtttga tatatttata    2520
   atttgtttag tttttaaaag atagtttgta tgtgttttag atggtttgtt taaggtaaaa    2580
   tgatttttgt tttttagata gtttttaatt ttgtattgat tattttggat attagaattt    2640
   gtagattgcg tggtattatt tttaagataa aagtcgttga taaatttgta atttggggat    2700
   atttttttgt gttttttaat tgttgagttt ttatattgtt atttatttat agttgtatga    2760
   taattaaagt atcgtacggt atttttttta ttagtataaa gaaattttgt tcgtgttagt    2820
   gttgatgttg aaataggatt attatttgga aaattagtaa aagtttttaa tttattaaat    2880
   ttttttataa attttttttt tttattgatg tttgtaggta tataagtttt agatttttta    2940
   aaattgttaa aagttatttt tttttgaaaa taggatttgt ttattttttt taaaaagaga    3000
   atattatatt aggaaattta aaatgtgaaa tataagtaat agatttttgt tataggagtt    3060
   aataaattat ttatttatga tataaaaata attatacgat cgttaagaaa tattttaatt    3120
   ttaaatatta gagttcgata ttattatatt atttaagtta taaagtaaaa tttagtattt    3180
   ttgttttttt tttttgagga aagtaatatt taagtaggta gaataaaatt aataattttt    3240
   aaatgtttat tttatataaa tatgtaaatt gtgatgtaga atatattata attattaatt    3300
   atgttaaatt attaattagg aaaaatatga aagtataaaa tatgtagatt attaaaattt    3360
   atgtataatt gttataatga attattatat atttaaggta ttaaagttgt ttttataagt    3420
   taaaaaatgt ttagaatgtt aatgaattaa tttttaatat ttttttgttt agtaaaatat    3480
   gttttaatta taatattggt tttaatgtgt tttttaagag tatgaaatat ttttgatttt    3540
   tatgtatgaa tgagagtttt ttttttgttg taattggata attatagata tatatgtaat 3600
   attgtttttt agagggtggt ttaggaaaaa ttttaagtat ttgtaaattt taattgttgt 3660
   tatttttttt tttttgttta tgatatatat gtgttttgta gttatttttg ttattaattt 3720
   tttttggtta agtaataatt tgtttaaatg gaagataagt attgttgtag ttttattaga 3780
   gttgttaaat tatagttggt aattttaatt tagataaaat attaaaagga attcgttagg 3840
   ttatgagaag ggtattaatt attagaaaga taatttattt tttaaatttg gatatttttt 3900
   aaagtgaaaa attatattag taataattat attgggataa taggtataaa ttagggttgt 3960
   tttaggtaaa ttagagtata tggtaatttt agttatgagg taaatttggt tttttagaat 4020
   tttttatatt gtttgataat atgtttaata taaatttgtt aaaggtaaaa taagagaatg 4080
   gtaataatga ttttgttttt atattatgcg tgtattaatt gatttttttt ttttttgaga 4140
   tggagttttg tttttgttgt ttaggttgga gtgtaatggt gtgatattgg tttatgcgat 4200
   tttcgttttt taggtttaag taattatttt gttttagttt tttaagtagt tgggattata 4260
   ggtatgcgtt attatgttcg gttaattttg tattttttag tagagacggg gtttttttag 4320
   gttggttagg ttggttttga atttttgatt ttaggtgatt tgttcgtttc ggttttttaa 4380
   aatgttggga ttataggcgt gagttatcgc gttttgttat taattgattt tttgagaaga 4440
   attagttagt ttttttgttt ttatgttgat ataatatgtt ttaaaattta attttttttg 4500
   gagtaatgtt taatataaag taagttggta atggatttta gttttttttg gttatgttaa 4560
   tttatagtgg tattaattta tatattttaa tagttgagtt atatttgtat aaaatttttg 4620
   gaatatagtt tttatgattt ttgatgaatt attatttttt atatgtttga aaatatttta 4680
   atattagttt taaggttttt tgttgaatta gtataataaa ttggatgata aaaattaaga 4740
   tttaaagaaa ttaaggttga ttaaattttt tgttttattt tttttttttt ttttattttg 4800
   tatttgatta gttttaaaaa aaatagtcgg gtatggtggt ttatatttgt aattttagta 4860
   ttttgggagg tcgaggtggg taaattattt gaggtcggga gtttgagatc ggtttgatta 4920
   atagggagaa atttgttttt tattaaaaat ataaaattag ttgggcgtgg tggagtatgt 4980
   ttgtaatttt agttatttgg gagattgagg taggagaatc gtttgaattc gggaggtaga 5040
   ggttgtagtg agttgagatc gcgttattgt attttagttt gggtaataag agtgaaattt 5100
   tgttttaaaa aaaaaaaaaa aaagaaaaga aaagaaaaga aaaaaattaa ggttatttga 5160
   taggttaatg agataaaatt taatagggat aagtataaag ttttatattt ggtttttaaa 5220
   aaatatttgg tgtataggtc ggatacggtg atttatgttt gtaattttag tattttggga 5280
   ggttaaggtg gtcggataat ttgaggttag gagttcgaga ttagtttgat taatatggtg 5340
   aaatttttat ttttattaaa aatataaaaa ttagttaggc gtggtggtag gtatttgtaa 5400
   ttttagttat tcggaaggtt gaaatagaag aatgttttga attcggggag cggaggttgt 5460
   agtgagttaa gattatgtta ttgtatttta ttttgggcga tagagcgagg ttttatttta 5520
   aaaaaggtcg ggcgtgatgg tttacgtttg taattttagt attttgggag gttgaggagg 5580
   gtgggttatt tgaggttagg agttcgagat tagtttgatt aaaatggaga aatttcgttt 5640
   ttattaaaaa aaaataaaaa agaaagaaag aaaattagtt aggtatgatg gcggatgttt 5700
   gtaattttag ttatttggga ggttgaggta ggagaattgt ttgaatttag gaagtggagg 5760
   ttgtagtgag ttgagatcgt gttattgtat tttagtttgg gcgatagagc gagcgtttta 5820
   aaaaataaat aaaaaaaatt tgttgtataa ttaaaagaag attgagattt agtttaagaa 5880
   gaaattattt aaaggtaatt ttttagatta aaattttggg attttattta attataaatt 5940
   tattataagt taatattata agtcgattaa aaaatttata gtaattttag attgtattag 6000
   tgtttagatg tgtaatattt agatttaatt tagttattaa gggatattat ttttagtttt 6060
   aaatatattt tggaagggat attaataaag tttaatattt ttagggagta atcgggatga 6120
   taaaaatgat attatatgag aaatagttgg aatagcgaag gatatttatt ttaaagggtg 6180
   gaaaaattgt gaggggaagg gtagagagga tagattaatt attttaagat atttaaaaga 6240
   ttattatgta gaaaatagat ttaaatttta tatagtttga gggtagaatt aaggttatta 6300
   aatataattg taaatagtag attttaattt aaggatgggt tttataataa tcgatggtag 6360
   tattaaatgg attgggttgt tttgtagatt atgagttttt tttattattt aaaggaattt 6420
   tagtatggat tgggagattg ggttaaattt ttttaagttt ataattattt tgaagaatta 6480
   atgtttagta taatttaaaa tatatgagaa ttgttttttt tgaggtgtta aattttattt 6540
   taagttaatt aaaatagatg taagtggtta ggtatagtgg tttacgtttg taattttaat 6600
   attttgggag gttgaggtgg gtggattatt tgaggttagg agtttaagat tagtttggtt 6660
   aatatggtga aattttattt ttattaaaaa ttataaaaat tagtcgggtg tggtggtata 6720
   tgtttataat tttagttatt tgggaggtta agggacgaga attatttgaa tttgggaggt 6780
   ggagggtgta atgagttaag attgttattg tattttagtt tgggtgatag agtgagattt 6840
   tgttttaaaa aaaaaaaaaa aaatagaagt ataaaattaa agatattttt attttataat 6900
   tttgtgtagt ttaagtagtt attttatttt agaaaatatt agataaaatt attatttaat 6960
   atgagtagga gaattttaat ttgaagtttt tgagtggaga ttttattttt ttagaggttg 7020
   tttttatgtt aatagtttag attttattgt tttgaaaagt aaatatttat ttagaagtat 7080
   ttaagtgaat aatatatata tattagtaga gtgtgagttt tttgagaagg aggaaaatta 7140
   gaattattag agtttaagag tgaaaggtgg aaggatgatt gatgaaagat agaaattttg 7200
   ggtttggagg aaggtattaa ataaaaaggg taagataggg taaaaaaata taggatttaa 7260
   gtgaaatgga ggtggggaaa atgatattat agaaaataag tatatatgga gtagatgaat 7320
   tgagaatata gaagaaaata gggtatgaat attagataag ggtgataaaa aggtttttga 7380
   agtgaaaagg tgtgggtgta ataaagtatg ttgggataat ttaaggtttt tttgtgaaag 7440
   tatttttagg ttttgtattg ggtaagtgga aaggtaggaa ggtacggaaa gagaaaaatg 7500
   ttaaagaaag tttttggtat aaaggggttg ggaagttagg aattttaagg gtaatagtag 7560
   ttgggaaaat agtattatgg aagtaaaaaa tgggataaaa aggattagta agtaatggtg 7620
   tatgtttaaa tgaagggttt gggagatttt ataatggtga tatgtaaggt tttatagtat 7680
   atatgtatgt atatatattt agtttagtta attgcgtaaa gagagttatt aggaggtgtt    7740
   aaggagagga ggttgatagt taatttagcg agaatgtggg aatgtggagg tgggggatat    7800
   agagataaag gtatatataa aattttggtg tttgtatacg atataaaagt ttttattttt    7860
   tttttttttt tttttttttt ttgaaataga gttttgtttt tatggtttag gttggagtga    7920
   aatggcgtga ttttaattta ttgtaatttt cgttttttag gtttaagtta tttttttgtt    7980
   ttagtttttt                                                           7990
<210> 9
   <211> 5314
   <212> DNA
   <213> Artificial Sequence
<220>
   <223> chemically treated genomic DNA (Homo sapiens)
<220>
   <221> unsure
   <222> (17, 36, 247, 958, 1442, 1554, 1558, 1752, 2756, 2766, 2807)
<220>
   <221> unsure
   <222> (2961, 3189, 3237, 3592, 3750, 4732, 4735, 4737, 5216)
<400> 9
   tagttttagg agggagntgg cgtttatata ggacgnaggg gtttggtttg ggtagaagag 60
   ggtagagatt tttttttgga ttgaatttaa gggaggagta ttttaggcgg gagttgtagg 120
   aggttgtgtt ggttttttta ggggaaggga aggggaggga aagaaaattt tattttaaaa 180
   aaaaaaaaag aaaaaagaag tttagaaatt cgcggagata ttattatatt atttttttta 240
   ggttggnagt gtagtggttt aattatggtt tattgtagtt tcgatttttt aggattaagt 300
   gattttttta ttttagtttt tcgagtagtt ggaagtatag gtgtacgttc gattgatttt 360
   tttttttttt tttagacgga gttttatttt tgttgttttg gttggagtgt aatggtagga 420
   tttcggttta ttgtaatttt tgttttttag atttaagcga ttttcgtgtt ttagtttttc 480
   gagtagttgg gattataggt gtttattatt atgttcggat aattttttgt atttttaata 540
   gagatagggt tttattatat tggttaggtt ggttttaaat ttttgatttt aggtgattta 600
   tttgttttag ttttttaaat tgttgggatt ataggcgtga gttatcgggt atggtttttt 660
   ttggttaatt ttttaaattt ttgatagaga tggggtttta gtgttgttta ggttgatttt 720
   gaatttttag atttaagtga tttttttttt ttggtttttt aaagtgtaga aacgataagt 780
   ttaatttaaa ttttcgagag agaaaatatt tttttaaacg agggtaatgt gaatttgaaa 840
   tgttatttga ggaaagattt gttcgtttga tattttgttt gagtttgggt ggataggata 900
   gtatttgtta gtatcgggaa gtattgtaga tgggaagagg tttggttatt ttttaaangt 960
   ggtaggagtt ggagggggtg agttgaaggt aaggagaaag gaggtgggga tttaggagat 1020
   aggggttgcg tagcgggttc ggggttgata tttttacgga tatagtttat tggggtttaa 1080
   atataaaagg aatttaatta ttgtgggagg aaaagatttt tttgtttttt tgtttttttt 1140
   tttttttttt tttttttttt tttttttttt tttttttttt gagatagagt tttgttttat 1200
   cgtttaggtt ggagtgtagt ggcgtgattt cggtttattg taagttttgt ttttcgggat 1260
   tacgttattt ttttgtttta atttttcgag tagttgggat tataggcgtt tgttattata 1320
   ttcggttatt tttttgtatt ttttagtaga gatggggttt tatcgtgtta gttaggacgg 1380
   tttcgatttt ttgattttgt gattcgttcg tttcggtttt ttaaagtgtt gggattatag 1440
   gncgtgagtt atcgcgtttg gttttttttt tttttttttt ttttttttcg agatagagtt 1500
   ttatttttgt tgtttaggtt ggagtgtagt ggcgtaattt tggtttattg taantttntt 1560
   atttttaggg tttaagcgat ttttttgttt tagttttttg agtagttggg attgtaggcg 1620
   cgtattatta cgtttggtta atttttgtat ttttagtaga gatagggttt tattatattg 1680
   gttaggttgg tttcgaattt ttgattttgt gatttgttta ttttagtttt ttaaagtttt 1740
   gggattatag gncgtgagtt atcgtgttta gtttgatttt tttgtttttt taaaaattat 1800
   gttcgttttt ttatagtttt tttttgttat attaagttta tatcgtaggt ttaatttgtt 1860
   tagtgaatgt tatgtaaata ttttatgtat ttgttgatcg taggaatgat atgtatttgg 1920
   tacgtattga tcgtatttcg gggtgggaga agagagggta aggaagtaaa gaatagtttt 1980
   tttttttttt ggtgtatttt tagatgtgtc gatggggttt aggttcgttg tagatggttt 2040
   tttttttaga gataggggag gattttttat ttatttttta gtttttagga ttattttgat 2100
   ttttgttttt aggtatttaa gttatgcgtt tagatatgcg gatatattta agttgggtat 2160
   agtatagtag ttttatttta ggtagtttga aattagagtt ggggtttaaa gggattatat 2220
   ttcgagggat tgtgtggggg tcggggtata taggttattg ttttttttcg ttttttttag 2280
   ttatttttga agttagtttt attttgtttt ttagggattt taaatgtgta gagattttgg 2340
   tatttttgta gaagtttttt ttggttttaa tttatatttg gatgttgtgg ggttgtagtt 2400
   gttgttcggg ttcgggagga tgttgggggt tcggtgttta tgagtttttg aagtttttgg 2460
   aattcggttt tgagggtgtt taggtttagg tggatatttg ggttgttttt gtttatgtat 2520
   ttgatgatat tgtgtgtaga agtgaaaagg agttaggtcg ggtatgttgg tttatgtttg 2580
   taattttagt attttgggag gttgaggcgg gtggattacg aggttaggag tttaatatta 2640
   gtttggttaa gatggtgaaa tttcgttttt attaaaaata taaaaaaatt agtcgggtat     2700
   ggtggcgggc gtatgtaatt ttagttattg ggggggttga ggtagagaat tgttgngaat      2760
   ttaggnagat ggaggttgta gtgagttaag attgtgttat tgtattngta ttttagtttg     2820
   gcgatagagt aagattttgt tttaaaaaaa aaaaaaaaaa gtgaaaagga gttgtttttt     2880
   tttttttttt tgagggtagg taattgttgc ggttgttagt ggaggtggtg cgtttttggt     2940
   ttgtgtttgg gggttatttt nagtagaggt tatggtggtg ttagggttcg gttagcgagt     3000
   taattagtag gatttagggg cgatttgtta aagttaattg gatttgataa ttgtagcgaa     3060
   gttaagtttt ttgattttga tgattgtgtt gtggttgtgt aagagaatga agtatttcgg     3120
   ggtagtatgg taatgttttt aatttataaa cggtttaggt aaattattta tatatatata     3180
   tatatatgnt atgtgatata tatatatata gggatgtgtg tgtgtttata tatatgnagg     3240
   ggagagagat taggggagag aaagtaggtt ggggagaggg agagagaaag gaaaatagga     3300
   gatagagaga gagcggggag tagagagagg gaaggggtaa gagagggaga ggaggagaga     3360
   aagggaggaa gaagtagaga gtgaatgtta aaggaaatag gtaaaatata aatagaaaat     3420
   ttgggtgaag ggtatatgag tattttttgt attatttttg taattatttt ttatttaaat     3480
   tgatatcggg tcgggcgtag tggtttatat ttgtaatttt agtattttgg gaggtcgagg     3540
   taggtagatt atttgaggtt aggagtttga gattagtttg gtaaatatgg tngaaatttt     3600
   atttttatta aaaatataaa aattagtttg gtgtggtggt gtatgttttt aattttagtt     3660
   attcgggagg ttgaggtaga ataatcgatt gaattcgtgg cggggaggag gttgtagtga     3720
   gttgagatta tgttattgta ttttagattn gggcgataga gcgagattta gttttaaata     3780
   aataaataaa tattaaaata aaaagttatt gtataaaaga atgggggcgg ggtgggaggg     3840
   gtggggagag gttgtaaaaa taaataaata aataaataaa ttttaaaatg aaaaagatag     3900
   tggaggtatt aggtttgcgt ggggttggag ggttaataag gttaggtttt ttatttttgg     3960
   ttatagaatt agagaagtga gtggatgtga tgtttagttt tataagtgat tttagaatat     4020
   tttgttttaa agtaaaggat atattgattt tttttttaat aggttgtagg atttattgtt      4080
   ggtgggacgt tttgttttgc gaagggaaag gaggagtttg ttttgagtat aggtttttat     4140
   tttttattgg gtttttttta gtttttttgt ttttttatta cggtagtggt ttagtttttg      4200
   gtttttgatt ttagaaggtg gtttttttgg aaatttaggt cgtgtagtta acgatgtatt     4260
   cgtcgggata gcgatgtttg ttgtatttta tttttttttt gtttatttgt tttttatgtt     4320
   cgtttggagt agatgttttt tgtagaggtg gtattttgta aagttttttt gtttgatttt     4380
   tttttttttt ttagattgag ttttgttttt gttgtttagg ttggagtgta atggtataat     4440
   tttagtttat tgtatttttt gtttttcggg tttaagcgat ttttttgttt tagtttttcg      4500
   agtagttggg attataggta tgtattatta cgtttagtta atttttgtat ttttagtaga     4560
   gataaggttt tatcgtgatg gttaggttgg ttttgaattt taggatttaa gtgatgtttt     4620
   tgtttaggtt ttttaaagtg ttgggattat aggcgtgagt tattgtattc ggtttgtacg     4680
   cgttttttga aagtagtcga gggggcgtta ggtgtgggta gggacgagtt gngtngncgg     4740
   cgtcgttggg tgtatcgcga ttacgggtag agttacgcgg cgggaggatt ataattttcg     4800
   gtatatttcg cgtcgtttcg tttttatttt tagaaggtcg cggggggtgg atcgtttaag     4860
   agggcgtgcg ttttcgatat gtttcgcggc gcgttattaa tcgttagatt tgaatcgcgg     4920
   gattcgttgg tagaggtggc ggcggcggta tgggtgtttc gacgttgttt tttgtttggt     4980
   agtttttttt taaggattat cgtattttta tatttaagaa ttggtttttt ttggagggtt     5040
   gcgtttgtat ttcggagcgg gtgagattgt tcggtttttt ggggtttttt acgttcgttt     5100
   tgttttgttt ttagcgaggt tattgtgatt gggtttcggg ggtataagtc gttttttttt     5160
   tttcgttttg tttttagcga ggttattgtg gttgggtttt ttgggtttag gtcggntttt     5220
   ttttttttgt tttgttttta tcgaggtttt tgtggttggg tttcggggtt tcgggttgtt      5280
   acgtttattt acgagttgtg ttgttttttg taga                                  5314
<210> 10
   <211> 5314
   <212> DNA
   <213> Artificial Sequence
<220>
   <223> chemically treated genomic DNA (Homo sapiens)
<220>
   <221> unsure
   <222> (99, 578, 580, 583, 1565, 1723, 2078, 2126, 2354, 2508, 2549)
<220>
   <221> unsure
   <222> (2559, 3563, 3757, 3761, 3873, 4357, 5068, 5279, 5298)
<400> 10
   tttgtaaggg atagtatagt tcgtgagtgg acgtggtagt tcggaatttc gaggtttagt      60
   tataaaggtt tcgatgggga taaagtaggg aggggaggnt cggtttggat ttaaggggtt     120
   tagttatagt ggtttcgttg gggataggac ggggagggga gggcggtttg tattttcgag     180
   gtttagttat agtggtttcg ttagggatag ggtaaggcgg gcgtggggga ttttaggagg     240
   tcgggtagtt ttattcgttt cggggtgtag gcgtagtttt ttaagaaggg ttagtttttg     300
   aatgtagaga tgcggtggtt tttgagaaag ggttgttagg tagggggtaa cgtcggggta     360
   tttatgtcgt cgtcgttatt tttgttaacg ggtttcgcga tttaaatttg gcggttaatg     420
   gcgcgtcgcg gggtatgtcg ggagcgtacg tttttttagg cggtttattt ttcgcggttt     480
   tttgggagta gaggcggggc ggcgcggggt gtgtcgggag ttgtagtttt ttcgtcgcgt     540
   ggttttgttc gtggtcgcgg tgtatttagc gacgtcgntn gtntagttcg tttttgttta     600
   tatttagcgt tttttcgatt gtttttaaag aacgcgtgta ggtcgggtgt agtggtttac     660
   gtttgtaatt ttaatatttt gagaggttta ggtaggagta ttatttgagt tttggagttt     720
   aagattagtt tggttattac ggtgaaattt tgtttttatt aaaaatataa aaattagttg     780
   ggcgtggtgg tgtatgtttg taattttaat tattcgggag gttgaggtag gagaatcgtt     840
   tgaattcggg aggtagaggg tgtagtgagt tgagattgtg ttattgtatt ttagtttagg     900
   taataagagt aaaatttagt ttaaaaaaaa aaaaaaagtt agataggaga gttttatagg     960
   gtgttatttt tgtaaaaagt atttatttta gacgggtatg aaggataaat gaatagggga    1020
   gggatggagt gtagtagata tcgttgtttc ggcgagtata tcgttgattg tacgatttgg    1080
   gtttttagga gggttatttt ttggagttag gggttaggga ttgggttatt atcgtgataa    1140
   gaagataagg gagttgggga aagtttagtg gagggtgggg gtttgtgttt agggtaaatt    1200
   tttttttttt tttcgtaaag tagggcgttt tattaatagt aagttttgta gtttattaaa    1260
   aaaaaaatta gtgtgttttt tgttttggaa tagggtgttt tggagttatt tgtggagttg    1320
   ggtattatat ttatttattt ttttggtttt atggttagag ataagaggtt tggttttatt    1380
   agttttttag ttttacgtag gtttggtgtt tttattgttt tttttatttt ggggtttatt    1440
   tatttattta tttatttttg taattttttt ttattttttt tatttcgttt ttattttttt    1500
   atatagtaat tttttatttt gatgtttatt tatttatttg aaattgagtt tcgttttatc    1560
   gtttnagttt ggagtgtagt ggtatgattt tagtttattg taattttttt ttcgttacgg    1620
   gtttaatcga ttgttttgtt ttagtttttc gagtagttga gattaaaggt atgtattatt    1680
   atattaggtt aatttttgta tttttagtag agatggggtt ttnattatgt ttgttaggtt    1740
   ggttttaaat ttttgatttt aagtgatttg tttgtttcgg ttttttaaag tgttgggatt    1800
   atagatgtga gttattgcgt tcggttcgat gttaatttaa ataaaagata attgtaagaa    1860
   tagtataaag aatatttata tattttttat ttagattttt tgtttatgtt ttgtttgttt    1920
   tttttaatat ttattttttg tttttttttt tttttttttt tttttttttt ttttttattt    1980
   tttttttttt tttatttttc gttttttttt tgttttttgt tttttttttt tttttttttt    2040
   ttttaattta tttttttttt tttagttttt tttttttnta tatatgtgaa tatatatata    2100
   tttttgtatg tgtatatatt atatgntatg tatatgtatg tatatgggtg gtttatttga    2160
   atcgtttgta agttgaaggt attattatat tatttcgaaa tattttattt ttttatataa    2220
   ttataatata attattaaaa ttaggaaatt taatttcgtt gtagttatta aatttagttg    2280
   attttggtag gtcgtttttg ggttttgttg attggttcgt taatcgggtt ttggtattat    2340
   tatggttttt gttnggggtg gtttttaggt atagattaag gacgtattat ttttattggt    2400
   aatcgtagta gttgtttgtt tttaggaggg aggaaaggaa taattttttt ttattttttt    2460
   tttttttttt tgagatagag ttttgttttg tcgttaggtt ggagtgtnag tgtagtggta    2520
   taattttggt ttattgtaat ttttatttnt ttgggtttnt agtaattttt tgttttagtt    2580
   tttttagtag ttgggattat atgcgttcgt tattatgttc ggttaatttt tttgtatttt    2640
   tagtagagac ggggttttat tattttggtt aggttggtat tgaatttttg atttcgtgat    2700
   ttattcgttt tagtttttta aagtgttggg attataggta taagttagta tgttcggttt    2760
   aatttttttt tatttttgta tataatgtta ttaaatgtat ggataaggat agtttaggtg    2820
   tttatttgga tttgaatatt tttaaaatcg agttttagga gttttaaaag tttatgggta    2880
   tcgggttttt agtatttttt cgagttcgag tagtagttgt agttttatag tatttaggtg    2940
   taagttagga ttagaagggg tttttataaa agtgttagag tttttgtata tttgaaattt    3000
   ttgagaagta gagtgaggtt gattttagga atggttgaga aagacggggg gaagtagtgg    3060
   tttgtgtgtt tcgattttta tatagttttt cggggtgtgg tttttttgga ttttagtttt    3120
   gattttaagt tgtttggggt ggggttgttg tgttgtgttt agtttgaata tattcgtatg    3180
   tttagacgta taatttgagt gtttgaaggt aggagttagg atggttttgg aggttgggga    3240
   gtgggtggag gatttttttt tgtttttggg aagggggtta tttgtagcga gtttgggttt    3300
   tatcggtata tttgaaggtg tattaggaaa ggagggggtt attttttgtt tttttgtttt    3360
   tttttttttt atttcgaggt acgattagtg cgtattaagt atatattatt tttgcgatta    3420
   gtaggtgtat gaaatatttg tatagtattt attggataaa ttaggtttgc ggtgtggatt    3480
   taatatgata agagaaggtt gtgagagaac gaatatagtt tttgaaaggg tagaggggtt    3540
   aggttgggta cggtggttta cgntttgtaa ttttaggatt ttgggaggtt gaggtgggta    3600
   gattataagg ttaggagttc gagattagtt tggttaatat ggtgaaattt tgtttttatt    3660
   aaaaatataa aaattagtta ggcgtggtgg tgcgcgtttg tagttttagt tatttaggag    3720
   gttgaggtag gagaatcgtt tgaattttgg aggtggnagg nttgtagtga gttaagattg    3780
   cgttattgta ttttagtttg ggtaataaga gtgaaatttt gtttcggaaa aaaaaaaaag    3840
   aaagaaaaaa gagttaggcg cggtggttta cgntttgtaa ttttagtatt ttgggaggtc    3900
   gaggcgggcg gattataagg ttaggagatc gagatcgttt tggttaatac ggtgaaattt    3960
   tatttttatt aaaaaatata aaaaaatagt cgggtgtggt ggtaggcgtt tgtagtttta    4020
   gttgttcggg aggttgaggt aggagaatgg cgtgatttcg ggaggtagag tttgtagtga    4080
   gtcgagatta cgttattgta ttttagtttg ggcgatagag taagattttg ttttaaaaaa    4140
   aaaaaaaaaa aaagaaagaa agaaaaagaa aaaaagaaaa ggtagaaagg tagaagagtt    4200
   tttttttttt ataatagttg ggtttttttt atgtttgagt tttagtgaat tgtattcgtg    4260
   ggggtgttag tttcgagttc gttgcgtagt ttttgttttt tgggttttta tttttttttt    4320
   ttttattttt agtttatttt ttttaatttt tgttatnttt ggagagtgat taagtttttt    4380
   tttatttgta gtgtttttcg atgttggtag gtgttgtttt gtttatttag gtttaaatag    4440
   gatgttaggc gaatagattt ttttttaaat ggtattttag atttatattg ttttcgttta 4500
   aaaaagtatt ttttttttcg ggggtttggg ttgggtttgt cgtttttgta ttttgggaga 4560
   ttaaggaggg aggattattt gaatttagga gtttaagatt agtttgggta atattgagat 4620
   tttattttta ttaaaaattt aaaaaattag ttaggaaagg ttatgttcgg tggtttacgt 4680
   ttgtaatttt agtagtttgg gaggttgagg taggtggatt atttgaggtt aggagtttga 4740
   gattagtttg attaatatgg tgaaattttg tttttattaa aaatataaaa aattattcgg 4800
   gtatggtggt gggtatttgt aattttagtt attcgggagg ttgaggtacg agaatcgttt 4860
   gaatttaaga ggtagaggtt gtagtgagtc gagattttgt tattgtattt tagttagagt 4920
   aataagagtg agatttcgtt taaaaaaaaa aaaaaaaatt agtcgggcgt gtatttatat 4980
   ttttagttat tcgggaggtt gaggtggaag gattatttga ttttgggagg tcgaggttgt 5040
   agtgagttat gattgggtta ttgtattntt agtttggggg ggatagtgtg atagtgtttt 5100
   cgcgggtttt tggatttttt tttttttttt ttttttttga gatgaagttt tttttttttt 5160
   tttttttttt tttttagaag gattagtata gttttttata gttttcgttt ggggtgtttt 5220
   ttttttgaat ttagtttagg aggaagtttt tgtttttttt tgtttaggtt aagtttttnc 5280
   gttttgtgtg gacgttantt tttttttgga gttg 5314
<210> 11
   <211> 17580
   <212> DNA
   <213> Artificial Sequence
<220>
   <223> chemically treated genomic DNA (Homo sapiens)
<400> 11
<210> 12
   <211> 17580
   <212> DNA
   <213> Artificial Sequence
<220>
   <223> chemically treated genomic DNA (Homo sapiens)
<400> 12
   tttgtagagg gttagtgggt aggggggcga cgttaatttt agttttttta agtttttggt    60
   tttgtttgaa ttttttttat tagttttgta ggtttattcg tgggtttttt aggggcgtat   120
   tatagtttat tttttagagt tttagttttt tgtatgtgaa tcgaggggaa gtttaggggt   180
   ttttggtgaa gatttaggtt aattttttat taattttacg aatttttatc gagtgttaat    240
   aatgggtgag gttttgtgtt tagtataaaa taagggtata agaaatatta gttatattat   300
   aaggttagga gatcgagatt attttggtta atatggtgaa attttgtttt tatttaaaat   360
   ataaaaatta gttgggtata gtggtacgtg tttatagttt tagttattcg ggaggttgag   420
   gtagaagaat cgtttgaatt taggagggag aggtcgtagt gagtcgagat tacgttattg   480
   tattttagtt tgggcgatag gttttttttt ttaaaagaaa aaaaaaaaaa gtaagaagta    540
   ttattagtgt aagattttta tggggttttt tggtaatttt tgggttggta gttattttat   600
   agtgtttgtt ttatatcgga ggaagttaat tttttggata ggaaagtgtt ttgttatagt   660
   cggtaggatt gaatgttttt tttgtaaggg atttgtagag tttttggaga ataagaattt   720
   ggtcgttata tgttcgtatt ttttatagag ttttttttgg agatagtgtt tggaattgtt   780
   gacgttttta ggtattattg atggtgtatt tacgtgtagg tcgagatatt tttttattat   840
   ttttttaatt attttgggag gtgagtatta aatatgttta ggtgagaaga tagaagttta   900
   gagagttaag atttgtttag tttttgtgaa gttgtggagg tttgaggttg gaaggtttta    960
   aaggtttggt ggaatttaga gtttagtgat gattagcgga gttttgtgtt tttttttttg   1020
   tttagttagt tttcgatttt tttagtttgt gtttttattt ttatgagggg ataattttag   1080
   ttttgatttt tagtggttgt gtgaatatta tataaaatta aaaaatgtga aagtgttttg   1140
   tggttgggcg tgatttttta cgtttgtaat tttagtattt tgggaggttg aggtaggtgg   1200
   attgtttgag tttaggagtt cgagattagt ttaggtaata ttgtaaaatt ttatttttat    1260
   taatttaaaa aattaataaa agaaggttag acgtggtggt ttacgttagt aattttagta   1320
   ttttgggagg ttaaggtagg tggattattt gaggttagaa gttcgagatt agtttggttt   1380
   taaattttgg tgaaatttcg tttttattaa aaatataaaa attagttagg tgtggtggtt   1440
   tacgtttgta gttttagtta tttaggaggt tgaagtagga gaattgtttt aatttagtgg   1500
   gtggaggttg tagtgagtcg agattgtgtt attgtatttt agtttgggtg gtagagcgag   1560
   attttgtttt aaataataat aataataata aaagaaaaaa gaaagtgttt tgtaggttgt   1620
   aagtgtatta tgggttatta tttagaggtt cgtttattat ggtttatttg aattgtgttt   1680
   tgtgttattt tttaattttt gttaatttta tttttttatt tagatcgata aagatttaga   1740
   attatttttt tgagtaaata tttagagtgt aatgatagag tgagaagtag gtggggggtt    1800
   gggcgtagtg gtttacgttt gtaattttag tattttggga ggtcgaggtt ggggagatta   1860
   cgaggttagg agtttaagat tagattgatt aagatggtaa aatttcgttt ttattaaaaa   1920
   tataaaaaaa ttagttaggc gcggtggtag gtatttgtaa ttttagttat tcgggaggtt   1980
   gaagtagaag aatcgtttga atttgggtag tagaggttat agtgagttaa gattacgtta   2040
   ttgtatttat tttaaaaaaa aaaaaaaaaa aaaaaaaaaa gagaggtagg tagggaaata   2100
   tgttgggtgt ttatggtaag atatgtttgt tagggaggtt ttttggagga ggtaaattat   2160
   aggttaggaa ttgaaggcgg gggataatta ttggaatgtt tttttttttt ttagaggatt   2220
   tgaggatttg gtttgtgggt tagtgtaggg tttttagggg ttttagttag gatatataaa   2280
   tgagatatat gattaagaaa gtaagtaggt ggtcgggtgc ggtggtttac gtttttaatt   2340
   ttaatatttt gggaggtcga ggtaggcgta ttacgaggtt aggagatcga gattatttta   2400
   atacggtgaa attttatttg tattaaaagt ataaaaaatt agttgggtat ggtggttggc   2460
   gttttagttt tagttattcg ggagggtgag gtaggataat ggcgtgaatt cgggaggcgg   2520
   agtttgtagt gagttgagat tacgttattg tattttagtt tgggtgatag agtgaaattt   2580
   tatttcgaga aaaaaaaaaa aagtaagtag gcgtagtggt ttacgttagt aattttaata   2640
   ttttgggagg ttaaggtagg tggattattt gaggttagga gttcgagatt agtttgattt   2700
   aatatggtaa aaatttattt ttattaaaaa tataaaaaaa ggttaggtgt ggtggtttac   2760
   gtttgtaatt ttagtatttt gggagtttaa ggtaggtgga ttacgaggtt aggagttcga   2820
   gattagtttg atttatatgg tgaaattttg tttttattaa aaatataaaa attagttagg   2880
   tgtggtggcg cgagtttgta attttagtta tttaggaggt tgaggcggga gaattatttg   2940
   aatttaggag gtagaggttg tagtgagtcg agattgtatt attgtatttt agtttgggtg   3000
   gtagagtgag attttgtttt aaaaaaaaaa agaaattagt taggtatggt ggtgtgggtt   3060
   tgtagtttta gttattaggg aggttaaggt ataagaatta tttgaatttg ggaggcggag   3120
   gttgtagtga gttgagattt tattattgta ttttagtttg gtagtttggg agatagagtg   3180
   agattttgtt ttaaaaaaaa aaaaaaaaaa aaaagcgagt aagggaacga gaggacggaa   3240
   ttcggaaagg tttagggtaa gataggttgt gattagagtg tttgggtatt ttgagtatta   3300
   ttaaagttta gagttgtttt taggttaggt ggggatattt taagattttg gaggagtgag   3360
   gtggtagtgg ggagaggtat attaggttag gagtaaagat tatagatgat agttaggcgt   3420
   ggcggtttat gtttataatt ttagtatttt gggaggttga aggatttcgg ataggggtag  3480
   gagagtcggg aatagaaggg gtattgacgg aataggtttg tttgtagagt aatttttagt   3540
   tttgtggtgc ggtgattgtg gagtaggggt tagttaggtg ggttaggttt tggttatatt   3600
   tttgttttga ttttagttta ttttttaatt ttttggggtt ttagtgtttt ttgtgaaatg    3660
   ggggtagttt tttatttgtt aggatcgtta tgataataaa tgagagtagg aaagtttttt   3720
   attagttttt tatagtttga ggtttttgag agttttagtt aggtgaatat ttagtttttt   3780
   ttgttgtata gatgaggaaa tagaggtaga ggttaagaat ttgtttgagg tcgtgtggtt   3840
   gggatagttt gtttagttat ttaaatacgt aggaaacggt agttgttttt ttttattagt   3900
   tttgagtggg gttataggat tattacgttt gtcggatata aggttttata ggtggaaggt   3960
   gagtgatttg tatttaggta tttggtttgg gtattgtttt ttgtttaggg attagaataa   4020
   gtagaagata ttgggtaggt atggtggatg gtcggaagtt ttttttgtgt atttatttat   4080
   ttgatttttg ttatatttta ttagggaggt tttttatggt tgttatttta tagaggaaga    4140
   aattaaggta gagagtatag gtaatttgtt taaggttatt gatttagtag gtagtagagt   4200
   tgggatttta atttaattaa tgtggtttta ggggttgtgt gtttagttgt tatgttttag   4260
   gtttttttta gttgtatttt gggttgtttt ggaggtttag gttggggaag gtttttttgg  4320
   gttttttgtt agtattagtt ttgttaggaa tttgttttat ggggtgggag ttttttatta 4380
   aattcgtgga tttagttaag ttagttgtta ttaagatgat atttatttgt gtaaattgaa   4440
   ttatatatat gtaaatataa atatatatat atttatgtgt gtgtgtgtgt atatatttgt   4500
   gtgtgtatgt atgtgtgtgt gtgtgtgtat atatatatat atgtattttt ttttttttag  4560
   agggagtttt gttttgttat ttaggttgga gtatagtgat gtgattttgg tttattgtaa   4620
   tttttgttta ttaggtttaa atgatttttt tgttttagtt ttttgggtag ttgggattat   4680
   aggtgttcgt tattatgttt agttaatttt tgtattttta gtagagacgg ggttttatta   4740
   tgttggtcgg gttggttttt aatttttgat tttaggtaat ttaagtgttt cggtttttta   4800
   aagtgttggg attataggcg tgagttacgg tgtttagttt taaattgaat ttttaaaaag   4860
   tgttttattt atagggaaaa attttggggg agtgtttgtg aattttttgg gggaaggaga   4920
   gggttggggt agggtttggg ttagattttt ttttttttgg ggaagggaag gggtgtggtt  4980
   tttttttggg tgtgaacgtg ggtgggtgtt taggtttgtt tatttttgtt attcgtttag   5040
   agtttggttc ggagttgggt gtggaagtgg agggtagtgg ttgtggttag aagtgtttta 5100
   ggttttagta tggtattttt agttttatat tttttgcgtg ttgggttttt gagttagcgg 5160
   ttggggcgga tatttttgtt gatgttttcg cgagtatttt tgtattggga tttttagagt 5220
   ttttagattt ttaagaagga gaatacgaag ttttttttgt tttatagatg aggaaataga 5280
   ggtatattta attataagat atttgtatat tgaattgtat atttaattaa aatagtggtg 5340
   ttttagatcg tttggagtag tgaagttttg ttattaggta agggttgggt ttggattttg 5400
   tttttattat atgtcgatta tatataattt gttgtcgggg gaggttgacg ttagtggtta 5460
   gtatagtggt tagtgttgtt attttatttt gggtagatat tcgtagtttg ttcgttttcg 5520
   gtatttgtag gttttagttt tgattatagt ttttcggtat agaagttttt ttaggtttgt 5580
   agagaatatt tttaattttg tttagagttt ataaaattag aaagtatttt aaagttggta 5640
   aatgtttttt ttttgttttt aaaaaatagc gatagggttt ttttttgtta tttagattgg 5700
   agtggggtgg tacgatttta gtttattgta gttttaaaat tttaggcgta atcgattttt 5760
   ttattttagt tttttgagta gtttggatta taggtattat tatgttcggt taatttttta 5820
   atttaaaaaa tttttagaga agtgttttgt tattttgttt aggttggttt cgaatttttg 5880
   agtttaagtg atttttttgt tttggttttt tagagtgttg ggattatagg tatgaaaggt 5940
   tggttggtaa atgtttgttt attttttttt tttttttttt aagtgagttg aaaatgtttt 6000
   ttataaaggg ggtttagtgg gttacgtggt ttttgtgata attatttatt tttgtggtta 6060
   gagttgaaag gaaattataa ttaatgtgta aatgaatata ttgtattttg ttgtgtttta 6120
   ttaatatttt ttttagggat tttgaaattt gaattttata gaatgtttac gttataaaat 6180
   atagtttttt ggatttttgt ttataattta aaaatatagg tttaatgtgg tggtttatgt 6240
   ttgtaatttt aatattttgg gaggttaagg taaaaggatt gtttgaggtt aggagttcga 6300
   gattagtttg agtaatatag aaagattttt gtttttataa aaattaaaat aaaaaaatta 6360
   gttaggtatg gtggcgtgcg tttgtagttt tagttattta ggaagttgag gtgggaggat 6420
   ggttggagtt tagaaatttg aggttttata aatatataaa gttaagaatg tatataaagg 6480
   taagaaaatt atttgaattt gggaggtaga ggttgttgcg aggcgagatt gtattattgt 6540
   atttagtttt gggcgataga gtgaggtttt attttggaaa aaaaaattat atatatatat 6600
   ttatatataa atatatatat atataaataa taataatggt agttttaata ataagagtcg 6660
   taaatattta gttttttttt tgcgttaggt attgttttta agtatttttt ttatattaat 6720
   ttattaggtt ttcgttatag ttttgcgagg tgggattagt tttattttta tttatagatg 6780
   aggaaattta ggtagatgga gttaattaat ttgtttaagt ttaatttgtt aggaagggta 6840
   gagttacgtt tgaattttgt agtttggttt aggggttagt attgggttgt ttttttttgt 6900
   ttttttattg tttatatttg ttttagatat tgggttgtgt gggttgttat agtataaggt 6960
   ggttgtagtt tttgttttgg ggatatttta gttatttagt aggggggttt ggtggtaggg 7020
   attatgagga gtgatcgaat taagtgtgag ttgttttgcg taggtagaga gggtagaggt 7080
   ttcgtggttt tggcggggta ttggtatttg ttgatttttt ttgggaaatt atttgttttt 7140
   ggagatattg aaaattaaat taaaaggtta agaaatttag ttttgttttt atttggtagt 7200
   tgtttttttt cgtagttttt ttttttacgg gtttgtagtt ggggttgggg ggaggtgtcg 7260
   tttttggttt gtgtagaatg ttatttttgt tttttgttat tttattagta ttttgttttt 7320
   tttttgtatt ttgttatttt ttttattatg attttgtttt attttttttt tagatttatt 7380
   tttagggata taatttaggt tttgttatta ttaaataatt ttattttaag ttttttatta 7440
   gttgatttaa tttgtgtgga ttatgttttc gggattttaa ttttaacgat gttgcggttt 7500
   tttagggttt gttttttttt ttgaagttta gttagtttgg attttatatt tattattttt 7560
   ttttttgtat atatttgaag tttttatttt taattggatt gagataagta taattttggt 7620
   ttagatttta agtttttgtt agtttttatt agtattttgt taaaaatttt attagatgtt 7680
   taaaagttat ttttagcggt tagacgcggt ggtttatgtt tgtaatttta gtattttggg 7740
   aggttgaggc gggtggatta cgaggttagg aattcgagat tagtttggtt aatatggtga 7800
   aattttcgtt tttattaaaa atataaaaat tagttgggta tggtgtatgt ttgtaatttt 7860
   agttattggg gaggtttagg taggagaatg gcgtgaattt aggaggtgga agttgtagtg 7920
   agtcgagatc gtgttattgt attttagttt gggtgataga gtaagatttt attttaaaaa 7980
   aaaaaaaaaa aaaaaatttt tttttatttt attttttagg gtagttttta gatttttttt 8040
   tattttttag tgtttttttt ttattaaaag taattttatt ttatcgggtg tttaagttag 8100
   attttttggt gttggttggg tattgtggtt tatgtttgta attttagtat tttgtgaggt 8160
   taaggtggga ggattatttg gggttaggag tttaagatta gtttggttaa tatagtaaga 8220
   ttttgttttt aaaattaata ataatttggt taggcgcggt ggtttacgtt tgtaatttta 8280
   gtattttggg aggttaaggc gggcggatta taaggttagg agatcgagat tattttggtt 8340
   aatatggtga aattttattt ttattaaaaa tatataaaaa aattagttgg gtatggtggc 8400
   gggaggttgt agttcgagtt attttggagg ttgaggtagg agaatggcgt gaattcggga 8460
   ggtagagttt gtagtgagtt gagatggtgt tattgtattt tagtttgggc gatagagtaa 8520
   gattttatgt taaaaataaa ataaaataat aataataata ataataataa tttttttttt 8580
   tgagacggag ttttgttttg tcgtttaggt tggagtgtag tggtattatt tcggtttatt 8640
   gtaagttttg tttttcgggt ttatgttatt tttttgtttt agtttttcga gtagttggga 8700
   ttataggtat ttattattac gttcggttaa ttttttgtat ttttagtaga gacggggttt 8760
   tatcgtgtta gttaggatgg ttttgatttt ttgatttcgt gattcgttcg tttcggtttt 8820
   ttaaagtgtt gggattatag gcgtgagtta tcgcgtttgg tttaatttat tttattttta 8880
   tttatttttt gttttttgag acggattttt gttttgttat ttaggttgga gtgtagtggt 8940
   aggattttag tttattgcga tttttatttt ttgggtttaa gtgatttttt tgttttagtt 9000
   ttttaggtag ttgggattat aggcgtttat tattatattt agttaatttg tgtatttgta 9060
   gtagagatag gattttatta agttggttag gttgtttttg aatttttgat tttaagtgat 9120
   ttgtttgttt tagtttttcg atgtgttggg attataggtg tgagttatta cgttcggtta 9180
   ataataattt tttttttgag acggaatttt gttttgttat ttaggttgga gtgtagtggt    9240
   gtaatttttg tttattgtaa gtttcgtttt tcgggtttac gttatttttt tgttttagtt    9300
   tttagagtag ttgggattat aggcgtttat tattacgttt agagaatttt ttgtattttt    9360
   agtggagacg gggtttatcg tgttagttag aatggttttt attttttgat tttatgattt    9420
   gtttatttcg aattattaaa gtgttgggat tataggcgtg agttatcgcg tttagtttaa    9480
   tagttttttt aaaatttttt ggtattattt tgattttttt ttttttttcg tattttatat    9540
   ttagtttatt agtagatttt gttagttttg ttttaaaaat aaatttaggg attagttatt    9600
   ttttattatt tttttggtga ttttttgaat gtttaaattt gattatgttg tttttgttta    9660
   aatttttttt aggtttttat ttaaaggtaa tgttagagtt tttagaatga tttttaattt    9720
   taggttttta aatatttttt tttaaatttt tttttttagg tttattttag ttaggagtag    9780
   gtgttgtttt cgaggtttta ttatttgttt ttttttttgt ttggaatttt ttttttatta    9840
   tttttgtggt tttggtttta gtggtgtttt ttattaaggt gagtgttgat tatttttgtt    9900
   attattattt ttttcgtttt tgtgatttcg gattttttta attttttttt ttgagatgag    9960
   ttttattttg tcgttaggtt ggagtgtagt ggtacgattt tggtttattg taattttcgt    10020
   ttttcgggtt taagcgattt ttttgtttta gtttttcgag tagttgggat tataggtgcg    10080
   cgttattatg tttagttaat ttttgtattt ttggtagaga tggggtttta ttatgttggt    10140
   taggatggtt tttatttttt tattttgtga tacgtttatt taggtttttt aaagtgttgg    10200
   aattataggc gtgagttatc gcgttcggtt ttttttaatt tttttatatg ttgtttttat    10260
   ataaattttt ttacgtatga taggatgttt ttgtgtttgt ttgtttatgg ttttattagt    10320
   ttattagaag gtaagtttta taagggtaag gatttttgac gtttgggttt atcgaggttt    10380
   tttagtaatt ggaatagttt gtgttatacg gtgagtattt attgtgtggc gcgagtgaat    10440
   gttgagtgtg tgtggggggt agagagtagg ggaagtttgt gagtagaaaa atggttaagt    10500
   gtaagtttag gaagttaaag aaagttgtgg aggttgagta tggtggttta cgttagtaat    10560
   tttaatagtt tgggaggtta aggtgagagg attgtttggg tttaggaatt cgagataaga    10620
   ttgggtaata aagtaagatt ttatttttat aaaaaataaa aaatgagttg gtgtggtggt    10680
   atgtatttgt ggttttagtt gttttggagg ttgaggtagg aggatcgttt gagtttagga    10740
   gtttgaggtt tgtttgggta ataaaaggag atttcgtttt tgtttttatt ttatgaaaat    10800
   gtgaaaaatg ttgggtatgg tggtttacgt ttgtaatttt aatattgttg ggaggttgag    10860
   gcgggtggat tacgaggtta ggaaatggag attattttgg ttaatacggt gaaattttat    10920
   ttttaataaa aatataagaa attagttcgg tatggtgata cgcgtttgta gttttagtta    10980
   ttttggaggt tgaggtagga gaattgtttg aatttaggag gtagaggttg tagtgagtcg    11040
   agattgtatt attgtatttt agtttgggta gtagagtgag atttcgtttt aggaaaaaaa    11100
   aaaaaaaaaa aagaaaaagt aaaaaataaa ttaattgggt gtggtttata tatttgtatt    11160
   tttagttacg tgggaggatt atttaagttt agaagtttcg ggttgtagtg agttgagatc    11220
   gtatttttgt attttttttt gggtgataga gcgagatttt gttttaaaat aaaataaaat    11280
   aaatagaaaa aaagaaagta agttatgggg tttaatttga gagaatcgag tgtgggtgtt    11340
   tattcgtaag agagcgaggg gaaagaggtt ttttaagttt ttttaatttt gaaaatgggg    11400
   aggaggtttt ttaagttgta gtgttagagt gatatgattt agttttttat ttttagagat    11460
   ggggtttggt tatgttgttt aggttggttt tgaatttttg ggtttaagtt agttttttga    11520
   gttgttggga ttatagatat gtgttatttt atttagtttg atttggtttt tgtgtttaga    11580
   agatatttgt tgttgtttta ggtgttaagg ttagtgttgg gtataggggt gttagagttt    11640
   attgagaaat ggatatagaa ttttagtgtt tattttttta ttttggagtt tgtgtttttt    11700
   attttatata gttatgttgt ttgggaggat tgtttgtttt ttgaggttat ttgtttttgg    11760
   ttattgtgtt aggatagttt tgttgtgggt acgagttata gggtaagaaa gaatatattt    11820
   gggagttgga aggtttaatt ttagggaaag tatgtagttt ggggtagggt attaaggagg    11880
   tttttagttt ttatatttta aggaaagaaa tattaggaag gtgtggtttt aggtaggttt    11940
   tttttttttt tcggattatt gtttgtttgt tattgatttt tgttttaatt ttatattaat    12000
   agttgttata tatatatata tgtattttaa gagttggagt ttttttaagt tatttaggtt    12060
   ggttttgaat ttttgggttt aagcgatttt tttgttttgg ttttttaaag tgttgagatt    12120
   ataggtgtga gttattacgt tcggtagtaa ttatttttta gttttaatgt gtattagttt    12180
   tgtaattaag tgtattttat atgtttttgt taattaattt aatagtttta taatggtggt    12240
   tattaattgt tgttttacga aagaggaaat tggggttttg agagtttggg gatcgattta    12300
   aagtatttag tgtttgtgga tgtaattggg atgtagggag ttagtgggtt gggggtgagg    12360
   tgtttcggta ggtggaggtg gtggggtatt tatttttatg atggttgttg ttggttggtt    12420
   gtttttgttg gtgattggcg ttttatagga ggtttggggt ttggcgatga tgtttgtcgg    12480
   agtttgaggg ttcgtttttt gcggggttgg ggtttaggtt tttttttgta gagttggagt    12540
   ttttttgttt attcggttta aattttggga tttggaatat gttttgggtt gtgaggataa    12600
   gatgttacgt agttaaggta tagttggggt taacggtggt tttggaaggt agaggtaggt    12660
   atttttggtt ttttttttta tcgtagcgtt tttaggttcg ttaggttttt tgggttttta    12720
   tttgtttgtc gggtttggtt tggtagggag ttgaggttgt cggtttagga ttttagtttt    12780
   tttttagaat ttcggtggat ttaggtttcg tttcgttcgt ggtgacggcg tataggtttt    12840
   attttaagtt cgatttcgag gtttttgatt cgggtttgtc gttttttttt agtatttcgg    12900
   gtttcgggtt ttagttgttt gggtaacggg aattgttagc gatttggttt aattattttt    12960
   cggaacgtta ttcgggtagg tttagaggcg gggttaattc ggttcggttt cgttttttcg    13020
   tcggatttaa ggtttttcgt tcgtagtttt tttttttttt ttttcggttt cggggtttat    13080
   attttcgttg ttttatattt tgggcgtgtg tgggagtagg agagggattt tttggggttt    13140
   ttatttggaa tttggagatt tatttttgtt aataagtttt ttagtttttt tgggttttag    13200
   ttttttttta gtgaagtggg gatagtgata gttttttcgg gagttagtga aaattttgtg    13260
   aagggagtga ttattgcggg cgaaggcgtt tggtatagtt ataattgaag tttagtcgtc    13320
   ggatgtgatt attgttgtta ttgttatcgt ttcgtttgga ttgggtgttc ggtcggaaag 13380
   tttcgttttt cgtttttttt tttaggcgcg gaagtttcgc gtcgttcgtt tcgggggata 13440
   tttttcgcgt tatttcgggg gtattggaga taaggaaagg aatttttttt ttggttggga 13500
   tggttgttcg ggcgcgggag gagggtcggg gattcggtga gggaggatcg tagggttcgt 13560
   gggggtttta cgtttggtag gcggtcggtt aggtgagtag ggtcgcgggc gcgtcggggc 13620
   gaggggcggg ttcgcgcggg ggattcgggg tttcgcgggt ttaggttcgg gggcggggtt 13680
   cgggggttat tttggttatg ggcggaagtt ttttttagag atcgagggac gggaattcgt 13740
   tgtcgttttc ggtcgggcgg tacgatttcg atttgggtat aggagagacg agcgtgggaa 13800
   ttggcggatt tttatataag taaaggcggg cggtacgggg tacggtttat tcggggattt 13860
   tttggatgcg gtacggtttt cgtaattcgg tcgttcgttt gtcgtttttc gtaatttcgg 13920
   gcgttcgtta gcggcgtagt tcgcgttaat tttcgtacgg ttttacgtcg ggtgcggcgt 13980
   tggattgcgg atttagtcgg gtcgttgcgt aatttgcgta gcgaacggcg ttcgcgtagg 14040
   cgcggacggg gaagggggcg gagtagcggg agtcggggag tcggagtttc gggtttttac 14100
   gatttgagtc ggttttttta ttagcggttt gaggatttgg cgttcgtttt tttttttttt 14160
   ttgaggttgg agcgtggacg cggtggggga gggtggggcg gggtagtttt tcgagaatta 14220
   cgaggatagg aggtagaggt tttttttatt cgtattattg gaggagagag agtttttttt 14280
   agtttgggga gggggagagc ggggtattgg gcgtttttcg tagcggtcgt tgttttgatt 14340
   cgacgggtat tagtcggttt ttttttttta tttaggacga tatgaacgat cgaggttagg 14400
   gagttttttt tttgggtttt tgtatttttt tatttttggt tttggggtag gtttagggag 14460
   gagatatttt taatttttat tcggtttgtt ttggagaaaa gagattgttt ttttatgttt 14520
   ttgagtgagg ggtttggggt ttaggttgtt tgtgtttttt aagggtaagg gtttttttgt 14580
   tgaggaggag gggtttgtta gttataattt tttttttttt gagcgtttta tttttttttt 14640
   tgtattttgt aatttttatt ttttcgtatt tatttttttg gtttcgtcga tagttttttt 14700
   ttgtttgttt tcgggattta ggtttttttt tttgatatgg agagtaattt gtttggtttg 14760
   gtgtttgttg tcgggttggt gtttgcgttg ttatttgttg tgattttggg gttgatagtt 14820
   gtttatatta ttttgtatgt tttgtttttt tttttcgttt atgtttagtt ttggttggtg 14880
   tttttgtatg ggtataagcg ttttagttat tagacggtgt tttggttttt ttgtttgttt 14940
   tgggtcgttt tgcgtattat tttttttttt ttttattttc gagatatttt tcgcgttaat 15000
   cgtttggggt ttttgttttt ttggtttttt tattgttgtt tcgtttgttt gtagtttttt 15060
   attttgacgt ttatgaattt ttattttgtt taggtaatta attgtggttt cgggtggggg 15120
   gcgggaggtg gtaagtttta aggatttata ggagtgaagg gtatttttgt ttttaggtgg 15180
   tgtttaaggt taaggtgaag cgtcggtcgg agatgagtcg aggtttgtaa gtattcggga 15240
   tattggtggg tttagttttt aggttagggg taggtgatag ttttatgtag attttttttt 15300
   attttttagg gttgagataa aggtaattta gggaatgttt gtggaagatt ttggaaaagt 15360
   taaagtattt ggcgtagatg gtttagtaga ggttttgtag atttgtaagg tataggaata 15420
   ggaatgtttg tggtagtgtg agggagaggg ttttagtgtt atttattatg gagggcgagt 15480
   tggaggtttt agtttatgga agtattaaag ttatatgtat ttttgggtga tttggggtcg 15540
   taatgttttc gttatttgta tagtgtttta cggtttataa agagtttttt tagttatttt 15600
   ttatttgtat aatttttttg agaggttatt attttttttt gagagatgaa gaaattgagg 15660
   tttagttagg taagatagta ggattaaggt cgtagagttg gggtttagat agagattttt 15720
   gattttagtt ggtgtatttt tcgaagtatt atggttgtta gttatttttt gatttttttg 15780
   ggttttagtt tttttatttg taagagtgga ggaatagggt ttagattgga tgttttgagt 15840
   tttgtcgata ttggggtttt ttgtaggagg tggggtaggt ggtgagtgtt gagggttgtt 15900
   gagggttcgc gttgattgtg ttgtggttgt aggttcgttg ttcgaggggt ttttgtgggg 15960
   gtttcgttgt tttttttgtt ggtgaacgtg ttgtgtgttg tgttttttta tcggcgtcgg 16020
   gtatagtttt gggttttgtt gtttgttcgc gttttggtga gcgatttttt gttcgttatt 16080
   tgcgcgttgt tttttgttgt ttgtttttgt ttcgtcgtta ggcgggcgtt ttttattagt 16140
   atttatttgg aggttaaggt agggttgtag tattgatgtt taggtgtttt ttgggttttt 16200
   cggtagcggt ttttagggtg tagaggaagt tgggagtttt ttttttttga gggttttttg 16260
   ttgttatttg tgttagggga ttagtgtgtg ttaggcggtc gcgatgggtg gcgttatggt 16320
   tttgttttat gttagtcggg tttgttataa tttgatagta ttggttttgg ttttttagag 16380
   tcggttggat attttcgatt acgattggta taatgtgttt gattaggtgg gtatacgtat 16440
   gtttgttatt tttttagtag tcgtggtatt ttggttttag ttggtttggg ttttgtttta 16500
   ttattttttt ggcgtgattg tagattggta ttagtttttg tttttttata ggcggatttg 16560
   gtgaatgatt tggggaataa aggttatttg gtatttggtt ttattttttt cgtgtgggag 16620
   ttattgttta ttattttgtt ggtgggtttt tttcgggtgt atcggttttt ataggatttg 16680
   gtaagggttt gttttttttt ttgtggggtt agtggagggg gcggatgttg taaattttgg 16740
   gttggagttt ggggttgggt ttgggaggtt agtgtaggat aggagtaagt atcgatgatg 16800
   gttttttttt ttagagtatt agttatattt ttaatgggta ggtttttgtt tttcggtttt 16860
   atttttttga tcgggttggg tattgtgaag atgagggttg tttttgggag tatagtcggg 16920
   gtgagagtat taggtaggag tcgtggtatt gttttagtat ttttgtttta ttcgtttatt 16980
   tcgttggttt tattaagtta tgggggatcg agatagtcgg gttttgtttt ttatttttgt 17040
   tatggttttt gtttttttat ttgtattagg tgaggttttt tgggttttta ttgttaaagt 17100
   ttttattttt tgtttagaga tgtattttgt tttgggtttc gaaggtttag gttatagggg 17160
   ttttgtttta agtttgtttt ttttttgtag tttttgtgat tgtggttttg gttattgttt 17220
   tgagattgat tttgtttttt tgttgtagta tgtcgggtag tttaggtttt gggagttggt 17280
   atggtgttat cgggcgtgag tcgggttggt atgggggtag ttagacgaag attatttttt 17340
   tgtttttttt ttaggtgtta ggattaggcg gttattatta tagtttttat tttattttat 17400
   agacgtgatt tttttttttt ttttatagaa tatttaggtt ttagtttttt ttattttagg 17460
   tttttgtgtt aagtttgttt gtcgtttttt gtttaggatt ttgggggtcg tggttatttt 17520
   tttttttggt cggttttttg ttgtttttgt tatagtgagt ttgtgtcgtt tttttaggat 17580
<210> 13
   <211> 6314
   <212> DNA
   <213> Artificial sequence
<220>
   <223> chemically treated genomic DNA (Homo sapiens)
<400> 13
   atgtatttat taaaattagt tttattaggt tatatacgtg aaaaagttgt agataaattt 60
   taatattaaa taataaaaaa tgtaatgggt tgttgttatt atttagagta taagagatgt 120
   ttataaatta atatatatta aattgattaa tgttttttaa aaagtttata agtgtttttt 180
   tatttcgaag tagaattaag tgtttttttg gggagagagg aaaaaatttt ttaaatgaaa 240
   attgataagt ttagcgagta tttaggataa taattaatag tttaagttgt attatttttt 300
   cggttaatgt atttaatgtg aagtttaagt ttattggaaa attgaaaaaa gtagtatagt 360
   aacgcggatt tagataatat tagtagtgaa aagaattaga gtagtgtggt agagtgggaa 420
   aaggataggg cgtggtatta ggaagtttga ttagttggtt ttagattttt atgttggttt 480
   tttttttttt tttttttttt tttttttttt tttgagatag agttttattt tgttatttag 540
   gttgtagtgt agtggtataa tttcggttta ttataatttt tgttttttag gtttaagcga 600
   tttttttgta tcggtttttt tagtagttgg gattatagga gtttgttatt atgtttggtt 660
   aatttttata tttttagtag agatggtgta ttattatgtt agttaggttg gttttaaatt 720
   tttgatttta gatgatttat ttatttttat tttttaaagt gttgggatta taggcgtgaa 780
   ttatggcgtt tagttaaaga tttttatgtt tgatatttaa attattttat gttatgttag 840
   tttttatatt tgtaaattga taaatttaat ttagatgatt attattttat ttgtagaatt 900
   attttgtttt taaaagatga gtttttattt tatattttgt ttgtaattat agaaggtttt 960
   aaataaaaat ataatgtttt aggtatttaa aatgaagatt aataataagt tagagtagaa 1020
   atttaatgtt tattgagggg gtatgtgttg ttttggggtt tagatatata gtagagaatg 1080
   agatttagaa agttattaat tttatgaatt tatatttatg ttggaggagg ataaataaat 1140
   aagaaaataa tttattgtga ttaggataat ggaataataa attagtaagg tagtatgatg 1200
   aagggagaga agtggttata gaaggaaggg tggtaaggga aagttttttt gaagaggtga 1260
   ttttgagttg atattgaaga atgaaaataa gttagttatt agcgtattta gtaaaatttt 1320
   ttttatttta tgtttaaagt attttttaga tgtagaatag aggaaaaata tagtgatttt 1380
   gttttgaagg aatttatatt tttttttgcg ttataaataa gtattataga aagtaaattt 1440
   taattatatg agattataga gagagaaatt gtatttttga aggaggtatt agagttggat 1500
   tttaaaaggt agataatttt agggtataag aagagtgata ggaagggggc ggggggggag 1560
   aaggaaattt gtaaattaaa gaggaggaaa aaagggttat atatttatgt ttagttaaaa 1620
   gttgagggtg tgaagggaaa gaatagtaga taaagttaga aaagtattgg gcgtaggttg 1680
   tatttggatt tgattttata gggaataaga aaatattaag tgatggagtt tgtttattga 1740
   gtttgtataa gtgggtatgt gtgagagggt ggggtgttga agatgtgagg taaagatgta 1800
   ggagttttat gagatattat aaaattattt attaagagtt gggaataaaa gtagtaagtt 1860
   tagggtgaag gttattagtt ttttgtgatt ttaaagtgtt attaggatat ttaatagaaa 1920
   tatacgtaga tagttggaaa tagtaagatt ggaaaattaa agatattaag attagagaaa 1980
   gtaattttgg ataaatttat aaagaggtga tataaaaaat taaagtatag gagatttatt 2040
   aaagatttaa agaagtaggt tatatgtaat ttaagtgatt atttattttg gagaatagta 2100
   agagttagag aagtaagaaa attattaagt attagttgtt agagaaatta agagaagaga 2160
   attaaagaat ttggtgggga gagtattata gtgttgtaaa ggggagagaa tatagaagaa 2220
   gattttagac gaataatttt gaagaatgta gtttgagtaa ttttaggata aaaattagat 2280
   tgtaggagta atgtaagtag tttattgaag gtgattaaga aatagataaa atgaaaatga 2340
   ttcgaggttt ggggataaag atatgaaaaa agggatgggt atatgaagag aacggagaaa 2400
   atatttgaga tggatagata gtaatatatg aggggaagaa cgtaggggtt ggggagatag 2460
   aggatataaa agaaaaaggt taataacgaa aaaaggtttt agaagtagtg agaggagata 2520
   gtaataatag atagataaaa agggaggaat tttttttatt taaattaaag gatgggtgaa 2580
   aatatagaaa tataaggagg aaattgtgtg ttagggttgg ggagtaaagg gtgtatatta 2640
   aatggtttta aagtttttat aaagtagggg gtttggaatt tataaagagg taggggttag 2700
   agggttaaag agtaaaattg atttttagta aaatggtagg atgtgttaga ggatatataa 2760
   ggagagatga gggtaaaaga gtattttcga gtaattaaaa tattttgtta tacgtaatag 2820
   gtttatatag atgttaatat atatttagtt taattatatt atattttttt tatatagtat 2880
   aataagtaat ttgtgtttaa aaaaatgatt tgtcggtcgg aggcggtggt ttacgtttgt 2940
   aattttagta ttttgggagg tcgaggtggg tggattattt gaggttagga gttggagatt 3000
   agattgttta gtatggcgaa atttcgtttt tattaaaaaa tgtaaaaatt agttgggcgt 3060
   gttggtaggc gtttgtaatt ttagttattt gggaggttga ggtataagaa ttgtttaaat 3120
   tcgggaggtg gaggttttag tgagtcgaga tcgcgttatt gtatattata gtttgggcga 3180
   tagagtaaga ttttgtttta aaaaaaaaaa aaaaaaaaga aaagaaaaga aaaaagattt 3240
   gttaattata aaatgatttt tttttttttt ttgagacgga gttttgtttt gtcgtttagg 3300
   ttggagtgta atggtgcgat ttcggtttat tgtaattttt ttttttcggg tttaagcgat 3360
   tttttcgttt tagtttttcg agtagttggg attataggta tcgttatagt gtttagttaa   3420
   ttgtaggaac ggggttttat tatgttggtt aggttggttt tgaattttcg attttaggcg   3480
   atttgttcgt ttcggttttt taaagtgttg ggattatagg cgtgagttat cgcgttaggt   3540
   tcgtaaaatg aaattttaat tatagatttt aaattgcgta tttatagagt aattgagacg   3600
   attgtaattt atttaaaatt atgtgtaaat tattttgaaa taagttaatg agaaagatta   3660
   tatgattaaa ataataaaaa atgttgtttg aggaataatg tatagacgga atttagaaaa   3720
   aaggaaagtt agaataattg tagacggttt ttttcgtttt tgtataggat tgagtttttt   3780
   tttcggataa aaattaggaa taagggaggt ttttttagta aaagtaaaag taaattgttt   3840
   taaaaaaaaa aaaaaggttg taagtgattt ggttttttta gaaaagttaa tattatgttt   3900
   aatagtggtt tttattataa taatttgaag gtgttaatga tttttatagt aaaataagtt   3960
   agtaaatggt tatatcgtag agcgtttata tttattgtta attaagttta attgtcgttg   4020
   ttttaatatt ttcgtttttt aatgaatttt ttttttaggt atttcgattt atttatcggt   4080
   aatacgttga tatttcgaaa tattgtaggt aggagtggac gggggagaag tttttttttt   4140
   tttcgggttt ttttcgtgtt cgggtttttg ttgtcgcgat ttacggaagt aagaatgagt   4200
   tttatcgttt ggggtttatt agttagaggg ttggcggagg ttgcgagggg tagaagtagt   4260
   agtaggttat tgggtgggta agcggtgtgt atcgtagttg tggaggatat cgacgcggaa   4320
   cgttcggatt gagtttagaa tatatcggtt tcgttcggga tttattttat aagttttttc   4380
   ggaagaggcg ttcgttcggg aggaagggtt taatttcggg ttttatcggg aggagataaa   4440
   ttatttattg ttaatgtttt cgatgaggtt ggcggtgtta ggtatatagt ttattttgaa   4500
   ttgaaataat gttgtaatgt atagcggcgg gaggttagcg cgtttaaaat tttttttttt   4560
   ttatcgtagt cgtcgtttcg gtgggattaa gttcggaatt tcgatcgaga ttagtatttt   4620
   tgtttcggtt tttagtcgtc gggggttgtc ggaagttttt ttatattatt tttatttatt   4680
   ttttgtaata cgtgtgggtt tttaggaaga ggcggggtag tcgtatcgtt gggggatatt   4740
   ttttttttgt ttttgttaag taggtaagag gaaattttat tggataaatg gcgaggaaac   4800
   gtatattttt ttttaaggaa tacggtgttt tttttcgttt tcgggttttc gagattttag   4860
   agttattgat tttcgttttt tagttttcgg ggttcggtag tagaaggggc gggttcgggt   4920
   ttgggattgg ttggcgtcgt tcgatttttt tcgttgtttt ttattcgtaa tcgttcgcgg   4980
   gcgtttgcgc gatgggtcgg tcgtggggag cggggcggga agcgttttag ggtagcggat   5040
   tttatgtcgg ttttgaggcg ttcgggttac ggttttagtg acggttcgtt ttacggtcgt   5100
   tattacgggt ttgggggtgg agatgtgtcg gtatatttat ttttattttt atattttttt   5160
   cgttttgaat ttttttagtt ttttattttt tggcgggtgc gcgggggcgg ttcggcggag   5220
   attatttggt tgggagaagg cggaggaggc gatggttatt atttttcggg aggcgtttgg   5280
   ttagagtttg gtcgagtcgg aggaagttat taggtaagtt ttgtattttt tgtttttgcg   5340
   gggaggtgag ggtttttggg gttggggttc gtaaaggatc gggttttata tttgtttttt   5400
   ttatgtggag gagggtgtgt tgcggggggt gttggagaga tcgagattaa gattagaggt   5460
   tggggggata tgtttgtgtt ttttttgttt tttttttttt gtttcgattt taaaaaatat   5520
   gtttattatt atttaaaaaa gaaagatttt gcgcggattt attaagttta gtagtaggtt   5580
   gtagattttt tttttgggag cggtgttggg taggggtggt acggatttat tgttttagtt   5640
   ttagtaattt attttgcgtg gttggtaggt cggttttttc ggggaaggga agtggttgta   5700
   gttttcgatg tcgtttaggg attttttcga ggggaaggaa tttttgcgag gaggagggat   5760
   ttgggtattt agttgtttgt ttttaatgtt ttgtttgaga tcgaattatg aataattagt   5820
   gttgtaaagt atttttacgc ggattgattg atttaatttt tacggcgatt gtaaggtaaa   5880
   atggtattat agcggttttt ttttttgttt tcgaataagg aatcggggtt ttgcgaggtt   5940
   aatttatttg tttaatatat ttggatttgt aatattttat ttttcgtttt tttgtcggat   6000
   atatattttt gttttgttaa tatagttttt tgggtagtgg gttgtcgttt tgcggtggta   6060
   gtatattttt ttgttcgttt aagaattatt tttatttgtt gaaaatgtaa tagtgaaatt   6120
   ttcgtatatt gttggtgggg atgtaaaatg gtggaaaata gtttggtagt tttttaaaat   6180
   attaagtatt agaattagtt tatgatttag taagtttatt tttggttata tatttaagaa   6240
   aattgataac gtatgtttat ataaagtttg ttcgtttatg tttatagtat tattatttat   6300
   aatcgttaaa aagt                                                      6314
<210> 14
   <211> 6314
   <212> DNA
   <213> Artificial Sequence
<220>
   <223> chemically treated genomic DNA (Homo sapiens)
<400> 14
   attttttggc gattatgaat aatgatgtta tgaatatggg cggataagtt ttatgtgaat       60
   atacgttatt agtttttttg ggtatatagt taggagtggg tttgttgggt tatagattaa     120
   ttttaatgtt taatgttttg aggaattatt aaattatttt ttattatttt atatttttat     180
   tagtaatgta cgagaatttt attattatat ttttaataaa tgaggataat ttttgaacga     240
   atagagaggt atgttgttat cgtagaacga taatttatta tttagaagat tgtattaata     300
   aagtagaagt atatgttcgg taggaaaacg gagaataaaa tgttgtaggt ttaagtgtat     360
   taggtaagtg agttaatttc gtagagtttc ggttttttat tcgaaaataa aaaaaagggt     420
   cgttgtaatg ttattttatt ttgtagtcgt cgtgaaaatt aaattagtta attcgcgtaa     480
   aggtatttta taatattgat tatttatgat tcgattttag gtaaggtatt aggggtaaat    540
   agttaaatgt ttagattttt ttttttcgta aaggtttttt tttttcggga gggtttttga    600
   gcggtatcgg gggttgtagt tatttttttt tttcgggaga gtcggtttgt tagttacgta    660
   ggataaattg ttagagttgg agtagtagat tcgtgttatt tttatttagt atcgttttta    720
   ggagagagat ttgtagtttg ttgttaagtt tagtaagttc gcgtaggatt tttttttttt    780
   aaatggtaat aaatatattt tttggggtcg aagtagaaaa ggaggggtaa agggaatata    840
   agtatgtttt tttaattttt gattttagtt tcggtttttt taatattttt cgtaatatat    900
   ttttttttat ataaggaaaa taagtatgaa attcgatttt ttacgaattt tagttttaag    960
   ggttttattt ttttcgtaag gatagagggt gtaaagttta tttggtggtt tttttcggtt   1020
   cgattaggtt ttggttaggc gtttttcgag ggatagtagt tatcgttttt ttcgtttttt   1080
   tttagttagg tggttttcgt cgggtcgttt tcgcgtattc gttaggaaat gggaggttgg   1140
   ggaggtttag ggcgaagagg atataagggt ggaggtgggt gtatcggtat atttttattt   1200
   ttaggttcgt agtagcggtc gtaggacgga tcgttattgg ggtcgtagtt cgagcgtttt   1260
   agggtcgata tgggattcgt tgttttgaag cgtttttcgt ttcgtttttt acggtcgatt   1320
   tatcgcgtag gcgttcgcgg gcgattgcga atggagagta gcgaaggggg tcggacgacg   1380
   ttagttaatt ttaggttcgg gttcgttttt tttgttgtcg aatttcgaaa gttgagggac   1440
   ggaggttagt gattttgggg tttcgggaat tcggagacga aggaagatat cgtgtttttt   1500
   aagagggagt atacgttttt tcgttattta tttaatggag ttttttttta tttatttaat   1560
   aggaataggg aaagggtgtt ttttaacggt acggttgttt cgtttttttt tgggggttta   1620
   tacgtgttat aggaagtggg taagggtaat atggaggagt tttcggtagt tttcggcggt   1680
   tgaaagtcgg ggtagaagtg ttggtttcgg tcgggatttc gggtttggtt ttatcgaggc   1740
   ggcgattgcg gtaggaggga agaggttttg gacgcgttgg tttttcgtcg ttgtgtattg   1800
   tagtattatt ttagtttaaa atgaattata tgtttggtat cgttagtttt atcgaggata   1860
   ttgatagtga gtagtttatt ttttttcggt ggagttcggg gttagatttt ttttttcggg   1920
   cgggcgtttt tttcgaagaa gtttgtagaa tgagtttcgg gcggggtcgg tgtgttttga   1980
   gtttaattcg ggcgtttcgc gtcgatgttt tttatagttg cggtatatat cgtttgttta   2040
   tttagtagtt tgttgttatt tttgtttttc gtagttttcg ttagtttttt agttggtgag   2100
   ttttaagcga tggaatttat ttttgttttc gtggatcgcg gtagtagagg ttcgaatacg   2160
   aaagaagttc ggggaggggg ggggtttttt tttcgtttat ttttgtttgt agtatttcga   2220
   agtattagcg tgttatcggt ggatggatcg gaatatttag agagaggatt tattaaaaag   2280
   cgaaaatatt aagatagcgg tagttggatt taattggtag taaatgtagg cgttttacgg   2340
   tgtggttatt tgttagttta ttttattgtg agggttatta gtatttttag gttattgtag   2400
   tagagattat tgttagatat ggtattgatt tttttggaaa gattaaatta tttgtaattt   2460
   tttttttttt tttaagataa tttatttttg tttttattaa gaagattttt tttatttttg   2520
   gtttttgttc ggaagaggag tttagtttta tataaaagcg aaaagaatcg tttgtagttg   2580
   ttttggtttt tttttttttt aaatttcgtt tgtgtattat tttttaagta atatttttta    2640
   ttgttttaat tatgtgattt tttttattaa tttgttttaa gatgatttat atatagtttt   2700
   aaataagttg taatcgtttt agttattttg tgaatacgta atttagaatt tatggttaaa   2760
   attttatttt acgggtttgg cgcggtggtt tacgtttgta attttagtat tttggggggt    2820
   cgaggcgggt agatcgtttg aggtcgggag tttaagatta gtttagttaa tatggtgaaa   2880
   tttcgttttt ataattagtt gggtattatg gcggtgtttg taattttagt tattcgggag   2940
   gttgaggcgg gagaatcgtt tgaattcggg aggagaaggt tgtagtgagt cgagatcgta   3000
   ttattgtatt ttagtttgag cgatagagta agatttcgtt ttaaaaaaaa aaaaaaaatt   3060
   attttatggt tgatagattt tttttttttt tttttttttt tttttttttt tttttgagat   3120
   agagttttgt tttgtcgttt aggttgtagt gtgtagtggc gcgatttcga tttattggaa   3180
   tttttatttt tcgggtttaa ataatttttg tgttttagtt ttttaagtaa ttgggattat    3240
   aggcgtttgt tagtacgttt agttaatttt tgtatttttt ggtagagacg aggtttcgtt   3300
   atgttgggta gtttggtttt taatttttga ttttaggtga tttatttatt tcggtttttt   3360
   agagtattgg gattataggc gtgagttatc gttttcggtc gatagattat ttttttaaat  3420
   ataaattatt tattgtgtta tgtaagggaa atgtagtgta attggattaa atatatatta   3480
   atatttatat gaatttgtta cgtgtaatag gatgttttag ttgttcgagg atgttttttt   3540
   gtttttattt ttttttatat attttttaat atattttgtt attttattaa aaattagttt  3600
   tgttttttgg ttttttaatt tttatttttt tgtagatttt agatttttta ttttatgaaa 3660
   attttgaagt tatttgatat gtattttttg ttttttaatt ttaatatata gttttttttt 3720
   tgtgtttttg tatttttatt tattttttaa tttgagtagg aaggattttt ttttttttgt 3780
   ttgtttgttg ttattgtttt tttttattgt ttttggaatt ttttttcgtt gttagttttt  3840
   tttttttata ttttttgttt ttttaatttt tgcgtttttt tttttatgtg ttattgttta 3900
   tttattttaa atattttttt cgtttttttt atatgtttat tttttttttt atatttttat 3960
   ttttaaattt cgagttattt ttattttgtt tgttttttga ttatttttaa tagattattt 4020
   atattatttt tgtaatttaa tttttatttt gaaattattt aagttgtatt ttttaaagtt  4080
   attcgtttaa ggtttttttt tgtgtttttt tttttttgta gtattgtgat atttttttta 4140
   ttagattttt taattttttt tttttgattt ttttaataat tggtatttgg tgattttttt  4200
   atttttttgg tttttattgt tttttaaaat agatggttat ttggattata tgtggtttat  4260
   ttttttaaat ttttagtaga ttttttgtat tttagttttt tatattattt ttttgtagat 4320
   ttatttaaaa ttgttttttt taattttgat gtttttggtt ttttagtttt attgttttta  4380
   attatttgcg tgtgttttta ttaagtgttt tgatagtatt ttagaattat aaagagttaa  4440
   taatttttat tttgagtttg ttgtttttat ttttaatttt tggtaaatag ttttgtgatg  4500
   ttttataaaa tttttatatt tttattttat atttttagta ttttattttt ttatatatat  4560
   ttatttatat aaatttagta aatagatttt attatttaat gtttttttgt tttttatgaa  4620
   gttaaattta gatgtagttt gcgtttagta tttttttgat tttatttatt gttttttttt 4680
   tttatatttt tagtttttag ttaaatatgg atgtatagtt tttttttttt tttttttaat 4740
   ttatagattt tttttttttt tttcgttttt tttttattat tttttttgtg ttttaaaatt 4800
   atttattttt taaggtttaa ttttgatatt ttttttagaa atatagtttt tttttttgta 4860
   gttttatata attaagattt gttttttgtg gtatttattt atagcgtaag gaagagtata 4920
   aattttttta gggtagagtt attgtgtttt ttttttgttt tgtatttaga aagtatttta 4980
   ggtatggaat gaaagaaatt ttgttgggtg cgttagtaat tggtttattt ttatttttta 5040
   gtgttagttt aaagttattt ttttagagag gttttttttt gttatttttt tttttgtaat 5100
   tatttttttt tttttattat attgttttgt tagtttatta ttttattgtt ttaattataa 5160
   taggttattt ttttatttat ttgttttttt ttaatatgaa tataaattta taaggttggt 5220
   gattttttaa gttttatttt ttattgtatg tttagatttt agaatagtat atgttttttt 5280
   agtaaatatt gaatttttgt tttagtttat tattagtttt tattttaaat gtttgaagta 5340
   ttgtgttttt atttaaagtt ttttatggtt ataaataaaa tgtgagatag aagtttattt 5400
   tttgaggata aaataatttt ataaataaga taatagttat ttagattaaa tttgttaatt 5460
   tatagatata aaaattgata tgatatgaga tggtttaagt gttaaatata agggtttttg 5520
   gttaggcgtt atggtttacg tttgtaattt tagtattttg ggaggtggag gtggatggat 5580
   tatttgaagt taggagtttg agattagttt agttaatatg gtgatatatt atttttatta 5640
   aaaatatgaa aattagttag gtatggtggt aaatttttgt agttttagtt attaaggagg 5700
   tcgatgtagg aggatcgttt gaatttggga ggtagaggtt gtagtaagtc gagattgtgt 5760
   tattgtattg tagtttgggt gatagagtga gattttgttt taaaaaaaaa aaaaaagaaa 5820
   aaaggaaaaa aaaaaaatta atataagggt ttagagttag ttagttaggt tttttggtgt 5880
   tacgttttgt ttttttttta ttttgttata ttgttttgat tttttttatt attgatgtta 5940
   tttaaattcg cgttgttgtg ttgttttttt tagtttttta atgaatttga attttatatt 6000
   aaatgtattg gtcgaggaag tggtatagtt taaattgtta attattattt taaatattcg 6060
   ttgaatttgt tagtttttat ttaaaagatt tttttttttt tttttagaaa agtatttggt 6120
   tttgtttcga gatggaagga tatttatagg ttttttaaga agtattgatt aatttggtat 6180
   gtattaattt atggatattt tttatatttt ggataataat agtagtttat tgtatttttt 6240
   gttatttaat attgaaattt atttgtaatt tttttacgtg tgtgatttag taggattgat 6300
   tttagtgagt atat 6314
<210> 15
   <211> 7558
   <212> DNA
   <213> Artificial Sequence
<220>
   <223> chemically treated genomic DNA (Homo sapiens)
<400> 15
   gagttgtggg tggggttgtt ttgttttttt tagtaaatgt ttattgtgtg ttatatatcg      60
   ttttgggtat tttataaaga gtattttagt ttatttttgt aatttttttt aaggatagtt     120
   attattatat ttttatttta aagacgagga aatagacgta gagaggttga gtaagttgtt     180
   atttatcgta cggtttagta tgttagaatt ggtatttgaa tttaggtagt ttgattttag     240
   tagtttttta aaatgtgttt cgtgttttgt taggttttat atttttaaga agttttatag     300
   gaagagggtt cgtttttggc gtttttttta tgtttatggt tattatattt atttttaagt     360
   agatattttt tttttttggt ggagggttta gggttgattt tacggggttt tgtggttttt     420
   tgtaggtatt ttttttttaa ttgtaggatt tggataaagt ggttgttaat tttaaagtat     480
   agttaggtga gggaggtagg tggggtttag agatgtgata gagatttttt aaattttttt     540
   taaggtttat ttataggttt gttggttttt tgtagttttt tttttttatt agttttttgt     600
   tttgtatttg gggtttttat tatagagggg gattatcgtt tttaaaagta gggtgatgag     660
   ggggtgggga atagagagag gtgggttttt gtttttatag gggatggttg ttaggtttgt     720
   agaaggagtt gggagtaata ggatttttcg tttttttggt tttggtatat ttttttttta     780
   ttttttagtt tgtttttttt tatatttggt attttttatt tataattttt tatttgtggg     840
   aggattttat tttttttttt tttttaaatt aaatttttat ttttagtttt ttttttatta     900
   gatgtttttg ttcgttggtt ttatttatat ttttttgttt ttttttatta ggtgttttgt     960
   tttagatttt tatttattta aggttttttt ttttagattt tttttagtat gggtttttta    1020
   ttttaggttt atgttttttt tttattttat gtttaggttt ttttttagta tttttgtttt    1080
   aggttttgtt aatttgggta ttttttaatt ttagtttttt atttgtttga ggtttttgtt    1140
   tttttagagg cgttttttgt tggttttttt tttttttttt taggtaggtt gtttaatatt    1200
   ttttggtttg ttatgggtag aattgatttt attttttcgt taggtttttt tgtttttgta    1260
   ggtgtaggtt attatacgtt ttatgtttag ttttaaggga ggttgttgtg atcgttttga    1320
   gaagtttttg ggtatggtta tagagaaggg gtggggcggt aaggtagatg gtttcggttt    1380
   tttttttttt atatgttttt tttggtttat tattagggaa atggtatttt ggtggcggaa    1440
   tatggaggag tagtttgaaa tttatgatta gggtttcgga gttggggttt ttattttgag    1500
   aaggttttta atttagattt tgtaatagtt attttaggat taattgttta agaataggaa    1560
   gagtttttaa attaaaattt ttagttggaa attttttaga attttgggaa gtaaagattg    1620
   gggaggatgg gaggttgggg gagattagtt ggaggtagag gggtaggggg tttgatgaag    1680
   tagggaagga ggtgtggggg gtaggtaggg attttggggg aaagggggtg gagatttgga    1740
   ggaaaagatg tgagagtttt aggaagtttg gtagggagta aatagggaga ggtagggagg 1800
   gaattgaatt ttgagggtag gggttattga ggattgtata tatttttgtt ttttttgggt 1860
   ttgttttgtt tttaagaata tttttagtag ttagatagta tttgtttttt tttttagtat 1920
   ttaggttttg tgtttaggtg gtagtataga ttttattaag tttatttatt tatttgtttt 1980
   tatttttatt tatttatttt tttgagatag agtttcgttt tgtcgtttag gttggagtgt 2040
   agtgggttat ttcggtttat tgtaagtttt gttttttggg tttacgttat ttttttgttt 2100
   tagtttttta agtagttgga attataggcg tttgttacgg cgtttagtta attttttgta 2160
   tttttagtag agatggggtt ttatcgtgtt agttaggatg gtttcgattt tttgatttcg 2220
   tgatttattc gtttcggttt tttaaagtgt tgggattata ggcgagagtt atcgggttcg 2280
   gttttgtttt tatttttatt tttaattttt tgagatggag tttagttttg tcgttaggta 2340
   ggagtgtagt ggtataattt tggtttattg taattttcgt tttttggttt taagcgattt 2400
   ttttgtttta gttttttgag tagttgggat tataggtatt cgttattacg ttcggttaat 2460
   ttttgtattt ttagtagaga tagggtttta ttatgttggt taggatggtt tcgatttttt 2520
   gattttgtga tttatttatt tcggtttttt aaagtgttgg gattataggt atgagttatc 2580
   gtgttcggtt ttatttttat tttatttttt tgagatgggg ttttattttt gttatttagg 2640
   ttggagtgta gtggtataat tttagtttat tgtaattttc gttttttggg tttagtaatt 2700
   tttttgtttt aattttttaa gtagttggga ttataggcgt acgttattat aatcggtttt 2760
   attttattta tttattatta ttattatttt ttgagatgga gtttcgtttt tgttgattag 2820
   gttggagtgt aatggcgtaa tcgtagttta ttataatttt gtttttcggg tttaagtaat 2880
   ttttttgttt tagtttttag agtagttggg attataggta tgcgttatta cgtttggtta 2940
   attttgtatt tttaataggg gtggagtttt tttacgttgg ttaggttggt ttcgaatttt 3000
   taattttagg tgattagtta gtttcggttt tttaaagtgt tgggattata ggcgtgagtt 3060
   atcgtgttcg attaattttt tttttttttt tttgagacgg agttttattt tgttatttag 3120
   gttggagtgt agtggcgtga tttcggttta ttgtaagttt tgttttttga gtttacgtta 3180
   tttttttgtt ttagtttttt atgtagttgg gattataggc gtttgttatt atgttcggtt 3240
   aatttttttg tatttttagt agagacgggg ttttattatg ttagttagga tggttttaat 3300
   tttttgattt taggtgattt atttgttttg gttttttttt tgtgtatttt taaaagagac 3360
   ggggtttcgt tatgttaggt aagttggttt tgaatttttg attttaggtg atttgtttgt 3420
   ttcgggtttt taaagtgttg ggatgggatt ataggcgtga gttatcgtgt tcggtttttt 3480
   tttttttttt tttttttttt ttagagatag attttttttt tgttgttttg gtgtaattat 3540
   agtttattgt agtttcgatt ttttgggttt aagtaatttt tttattttag tttttttagt 3600
   agttaagatt ataggtatat aatattatgt tttgttaatt tttttttttt tttttttttt 3660
   ttttggtata agtagggtat tgttgttgtt taggttggtt tggaattttt gtatttggtt 3720
   ttaagcgatt tttttgtttt ggttttttaa atggttggga tgggatttta ggcgtgatat 3780
   atcgtagttg gttgtttttt ttgttgttgt tgagataagg ttttgttttg ttgtttaggg 3840
   cggaatgtag tggtgtaaat atggtttatt gtggtttcga ttttttgtgt ttaggtgatt 3900
   tttttgtttt agttttttag gtagttggga ttattaaatg tataggtgtg tattattata 3960
   tttagttaat ttttaatttt tttttgtaga gatatggttt tattttgttg tttaggttgg 4020
   ttttgaattt ttgggtttaa gtaatttttt tattttagtt ttttaaagtg ttgggattat 4080
   aggcgtgagt tattgggttt agtttttatt gagttttaat tttcgtttat ttgattatta 4140
   tttttaggat tttaaatatt tagagattat taaggtgtat ggtgtatagg tttaaattgt 4200
   aggttgaatt ttaatttagt attagtattt tttaatgcga ttatagaatt gattattatt 4260
   atttattttt tttgagatgg agttttgtat tgttattagg gttggagtgt aatggcgtaa 4320
   ttttggttta ttgtaatttt tattttttag gtttaaggga tttttttgtt ttagtttttt 4380
   aattagttgg gattataggc gttcgttatt atatttagtt aattttttgt atttttagta 4440
   gagacggggt tttattatgt tagttagaat ggtttcgatt ttttgatttc gtgatttgtt 4500
   tgttttagtt ttttaaggtg ttgggattat aggcgtgagt tatcgcgttt ggtttagaat 4560
   tgatgattaa tttagttgag tttttgttta tttgaatggg tattgttaat agtatttatt 4620
   tataagagtt gttgagaaga tttaatgaga taaatagttg ttaaagtgtt aggtatgtag 4680
   tagtgtttaa gaaatttttt attttgggtt ttttattggt attgattgat gtagaggtgg 4740
   gggagaagat taaagataag gattgagaat tagggatggg aaaagtagtg ggttattgat 4800
   agtcgttttg tttgtttggg aggtggggtg gggaaagtgg gcgggatatg tttttgggtt 4860
   tggtttattt agattatttt ataggttgtc ggtttgtgcg tttgtattcg gtggttatag 4920
   agtaattttt tttagaggga gttgattgga gtcgggtgtc gttggtattt ttatgattat 4980
   tggagtttcg cgggtttttc gggttgtata gggataagta aaggttatat ttagatgtcg 5040
   ggaatgtatt gacgtttatt tttggaaatt gggtttttat ttagtttttg ggagtagtag 5100
   tcgttagttt ttcgggattt ttatttttat tttgttgagt tattcgggtt gggttaggat 5160
   ttcggtaggt aagttggaag ggttttgttt atttttttag attttagtag ttttagtttt 5220
   agggtggggt agggagtttg tcgggagtcg ggtggggaag gggaagttta aggttttttt 5280
   gggtaggttt gtcgtttcgg ttggggattt gattttgtta tgtttgtttt tggttgtttt 5340
   ttatagtttt tttttttggt ttagttttgg atgtcggaga attgtaagaa ttgggttttt 5400
   taatagtttg ggagatggga gtggaggtta gagttaaggt taagggtaga gagagaattt 5460
   ttttagcgtt tgttgttgtt taatattttt agattgggtt tagggtttgg ttaggtatgt 5520
   atttttgggt aatatttatc ggggtttagt aagtttagga agtcgggggt ggtttttttt 5580
   atcgggaatt taggttattt ggatggggga ggtagagtta ggtttgagtt agtataggtt 5640
   gttggttttg gtgggtgttt tgaggtttta tttgtttttt tcggaagttt ggggtgttgg 5700
   tagagggagt tggaggtgta gttagtattt tttagtttta ttgttttggg ggtgtcgggt 5760
   tttggagatt ggggaagaag gaaggttatt ttatgtaagg agttacgggg ggtgggaggt 5820
   aagtaaaatt tttttttttt gttttttgaa atggagtttc gttttgttgt ttaggttgga 5880
   gtgtagtggc gtaatttcgg ttgatcgtaa ttttcgtttt taaggtttaa gcgatttttt     5940
   tgttttagtt tttcgagtag ttgggattat aggcgtacgt tattatgttt agttaatttt     6000
   tgtattttta gtagagatgg ggttttatta tgttggttaa gttggttttg aatttttgat     6060
   ttcgtgggtt attatgttcg gttgtaaagt tttgttttaa taaggttttg tttttagagg     6120
   tggaggagag gaaggtttgt ttttattttg tttttgttcg gtagatcgag gaggagtggg     6180
   gagttgggtg agggtatagg tggtttagtt tttttttatt tttttttgag agtttttggg     6240
   gtattttcga aaatattttt aggtttagcg gaaggagggt gggcgaaggt ttttaggttt     6300
   tgggcggtgg gggtggggtt gtgtgtttgg tttagggtgg ggttgtatat tttttttttt     6360
   tgggatagga ggagggcgtt ttttttttga gggttggagg ttgtttgggg aaatggggtt     6420
   ttgggagggg tgtaaattga aagtgaaata gttgatattt aggaaatatt cgttttgatg     6480
   aggggttata gtaggttggg gttgcggtta ggattaggta aagaggaaaa ttggggtcgg     6540
   ggatagaaga ttaggtgtgt ggtgggagta cgaggtaggt tatggggttt taaagaaggt     6600
   tttgattttg agggtttttt tcggttattg taaggtagta gtggggaagg tatagatagg     6660
   aggtaaatag ggaaagttgt tttgagaaag aatatatttt gaggtttata aattggaaaa     6720
   gaaattttgt atgcgtgttg agtatatgga tttacggaga tttagataaa taatttgatt     6780
   tttcgtgatt aacgatgtga tttcgggtat ttaatttttt gtgttttatt ttttttgttt      6840
   gtaaagtggg tatgatggcg tttattttgt tgggtttatg tgagttttta gtgtttatta     6900
   tttatagagt gtttttaagt gggagagtat attttaggtt tttaggaaat gtttgcggtt     6960
   aatagtttag agttaaaaaa taggtgtgtt ttggttagtt agagggaagt agggtttttg      7020
   aggatagttt ttatgggtta ttggttgggt agtggttcgt ttgtaataag tatgtgttgg     7080
   gtgggttgta ggaggtttta ggaatagtta aaaatttttt aggtttttgt tttaggagtg     7140
   gtatgaattt gagagttagc gggtattgtt gtagttatat ttttttcgat ggtgtagata     7200
   ttttagtttg tttttggttg ttttattttt ttattttgtt ttttttaaag agggagtgtt     7260
   tagtaagttg ttttttttta gtagatttta ttgggattta tgttggagta agaataaaaa     7320
   gttttagagg aggttaggta cggtggttta tatttgtaat tttagtattg tggatggtcg     7380
   aggtagattt acgaggttag gagtttgaga tcggtttggt taaagtttta gaggattaag     7440
   ggtttttttg ggaatggggg attttttttt ttatgtggat atggtaattt gtatggggtt     7500
   tttagttata ggtttgtgtt tatttttatt tttgtttttt ttcgtttttt ttcgtagg       7558
<210> 16
   <211> 7558
   <212> DNA
   <213> Artificial Sequence
<220>
   <223> chemically treated genomic DNA (Homo sapiens)
<400> 16
   tttgcgggga agggcgagaa aaagtagaga tgggggtgag tatagatttg tgattgggga      60
   ttttatatag gttgttatgt ttatatagga gagaggattt tttattttta gaaaggtttt     120
   tagttttttg ggattttggt taggtcggtt ttaaattttt gatttcgtga gtttgtttcg    180
   gttatttata gtgttgggat tataggtgtg agttatcgtg tttggttttt tttgggattt     240
   tttattttta ttttagtatg ggttttagtg aagtttgttg ggaggaaata atttattgaa     300
   tatttttttt ttgagggaga tagggtaaga aggtgaggta gttaagggta gattgaggta    360
   tttgtattat cgaggagggt gtggttgtag tagtgttcgt tggtttttaa gtttatgtta     420
   tttttggggt aagagtttgg ggggttttta gttgtttttg gggttttttg tagtttattt    480
   agtatatgtt tattgtaagc gagttattgt ttagttaatg gtttatgaag gttgttttta     540
   gaggttttat ttttttttgg ttggttagaa tatatttgtt ttttaatttt gggttgttag     600
   tcgtaaatat tttttgagag tttaagatat atttttttat ttaggagtat tttgtgggtg     660
   gtgaatattg gaaatttata tgaatttagt agggtgagcg ttattatatt tattttatag     720
   gtaggaaaag tgaggtataa agagttgagt gttcgaggtt atatcgttag ttacggaaag     780
   ttaggttgtt tgtttgaatt ttcgtgagtt tatgtattta atacgtatgt aagatttttt     840
   ttttagtttg tggattttag agtgtgtttt tttttagggt aatttttttt atttattttt     900
   tgtttgtgtt ttttttattg ttgttttgta gtgatcggag ggagttttta ggattagggt     960
   tttttttgaa gttttataat ttgtttcgta tttttattat atatttggtt ttttgttttc   1020
   ggttttaatt tttttttttg tttggttttg atcgtagttt taatttgttg tgatttttta   1080
   ttagggcgag tgttttttgg atgttagttg ttttattttt agtttgtatt ttttttagag    1140
   ttttattttt ttaggtagtt tttagttttt agaaggagag cgtttttttt ttattttaga    1200
   gggagggggt gtgtagtttt attttgggtt aagtatatag ttttattttt atcgtttagg    1260
   gtttggggat tttcgtttat ttttttttcg ttaggtttgg gagtgttttc gaaagtgttt   1320
   taagagtttt taagaagggg tggaggaggg ttggattatt tgtgttttta tttagttttt    1380
   tatttttttt cgatttgtcg gatagggata gggtgaaggt agattttttt tttttttatt   1440
   tttaggggta aggttttgtt aaaatagaat tttgtagtcg ggtatagtgg tttacgaggt    1500
   tagaagttta agattagttt ggttaatata atgaaatttt atttttatta aaaatataaa    1560
   aattagttgg gtatggtggc gtgcgtttgt agttttagtt attcgggagg ttgaggtagg    1620
   agaatcgttt gaattttgga ggcggaggtt gcggttagtc gagattgcgt tattgtattt    1680
   tagtttgggt aatagagcga gattttattt taaaaaataa aaaaaaagag ttttgtttgt    1740
   tttttatttt tcgtagtttt ttatataaga tggttttttt ttttttttag tttttaggat   1800
   tcggtatttt taggatagta gggttggagg atgttgattg tatttttaat tttttttatt   1860
   aatattttag gttttcgagg ggagtaggta gagttttaga atatttatta aggttagtaa   1920
   tttatgttga tttaggttta gttttgtttt ttttatttaa gtggtttaaa ttttcggtga   1980
   ggggagttat tttcgatttt ttgggtttgt tgggtttcga tgaatgttgt ttagggatat   2040
   atgtttggtt aggttttgga tttagtttag ggatgttggg tagtagtaag cgttgagaaa   2100
   gttttttttt tgtttttgat tttggttttg atttttattt ttatttttta gattgttaaa   2160
   ggatttagtt tttatagttt ttcggtattt agggttgggt taagagggga agttgtgagg   2220
   ggtagttaga ggtaggtatg gtaggattag gtttttagtc ggggcggtag atttgtttag   2280
   ggaagttttg agtttttttt tttttattcg gttttcggta ggttttttgt tttattttgg   2340
   ggttggggtt gttgagattt gggaggatgg ataagatttt tttagtttat ttgtcgggat   2400
   tttggtttaa ttcgggtggt ttagtagggt ggagatggag gtttcgaggg gttggcggtt   2460
   gttgttttta ggggttgagt gggagtttag tttttaggaa tgggcgttag tgtattttcg   2520
   gtatttggat gtagttttta tttgtttttg tgtagttcga gggattcgcg agattttagt   2580
   gattatagag gtgttagcgg tattcggttt taattagttt tttttagagg aagttgtttt   2640
   gtggttatcg gatgtagacg tataggtcga tagtttgtag ggtgatttgg gtgggttagg   2700
   tttaggagta tgtttcgttt atttttttta ttttattttt taggtaggta gggcggttgt   2760
   tagtggttta ttgttttttt tatttttgat ttttaatttt tgtttttgat tttttttttt   2820
   atttttatat taattaatat taataaaaaa tttagggtga gaagtttttt aagtattgtt   2880
   gtatgtttgg tattttagta attatttgtt ttattggatt tttttagtaa tttttgtaag   2940
   tgagtgttgt tgataatatt tatttagatg aatagaggtt tagttgggtt aattattagt   3000
   tttgggttag gcgcggtggt ttacgtttat aattttagta ttttgggagg ttgaggtagg   3060
   tagattacga ggttaagaga tcgagattat tttggttaat atggtgaaat ttcgttttta   3120
   ttaaaaatat aaaaaattag ttgggtgtgg tggcgggcgt ttgtaatttt agttagttgg   3180
   aaggttgagg taggagaatt ttttgaattt gggaggtgga ggttgtagta aattaggatt   3240
   gcgttattgt attttagttt tggtgatagt gtaagatttt attttaaaaa aaataaatag   3300
   taataattag ttttgtagtc gtattgggga atattaatat tagattgaga tttaatttgt   3360
   aatttagatt tgtgtattat gtattttggt ggtttttgga tgtttgaaat tttgaaggtg   3420
   atagttaagt aaacggagat taaaatttaa tagaggttgg gtttagtggt ttacgtttgt   3480
   aattttaata ttttggaagg ttgaggtggg aggattgttt gagtttagga gtttaagatt   3540
   agtttgggta ataaagtgag attatgtttt tataaaaaaa aattagaaat tagttgggta   3600
   tggtagtgta tatttgtgta tttggtggtt ttagttattt aggaggttga ggtaggagga   3660
   ttatttgagt ataggaagtc gaggttatag tgagttatgt ttgtattatt gtatttcgtt   3720
   ttgggtaata gagtaagatt ttgttttaat aataataaaa aaggtagtta gttgcggtgt   3780
   gttacgttta gaattttatt ttagttattt gggaggttaa ggtaagagga tcgtttgagg   3840
   ttaggtgtag gagttttagg ttagtttggg taatagtaat attttgttta tattaaaaaa   3900
   aaaaaaaaaa aaaaaaaaaa ttagtaaggt atggtgttgt atgtttgtgg ttttagttat   3960
   tggggaggtt gaggtgggag gattgtttga gtttaggagg tcgaggttgt agtaagttat   4020
   gattgtatta gggtaataga gggagagttt gtttttagaa aaaaaaaaaa aaaaaaaaaa   4080
   aaggtcgggt acggtggttt acgtttgtaa ttttatttta gtattttggg agttcgaggt   4140
   aggtagatta tttgaggtta ggagtttaag attagtttgt ttaatatggc gaaatttcgt   4200
   tttttttaaa aatatataaa agggaggtta aggtaggtgg attatttgag gttaggagat   4260
   tgagattatt ttggttaata tggtgaaatt tcgtttttat taaaaatata aaaaaattag   4320
   tcgggtatgg tggtaggcgt ttgtagtttt agttatatgg gaggttgagg taggagaatg   4380
   gcgtgaattt aggaggtaga gtttgtagtg agtcgagatt acgttattgt attttagttt   4440
   gggtgataga atgagatttc gttttaaaaa aaaaaaaaaa aaattggtcg ggtacggtgg   4500
   tttacgtttg taattttagt attttgggag gtcgaggttg gttgattatt tgaggttgga   4560
   agttcgagat tagtttgatt aacgtggaga aattttattt ttattaaaaa tataaaatta   4620
   gttaggcgtg gtggcgtatg tttgtaattt tagttatttt ggaggttgag gtaggagaat   4680
   tgtttgaatt cgggaggtag agttgtggta agttgcgatt gcgttattgt attttagttt   4740
   ggttaataag agcgaaattt tattttaaaa aataataata ataataaata aataaaataa   4800
   agtcggttgt ggtggcgtgc gtttataatt ttagttattt gggaggttga ggtaggagaa   4860
   ttgttgaatt taggaggcgg aggttgtagt gagttgagat tgtattattg tattttagtt   4920
   tgggtgataa gagtgaaatt ttattttaaa aaaataaaat aaaaataggg tcgggtacgg   4980
   tggtttatgt ttgtaatttt agtattttgg gaagtcgagg taggtggatt ataaggttaa   5040
   gaggtcgaga ttattttggt taatatggtg aaattttgtt tttattaaaa atataaaaat   5100
   tagtcgggcg tgatggcggg tatttgtagt tttagttatt taggaggttg aggtaggaga   5160
   atcgtttgaa gttaggaggc ggaggttgta gtgagttaag attgtgttat tgtatttttg    5220
   tttggcgata gagttagatt ttattttaag aaattaaaaa taaaaataaa aataaggtcg   5280
   ggttcggtgg ttttcgtttg taattttagt attttgggag gtcgaggcgg gtggattacg   5340
   aggttaggag atcgagatta ttttggttaa tacggtgaaa ttttattttt attaaaaata   5400
   taaaaaatta gttgggcgtc gtagtaagcg tttatagttt tagttgtttg ggaggttgag   5460
   gtaggagaat ggcgtgaatt taggaggtag agtttgtagt gagtcgagat agtttattgt   5520
   attttagttt gggcgataga gcgagatttt gttttaaaaa aataaataaa taaaaataaa   5580
   aataaataaa taaatagatt tagtggggtt tgtgttatta tttggatata aaatttggat   5640
   gttaagaggg aagatagata ttgtttggtt gttgggagtg tttttgagga tagaataaat   5700
   ttaggaagga tagaggtgtg tgtagttttt aatagttttt gtttttaggg tttagttttt   5760
   tttttgtttt tttttgtttg ttttttgtta agttttttgg ggtttttata tttttttttt   5820
   taagttttta tttttttttt tttaaaattt ttgtttgttt tttatatttt tttttttgtt    5880
   ttattaaatt tttgtttttt ttatttttaa ttaatttttt ttaatttttt atttttttta     5940
   gtttttgttt tttagaattt tgggaagttt ttaattggag gttttaattt gaaaattttt    6000
   tttgttttta agtagttggt tttggggtgg ttgttatagg atttgaattg gggatttttt    6060
   taggatggag gttttagttt cgaagttttg attatgaatt ttaggttgtt tttttatgtt    6120
   tcgttattag ggtgttattt ttttgatgat gagttaagag ggatatgtga ggaggagggg    6180
   gtcgggatta tttgttttgt cgttttattt tttttttgtg gttatattta ggggtttttt    6240
   aaaacggtta tagtagtttt ttttaaagtt gggtatgggg cgtatggtga tttatatttg    6300
   taagagtaag aaggtttggc ggagaggtga agttagtttt gtttatagta agttaggagg    6360
   tgttgggtaa tttgtttgag aaggagggga aggagttagt aggggacgtt tttgggaaaa    6420
   taggggtttt agataggtga ggggttggaa ttggggggta tttaggttgg taaggtttgg    6480
   gatagaggta ttgagaagag gtttggatat gagatggggg aagggtatgg gtttgagatg    6540
   ggggatttat gttggggagg gtttggggag ggaggttttg ggtgggtaaa ggtttggaat    6600
   agggtatttg gtggggaagg atagggagat gtgggtgggg ttaacggata gaagtatttg    6660
   gtggggagga ggttggagat ggggatttgg tttgggaagg agggagagat ggggtttttt    6720
   tataagtgag gggttgtgga tgggggatat tagatatgaa agaggatagg ttagggagta    6780
   gaaagggaat atgttagggt tagagggacg gggggttttg ttgtttttag ttttttttat    6840
   aggtttggta gttatttttt gtgaggatag ggatttattt ttttttgttt tttatttttt    6900
   tattatttta tttttgagag cgataatttt tttttatgat gaggatttta gatgtagagt    6960
   agggggttga tgggagagga aggttgtaga gggttagtag atttgtaggt gagttttggg    7020
   ggaggtttag ggggtttttg ttatattttt gagttttatt tgtttttttt atttgattgt    7080
   gttttgggat tagtagttat tttatttaga ttttgtagtt gaaggaagaa tatttgtaga    7140
   gagttatagg gtttcgtgag gttagttttg agttttttat tagggagaag gagtgtttgt    7200
   ttgaaggtga gtgtggtggt tataggtata ggggaagcgt tagaaacgga tttttttttt    7260
   gtagaatttt ttaggggtgt ggggtttggt aggatacggg atatattttg agggattgtt    7320
   ggagttagat tgtttgggtt taaatgttag ttttaatatg ttgagtcgtg cgatgggtgg    7380
   tagtttgttt aatttttttg cgtttgtttt ttcgttttta aagtgggaat ataatagtag    7440
   ttatttttga aggaagttgt aaggatgaat tgagatattt tttgtaaaat gtttagaacg    7500
   gtgtgtggta tatagtaggt atttgttaaa gaaaataaga taattttatt tataattt      7558
<210> 17
   <211> 5518
   <212> DNA
   <213> Artificial Sequence
<220>
   <223> chemically treated genomic DNA (Homo sapiens)
<400> 17
   gtataaatta tatttttaag agtggggaga atattgtaaa gtattttttt aagtggggtg      60
   gaggggagaa tatattatat tagttagagt ggggtaggaa taaattatta tggtggaatg     120
   ttattagtta aggttatttt tatttttttt gtggattttt agttgttttc ggttatttgg     180
   atgtgtacgt gtaggttata ggggatatga cggtttagtt tgggtttaga ggtttgatat     240
   ttattatatg gtagttggtt ttttttaaag gaagtgattt gaaagggaat aaggtggaag     300
   ttgtagtggt ttttttgatt tgttttgtga aattatattt tgttattttt ataatatttt     360
   tttggttata ttgtttagtt ttattgagtg tgggtagaga ttatatagta gtatgaatat     420
   ttggaggtgt gattttttag gtgttatttt ggatgtgggt ttaatagttt tgatttagta     480
   tttttattat tataaagagt ttggttgggt gcggtggttt acgtttgtaa ttttagtatt     540
   ttgggaggtt aaggcgggcg gattatgagg gtaagagatc gagattattt tggttaatat     600
   ggtaaaagtt tatttttaat aaaaatatta aaattagtag ggcgttgtta tacgtttata     660
   gttttagtta tttaggaggt tgaggtagga gaattatttg aattcggagg cggaggttgt     720
   agtgaattga gattatatta ttgtatttta gtttggcgat agagtaagat ttcgttttaa     780
   aaaataaaat aaagtaaaat aaaataaaat atataaaaaa gagttttagt taggtatagt     840
   gacgtatgtt tgtagtttta gtattgtggg aaattgaggt aggtggttta tttgagttta     900
   ggggtttgag attagtttgg gtaatatggt aaaatttttt ttttataaaa tatataaaat     960
   tagtagtgta tagtggtata tacgtgtagt tttagttatt ttagaagttg aggtgggagg    1020
   attttttgag tttaggaggt cgaggtggta gtgagttata gttgtgttat tgtattttag    1080
   tttggacgat agagggagat tttgttttaa aataaataaa taaaaataaa aataaatagt    1140
   tttgatttta taattagtta aaaagtgtat ttttttattt agtgattgta ttgtttagag    1200
   gtgtatattt gtattaaaag tttttttttt ttttttttgt aattggagta gggaagggtt    1260
   attttttgtt atgattggaa gttttttgag gtttttttag aagtagaagt ttgtgttata    1320
   tttgttgtat atagtttgta gaattatgag ttaattaaat tttatttttt ttttttttga    1380
   gatgaagttt cgttttgtta ttcgaggtgg agtgtaatgg tacgattttt gtttattgta    1440
   gtttttgttt tttgggttta agtgattttt tggttttagt tttttaagta gttgggatta    1500
   taggagtata ttagtacgtt tagttaattt ttgtattttt agtagagata gggttttatt    1560
   atgttggtta ggttggtttt aaatttttga ttttaggtga tttgtttgtt ttggtttttt    1620
   aaagtggtgg gattttaggt gtgagttatt acgttggtta aggatatggt tttttataga    1680
   gattttgttt tttaaattta taaattgttg gaaattttat tagtaaaaat gaaatattcg    1740
   agttttgttg ataggattta atttatttga tatagagtag tagttttgat ttttaaagta    1800
   ggtgtatagt tttagataaa gggtttttgg atgtaatatt ttatatgtat ttttttgttt    1860
   ttagcgattt aggatattgg ttttatttta tagttttgat ttaatgttga ttttgttttg 1920
   ttttaagtta ttatttggtt gttatttaag ttttattttt tttttttatt ttggtttttt 1980
   tttttttttt ttttttttga gataggtttt ttttttgtta tttagtttga ttgtagtgat 2040
   gtaattgatt atagtttacg gtagtcggga ttttttaagt ttaaataatt tttttatttt 2100
   agttttttaa gtagttggga ttataggtac gtattattat atttagttaa tttttttttt 2160
   tttttgtttt tttttttttt tgtttttttt tagatagagg tttgttttgt tgtttaggtt 2220
   ggggtgtagt ggtacgattt tggtttattg taatttttgt tttttgggtt taagcgattt 2280
   ttttgtttta gttttttaag tagttgggat tgtaggtacg cgttattatt tttagttaat 2340
   ttttttgtat ttttagtgga gacggggttt cgttatgttg gttaggttgg ttataaattt 2400
   ttgattttag atgatatatt tatttcggtt ttttatatag ttggtattat aggtgtgagt 2460
   tattacgttc ggtttttttt tttttttttt gttttttagt tgatataggg ttttattatg 2520
   gtatagttta ggttggtttt gaatttttgg ttttaggtga tttttttgtt ttggtttttt 2580
   aaagtgttgg gattatagga atgagttatt atatttggtt ttttttttta atttttaaaa 2640
   taaattgatt ttttaaggtt aagaggaaat attttttttg agaagttttt tttgaatgtt 2700
   agaggtagat aatgtttgat ttttgtatgt tttttaatat ttaattatat agttattgaa 2760
   taatatattt tgagagataa ttatgaatta agtaatatgt tggtttttgg gagaattgag 2820
   gataaattaa ttttgtggaa attttgggtg gatgaaaaaa attaatatga aattaaaata 2880
   ttgtatatat ttatagttgt gagaagtatt atatattttg ggtgttatgc gagttttttt 2940
   tttttttttt tttttttttg agttggagtt ttttttttgt tattgaggtt ggagtgtaag 3000
   gttacgattt tagtttattg taatttttgt ttttagattt aaacgatttt tttgttttag 3060
   ttttcgagta gttgggatta taggtgtttg ttattatatt tggttaattt agaattttta 3120
   gtagggatag ggttttatcg tgttggttag gttggtttta aatttttgat tttaggtgat 3180
   ttatttattt cggtttttta aagtgttggg attatagaag tgagttattg agtttaatta 3240
   ggagtttttt cgagaaagaa ggaagtttaa gagatttttt tgatatttta gtttgatttt 3300
   gttttttgtt tgtttaaaat ttttttatgt tttttagcgg ttttttggat atagattaag 3360
   tttttttttt gataggttta aattttttat tatttgattt tagtttattt ttttgagttt 3420
   tttttagttg ttattatttt ttgtttaaag tgatatgtta taatatttat aaagtatata 3480
   agttttatgt gtatagttta atgaattgta aatatgtgta tattcggtcg ggtatagtgg 3540
   tttacgtttg taattttagt attttgggag gtcgaggtag gtggattatt tgaggttagg 3600
   agtttgagat tagtttgatt aatatagtga aattttattt ttattaaaaa tataaaatta 3660
   gttgggcgtg gtgtcgtatg tttgtaattt tagttatttg ggaggttgag gtaggagaat 3720
   tgtttgaatt cggaggtaga ggttgtagta agtcgagatc gtgttattgt attttatttt 3780
   gggtaataag agtaaaattt cgttttaaaa taataataat aataataata ataataataa 3840
   taataatgtg tatatttatg taaatattat ttagataaaa atatggtata tttggggtat 3900
   tcggggagtg ttttttgtgg tttttttttt ttatattttg ttgatttatt agtatagatt 3960
   agtttttgtt attttttaaa ttttatattt tttttttttt tatataaata taaatattcg 4020
   agttatgatt gggtggggtg gtttaagttt gtaattttag tattttggga ggttaaggtg 4080
   cgaggatcgt ttgagtttgg gagtttagag attagtttgg gtaatataga gagattttat 4140
   ttttatataa aaagttttaa aaattaatta ggggcggtgt agttttagtt atttaggagg 4200
   ttgaggtggg aggttttagt tcgggaattt tagattgtag tgagttatga ttgggttatt 4260
   gtattttagt ttgggtaata tagtgagatt ttgttttaaa aaaaaaaaaa aaaaaaatag 4320
   gaaaaaataa ataaatagaa aagtaggttt ggcgcggtag tttatgtttg taattttagc 4380
   gttttggaag gttgagacgg ggttattttt tgggtttata agttagagat aagtttgggc 4440
   gtgggttata ttgttagatt taggtttttg taaaaaataa aattatttag tttttagtta 4500
   tttataacgt tttgtttgga agtatgttat tttgggtttt tgagtttttg tatttgttaa 4560
   ttttttttgc gttggggaga gtttaaattt tgttcgaaat ttttaaaaat ggtgtttgga 4620
   taaatgaagg tattagagtt gcgattggac ggacggttgt tggacggcgt tattgttggt 4680
   atttatcggg agatgtttat tggatagtta cgtgacggga ttaaattttt cgagggagcg 4740
   aggtaggtgc ggttacgtga ttcggcggcg ttgcggggta gcggttattt tgcggggcgg 4800
   ttacgtgaag gacgtacgtt tagcggggtt tttacgtgat tcgggcgcgt tgcggtcgtt 4860
   cgcgcggatt cggcgagagg cggcggcggg agcggcggtg atggacgggt tcggggagta 4920
   gtttagaggc gggggtgagg cgggaggtag acgggcggga ggagggcgag ttttttcgtc 4980
   ggttcgttcg ggattttttt tatcggtttg gggttgtgcg atttttaagt attgaggggt 5040
   agaaattttc ggatcgggcg ttgttagttt ttagtttttt tcgttttcgg aggtttttgg 5100
   ttttttgatt ttcgtgtttc gatttttgtt tttttggcgt tttcggattt tcgagaatta 5160
   ggggatttcg gaagttaagt tttcgggtag gttcgggttt gtcgttcgta ttattttttg 5220
   tttttggtat tggtgggagg ggcgggtttc gtttttgttt ttttaggtta ggggtttgga 5280
   tgtatatagc gtttttttag tttttttttt cggggagaat gtaggatata ggtttagttt 5340
   tttggttttt ttttattagg gatttagttg tttgggtttt ttcgttattt tatttgttag 5400
   ggttttagaa gtttagggtt ttagttttgt ttttttttag ggtcgtgagt ttttatagtt 5460
   ttttgatttt ttagaattta agagtttagg tatttttttt tttttttttt tttttagg 5518
<210> 18
   <211> 5518
   <212> DNA
   <213> Artificial Sequence
<220>
   <223> chemically treated genomic DNA (Homo sapiens)
<400> 18
   tttagaggag aaaggaaggg aagaggtatt tggatttttg ggttttaggg gattaggaga 60
   ttgtggagat ttacggtttt gagggaggat agagttggga ttttggattt ttgggatttt 120
   agtagataaa gtgacggggg ggtttagata attgagtttt tgatggagaa aggttaggag 180
   gttgggtttg tattttatat ttttttcggg gaaagaggtt aggggaacgt tatatgtatt 240
   tagatttttg gtttgagggg gtagaggcga gattcgtttt ttttattagt gttagaggta 300
   ggaagtggtg cgggcgataa gttcgggttt gttcgggggt ttggttttcg agattttttg 360
   gttttcgagg gttcgagagc gttagagagg tagggatcgg gatacgggga ttagagagtt 420
   aggaattttc gaggacggag gggattggag gttggtagcg ttcgattcgg gagtttttgt 480
   tttttagtgt ttggagatcg tatagtttta ggtcggtagg aaggatttcg gacgggtcgg 540
   cgagggggtt cgtttttttt tcgttcgttt gtttttcgtt ttattttcgt ttttgggttg 600
   tttttcggat tcgtttatta tcgtcgtttt cgtcgtcgtt tttcgtcggg ttcgcgcggg 660
   cggtcgtagc gcgttcgggt tacgtgagag tttcgttgaa cgtgcgtttt ttacgtggtc 720
   gtttcgtaaa atggtcgttg tttcgtagcg tcgtcgggtt acgtgatcgt atttgtttcg 780
   ttttttcggg aggtttggtt tcgttacgtg attgtttaat gagtattttt cgataagtgt 840
   tagtagtggc gtcgtttaat agtcgttcgt ttaatcgtag ttttaatgtt tttatttatt 900
   taggtattat ttttagaagt ttcgggtagg gtttgagttt tttttagcgt agaaggaatt 960
   agtaagtgta aaagtttaga ggtttaaaat agtatgtttt taggtaggac gttatagatg 1020
   attaaaaatt gagtggtttt gttttttgta gagatttgga tttagtaata tagtttacgt 1080
   ttaggtttgt ttttaatttg tgagtttaag agataatttc gttttagttt tttaaagcgt 1140
   tgggattata ggtatgagtt atcgcgttag gtttgttttt ttgtttgttt gttttttttt 1200
   gttttttttt tttttttttt tgagataggg ttttattgtg ttgtttaggt tggagtgtag 1260
   tggtttaatt atggtttatt gtagtttgga attttcgggt tgaagttttt tattttagtt 1320
   ttttgagtag ttgggattat atcgtttttg gttaattttt aaaatttttt atgtggagat 1380
   gaggtttttt tatgttgttt aggttggttt ttgaattttt agatttaagc gattttcgta 1440
   ttttggtttt ttaaagtgtt gagattatag gtttgagtta ttttatttag ttatgattcg 1500
   aatgtttgtg tttgtgtaaa aagaaaagga atatgaagtt taaaaagtgg tagaaattaa 1560
   tttgtgttga tagattagta gggtatggag gggaggggtt ataagagata tttttcgggt 1620
   gttttaaata tgttatattt ttatttgaat ggtgtttata tgggtatata tattattatt 1680
   attattatta ttattattat tattattatt ttgagacgga gttttgtttt tgttgtttag 1740
   gatggagtgt aatggtacga tttcggttta ttgtaatttt tgttttcggg tttaagtaat 1800
   ttttttgttt tagtttttta agtagttgga attgtaggta tgcgatatta cgtttagtta 1860
   attttgtatt tttagtaaag atggggtttt attatgttgg ttaggttggt tttaagtttt 1920
   tgattttaag tgatttattt gtttcggttt tttaaagtgt tgggattata ggcgtgagtt 1980
   attgtgttcg gtcgggtata tatatattta taatttattg agttgtatat ataagatttg 2040
   tgtgttttat gaatattatg atatgttatt ttagatagaa aataatagta attaaggaaa 2100
   atttagaaaa atgagttagg attagatgat aaagggtttg ggtttgttag agaagggatt 2160
   tgatttatgt ttagaaggtc gttgggaagt atggggaaat tttgagtagg taaagggtag 2220
   agttagatta gggtgttagg aagatttttt ggattttttt ttttttcgaa aaagtttttg 2280
   gttgggttta gtggtttatt tttgtaattt tagtattttg ggaggtcgag atgggtagat 2340
   tatttgaggt taggagtttg agattagttt ggttaatacg gtgaaatttt atttttatta 2400
   aaaattttaa attagttagg tgtggtggta ggtatttgta gttttagtta ttcgggagtt 2460
   gaggtagggg aatcgtttga atttggaggt agaggttgta gtgagttgag atcgtgattt 2520
   tgtattttag ttttagtaat aagagggaaa ttttaattta aaaaaaaaaa aaaaaaaaaa 2580
   aaaagttcgt atagtattta ggatgtgtaa tgttttttat agttgtaaat gtgtgtagtg 2640
   ttttaatttt atgttggttt tttttattta tttaaaattt ttataaggtt aatttgtttt 2700
   taattttttt agaaattagt atgttatttg atttatagtt attttttaaa atgtgttatt 2760
   taataattgt atgattgaat gttggggagt atgtagaaat tagatattgt ttgtttttga 2820
   tatttagaga agatttttta gaggaggtat ttttttttga ttttgaagga ttagtttgtt 2880
   ttgaaaattg aagaaagggg ttaggtgtga tggtttattt ttatagtttt agtattttgg 2940
   gaggttaagg taggaggatt atttgaagtt aggagtttag gattagtttg ggttgtatta 3000
   tggtgagatt ttgtgttaat taaaaaataa aagaaaggag ggggggtcgg gcgtggtagt 3060
   ttatatttgt aatattagtt gtgtgggagg tcgaagtggg tgtattattt gaggttagga 3120
   gtttgtgatt agtttagtta atatggcgaa atttcgtttt tattaaaaat ataaaaaaat 3180
   tagttgggag tggtggcgcg tgtttgtagt tttagttgtt tgggaggttg aggtaggaga 3240
   atcgtttgaa tttaagaggt agaggttgta gtgagttaag atcgtgttat tgtattttag 3300
   tttgggtaat agagtaagtt tttatttaaa aaaaaataaa aagaaaagga agtagaaaaa 3360
   aaagaaaatt agttggatgt ggtggtgcgt gtttgtagtt ttagttattt gggaggttga 3420
   ggtgggaaga ttgtttgagt ttgggaggtt tcggttgtcg tgagttgtga ttaattgtat 3480
   tattgtaatt agattgggtg atagaggaag agtttgtttt aaaaagaaaa aagaaaagaa 3540
   aaagttaaga taaaggggga gggtagagtt taggtgataa ttaaatgata gtttagagta 3600
   aagtaaggtt aatattggat taggattgtg aagtgaaatt agtgttttga atcgttggaa 3660
   ataagaaggt atatgtgaaa tgttgtattt agaaattttt tatttgaagt tgtgtatttg 3720
   ttttggaaat taaggttgtt attttgtatt aagtggatta gattttgtta gtaagattcg 3780
   gatgttttat ttttattgat agaattttta ataatttatg aatttagaga ataaagtttt 3840
   tgtaagaaat tatgtttttg gttagcgtgg tggtttatat ttgaaatttt attattttgg 3900
   gaggttaaga taggtagatt atttgaggtt aggagtttga gattagtttg gttaatatgg 3960
   tgaaattttg tttttattaa aaatataaaa attagttggg cgtattggtg tgtttttgta 4020
   attttagtta tttgggaggt tgaggttaga gaattatttg aatttaggag gtagaggttg 4080
   tagtgagtag agatcgtgtt attgtatttt atttcgggtg atagagcgag attttatttt 4140
   aaaaaaaaaa aaaatgaggt ttaattggtt tatggttttg tagattgtgt atagtaagtg 4200
   taatataggt ttttgttttt ggggaggttt taggaagttt ttagttatgg tagaaggtgg 4260
   ttttttttta ttttagttat agagaaggaa ggaaagggtt tttaatgtag gtatatattt 4320
   ttgaatagta tagttattga gtgagggaat atatttttta gttagttgtg aagttaaaat 4380
   tgtttatttt tatttttatt tatttatttt gagataggat ttttttttgt cgtttaggtt 4440
   ggagtgtaat gatataattg tggtttattg ttatttcgat tttttgggtt taagggattt 4500
   ttttatttta gtttttggag tagttgggat tatacgtgtg tgttattatg tattgttaat 4560
   tttgtatgtt ttgtagggag agggttttgt tatgttgttt aggttggttt taaatttttg 4620
   ggtttaagtg agttatttgt tttagttttt tatagtgttg ggattatagg tatgcgttat 4680
   tgtgtttgat taaaattttt ttttgtgtgt tttgttttgt tttgttttgt tttgtttttt 4740
   gagacggagt tttgttttgt cgttaggttg gagtgtagtg gtgtgatttt agtttattgt 4800
   aattttcgtt ttcgggttta agtgattttt ttgttttagt tttttgagta gttgggatta 4860
   taggcgtgtg ataacgtttt gttaattttg atatttttat tagagatagg tttttgttat 4920
   gttggttagg atggtttcga ttttttgttt ttatgattcg ttcgttttgg ttttttaaag 4980
   tgttgggatt ataggcgtga gttatcgtat ttagttaaat tttttataat aataaagatg 5040
   ttgaattaaa attattaagt ttatatttaa gatggtattt gaggagttat atttttaggt 5100
   atttatgttg ttgtgtggtt tttgtttata tttaataggg ttaaatagtg tgattaagag 5160
   gatattgtgg aaatgataga gtgtgatttt ataggatagg ttagaaaagt tattatagtt 5220
   tttattttgt ttttttttag attatttttt ttaaggaaag ttagttgtta tgtggtgagt 5280
   gttaggtttt tgagtttaag ttaagtcgtt atattttttg tgatttgtac gtatatattt 5340
   agatggtcgg aagtaattga agatttataa aagaagtgaa aatagtttta attgatgata 5400
   ttttattatg gtgatttgtt tttgttttat tttaattgat atgatatatt ttttttttta 5460
   ttttatttaa gaaggtattt tgtaatattt tttttatttt tgagaatgta atttgtat 5518
<210> 19
   <211> 5989
   <212> DNA
   <213> Artificial Sequence
<220>
   <223> chemically treated genomic DNA (Homo sapiens)
<400> 19
<210> 20
   <211> 5989
   <212> DNA
   <213> Artificial Sequence
<220>
   <223> chemically treated genomic DNA (Homo sapiens)
<400> 20
   ggtaagtggt tcgggttagt ttttttataa ggtatcgttt tcgtgggtgg ggttcgttag      60
   tgtttaggta gttggtattt ttagtttttg ttgtttgttt atttcggggg agttcggttt     120
   gggtttgtta gtattttggg tttttttttt ttttttagat tttgttttat aattttttgt    180
   tgttacgttt gttagttatt gttttggtta tatataaacg tatttatata tatatatata     240
   tgtatatata taaatagttt aattataata ttaaataaat ttttgaaaga aaaaaagaaa     300
   ttatttataa ttttattatt ttaatatttt atttaacgtt tgtatatttt ttggttattg     360
   tttattataa atatagtttt tttttattag ttaatttgtg taatggagat agatatgttt     420
   attttttagg gttgtcggga ttatatgtaa agtttttaaa ggtagttggg tatatagtat    480
   gtatttaatt gattatttat ttatttattg agataggatt ttattttgtc gtttaggttg     540
   gagtgtagtg gtgcgatttt cggtttattg tagtttttat ttttagggtt taggcgattt     600
   ttttatttta gttattaagt agttgggagt ttagttagtt tttttgtatt ttttatagag     660
   atggagtttc gttatgttgt ttaggttggt tttaaatttt tgggtttaag ttatttattc     720
   gtttcggttt tttaaagtgt agggattata ggtatgagtt atttagttta ttttatttaa     780
   atatttattg agtttttatt aatatgtttt aggtaattta tattatgagt tttttatttt    840
   atagggggag attgatgaag agacgattat ttttagatcg tattttaatt attatggcgt     900
   tgtgtaagaa gtaatttgag aagtgtggat gaaaagttat aggtagagtt ttgggtttaa     960
   gacggtggtt tggataatat gttaaataaa ttataaatta gatggtattt gaagttacga    1020
   gagtagataa aaatgatagg aggattaagg agaaatttgg gaacgttaat gtttaagaaa    1080
   cgggaaagta aaaaaaaaaa aaaatatata taaaaaaata atgtaattag agttataaaa    1140
   attagaatta tagtaaggga ttttgatgtt tatattatgt tttgttttta tgttgatata    1200
   ttatataagg atagtttttt tttaattgtt taatttttta ttatttttta ttcgatttat  1260
   tgaattattt ttaattttag gatgaatttt tttatttaat aaatgtgttt tgaatgttta    1320
   ttatgtgtta gatattgttt taggtgttag gaaaatagtt gtgaataaaa ttaataaaaa    1380
   tttttttttt tatggaattt atattttagt ttatttgtgt ttggggtaga cgaggtaggt    1440
   atttgaaaaa ttttatttaa gtttatttat ttatttattt ggagatagaa tttcgttttg    1500
   ttacgtaggt tggagtgtag tggtacgagt ttttgtttat tgtaattttt tttttttagg   1560
   tttaagtgat ttttttgttt tagttttttt agtagttggg attataggcg tatgttatta  1620
   tattcggtta tatttaagtt attttaaaaa aataattaat taatttattt ttttgagacg    1680
   gagttttatt ttgtttttta ggttggagtg taatggtatt attttagttt attgtaattt    1740
   ttgtttttcg ggttcgagcg atttttttgt tttagttttt tgagtagttg ggattatagg    1800
   tatatattat tacgtttagt taatttttgt atttttagta gagataaggt tttattatat    1860
   tggttaggtt ggtttcgaat ttttgatttt aggtgatttg tttattttaa ttttttaaag   1920
   tgttgggatt ataggtatga ttttttacgt ttggttatat ttaagttatt taatttatta   1980
   gaagatgata aatattaaga ggaaaaaata aaatagggaa gtaggatgag gttgtatgga    2040
   ttggtaaaat ttaagttagg gtggttatga tttttttgtt tattttggag gagtttgttt   2100
   ttgttttgtt aatattaaga taagggattt gatttatgtt taaaggaagt gagggagtga    2160
   atcgtgtgga tataaggaga gagtattatg agtatagtta gtaattaata aaaatgttta    2220
   gtgtaattgg aaggagatga tttttttgtg tgttgagtta tatgggtgtg ttgagttaga    2280
   cgggaagtta ttggaaggtt ttgagtagag tgatatgatt tgatttgtga tttgaaaaga    2340
   agtattttta tttttgtgtt aaggggaaag aagatggaag agggaaaatt atttagggag    2400
   gtattgttat atttaggttt taggaggttg tggtttagat tagggtggag tagtgagggt    2460
   ggtgggagta ttttgaaatg aagtaaggaa gtagtttgat tttattttga aaggattatt    2520
   ttggttgtta agtggagagt agatggtagg ggtaagggta agaagtagag attacgtgga    2580
   ggttattata atgttaggtt tacgattgag ttaaatgtta tgtttggggt taggttttag    2640
   tttatgttga ggtttaaggg gagtgggtgg atgagtagaa agaatattta ggggatcgta    2700
   ggtaggtaaa agatgatttt atttagtagt agtttttatt aatagttttt ttatattagt    2760
   tttttatatt gtttgttttg ttttaggtgt ttagtttggc ggttttttta tatagttgta    2820
   tggtcgattt ttttttgttt tcggggttag tagtttaatt tttttttttt ttgggtatga   2880
   gtaagtcgag ttgtgttttg gttttttatt gttagttttt aagagggtta attttggttt    2940
   tttttttttt tttttgggtg ttagtacgtt tgtacgatgt taatagggta attatatttt    3000
   ttatagataa tagtgtttag agttaaggga tggttttttt atgttatggt tatatagttg    3060
   tgataataag tggagttata tgtttgcgtt ttaaattcgt tgagtttttg tggatgttta    3120
   tttcgtttta tttttgatta aagtatagtt atgtttttta tatatagtta tttaggtata    3180
   gtttaggatg gtttgattag ggtgggggta gtggtgagta ttttttattg atattttaaa    3240
   ttttttttaa gtgtttattg ttattaaaaa attgttatag aatttatata tttttatttt    3300
   tttttttttt aaattattat tgtttttgta cggggagaat tttttgtgtt tttttttttt    3360
   tttttttttt tattagggga tgttgagcgt tatatatatt attttatatg gcgagtcgtt    3420
   atagttttta tatatataat gatacgggtg ttatagttat atatatatat atagttcgtg    3480
   agtttttttt ttttagtatt gtgtgtttgt gtgtttttat atattatata tgttttttga   3540
   gaggttatat attttataat atatattttt atgtatatat atatatatat atattgttag      3600
   aaattatata tataatgtaa aatattttat tgattatttt ttatattgat tattatattg      3660
   atataatatt ttaggtagat aaggtttaat aaatgtatta atattaaaat taattttatt      3720
   tgttttattt tgttttttaa aattgtggtt attagaaaat gtgaaattat atatgtgatt      3780
   tttagtatat ttttagtgga tagagttggt atagaatatt tttagaattg ttttattttg      3840
   gtatatattt taagatttag gttttttatt taattttgta gtaggacgtt tatagtttag     3900
   tttagatttt gttgaattaa gttaagttta gttatttttt ttgttttttt ttttatgtga      3960
   ggtttatatg tttgttttgt agtttagtaa ggaattatgt gaacgagaat atataatttt      4020
   ataataataa ggtttatata taggcgtaat atttgatttt atatatgaag tgggttaatg      4080
   ttttaaataa tttattttgt gtttttattt attcgtagtt tgtatatttt ttttagtttt      4140
   gtgttttttg atttttttta tttgtatttt aatgattttt tttttagttt agaaaattcg     4200
   atatgagtta ttattgtttt aggggaagga tgtcgttttt gaagtagaga attagttaaa      4260
   tttatagtgt ttaagtttaa aagttattag tgtaggtata agatagaaat agtttgttgt      4320
   ataattgtaa aattgtaaag aaagttttgt tttttagatt aaggagttta tagattgggg      4380
   gatagggaga tgttaaggat tttaagggta gaaggagaga tagttgtttt ttatggattg      4440
   tagtttataa gtttatattt tttggagatt tttattggtt ttgaggattt agattagatg      4500
   ttttatttgt ttgtggtgaa tattgtttat ggtttgtagt tatagtgatg attagtaagg      4560
   tttaaagata attatttgtt ttttagggtt ttgtttagtt ttttgttttt tatatattac      4620
   gtgaaaagtt tttgttttta acggtttagg gtatttagaa gttttaggat tgaagatttg      4680
   gttttttaga gggtttttat ggaatagtat tcgggtaaga tgggattaga tagaattggt     4740
   tttaaatttt ttaataagtt aaggtagtgg tggatatatt gggttatata tttttatatt      4800
   ttttaggtag gaatatgttt ttaaaggaga tattattttt ggaaatgtgt ttttgtttat      4860
   agttaaatta atttttattt ttaatgtgta gagttatatt tttttttgaa gttatattat     4920
   tttatgttta tttttttttt attttttttt tagtttatta ataggatttt tttaatagag      4980
   gaatagtttt tttttttttt tgagacggag ttaagttttg ttatttaggt tggagtgtag      5040
   tggtacgatt tcggtttatt gtaagtttcg tttttcgggt ttacgttatt tttttgtttt      5100
   agttttttta gtagttggga ttataggtgt ttgttattaa gtttggttaa ttttttgtat      5160
   ttttagtaga gatggggttt tattatgtta gttaggatgg tttttatttt ttgattttat      5220
   gatttgttcg ttttggtttt ttaaaggaat agttttttaa atataaattc ggttgaaaat     5280
   tattaagtat taaaaaaaat agggtatgat tttatttagt gttattttat aatatatata      5340
   tttgggttat aaagttagaa aattaatgga atgagttaga aatagttatc gtaggaggta      5400
   taaggttgtt tgagttggtt tataattatt tatagagagg tgtgtttggg ttgtatttag      5460
   aatatatttt attttgtttg ttaaagtggt tggatttaag tggatatatt ttaagagttt      5520
   ttttaggttt tgatatcgat gaaagaggaa gatggaaagt tgtttatagg atttttgtag      5580
   tatttggttt ttttaggtta atgtagatat taaaatattg ttttgttatt gatttacgat      5640
   ttttgtttta gtttttttaa tattggaaat aagtgtttaa tgtttttagt gatttagtaa     5700
   atattttaga gggtagtttt ttatatttta tgtttttatt gttaggtgat tgtgggttaa      5760
   tgatagaaaa gataaatgga aaatatttgg atatttaaag ggtaatgata ataaattgtt      5820
   ttttagatat tggagtttga gtatattatt tgttgattaa ggtaaagatg gtaaatgttt      5880
   ttggggtgat ttttaattat atattttttt ttttgaattt tgtattagtt gttatattta      5940
   tatagtgtga tttttttttt tttatagttg agtggataag atgtagata                 5989
<210> 21
   <211> 14924
   <212> DNA
   <213> Artificial Sequence
<220>
   <223> chemically treated genomic DNA (Homo sapiens)
<220>
   <221> unsure
   <222> (208..209, 211, 213, 227)
<400> 21
   ggttttatag tgttatttgg taatgttata gaattagatt agagaaaatg ggattttttt        60
   attgtagtgt gtttaatgat ttttgtaaag agtatagtat aaaaaagttt tattagtttg       120
   aagatttatt ttgattgaat tgtaggattt ttgtttgggt ttcgtgtatt ttattaaaat       180
   gaaataaagt agttatattt aaattttnnn ntnttttttt ttttttngga gacggagttt       240
   tgttttgtcg ttaggttgga gtgtagtggt gttattttag tttagtgtaa tttttatttt       300
   ttgggtttaa gcgatttttt tgttttagta tttttagtag ttgggattat aggtggttat       360
   tattatgttc ggttaatttt tggatttttt cggtagagat ggggttttat tattttggtt       420
   tattgtaatt ttcgtttttt gggtttaagt gatttttttt ttttagtttt ttgagtagtt       480
   gggattatag ttatcggtta ttatgtttgg ttaattttta tagttttagt agagataggg       540
   ttttattata ttggttaggt tggttttaaa ttttttattt aaggtgattc gtttgttttg       600
   ggtttttaaa gtgttgggat tattaggtgt aagttattgc gtttagttat aatatgtaag       660
   aatttttaaa gtaatttttt ttggtatata gaagattaga aatataaagt tttaggttgg       720
   gtgtggtgat ttatgtttat aattttagta ttttgggagg ttgaggaggc ggattatttg       780
   aggttagtag tttgatatta gtttggttaa tatggtaaaa ttttattttt attaaaaata 840
   taaaaattag ttagaagtgt ttttaggcgt ttgtaatttt agttatttgg gaggttgagg 900
   cgggagaatt atttgaattc gggaagtaga gattgtcgtg aatttagatt acgttatcgt 960
   attttagttt gggcgataag agtaaaattt cgttttaaaa aaataaaaat aagggtttat 1020
   ttttttttta tttttattta ttgtttaagt taggatttag tgaaggtagg tagttgattt 1080
   ttgttgaata aatgaaatgt ttagttaatt attattattt ttatttttat ttttgagata 1140
   gagttttttt ttgttgttta ggttggagtg tagtggtgta attttagttt attgtaattt 1200
   ttattttttg agtttaaacg attgttttgt tttagttttt taaggagttg ggattatagg 1260
   tgcgtgttat taagtttagt taatttttgt atttttagta gagatagggt tttattatgt 1320
   tggttaggat ggtttcgatt ttttgatttc gtgatttatt tgttttggtt ttttaaagtg 1380
   ttgggattat aggtataaat tatcgtgttt ggttttatat tttttttttt ttataagtag 1440
   agatggagtt ttattatgtt ggttaggttg gttttaaatt tttgatttta agtgatttat 1500
   ttgttttggt tttttaaagt gttgggatta taggcgtgag ttattatatt tagttaaaga 1560
   tgtttgattg taattttaaa attatatgtt tttattatta ttattattat tattattttg 1620
   aggggatagg attttatttt gttgtttaga ttggagtgta gtggtataat tttagtttat 1680
   cgtaattttt gttttttggg tttaagtgat ttttttgttt tagtttttta agtagttggg 1740
   attataggta tgtgttttta ttgtttgtat ttttagtaaa gacgggtttt tattatgttg 1800
   gttaggatgg ttttgaattt ttgattttaa atgatttatt cgttttgatt ttttaaaggc 1860
   gtgagttagc gggttcggtt gtttttttta tttttaaaga aatatttagt attaaggaaa 1920
   tgtaatgaga gagagaaaat ttaatttttt tatttttttt ttggttttta tgatgtttaa 1980
   agatgattta aggaggtata gtagtttgtt tttggttttg tgtttttgtg ggttaacggg 2040
   tatagtttag gaggtacggg agaatttttt taggggaatt tttggagaag tggtttgata 2100
   atatatttgt atgttttttt gtgttttata ggttttatat tagtttggat attttaagaa 2160
   aatgttatga ggggattaga cgtttaatag atttattggg gtaaatgttt gtgagggaaa 2220
   agagggaggg aggtagaaga gattatagag ttgttagatt ataatgtagg attgattttt 2280
   gtaaaggata gaaggaggaa gatggggtag gaagtttttt atgattgtgt agtcgaagga 2340
   agttttagta aagtttatgg ggagttattg aagtaaagtt atttattaaa ggaagttgat 2400
   ttgtaggaat cgattttaag gtttttgttt tgtttagtta ttggttggga atagtttgta 2460
   ggatgtgtgg ttttagtgta aagttgtggt agatttaaag tattgcggtt ggggttttta 2520
   gttaattaaa ttattatagt aggtttgaaa gatgtatttt tatggttgtt acgttattaa 2580
   cgagtatttt aagtagtatt tttttttttt tttttaattt tatggtgata gtttagaaat 2640
   gtaggttata tatgaattat tttttttatt tgtagttggt tatagatagg ataaaagatt 2700
   taaagttatt ttatatgtgg atggaaaaaa aaagtgaatt taattttttt agcgattatg 2760
   agttattttt tttttttttt atttattgta aatttttttt taggattgtt gttatagtta 2820
   aattaaatga gatttgtatt ttatttatga agttatattt ttgataggta aagagatgat 2880
   tgggaggtta aattttttat tagtttataa aagaataatg aggaattagt tttgattagg 2940
   tacgtacgtt ttattaggta tggtgataga ggttttatat aggttatttt tttgaatttt 3000
   attttaattt tttgaggtta atatgtattg ttttatttta taaattaagt atagatttaa 3060
   ggtatatatt gagtaaagaa agagagattt ggttgggatt gagtttaggt tttttgattg 3120
   taagtttagt gttttttttg gttttgtaat ttttaattta tatatcggat agtttatttg 3180
   agttaaaaat gtgaattaag ttaggtttaa agtagaattt tgttttttat tagaaatttt 3240
   agagtaggtg gaaggtgaag attaaagaag gtattttttt tttttttttt tttttttttt 3300
   ttttgagatg gagtttcgtt ttgtcgttta ggttggagtg tagtggtata attttagttt 3360
   attgtaagtt tcgttttttg ggtttaaagg atttttttgt tttagttttt taagtagttg 3420
   ggattatagg tgttcgttat tatgtttggt taattttttg tatttttggt agagatgggg 3480
   ttttattgtg ttagttagga tggtttcgat tttttgattt cgtgattcgt tcgttttagt 3540
   tttttaaagt gttgggatta taggggtgag ttatcgtgtt tggtttaaag agggtatttt 3600
   tttttttttt ttcggtatta tttttattta attataaatg attaagggcg gtattgttag 3660
   agttatttcg agaggttttg ttttttgtta tttaggttgt tttgtagttt ttattttaaa 3720
   ttgtcgttat ttcggtttat ttcgtggatg gcgtttaaga ttttttttaa atttgaaatt 3780
   tatagaatta ttttaaagtg aataatttag tggtatttag tattttcgta gtgttgtgta 3840
   attttatttt ttatttagtt gtaaaatatt tttttttttt tttttttttg gagatagagt 3900
   ttcgttttgt tgtttagatt ggagtgtagt ggcgtgattt tggtttattg taatttttgt 3960
   ttttcgggtt taagtaattt ttttgtttta gtttttcgag tagttgggat tataggtata 4020
   cgttattcgt ttggttaatt ttttgtattt tagtatagac ggggatttat tatattgttt 4080
   aggttggttt taaatttttg agtttaggta attcgttcgt tttgtttttt taaagtgtta 4140
   ggattatagg cgtgagttaa cgcggtcggt tttgtaaaat atttttatta tataaaagaa 4200
   aattttttat ttattaagta gttatttttt ttttttttta gtttttgtta attattaatt 4260
   tgttttttat tttatggatt taattgtttg gatattttat agaaatagaa ttatataatg 4320
   tatgattttt tttgtttggt ttttttttat tagtttaatg tttttgaggt ttatttttgt 4380
   tgaagtatgt attagttttt tatttttttt ttgggatgaa taatgtttta taatttattt 4440
   attttttcgt ttgttgatga atatttgagt tgtgtttata ttttggttat tgtgaatagt 4500
   gttgttatgt atattcgtat gtaagtattt gagtatttgt tgggtgtata tttatgaatg 4560
   gaattgttgg gttttttgat aattttgtat ttaatttttt gagaaattgt taatattttt 4620
   ataggggttg tattatttta tatttttatt agtaatttat aaggatttta atttttttat 4680
   attttgtatt taagatttgt aaagttgaat taattatagg atgaggaagt ggtttttttg 4740
   aaatattttt tttttttata ttttaaattt ttttttttat atatgatatg aaatatagtt 4800
   tacgtacgaa agtgattagg aggaaggata ttataaagtg atgtaaatag aaattttatt 4860
   agtttttatg tattattatg tgttataatt tagttaagtt tagtgagtat ttttagtata 4920
   ttgttttaat agttttaagg tgagttagtt taagattttg ttttttatta ggtagaagat    4980
   gatagattgt gaatttggat atatttatag gttggtttag gattatttgt agtgcgtttt    5040
   atagatatta taatttggat taggtttaag taaaacgttt agagtgttat aaaatgttgc    5100
   gtttttagtt taaaaagaag tggaaaagaa tttgaagtta tgtttggata atgttaatgt    5160
   tgtgttcgta gatattgtta gaatattatt taattaagtg atggaaaagg agtttgaaga    5220
   cggtattatt aattggggaa gaattgtaat tatatttgta tttgaaggta tttttattaa    5280
   gaaattttta cgatagtaaa ttgtttcgga tgtggatatt tataaggaga ttttatattt    5340
   tgttgcggag tttataatga ataatatagg agaatggata aggtaaaacg gaggttgggt    5400
   atgtgtgatg gaaaaatttt ttattgtttt tttttgtgaa atagaaattg agaatttttt    5460
   tgttagttaa aataataata aaatatttta tttaagaata ttattttttt tgggtggttt    5520
   gttttaattt ttaaaataaa aatatatttt ttggtttatt tttaatgaat ttttgaattt    5580
   gaattttata gttggaaagt atttatgttt ttagttttta tttttaatag tataaattat   5640
   agtagtgagt tttggggatt aataaagtat tttttttatg tagttttata attagttgtg    5700
   tatatatttt aatttagtat ttagtttttg ttagggttgt tatgatagta tagatagtat    5760
   agaaattttt ataattagta tagaaagttg ttattagatg ttgttatttt taggtattta    5820
   tgggttgtaa aagataggtg taaatttttg taagtttatc gtttataaat tggtgttttt    5880
   tagtttatta atatataata aatagtaata tatattaata tatagtaaat tatattatat    5940
   ataatatata atatattatt attaataata aatttcgggt tgttaaagtt tgttgattaa    6000
   aatataatat attgtatgat tagtaagaat ttttatttaa gaatttttaa ataaaaattt    6060
   attttattat tagtgtttaa aaaagttaag attatgtaga ggtttgtgtt gaaatttatg    6120
   ttggtaaaaa gtttgatata gttggatgta tatggttttt tttatatttt aatatattta    6180
   ataattattt ttttaaagta tttaagtgtt tattgatttt ttttttaacg gtttttattt     6240
   tttttaagag gtaattattg ttattaatta ttttgtatgt aaaatatgtt tattgaattt    6300
   atttatagaa aataaataat atttttttgt aaaatatttt ttgtaaaaaa agttttatat    6360
   aaaatgtata tgtatgttta tgtatatgtt ttatataata tatatgttat attatataat    6420
   atatattttt tttatatata tatatagatt ttttatgtat atatataaat tttgtatata    6480
   tatttttata tgtgtatatg taaaaatagt atatggtatt aatgttgtta tttatttttt    6540
   tatttagaat tatttaaaat agtattatta agagtgttgt gtatattttg ttatatgtaa   6600
   gtgttgtaga gtatttggtt atttttagtt gatagttgtt taggttattt cgagtttgtt    6660
   gttatatata atgttttatt gaatattatt ttacgtaaat ttttttttgt gcgtgtgtgt   6720
   agttttttag ggtggatgtt gggttgtaga tgttgggtta tggtgtatat atatttttta  6780
   atttgttagg tttttgtttt ttaaagtggt tttattaagt tatattttta ttagtagttc    6840
   gttaggatat gtttcgggat ttttttattt tagttgatta atagtaggtt gttgtaattt    6900
   attataattt ttttaatttg ttgggttaaa aatatagagt ttattttgtt ttaatttttg    6960
   tttttttgat ttttagtaag gttaattttt tttttatatt ttgagtattt ttttatatat   7020
   taattgatta tttgtaaatg tagggaattg tttgtttata ttttttgttt attttttttt    7080
   tattttgttt tttttttatt gatttgtagg aattttttga atattttgga tataaattat    7140
   tatttttatt attgtttaaa ttttttgaga tagagttttt atttttgttg tttaggttgg    7200
   agtgtagtgg tgtgatttta gtttattgta atttttgttt tttggtttcg agtaattttt    7260
   ttgttttatt tttttaagta gttgggatta taggtatgtg ttattatatt tagttaattt    7320
   tgtattttta gtagagatag ggttttatta tgttagttag gttggtttcg aatttttgat    7380
   ttcgggtgat tttttttttt tggtttttta aagtgatggg attataggcg tgagttatgt   7440
   atttaaatta tttttatgta tgtggtagat attttttagt ttgttgatta acgttaatga    7500
   tgtttttggt tattataaat gtttaaaagt ttgttgtaat taaatttatt gattttttat   7560
   ttgatgtttt ttttattttg tttaaggttt ttttgtatga tagagtttat tgtagttatt    7620
   tttttttttt aattattttt tttttggaat ttagtttttt ttaattgagt ttgattgagg    7680
   ttgggcgaat tatgtgaaat gttttgttat gttttttttc ggttggttgt gggaggttat   7740
   atttgaggat tggtggaaat ttagttgtat tgtgtagatt ttaggtgaag gtaaatgttt    7800
   tttggtatag aggattatag atgagtttgt agagaaatat tttattagtt tgtaggtttt    7860
   atttgggttt ggattgtatt tttaacgttt agtataatat ttggagtata tagatagtga    7920
   ataaatgtta gttaaaaatt taattttttt ataataaatt ttgtgagttt ttggaattta    7980
   ttgttagtaa gaggtgtgga attgtttttg agaattttta gtagtatttt tattttttaa   8040
   gggtggtata gatgtaaata tgagtgtggt cggatgaatt tttttcgagt gtagatataa    8100
   atatatatat tgtttgtacg tgtatatatt tagttttttt aaagttttta agtttttttt    8160
   tttttttaaa aaaaatattt gaatagttta agttgtagat atgaaataaa tatttttgga   8220
   aatgttattt taaagtaatt tttatgtcgt ttttttagtt tagatgagat attgattttg    8280
   ttaacgatag gtagtgtttt tttgagttag tgtaaattgg tatggaaaat tgttattatt    8340
   tttttatagt gatgttgata aatttgttta aatgaatgaa tttatttttg aatgatttat   8400
   tttaggggaa atggtataat tatatattta tgttggtaga gttagtggtt tataagaaga    8460
   ggtaagagta gagaaggtat ttatagtttt attaagatgt gagtatatat aatttataat    8520
   ttaattaaat gttaaaattg tttagagatt ttatgaaaat tttgaattaa taatgcggta    8580
   tgtgtcgggt gtagtggttt atgtttgtaa ttttagcgtt ttgggaggtt gaggtgggtg    8640
   gattatttga ggttaggagt ttaagagtag tttagttaag atggtaaagt tttgttttta    8700
   ttgaaaatat aaaattagta gggtttggtg gcggatgttt ataattttag ttatttagga    8760
   ggttaaggta ggagaattat ttgaatttgg gaggcggagg ttgtagtgag tcgggattgt    8820
   gttattttag tttgggtaat agagtgattt tattttaaaa aaaaaaaaaa aaaaaagcgg    8880
   tatgtgagta tagtatagat gtttagagta ttaatttaga attagataga tttggtttta    8940
   tttttgggtt cgagtgttta ttatttgtgt gaattgtata agttgtttaa tttttttacg   9000
   ttttgttttt ttatttgtaa aaataggaaa atattgtaat ttatgttttg ggattggtat    9060
   aaggattaaa ttagttaata tatgtaaagt atttgaaata atttttgaaa atgagtattt 9120
   aatataagtt taggattttt gtttattatg taaaatagat tttttaatgg gaagaaacga 9180
   gattggttta ttagtaatag gatttataat ttttttcgtt attttttttt ttttttttgt 9240
   tagttgtttt tttttttttt ttgttttttt gtttttttgt ttttttgttt ttttgttttt 9300
   ttttgtttgt ggttttttat tttttttttt gttttttttt atatttatta tggttttttt 9360
   ttgttcgttt ttttaattgg aagtgttgag aggatgtgtt tttataagat ttgataggag 9420
   agttagtgtt atttggttgt ttttagtatt tataaggaaa ggatggtttt taaaaattat 9480
   tagaatgttt atgagatgaa atttatgttt ttttttttgg gattattttt ttttttgaat 9540
   tagatgggtt tttttatttt tttttttttt aatgaagtat ttttttttgt atatgttggg 9600
   ggagtagaaa gaatgtttgg tatttagttt tatatgggag aaattgtgtt tatttaatgt 9660
   attattgtgt attttatgtt tagtatagaa ggtaataata tttgttgatg ggatgattta 9720
   acgaattaat attgaaattg aatatttgaa ttttggtttt tttatttatt agaggtataa 9780
   ttttgggtaa attattgagg tattttgaga tttagatttt ttatataatg tatataataa 9840
   tatgatatga tatatgataa tttattatat atgtaatatt taatgaaaat aattttattt 9900
   atttttaaag gaaagttaaa ggattataaa atattttttg taaaatagtg ttttttaagt 9960
   tgttaaatat tgtgtaatta cgagagtcgt gggaaatttt aggaagttta gataggtaat 10020
   tttttgtttt tttgaagtag gatgtatagt tagagttggt gtttttttag taattattat 10080
   tttaaaggtt gggttatata gttaggttaa gaggtttttt tttttttttt tttttttttt 10140
   tgttattttt ttattttaaa taatagttcg gttaatttga ggttaaataa atgggtatat 10200
   agagatatgt atagatgatt aattaagtgt ttattttggt aaaggtattt ttaaaaggta 10260
   taaagattta gtagtaaggt gatattattt taagtgttta tatatatttt ttgattaaat 10320
   aagttttggg gtaaaatttt ttatgttttt tgaattttta tttgagattt taaaattatt 10380
   tagtgaaaaa taaaagattt ttaagttagg tagaaatgta tgatatttaa gtgttagttt 10440
   tttaattttg ggagttagtt tggatttttg ttttttatag tttatattta tttattagta 10500
   aatttttttg gttgtttttt tgaaatgttg taatattttg tgtatttagg tcgttttttt 10560
   tttttatttg attttgttag tagttttttg ttagatttta tggttttatt ttttatttgt 10620
   tagaatttgt tttttatata gtttttatag cgatattttt aaaatttgag gggtcgggcg 10680
   tattggttta agtttgtatt tttagtattt tgggaggtcg aggcgggcgg attacgaggt 10740
   taggagatcg agattatttt ggttaatata gtgaaatttc gtttttatta aaaatataaa 10800
   aaattagttg ggcgtggtgg cgtatgtttg taattttagt tatttggtag gttgaggtag 10860
   gagaattgtt tgaatttagg aggcggaggt tgtagtgagg tgagatcgtg ttattgtatt 10920
   ttagtttggg taatagagtg agattttgtt ttaaaaaaat aaaataaaat aaaaatttga 10980
   attagattat gttttggttt aattataatt ttttaagttg tttttttatt ttgggaataa 11040
   aagtataaaa ttttgattgt ggtttttaaa gttttatatg atttagtttt taatcgtttt 11100
   ttaaatttta tttgtgaatt ttttcgtgtt tttatagttt tagttttttt ggataagtta 11160
   agtatatttt atttttaagg tttttgtatt tgttgttttt tttgtttgga ataatttttc 11220
   gttagaagtt tgtttggttt attttttttt tttgtttagg tttttgtttt aaggttgttt 11280
   ttttagagaa gttttttttg attattttaa ttgagaaagt attattgtta ttttttgttt 11340
   ttttatttaa ttttattgtt tttataatag tttttatgat tggatatttt attgtatatt 11400
   tatttgttta tttgtttatt gtttgtgttt gtcgtgaaaa tgtaagttta gtgagggtag 11460
   ggattttgta tgtttttttt attattgtgt ttttagtgtt ttaaatggta tttggtatgt 11520
   ggttaatgtt taatgaatat tttttgaatg aataaatgaa taataatgtt tagagaagtg 11580
   ggagtaatat aatggtgttt gcgtgtattt tgtttgtttg tttgtttttt tgaaataggg 11640
   ttttgttgtt atttaggttg gagtgtagtg gcgtaatttt ggttaattgt agtttcgatt 11700
   tttagggttt aagtaatttt tttattttag ttttttaaag tgttaggatt ataggtttga 11760
   gttattgcgt tcggttgttt gtatttttaa aaattttgag attttgattt tttttggatt 11820
   attgatattg tatattattt tatgttttag gtttttttgt aattaaggta gttgaattta 11880
   ataaattatt attattttta gaaataataa ggtttgaatt atttttattt attattatat 11940
   tattgatagg atattgaatt tttgtttgta tatttatttt tatataatgg ttttaagata 12000
   taaatagtat tattaaagaa aatatatttg aaaataatat ttttgaaagg aagaaaagtt 12060
   atttttatta ttttaatata tttatatgta gatattgtat gtaattataa ttagggtgta 12120
   taagagtttt tttttgtgat atattgtaga tatttttaat attgttttat atattttatg 12180
   ttaatattag gtattaattt ttaatgttta tagaatattt tattaatttg ttatattata 12240
   atttatttaa ttaattttta attgttagat aatttggttt tttcgagttt ttttttttta 12300
   gatataatgt tttaatattg aagtatgggt atgttttttt ggtgtttttt attttttaat 12360
   attttagaag attgttttat gataaatttt taatattgga atgattggtt tataagatgt 12420
   gtgtataatt ataatatttt agtatattgg aaaatatatt ttttttttaa aagtattata 12480
   ttaatggaat aatgttataa taaatttaat gatgtgggtg gtattattaa taattttgag 12540
   gtttgttagg tataaaataa taaagttaat ttaataggtt taaaaaataa taagattgtg 12600
   ttaaaagtat atttttaatt gttattagtg ttttttttat atatttgtta ataatttata 12660
   tttttttttt taatggtttt ttttttttaa attattaagg gtttgaggat tttttgttat 12720
   atttgaatga atttttttat gttatatgta aatatttttt tagtttattg tttttttaat 12780
   tttaattttt tatagtttaa aaaaatttta ggttaggtac ggtggtttat gtttgtaatt 12840
   ttagtatttt gggaggtcga ggcgggtgga ttatgaggtt aagagatcga gattattttg 12900
   gttaatatgg tgaaattcgg tttttattaa aaatataaaa attagttggg tgtggtggcg 12960
   tgtgtttgta tagttttagt tatttgggag gttgaggtag aggtagaaga atcgtttgaa 13020
   tataggaggt agaggttgcg gttagttgag atcgcgttat tgtattttag tttgggtggt 13080
   agagtaagat tttgatttaa aaaataataa taataaattt taattttttt ttggttgaat 13140
   ttattatttt tttttttgtt tattgttttt tcgatattga tgtatataag gtgatatatt 13200
   atgattgttt aatgataata gataatgatt attgttataa taataaaaat tatgttgggt   13260
   gcggtggttt atatttgtaa ttttagtatt ttgtgaggtc gaggtgagtg gattatgagg   13320
   ttaggagttt aagattagtt tggttaatat agtgaaattt cgtttttatt aaaaatataa   13380
   aaattagtcg ggtatggtgg tgcgcgtttg tagttttagt tatttaggag gttgaggtag   13440
   gagaattgtt tgaattcggg aggtagaggt tgtagtgagt tgagatcgcg ttattgtatt   13500
   ttagtttggg taatagagta agattttatt ttaaataata ataataataa ttttagaggg   13560
   ttttattata tttgcgaaat atggggttta tgaaaatttt aggaagggga gtaatgaagt   13620
   ggatattttt tgagagtgta taggaaatta tgagtatgta ttggtaggag ggaaagagga   13680
   agaagagaga gggttagttt tttttttttg ttaaggtatg atagtatgtt ttagagtacg   13740
   atgtatattt aatacgtagg tagaaaatgg tttttttagg tagtttttta aatgaaattt   13800
   ttttggttgg tggtatttat ttgttttgtt agtgatgttt ataaaatgat tttaaggtat   13860
   cgtagagttt tcgagttatt tttaattatt aaagagttat gttcggttta tattttaata   13920
   ttagttatat tgggttttga gttatttgta ggtatttgta attttaaggt tttgaaattt   13980
   gtaaggtgaa gacgtaaatg tttgttagat gattatttga aaaaattatt ttaataattt   14040
   tttaattttt ttggtatttg tttggaaatg tatggagtgg aaagaagtag ggtgtttata   14100
   tttttattta agtttaggta ttaacgggtt ggttaatttt agataattta tttttttggg   14160
   tttcgatttt tttatttgtt aaaataatgg agggttttag gttagatgat tttagagata   14220
   gtttttggtg aggattattt gattatagat tgtaaattaa ttaaataaaa tgtttagtta   14280
   attgggttat tttatatttt taaaattatg tttgattata aagagagtag attataaaat   14340
   aaattattaa tttttttata gttttaaagg tattagttta atttggattt attgtgagtt   14400
   tttatgattt ttaatataat gtagtttata gtataatata atttatatta tatatataag   14460
   tgtattgaaa ttaatgttgg ataattgaat aagaacgaga aaaaaaaagg ttttttattt   14520
   tttagagttg tgtttgttgt ttattatgga agtgaaatgt ggttagtttg aattaagatg   14580
   tattgttaag tattaaatat ttattggatt tcgaaggttt gttatgaaaa aagaatattt   14640
   tattaatgat tttatattga ttatatatta aaataaaaat aattttaaaa ttattttaaa   14700
   agttagtttt atttattttt ttattttgta aaatatggtt ataaaaaaat ttaaattata   14760
   gacgtaggtt atattgtatt tttgttagat gttgttgttt aataggggta gaaggggtag   14820
   aagattaatt atttgtatta tttaaatgaa aatatttttg ttttttattt tagtatattt   14880
   aatttttaat gatgtttaaa gtgttaaatt tattttttta tagg                    14924
<210> 22
   <211> 14924
   <212> DNA
   <213> Artificial Sequence
<220>
   <223> chemically treated genomic DNA (Homo sapiens)
<220>
   <221> unsure
   <222> (14698, 14712, 14714..14715, 14717)
<400> 22
   tttataaaag aataaattta atattttaaa tattattgga gattaagtat gttggaataa      60
   aaagtaaaaa tatttttatt taaatagtgt aaatagttaa ttttttattt tttttatttt     120
   tgttaagtag tagtatttaa tagaaatata gtgtgattta cgtttgtaat ttaaattttt     180
   ttgtagttat attttataaa gtaaaaaaat agataaaatt aatttttagg atagttttag    240
   agttattttt attttaatat gtaattagta taaaattatt aatgagatat ttttttttta     300
   taataaattt tcgaaattta atgagtattt agtatttaat agtatatttt aatttagatt     360
   agttatattt tatttttata gtggatagta agtatagttt tagaggatag aaagtttttt     420
   tttttttcgt ttttgtttaa ttgtttaata ttgattttaa tgtatttata tatgtaatat    480
   gaattatatt atattgtaaa ttgtattgta ttgaaaatta tgagaattta tagtaaattt     540
   aaattaaatt aatatttttg aagttatgaa aaggttgata gtttatttta taatttattt     600
   tttttgtgat tagatatagt tttaaaaata taggataatt taattaattg gatattttat    660
   ttagttaatt tataatttgt agttaaataa tttttattaa aaattatttt tgagattatt    720
   tagtttgaag ttttttatta ttttaataaa tgaggaaatc gaggtttaga aaaataagtt   780
   atttaagatt agttaattcg ttggtgtttg gatttggatg agaatgtgaa tattttattt     840
   ttttttattt tatatatttt taaataagtg ttaaaagaat tagaaaattg ttaaaataat    900
   ttttttaaat ggttatttgg taaatattta cgtttttatt ttataggttt tagaattttg   960
   gggttataaa tgtttgtaag tagtttagag tttagtgtaa ttaatattga aatgtaaatc    1020
   gagtataatt ttttgatagt tgggagtgat tcgagagttt tgcggtgttt tggaattatt    1080
   ttatgagtat tattggtaaa gtagatgaat attattagtt agggggattt tatttgaaag    1140
   attgtttaag aaagttattt tttgtttacg tgttaagtgt atatcgtgtt ttgagatatg    1200
   ttgttatgtt ttgatagagg aaggaaattg gttttttttt tttttttttt tttttttttg  1260
   ttagtatata tttatagttt tttatatatt tttaagaggt atttatttta ttattttttt    1320
   ttttagaatt tttataaatt ttatgtttcg taagtataat gaggtttttt gaaattatta    1380
   ttattattat ttgagatgga gttttgtttt attgtttagg ttggagtgta gtggcgcgat    1440
   tttagtttat tgtagttttt gtttttcggg tttaagtaat ttttttgttt tagttttttg    1500
   agtagttggg attatagacg cgtattatta tgttcggtta atttttgtat ttttagtaga   1560
   gacggggttt tattatgttg gttaggttgg ttttgaattt ttgattttat gatttattta   1620
   tttcggtttt ataaagtgtt gggattatag gtgtgagtta tcgtatttag tatgattttt   1680
   attattatga taatgattat tgtttattat tattgagtag ttataatgta ttattttata   1740
   tatattaatg tcgaaaggat agtgaataaa gaaaaaaatg atgaatttag ttagaaagag   1800
   attaaaattt attattatta ttttttgagt taaagttttg ttttgttatt taggttggag   1860
   tgtaatggcg cgattttagt tggtcgtaat ttttgttttt tgtgtttaag cgattttttt   1920
   gtttttgttt tagtttttta agtagttagg attatatagg tatacgttat tatatttagt   1980
   taatttttgt atttttagta gaaatcgggt tttattatgt tggttaggat agtttcgatt   2040
   ttttgatttt atgatttatt cgtttcggtt ttttaaagtg ttggggttat aggtatgagt   2100
   tatcgtgttt agtttaaaat ttttttaaat tatgaaaaat taaaattaaa agaataatag   2160
   attaggaaaa tatttatata tagtatgaaa gagtttattt aaatgtaata gaaaattttt   2220
   aggtttttaa tagtttaaaa gggaaagatt attaaagaga gaaatataaa ttattaataa   2280
   gtatatggga aaaatattaa tagtagttaa aaatgtattt ttaatatagt tttattgttt   2340
   tttagattta ttaaattggt tttgttattt tatgtttagt aaattttaaa attattaata   2400
   gtattattta tattattaag tttgttgtga tattgtttta ttgatataat atttttggaa   2460
   agaaaatatg ttttttaata tgttagagta ttgtaattat atatatattt tatgaattag   2520
   ttattttagt gttagaaatt tattatgagg tagtttttta aaatattgga gggtaggggg   2580
   tattagaaaa gtatatttat gttttagtat tgaagtatta tatttaaaag gggaaaattc   2640
   gaaaaaatta aattgtttaa taattgggaa ttggttaagt aaattatgat ataataaatt   2700
   aatgaaatat tttgtagata ttagaaatta atgtttaatg ttaatatgaa atatgtgaga   2760
   tagtgttgaa aatatttata atatgttata ggaaaaaatt tttgtgtatt ttaattataa   2820
   ttgtatataa tgtttgtatg tggatatgtt agggtgatgg gagtgatttt tttttttttt   2880
   aaaaatgtta tttttaaata tatttttttt aatgatgtta tttgtatttt aaaattatta   2940
   tataaaaata aatatataaa taaggattta atattttatt agtaatgtag tagtaaataa   3000
   gaatagttta ggttttatta tttttgggaa tggtgatagt ttgttaaatt tagttatttt   3060
   ggttataaga aaatttaaag tataaaataa tatatagtat taatagttta aagaaaatta   3120
   aaattttaga atttttaaag atataggtag tcgggcgtag tgatttaagt ttgtaatttt   3180
   agtattttgg gaggttgaga tgggaggatt gtttgagttt tggaggtcga ggttgtagtt   3240
   agttaagatt gcgttattgt attttagttt gggtgatagt aagattttgt tttaaaaaaa   3300
   taaataaata aataaaatat acgtaaatat tattatgtta tttttatttt tttaaatatt   3360
   attatttatt tatttattta ggaaatattt attgagtatt ggttatatgt taggtattat   3420
   ttaaagtatt ggaaatatag tggtgaagaa agtatatagg atttttgttt ttattaagtt   3480
   tatattttta cggtagatat agataataaa tagataagta agtaaatatg taatgggatg   3540
   tttagttatg aaaattatta tgaaggtaat aaagttgggt agggagatag agagtggtag   3600
   tagtattttt ttagttgggg tgattaagga agattttttt gaggaggtaa ttttggaata   3660
   gaggtttgag taaagggaag aagtaagtta ggtaaatttt tggcggaaaa ttattttagg   3720
   tagagggagt agtaagtgta aaggttttga gggtggaatg tgtttagttt atttaaggag   3780
   gttggagtta tgagagtacg gagggattta tagatgagat ttgagaggcg gttagaggtt   3840
   aggttatgta gggttttgag gattatagtt aggattttgt gtttttgttt ttagggtaaa   3900
   agggtagttt ggagggttat gattaggtta agatatgatt taatttaagt ttttatttta   3960
   ttttattttt ttgagataga gttttatttt gttatttagg ttggagtgta gtggtacgat   4020
   tttattttat tgtaattttc gtttttgggg tttaagtaat ttttttgttt tagtttatta   4080
   agtagttggg attataggta tgcgttatta cgtttagtta attttttgta tttttagtag   4140
   agacggggtt ttattgtgtt agttaggatg gtttcgattt tttgatttcg tgattcgttc   4200
   gtttcggttt tttaaagtgt tgggaatata gatttgagtt aatgcgttcg gttttttaag   4260
   ttttaaaaat atcgttgtgg gggttatgtg gaaaataaat tttagtagat aaaggatgga   4320
   attatggagt ttggtaggag gttattgata gagttaagtg agagaggaag gcggtttaga   4380
   tgtatagggt attatagtat tttaaagggg tagttagggg gatttgttga tggatggatg   4440
   tgggttatga gagataggag tttaggttga tttttaaggt tagagaatta gtatttaagt   4500
   gttatatatt tttgtttgat ttgagggttt tttatttttt attaaatgat tttagagttt   4560
   taagtaaaaa tttaaaagat atagaaaatt ttgttttaaa atttatttaa ttaaaggata   4620
   tatatgaata tttgagatag tattattttg ttgttagatt tttatatttt ttggaaatgt   4680
   ttttgttaaa gtgaatattt agttaattat ttatatatgt ttttgtgtgt ttatttattt   4740
   ggttttaaat tgatcggatt attatttaga gtaagaggat gataaagaaa aaaaaaaaaa   4800
   agaaaaaaaa ttttttgatt taattgtatg atttaatttt taaaatggtg gttattggga   4860
   aagtattaat tttgattgta tattttattt tagaagggta aagaattatt tatttaaatt   4920
   ttttggaatt ttttacgatt ttcgtagttg tatagtattt gatagtttgg aaagtattgt   4980
   tttataaaaa gtgttttata attttttagt ttttttttaa aggtaggtaa gattgttttt   5040
   attaaatatt atatatataa taaattatta tatgttatgt tatattatta tatatattat   5100
   ataaggaatt tgagttttag agtattttag taatttattt aaggttatat ttttagtgag   5160
   tgagaaaatt agagtttaaa tatttagttt tagtgttggt tcgttgaatt attttattag   5220
   taaatattgt tattttttat gttaggtatg aggtatatag tgatgtatta agtagatata   5280
   atttttttta tgtggagttg agtgttaagt atttttttta tttttttagt atatgtagag   5340
   gagaatgttt tattgaaggg ggaggaagta agaagattta tttggtttaa aaggaagggt   5400
   gattttaaga gaagagatat gagttttatt ttatgaatat tttaatggtt tttaagaatt   5460
   attttttttt tatgaatgtt ggaggtagtt aaatggtatt agtttttttg ttagattttg   5520
   tgagagtata tttttttagt atttttaatt aggagagcgg ataggaggaa attatggtaa   5580
   atatggaaga ggataaaaag agagatgaga agttataaat aaaagaaggt aggaaggtag   5640
   gaaagtagga aggtaggaag gtaggaagga aggaaagata attgataaaa aggagaaaga 5700
   gaataacgaa agagattatg agttttatta ttgatagatt agtttcgttt ttttttatta 5760
   ggggatttgt tttatatggt aaataaaaat tttaaatttg tattgagtat ttatttttag 5820
   gaattgtttt aagtgttttg tatatattaa ttaatttgat ttttatatta gttttaggat 5880
   ataggttgta atattttttt gtttttatag atgaggaaat aagacgtagg gaagttaagt 5940
   aatttgtata atttatatag gtagtaagta ttcgagttta gaaatgaagt taggtttgtt 6000
   tgattttgag ttggtgtttt gaatatttat gttgtgttta tatatcgttt tttttttttt 6060
   ttttttttga gatggagtta ttttgttgtt taggttggag tggtataatt tcggtttatt 6120
   gtaattttcg ttttttaggt ttaagtgatt tttttgtttt agttttttaa gtagttggga 6180
   ttataggtat tcgttattag gttttgttaa ttttgtattt ttagtagaga tagggtttta 6240
   ttattttagt taggttgttt ttgaattttt gattttaggt aatttattta ttttagtttt 6300
   ttaaagcgtt gggattatag gtatgagtta ttgtattcgg tatatatcgt attgttgatt 6360
   taaagttttt ataaagtttt tgggtaattt taatatttaa ttaaattata gattgtatgt 6420
   gtttatattt tgataaagtt gtaagtgttt tttttatttt tatttttttt tgtgggttat 6480
   tgattttatt agtataggtg tgtgattgtg ttattttttt tgaaatgaat tatttaagag 6540
   taaatttatt tatttaagta gatttgttaa tattattatg aaaagatgat aatagttttt 6600
   tatgttagtt tgtattgatt taaaaaagta ttgtttatcg ttgatagaat taatgtttta 6660
   tttgagttgg gaaagcggta tggagattgt tttgaaatga tatttttaag agtatttatt 6720
   ttatgtttat aatttgaatt atttaggtgt tttttttaaa aaaaaaaaag agtttggaaa 6780
   ttttagaaag gttgagtgtg tgtacgtgta ggtaatgtgt gtatttgtat ttatattcgg 6840
   gagaggttta ttcggttata tttatgttta tatttgtatt atttttgaaa gatgagggta 6900
   ttattagaaa tttttagaga taattttata ttttttgtta atagtgagtt ttaggagttt 6960
   ataaaattta ttgtggagaa gttaaatttt taattaatat ttatttattg tttgtgtgtt 7020
   ttaagtatta tgttaagcgt tggaaatata atttaaattt aaatagagtt tgtaaattag 7080
   tagagtattt ttttgtaggt ttatttgtgg ttttttgtgt tagagaatat ttgtttttat 7140
   ttgaaattta tatagtgtag ttgaattttt attagttttt aagtatagtt ttttatagtt 7200
   aatcggaaga aaatatgata aaatatttta tatgattcgt ttagttttag ttaggtttag 7260
   ttagaaagaa ttaagtttta ggaagaaggt gattgagaaa ggggagtagt tgtagtaagt 7320
   tttattatgt aggaaggttt taaataaaat gaaaaaggta ttaagtaaaa gattaataaa 7380
   tttgattgta ataaattttt aaatatttat aatgattaaa gatattatta gcgttaatta 7440
   gtaaattaaa agatatttgt tatatatata aaaataattt gggtgtatgg tttacgtttg 7500
   tgattttatt attttgggag gttaaagggg gaggattatt cgaggttagg agttcgagat 7560
   tagtttgatt aatatggtga aattttgttt ttattaaaaa tataaaatta gttgggtgtg 7620
   gtggtatatg tttgtaattt tagttatttg ggaggatgag gtaggagaat tgttcgaaat 7680
   taggaggtag aggttgtagt gagttgagat tatattattg tattttagtt tgggtaataa 7740
   gaatgaaaat tttgttttaa aaaatttaaa taataataaa aataataatt tgtatttaga 7800
   atatttaaag aatttttata aattaataag aaaaagataa aataaaagga aaatgggtaa 7860
   agggtgtaaa tagataattt tttgtattta taaatgatta gttaatatat gaaaagatgt 7920
   ttaaaatgta aaaaaaaaat taattttatt agaaattaag aaaataagaa ttgaaataag 7980
   ataaatttta tatttttagt ttaataaatt aagaaaatta tgatgaattg tagtaattta 8040
   ttgttagtta gttggggtag gagaatttcg gggtatattt taacgagttg ttgatgggag 8100
   tgtaatttgg tagagttatt ttggagggta aggatttaat aaattaaaaa atgtatgtat 8160
   attatgattt agtatttata atttaatatt tattttagaa aattatatat acgtataagg 8220
   gaagatttac gtaaaatgat atttaatgaa gtattgtgtg tgatagtaaa ttcgaaataa 8280
   tttaaatagt tattagttag aaatggttag gtattttata gtatttatat ataatagaat 8340
   atatatagta tttttaataa tattatttta aataatttgg ggtaaaaaaa taagtggtag 8400
   tattagtatt atgtattatt tttatatata tatatgtaaa aatatgtatg taaaatttat 8460
   atgtatatat aaaaaattta tgtgtatatg taaaaaaggt atgtgttata tggtataata 8520
   tatatgttat gtaaaatata tatataagta tatatatata ttttatataa aatttttttt 8580
   ataaaaaatg ttttataaag aagtattgtt tattttttat ggatagattt agtaggtatg 8640
   ttttgtatgt aaaataattg ataatagtgg ttattttttg ggagaggtga aggtcgttgg 8700
   agagaggatt aatgaatatt taagtgtttt aaaaaaataa ttattaagta tattggaata 8760
   taaaggaaat tatatatatt tagttgtgtt aggtttttta ttaatatgag ttttaatata 8820
   ggtttttata tagttttggt ttttttaggt attgataata aagtaaattt ttatttgaaa 8880
   atttttgaat aaaggttttt gttggttata tagtatatta tgttttaatt aataaatttt 8940
   aatagttcgg aatttattat tagtaataat gtgttatata ttatatgtaa tgtaatttgt 9000
   tatatattaa tatatgttat tatttattat gtattagtaa attgaaaaat attaatttgt 9060
   aagcgatgaa tttataaaaa tttatatttg ttttttgtaa tttataaata tttaaaaata 9120
   atagtatttg atagtaattt tttgtgttga ttgtagaaat ttttgtattg tttgtattgt 9180
   tatgataatt ttagtaaaag ttaagtatta gattagagtg tgtgtatagt tgattgtgaa 9240
   gttgtatgag agaggtgttt tattaatttt taaagtttat tattgtaatt tatattgtta 9300
   agaataaaaa ttgaaaatat aagtgttttt taattataaa gtttaagttt aaggatttat 9360
   tggaaataag ttagaaagta tatttttatt ttaaaaatta aaataaatta tttaaggaaa 9420
   atagtatttt taggtaaagt gttttgttgt tgttttaatt agtaaggaaa tttttaattt 9480
   ttattttata ggaaagaata atgaagaatt tttttattat atatatttag ttttcgtttt 9540
   gttttattta tttttttgtg ttatttatta tgaatttcgt aataaaatat gaaatttttt 9600
   tataggtatt tatattcggg gtaatttgtt gtcgtagaag ttttttgatg agaatatttt 9660
   taaatgtaaa tatggttata attttttttt agttaatgat gtcgttttta aatttttttt 9720
   ttattatttg gttgaatagt gttttggtag tgtttacgga tataatatta atattgttta 9780
   agtatgattt tagatttttt tttatttttt tttggattga gaacgtaata ttttgtagta   9840
   ttttggacgt tttgtttgga tttgatttag gttgtggtat ttgtaggacg tattgtagat   9900
   agttttgagt tagtttgtaa atatatttaa atttatagtt tgttattttt tgtttggtgg   9960
   agagtaaagt tttgagttgg tttattttga agttgttgag gtaatgtgtt gagaatgttt  10020
   attgagtttg attgagttat gatatatgat gatatatgga ggttggtgga atttttgttt  10080
   gtattatttt ataatatttt tttttttagt tattttcgtg cgtaggttgt gttttatgtt  10140
   atgtatgaaa gagaaagttt aaaatgtgaa agaggaaaat gttttagaga agttattttt  10200
   ttattttgtg attaatttag ttttgtaagt tttaaatgta ggatgtggag aaattagaat  10260
   ttttgtagat tgttggtggg aatgtgaaat ggtataattt ttgtgaaaat gttggtagtt  10320
   ttttaaaaag ttaaatatag aattgttaaa agatttagta attttattta taggtatata  10380
   tttaatagat atttaaatat ttatatacga atgtatatgg tagtattatt tataatagtt  10440
   aagatgtgga tataatttaa atgtttatta atagacgaaa ggataaataa attatggaat  10500
   attatttatt ttaaaaaagg aatgaagaat tgatatatgt tttaatagga atgaatttta  10560
   aaaatattag gttaatagaa agaagttaaa tagaaaaggt tatatattat gtgattttat  10620
   ttttatgaaa tatttagata gttaaattta taggatagaa agtaaattgg tggttgatag  10680
   gggttgagaa gaggaaggag taattgttta atgggtagaa ggtttttttt tgtgtgatga  10740
   aaatgtttta taaggtcggt cgcgttggtt tacgtttgta attttagtat tttgggagga  10800
   taaggcgggc ggattgtttg agtttaggag tttgagatta gtttgggtaa tatggtaaat  10860
   tttcgtttgt attaaaatat aaaaaattag ttaggcgggt ggcgtgtgtt tgtaatttta  10920
   gttattcggg aggttgaggt aggagaattg tttgaattcg ggagatagaa gttgtagtga  10980
   gttaagatta cgttattgta ttttagtttg ggtaatagag cgagattttg tttttaaaaa  11040
   aaaaaagaaa agaaaatgtt ttgtagttag atagaggtgg aagttgtata atattgcgaa  11100
   ggtattaaat gttattgaat tgtttatttt aaaatggttt tgtgaatttt aaatttaaga  11160
   aaagttttaa acgttattta cggggtaaat cggagtaacg ataatttgga atggagatta  11220
   taaaatagtt tgggtggtag agaatagggt ttttcgaggt gattttggta gtatcgtttt  11280
   tgattatttg taattgagta aggatgatat cgagagggaa gaaaaaaata ttttttttgg  11340
   gttaggtacg gtggtttatt tttgtaattt tagtattttg ggaggttgag gcgagcggat  11400
   tacgagatta gaagatcgag attattttgg ttaatatagt gaaattttat ttttattaaa  11460
   aatataaaaa attagttagg tatggtggcg ggtatttgta gttttagtta tttgggaggt  11520
   tgaggtagga gaattttttg aatttaggag gcggagtttg tagtgagttg agattgtgtt  11580
   attgtatttt agtttgggcg atagagcgag attttatttt aaaaaaaaaa aaaagaaaaa  11640
   gaaaaagaaa atattttttt tggtttttat tttttattta ttttagaatt tttgatagaa  11700
   aataaaattt tgttttaagt ttgatttaat ttatattttt aatttaggta aattattcga  11760
   tgtgtggatt agaaattgta ggattagaaa gagtattgga tttatagtta gagggtttgg  11820
   atttagtttt agttaaattt tttttttttt atttagtatg tattttgggt ttgtgtttaa  11880
   tttgtaaaat gaggtaatat atgttgattt tagagggttg aggtgaagtt taaagagatg  11940
   atttatgtaa agtttttatt attatgtttg gtaaaacgta cgtgtttaat taaagttagt  12000
   tttttattgt ttttttgtgg attaatgagg aatttggttt tttaattatt tttttattta  12060
   ttaaggatat aattttatag gtgaaatgta aattttattt gatttagtta tggtagtaat  12120
   tttgagaaaa ggtttgtagt ggatggaaga aaaaggagat aatttatggt cgttggagaa  12180
   attgaattta tttttttttt ttatttatat atggagtgat tttgagtttt ttattttgtt  12240
   tatgattagt tgtaaatagg aaaaatgatt tatatataat ttgtattttt gaattattat  12300
   tatagggtta aggaggaaag gagaggtgtt gtttagaatg ttcgttaata gcgtggtagt  12360
   tatgaaaatg tattttttag atttattgtg gtggtttaat tgattaaggg ttttagtcgt  12420
   agtgttttga atttattata gttttgtatt gaggttatat attttatagg ttgtttttag  12480
   ttaatgattg agtaaagtag gaattttaag atcggttttt gtaagttaat tttttttgat  12540
   aggtaatttt gttttaatga ttttttatgg gttttgttga aatttttttc gattgtatag  12600
   ttatggaaga ttttttattt tatttttttt tttttgtttt ttgtagaggt tagttttgta  12660
   ttgtagtttg atagttttgt agtttttttt gttttttttt tttttttttt ttataggtat  12720
   ttattttagt aaatttgtta gacgtttaat ttttttatga tatttttttg gagtatttag  12780
   attgatatag gatttgtaga gtataaagga gtatatagat atgttgttaa gttatttttt  12840
   taggaatttt tttgggaaag ttttttcgtg ttttttagat tgtgttcgtt ggtttatagg  12900
   agtataaagt taagagtagg ttgttgtatt tttttaaatt atttttgagt attataagaa  12960
   ttagggaaag gatgggaaga ttgagttttt ttttttttat tatatttttt tggtgttgaa  13020
   tattttttta aaaataagag aagtagtcgg attcgttggt ttacgttttt gggaggttaa  13080
   ggcgggtgga ttatttgagg ttaggagttt aagattattt tggttaatat ggtgaaagtt  13140
   cgtttttatt aaaaatatag gtagtagagg tatatgtttg taattttagt tatttgggag  13200
   gttgagatag gagaattatt tgaatttagg aggtagaggt tgcggtgagt tgagattgtg  13260
   ttattgtatt ttagtttggg taatagagtg agattttgtt tttttaaaat aataataata  13320
   ataataataa tagaagtatg tggttttaaa attgtaatta agtatttttg gttgggtgtg  13380
   gtggtttacg tttgtaattt tagtattttg ggaggttaag gtaggtggat tatttgaggt  13440
   tagaagtttg agattagttt ggttaatatg gtgaaatttt atttttattt ataaaaaaaa  13500
   aaaagtataa gattaggtac ggtggtttat gtttgtaatt ttagtatttt gggaggttaa  13560
   ggtaggtgga ttacgaggtt aagagatcga gattattttg gttaatatgg tgaaattttg  13620
   tttttattaa aaatataaaa attagttggg tttggtggta cgtatttgta gttttagttt  13680
   tttgggaggt tgaggtagga taatcgtttg aatttaggag gtggaggttg tagtgagttg  13740
   agattgtatt attgtatttt agtttgggta atagagggag attttgtttt aaaaataaaa  13800
   ataaaaataa taataattag ttaggtattt tatttattta ataaagatta attatttgtt  13860
   tttattgggt tttggtttgg gtaatgggtg gaaatagaaa aaagatagat ttttgttttt  13920
   gtttttttga gacggagttt tgtttttgtc gtttaggttg gagtgcgatg gcgtaattta 13980
   ggtttacggt aatttttgtt tttcgggttt aagtgatttt ttcgttttag ttttttaagt 14040
   agttgggatt ataggcgttt gggaatattt ttggttaatt tttgtatttt tagtagagat 14100
   ggggttttat tatgttggtt agattggtgt taaattgttg attttaagtg attcgttttt 14160
   ttagtttttt aaagtgttgg gattataggt atgagttatt atatttagtt tagggtttta 14220
   tatttttaat tttttgtatg ttaaaaggag ttgttttaaa aatttttata tattgtggtt 14280
   aggcgtagtg gtttatattt ggtaatttta gtattttggg agtttaaggt aggcgaatta 14340
   ttttaggtga ggagtttgag attagtttga ttagtatggt gaaattttgt ttttattaaa 14400
   attataaaaa ttagttagat atggtggtcg gtgattgtaa ttttagttat ttaggagatt 14460
   gaggaagaag aattatttga atttaggagg cggaggttgt agtgaattaa gatggtgaaa 14520
   ttttattttt atcgaaaaaa tttaaaaatt agtcgggtat ggtggtggtt atttgtaatt 14580
   ttagttatta ggggtgttga ggtaggagaa tcgtttgaat ttaggaggtg gaggttgtat 14640
   tgagttgaga taatattatt gtattttagt ttggcgatag agtaagattt cgtttttnaa 14700
   aaaaaaaaaa anannnnaaa atttaagtat agttatttta ttttatttta atagaatata 14760
   cgaggtttaa atagaaattt tgtagtttaa ttaaaataag tttttaaatt aatgaagttt 14820
   ttttatgtta tattttttat agagattatt aagtatatta taatgaagaa attttatttt 14880
   ttttgattta attttgtaat attattaggt aatattgtgg agtt 14924
<210> 23
   <211> 7231
   <212> DNA
   <213> Artificial Sequence
<220>
   <223> chemically treated genomic DNA (Homo sapiens)
<400> 23
   ttaattattt taagtaagtt tttaaattaa gttgggcgta tatatagagg tttaaattta 60
   gagatttaag tgaaggtttt tattagagta tgtggttgaa agttagttag taaatatttg 120
   ttgagtattt attttgttag gatttgtgtt aggtattgga taatagagag atgaaagaga 180
   attttaggaa tttagagtta gtatgtgaat aggattgtag aataatatag gagttgttat 240
   agatatgtaa agggttttag gtatattatt gttgttgagg tgtttttatg gatataggta 300
   ttatatttag tggtgtatta gagaattgat agatttgggt ttttagtatt ttggattttt 360
   gaagaaattt tttttgtttt atgtgagtga tattaaggtt attaggaagt aaaggggagg 420
   ggtatggttt tttatggatt ggttttgtat taggatttga tttttaaaga ttattgttta 480
   taggattttt tgttaagatt ttgagattgt taaagggttg ggtgagttag attagtatag 540
   tttttaggaa attaaatttg tggatagtat ttagttagtt gggttattta gttggtgagt 600
   tgtttagtta tagtatagtg gagaatttga aagaaagaaa atggttggga aatgttataa 660
   tattttagtt aaaaggttta gagtaggaag aaggtgttag aaagttttag gggagggagt 720
   ggtatttttt aagattttgt taggtgtttt tttgttatag gaaatttagt tgttattttt 780
   aagcgtttaa tgaggttggt ggttatgttt attttatagg agataagatt tagatgagga 840
   gattagtttt agatttattt tatatgatta tagagtttta atttaatttt agtcggtttg 900
   ttgtattata gaaataattt gtatttattt ttttgggatt aggttaagtt ataggtaata 960
   tttggaagga tgtttttgta gcgggggata aaggttttag aatttaaaaa ttaaaaagaa 1020
   agttaaaatt tagtagataa ataaaagtta gtttaaggat tagtgggtaa aggtgatagg 1080
   ggaagtaaaa tagagttgag aggaattagt gattgggtgt aatgattatt agattgtata 1140
   tttgtttttc gaagatggtt tttagtttaa ggtttaggat tgggtttttt tattggaagg 1200
   aagtatattg gagggtagag aaaaagggag tttatgtttt tttttttagt tgtagtataa 1260
   gagatgtaat tggggattgt tgattgtata tgaatattta ataagaagta tgatgtgaac 1320
   ggggttttaa gggtacgttt gtaggtttgt tagttgttgg ttttggggtt tttttgttgt 1380
   tttaaatagg gggtttttat gtgagtattt aaaatattgg agtgatttga agaaagtaaa 1440
   ttttagtttt tttaaaaggt tttaatttga aaatattatt aggaataaat ttaatagaaa 1500
   atgtatataa ggtttaggat aaagaaaatt ttgaaagtat tgtgaaggat atagaaggaa 1560
   attggaataa attgaaagat atattaagtt tttatatagg agtagtgata tagaattaat 1620
   agtggtaagt taaattaaaa atataatatt gttaaaaaat gttattttaa gaaaatatat 1680
   taagagggtt taaaaaaaaa gtttatatag ggataaaata gttggaagat gttggtaggt 1740
   agtggggttg agggggtagt gatgtagggg ataaggggaa gagttattta attttagtag 1800
   atattaaaag tcgttataaa attataatag ttaaaattaa ttttgatata tgaataggtt 1860
   agttaagtag aatagaaggt ttaaaagtag atttaaatat atacgataat ttagtatggt 1920
   aaatgtggta tttagaatta gtgagtaaaa ggtagatttt ttattaaata gtattggaat 1980
   aattaagtaa ttttcggaaa aagtaaaatt gggtttatat tttatattat aagttaatat 2040
   atattttaaa atagataatg atttaagtgt aaaaaaaaaa aaaaagttat aaaaaattat 2100
   ttataaaaat attataagga aatgtgggta atttttttaa atttttgtaa tttggattac 2160
   gaatttagga attataaacg aagattgata aatttaatgt aaaataagat taataaaaat 2220
   tataagttaa attaaagttt tatagatttt ttaaggttgt agatgttttt aaaaaaaaat 2280
   gaaaatagaa aagttttgaa atttattttt aaggattaat ttttttaata tatagagttt 2340
   ttggaaacgg atatgaataa aattaataat ttaatagaat aatgagtaga taatagaata 2400
   aatgtaaata gttttgaagt ataggtagag ttttttagtt ttttttatga ttagaaaagt 2460
   ataaattaaa gaaggtattt ttatttaatt agattggtaa aatttttaat gtttgttaat 2520
   ttattttgtt gataaggtta cgggaaatag gtatttatgt tatgttgttg gagggagtat 2580
   ggattgatat aatttttatg aagagtaatt tggtaatatg ttagaatttt taaatgtata 2640
   tattttttga tttagtaatt ttatttgttt tgggtaagtt attatttttt tttgtttcgt 2700
   ttttatttta tagagttgtt gtaagagtta agcgagatat atatgaagcg tttagtataa 2760
   agtttttggt atataggaag tgtttttaaa atgtgggtta gtagtagaat aatagtagtt 2820
   aacgggtatt gagattttat tatatgttat attagtatat tgtgtttggt gttttttatg 2880
   tattttttta tttaattttt taatttgtga agtaggcgta attgattatt attagttagt 2940
   tttcgaggag atagaggtat aggtatgcga agtgtgtggt ggataggatt tgagtttagg 3000
   tagatgttaa gtttgtgata tattgttttt ttattaggtg tagtggggtg atgaatattt 3060
   tacgagaggg tgtgtagagg gttgtaggga tatagataag tatatagttt cgtttataat 3120
   aggtggggat tatattgatt ttttggggaa aagatggttt ttagtagagg ttgtgttatt 3180
   ttttttgttt tttttttttt tttttttttt ttttttgaga tggagttttg ttttgtcgtt 3240
   taggttggag tagtatagtg gggcgatttc ggtttattgt aagttttatt ttttgggttt 3300
   atattatttt tttgttttag ttttttaagt agcggggatt ataggcgttc gttattacgt 3360
   tcggttaatt tttatatttt tagtagagac ggggttttat cgtgttagtt aggatgattt 3420
   cgattttttg atttcgtgat ttattcgttt cggtttttta aagtgttggg attataggta 3480
   tgagttatcg tgttcggttt aggcgtgttc ggtttaagtt ttgttatttt taagataggt 3540
   aattttgtaa tagggttgat tgttttttgt tttaatttat tttaagtttt atttttgatt 3600
   tgtgataata gtgtagtata attttaaggg tggttgtgag gattaagaga gatgaatatg 3660
   gtcgggtata gtggtttacg tttgtaattt tagtattttg ggaggtcgag gcgggtggat 3720
   tacgaggtta gttcgagatt aatttgatta atatggtgaa tttcgtgttt atgaaaaata 3780
   taaaaattag ttgggtatgg tggtacgtat ttgtaatttt agttatttag gaggttgagg 3840
   taggagaatt gtttgaattt gggaggtaga gtttgtagtg agttaagatt gtgttattgt 3900
   attttagttt gggtaataga gcgagatttc gttataaaaa aaaaaaaaaa aaaaaaaaag 3960
   agagagagat gaatatttaa gttttaagtt tttaggtttt tataatttat gtttgttgaa 4020
   gtgattcgaa taaatgtaga tggagtgggg tcgagtgggg gaaacgtttt ttagggagag 4080
   ggaatattat attttaaggt tatatattta gaaggagttt gagtaattta ggaagggcgt 4140
   agggtgcggt ggttgatggt cggggtggtt gatggttttg aggattagtt aagaaatttg 4200
   gattatttat taataaaaat aaaagagtta ttatttgttg agggtttgtt ttgatatttt 4260
   tattaaaatt attttagtaa ggtagattta ataaatttag ttgtatataa aagggaaata 4320
   cgagaagtta tagaagggtt gtatataggt aggtgatttg attaatttcg tgttttataa 4380
   agatttttta gattatgtgt tggagtgata ggaggtattt tatcgatttg ttttgaattt 4440
   tatattttaa aaaatatatg acgtttatta tttttttttt tattttataa tttatattta 4500
   ttgtagaaaa tattaaaaat ataagaaagt ttgaagaaat aaaaaattat tattggtatt 4560
   ttttagagat attttaattg atattttttt tttagttttt ttttttttat gtaaaaaata 4620
   tatagttatt agaatgtttg atagaaggaa gatggaaaga gattattgtg aagcgttttt 4680
   gatatttttt tttttttttt tttttatgtt attagggtta tattaagagg agaggttgat 4740
   ttttttatac gtgttaggga aagttttaat tttgcgtagg gattttcgtt tgttattttc 4800
   gtcggttttt agggggcgtt gtttcgagat tgggtgggga gggaaagaag gtggtgcgaa 4860
   aacgcgtttg aatttgggtt ttgtcgttgt tcgtagtcgg cgtttttaat atggcggttt 4920
   tttaagacgt ttacgttcgg atttgtaatt aagagattgt taaatttgat ttggaggtga 4980
   aggcgtttat ttaggtattg atttcgggat ttttgtcggg ttttagtagt tttaatttaa 5040
   gttttgttgg tttgtgagtt tggtagtgtt attttcgaat tttttttatt tggtttttta 5100
   gattataggt ttcgttttat ggtcggttat tttttcggtt tttatttggt tgtgtttcgg 5160
   tgattgttgg ttttgatttt tttttcgggt gtcgtcggat tttttatttt tagagtattt 5220
   tattgttttt cgtttttgat ttagttattg tttcggggtt cggttttgtt ttttttattt 5280
   gtagttttgg tcgtcgtttc gtttttgatt attttataat ttatttgtta ttttgtttgg 5340
   tttcgttttt tgacgttgtt ttagtatttt ttggtagcgt tcgggaggta gttagtttcg 5400
   ttttttgttg gttagttttt ttggtattta aggttatttg gtacgggagg tatcgggtga 5460
   aacgaatgta atttggatcg tagttgtgat tttatgtttg aggaaatcga gtacggcgtt 5520
   aaggtttagg ttagaaggta tatttgggtt agtggtaaaa ttgggatttg gattgtgaaa 5580
   gttaatttag gaataatatt ttagagtgtt atttcgtttg aatagatagg aaatgaagtg 5640
   tttcgtttta agttatttaa gggtagaggt aaaataaacg agtggattta ggtaagaatt 5700
   gggagtgtag atacggtttt atgagtttag taaaaagaaa tcgagtttta aattttgttt 5760
   ttgtttgttt gtttttgttt ttttgagacg gagtttcgtt ttgtcgttta ggttggagtg 5820
   tagtggcgcg atttcgattc gcggtaagtt tcgtttttcg ggttttcgtt atttttttgt 5880
   tttagttttt tgagtagttg ggattatagg tattcgttat tacgttcggt taatttttgt 5940
   gttttgttta gtagagacgg gattttatcg tgttagttag gatggtttcg attttttgat 6000
   ttcgtgattc gttcgtttcg gttttttaaa ttgttgggat tataggcgtg agttatcgcg 6060
   ttcggtttaa attttggttt ttaaaaataa ttatttttaa taaagttatt aaatattaga 6120
   tgtagattta gagtttgtaa taaggtgtgg taaaaagtgt aggaaaatag ggttttttag 6180
   ataaattaga tgtagaataa attaaattag atgtagaata aataaatacg gtgtaaagaa 6240
   gtgtgattaa atgaataagt tagagaaaaa gtgaaatttg atgtagatat ttaaaatttt 6300
   ttgaatttta tttttttgat gtatatatta taaaattttt gggttaatta tagaaaaata 6360
   aataggtttt atataaatga aattatattt ttatataatt taaattgttt aggtattttt 6420
   ttgtattttt tttaatattt gggtttttgt tattgttgat gagaaagatt gattatagtt 6480
   ggatgtattg cgtttttatc gttttttagt agttttgtta agtgttttat atatatatat 6540
   aatttatgat gttttatgag gtaagtagtg ttgtgataag gaaatttaga tatagagaat 6600
   ttaaataatt ttttaggtaa ttttatatag tttataagtt gttatagtta ggattcgaat    6660
   ttatttggtt tgtttttaga gtttgtcgtg aaagttggta ttagtaattt tgaatgttta    6720
   gtaatagatt ttaaatttgt ttttaaatat gtttatataa atattttaat ttattattaa    6780
   tggtttgtaa aattatattg gagaagttaa ttttattata tgatgttaga tatttaaata    6840
   ttttgatttt ttaagttgga aggatttaag gttttaaagt gatagtatta tttttgtatg    6900
   tttatttttg ttgggtatat aagttgtttt aagttgtttt ttattggtta ggtaatgttt    6960
   tgttaaaatt taggtattaa aaattaggtt aaagtagttg tgtttttttt taggttttta    7020
   tattaattta atattaattt aaaaaggaat gagaaaaatg taaatgataa tgtttttgtt    7080
   tgtgttgtag aggtaaatag taaagattag gatgaattat agttagtacg ttaatattag    7140
   ttggtttaaa ttaattttat tattgtatgg atggtgttat tttattttat tgttttgaga    7200
   aaggtttttt atttgtttaa tgttttttag g                                    7231
<210> 24
   <211> 7231
   <212> DNA
   <213> Artificial Sequence
<220>
   <223> chemically treated genomic DNA (Homo sapiens)
<400> 24
   tttaggagat attgaataga tgaaaggttt tttttaaggt aatgaaatga aatgatatta      60
   tttatatagt gatagagttg gtttagatta attaatatta acgtattaat tataatttat     120
   tttgattttt gttgtttatt tttgtaatat aagtaaaaat attattattt atattttttt     180
   tatttttttt tagattaatg ttgaattaat ataaaagttt agaaagaaat ataattgttt     240
   tgatttaatt tttagtgttt aaattttaat aaaatattat ttggttagta ggaaatagtt     300
   taaaatagtt tgtatgttta ataagaatgg atatgtaaaa gtgatattat tattttagaa     360
   ttttagattt ttttaatttg gaagattagg atgtttaaat atttggtatt atatgatgag     420
   attaattttt ttagtgtgat tttataaatt attagtgata agttagagtg tttatataga     480
   tatgtttaag ggtagattta gaatttgttg ttggatattt agaattattg gtgttagttt     540
   ttacggtagg ttttggagat agattaggtg gattcgaatt ttggttgtag taatttatga     600
   gttgtgtaaa gttatttgga aagttattta aattttttgt gtttgagttt ttttattata     660
   atattattta ttttatagaa tattatagat tatgtatgta tgtaaagtat ttagtagagt     720
   tgttggaaaa cggtaagaac gtaatatatt tagttataat tagttttttt tattagtaat     780
   gataagaatt taaatgttag aaagagtata gaagaatgtt taagtaattt agattatgtg     840
   aaaatgtaat tttatttgta tgaaatttgt ttattttttt ataattggtt tagaagtttt     900
   gtaatgtata tattagagaa gtgaagttta agaagtttta aatatttgta ttaaatttta     960
   tttttttttt ggtttattta tttaattata tttttttgta tcgtatttgt ttattttgta    1020
   tttaatttgg tttattttgt atttggttta tttggggagt tttatttttt tgtatttttt    1080
   gttatatttt gttataaatt ttgagtttat atttaatgtt tagtgatttt gttgaaaatg    1140
   attattttta aaaattaagg tttaggtcgg gcgcggtggt ttacgtttgt aattttagta    1200
   atttgggagg tcgaggcggg cggattacga ggttaggaga tcgagattat tttggttaat    1260
   acggtgaaat ttcgttttta ttaagtaaaa tataaaaatt agtcgggcgt ggtggcgggt    1320
   gtttgtagtt ttagttattt aggaggttga ggtaggagaa tggcgggaat tcgggaggcg    1380
   gagtttgtcg cgagtcgaga tcgcgttatt gtattttagt ttgggcgata gagcgagatt    1440
   tcgttttaaa aaaataaaaa taaataagta aaaataaggt ttaaaattcg gttttttttt    1500
   gttgaattta tggaatcgtg tttatatttt taatttttgt ttaagtttat tcgtttattt    1560
   tatttttatt tttaaatggt ttaaggcgaa atattttatt ttttatttgt ttaaacgggg    1620
   taatatttta aggtgttatt tttaggttga tttttatagt ttaggtttta gttttgttat    1680
   tgatttaggt atgtttttta atttaggttt tggcgtcgta ttcgattttt ttaagtataa    1740
   aattataatt acggtttagg ttatattcgt tttattcggt gtttttcgtg ttaggtggtt    1800
   ttggatgtta ggagagttgg ttagtagggg gcggggttag ttgtttttcg gacgttatta    1860
   gaggatgttg aagtagcgtt agagggcggg gttaagtagg gtgataggta ggttgtgagg    1920
   tagttagggg cggggcgacg attagggttg taggtggaag gggtaaagtc gagtttcgaa    1980
   gtagtagttg agttaggggc ggagaatagt ggggtgtttt ggggatggga gattcggcgg    2040
   tattcggaaa aaggattaag gttagtagtt atcgggatat aattaaatgg ggatcggggg    2100
   ggtagtcgat tatggggcgg agtttgtggt ttgggaaatt aagtggggaa agttcgggag    2160
   tgatattgtt aagtttatag attagtagag tttgggttag ggttgttgga gttcggtagg    2220
   ggtttcggga ttagtatttg aataagcgtt tttattttta ggttaaattt gataattttt    2280
   tggttataga ttcggacgtg gacgttttgg ggagtcgtta tgttggggac gtcggttacg    2340
   ggtagcggta ggatttaagt ttaaacgcgt tttcgtatta tttttttttt tttttatttt    2400
   agtttcgggg tagcgttttt tagagatcgg cgaaaatggt aagcgagggt ttttgcgtag    2460
   agttgggatt ttttttgata cgtgtaggga aattagtttt tttttttggt atggttttaa    2520
   taatatggag aggaagggaa gagggaatat taagaacgtt ttatagtggt ttttttttat    2580
   tttttttttg ttaggtattt taataattat atatttttta tatagaaaaa aaaagattgg    2640
   aagaaaagta ttaattgaga tgtttttgga aagtattagt aataattttt tgttttttta    2700
   aattttttta tatttttaat attttttata gtgagtataa attgtggagt aaaagaggaa    2760
   atgataaacg ttatatattt tttaaagtat ggagtttaag gtaggtcggt agagtatttt    2820
   ttattatttt aatatatgat ttaagggatt tttgtaaaat acgaaattga ttaagttatt 2880
   tatttgtatg tagttttttt atggtttttc gtgttttttt tttgtatata attggattta 2940
   ttaaatttat tttgttaagg tagttttgat ggaaatgtta gagtaaattt ttaataaatg 3000
   gtggtttttt tatttttgtt agtaaataat ttaagttttt tagttggttt ttaaggttat 3060
   tagttatttc ggttattagt tatcgtattt tgcgtttttt ttggattgtt taagtttttt 3120
   ttggatgtgt agttttagaa tgtggtgttt ttttttttta gaaagcgttt tttttattcg 3180
   attttatttt atttgtattt gttcgggtta ttttagtaag tatagattgt gaggatttaa 3240
   aggtttagga tttgaatgtt tatttttttt tttttttttt tttttttttt ttttttgtgg 3300
   cggagtttcg ttttgttgtt taggttggag tgtaatggta taattttggt ttattgtaaa 3360
   ttttattttt taggtttaag taattttttt gttttagttt tttgagtagt tgggattata 3420
   ggtgcgtgtt attatgttta gttaattttt gtatttttta tagatacgga gtttattata 3480
   ttggttaggt tggtttcgaa ttgatttcgt gatttattcg tttcggtttt ttaaagtgtt 3540
   gggattatag gcgtgagtta ttgtgttcga ttatgtttat tttttttaat ttttataatt 3600
   atttttgaaa ttgtattata ttattattat agattaggag tagggtttag agtagattaa 3660
   aataaaaaat aattaatttt gttgtaagat tatttatttt gaaagtggta aagtttaggt 3720
   cgggtacgtt taggtcgggt acggtggttt atgtttgtaa ttttagtatt ttgggaggtc 3780
   gaggcgggtg gattacgagg ttaagagatc gagattattt tggttaatac ggtgaaattt 3840
   cgtttttatt aaaaatataa aaattagtcg ggcgtggtgg cggacgtttg tagttttcgt 3900
   tatttgggag gttgaggtag gagaatggtg tgaatttagg aggtggagtt tgtagtgagt 3960
   cgagatcgtt ttattgtatt attttagttt gggcgataga gtaagatttt attttaaaaa 4020
   aaaaaaaaaa aaagaaaaaa gaaataaaga aagtggtata gtttttattg aaagttattt 4080
   tttttttaaa aaattaatat ggtttttatt tgttgtaagc ggggttatgt gtttgtttgt 4140
   gtttttatag ttttttatat attttttcgt aaggtattta ttattttatt gtatttaata 4200
   gagaggtagt gtattatagg tttggtattt gtttgggttt aaattttgtt tattatatat 4260
   ttcgtatgtt tgtgtttttg ttttttcgga agttaattgg tgataattaa ttgcgtttat 4320
   tttataggtt ggagaattaa atgagaaaat atatgaaaag tattaagtat agtgtgttaa 4380
   tgtggtatat agtaagattt taatgttcgt tagttgttgt tattttatta ttagtttata 4440
   ttttgggagt attttttatg tgttaggaat tttgtattaa gcgttttatg tgtgtttcgt 4500
   ttaattttta taataatttt gtgaggtaaa aacggggtag agaggaataa tgatttgttt 4560
   agagtaaatg gagttgttag gttaaaggat atatgtattt aaaaattttg atatattatt 4620
   aaattgtttt ttatagggat tatgttaatt tatatttttt ttagtaatat ggtataaatg 4680
   tttgtttttc gtggttttgt taataggata ggttaataaa tattggaagt tttgttaatt 4740
   tgattaggta gaaatgtttt ttttagttta tgtttttttg attatgaagg aggttgagaa 4800
   attttattta tgttttagag ttatttgtat ttattttatt gtttgtttat tgttttattg 4860
   ggttgttggt tttatttata ttcgttttta ggaattttat atattaggag gattagtttt 4920
   tagagatgag ttttaaagtt tttttatttt tatttttttt taaagatatt tatagtttta 4980
   aaaagtttat agaattttga tttagtttat ggtttttgtt aattttgttt tatattgaat 5040
   ttattaattt tcgtttatga tttttggatt cgtggtttaa attataggga tttaaaggga 5100
   ttgtttatat ttttttgtag tgtttttatg ggtggttttt tgtagttttt tttttttttt 5160
   tatatttaga ttattattta ttttgaaata tatgttggtt tatggtgtga gatatagatt 5220
   taattttatt tttttcggag gttatttagt tattttaata ttatttaatg aagagtttat 5280
   tttttgttta ttggttttgg atgttatatt tgttatatta aattgtcgta tatgtttgag 5340
   tttattttta gattttttat tttgtttgat tggtttattt atgtattaaa attagtttta 5400
   attattatgg ttttataacg atttttagta tttgttagga ttaaataatt tttttttttg 5460
   ttttttgtat tattgttttt ttagttttat tgtttattag tattttttag ttattttatt 5520
   tttgtgtaaa tttttttttt aaattttttt ggtatgtttt tttaggatga tattttttag 5580
   taatattata tttttaattt aatttattat tgttagtttt atgttattgt ttttgtgtaa 5640
   gaatttggtg tattttttaa tttattttag tttttttttg tgttttttat agtattttta 5700
   aagttttttt tattttaggt tttgtatata ttttttgtta agtttatttt tggtaatatt 5760
   tttaggttaa aattttttaa agaaattgag gtttattttt tttaggttat tttagtgttt 5820
   tggatattta tatagaagtt ttttgtttag gatagtagag aagttttaaa gttagtagtt 5880
   ggtagatttg tagacgtgtt tttgggattt cgtttatatt atgttttttg ttgaatattt 5940
   atatgtaatt aatagttttt agttgtattt tttatgttgt agttaaagaa gaaaatataa 6000
   gttttttttt tttttatttt ttaatatatt tttttttaat aaggggattt aattttaaat 6060
   tttaagttag aggttatttt cgaggaatag atgtgtagtt tggtgattat tatatttaat 6120
   tattgatttt ttttaatttt gttttgtttt ttttattatt tttatttatt gatttttaga 6180
   ttagtttttg tttatttgtt aggttttggt tttttttttg gtttttagat tttaaggttt 6240
   ttatttttcg ttatagagat atttttttaa atattgtttg tgatttgatt tgattttaaa 6300
   gagatgaata taaattattt ttgtagtgta gtaagtcgat tggagttgaa ttggagtttt 6360
   ataattatat aaggtaggtt tggggttagt ttttttattt gagttttgtt ttttgtaaaa 6420
   tgaatatgat tattagtttt attgggcgtt tgagagtagt agttgagttt tttgtaataa 6480
   aaaagtattt agtagagttt taaggaatgt tatttttttt tttagggttt tttaatattt 6540
   tttttttgtt ttgaattttt tagttgggat attatagtat tttttagtta tttttttttt 6600
   tttaaatttt ttattgtatt gtggttaaat agtttattag ttgaatgatt tagttaattg 6660
   gatgttattt ataggtttag ttttttaagg attatattgg tttggtttat ttagtttttt 6720
   aatagtttta agattttgat agaggatttt gtgagtagtg gtttttagag attaagtttt 6780
   gatgtagaat tagtttatgg gaggttatgt tttttttttt tgttttttga tggttttaat 6840
   gttatttata tgaaataaag agggtttttt taagggttta ggatattaag aatttaagtt 6900
   tattaatttt ttaatatatt attggatgtg gtatttgtgt ttatgaaaat attttagtaa 6960
   taataatgtg tttagagttt tttgtatatt tgtaatagtt tttgtgttgt tttgtaattt     7020
   tgtttatatg ttggttttga gtttttggag ttttttttta tttttttatt gtttagtatt    7080
   tagtataggt tttggtagag taagtgttta ataaatattt gttggttgat ttttagttat     7140
   atattttgat ggaagttttt atttaggttt ttggatttaa gtttttatat atacgtttag     7200
   tttagtttgg agatttattt agagtgatta a                                    7231
<210> 25
   <211> 11670
   <212> DNA
   <213> Artificial Sequence
<220>
   <223> chemically treated genomic DNA (Homo sapiens)
<400> 25
   tgttaaagtt tatgatttta taatataaaa tgtatatttt tagataattt taaatttata       60
   tttgtaagtt tttatgattg tagaaatatt tttataaagg atttgaatat gaatttttaa      120
   gattttttta tattggttag gcgcggtggt ttatatttgt aattttagta ttttgggagg     180
   tcgaggtggg ttgattattt gagaatagga gttttaggtt agtttggtta atttggtgaa      240
   attttatttt tattaaaaat ataaaaatta gtttgacgta gtggcgtgtt tgtagtttta      300
   gttattcggg aggttgatgt aggagaattg tttgaatttg ggaggcggag gttgtagtga      360
   gttgagattg tgttattgta ttttagtttg ggtgatagag tgagattttg ttttaaaaaa      420
   aaaaaaaaaa aaaaataatt tttttatatt attggttttg atgagttatt taagttagtg      480
   ttagtttttt ttaatttttg ttggatgaaa tgtatatttt ttttttttta gtaaatacga     540
   ttatgtttat atttgaacgt ttagttttat tttattgtaa attataattt agttattttt      600
   ttgaatgagt tgaattttta aagttttgtg taataaaatt ttttgataat taagtttagt      660
   tttttttttg aatggttata atttaattgt atatggaagt attttttttt tatttttttg    720
   aaatggagtt ttatttttgt tgtttaggtt gaagtgtaat ggtatataat aagtaattgt      780
   aaaaatattt taaattttgt ttaaaaagtt tggaaatata gggaatatag tgggtttttt      840
   tgtttgtttg ttttttgaga tagagtttcg ttttgttatt taggttggag tttagttgcg       900
   cgatttcggt ttattgaaac gtttattttt taggtttaat cgattttttt gttttagttt      960
   tttaagtagt tgggattata gatgtgtgtt attatattta gttaaatttt gtatttttag     1020
   tagaggtggg gttttattat gttggttagt ttggttttta attttagatt ttaggtgatt     1080
   cgtttatttt agttttttaa agtgttggga ttataggcgt gatattacgt ttggtttata     1140
   gtgtatattt aatatcgtat tttggattaa taattttatt tattttttta gagatattta     1200
   tttaagatag tatgtggaag ttaaaggaaa atatattgag taatattatt tcgaaatgtt     1260
   attaatatat tgtttgaaaa taaattagtt ttatgttttt gattttttga atatttatga      1320
   agtgttgttt agcgttttgg gaggttaagg cgggaggatt gtttgaggtt aggagtttaa     1380
   gattagtttg attaatatgg tgaaattttg tcgttattat ataaaaatta gttaggcgtg     1440
   gtggtgtatg tttgtaattt tagttatttg agaggttgag gtaggagaat tattttaatt     1500
   cgggaggtag agattgtagt gagtcgagat cgtattattg tattttagtt agggcgatag     1560
   agtgagattt tattttaaaa aaaaaaaaaa aaaaaaaaat tgaataaggt cgggcgtggt    1620
   ggtttatatt tgtattttta gtattttggg aggttaaggt gggtaggagt ttatttgagg     1680
   ttaggagttt aggataattt cgggtaatat ggtaaaaatt tgttttttta aaagtatata     1740
   atttagatat ggtggtatat gtttataatt ttagttattt tagaggttga ggtacgagaa     1800
   ttatttaaat taaaaagtgg aggttgtagt gagttgagat tatgttattg tattttagtt     1860
   tggtaataaa gtgagatttt gttttaaaag aaaaaaaaaa aaggaataat ttaaaagttt     1920
   aataaaggat tgtttaatat atatattatt tataatgaaa ataatagtat tggaatgtat     1980
   ttattgattt gaaaaagagt ttataatata ttttaattat ttatttttat aatatagatt     2040
   tttatatttt tttatagatt gtagagggtg aagttttaga atttgtattt ttatttattt     2100
   atttattttt gagatatagt tttattttgt tgtttaagtt agagtgtatg gtgtgatttt     2160
   ggtttattgt aatttttatt ttttgagttt acgtgatttt tttattttag tatttttaag     2220
   tagttgggat tataggtgtt tattattata tttagttgat atttgtaatt ttagtagaga     2280
   cgtggttttt ttacgttggt taggttggtt tcgaattttt gatttcgggt gatttattta     2340
   ttttagtttt ttaaagtgtt gggattatag gtgtgagtta ttgtatttag tttatatttt     2400
   aaatgaaaag aagaaaatgg agattataaa ttattaagta tattatttga tttttataaa     2460
   gatttatatg tgtaatttat atataaaata tatatgtgtg tgaatatata tatatatata     2520
   atttgaaagg ttatatataa atatgttaat aataattatt tttttggttt atttgtagtt     2580
   tgtaatgttt taataataag tatggataat taataagata gggataaaaa gataagtaat     2640
   aggatggttt attcgaaatt aaagtaaata ttgattaagt ttattaaata ataaagttat     2700
   taaaatgaag aaggaattga agattttaaa aaaaagaaat tatataatta tatatgattt     2760
   ttagtgattt tttgttattt gtggggaatt ttgtatattg tagaattgaa gaagtaaagt     2820
   atgaaattat aaaaattttt gtaaattaag atgaaatttt ttggtttaag tagttgttta     2880
   gttaaagttt tgttgattag ttttgttttt tttattagtt atttagtgaa attgttagat     2940
   taggataaaa agaatattta atatttaaat gtgattataa tatgataatt aaggttttaa     3000
   tatatatata tgaatttatt ttataaaaat tttataaggt aggtattatt ttttttttat    3060
   tttattaaag agacggattt taatttatta atggtgggta aatgttgtta aagtaatgtg     3120
   aaatttaggt agaatgttgt atttttagtt aatgatttaa aattttgagt ttttttgttt    3180
   tttttaggtt tgatcgtatt ttaatggaat tatttaaata tttgatgttg agttttttta 3240
   tggatttatt agatgaaatt ttggaaaggg attttttttt ttttttaaga tagggtttcg 3300
   ttgttattta ggttggagtg gagtggcgcg attttggttt attataatcg tcgtttgtta 3360
   ggttttagag agttttttat tttagttttt taagtggttg ggattatagg tgtatgttat 3420
   taagtttatt taattttttt tttttttttt tttttttttt tttttttttt tgtttttttt 3480
   tttttttttt tttttttgtt tttttttttt tttttttttt taagagatgg ggtttcgtta 3540
   tgttgttttg aggtttggcg atttatcgat tcggtttttt aaagtgttgg gattatagtt 3600
   atgagttagt acgtttaatt gagagagatt atgatgtttt ttaaataaat tttatttggt 3660
   tgaaatttta tagttgagta tggtttaatt tgagtagttt attttgagta ttttaattat 3720
   ggtttataga tttgggtttt tttgagaatt gtttgtaaat ggtttataga gagagagtag 3780
   aaaattttat gttaattttg taggtaattt tatggttgtt aattttaacg ataaaaagtt 3840
   ttgatttgga ttttgtatgt ttttttattt ttttgtttta ttaatttgtt aaaaattaat 3900
   tgaaaattga aaaaaataaa tcgatcgttt ttttagtgaa gagaagtatt gttgtaaagt 3960
   tttttatatt taaatattat taaattttaa ggtcgttttt gaggtttaat tttttaaaat 4020
   gatgtttcgg atattttttt tcgttttatt tttttttttt tttttttttt tttttttttt 4080
   tttttttttt tttttatttg agacggagtt tttgttttgt tgtttaagtt ggagtgtaat 4140
   ggtacgattt cggtttattg taaattttaa tttcgttttc gtttcgtttt cgttttttga 4200
   gtagttggga ttatagacgc gcgttattac gtttagttaa tttttgtttg tttagtagag 4260
   acgaggtgtt attatgttgg ttaggttggt ttcgagtttt tgatttcggt aatttattcg 4320
   tttcgttttt ttaaagtgtt gggattataa gcgtaaatta tcgcgttcga tttttttttt 4380
   ttattttatt tattttataa tttatattat tgatcgttga taattgtatt tattgtaatg 4440
   atattgataa ttgtatttgt tgtaataata ttgatattga taattgtatt tattgtatta 4500
   taatattatt attgatatcg ataattttat ttattgtaat gattaataaa ggtaggtaga 4560
   aagaaaataa aagtaattga aattattagg tagcgttttt gcgaatagta aagagggaag 4620
   aaacgttttg agcgtagtaa cgttattaat aaggagtcga cgcgacgggt tcgtttatag 4680
   gtaaggaagg tcgatttcgc gagtgtaggc gcgagcgtta gttcgtgacg tttgaggggg 4740
   ttggggggat gttgtgtggg ttttaagaag acgttacgtt ttcgtcgtcg ttacgttttc 4800
   gtcgttttta tcgtcggttg tcgcgttcgg aggaggtcgg gatgttatta ttttcgtcgg 4860
   ggttttttat tgtgttcggg ttagttgttg tcgtcgacgt cgtatcgttg cggtcggggg 4920
   attgggtcgg ggtttttatc gtcgatcgag gggagcgggt tcgttcggtt ttgttttttg 4980
   cgatttggtc gttagtttta cgtcgtcggt ttggaggggc gaagaggacg agggggttaa 5040
   ggtttttttc ggggtgagtg cggtttggga gaattcggta cggtggtttc ggagttggat 5100
   tgggaatcgg tttaggggcg aaagtttgtc ggcgcgtgtt tacgacgttc ggggagagga 5160
   aatttagaga gttatttttg gtagggggag ttatagaatt tgttcgggag gtgttttttg 5220
   gattttcgtt ttgtttaggt ttaatttttg gtttgggttt tgtttgtatt tggtagtttg 5280
   gttttttatt tcgtcgtttt ttcgtttttg gtagttgtgt tcgggatgga attttgtttt 5340
   tttttcgttt ttttattttt ttattttggt gtgggtgaag tggttgggtt acgatggttt 5400
   agtatttatg attagcgttc gttttggtat agaataaaga aatttggatt tttggtatgt 5460
   ttcgtaatcg cggggaatag atacggaagt agtataagta attagggttt taatagattt 5520
   tttttagtac gttaaattaa atatagagtg tgtacgttaa aagtcgagtt ttcgcgtgaa 5580
   tttagatata attttttttg tttaaatatt tggtaaaatg gtttggattt ttggatgtat 5640
   tttatgtcgt tggagagtta tttgtttgtg ttttgtgagt tttttgacga aatagttttt 5700
   tcggtgttgt attaagatag ttacggtgtt ttttatttat ttaattgaat aaattatgtg 5760
   attgtatttt agtgttgttt tttttatttt tatttttttt tgattaggat ttttatttta 5820
   tttaatattt gatttcgtat ttttaattaa atgtagtttt tttaatattt tataggaatt 5880
   tttgtaattg tagaaataaa ttttgtaatt tgtaaatgga tgttttttta aagttataat 5940
   tgattttatt taattaagtt atttttgtag attagttttt tgattatttt gtatatattt 6000
   ttagtttttt ttggtttata ttttttttaa attttatttt tagtttttat attttttagt 6060
   gtagtttaat attgttgggt ttagatcgga agtatgatag atttattttt gtaaacggaa 6120
   tttgaattag attattataa gttagtgtta ttatggtttt taggtattat aagttttttg 6180
   aagttaagtg cggttaaatt aggtattata ggtttttttt gtataaaatt gttagtgtat 6240
   tggagacggt gtgttgtatt agaaatagtt tcggattaga tatggggatt aggatgtagt 6300
   gttattgttt tatatataag ataaaatgat gataatataa gtgttatgta ttttaaagtt 6360
   gttggttgga atatgagatg atggatgtaa aagtggttag taaatgtgaa aagtattttg 6420
   gaaaagtttt gtttatttta tttggagtaa tgttggtaga aggaataata gggaggaagg 6480
   tgttagagaa agtatatgta attagattta gtaagttatc gggttgatag attgaaagat 6540
   gttttgttat tttgggtggt taaggtgggc ggattatttg aggttaggag taaaaaatga 6600
   taaagtaatg agatgagata aatttggtta acgttatgaa attttatttt tattaaaaat 6660
   ataaaaatta gttaggtatg gtcgttcgtg tttgtaattt tagttattcg ggagattgag 6720
   gtataagaat tatttgaatt tgggaggtgg aggttgtagc gagtcgagat tgtagtattg 6780
   tattttagtt tgggcgatag agtgagattt cgttttaaaa aaaaaaaaaa aaaggatgaa 6840
   aagatgttat gttatatttt atgaagtaag tttatatttt ataaagtaga atattagtat 6900
   aatgtggtga tagtaatatt ttacgattat agttttatat tgtaattaat atttttaatt 6960
   ataataaatt gagatattag ttagtagatt atttttttaa agattttagt atttttaagt 7020
   gtgtttcgta tagtgataat aaatgtaatt tttgtaggtg gtatagttat tttttgattt 7080
   atttttataa tttcgagaga atttagtatt attttagttt tttttttaaa ttttgtgtta 7140
   tatttatttt attttattta gtttttttga gatgtagttt cgttttgtta tttaggttgg 7200
   aatgtaatgg cgtaatttcg gtttattgta atttttgttt tttaggttta agcgattttt 7260
   ttgttttagt ttttttgatc gtttggatta taggttagtg ttattatttt tggttaattt 7320
   ttgtattttt ggtagagatg gggttttata tgttggttag gttggttttg aatttttgac    7380
   gttaggtgat tcgttcgttt tagtttttta aagtgttggg attataggtg tgagttatta    7440
   tgttcggttt gtgttaatat ttttgataga gtagtttatt ttattgtttg aaaattattt    7500
   ttatgggagt aatgataagt atatgtttag gattttttta agtatgtttg gtgatttttt    7560
   aaatatatag gttgttggtt aaaaagtgaa tataaatgtt tagaatgaaa tttttttgtt    7620
   taaatgggga agtgaaaggt ttgttcgggc gtttttattt gtttattgta attgattttc    7680
   gtataaacgg ttattattgg tatttattgt agtattagta ttaaattgta tatagtaaaa    7740
   ttattgtgta aaattgtgtt ataatttaaa tagttttaat gcgattttaa aaaaaattgt    7800
   agagatagat tttattgtgt tgtttaagtt aggtttgaat ttttgggttt aaattatttt    7860
   tttattttgt tttttaaagg tgttaggatt ataggagtga gttattgagt ttggtttaaa    7920
   tgtgatttta atatttaatt tttgtttatg ttgttaaatt gtttggggaa agttgtttta    7980
   ttttggttgg atttattagt attttttttt tttgaagtta tgttagaagg aattaattat    8040
   ttttttgatt tttaatttaa ggttaataat tgttttgaat ttatttttat gattgtaata    8100
   agtaagtggt ataaggatgt gttattatta ttaagagtta atttattgag aaattaagtt    8160
   atttttgtgt taataggtta ttttaataaa tattatttta aagtgttatt ttttatttta    8220
   atttttaaaa aaaaatatat gaataagaaa tgggaagtaa taatagttaa tttgtgagtt    8280
   agtagtattt tgattttttt cgaagaggtt gtggttatat taattttttt tttgtttgtt    8340
   ttttgttttt aatagttgtt tgatgataaa gtgtttttaa gaaatagtat ttttttaatt    8400
   gtttaaattg ttttgattta tttagtgtat ttttttttta aaatttatga tgaaatggta    8460
   gagtaaattt atttatattt ttttttttaa gttttgttta attagataat tgattatttt    8520
   taaattaaat atttatgttt atgtgttgag gtattaatat atgatttaat atgatattaa    8580
   ggtaaaaatg gaataaatag gttgagatag ttgaaagatt tgggttgttt gtgtttcgag    8640
   gagttattta gtttttgttg ttgtttgttt tgttttgttt tttgagatag agtttttttt    8700
   tgttatttag gttgtagtgt agtggtgtaa ttttagttta ttgtaatttt tatttttggg    8760
   gttttagcga tttttttgtt ttagttttta tagtagttgg gattataggt aggcgttatt    8820
   acgtttggtt gattttttgt attttttgtg gaaacggggt attattattt tggttaggtt    8880
   ggttttaaat ttttgatttt aggtgattcg tttattttag ttttttaaag tgttgggatt    8940
   ataggtgtga gttattacgt tcggttttaa ggagttgttt ttatagagat aaatttgttt    9000
   tattggttaa gtagttgaaa ggaatggatt gttttttaga attttttaag ataaattttt    9060
   ttttttatta gtgaggaata attttataaa tagaaattaa tattgttggt gtgtagtttt    9120
   ttatagaata tttttgttat atttttatgg ggtaatatat gataaatttt atttatgtag    9180
   tttaataagt aaaataagtt taagagattg agtaaaggtt tgttgattag tgtttatatt    9240
   ttataggtaa gtattattta tttaattttt ataggtttgt tatgtgttag gtattatggt    9300
   aggtgttggg aatatattgg gtataatttt tagtttttgt ttttattttt gaaatgttaa    9360
   atattttttt gagtatttga gatatttgaa aaagatattt agataatatt ttggtgtgtt    9420
   ttttaatata taataaaaag gtacgataag ataatagaaa gtttttttga ttattgaatt    9480
   taggaaaata tattattcgt tttagagtga gtattaaaga tttaagaaaa gttaaatatt    9540
   gggaaatata gtaaattaaa tatttagaaa agatttatta gtttttagtt tttagttata    9600
   gtatgttttg gttttatttg agtattgtaa ttttagagta gtaagtattt ttattatttg    9660
   ggtttgttga ttgtaattgt gggttttttt ttattatttt tgttttaaat agaggggatt    9720
   gtttggtaag gtaggtgttt ttttttttgt ttattttttt tttttaatag agaaagtgtt    9780
   aaataagtaa agattatttt aaatgttttt tattaattta taaaaattgt ttagattatt    9840
   gtttttttag agaattaaaa atttattttt tatatgtaaa tgattttttt aagtaaataa    9900
   gttggtgtgg aaaagttagt atttattatt ttttttttgt ggtaataagt gtattattaa    9960
   atattgaaat tttttgggaa tgtttaaaat ttttttttga gggtaagtaa aaatttaaat   10020
   tgggaagaga gttttttagt tttggtagag atataaattt agtttatttt gtgaaattta   10080
   atattttagg atatttttat tgtaattttg ttattacgga atataaggtt tttttttttg   10140
   taattttgtg tattttttga atgttgacgt agtagataag atagttatag gatatgatta   10200
   tatttttttt ttagaagatt ggttgatgtg aatggttgat ataattgatt ttatttaata   10260
   aatatttttt aaagttatag atagtattgt tttaaaaatt aaatggtata gacggattta   10320
   ttatgaaaag tattagttat tttttgtatt ttattttttt ttttttgtta ttggttttgt   10380
   tttgttattt agattggagt gtaatggtgt gatttcggtt cgttgtaatt tttatttttt   10440
   aggtttaagc gatttttttg ttttagtttt ttaagtagtt gggattatag gcgtttgtta   10500
   ttatattcgg ttaatgtttt tgtattttta gtagagattg ggttttatcg tgttggttag   10560
   gttggttttg aatttttgat tttaagtgat tcgtttattt cggtttttta aagtgttagg   10620
   attataggcg tgagttatcg cgttcggttt agtgttaacg atttttaatt ttatttttag   10680
   tattgttagt gatgtttcgt attttttaaa tatgttattg taaaaagaaa agaaaagaat   10740
   agaaatatgt tattgttgtt tgttgaataa ttttagatat ttattgattt gaatgtaaat   10800
   gaattaaata ttcgtgtata tttttttttt tttatagttt ttattgtttt gttgtattgt   10860
   taattagttg atttttattt tattttaatt tttatttttt attttattat tagagggagt   10920
   atttttagat tttttatttt taaaatgagg atattggttt tacgtgattt tttggaaaaa   10980
   ttaaaattaa agtttatggt atatgttaaa atacgtagtt gtataaaagg gtatttatta   11040
   agtttagtgt ttgaaaatat atgtcgagtt atatgagaag ggtatagtat agatatgaaa   11100
   taaattaaaa gattgtataa tttatttacg ggataagtta ttgaatagat ttttttaagt   11160
   ggtttaggaa ttgtggtatt aaattattga gaaggaaata tgaaagatag tatattattt   11220
   gattttgttg aattatgagt ttaaatatta ttgatttatt attatttacg gtttagaatt   11280
   aatttttatt taagggatag aaaatttaat tatgagtttg taataaagaa tatttttatt   11340
   ttttatttta ttttaaattt tttaatataa tttatagtat gaaatatatt tttaattata   11400
   atttgagata ttatatatgt aattgaaaat aagtattttt attgaatagt atttttatta   11460
   taaatagtat ttaatttttt tagaatatat gtagtttgag tatttttttt tttttaaaaa   11520
   aaaaaaaaaa ttgtaatttt gtaaatgaat tttatgatta gattattgta ttaaatatta   11580
   agtatgaatt aatatataag attattacgt ttttttagtg gataaaaagt aaagtggaaa   11640
   ttaagttata tatttttttt tttttatagg                                    11670
<210> 26
   <211> 11670
   <212> DNA
   <213> Artificial sequence
<220>
   <223> chemically treated genomic DNA (Homo sapiens)
<400> 26
   tttatgaaga gagaaaaatg tataatttaa tttttatttt attttttatt tattgaaaag      60
   gcgtaataat tttatatatt agtttatatt tgatgtttag tataatggtt tggttatgaa     120
   atttatttat agggttgtaa tttttttttt tttttaaaga aaagagaata tttagattat     180
   atatattttg ggaaagttaa atattgttta tagtaaggat attgtttagt gaaaatattt     240
   gtttttagtt gtatgtatag tattttaggt tatgattgaa aatatatttt atattatgga     300
   ttatattaaa aaatttgaag tggggtaaga aataaaaatg ttttttatta tagatttata     360
   attaaatttt ttgttttttg gatagaggtt gattttagat cgtaaataat agtaaattaa     420
   taatatttaa atttatgatt tagtagagtt aggtggtgtg ttgtttttta tatttttttt     480
   ttaatagttt gatgttataa tttttgagtt atttaaagaa gtttatttaa taatttgttt     540
   cgtaaatgaa ttatgtaatt ttttgatttg ttttatattt atattatgtt ttttttatat     600
   agttcggtat atgtttttaa atattgaatt tggtgaatgt ttttttatgt aattacgtat     660
   tttaatatat gttatgaatt ttagttttag tttttttaag gaattacgtg aggttaatat     720
   ttttatttta aaagtaagaa atttagagat attttttttg gtgatggagt aagaaataga     780
   agttgaggta aggtaaagat taattggtta ataatgtaat aaaatagtga aaattataga     840
   aggaaagagg tatatacgag tatttagttt atttatattt aagttaatag atgtttaaag     900
   ttatttaata aatagtagta atatattttt gttttttttt tttttttttg tagtaatata     960
   tttaagagat acggagtatt attagtagta ttaaaaataa agttaaaagt cgttgatatt    1020
   aggtcgggcg cggtggttta cgtttgtaat tttagtattt tgggaggtcg aggtgggcgg    1080
   attatttgag gttaggagtt taaaattagt ttggttaata cggtgaaatt tagtttttat    1140
   taaaaatata aaaatattag tcggatgtgg tggtaggcgt ttgtaatttt agttatttgg    1200
   gaggttgagg taggagaatc gtttgaattt gggaggtgga ggttgtagcg agtcgaggtt    1260
   atattattgt attttagttt gggtgataga gtaaaattag tagtagagga aagagggtga    1320
   aatgtagaaa atgattaatg ttttttatag taagttcgtt tatattattt gatttttaaa    1380
   ataatattat ttgtgatttt aaaagatatt tattaaataa aattaattat gttagttatt    1440
   tatattaatt agttttttaa aagaaaaatg tggttatgtt ttatagttat tttatttgtt    1500
   acgttaatat ttaaaagatg tataaagtta taaagaaaaa agttttatgt ttcgtaatga    1560
   taaaattata atggaaatat tttgaagtgt tagattttat aaggtaaatt aaatttgtat    1620
   ttttgttaag gttggaagat ttttttttta gtttgaattt ttatttattt ttaagagaag    1680
   gttttaaata tttttaaaag gttttaatgt ttgatgatat atttgttatt atagaaaaaa    1740
   aatggtagat gttgattttt ttatattaat ttatttgttt gaaaaagtta tttatatata    1800
   ggagtaaggt ttttaatttt ttgggaaaat aatgatttag gtagttttta taagttaatg    1860
   aaaaatattt aaaataattt ttatttattt agtatttttt ttgttgggag ggaaaaataa    1920
   ataaaaagag ggatatttat tttattaaat aatttttttt gtttagaata gaaatagtag    1980
   aaaggagttt ataattataa ttaataagtt taaatggtga aaatatttat tattttagaa    2040
   ttataatgtt taagtgagat taaagtatgt tatgattaaa ggttgaaaat tggtaagttt    2100
   tttttagatg tttaatttat tgtatttttt aatgtttaat tttttttaag tttttggtat    2160
   ttattttaaa gcgaatgatg tattttttta aatttaataa ttagaaaagt tttttattat    2220
   tttgtcgtat ttttttattg tgtgttgaaa aatatattag gatgttgttt gggtgttttt    2280
   tttaaatatt ttaaatattt agaaaaatat ttagtatttt aaaaataaaa gtagaaattg    2340
   aaagttgtat ttaatatatt tttaatattt attatggtgt ttagtatata ataggtttat    2400
   aaaaattaag taaataatat ttatttgtag ggtatggata ttggttagta aatttttgtt    2460
   taatttttta ggtttatttt gtttattaaa ttatatgaat aggatttgtt atgtgttatt    2520
   ttataaaaat gtaataaaaa tattttgtgg gaaattatat attagtaata ttaattttta    2580
   tttgtaaaat tgttttttat tggtaagagg aaaggtttat tttgggaaat tttagaagat    2640
   aatttatttt ttttagttgt ttaattaatg gaataagttt gtttttgtga aaatagtttt    2700
   ttgaggtcgg acgtggtggt ttatatttgt aattttagta ttttgggagg ttgaggtggg    2760
   cggattattt gaggttagga gtttgagatt agtttgatta agatggtgat atttcgtttt    2820
   tataaaaaat ataaaaaatt agttaggcgt ggtggcgttt gtttgtaatt ttagttatta    2880
   tggaggttaa ggtaggagaa tcgttggaat tttagaggtg aaggttgtag tgagttgaga    2940
   ttgtattatt gtattatagt ttgggtgata aagggagatt ttgttttaaa aaataaaata    3000
   aaataaataa taataaaaat taaatagttt ttcgaaatat aggtagttta agttttttag    3060
   ttattttagt ttatttattt tatttttatt ttaatattat attgaattat gtgttaatat    3120
   tttagtatat agatatggat atttagtttg aggataatta attatttagt taaatagaat    3180
   ttggggagag agatatagat agatttattt tattatttta ttatgaattt taggggaaaa    3240
   gtatattagg tagattaaaa taatttaaat aattgaggga gtattgtttt ttggaagtat 3300
   tttattatta aatagttgtt aagaatagga aataaataag aaaggagtta atataattat 3360
   aattttttcg aagaaaatta ggatattgtt gatttataag ttaattgttg ttgtttttta 3420
   ttttttgttt atgtattttt ttttggaaat tagaatggga aataatattt taagatgata 3480
   tttgttaaaa taatttgttg gtataggaat aatttgattt tttaatagat tgatttttgg 3540
   tagtggtggt atatttttat gttatttatt tgttgtagtt ataaagataa atttaaaata 3600
   gttgttgatt ttaggttaaa aattaaagag ataattgatt ttttttgata tagttttaag 3660
   ggggaggagt attggtaaat ttaattagga tgaagtagtt ttttttaggt agtttagtaa 3720
   tataagtaaa aattaaatgt taagattata tttaggttag gtttagtggt ttatttttgt 3780
   aattttaata tttttggagg ataaggtggg aagatggttt aagtttagaa gtttaagttt 3840
   agtttgggta atatagtgag atttgttttt ataatttttt ttaagatcgt attagaattg 3900
   tttagattat aatataattt tatatagtga ttttattata tgtagtttaa tattggtgtt 3960
   gtaataggtg ttagtaatga tcgtttatgc ggaaattaat tatagtaggt agatggaaac 4020
   gttcgggtaa gttttttatt tttttattta aataaaaagg ttttatttta aatatttatg 4080
   tttatttttt aattaatagt ttgtatgttt aggagattat taaatatgtt tgaaaaaatt 4140
   ttagatatgt gtttgttatt gtttttatga gggtagtttt taaataatga aatgaattgt 4200
   tttattagaa atgttaatat aagtcgggta tggtggttta tatttgtaat tttaatattt 4260
   tgggaggttg aggcggacgg attatttgac gttaggagtt taagattagt ttggttaata 4320
   tgtgaaattt tatttttatt aaaaatataa aaattagtta ggagtggtgg tattggtttg 4380
   taatttaagc gattagggag gttgaggtag gagaatcgtt tgaatttggg aggtagaggt 4440
   tgtagtgagt cgagattgcg ttattgtatt ttagtttggg tgatagagcg agattgtatt 4500
   ttaaaaaagt taaataaaat aaaataaatg taatataaaa tttagaggaa aaattagaat 4560
   ggtgttgagt tttttcgaaa ttgtgaaaat aaattaaaag atgattgtgt tatttataaa 4620
   agttatattt attgttatta tgcggaatat atttgaaaat gttgagattt ttaaaaagat 4680
   aatttattaa ttgatgtttt agtttgttgt agttagaaat attagttgta atataaaatt 4740
   ataatcgtgg aatattatta ttattatatt atattagtat tttgttttat aggatgtaaa 4800
   tttattttat aggatgtaat atgatatttt tttatttttt tttttttttt ttttgagacg 4860
   gagttttatt ttgtcgttta ggttggagtg tagtgttgta atttcggttc gttataattt 4920
   ttatttttta ggtttaagtg atttttgtgt tttagttttt cgagtagttg ggattgtagg 4980
   tacgagcgat tatgtttggt taatttttgt atttttagta gagatggggt tttataacgt 5040
   tggttaggtt tgttttattt tattgtttta ttattttttg tttttgattt taggtgattc 5100
   gtttattttg gttatttaaa gtaataagat attttttaat ttattagttc ggtaatttat 5160
   tagatttggt tatatatatt ttttttgata tttttttttt tattgttttt tttattagta 5220
   ttattttaga tagaatgaat agaatttttt tagagtgttt tttatattta ttaattattt 5280
   ttatatttat tattttatgt tttaattagt aattttggga tgtatgatat ttgtgttatt 5340
   attattttat tttatatatg aggtaatgat attatatttt ggtttttata tttagttcgg 5400
   aattgttttt aatatagtat atcgttttta gtgtattggt aattttatat agaagaagtt 5460
   tgtaatgttt gatttggtcg tatttagttt taaagggttt gtaatgtttg aagattataa 5520
   tagtattgat ttatgataat ttagtttaag tttcgtttgt agaagtggat ttgttatatt 5580
   ttcggtttaa gtttagtagt attaggttgt attagagaat atgaagatta ggaatagagt 5640
   ttagagaaaa tgtaaattaa aaagaattaa gaatatgtgt aaaatgatta agaaattagt 5700
   ttgtaggggt aatttaattg ggtaaggtta gttgtaattt tgaggaaata tttatttata 5760
   aattatagaa tttgttttta taattgtaga gatttttata aaatgttaga aaaattatat 5820
   ttaattgaaa atacgaaatt aggtattagg tgaaatagag attttaatta agagaaggtg 5880
   agaatgaaaa aagtaatatt aaaatgtaat tatatagttt atttaattaa ataaatagga 5940
   ggtatcgtaa ttattttgat gtaatatcgg aaaagttgtt tcgttaaaga gtttataaga 6000
   tatagataag taatttttta gcggtatgaa atgtatttag aaatttaaat tattttgtta 6060
   ggtatttaaa tagaaagggt tgtgtttaga tttacgcggg gattcggttt ttgacgtata 6120
   tattttgtat ttggtttagc gtattaggga agatttatta aagttttgat tatttatgtt 6180
   gttttcgtgt ttatttttcg cggttacgag gtatattaga agtttaagtt tttttgtttt 6240
   gtattaggac ggacgttggt tataaatatt gaattatcgt agtttagtta ttttatttat 6300
   attagggtaa aagggtgggg gagcggagga ggggtaaggt tttatttcga gtataattat 6360
   tagaaacgga aaagcgacgg ggtggggggt taaattgtta aatataaata ggatttaagt 6420
   taagggttgg atttaagtag ggcggggatt taggaagtat ttttcgagta ggttttatgg 6480
   ttttttttat taagggtaat tttttgggtt tttttttttc ggacgtcgtg ggtacgcgtc 6540
   gatagatttt cgtttttagg tcggttttta gtttagtttc ggagttatcg tgtcgagttt 6600
   ttttaggtcg tatttatttc ggaggaagtt ttggtttttt cgtttttttc gtttttttag 6660
   gtcggcgacg tggggttgac ggttaggtcg taaaaagtag ggtcgagcgg attcgttttt 6720
   ttcggtcggc ggtggagatt tcggtttaat ttttcggtcg tagcggtacg gcgtcggcgg 6780
   tagtagttga ttcggatata gtgagaagtt tcggcggaag tgataatatt tcgatttttt 6840
   tcgggcgcgg tagtcggcgg tggaggcggc gggggcgtgg cggcggcgga ggcgtggcgt 6900
   ttttttgaaa tttatatagt atttttttaa tttttttaga cgttacgggt tagcgttcgc 6960
   gtttgtattc gcggggtcgg ttttttttgt ttgtgggcgg attcgtcgcg tcgatttttt 7020
   attggtggcg ttattacgtt taaggcgttt tttttttttt tgttgttcgt agggacgttg 7080
   tttggtagtt ttagttattt ttgttttttt tttgtttgtt tttattgatt attatagtag 7140
   gtgaaattat cgatattaat aataatattg taatataata ggtgtaatta ttaatattaa 7200
   tattattata ataggtgtaa ttattagtat tattataata ggtgtaatta ttagcgatta 7260
   ataatgtgaa ttataaaatg gatgaaataa ggggaggagg tcgggcgcgg tggtttacgt 7320
   ttgtaatttt agtattttgg gagggcgagg cgggtggatt atcggagtta agagttcgag 7380
   attagtttgg ttaatatggt gatatttcgt ttttattaaa taaataaaaa ttagttgggc 7440
   gtggtggcgc gcgtttgtaa ttttaattat ttaggaggcg agggcggagc ggaggcggag 7500
   ttggagtttg tagtgagtcg agatcgtgtt attgtatttt agtttgggta atagagtaaa 7560
   aatttcgttt taaataaaag gaaaaaggaa aaaaaaaaaa aaagaaaaag aaaaaaaaga 7620
   aataaggcga ggaaaaatat tcgaggtatt attttgggga gttaggtttt agaagcgatt 7680
   ttagagtttg gtaatatttg agtgtgaaaa gttttatagt agtgtttttt tttattgggg 7740
   gaacggtcgg tttatttttt ttaattttta attagttttt gataagttaa taaaatagaa 7800
   aaatgaaaag atatataaag tttaagttaa aattttttat cgttagagtt aatagttata 7860
   aaattatttg taaaattgat atagagtttt ttattttttt tttgtgaatt atttgtaaat 7920
   agtttttaga agagtttagg tttatagatt atggttagag tatttaaggt aaattattta 7980
   aattagatta tatttagttg tagagtttta attaaatgag atttgtttgg agaatattat 8040
   agtttttttt agttgggcgt gttggtttat gattgtaatt ttagtatttt gagaggtcga 8100
   gtcggtagat cgttagattt tagggtaata tggcgagatt ttatttttta aaaaaaaaaa 8160
   aaaaaaaaga gatagagaaa gaaagaaaga aaaagaagta aagagaaaga aaggaagaaa 8220
   gaaagaaaag aagaaaatta ggtgggtttg gtggtatgta tttgtggttt tagttatttg 8280
   ggaggttgag gtgggagatt ttttggagtt tggtaggcgg cggttgtagt gagttaagat 8340
   cgcgttattt tattttagtt tgggtgatag cgaaattttg ttttaaaaaa aaaaaaaagt 8400
   ttttttttaa agttttattt gataaattta tagaaaggtt tagtattaag tatttgggtg 8460
   attttattgg ggtacgatta ggtttgaggg gaatagaaaa gtttaaaatt ttaaattatt 8520
   gattaaaaat gtagtatttt gtttggattt tatattgttt tggtaatatt tatttattat 8580
   tggtagatta aagttcgttt ttttgataaa atgggaagaa aatagtattt attttatagg 8640
   gtttttgtga gatgaattta tatatatgta ttaggatttt ggttgttata ttgtaattat 8700
   atttaagtat taggtatttt ttttatttta gtttagtaat tttattaaat agttagtaga 8760
   gaaaataaaa ttgattaata gagttttagt taaataatta tttgggttaa gaaattttat 8820
   tttgatttat agaagttttt gtagttttat attttgtttt tttagttttg taatgtgtag 8880
   aattttttat aggtggtaga aaattattgg aaattatata tagttatgtg attttttttt 8940
   tttaagattt ttagtttttt ttttatttta gtaattttat tatttaataa gtttgattag 9000
   tgtttgtttt ggtttcgagt aagttatttt attatttatt tttttatttt tattttgtta 9060
   attatttata tttattgttg aaatattata aattataagt aagttaaaaa gataattatt 9120
   attaatatat ttatgtatag ttttttagat tatatatata tatatattta tatatatata 9180
   tattttatgt ataaattgta tatataagtt tttataaaaa ttagataatg tatttagtgg 9240
   tttataattt ttattttttt ttttttattt aaaatatgag ttgggtgtag tggtttatat 9300
   ttgtaatttt agtattttgg gaggttgagg tgggtggatt attcgaggtt aggagttcga 9360
   gattagtttg gttaacgtgg agaaattacg tttttattaa aattataaat attagttggg 9420
   tatggtggtg ggtatttgta attttagttg tttgggggtg ttgaggtagg agaattacgt 9480
   gaatttagga ggtggaggtt gtagtgagtt aaaattatat tatgtatttt agtttgggta 9540
   atagagtgag attgtgtttt aaaaataaat aaataaataa aaatatagat tttgaggttt 9600
   tattttttat agtttatgga aagatatggg aatttatatt ataaaaataa ataattaaaa 9660
   tatattatga attttttttt aagttaataa atatatttta atgttattat ttttattatg 9720
   gataatgtat atattaaata atttttattt gaatttttag attgtttttt tttttttttt 9780
   tttttgagat agggttttat tttgttgtta ggttggagtg tagtggtatg gttttagttt 9840
   attgtagttt ttattttttg atttaagtga ttttcgtgtt ttagtttttg gagtagttgg 9900
   gattataggt atgtgttatt atgtttaagt tgtgtatttt tggagagata ggtttttgtt 9960
   atgttgttcg ggttgttttt gaatttttgg ttttaagtga atttttgttt attttggttt 10020
   tttaaaatgt tgggaatata ggtgtgagtt attacgttcg gttttgttta attttttttt 10080
   tttttttttt ttttgagatg gagttttatt ttgtcgtttt ggttggagtg tagtggtgcg 10140
   atttcggttt attgtaattt ttgtttttcg ggttgaagtg atttttttgt tttagttttt 10200
   taagtagttg ggattatagg tatgtattat tacgtttggt taatttttgt gtagtagcga 10260
   tagggtttta ttatgttggt taagttagtt ttgaattttt gattttaggt aattttttcg 10320
   ttttggtttt ttaaagcgtt ggataatatt ttataggtgt ttaaaaagtt agaaatatag 10380
   gattaattta tttttaaata atgtattagt aatatttcga aataatattg tttaatatat 10440
   ttttttttga tttttatata ttgttttagg taagtgtttt taaagaaata aatggaattg 10500
   ttaatttaaa atacgatgtt aaatatatat tgtaggttag gcgtggtgtt acgtttgtaa 10560
   ttttagtatt ttgggaggtt gaggtggacg gattatttga ggtttggagt tggagattag 10620
   gttggttaat atggtgaaat tttattttta ttaaaaatat aaaatttagt tgggtgtggt 10680
   ggtatatatt tgtaatttta gttatttgga aggttgaagt agggaaatcg gttgaatttg 10740
   ggaggtggac gttttagtga gtcgaggtcg cgtaattgaa ttttagtttg gatgatagag 10800
   cgagattttg ttttaaaaaa taaataaata aaaaaattta ttgtattttt tgtgttttta 10860
   gattttttgg ataagattta gggtattttt ataattattt attgtgtgtt attgtatttt 10920
   agtttgggta ataagagtgg aattttattt taaaaaaata aaaaagaaat atttttatat 10980
   ataattagat tatagttatt taagggaaaa attaggttta gttgttagag gattttattg 11040
   tatagaattt tagagattta atttatttaa agaaatagtt aggttataat ttgtagtgaa 11100
   gtgagattga gcgtttagat atggatatag tcgtgtttgt tgaggaggag ggggtgtgta 11160
   ttttatttaa tagaagttga gaaaggttga tattagttta aatgatttat taaaattaat 11220
   agtgtgaaga aattgttttt tttttttttt ttttttgaga tagggtttta ttttgttatt 11280
   taggttggag tgtaatggta taattttagt ttattgtaat tttcgttttt taggtttaag 11340
   taattttttt gtattagttt ttcgagtagt tgggattata ggtacgttat tgcgttaggt 11400
   taatttttgt atttttagtg gagatggggt tttattaagt tggttaggtt ggtttggaat 11460
   ttttgttttt aggtaattag tttatttcgg ttttttaaag tgttgggatt ataggtgtga 11520
   gttatcgcgt ttggttagtg tgaagaaatt ttaagagttt atatttaagt tttttgtgag   11580
   gatatttttg tagttataaa aatttataaa tatgagttta aaattatttg aggatatata   11640
   ttttatatta taaaattata aattttggta                                    11670
<210> 27
   <211> 8201
   <212> DNA
   <213> Artificial Sequence
<220>
   <223> chemically treated genomic DNA (Homo sapiens)
<400> 27
   gataaaatat tatgagaggt tgggtatggt ggtttatatt tataatttta gtattttggg      60
   aggttaaggt ggttagatta cgaggttagg agtttgagat tagtttggtg aatatagtga     120
   aattttattt ttagtaaaaa tataaaaaat tagttaggtg tggtggtgtt tgtaatttta     180
   gttatttggg aggttgaggt aggagaatta tatgaattcg ggaggcggag atggtattat     240
   tgtattttag tggggtgata gtgcgagatt ttgttaaaaa ataaaaaata aaaaaaaaaa     300
   acgatgtttt tttatatagt gagttttagt taaatttaaa tatatttaat tttttattta     360
   gagtatagaa aataaaattg gtagtttata tgttaaaagg tggatgtttt tttaattttg     420
   ggtttttaat ttttaaaaat attaaggtaa taaattagga gaatttttgg taaagaattt     480
   gataaacgta atatatcgtg tgtattttgt aattagttta ttttttatta aatagattta     540
   taaaataaat tagttatatt gagtttgttt ttgggataag taattgaata tgtgattgta     600
   aaggtagaga ttttttttgg ttttttttgg tttttttgtt taaagtttat gagttttgta     660
   atattttgtt ttttattttg ttaatttttt ttttattatt tttattattt gttatatttg     720
   aatagtttga atattttagt tatatattat atattaggtg tattagtttg tttttatgtt     780
   gttaataaag atatatttaa gattgggtat ttataaagaa aaaaagaggt ttaatgggtt     840
   tatagtttta tatggttggg gaagttttat aattatggtg gaaggtaaaa gaggagtaag     900
   gatatatttt ttatggaagt agataaaaag gtatgtgtag gggaattttt ttttatgaag     960
   ttattagatt ttatgagatt tttttattat tatgagaata atatgggaaa aatttatttt    1020
   tatgatttaa ttatttttta ttaggtgttt tttatgatat taggggatta ttataattta    1080
   aggtgaggtt ttggtgggga tatagagtta aattttgtta ttttgttttt ggtttttttt    1140
   aaatgttatg tgtttatatt ttaaaattaa ttttgttttt taattgtttt ttaaagtttt    1200
   tatttatttt agtattaatt taaaagttta agtttaaagt tttttttaag ataaggtaag    1260
   ttttttttat ttatgagttt gtaaaattaa aagtaagtta gttatttttt agatataacg    1320
   gggatatagg tattgggtaa atatatttat tttaaatggg agaaattggt taaaggaagg    1380
   ggttatattt tttatgcgag tttaatattt agtagggtag ttaaatttta aagttttaaa    1440
   atgatttttg attttatgtt ttatatttcg gttatgttga tgtaagagtc ggggttttat    1500
   ggttttgggt agttttattt tcgtggtttt gtagtgtata gtattttttt tagttgtttt    1560
   tatgggttgg tattgagtgt ttgagtattt taggtgtatg gtgtaagtcg tttgtggatt    1620
   tattatttta ggatttggag gatggtggtt ttttttttat ggttttatta ggtagtgttt    1680
   tagtgaggat tttgtgtggg agtttatatt ttatattttt tttttgtatt gttttagtag    1740
   aagtttttta tgagggtttt atttttgtag taaatttttg tttggatata taagtatttt    1800
   tatatatttt ttgaaattta gatagaggtt tttaaatttt aatttttgat ttttgtgttt    1860
   ttataggttt tatattatat ggaagttgtt aaggtttggg gtttgtattt tttgaagtta    1920
   tagtttaagt tattttttgg ttttttttag ttatggtttt agtggtttag gaagaagggt    1980
   attaagtttt taggttgtat atagtagaag ggttttcggt ttattttatg aaaatatttt    2040
   tttttttttt aggtttttag ttttgtgata ggaggggttg tttggaaggt ttttgatata    2100
   ttttggagat atatttttta ttgttttgga gattaatgtt ttagagatta atatttggtt    2160
   tttcgttatt tttgtaaatt tttatagttg gtttgaattt ttttttagaa agtgggtttt    2220
   gtttttttag tatattattt ggttgcgaat gttttaaatt tttatgtttt gttttttttt    2280
   taaatataag ttttaatttt aaattatatt tttgtaaata tataaaaatg aatgttttta    2340
   atagtattta aattattttt ggaatgtttt gttgtttaga aatttttttt gttagatgtt    2400
   ttaaattatt ttttttaagt ttaaagtttt atagattttt agggtagggg taagatgtta    2460
   ttattttttt tattaaaacg tagtaatagt tatttttatt ttatttttta ataagttttt    2520
   atttttattc gagatgagta gtatttagtt aaagttattt aataagtttt tagaaagttt    2580
   tagattttat ttttttattt ttttttaagt ttttagaatt gttttaattt ttgtatttta    2640
   tttagtttta atatcgtttt tatatttttg gagattttta tagtagtatt ttattattta    2700
   gtataaattt attgtattag ttatttttta tattgttaat aaagatatat ttaaggttgg    2760
   gtaattatat agaataaaaa agatatttaa tagatttata gttttatatg gttggggagg    2820
   ttttagaatt atagtaaaag gtaaaagagg agtaaggata cgttttatat gggtgtaggt    2880
   aagagggtat gagtagagga attttttttt ataaaattat cggattttat gagatattta    2940
   ttattaagag aatagtatgg aaaaaaaata gtatttatga tttaattata ttttattaga    3000
   tattttttat aatatatgag gattattata atttaaggtg agatttgagt agggaaatag    3060
   agatagatta tattattagt ttttatgttt gttttttttt tttgaatgtg agattaaagg    3120
   ggaaaggatt atgatatgtt ttgattgaat ttttggtatt tagagtaatg tcgttcgtat    3180
   atgtttgtta aatgaatgat tagatgtatt gtttgaggat tttaataaaa gataaattta    3240
   gttaaataat ttgtaaataa ttatttttga ttattttttt aattttaaat atttgtatta    3300
   attataataa tattatttat tatttgttta ttatttgtta ggttatgttt tatgtatttt     3360
   ttatattttt ttttatttaa tgtttaaaat tgtgtaggta tatattatta ataaaattat     3420
   tttatatata gaggaattaa tgtataggga agtttattat tttgttaagg aagggaagtt     3480
   taaaatgtta agttcgatat ttttatttta gagtttatgt tttgggtttt tataatgtaa     3540
   ttttttttaa aaataaattt tttaggatgg gtgggtggtt tatgtttgta attttagtaa      3600
   tttgggagat taaggtgggt atattgtttg agtttgggag ttgaagataa gtttaggaaa     3660
   tataaggaga ttttgttttt ataagagata taaatattag ttggttatgg tggtatgtgt     3720
   tagtaattgt agttattcgg gagtgttgag ttggaagaat tatttgagtt tgggaagtgg     3780
   agtgttgtag tgagttaagg ttaaataatt gtatttcggt ttgtacgata gaatgatatt     3840
   ttgtattaga aaaaaaaaaa aatagaaaga aaaaagtaat gtgaaaagaa ggatattaaa     3900
   taagaaaagt tttaattttg gtgaaaaatg tttttatttt ttaaagtaaa aatgttttat     3960
   agggatatat attatttata atttttttaa gagttatgag gtggggttag gggaatagtg     4020
   gtttatatat tttaaaggtt gtgttatttt ttttcgttaa gttttttaat gattgagaaa    4080
   ttttttattg tgtgaattta attgtatata tattttttta attttgtttt attttttaag     4140
   ttttattttt tttttatttt gatttattta agggttggtg aagaaaattt tttatggata     4200
   tattatttga tgtaggttat agttttgatt ttttaatggg aagaaaaata aagatatgta     4260
   tatgtatgta tagtgagtat tttttaatat atgtataggt tttgttaaaa aggaaggcga     4320
   agtatatttt tagttttagt tatataagaa gggaggagag aaaagttatg gtcggtgagt     4380
   tttttattgt tttaagaata ggtttttggg aagaaagagt tttggaaggg gttggttttt     4440
   ttggaagtta taattagtat ttgtggaagt ttatttgagt agaagtaagg aaaagggggg     4500
   agagggagga gagaaggaat tagaagattt ttgtagattt tagaaaggaa aggtaagttt     4560
   agaaaggaat tgaggtttta ggggaagttt gatgttattt tgtaatttta attaaatttt     4620
   tatttatatg ttgaaagttt gatagagggt atagaattgg tataggagga gtattagtag     4680
   tagtggtttt taaatgaaat gggttatttt tgttttatta ttttattaat tagagttaaa     4740
   taaatatgcg atataagagg atgatttttg ttttttaggg attgatgtgg aaatggtaaa     4800
   tttagttttt aggttatttt atgtatatgt atggagatta ttgagggaaa taagtaggat     4860
   agtaatatag taagaagagg gattatttaa ttgtgaggag ggggatattt tattgatgaa     4920
   atttaaatga ggttattttt tgttgattat atgagaggaa tatgaggttg tttttatgat     4980
   gtgagaaatt tttgtgtttt aaatagataa ggttttgaag gagaagagaa agttgtttat     5040
   tcgttttatg ggtgaaggta taataaatgg tttattggat gaattattat agataagggt     5100
   gttgaataag gaagagatgg agaaagtaaa acgtgaaaat gttatagtta tggataagat     5160
   tcgagttttg attgatttcg ttatttcgaa aggggtatag gtatgttaaa tttgtattat     5220
   atatatttgt gaagaagata gttatttggt agggacgttg ggatttttag taggtaaggg     5280
   ttagtgattt atatttaagt ttatttaggg tttgttttcg tgtttaatat ttgatttttt     5340
   tattagttat gttaattgga agaaaatttt tttatttttg gttttagggt tatatttttg   5400
   tagtagggtt tttattgtat gttttttgat ttttttttga tttttttttt taatttttag   5460
   tggtattttt ttgattaaag tttatttttt gttttgagta tgtggtagtt tttttattga     5520
   agtttttgag aattatttga aaagttttat gttgtgtttt ttagtgtttt tggtaaggta     5580
   aaatatttat ttgtggattt atttttgttg agtgtaaggg gtttgtaggt atttatgtag     5640
   ttgtaaagtt atatgaagtt ttaaggttta taattaaata ttttttataa ttttttttta     5700
   aaattttatt tttttaaaag aataaaatat attttaatga tggcgatata tttagagaat     5760
   atattgaacg tatagaaaag tgaagatttt taatgggttt atttttttag ttgttttttt     5820
   gtattttttt tgagtaagat tatttggttg tttgttatta ggttgtgggt tatgttgtga     5880
   ggtttggaaa ttttttattt gtaatttttt ttaggtttaa tatagggttt aggaattgag    5940
   ttggttaaat tttatgatta tttggattta ttacgtggtg aggatgagag ttttggtgga     6000
   tttttagtta ttgatgtttg tggtattttt taaagttatt ttagagggag atgttttttg    6060
   aaagtaaaag ttttttgttt taagggttag ggaaagttta tgtttgttta agtagatatt    6120
   gtagatgttt ttgtttttat ttatgtgtaa taaatacgta tttttttatt aatgtttttt     6180
   ttatttttta ttttttatta ttttttaaat taaagaatat ttttaatatt atttaatgtt   6240
   agttagttag ttagtgttat gggagaaatt tttagtgggg agtgttttat agattggggt     6300
   aaagtttttt agagatgata agtgattatt ttaatatttt ttattttttt atagattaaa     6360
   tatttggaaa ttattttaat atgtaagatt tttaaggagt attttttttt tttttaggta     6420
   atgttgtttt taaaggaaga gtattttttg atttatattt tttttgatat taattttgta     6480
   ggattaaacg tgggtaaatg ggttatggag aatttttata gattggttat ataatttttt     6540
   ttttgtaatt tattttgttt ttgtagaaat tgtttttata gttttattat ttatatttat      6600
   attaaaaatt ttgaatttgt gttttaaatt ttgattattt aaataagtat taaattttat     6660
   tatttaagtt tattgaaata tttattttta ttttttagag atattttatt ttaaattttt   6720
   ttagagttga aaatgttata tttttatatt ttgttttttt ttcggttttg tgtaatttgg    6780
   ttattggtgt tatcgttatt agttgtttaa attaggaatt tgaaatttat atgggatttt     6840
   ttttttttat gtatttttaa tatttaattt atggttaagt tttatgtagg ttttttttaa   6900
   atttggaatt atttttttat ttttattttt attattttat taaagaattt ttatttttat   6960
   agtgtatttt taaatttata tttgaaagta atgtagaggt taggaatgta aattatttat     7020
   taagtttaga tattatttta ttgaatttat taaggttggg tattatgaat atttttttaa    7080
   atgttttata gtatgggata tttaatatat aaagaaatat tgtatttttt attatgaaaa     7140
   aatagtttaa aaataattta attattttta gttttttttt attgttttta tattttttta     7200
   tgaagaaatt taatatttta tttaataaat gattatttta taattttttt taatatattt    7260
   attttaattc gttttttttt atattagtat attttatgtt gtttttgttt atttaattga    7320
   ttgtataata attttttaaa tttgttttta aaattaaata ttattttttg ttttaatagt    7380
   ttttaaaaga ttttttatat atttagaaaa tttattttta ttttaaaata aaggttatat     7440
   taattttttt aataaggata ttatattgtt ggattgtgtt gaatttgtga tgaaataaaa     7500
   ggtttaagga tttttttata taaattttgt gaaaatattt ttttaagtgt ttgtgtaatg     7560
   aatgtttagt gttataaatt tatttagtgt gtattttgat tttagtattt attattatat     7620
   ttttatttta ttttatatat ttaatattta tgttttttta tatgtatata tagaaggaag     7680
   agattttgaa ggataggata aagatttatt aaaaaatatt tttagtgttt attattatgt     7740
   atattaagta aataaataaa aagataaata tgtattttaa aaaatatttt agtagattaa     7800
   attaagattt gattttatta aattttattt tagggtttga atattttgaa aaaagtttat     7860
   atttttaatt attttatttt atatattttt tgttttttga gtgttttatt aggtatagat     7920
   tttaaatttt ttttttttat gttagtattt aaaatgtttg tagaaaggtt gattttgaaa     7980
   tatgaaagaa gatatttgta atgtatattt ggaaattttt tgtttgaaat gtgttttatt     8040
   aggagtaaat ttatttagtt gtgttaatga tgtagattag gattgttttt tttagagtgt     8100
   attttttttt ttgtggtttt atttataggt gaggttgtaa taaatataaa gtggaataaa     8160
   tagggatgtt attgtttatg atgttttttg ttttttgtag t                       8201
<210> 28
   <211> 8201
   <212> DNA
   <213> Artificial Sequence
<220>
   <223> chemically treated genomic DNA (Homo sapiens)
<400> 28
   gttgtaagag ataaagaata ttatgaatag tggtattttt gtttgtttta ttttgtgttt 60
   gttatagttt tatttatgga tgaagttata gaagaggaaa tgtattttga ggaggataat 120
   tttgatttat attattaata tagttgagtg agtttgtttt taatggagta tattttaagt 180
   aagagatttt taggtatgta ttataagtat ttttttttat attttaggat tagttttttt 240
   ataagtattt tagatattaa tatagaaagg gaaaatttaa agtttatgtt tgataaaata 300
   tttagaaaat aaaaaatgtg tgagataaaa tagttaagaa tataaatttt ttttaaaata 360
   tttaaatttt aggataaaat ttaatgggat taggttttaa tttaatttat taaaatattt 420
   tttaaaatat atgtttattt ttttatttat ttatttaata tatatggtag taagtattag 480
   gaatattttt taatgaattt ttgttttgtt ttttaggatt tttttttttt atgtatatat 540
   atgaaaggat atgaatattg aatatatgaa gtagaatgaa gatataatga taaatgttaa 600
   aattaaggta tatattagat ggatttatag tattaaatat ttattgtata gatatttgag 660
   gaaatgtttt tatagagttt atgtgagaaa atttttgagt tttttatttt attataggtt 720
   taatataatt taataatgtg atgtttttgt taaagaaatt aatgtaattt ttgttttaaa 780
   atagaaataa gttttttaga tgtgtggagg attttttaga agttattaag gtaaaaaata 840
   atatttaatt ttaaaaataa atttgaaaaa ttgttatata attagttaga taaataaagg 900
   tagtatgaaa tgtgttgatg tggagaagaa cgaattagaa tagatatatt ggaaaaaatt 960
   atagaataat tatttgttgg ataaaatgtt ggattttttt ataaagaagt gtgaaaataa 1020
   tagaaaaaaa ttagaagtag ttaggttatt tttgagttat ttttttatag tagagaatat 1080
   agtatttttt tatatattga atattttatg ttgtgaaata tttaagaaaa tatttatgat 1140
   atttaatttt aataaattta gtagagtaat atttgaattt gataaataat ttgtattttt 1200
   agtttttata ttgtttttag atataagttt aagagtgtat tatgggaata ggggtttttt 1260
   aataaaataa tagaagtaga aatagagaga tggttttaaa tttgagaaaa gtttgtataa 1320
   aatttggtta tgggttgaat gttagaggtg tatgagaagg aagggtttta tatgagtttt 1380
   aagtttttgg tttgaataat tggtgacggt ggtattaata attaagttat ataaaatcgg 1440
   aagagaagta gggtgtggaa gtatagtatt tttagttttg ggaaagttta gaataaggtg 1500
   tttttgggag atgggagtga atattttagt aggtttaaat aatgaagttt ggtatttatt 1560
   tgggtaatta ggatttgaga tatagattta gaatttttaa tataaatgtg gataatgaag 1620
   ttgtggaaat agtttttata aaagtagaat agattataaa aagaaaatta tatgattaat 1680
   ttatgagaat tttttatagt ttatttattt acgtttgatt ttataaaatt aatgttaagg 1740
   aagatgtggg ttaaagaatg tttttttttt aaaaatagta ttatttggaa aggaagaaag 1800
   tattttttga gagttttgta tattaaggta atttttagat gtttgattta tgaaaagata 1860
   aagggtattg aagtagttat ttattatttt tggaaaattt tattttaatt tgtaaaatat 1920
   tttttattaa aaattttttt tataatattg attgattgat tgatattaaa taatgttaaa 1980
   gatgtttttt aatttagaga gtggtgggag gtagaaaatg aaagaggtat tggtagaaaa 2040
   atacgtgttt attatatata gatgaaaata gaaatatttg taatgtttgt ttagataggt 2100
   ataagttttt tttaattttt agaatagaag gtttttgttt ttaaagaata tttttttttg 2160
   aagtggtttt aggaagtgtt atagatatta atgattggga atttattaga atttttattt 2220
   ttattacgtg gtaggtttaa gtggttatga gatttggtta gtttagtttt tgagttttgt 2280
   attgggtttg aggagggttg taagtggagg atttttaggt tttatagtat gatttatagt 2340
   ttggtaatag gtaattaggt gattttattt aaagaaaata taaggaggta attggaggaa 2400
   tgagtttatt agaaattttt atttttttat acgtttaatg tgttttttga gtgtatcgtt 2460
   attattgaaa tatattttat ttttttggga gggtaaaatt ttggagaaaa gttataaaaa 2520
   gtatttgatt ataaatttta aagttttatg tgattttata attatataag tgtttataga 2580
   ttttttgtat ttagtaaaaa taaatttata ggtaaatatt ttgttttgtt aggaatattg 2640
   gaaaatatag tatgaagttt tttagatggt ttttaagagt tttagtaaag gagttattat 2700
   atatttagaa taggaaatga gttttaatta gaagagtgtt attgaggatt aaggaaaaga    2760
   attaaaggag agttaaggga tatgtaatag ggattttgtt atagaaatat ggttttgaag    2820
   ttagaaataa gaaagttttt ttttaattaa tatgattagt aaagaaatta aatgttaggt    2880
   acgaagatag gttttaggtg aatttgagtg taagttattg atttttattt gttgagagtt    2940
   ttagcgtttt tgttaggtaa ttgttttttt tataaatgta tgtgatgtaa atttggtatg    3000
   tttgtgtttt tttcggaata acggagttaa ttaaagttcg ggttttattt ataattgtag    3060
   tatttttacg ttttattttt tttatttttt ttttgtttag tatttttgtt tgtaataatt    3120
   tatttagtaa gttatttatt gtatttttat ttatggaacg gataaatagt tttttttttt    3180
   tttttaggat tttgtttgtt tagagtataa ggatttttta tattatgaaa atagttttat    3240
   atttttttta tgtaattaat agaaagtgat tttatttaga ttttattagt gaaatgtttt    3300
   tttttttata gttgggtaat tttttttttt attgtattgt tgttttattt gtttttttta    3360
   atagttttta tatatgtgta tggagtgatt tgaagattga gtttattatt tttatattag    3420
   tttttggaaa gtagagatta tttttttatg tcgtatgttt atttaatttt ggttaataaa    3480
   gtaatgaagt agaaatagtt tattttattt aggaattatt gttgttagta ttttttttgt    3540
   gttaattttg tattttttgt tagattttta atatgtaagt aagagtttaa ttaagattgt    3600
   aaaatgatat taggtttttt ttaaagtttt agtttttttt tgggtttgtt tttttttttt    3660
   aaagtttgta agaatttttt agtttttttt tttttttttt tttttttttt tttttgtttt    3720
   tatttaagta aatttttata gatgttaatt atggttttta gaagagttag ttttttttaa    3780
   aatttttttt ttttagggat ttgtttttgg aatagtaaaa gatttatcgg ttatggtttt    3840
   tttttttttt ttttttgtgt gattgaaatt gaaagtatgt ttcgtttttt tttttggtag    3900
   ggtttgtata tgtattggga aatatttatt gtgtatgtat atgtatgttt ttattttttt    3960
   ttttattaaa gaattagaat tgtagtttgt attaggtagt gtatttatgg gaaatttttt    4020
   ttattagttt ttggatagat tagggtagga ggggaatggg gtttggagag taagatagga    4080
   ttgaaaaagt atgtgtataa ttaaatttat atagtgaaga gttttttaat tattagaaag    4140
   tttgacggag aaaagtaata taatttttgg agtatgtgaa ttattgtttt tttgatttta    4200
   ttttatagtt tttgggaaag ttgtgggtaa tgtatgtttt tgtgaaatat ttttgtttta    4260
   agaaatagaa atatttttta ttagaattaa agtttttttt atttaatgtt ttttttttta    4320
   tattgttttt ttttttttgt tttttttttt ttttgatata gggtattatt ttgtcgtgta    4380
   ggtcggaatg tagttatttg attttggttt attgtagtat tttatttttt aggtttagat    4440
   gattttttta atttagtatt ttcgagtagt tgtaattatt ggtatatgtt attatggtta    4500
   gttaatattt gtattttttg taaagatagg gtttttttgt gttttttagg tttgttttta    4560
   gtttttaggt ttaagtaatg tgtttatttt ggttttttaa attgttagga ttataggtat    4620
   gagttattta tttattttgg agggtttatt tttgagggaa attgtattgt agaggtttag    4680
   agtataggtt ttggaatgag aatgtcgaat ttagtatttt aaattttttt tttttggtaa    4740
   gataatgaat ttttttatgt attggttttt ttgtatgtga aataatttta ttaatagtat    4800
   gtatttatat agttttgagt attaagtgag agaaaatgtg gaaaatgtat ggaatatagt    4860
   ttggtaaatg gtaagtaaat aatgagtgat attattataa ttaatataaa tgtttaaggt    4920
   tagaaaaata attaaggatg attatttgta gattatttag ttaagtttat tttttgttag    4980
   aatttttaag tagtgtattt agttatttat ttagtaaata tatacggacg atattgtttt    5040
   aggtgttaga ggtttagtta aaatatatta tggttttttt ttttttggtt ttatatttaa    5100
   gagggaaaga taaatatgaa ggttggtgat atggtttgtt tttgtttttt tatttaaatt    5160
   ttattttgaa ttgtaataat ttttatgtgt tatgggaggt atttggtggg atgtaattga    5220
   attatgggtg ttgttttttt tttatgttgt ttttttgata atgaatgttt tatgagattc    5280
   gatggtttta taaaagggaa tttttttgtt tatatttttt tgtttgtatt tatgtaagac    5340
   gtgtttttgt tttttttttg ttttttgtta tgattttgag gtttttttag ttatgtggaa    5400
   ttgtgagttt attaaatatt ttttttgttt tatataatta tttagttttg agtatgtttt    5460
   tattagtagt gtgagaaatg attaatgtag taaatttata ttgagtaatg gagtgttgtt    5520
   gtaaagattt ttaaaaatgt ggaagcgatg ttggaattgg gtaaaatgta gaggttggaa    5580
   tagttttgag ggtttagaga aagataggaa aatagaattt ggaatttttt agagatttgt    5640
   tgaatggttt tgattaaatg ttgtttattt cgaatgaaga taggaatttg ttgggaagtg    5700
   gaataaaagt gattgttgtt gcgttttagt gaagagagtg gtggtatttt gtttttgttt    5760
   tagagatttg tggaattttg aatttgagag agatgattta gggtatttgg tagaagaaat    5820
   ttttaagtag taaagtattt tagaagtgat ttgggtgttg ttaaaagtat ttatttttat    5880
   gtatttgtaa agatatggtt tggaattgga atttatgttt aaaagggaag tagagtataa    5940
   aagtttgaaa tattcgtagt tagatgatgt gttgggaaag taaaatttat tttttaagga    6000
   gaaatttaag ttaattgtag aaatttgtag aagtaacgag gagttaaatg ttaattttta    6060
   agatattaat ttttaagata atggggaata tgtttttaga gtatgttaga gattttttag    6120
   gtagtttttt ttattatagg attggagatt taagagggaa aaaaatgttt ttatgaggta    6180
   agtcgagggt ttttttgttg tatgtagttt aaaggtttag tgtttttttt tttgggttat    6240
   tgaagttatg gttaaaaggg gttaagaaat agtttgggtt gtgattttaa agggtgtaag    6300
   ttttaagttt tggtagtttt tatgtggtgt ggagtttgta ggggtataga agttaagaat    6360
   tgaggtttgg gaatttttgt ttagatttta gaggatgtat ggaaatgttt gtatgtttag    6420
   gtagaagttt gttgtagggg tgggattttt atgaagaatt tttattaggg taatgtagaa    6480
   gggaaatgtg gggtgtgagt ttttatatag agtttttatt ggggtattgt ttagtggagt    6540
   tatgagaaaa gggttattat tttttagatt ttaaaatggt agatttataa acggtttgta    6600
   ttatgtattt ggaatattta ggtatttaat gttaatttat gaaagtagtt aggagggatg    6660
   ttgtatattg taaagttacg agggtggagt tgtttaaggt tatgggattt cgatttttgt    6720
   attagtatga tcgagatatg agatatggag ttaaagatta ttttgaagtt ttaagatttg    6780
   gttgttttat tggatattgg attcgtatgg ggaatgtagt tttttttttt ggttaatttt    6840
   ttttatttgg aatgggtgta tttatttaat gtttgtattt tcgttgtatt taggaagtaa 6900
   ttaatttgtt tttgatttta taggtttata ggtggaaggg atttgttttg ttttagagaa 6960
   gattttggat ttggattttt gggttaatgt tggaatgagt gaagattttg aggaatagtt 7020
   ggaaggtagg attggttttg aaatgtgagt atatgatatt taggagaggt taggggtaga 7080
   atgatagagt ttggttttgt gtttttatta aaattttatt ttaaattgta ataatttttt 7140
   ggtgttatgg gaggtatttg gtgggaggta attgaattat ggaggtgggt tttttttatg 7200
   ttgtttttat gatagtgaaa aagttttatg agatttgatg gttttataaa ggggagtttt 7260
   tttgtatatg tttttttgtt tgtttttatg gaagatgtgt ttttgttttt tttttgtttt 7320
   ttattatgat tgtgaggttt ttttagttat gtggaattat gagtttatta aatttttttt 7380
   tttttttata aatatttagt tttgggtatg tttttattag tagtatgaga atagattaat 7440
   atatttggta tgtaatatgt aattggaata tttagattgt ttaaatgtag taagtgatga 7500
   gaatgataaa aaaaaaattg gtaaagtagg agataaaata ttgtaggatt tatgaatttt 7560
   agatagagga gttagggaag gttagaaaag gtttttgttt ttgtaattat atgtttaatt 7620
   gtttatttta gaaataaatt taatgtggtt aatttatttt ataaatttat ttgatgaaag 7680
   gtgggttaat tgtaaaatgt atacggtatg ttacgtttat taaatttttt attaaaaatt 7740
   tttttgattt gttattttaa tatttttgga gattaaagat ttagagttag aaaaatattt 7800
   attttttgat atgtgggtta ttagttttat tttttgtgtt ttaggtgaag ggttaaatgt 7860
   gtttgggttt gattagaatt tattgtgtag gaggatatcg tttttttttt ttatttttta 7920
   ttttttgata gagtttcgta ttgttatttt attggagtgt aatggtgtta ttttcgtttt 7980
   tcgggtttat gtgatttttt tgttttagtt ttttaagtag ttggaattat aggtattatt 8040
   atatttggtt aattttttgt atttttatta gagatggggt tttattatgt ttattagatt 8100
   ggttttaaat ttttgatttc gtaatttggt tattttggtt ttttaaagtg ttgggattat 8160
   agatgtgagt tattatgttt aattttttat ggtgttttat t 8201
<210> 29
   <211> 7603
   <212> DNA
   <213> Artificial sequence
<220>
   <223> chemically treated genomic DNA (Homo sapiens)
<400> 29
   ttggttacgg atattttaat ttagtggtgt tagaggaatt agatatatat atagaaatat     60
   agagtgtgga gtgggaaatt agggggttga tagtttttag agttgatagt ttcgaataga    120
   gagttattta tatatttatt gatattaagt tagtgataag tattgttttt atagattata    180
   gattaattaa aagtattttt tatgggaaat aaagggatgg gtcgaaataa agggatgggt    240
   tttggttagt tatttgtagt aggaatatat ttttaaggta tatattattt atgttattgt    300
   ttgtggttta ggaatgtttt tttgtagttt ttcgttttgg gtgggttagg tgttttttgt   360
   ttttatttcg gtaaaataat agtttttagc gtggttatta tggttattaa gaatatgtta    420
   tagtgttgta gagattttgt ttatggttag ttttcgggtt agtttatggt tagatttggg    480
   ggattgtttt taatattagg gtgtggttgt agttattttt ttaggtgtag gatttgggta    540
   tttttgtagt ttgtattttg ggggttttag gaaggatttt tttttatttt atttcgtagg    600
   tttgggggtg tttgttttcg ttgtttggtt tttttttatt tttggtattt tttttaattt   660
   tgaagtgggg ttggggttaa gttttggggt tttgaatggt aatgggaggt agaagggggt    720
   aggtttttag taaggttttt ttttaggtta gggagggttg aaggttgggg gtggggttgt    780
   tagttttgtg gattgtagta gggatttatg gtgttttttt tgggtgttcg tggataaatt    840
   ggtagttatt tttttttttt tgaggtttat aaaagttttg ggtttagtta gagtagggta    900
   gaggatggtt agaggataaa gagggtagag aggtggtggg atgattagtt gtagagagga    960
   gtattttttg ttgatagttg gagataatgg gatggttggt tgtagagaag agttattttt  1020
   tttattgaga gttgtagaga taatttgtta gtagagagaa gttatttttt ttagggtttt   1080
   ttttttattg agaattgagt attggataga tgatttgttt atagagagga gttatttatt    1140
   gtgggttttt tttgagttgt tttagtatta aataaagttt ttttttattt tgtttatttt   1200
   ttatttgttt gtgtatttta ttttttttgg acgtaggtta gaattcgggt aaaggtgttg  1260
   tagttagaga ggtttttagt tagaaaattg atattttaaa gatttcgtaa taaaggtatg    1320
   gagtaaagtg ggggtaagga ggttttggag gttgggggtg ttaaatttag aagatttgtt    1380
   attttaagat tttttgagga tttttatagg ttggggtgga ggttttagtg ttttgagttt    1440
   ttttttagtt agtatatttt tttttttttt tttatggttt atgttaattt tttaggagtt   1500
   attatttttt ttttttggat ggagtttcgt ttttgttatg taggttgtag tgtaatggta   1560
   tgatttcggt ttattgtaat ttttgttttt cgggtttaag tgattttttt gttttagttt    1620
   tttgagtatt tgggattata ggtatgtgtt attatgttcg gttaattttg tatttttagt    1680
   agagatgggg tttttttatg ttggttaggt tggttttaaa ttttcgattt taggtgattt    1740
   atttatttcg gttttataaa gtgttgagat gataggtgtg agttatcgtg tttagttagg    1800
   agttattatt ttttagttta tttagataga gggtggggaa tttggattat agattgtatg    1860
   ttaaggaaag ttaattattg ttattattat tttttattaa tgtggaatta aagtagtagt    1920
   tgatatttat ttttataaaa ggttatgaag gttttgattg gttttttacg tgtatttagt    1980
   agattgtaat taattaggaa agagattgta agacgtgttt attttattat tattaatatg    2040
   tttttagtaa ttgtgtgtag gaggaagtag aaaatgttga gtataaatat ttgtattgta    2100
   cgttattttt tttttgtaat ttgatttaaa tgtttttttg gagtagtagg ataaaagtta    2160
   tagttttatt ttttgggttt ttattttatt tttatttttt agatttagat taaatatttt    2220
   tttttttaag gaatgtttga ttgattgttt ttttttaaag tttttgtggt attaaagtga    2280
   agttttattg tggtattatt attttttgtt cgtagttata gatgttatac gatgttatat    2340
   tgagattcgg gtaatggtag ttcggggatt aagagtaagg ggttcggagt tagagagatt    2400
   tacgtttgag ttttgaattt ttgtttatag ttttgggatg gtggtaagat tttttgtttt    2460
   tttgaatttg ggttttttta tttattgaat gtggataata atgttagtta aggttgggag    2520
   ttgttatgag tttattagag aagtttagaa atggtagtat cggtcgttat agttattatg    2580
   gaaagtaggt tattttgttt tttaattgtg tttaatttag ggttttgaat tgtttgttaa    2640
   atatttaata ttattgaatt aaagttgaaa gagttaatat attttatgaa tagagtgaga    2700
   gaaagtgata gttttaataa gttgtttttt taaatggttt aataaattta atatttataa    2760
   taaaataatt aaatagaaga ttaagattta cggtttgttt tttttttttt ttaagttatt    2820
   ttttagaaaa taatatttat attttaagtt aaagtaattg taaattggaa attatagggg   2880
   gagttttagg ttgggataaa taatgattag tttggtttta atagaatagg taaagaaggt    2940
   ggtttttata ttttttatgg ttagttgggg tttttttttt attttgttgt agtattttag   3000
   ggttagggtt ttattttttt tggaattggg gtagtgagta atttagtttg ttgtgtttcg    3060
   gttttttttt tttaagaagg tttttttttt tttttttttt gagagagttt ttttttgtcg   3120
   tttaggttgg agtgtaatgg tacgattttg gtttattgta atttttgttt ttcgggttta    3180
   agcgattttt tagttttagt tttttagtag ttgggattat aggtatgtgt tattaagttt    3240
   ggcgaatttt atgtttttag ttgagatgtg gttttattat gatggttagg ttggttttga    3300
   atttttgatt ttaagtaatt tgtttatttt agttttttaa agtgttggga ttataggtgt    3360
   gagttattgt gtttggttag gatttgtttt tttttttttt tttttttttt gagaaggagt    3420
   tttgttttgt agtttaggtt ggagtgtgat ggcgtgatat tcgtttatcg taatttttat   3480
   tttttgtttt taagcgatta ttttgtttta atttttcgag tagttaggat tataggtgtg    3540
   tgttattacg tttagttaat tttttgtatt tgtagtagag atagcgtttt attattttgg    3600
   ttaagttggt ttcgtgattc gtttgtttta gttttttaaa gtgttgggat tataggcggg   3660
   agttattgta tttggtttta ggatttgttt tttttaagta tagaagtttt ttgaatgatg    3720
   tttaggatat ggaaaggata ttttaatata tttgttaatt aaagtattat aaattttgtt    3780
   tttttttttt atagataaaa tattttttta agattttgta ttttttttta gagtgttgta  3840
   tatttgagtt agtttaatta tagtgtagtg attttgattt aaatttgttg gtttaaattt    3900
   tggttttttt agtcgtttat tgtgtgattt tgggtaaatt aattaatttt tttgtgtttt   3960
   agtttttata tgttaaagta gtagttatag taatagtatt tattttatag tgttgttggg    4020
   atggtgaaat gagtaaatat ttgtaaatgt tttggtatat ggtagttgta atattagtgt    4080
   gtagtattgt ttttatgatt agaaataatg tttttaaagt tttatttttt tgttggtttg    4140
   aatgtgttgt gagaatatat attgttagtt ttgtttatta taaatttgtt gagttttttg     4200
   taggtatttt taaatgggtt tgtaatttat tatttttatt tttttttttt tttttttttt    4260
   tttttttttt tattttttaa ttttttttat aaagattgaa agtgaatttt tatttttttt   4320
   ttatttttta aaaattttat ttgtgggtcg ggtacggtgg ttttatgttt gtaattttag  4380
   tattttggga ggttaaggag ggtggattat aagtttagga gttcgagatt agtttggtta    4440
   atatggtgaa atttcgtttt tattaaaaat ataaaaaatt agtcgggcgt ggtggcgtat    4500
   gtttgtaatt ttagttattc gggaggttga ggtaggagaa ttatttgaat ataggaggta    4560
   gaggttgcgg taagtggaga tcgttttatt gtttatttgt atgtggatat aagaaagggt   4620
   taatatggtc gataattatt ttatgagttt tttggtttta tttgaaaagt gaagtgtgtt    4680
   tggggaagga gttggttagg aaaatagatg tttttttttt tgatttatta aggaaatgga    4740
   gatttaaagg aaagtttgaa gtattttgtg gtttttacgg gttcgttttt aggaagtttt    4800
   tgttttattt ggggaaattt taagttttat agtgagaaag ttgtaaatta gtttttagga    4860
   gacgttgttt gttaaagtta ttttaatttt ggaaaagggg agggaaaaag gtagataagt    4920
   agtgatgata ttagtaggaa attagtatta ggttatttag ttttgaagat ttagttttta    4980
   tttaaataat tagtaagttt tgaagttttt ttttaagtaa atgggagttt ttttggattt    5040
   tggagtatat agaggatttt atttttttta aaattttgtt tttaggtaat ttttttgaga   5100
   atcgtttatt tttagaagat tggtggagtt tgatttgaag gtatgtaatt aataattttt    5160
   tttattttag agttttatgt ttgaattttt ttttttgttt tatggtttcg tgttgtagaa    5220
   atttgtaaaa atttgttagt aatggtatta ttttttttta gatttttttg ttatgtttgg  5280
   gaaaataaaa gttaaaaaat tcggaagttt ttaatatagt tttttatcgg ggtagaaata    5340
   tgttagttta tagagataaa gtttggtaga tttattaaaa ttagagatta gaattgttaa    5400
   gaaattataa ggtaaatgtt agatgcggta gaatagaaag gtgtttttaa taaattattt    5460
   atttgatttt agggttagta gtagggaaga ttttttggat tattatatta aaaatgatat   5520
   ttttttgttt tttttaatat taaaagtaat gtgtatgttt atatatgtat aaaatttgag    5580
   aaatttaatt ggaagtttag aaggaaattt gtattattta tagttatgtt atttcgatat    5640
   aattattgtt tgttatggag gttttgattt ttgttgtgtt tttttttttt tatttattta    5700
   tttatttatt tatttattta tttatttatt tatttgagac ggagtttttt ttttttattt   5760
   tttagtattt aagttggagt gtagtggtat aattttggtt tattgtaatt ttcgtttttt    5820
   aggtttaagt aattttttta ttttagtttt ttatgaagtt gggattatag gtatatatta    5880
   ttaagtttag ttaatttttg tattttttgt agagacgggg ttttatcgtg ttgtttaggt   5940
   ttgtttcgaa tttttggttt tatagtgatt tatttgtttt agttttttga agtgtttttt   6000
   tgattataat gaaatttagt atgtagaata ttatagttta tagattagtt agatggaaat    6060
   attgatttat gtaattttta ttttttttcg ttgatttgag taatttttgt ttaaaattat   6120
   tttttttttt tgaggtttag gaggtttttt tgttgttgtt ttgttttgtt tttgaggtag    6180
   ggttttgttt tgttatttag ggtggagtgt agtggtataa ttatggttta ttggaatttt    6240
   tattttcggg gtttaaatga tttttttgtt ttagtttttt gagtagttgg gattataggc   6300
   gtgtattatt atgtttggtt aatttttgta ttttttgtag agatggggtt ttattacgtt   6360
   gtttagtttg gttttgaatt tttggtttta agtgatttat tcgttttggt tttttaaagt   6420
   gttgggatta taggcgtaag ttatgatatt ttatttgttt ttttttttat aaagtatttt   6480
   tttgttttta aaaatagtta agttttgtat attgattata tattttaagt gtttatagaa   6540
   taaatagtat tataatgaaa attttattat tgtattgttt ttttgattat gatgaaattt   6600
   agtatagaga atagtatagt ttataaattg attagatgga aatattgatt tatgtaattt   6660
   gttttttttt agtgaaaata agtttattag agaagtaaag aaataaaaga atggttattt   6720
   tatagataga gtagtcgatt tatgtaattt ttaatttttt atattgattt gagtaatttt   6780
   tgtttaaaat tatttttttt ttttgaggtt taggatattt tatttattta tttttttttt   6840
   tgaaataggg ttttattttg ttatttaggg tgaagtgtag tggtatgatt atggtttatt   6900
   gtaatttgtg ttttttaggt ttaaatgatt tttttatttt cggttttttg agtagttggg   6960
   attataggta tgtgttatta tgttttgata attttttgta tttttagtag agattaggtt   7020
   tcgttatgtt gtttaggttt gaggtttagg attatgtttt ttttttttgt aagataaagg   7080
   tttgtttttt tataggtagt agtttgaagt taagatgtaa tatatatatt ttacggtatt   7140
   ttgttgtttt tgggttgata gattataagg ataattttgt tttttttaga ttgttttgtg   7200
   tttaaaattt aaagtataga gttaagtgtt atttttgtta cgtgttggaa atgaatgtaa   7260
   gggttttaat taggttatta ggtttgagaa aaattgtgtt gtgttttttt attttaattt   7320
   tttttttttg ggaatatttt gatttgggag aaattaaggt gaattgaatt ttaatataag   7380
   tattttttaa gttttttaaa aagtaatttg taagtgttgg ggaaaagttt tttgggttgt   7440
   tgttttttga agtagtttcg tcgtaatttt tattttttag tatttttagg aaagtttaat   7500
   gtttagaacg gagaaggttg tggtgagggg agggtttggg aagttagggg gttatatgtt   7560
   tttatttttt aattttttgt tttatttttt tgtttttttt agg                     7603
<210> 30
   <211> 7603
   <212> DNA
   <213> Artificial Sequence
<220>
   <223> chemically treated genomic DNA (Homo sapiens)
<400> 30
   tttgaaaggg atagagaggt ggggtaggga gttagagagt gaggatatat ggttttttga     60
   ttttttaggt ttttttttta ttataatttt tttcgttttg ggtattagat ttttttagaa    120
   gtgttgaaaa ataaaaatta cggcgaggtt attttaaaaa gtaatagttt agaaaatttt    180
   tttttaatat ttgtaagtta ttttttagga aatttggagg gtgtttatat tggaatttaa    240
   tttattttga ttttttttaa attaaaatgt ttttagagag agaaagttaa gatggaggga    300
   tatagtataa ttttttttaa gtttggtagt ttgattggag tttttatatt tatttttagt    360
   acgtggtaga aatagtattt ggttttatgt tttaagtttt agatataaag taatttgggg    420
   gaggtagggt tatttttatg atttattaat ttaaagatag taaggtatcg tgaggtatgt    480
   gtgttatatt ttaattttag attgttattt gtaagagggt aggtttttat tttatagaaa    540
   gggaaggtat aattttagat tttaagtttg agtaatatgg cgagatttgg tttttattaa    600
   aaatataaaa aattattagg gtatggtggt atatgtttgt ggttttaatt atttaggagg    660
   tcgaggatgg aaggattatt tgagtttggg gagtataggt tgtagtgagt tatgattatg    720
   ttattgtatt ttattttggg tgatagagtg agattttgtt ttaaaaagaa aaataaataa    780
   ataaaatatt ttagatttta ggaggaaagg gtggttttag ataagagttg tttaaattag    840
   tgtaggaagt taaaaattat atgagtcggt tgttttgttt atggagtagt tatttttttg    900
   ttttttattt tttttaataa atttgttttt attaaaaaaa aataaattat atgagttaat    960
   gtttttattt gattagttta taaattatgt tattttttat attaaatttt attataatta   1020
   gaggagtaat gtagtgataa aatttttatt gtaatattat ttattttata agtatttgag   1080
   gtatgtagtt aatatgtaga atttggttat ttttagaaat aagaaaatat tttataaagg   1140
   aaggaatagg tggggtgtta tggtttacgt ttataatttt agtattttgg gaggttaagg   1200
   cgggtggatt atttgaggtt aggagtttaa gattagattg ggtaacgtgg taaaatttta   1260
   tttttataaa aaatataaaa attagttagg tatggtggtg tacgtttgtg gttttaatta   1320
   tttagagggt tgaagtagaa ggattatttg agtttcgggg atggaggttt tagtgagtta   1380
   tgattgtgtt attgtatttt attttgggtg atagagtaag attttgtttt aaaaataaaa   1440
   taaaataata ataaaaaaat tttttagatt ttagaaggaa agggtgattt tagataagag   1500
   ttgtttaaat tagcgaagga agataaaaat tatatgagtt aatgttttta tttgattagt   1560
   ttataaatta tggtatttta tatattaaat tttattataa ttagagaagt attttaggag   1620
   attgaggtag gtggattatt gtgaggttag gaattcggga taagtttggg taatacggtg   1680
   aaatttcgtt tttataaaaa atataaaaat tagttgggtt tggtggtgta tgtttgtggt   1740
   tttagtttta taggaagttg agatgggagg attgtttgaa tttgggaggc ggaagttgta   1800
   gtaagttaag attgtgttat tgtattttag tttgagtgtt ggagggtgaa agagggagat   1860
   ttcgttttaa ataaataaat agataaataa ataaataaat aaataaataa ataaaggaag   1920
   gaagatatag taaaaattaa aatttttata ataaatagtg gttatatcga ggtgatataa   1980
   ttatagataa tataaatttt tttttgggtt tttaattgaa ttttttaaat tttatgtata   2040
   tgtgagtata tatattattt ttggtgttaa aagaaataaa aaaatgttat ttttaatgtg   2100
   ataatttagg ggattttttt tgttattagt tttgaggtta aatgagtggt ttattggaga 2160
   tattttttta ttttatcgta tttagtattt gttttgtggt tttttgataa ttttgatttt 2220
   tagttttagt gagtttatta aattttgttt ttgtgagtta atatattttt gtttcggtaa 2280
   aaggttatgt tggaggtttt cggatttttt aatttttgtt tttttaggta tgataaagaa 2340
   atttaagaaa aaatagtgtt attgttgata agtttttgta agtttttata atacgaagtt 2400
   atgaagtaaa ggaaaagatt taagtatgag attttggagt aagaagggtt gttaattata 2460
   tatttttaga ttaagtttta ttaatttttt ggaagtaaac ggtttttagg gaaattgttt 2520
   gaaaataaag ttttaaagaa agtagaattt tttgtgtgtt ttaaggttta aagaagtttt 2580
   tatttgtttg ggaagagatt ttaagatttg ttggttgttt aagtgaaaat tgaattttta 2640
   ggattgggta gtttgatatt agttttttgt tgatgttatt attgtttatt tgtttttttt 2700
   tttttttttt tttagggtta agataatttt gataaatagc gttttttaaa ggttaattta 2760
   taattttttt attgtagagt ttggggtttt tttaggtaaa gtaaaagttt tttagaaacg 2820
   aattcgtgga agttataaag tgttttaggt ttttttttgg atttttattt ttttggtaag 2880
   ttaaaaaggg aagtatttgt ttttttaatt aatttttttt ttaaatatat tttatttttt 2940
   aaataaagtt aaaaagttta tgaaatagtt gtcggttatg ttaatttttt tttatgttta 3000
   tatgtaaata aataatggag cgatttttat ttatcgtaat ttttgttttt tgtgtttaag 3060
   tgattttttt gttttaattt ttcgagtagt tgggattata agtatgcgtt attacgttcg 3120
   gttaattttt tgtattttta gtagagacga ggttttatta tgttggttag gttggtttcg 3180
   aatttttgag tttgtgattt attttttttg gttttttaaa gtgttgggat tataggtatg 3240
   gagttatcgt gttcggttta taaataaaat ttttaaaaga taaaagaaaa gtggggattt 3300
   atttttagtt tttgtgggag aaattaagaa ataaaaagaa aaagaaagga aaaaaaaaag 3360
   aaaagtgggg atggtgaatt ataagtttat ttggaggtat ttgtaagaga tttaataaat 3420
   ttgtggtagg taaaattgat agtatgtgtt tttatagtat atttaaatta gtaggaaagt 3480
   gagattttga agatattatt tttaattata aaaataatat tgtatattaa tattgtaatt 3540
   attatgtgtt agggtattta taaatattta tttattttat tattttaata atattgtaag 3600
   ataaatatta ttattatgat tgttgtttta atatgtgaaa attaaggtat agaaaggtta 3660
   attaatttgt ttaaggttat atagtgagcg gttggaagag ttaagattta aattagtaag 3720
   tttggattaa agttattata ttataattaa gttaatttaa atgtgtagta ttttggaaag 3780
   aggtatagag ttttagaaag atgttttatt tatggggaaa aaaagtaaga tttatgatgt 3840
   tttgattagt aaatgtatta aaatgttttt tttatgtttt aggtattatt tagagaattt 3900
   ttgtgtttaa gaaagataag ttttggggtt aggtgtagtg gttttcgttt gtaattttag 3960
   tattttggga ggttgaggta ggcggattac gagattagtt tggttaagat ggtgaaacgt 4020
   tgtttttatt ataaatataa aaaattagtt gggcgtggtg gtatatattt gtaattttag 4080
   ttgttcggga ggttgaggta ggataatcgt ttgaaggtag gaggtggagg ttgcggtgag 4140
   cggatattac gttattatat tttagtttgg gttatagagt aagatttttt tttaaaaaaa 4200
   aaaagaaaag aaaaagataa gttttggtta ggtatagtgg tttatatttg taattttagt 4260
   attttgggag gttgaggtgg gtagattgtt tgaggttagg agtttaagat tagtttggtt 4320
   attatggtga aattatattt taattaaaaa tataaaattc gttaggtttg gtggtatatg 4380
   tttgtaattt tagttattag gaggttgagg ttggagaatc gtttgaattc gggaggtaga 4440
   ggttgtagtg agttaaaatc gtgttattgt attttagttt gggcgataga gaaagatttt 4500
   tttaaaaaaa aaaaaaaaaa aaattttttt gggaaagggg ggtcggagta tagtaggttg 4560
   ggttatttat tattttagtt ttagggaagg tggggttttg attttagaat gttgtagtag 4620
   agtgaggagg gggttttaat tgattataaa gagtgtaggg attatttttt ttgtttgttt 4680
   tgttgggatt aggttagtta ttatttgttt tagtttgggg ttttttttgt ggtttttaat 4740
   ttgtagttat tttaatttaa aatataaata ttattttttg gaaaataatt taaaaaggaa 4800
   gaaagataag tcgtaagttt tggtttttta tttagttgtt ttattataaa tattaaattt 4860
   attgggttat ttagggaggt aatttattga aattattatt tttttttatt ttgtttataa 4920
   agtatattaa tttttttaat tttgatttaa taatattaaa tatttagtag gtaatttaaa 4980
   gttttgaatt gggtataatt ggaaagtaaa gtgatttgtt ttttatagta attatggcga 5040
   tcggtattat tatttttaaa tttttttgat gaatttataa taatttttag ttttagttgg 5100
   tattattatt tatatttaat agatgaggaa atttagattt agagaggtaa ggaattttgt 5160
   tattatttta aagttatgag taagaattta gggtttaaac gtgggttttt ttgatttcga 5220
   attttttgtt tttgattttc gggttattat tgttcgaatt ttagtatgat atcgtgtagt 5280
   atttgtaatt gcgggtaggg ggtggtgatg ttatagtgag gttttatttt ggtgttatag 5340
   gggttttaag aagggataat tagttagata ttttttggag gaggaagtat ttgatttagg 5400
   tttggaaggt aaaggtagaa taaagattta ggaagtaaaa ttgtaatttt tattttattg 5460
   ttttagggaa atatttgaat taggttatag aggggagatg gcgtgtagtg tagatatttg 5520
   tatttagtat tttttatttt tttttatata tagttattga gggtatgttg atggtggtag 5580
   aataaatacg ttttataatt ttttttttga ttggttgtag tttattgagt gtacgtgaag 5640
   ggttaattaa aatttttatg gttttttgta gaggtggatg ttaattattg ttttagtttt 5700
   atattagtag ggggtagtgg tagtagtggt tggttttttt tgatatataa tttgtggttt 5760
   aagtttttta ttttttgttt gggtagatta aagggtaatg atttttggtt gggtacggtg 5820
   gtttatattt gttattttag tattttgtga ggtcgaggtg ggtgaattat ttgaggtcgg 5880
   gagtttgaga ttagtttgat taatatggag aaattttatt tttattaaaa atataaaatt 5940
   agtcgggtat ggtggtatat gtttgtaatt ttagatattt aggaggttga ggtaggagaa 6000
   ttatttgaat tcgagaggta gaggttgtag tgagtcgaga ttatgttatt gtattgtagt 6060
   ttgtataata agagcgaaat tttatttaaa aaaaaagagt aatgattttt aaaggattgg 6120
   tataagttat gggggaggag gaagggatat attggttaga gaagagttta gagtattgaa 6180
   atttttattt taatttatga gggtttttag aaggttttga agtagtaagt tttttagatt 6240
   tgatattttt aatttttaag atttttttat ttttattttg ttttatattt ttgttacggg 6300
   atttttgggg tattaatttt ttggttggaa atttttttga ttgtagtatt tttgttcgag 6360
   ttttggtttg cgtttaggaa gaatgaggta tatagataag tgaagggtga ataagatgaa 6420
   gaagagtttt atttagtgtt agaatagttt agaggaggtt tatagtgggt agtttttttt 6480
   tgtaggtagg ttatttgttt agtgtttagt ttttagtgga gaggaggttt tggaaagggt 6540
   ggtttttttt tgttggtagg ttgtttttgt agtttttagt ggagaaggta gttttttttt 6600
   gtagttagtt attttattgt ttttagttat tagtagagag tatttttttt tgtagttggt 6660
   tattttatta tttttttgtt ttttttgttt tttggttatt ttttgttttg ttttggttga 6720
   gtttagggtt tttatggatt ttagagagga ggaagtggtt gttaatttgt ttacgggtat 6780
   ttagaagagg tattatgagt ttttattata atttatagga ttggtagttt tatttttagt 6840
   ttttagtttt ttttggtttg aaggaggatt ttattgggga tttgtttttt tttgtttttt 6900
   attgttattt aaggttttag gatttggttt taattttatt ttaagattgg agaaggtgtt 6960
   aggagtgaag agaggttagg tagcgggagt agatattttt aagtttgcgg gatggggtgg 7020
   gagggggttt tttttggggt ttttagggtg taggttgtag agatgtttag gttttgtatt 7080
   taggagggtg gttgtagtta tattttggtg ttgggaatag ttttttaaat ttggttataa 7140
   attgattcga aaattggtta taaataaaat ttttgtagta ttgtgatatg tttttgatgg 7200
   ttatgatggt tacgttgaag gttgttgttt tatcggaatg agggtaagga atatttggtt 7260
   tatttagggc ggaaaattgt aaaaaggtat ttttaaatta taaataatag tatgagtgat 7320
   atgtgtttta aggatatgtt tttgttgtag ataattagtt agaatttatt tttttgtttc 7380
   ggtttatttt tttgtttttt ataagggata tttttaatta atttataatt tatagaaata 7440
   atgtttatta ttggtttgat gttaataaat atgtgggtaa ttttttgttc ggagttgtta 7500
   gttttaaagg ttgttagttt tttgattttt tattttatat tttatatttt tgtatgtgtg 7560
   tttaattttt ttagtattat tgggttaggg tgttcgtgat taa 7603
<210> 31
   <211> 6853
   <212> DNA
   <213> Artificial sequence
<220>
   <223> chemically treated genomic DNA (Homo sapiens)
<400> 31
   ttatttaaat agaaggtaaa ttgtttaggg ttgtaattaa tatataatat gcgtcgagag 60
   ggaaaggtta atgttaattt cggttttaag tataaagatt attgggagtt aggggtgtat 120
   ttttattagg gtatagtatg ggtgggtgga gagagtaaaa attagaataa attaaggttt 180
   agtagttgga agttagtttt agggagaaaa ggggcggaga ttgattgggt tagaataggg 240
   attgggatag aaaaagaggg tggggttagt gggttttatt tattcgtttc ggtttttttt 300
   attatttttt attttattta ttttttatag tttgtggaga taggtgagcg acgaattttt 360
   gagataggtg tgggtgcgag ggtcgggagg gttatgggat tgggatcgag gtgtgaggag 420
   ggaatttgta attttttgtt atatagagcg ttggtaattt ttgataggtt gtttttgggg 480
   tatgggttgt ttcgggttgt tgttgttata aggaaagaaa agagtttttt tgtttatcgt 540
   tttttagtta ttgggttatt ttttggtagg aaatttgtaa attgagttta ataagttagg 600
   attagtagag ggtagaggag ggttttggta gatgtggggt ttagaagagg ataggagtta 660
   ttagggtttt cggttattgt gttgggtttt tgtttgtata attgtttttt aagtagttgt 720
   gtttttgtgg ttttggtgcg tttgtgtgta tttttttttt ttatttggag tatgggttaa 780
   tatcggagga aaggaaaaga tagagttaga tagggtaagt ggggtttttt tttttttttt 840
   ttttaacggg gttggattga gggtttttgg ttggggaggt gggggtggag atttagtagg 900
   agtaataata gaggaagggt agggtttgtt ttattatttg aattttagag atgttagtgt 960
   gtattgaagt tttaggtagg gcgtgtttag gatcggattt tggatggtgg taagggataa 1020
   agttggaagg gagattatag ggaaggagaa aggaaagaag gtagagttat gatatagtta 1080
   atttatagaa tggttgggag ttaaagtttt tgggttggtt ttttggttat tttatatatt 1140
   ttaagatata gagagaattt cggaattgga ggggatttta tagatgattt atttgagatt 1200
   gaagaattta gaaattaaat aaagaaatat tatatggtta ttttggtatt tgatatagtt 1260
   atgatattaa ttgttagtgt agatataata gttgtgtgtt tttttgtagg agtttgggga 1320
   ggggttatgg tgttaatgta tttattgggg agattggaga agtcgttttt ttttttgtgt 1380
   tgcgtttttt ttttattggg gttggttttg ttgggtataa agacggatat tattttcgtt 1440
   gtttattttt tttttatatt gggtggtttt tttttgtttg tttatttttt ggttcggttt 1500
   ttggaatggg ggtttcggtt ttagttttaa ttaatgtaga ttgagagttt agggttttta 1560
   ggtaatgtac ggtgagttaa gggtgggtat cgtaagagga atatattttg ggtttatttt 1620
   tttttttttt ttgtagtttt tgaggtagag agtttttggt tttttgttat tggtattagc 1680
   gggaggaggg gttagaggtt aatagaggat tttttgttta tagtagataa aaaaaaggga 1740
   aaaagggttt tttttatgga agttattaat ggttttattt tttttttttt agggataatg 1800
   aagtttttga agtgttagtt tatgaagagg tcgtggtggg attagaattt tagtgtcgtt 1860
   tttaagagtt ggattaatta tttttttata gtattgttgt gatattttta gtatttgagg 1920
   aggaataatt tagttattta gaggggttta ggagagttaa attggaatag aggcgaatgg 1980
   ttttagaggg gtttatggtt taggaaggaa gttttggaag agttttaatt aattttcggt 2040
   ttcggggatt acgggttgtg tttattgttt ttgatttgta gagtttggcg gtagttttta 2100
   tattagagtt tttgatttta ttttttgttt atgatgtttg ttttggttat tttgatgatg    2160
   atagtgtttt ttatgaggat aattgggtat ttttttaaat gattttttta agattttttt    2220
   tttttttata ttagatttcg tttatttgat taatattttt tagcgtttat tatgtgttag    2280
   aaataagtgt ttttgtttgg atattataaa tggggatttg gattttgagg agagttaggt    2340
   tacggtaagt tttttttagt tgagatatgg gtggtataat ttgagttttt tggtaatatt    2400
   tggtgattta ttttatattt aatattttta gcgttagatt gaggatgagg tagggaggtg    2460
   atttagagaa ggcggagaag gaagaagtaa tttttgagtg gcggttattg tttttgtttt    2520
   aggtgttgtt cgagttgtta gaatttttag gtttgatagt tttgtgagtt attattgaaa    2580
   aatgaggatt ttaagagtta gaggagtttg ataatgtgta cgagggtata ttgttagtaa    2640
   ataatattaa aataattgga atgaattttt gattttaaag tttattgtgt ttttaatata    2700
   aggtaatagg aagttaagta atttgtatgt tttttaattt ttgtttaggg atagttgttg    2760
   gtgtaggatg gaagattttt tataaataga atgggcgatt aattgtgttt atagaggggg    2820
   tgggttgagt atgggtacgt atgtttttag ttttttggac gagtaaagtt agttaaagcg    2880
   ttggtgattt ttgtttcgcg tgcgtagtag tgtttgtgtt ttttttgttt aggggttaga    2940
   gagtagtttt tagtgtaggg tttttatttt atttgaaaat agtggagtta gtgattttag    3000
   gtggagggat ttttagtttt aataatgggt tggtatgaag gttagaagga gtgaaatatg    3060
   tgaatttttt ggcgaggaga tttttttttg aattatttat atgtagtttc ggggttattt    3120
   gcgaagagtt tttaatagtt tttgttttat ttttaagttt tgatcgttag tttttatttt    3180
   agtcgtcggt tttgattttt gagattttaa tattgagtat taggtttttt ggaattgtgt    3240
   gttgcggttg tgatgtaggt ttcggtcgtt ggaagttgtt aaattatagt tgacgttttt    3300
   ttttaagtta gttttttttt ttcgcgcggg tatttgttta gggttttgtc gcgtttttag    3360
   tttttttcgt agtttggttt taatcgttta attttaataa agtaaaattt tagttacgtt    3420
   cgttttcgtg aagttatcgt tataggtcgg ttagggggcg cgagaggtat cggggtgatt    3480
   ttcgcgggaa tcgataatta atcggatttt taggtcgaac ggagtatatt cgttcgtttt    3540
   cgtttttttt tcgttttttg tttcgtttcg tttttttttt tttttttgtt tgttcgtcga    3600
   gtattttatt ttattttatt ggatcgcgcg gtcgtcgtta gagttttgcg tttgcgtacg    3660
   tatcgggtcg gggattgggt ggtttggtgt gtgggcgcgg tagggcgtag cgtaggcgta    3720
   gtgtgcgttc gcgtttgagg ggagggatgt gggggaagcg acggttttcg gtttgtttgg    3780
   gttgtgggcg gtgcgtagcg gagagttcgg gaaaagcggg aaatggcggc gtcgagcgcg    3840
   gggttttggt ttatttttta gtataaggag ttgatggtcg ttgatagggg acgtaggtaa    3900
   gtagggaacg gttttagggt tttttagggg agtttaggga aggggagcgt ggtttttagg    3960
   cgtgcggttt tgtagttttt ttttttcgga gtttcggaaa gtagtcgtgt tcgcgttttt    4020
   tgttaagggt gggacgttcg ttcgagtcgt ttcgagtttg atttcgcgta agggttcgcg    4080
   ggcggatttt ttggttttgt ttttcgtggg aaaagattgt tttagttttt ttttgtttaa    4140
   tttacgaggg ttttaaaggg gaggtttttg tagttttgtt cgtttttggg tttttttgaa    4200
   gattttttat tttttttttt ggttttttta tgagggaaat tataaaaaga ttatattttg    4260
   tttttttaaa aatcgacgag attttttgta gatttcggtg ttttgtttga tgtgtatttg    4320
   atttgtatcg attgtattat gatttgtatg gttttgtttt ggtagaataa gggggttttt    4380
   gtggtttcga gtgtttatgt ttttgtttcg gttattttat tttatggtag ttattattta    4440
   ggagaatatt gtaatgtttt ttgttcgaga ttgaagatta aggttttcgt aaagggaatt    4500
   tgtagaagga tatagaatta agacgtagga atttcgtggt gtttttcgtt tttttttttt    4560
   ttttagttgt gttttgaaat tttaaatgat tggaaatttt tataatgttt acgtttaaga    4620
   tttttatatt atagagtaga ggaaggagcg ggtgtgtaga gggggtgatg ttttagatta    4680
   aaattaaaga gttgattgaa aatttaagtt tggaattcga gagagttaag aattataagt    4740
   tagtaagttg taagtatggg tgagattgtt tagggaaagg ttttttgaag gggcgttttt    4800
   aatggaatat taatattaag ggtaggttaa aggtttttta aagaaaatag gaattgggta    4860
   ggtagagaga agatttttaa agaatgttgt tatggaaatt agaggaagag aacgatttaa    4920
   ggagcgaata ttattggtaa attaatggaa gaaatttgtt gtattattgg ttagtatttt    4980
   agttgtagtg ttatatgtga tcgttattat tgttttgacg tattcgaaag tgatttattt    5040
   ttaaataggt ttttttattt gtatagtttt tttttttgta gttagttttt tttgaaagtt    5100
   ttgggtaatg gaaatattat agtaaaaaga agaaaaaatg gagttttaga tttatttttt    5160
   taaattttag atttattggt tgtttttaat ttattttatt tgttttagga ttttatttgt    5220
   tattttagtt tttttagttt ttttaggatt tagttttttt tttttttaat tataaatttt    5280
   ttatttaata gtatttgtaa gttaagtgta tttattttat agatgtgtgg tattttattg    5340
   attttaagat atatattttt tttttttttt tttttttttt tttttttttt tggacggagt    5400
   ttttgttgtg ttatttaggt tggagtgtag tggaatgatt ttagtttatt gtaattttta    5460
   tttttaaggt ttaagcgatt tttttgtttt agtttttcga gtagttagga ttataggtat    5520
   gtagtattat attcggttaa ttttttgtat ttttagtaga gatagggttt tattatgttg    5580
   gttaggttgg ttttgaattt ttgattttag gtgatttgtt tattttagtt ttttaaagtg    5640
   tagggattat aggtatgagt tatcgtgttc ggtttttttt tatgttttaa tattttagaa    5700
   attagaatgt attttagagt aggtttttaa gttataaatg gaagttattg tatttttatt    5760
   gttattaggt gttatttttt ttgttgttat atatgtggaa aatgtgacgg ggtaggttag    5820
   agaattttgg atttatgtaa attaggtatg tagatgatat attggtaata ggattatttt    5880
   agtttataag tagaagttga tttttttgta agtttgtagt aaaattagtt ggaggtgata    5940
   gtatatgttt gtagttttaa ttatttggga ggttgaggta ggtggatcgt ttgagtttag    6000
   gagttttggg ttatagtgtg ttatgttagt tacgtgttta tattaagttt agtattagtg    6060
   tggtgatgtt ttgggagtta gggtttatta ggttgtttaa ggaagggtga attcgtttag    6120
   gttaaaaatg gagtaagtta aaatttttgt gttgttttgt aatgggatta tatttgtgaa    6180
   tagttattgt attttagttt gggtggtata gtgagatttt tttaaagtaa atagataaat    6240
   atttatttat tgtgattttt tgttatattt tttgtttatt tatattttaa taggattcgt      6300
   tattttgtta ttggaattta aaagttttaa aaaatatgta attatatgtt tatattatta      6360
   gtgaaagaga gttagtgttt gttttattta ttttttgtta aagttattgg tttgagtttt      6420
   tattatagtg ttttaatttt tatttaagat tattataaat ttatattatt tttattagtt      6480
   tagataatgt gagatgtttt ttgaaataat tttttttatt aaaattggtt tattagagaa      6540
   gtattttttt ttataaatat gtgtatttgg gggtattgtt ttgtttaaga tagatatttt      6600
   ttttttttag ttagattatt tattttagat gattttagtt gaatataaat tataaggaat      6660
   tagatattat ttttttatga ttaatgtgtt tttgtttagt atatttttgt tttaattaga      6720
   tttaagaaat atgtataatt ttggttattg taagttttat tttttttgtt ttttttattt      6780
   atgttttttt tattttattt ttttgtgtta aattgtgatt tgatagtttt tttttttatc      6840
   gtttatttgt agg                                                         6853
<210> 32
   <211> 6853
   <212> DNA
   <213> Artificial Sequence
<220>
   <223> chemically treated genomic DNA (Homo sapiens)
<400> 32
   tttatagata aacggtagaa ggaaaggttg ttaagttata atttgatata gaagagtgga      60
   gtggaagagg tatgaatagg aagaataaaa agaataagat ttataataat taagattatg     120
   tatatttttt aaatttggtt aaagtaaaga tgtattaagt aggaatatat tgattataag     180
   aaaataatgt ttaatttttt atggtttatg tttagttgag attatttgag atgaatggtt     240
   tgattagagg agggaaatat ttgttttaag taaaataata tttttaagta tatatattta     300
   tgagaaggaa tatttttttg atagattaat tttaataaag agaattattt tagaaaatat     360
   tttatattat ttaagttggt gaaagtgatg taagtttata atgattttag atgagggtta     420
   aaatattatg gtaaaaattt aagttaatgg ttttaataga aaataaatga ggtagatatt     480
   aatttttttt tattagtgat ataaatatat aattatatgt tttttaaaat ttttaagttt     540
   tagtgataaa atggcgaatt ttgttaagat gtggataagt agaaagtatg atagaaagtt     600
   atagtggata ggtatttatt tatttatttt aaaagagttt tattgtgtta tttaggttgg     660
   agtgtagtgg ttatttatag gtatgatttt attatagagt agtataggag ttttgatttg     720
   ttttattttt gatttgggcg ggtttatttt tttttaggta atttggtgga ttttgatttt     780
   taggatatta ttatattgat gttgaattta atgtggatac gtgattggta tagtatatta     840
   tagtttagaa tttttggatt taagcgattt atttgtttta gttttttaag tagttgggat     900
   tataggtatg tgttgttatt tttagttaat tttgttatag gtttgtaaag agattagttt     960
   ttgtttataa attaaaataa ttttattgtt aatatgttat ttatatgttt agtttatata    1020
   aatttagagt tttttagttt gtttcgttat attttttata tatgtaatag taggaagggt    1080
   ggtatttgat agtagtgaaa atataataat ttttatttat aatttaaaaa tttattttaa    1140
   aatgtatttt gatttttaag gtgttaaaat atgaggggag gtcgggtacg gtggtttatg    1200
   tttgtaattt ttgtattttg ggaggttaag gtgggtagat tatttgaggt taggagttta    1260
   agattagttt ggttaatatg gtgaaatttt gtttttatta aaaatataaa aaattagtcg    1320
   agtgtggtgt tgtatgtttg tagttttagt tattcgggag gttgagatag gagaatcgtt    1380
   tgaattttgg agatggaggt tgtagtgagt tgagattatt ttattgtatt ttagtttggg    1440
   tgatatagta aagatttcgt ttaaaaaaaa aaaaaaaaaa aaaaagggag ggaaaaaatg    1500
   tgtattttag agttagtgaa atattatata tttatggaat ggatgtattt ggtttgtaga    1560
   tgttattggg tgagagattt atagttagaa gagggaagag ttgggttttg gaagaattgg    1620
   gagaattaag atagtaggtg ggattttaaa atagatgaag tgggttggaa gtagttaatg    1680
   aatttgaggt ttggaaaaat gagtttgaaa ttttattttt tttttttttt attgtaatat    1740
   ttttattatt taagattttt aggaaaaatt ggttataaga aaaagagtta tataaataaa    1800
   aaaatttatt taagaatgaa ttattttcga atacgttaaa gtagtaataa cgattatata    1860
   taatattgta gttgggatat taattagtgg tgtagtagat tttttttatt agtttattag    1920
   tagtattcgt tttttaaatc gttttttttt tttggttttt atgatagtat tttttgggag    1980
   tttttttttt gtttatttag tttttgtttt ttttagaggg tttttggttt gtttttaata    2040
   ttgatatttt attagggacg tttttttaaa gagttttttt ttagataatt ttatttatat    2100
   ttgtaattta ttgatttata gtttttaatt ttttcgagtt ttagatttga atttttaatt    2160
   aattttttag ttttaatttg aagtattatt ttttttgtat attcgttttt ttttttattt    2220
   tgtagtatga gagttttaaa cgtgaatatt ataaaagttt ttaattattt agaattttag    2280
   gatataattg gaaggggagg aggggcgaag ggtattacgg agtttttacg ttttaatttt    2340
   gtgttttttt gtaggttttt tttgcgaaga ttttaatttt tagtttcgag taggaggtat    2400
   tataatgttt ttttgggtga tgattgttat gaaatggaat gatcggggta gaggtatgga    2460
   tattcggagt tataggaatt tttttgtttt gttaaaataa agttatgtag attatgatat    2520
   aatcgatata aattaaatgt atattaggta gagtatcgag gtttgtagaa ggtttcgtcg    2580
   atttttaagg agatagagta tggttttttt atagtttttt ttataaggga gttagaggaa    2640
   aggatgaaga atttttaaga gaatttaaga gcgggtagga ttgtaaagat ttttttttta    2700
   gaattttcgt gggttgaata aagggaggtt ggggtagttt ttttttacga gagataaggt    2760
   taagaggttc gttcgcgggt ttttgcgcgg agttaggttc ggagcggttc gggcgggcgt    2820
   tttatttttg gtaggaagcg cggatacggt tgtttttcgg ggtttcgagg ggagggggtt 2880
   gtagggtcgt acgtttgggg gttacgtttt ttttttttgg gtttttttaa ggagttttaa 2940
   gatcgttttt tatttatttg cgttttttgt tagcggttat tagttttttg tgttggaagg 3000
   tggattaaga tttcgcgttc ggcgtcgtta tttttcgttt ttttcgggtt tttcgttgcg 3060
   tatcgtttat agtttaaata aatcgggggt cgtcgttttt tttatatttt tttttttaga 3120
   cgcggacgta tattgcgttt gcgttgcgtt ttgtcgcgtt tatatattag gttatttagt 3180
   tttcggttcg gtgcgtgcgt aggcgtagag ttttagcggc ggtcgcgcgg tttaatgggg 3240
   tgaagtgggg tgttcggcgg atagataaaa ggaggggaag gagcggggcg gggtaaaggg 3300
   cggggaaaga gcgagggcgg gcgggtgtgt ttcgttcggt ttgggaattc gattggttat 3360
   cgattttcgc ggaaattatt tcggtgtttt tcgcgttttt tggtcggttt atggcgataa 3420
   ttttacggag acgggcgtgg ttggagtttt gttttattga agttgaacgg ttggggttag 3480
   gttgcgaagg agattgagag cgcgataagg ttttggatag atgttcgcgc ggggaaggaa 3540
   ggttggttta ggagggggcg ttagttatgg tttagtagtt tttagcgatc gaaatttata 3600
   ttatagtcgt agtatataat tttaggaaat ttggtgttta atattgggat tttaggaatt 3660
   aggatcggcg gttggggtga aagttagcgg ttagagttta ggagtgaggt agaagttgtt 3720
   agagattttt cgtaagtgat ttcggggtta tatatggatg atttaagagg gagttttttc 3780
   gttagaaagt ttatatgttt tatttttttt ggtttttata ttaatttatt gttaaaattg 3840
   gaagtttttt tatttggggt tattgatttt attgttttta gataggatgg ggattttgta 3900
   ttggagattg ttttttagtt tttgggtagg agaggtatag atattgttac gtacgcggag 3960
   tagggattat tagcgttttg attgattttg ttcgtttagg aggttgggga tatgcgtgtt 4020
   tatgtttagt ttattttttt tgtagatata attggtcgtt tattttgttt ataggaagtt 4080
   ttttattttg tattagtagt tgtttttaga tagggattgg gagatatgta agttgtttgg 4140
   ttttttatta ttttgtgttg aaaatataat aggttttggg attaggagtt tattttagtt 4200
   attttaatgt tatttattag tagtgtgttt tcgtgtatat tattaaattt ttttgatttt 4260
   tggaattttt attttttaat aataatttat aaagttgtta agtttaaggg ttttaatagt 4320
   tcgaatagta tttggaatag aagtaatagt cgttatttag aggttatttt tttttttttc 4380
   gttttttttg gattattttt ttattttatt tttaatttaa cgttggaaat attggatatg 4440
   gggtaggtta ttaaatgttg ttagaagatt taaattatgt tatttatatt ttagttggga 4500
   agggtttatc gtggtttgat tttttttagg gtttaagttt ttatttatga tgtttaggta 4560
   gaaatatttg tttttgatat atagtaggcg ttggaaaatg ttagttaaat gaacgaggtt 4620
   tggtgtggag agaagagaaa ttttgggaga gttatttaag gggtgtttta gttgttttta 4680
   taaaaaatat tattattatt agggtgatta aagtagatat tataggtagg gggtggagtt 4740
   agaggtttta atgtggggat tgtcgttaag ttttgtagat taggagtagt ggatatagtt 4800
   cgtggttttc gaagtcgaag gttgattgga gtttttttag ggtttttttt ttgggttatg 4860
   gatttttttg aggttattcg tttttgtttt agtttggttt ttttggattt ttttggatgg 4920
   ttaggttgtt tttttttagg tgttgggggt attataatag tgttgtaggg gggtggttgg 4980
   tttaattttt gggggcggta ttgggatttt agttttatta cggttttttt atagattggt 5040
   attttaaagg ttttattgtt tttgagggaa gagaaataag gttattggtg gtttttatga 5100
   aagaagtttt tttttttttt tttttgtttg ttgtgggtaa aagatttttt gttggttttt 5160
   gatttttttt ttcgttgatg ttaatgatag gaaattagag attttttgtt ttagagatta 5220
   tagggagaga gggaggatgg gtttaaggtg tatttttttt acgatgttta tttttaattt 5280
   atcgtgtatt gtttgagggt tttgggtttt tagtttgtat tgattggagt tgggatcgaa 5340
   gtttttattt tagaaatcgg attaggagat aggtaaataa gaagaagtta tttaatgtga 5400
   gaaagaaata agtaacgggg gtgatgttcg tttttatgtt tagtaaagtt agttttagta 5460
   ggaaggaggc gtagtatagg aggagaagcg gtttttttag tttttttagt aagtgtattg 5520
   gtattatggt ttttttttag gtttttgtaa aaagatatat agttattgtg tttatattag 5580
   taattggtgt tatggttgtg ttagatatta aagtaattat atgatatttt tttgtttggt 5640
   ttttaggttt tttaatttta aataaattat ttgtagagtt ttttttagtt tcgaaatttt 5700
   ttttatgttt tggagtgtgt ggaatagtta ggaaattagt ttaggagttt tgatttttag 5760
   ttattttgta agttgattgt gttatggttt tgtttttttt tttttttttt tttttgtagt 5820
   ttttttttta gttttgtttt ttattattat ttaggattcg gttttgggta cgttttgttt 5880
   ggggttttag tgtatattgg tatttttgga atttaggtga tggggtaggt tttgtttttt 5940
   ttttgttatt gtttttattg gatttttatt tttatttttt tagttagagg tttttaattt 6000
   agtttcgtta gagagaagag gggagggagt tttatttatt ttgtttgatt ttgttttttt 6060
   ttttttttcg gtgttagttt atgttttagg tggagggaga aagtgtatat aggcgtatta 6120
   aagttatagg gatataattg tttgagaaat aattgtatag gtaaaggttt agtataatgg 6180
   tcggaggttt tgataatttt tgtttttttt tagattttat atttgttagg gttttttttt 6240
   attttttgtt gattttaatt tgttaaattt agtttgtaaa ttttttgtta ggaggtagtt 6300
   tagtggttgg gaggcggtgg gtaggggaat tttttttttt ttttgtaata gtaataattc 6360
   gaggtagttt atattttaga aatagtttgt tagaagttgt tagcgttttg tgtagtaagg 6420
   aattgtagat ttttttttta tatttcggtt ttaattttat gattttttcg attttcgtat 6480
   ttatatttgt tttagaagtt cgtcgtttat ttgtttttat agattgtaga aagtagatgg 6540
   ggtggggggt ggtgggaagg gtcggagcgg gtaggtggag tttattggtt ttattttttt 6600
   tttttgtttt aatttttgtt ttggtttagt tagttttcgt tttttttttt tttaaggttg 6660
   atttttaatt gttgaatttt gatttgtttt ggtttttatt ttttttattt atttatgttg 6720
   tattttaatg agagtgtatt tttgattttt aatgattttt gtatttagga tcggagttag 6780
   tattaatttt tttttttcgg cgtatattgt atgttaatta tagttttggg tagtttgttt 6840
   tttatttggg taa 6853
<210> 33
   <211> 18683
   <212> DNA
   <213> Artificial Sequence
<220>
   <223> chemically treated genomic DNA (Homo sapiens)
<400> 33
   tttttaagtg tcggttagtt tgagaaataa agggaaagag tataaaagag agaaatttta     60
   aagttgggtg ttcggggtag atattatatg tcggtaggtt ttatgatgtt ttttgagtta    120
   taaaattagt aagtttttgt tagtgatttt taaaagggga gggagtgtac gaatagggtg    180
   tgggttatag agattacgtg ttttataagg tagtaaaata ttataaggta aatggaggta    240
   gggtgagatt atagaattag ggtgaaatta aaattgttaa tgaagttttg ggtacgtatt    300
   gttattgata atattttatt atgagatagg gtttgagagt agataattag tttgattaaa    360
   atttattagg tgggaatttt tttattttaa taagtttggg agtattatag gagatcgggg    420
   tttattttat tttttatgat aatcgtaaaa gatagatatt tttagagtag ttattttaga    480
   gatttttttt taggaacgta ttttgttttt tgttgagaaa aagaatttag taatattttt    540
   tttatttgtt tttgaaagaa gagaaatacg gttttgtttc gtttagtttt taggtagtta    600
   gatttaatgg ttattttttt tgttttttga acgtcgtttt tattttgttt tttttttaag    660
   gtgtttagat tttatattgt ttaaataatt tgtgtagtta acgtaattat tatagggttt    720
   taaggcgata tatattttta gtttataaag atgatgggat gaagagatta aagtaaagat    780
   aggtatagga aattataaga atattgattg gggaagtgat aagtgtttat gaaattttta    840
   taatttatgt ttagagattg tagtaaagat aggtataaga aattataaaa gtattaattt    900
   ggggaattaa taagtgttta tgaaattttt ataatttatg tttttttgtt atggttttag    960
   tcggtttttt tgttcggggt ttttgatttt ttataatatt agagtttatg tgaaatggaa   1020
   gtaaattatt tttgtgtata tttatattaa gaagaaagag tttaaaggtt gaattataaa   1080
   ttatagagtt tttaggtttt attatttttt ttgtttgttt taaatttatt attatttttt  1140
   ttttgagata aaaattattt tttgtttttg tttttgttag atcgtttttt atttttgtat   1200
   tgagtatttt aagtattgta gaaaagatgt tatttttttt tttaaggttg ttttgtaata   1260
   tatataagga ttggaattgt gtttttaaag aaaagtattt aagtatgata atatattatt   1320
   tgtgttttta ttattaaaag tgttgtttag tagtttaaat ttaaattatt taatgttatt   1380
   taataaagtg attaaaatgt gttttttatt gtatttttat agttttgaaa aaataaataa  1440
   tttaattata tatttttaat attttatagt ttttttaaga atgtataata gttaataaat    1500
   ataaaagaaa tagtagatag agtatgagat gttgaagaaa tattatagtg tattgaatgt   1560
   aaaattatgt atttgtgttg tgtaggggaa atagtttggg gttaaattag ttatttagtg   1620
   atgtgaagat tttgaagaat tggaagaatt ataaatttag ttgtaattat gagttttttt   1680
   taattttaaa tttattaaaa gtgaaggcga atttttgatt ttttgtaatt ttttaatgtt   1740
   aagttttata ttgttatttt attttaatat ttttttaagt tttggaatat atgtgtagga   1800
   tgcgtagttt gttatatagg taaacgtgtg ttatggtggt ttgttgtata gattaattta   1860
   ttatttagtt attaagttaa gtatgtatta tttttttttt ttaatgtttt tttatatttt   1920
   ttattttttt atttggtttt tatgtgtgtt gttttttttt ttgagtttat gtgtttttat   1980
   tgtttagttt ttatttataa gtgagaatat gtagtgtttg gttttttgtt tttgtattag   2040
   tttgttgcgg ataatatttt ttagttttat ttttgttttt gtaaaggata tgattttatt   2100
   tatttttatg gttgtattgt attttatggt gtatatgtgt tatatttttt ttatttagtt   2160
   tattattgat gggtattttg attgatttta tgtttttgtt attgtgaata gtgttgtaat   2220
   gaatatatgt atgtatgtaa tgtattttta taatagaatg atttatattt ttttgggtgt   2280
   atatttagta atggtatttt ttggttaaat ggtatttttg ttttaaatga tttattatat   2340
   atttttttat aattattgat ttaatttata tttttattaa tagtgttttt tgaatatagt   2400
   atattgatga gttttgtttt tttgtttagt ttgttattgt gtgtttttta attggggaat   2460
   ttggtttatt tattttaagg ttaatattgg tatgtgtgaa tttgattttg ttattatgat   2520
   gttgtttggt taattttgta gatttgttaa tgtagttgtt ttatagtgtt atgggtttgt   2580
   gtattttagt gtgtttttat agtggttgtt aatagatttt tttttttatg attattgttt  2640
   tttataggat gttttttaag gtaagtttgg tggtgataaa attttttagt atttgtttgt     2700
   ttgaaaagga ttttattttt tttttattta tgaagtttag tttggttaga tattaaattt  2760
   tgggttggaa attatttttt ttattaatgt tgaatattgg tttttagttt tttttggttt   2820
   gtaggatttt tgttgagaat tttattgtta gtttgatggg tttttttttg taggtgattt   2880
   ggtttttttt tttggttgtt tttaatattt ttttttttta ttttaatttt ggagaatttg    2940
   atgattatgt gttttgggat tgattttttt gtggagtatt ttattgggat tgtttgaatt   3000
   ttttgaattt gaatgttgat ttgttttgtt aggttgggga tgtttttttg gatgatattt   3060
   tgaagtgtgt tttttgattt ggttttattt tttatatttt ttttaggtat tttaattagt   3120
   tataggtttg gtttttttat atagttttat agttttggaa ggtattgttt attgtttttt   3180
   attttttttt gtaatttttt ttatttgttt tattttagta agatagtttt taagttttga   3240
   tatttttttt tttgtttggt tgatttggtt attgatattt gtgtttgaat tatgaaggtt   3300
   ttacgttgtg ttttttagtt ttattatgtt atttgtattt ttttttaaat tggttatttt  3360
   aattaatagt ttttgtaatt ttttattacg gtttttagtt tttttgtttt gggttagaat   3420
   ataatttttt agtttagtaa agtttgttat tatatatttt ttgatattta tttttgttag   3480
   tttatttatt ttagttttag ttttgttttg tgtttttgtt ggagaagtgt tgaaattatt   3540
   tgaggagaaa aggtatttta gtttttggaa tttttagcgg ttttgcgttg gtttttcgtt 3600
   atttttgtgg atttatttat ttttaatttt taaagttgtt gatttttgga tgggattttt 3660
   gtagggtttt ttttgttgat gttgtgttat tgttgtttta tttttttttg tttttttttt 3720
   aatatttagg tttttttttt gtaggtttgt tgtagtgtgt tgaggattta ttttagatat 3780
   tgtttattta tgtattatta atggaggttg tagaatagta aagagtgttg tttttttttt 3840
   ttttcggaag ttttttttta gaggattatt gatttgacgt ttgttggaat ttttttgtat 3900
   gaggtgtttg gtaatttttg ttgaatggtt taatttagtt aggaggttta ggattaggaa 3960
   tttatttaag gaaagagttt ggttattttt tagtagagat ggtgtattgg gttgggggaa 4020
   ttttttttgt ttggatcgtt gatatttttt agagttagta ggtaggaaag attaagttgg 4080
   ttgaatttga gatcgtagtt gttttttttc gtaggtgttt tgtattaggg aaatgagagt 4140
   tttgtttgta aattttaggt ggagttgttg aaatttttgt aagtattttt tgtcgggaga 4200
   ggagggatgg atttcggttt tatttaaaga agtagtttgg ttatgatttg ttatagcggt 4260
   tgtgttgtat tgtgaagatt gttgtttagt ttaatttgtt tagttttttt agtattggta 4320
   gggaaaaatc gttgattaga gttgtagtaa tggtagtcgt tttttttttg gggaattcgg 4380
   ttatttttgg cgtattttag gttgttatgt tggttagtgg ggattttaat ttagcggatt 4440
   taagtttgta ggtttttgtg agaatggatt tgttgtaagg ttattttgtt ttttggtttt 4500
   agtttttttt ttatgggagt ggacgggatt tttgttttat tagagtttcg ggagttgttg 4560
   gattatgtaa aaatttttgt agtttagtgt ttgtttaaaa agttggaaat atgaattatt 4620
   gggttaattt ttattaggaa atgaaaaaga gttgttagat taaatttttt tagttattgt 4680
   cgttttttgt aattagagaa agaaaaattg agagattagg aagagaagag atagatggat 4740
   agaagaagtt tgggtggtta taggaaataa gaaaagaaat aagttgaggt gttaatttat 4800
   aggataatat tttgttattt ttattttttt tttttaagtt agaaattaaa ttttagtaat 4860
   tggatgttaa tttgttgatg ataaggtatt ttttgataat gagtttggaa ttttgagtag 4920
   tagttttata atttgtaatt atacgttttc gttttttgta ataaggaaat atatagttta 4980
   tttttatttt tgattttgag gtttttttta acgttgtaaa aaaggataga ggttgttttt 5040
   tatggtaggt gagttttttt ttgattagga gtttgggagt aggaatttag aaaatatttg 5100
   atttattgtt ttgaaataaa tataagaaag gaagtgagtc gatagtatat atgttgaatt 5160
   ttttttttta ttgattgtgt gtagtttgaa ttatgggatt tggtgttaga gatattgacg 5220
   gaggttatat gttttttaga tgtttttgag ttagaaagaa gttaattttt tttttttagt 5280
   ttttggtaat taaagggagg gtaaagaaag ttagaggaag ttatttgtgt gagataattt 5340
   tagaagtttc gaaatgtatt ttgaaatttt ttttttgatt ttaaattaat tgtttagatt 5400
   ataaattttt ttcgtagaat tagatttgag agtttttgaa ttgttatttt tttcgatatt 5460
   ttttattgtg aattatattt ttggtttgtt taaagtgatt taattttttt ttttttttcg 5520
   ttggttttga tattgattat tttaatataa tattttatgg gttaatatta tagttgtttt 5580
   gttttgtcgt tgttattgtt tttaattggt attttttttt ttaggattta attttttaat 5640
   ttattttgta tataattttt atggttattt gtcgttatat atagaagttg aaaaagttta 5700
   agggatagag ttatttaggt agttagtatt ttagtaggtt ataacgtttt tttggtttta 5760
   ttttttttat aggaaaagga agaaattgga ttagattagt gaattttaaa ttttataata 5820
   tatcgtagta atttgtagtt atataaaaaa agttagatga ttattttttt ttttatcgcg 5880
   aatagtttaa ttttaaatat tttgtaggta tttattattt ttgaagatcg ttatataatt 5940
   tttatgaaat tgatttgata taggtttgtt ttattaggaa agttgattag ttagttaata 6000
   ttttttgttt atttgttagt gatttattaa tgtaattaat tttgtttagg gatatatagt 6060
   ggttttttat tgtttttttt tttttttatt atttgaattt ttagataatt ttttttattt 6120
   tttgaaatat gtgtatttag tttttttatt aatttttgta aagtatgatt ttaattttat 6180
   aaaaaaatat tgtatttatt ttatttatat tattttgttg ttgttttttt atttttttga 6240
   ttttttttat agaataattt ttttcggggt ttatgtttaa ttttataata tttaatttaa 6300
   gttttggaat taagggatta ttagataaag atagggtaaa ttgaaagtta ttagttaaaa 6360
   ataagtcgtg gtttgataat tataaataat taaatgtaga tagttattat tattttaaat 6420
   ttttgaggaa ttgtattttg taatgtgttt aagtattata ttattttgtt tggaattaaa 6480
   attgtggttt tgataagttt ttttttttaa ttgatttttg ttatttttat tattattgta 6540
   gtttttattt agtgattatt ataataataa aaaataattt atagtaatta tttttttaaa 6600
   aaatatttat tatatgatag ttaatttatg tatttttttt gatttaatat ttaatattaa 6660
   ttaatatagt tgtgttatta tttttatatt gtagttgaag gggatattta gagaatgttt 6720
   ttaaaatatg ttttaggtta aaagtttgtt gagaggagtg gttttaggtt ggtatgattt 6780
   ttaaattttg ttattaattt aattttttgt aaatattttt aaatatttgt tttgtttttg 6840
   attattgtta ttttagtttt ggttttatat ttttattttt tttaaagttt agttttttta 6900
   gttttttggt tatacgagat atttttaagt ttttgtgttt ttttttttat tgttttttgt 6960
   taaaagatta gtttttagtt ttttagggga taaattttta ttagtttttt aggttttagt 7020
   gttaagtttt tttttttttg gatagttgta tttgagatat attttgttta tgtttttttt 7080
   gagtttttgt taattattta tatttttata atatttttta ttttatttat ttgtatatgt 7140
   atttttttat ttgattgtaa attttaaagt gtatagatat gttgttaaag tggtattttt 7200
   aataatagta gagtaagttt taagaattta tgtttattaa atatatttga atggataggg 7260
   gaatgaatat gttttttttt tgttgaaata tttaatatgt aattattatt tattaaatga 7320
   aattaaattt atttggtttg ttattaagaa tttgtaatta ttaggtgggt ttattttgtt 7380
   tattgtttag atagagttaa tttattaaga taggggaatt gtaatggcgt aagagtaatt 7440
   tacgtagatt cggttgtttt ggagagggta gtgttattat tatttaaatt agttttttcg 7500
   agtatttaga aataagaacg tttaaagata atttggtagt taggggtttg ggaagtagga 7560
   agtgttgatt atttaggtgg gagatggaat tatagggtgt taaagtgagg tttttttgtt 7620
   gtgttttttt tttgggtgtg atggtagaat tggttgggtt agattattgg tttgggtggt 7680
   gttagttgat ttattgagtg tagcgtttga aaaatatttt aagtgttgat attagatttt 7740
   ataaagtgat attatttttg ggagtataat ggggatattt taattttagg agttagaggt 7800
   tgtatgtttt ttatattgta atttttagtt ttgtagttaa tttgttagtt ttgtaaaggg 7860
   agattggatt ttaggtaaga agagggtttt ttcgagaaaa ggttgttatc ggttttgttt 7920
   tagttaaatt atgaattgaa ttttttttta aagttagttc ggtttatgtt taggaatgaa 7980
   taagaatagt ttaagggata gaagtaagat agagttggtt aggttttatt ttttttattg 8040
   tcgtaattat tttagttata atattgtaaa ggtagtttta atttttttgt ttgggttttt 8100
   taatagttta attttaaggt gtaggttatg aagatgggaa aaggttgtcg attattttgg 8160
   tttttttttg ttgataggag atatagtgga aattagagtg aattttaagg tgagaagaat 8220
   gtaattattt tgtaattgtt tgagtatatt tatgtaggat tggttgggtt tttaatgttt 8280
   gtaatgtaaa aatattagta tttttttttg tagttttgtt ataatattta agttaatagt 8340
   tttttgtaag gtaaataata aattttagga tgaggagtat aatttttaat tttaaaagta 8400
   aaaattggaa agtattaatt tggggatttt taatttataa agaatttagg atttagttta 8460
   aattgtagga aaaaatttaa ataaaattaa taatgggtat attatatgtt tttttttgaa 8520
   gtataatttt tttttttaga ttttattttt attaaaagta aattataata gaattaattt 8580
   gtttattaaa ataagtttta gttttattgt atttggtttg attatttgta tgaagtgtaa 8640
   taagaataat tattttttat ttaggttgtt ttttgtttaa attggttttg atggaatttt 8700
   gttttatgag gaattttaga taagattttt taagagttaa ttttagttat gtatttgtat 8760
   ttttaaatat ttatgagttg ggtaaatttt ttttttttta aggttttaag ataatttgga 8820
   gtttatgaat ttgttagaaa gtgatatttt ttatttatta tagattaaga attttgtata 8880
   aggattttgt gtagataagg tatgaagtta gattttttaa ggggttttta ttggttttgt 8940
   aagttaattt cgatttttta agggaagagt gttattttag ttaaagtttt ggtaaaataa 9000
   ttaatttttt taattgtgtt atgttataaa agaaaataga tttttattgt gtttatggaa 9060
   ttaattatgt tgttataaat tgagaatatt tataaagagt ttttaaattt tggagaaatt 9120
   aggtagagag aaataaatat gttttaaaat ttttttatag gagtgtattt tatttatttg 9180
   ttaaaagttg taaatatttt taaagaaata agttattttg attgtgaaaa ttgaaggatt 9240
   ggtgatattt aagatattag ttttttatga gagttttgga agttttgttt ttttttttta 9300
   ttttaatagt ataattttta aagttatttg agatttgtat ttagagtttt atatttgatt 9360
   ataaattgtt ttttgaaaac gattaaagta agataaaatg tttgtggatg ataaaagttt 9420
   atagatatta ttaaagttat aattgattag gaattttgtt tatttttgtg gtatttaata 9480
   attttgtata ataattataa ttattaataa tgtatagtaa attttattag aattatagaa 9540
   gtttatataa atatatagat agatagaaaa tttagtattt gtaagatttt ttatttgtta 9600
   gttgttaatt ggattattgg ttttagagtg gagtttttgg aggaataggg ttaggaaagt 9660
   atgtattttt tttttaagtt aaataagtag gtatagttga aggtaaagat agatttttaa 9720
   aatgaaggat gttattttat attggatttt agatttttaa aaggaaggaa atattattgg 9780
   agaagagagt gtagtgtttt taatttgtat tttattgtaa ggtaatttaa agttaattaa 9840
   tttattttga aattagttta ttttttatgg aagttttatt ttttagttag gtatggggat 9900
   gttttcgtat ttttttggtg gtttagagta tgtttttttg atttaagtgt gcgaagagtt 9960
   aagttttttt ttgaaaatat tattagttat tttttaaagt atatttttta tttagttatt 10020
   atatattaaa gttattttat aatgtaaagt aatttttgat atttttaaaa tttaaattgt 10080
   tagataatat aatgtaaaat agaatagagt ttttgatttt gagagtgatt tatttatttt 10140
   taattttttg gttgttatga agaaaataga gggttttttg tttgtttttt gttgtttgtt 10200
   ttttttaaaa tggggtttgt ggtgtttttt ttgttttttt taaggagttt taggttatta 10260
   aaagttattt tagtgttttt tatgtgtgta ttaagagtgg taagataaaa aatggagaaa 10320
   aataatttag tttattaaga agaaaaaatt tttttttata gaaaaacgag atttaagaag 10380
   agaaaaatat aaaggttttt taaatgtatt tataatttgt ttatttattt ttaattaagt 10440
   tgatttttaa ttttagtgtt tttaataaaa tttttttaaa tttcgtatta tttaatttta 10500
   gttatgcggt taatattttt ggtttttgaa ttttattaaa agtaattttt taggtgtttt 10560
   aaaggtgtgg taagtagttt ttttttataa gatttagaat ttttttaagg tagtttagag 10620
   aaaggaaaat ttaagagagg aaattagaag ttatttatgg ggttgtgggg gaattttatt 10680
   aaatggtaaa gttatgtaaa tagtaaatta gaaggaaatc gttttagaag ttaagaatag 10740
   agtttgagtt tttatgttaa aaggtaaagt tttagttatt gaattatagt attgagtagt 10800
   ttttattgtt attttaagaa ggagattaga gaagttattt ttaattttgt taaggttttt 10860
   aattgtttaa gaaaaatttt tagggttgga tggtataaat taaaaggaag tatttatata 10920
   tggttataag attaagtttt taaggaggtt aaataagata gagaaatttt atatagtatt 10980
   ggttttaggg atttattata attggttagt ttgttgagtt ggtttgaaaa gtaggtttat 11040
   aggggtttta aatttatgtt ttattttgtg atattttttt ttttattata gaatattgaa 11100
   agataaattt ttagtaaaaa tatattagat ttgttatagg ttaagattag ttttgtaaat 11160
   tttttttttt attaattaaa attttataga gaaatatata gtgatgttta ttgtttatat 11220
   agatggagaa agagagagag attagaaatt tagttggtaa gaatttttta tattttttgt 11280
   tggtatatta gatttttggg tgtttttttt ttgtaatttt tagaagaacg gaacggtttt 11340
   tgattagttt gtttatttta ttatagttgt ggggtttaag ttattttata agaaaaaagt 11400
   tattattttt tgttttatgg aaatataggt aagattttta aaatgtaaga tgttgtttaa 11460
   ttagttgtat ggggaattat ttaatatttt ttattttagt agaatttata taataaaata 11520
   gatattagtt atttcgttta gtatttaata ttaatttggt aaagtttaga tttttttttg 11580
   ttcgtttttg ttgtttttga tttattttag gtggggaggg acgattttag aatagtaatt 11640
   tataatgggg tttttgggta aggtaaagag tagatagtta ttatgagata ggtttattga 11700
   atttttttta gggtttatcg aatgtaatta aataagaaga gttttttgag ttaggtttgt 11760
   tggatttttt ttagtaattt tttttgagat tttttttata tatataaata tatacgaaga 11820
   tgagatagat agaaggtttt ttaaattaga tgatttttaa ataggaattt taagagtatt   11880
   ttttttaaat tattttttta ttttttgttt gagaaatttt aaaattgttt tgattgagga   11940
   taagtttttt gaattaagat tttttttatt agttagagtt aattaagatt ttagtggagt   12000
   cgaatagata tttcgtggtg gagttataaa tagatatttt gtaatggggt tatagatatt   12060
   ttgtaatggg gttatagata ttttattata gagttataga taagtcgaga gaaggaagga   12120
   ggtattagta gtgtttagta gatttattaa ttttaatatt ttgtaatggg gttatagata   12180
   gatattttat tatggggtta tagaattagt taggagaagg aaggaggtat tggtagtatt   12240
   taggatattt attaatttag atatttttta atgtagttat agacggaaat tttgtgatag   12300
   ggttatagtt agaggatatt tttttaggat tattttttcg ttgtaattaa atgtatgtat   12360
   attgggttgg tagtgtttgt tagtggagag agtattagag ttagttttta gtttaagaga   12420
   attaggcggt tatttgggtt agtttttgga tttatcgttg gagcgggggt attaaattat   12480
   aagtaggtag ttataaaggt ttttttagtt tgatttcgtt tattgttgta atttttttag   12540
   ttataatttt gtaaaggtgg ttttatattt tttaagagtt gagttatata taagttttat   12600
   ttatttgttt ttatatggat ttgattattg atttagtaaa taattagtaa atatttagta   12660
   aataattagt aaaaagaatg ttaaaaatag tgtagatgta gtttatggtt tttgttttta   12720
   tagagtataa attttaatag tggatagaga taagtaggtt gggaattata attaaatgga   12780
   aaaataaaaa aaaaggttgg atgtggtggt ttaggtttgt aatttaatag tagtaatttg   12840
   ggagattaag atgggtggat tatttgaggt taggagttcg agattagttt ggttaatatg   12900
   gtgaattttt atttttatta aaaatataaa aattagttag gcgtggttgt gtgtatttgt   12960
   agttttagtt atttagaagg ttgaggtagg agaatcgttt gaattcggga ggtagagatt   13020
   gtagtgagtt aagatagtat tattgtattt tagtttgggt gatagagtga gattttgttt   13080
   tataaaaata tataaatata taaataaata aatggaaaaa aaatgttatt ttaaggagat   13140
   ataaggtgtt ttaggagtat atatttttga tgttgttttt tttggaagaa ttagagagga   13200
   tttttttaag aaaatattat ttaatttgat atttgaagga tagatagata gatagataga   13260
   tagagaatag tttaaaaatt agaagaaatt agaagcgggg gtgttgaaat tatatagatt   13320
   tttaagtaga gatttgattt tttgagaaag gtgttattta atttgagatt tgaaggttag   13380
   atagatagta gtaggaaaat aagaatgaat gagaaggtag gggttggaaa tatagagatt   13440
   tttaaataga atttttttag aaattaaatg aaggtagaac gttttgaatg taatgagtaa   13500
   ggtgggaata gtattagaaa atgagtgaat gagaaggttt agattagaat aggttgaaag   13560
   tttttatggt taaatttaga tattggaata tttttcgttg taaagagtag aatagaggga   13620
   cggagagtag attgataaag tttttatgaa tttatttttt aagaaagaaa ggatgttggt   13680
   tcgaatagaa gtaatgaata attagaattg agagaagtgg ttagattttg aaaatgttta   13740
   gtaaataaaa tcgatgtaat gtgaattaag aagggaaata agatatagag aaggttaaag   13800
   aggttgaata ttattaggta ttgttgtttt tttgtatttg gagtataggt attatttatt   13860
   agtatatgga aaagagaatg aaggatggaa atgaaaaaat gaatagttta tttttgtaag   13920
   ttgagttaaa tgaggttgtg agatgtagtt gagatatttt attgtttatt gaatgtatag   13980
   gttttaatat cgatatttat atttgggttg aatttataaa tttgagaatg atttatttat   14040
   ttttttaagt atataatgga tgttattaga atgtgtagat tatattttag gttatatttt   14100
   taatttattt atttatttat ttgataaata aatattgtat atatttttta tgtataattt   14160
   tttgttttta tgagtttaat ttgtttagag tttatgtata agtgagataa tgtgatattt   14220
   tgtgttttgg tgtttggttt ggtttatttt atttaatata atgtttttta tgtttattaa   14280
   tattgttaga aacgatagga tttattttta tttaaaagtt gaatagtgtt gtattttgta   14340
   tataatttat atttttttta tttattaatt tgtttataga tatttaggtt gattttatat   14400
   tttggttatt gtgaataatg ttgtaatgga aatagaagtt tagatatttt ttcgagatat   14460
   tgatttttta atttttgata tatatttaat agtgggattg tttgattata tttttagttt   14520
   tatttttaat ttttagaggt gtttttatat aggtttttat aaagattatg ttaatttatt   14580
   tttttttttt tttttttttt tttgggaaaa aaattttttt tttgggggtt cggggggggg   14640
   ggtatttttt tttaatttta aaggggtttt taaaaaaggt ttttaaaaaa atttttttaa   14700
   tttttttttt tttttttttt tttttttttt gagatagagt tttattttgt tgtttaggtt   14760
   ggagggtagt tatggaattt aggtttattg taattttcgt ttttagggtt ttagtaattt   14820
   tattgttttt gttttatgag tagttaagat tataggcgtt tattattacg ttcggttaat   14880
   ttttgtattt ttattagaag tagggtttta ttatgttgtt tagtttggtt tagaattatt   14940
   gattttaggt gatttgttcg ttttaatttt ataaagtgtt gggattatag tcgtgagtta   15000
   ttgcgtaggg tttaatttac gttttattaa tagtgtataa gagttttttt ttttttatat   15060
   tttttttatt atttgttatt ttttgttttt ttggtaatag ttattttaat aggtgtaaag   15120
   tgatatttta ttgtgatttt aatttttttg gtggttagag aggttgatta ttttttatat   15180
   atatattttt tgtatgtttt tttttgaaaa gtgtttattt tggttttttg tttattttta   15240
   aattagttta tttttttttt attgaattgt ttgtgttttt tgtattttgt atattaattt   15300
   tttattagat gtatggtttg taaatttttt ttattatatt gttgattatt tttttaattt   15360
   tttgattgtt tttttgattg tgtaaaagtt ttttagtttg atgtaatttt atttgtatat   15420
   tttagttttt gttttttgtt tttttttgtt atatttaaaa attattgttt agattaatgt   15480
   tatgaagttg tttttttttg ttttttttta gtagttttat agttttggtt tttatattta   15540
   tatatttaat ttattttgag tttatttttg tatatgttta gagattttta tttttttgta   15600
   tgtggtttta taattttttt aatattattt attgaagaat ttgttttttt ttagttgtgt   15660
   gtttatgaaa tttttgttga aaagttaaat taatgtaaat aattgggttt atttttgggt   15720
   tttttgtttt gtttatttgt tttttgtttt tatttttatg taattattgt gttattttga   15780
   tgattgtagt attttgtagt ttattttgaa tttagatagt gcgatgtttg tagttttgtt   15840
   ttttttattt aaaattgttt tgtttattta gggtttgttt ttttttgtgt gattttatat   15900
   aaattttagg atttgttttt atttttgtaa aagttgttat tagaattttg ataggaattg   15960
   tattgtattt ttagattttt ttgggtatta gggatatttt aataatagta attttttagt    16020
   ttattaattt aaaatatatt tttatttatt tttgtttttt ttagtttttt ttattaatat   16080
   tttatagttt tgagatttgt tattattgtt gatatttttg agataggatt ttattttgta   16140
   gttaagattg gagtatagtg ttataattat agtttattat aattttaaat ttttgggttt   16200
   aagtaatttt tttattttag atttttaaga agtaaggatt atagatatat gttattatgg   16260
   ttagttaatt ttttaaattt tttgtagaga tatagtttta ttatgttgat tgggttgaag   16320
   ttttatattt ttacgtgtat ttgtttttta tttattcggt taaattgatt tttaagtatt   16380
   tttgtagtaa ttttaaatgg aaatttattt atttttaaaa gatggtttat tgttatatag    16440
   aaatattata gatttttgta tgttgatttt ttttattttg taattttttt gaatttgttt   16500
   atttgtttta aaagattttt ggtggatttt ttagggtttt tcgtatataa gattatttta   16560
   tttttaaata ggtataatta aatttttttt tttttaattt tgatgttttt tatttttttt   16620
   tgttggcgaa ttgttttggt tagaattacg ttgaatggaa gtggtaaatg tgaatatttt   16680
   tgtttttttt ttgattttag agaaagggtt tttaattttt tattattgag tatgatgtta   16740
   gttgtgtgtt tgttatatgt gatttttttt gttaaaaatt ttttaatttt attgtttatt   16800
   atattttttt atatattatt tatttagtta atattatatt agtttattga gtatataaat   16860
   aaataaaata gaagtttaga atttaggtag tttggatatt ttatatatta atttgtgtat   16920
   tgtttgtaaa ttatattgtg ttgtaatatt tgtatttgat gttgtaagat taatattttt   16980
   gttatttttt tttagaagtt tttgtgattt tatttgttgg aatgtattaa tgttttttga    17040
   gttttttaaa tattttgttt atattttttt aagaagtttt ttattaattg ttaggtaatg    17100
   tagtttgatg aattaagaat gggggttttg gattttgaat ttttagattt taatttttgt   17160
   ttttagttat tatatattcg gtgttttttt atattatttg agttaattat ttattatttt   17220
   ttatttttta tataagagag gttaatatat gttttatatg gaatttttgt gaaattttag    17280
   taatgtattt ggtatgatga gggtttaatg aatattaatt attttaattg aattatatta   17340
   tgattaatta tatagattat ggtgaaaaaa ataagatttg aattttaatt tatgtgatgt   17400
   gatataagtt attatttaaa tggtgaatat aaaataggga atataataat tattttattg   17460
   gtttattgtg agggtttagt atattaatat ttgtaaaatt tttggtatag tgatatgtaa   17520
   atgataatga tgattataat aatgatttaa attgttttat atgtatttat tttttattta   17580
   taataagtat atgggagata ttatttttta ttaagttaat ttatattatg taattttttt   17640
   tttggtttgt aatgtttaat gtttttttta taatagttgt ttaataagta cgtatgaaaa   17700
   tattaattta agataattta gataatgagg ttggtgtttt tgtgttaaag tttttgtgta   17760
   atgttttgat atgatttttt tagatgttat tttgtgttag tattattttt atatttatat   17820
   ggattgtata attatttttg tagtattgat tgagaattga taagatattt agtatttttg     17880
   tgtataattt gtcgaaatta tatttgttgt attgaaataa aggatgtatt attttggtat   17940
   ttatattttt ttataaatat ttaaattatt ttaaggagta atatattaat atggtgttaa   18000
   attaaattaa tttattatat tggaagatta ggaattaggt tatttttaat tttaaatttt   18060
   tattgagaat aagatttgaa agaattataa atatggatgt atttatttat ttatttttta    18120
   tgtatttagt aagtatatta agtattttta aatattttga atataaagat gtgggtatag   18180
   gatgtaagtt aaaaaaatgt aaatttaaag atatagtttt tttattaaga aaattaaata   18240
   tagaaaagag taaaaattat ataggaagta aaaatttagt aggaagaatt tatattttag   18300
   ataagagtat tgattttttc gggaatatat attttaggta gattattatt attatgggtc    18360
   gtgaattagt aaaagagaaa aatgaatata gtggatgatt aggaagtttg tattaggttt   18420
   aatgttatag aaggaagttg agaagttaaa agaaaagaaa agaaaaaaga ataaaaaaga   18480
   aaataaaaag tatttagtgg taataaattg ttaaataatt tggtaggaat atgatgaatg   18540
   atagaatttt ataggtataa gaagatagat ggtgaagtag gatatatttt tagaagtatt   18600
   ttatattata gaattatatt taaagtgagg gtgaaatttt ttaagttgtg tttatagtta   18660
   aaggaatttt tttattttta ata                                           18683
<210> 34
   <211> 18683
   <212> DNA
   <213> Artificial Sequence
<220>
   <223> chemically treated genomic DNA (Homo sapiens)
<400> 34
   tgttagaaat agaaagattt ttttaattat gggtatagtt taaagagttt tatttttatt     60
   ttagatatag ttttataatg tgaaatattt ttgaaagtat gttttatttt attatttgtt    120
   tttttgtatt tatagagttt tgttatttat tatattttta ttaggttgtt taataattta    180
   ttgttattaa atatttttta tttttttttt tgtttttttt tttttttttt ttttttagtt     240
   ttttaatttt tttttgtggt attggattta atgtaaattt tttggttatt tattgtattt    300
   attttttttt tttattggtt tacggtttat agtgataatg atttgtttaa aatatatatt    360
   ttcgggaaag ttaatgtttt tgtttagaat ataaattttt tttattagat ttttattttt    420
   tatatgattt ttattttttt ttgtatttag tttttttggt gaaagaatta tatttttgaa  480
   tttgtatttt tttaatttat attttatatt tatatttttg tatttaaaat gtttaaaagt 540
   atttaatatg tttgttaaat atataaagaa taaatggatg agtgtattta tatttataat    600
   ttttttaaat tttgttttta atgaaaattt aaaattgaaa gtaatttgat ttttaatttt   660
   ttagtgtggt ggattagttt aatttgatat tatgttaatg tattattttt tagagtaatt    720
   tgaatattta taaggaaata tagatattaa agtaatatat tttttgtttt aatataatag 780
   atatgatttc ggtagattgt atatagaaat gttggatatt ttattaattt ttaattaatg 840
   ttataaagat aattatataa tttatgtagg tgtaaggata gtgttgatat aaaatgatat 900
   ttaaagaaat tatgttagga tattgtataa aagttttggt atagaagtat tagttttatt 960
   gtttgggtta ttttgaatta atatttttat acgtgtttat tgggtagtta ttatgagaaa 1020
   aatattagat attataaatt aggaaaaaag ttatataata taaattaatt tagtaaaggg 1080
   taatattttt tatgtattta ttataaataa aaaatgaatg tatataaaat aatttaaatt 1140
   attattataa ttattattat tatttatata ttattgtatt aaaggtttta taaatgttaa 1200
   tatattggat ttttataata agttagtggg gtagttatta tattttttat tttatattta 1260
   ttatttaaat aataatttat gttatattat atgaattagg atttaaattt tgtttttttt 1320
   attatagttt atatagttaa ttatgatatg gtttagttaa aatagttaat atttattgag 1380
   tttttattat gttaagtgta ttattgaaat tttataaaaa ttttatataa aatatatatt 1440
   aatttttttt atatggaaaa tggaagataa taagtaattg atttaaataa tgtaaaagaa 1500
   tatcgagtat atagtaattg ggaataagaa ttgaaattta ggggtttaga atttagagtt 1560
   tttattttta atttattaaa ttatattatt tgatagttgg tgaagagttt tttaaaagaa 1620
   tgtagataag atatttaagg ggtttagaaa atattagtgt attttaatag gtgagattat 1680
   aaaggttttt gagaagaggt agtaaaaatg ttagttttgt aatattaggt atagatgttg 1740
   taatatagtg tgatttgtaa atagtatata gattagtgtg tgggatattt aaattatttg 1800
   agttttgggt ttttgtttta tttatttgtg tgtttagtaa attaatatag tgttgattga 1860
   ataaatgata tgtgaagaaa tataataagt aataaaattg aagagttttt aataaaaaaa 1920
   gttatatatg ataaatatat agttaatatt atatttaatg gtgaaaagtt gaaaattttt 1980
   tttttaagat taggaagaag ataaagatat ttatatttat tatttttatt taacgtagtt 2040
   ttgattagag taattcgtta ataaaaagaa ataaaaggta ttaaaattgg aaaggaagaa 2100
   gtttaattgt gtttgtttaa agatgaaatg gttttatata cggaaaattt taaaggattt 2160
   attaaaaatt ttttagaata aataaataga tttaagaaaa ttgtaggata aaaaaaatta 2220
   atatataaaa atttgtagtg tttttgtata ataataaatt atttttaaaa aataaataaa 2280
   tttttattta aaattgttgt aaaaatattt agaaattaat ttaatcgagt aggtaaaaaa 2340
   taagtatacg taaaagtata aaattttaat ttaattaata tggtgagatt atgtttttat 2400
   aagaaattta aaaagttggt tggttatagt agtatgtgtt tatagttttt gttttttgga 2460
   agtttgaggt aggaggattg tttaagttta ggaatttgag attatagtga gttataattg 2520
   tgatattata ttttagtttt ggttatagaa tgagattttg ttttaaaaat attagtaata 2580
   ataataaatt ttaaaattat aaaatattga tagggaaaat tgaagaagat aaaaataagt 2640
   agaaatgtat tttgagttga tggattggaa aattattatt attaaaatgt ttttaatatt 2700
   taaagaaatt taaagatata atgtaatttt tattaaaatt ttaatggtag tttttataaa 2760
   aatagaaata aattttaaaa tttatatgga attatataaa aaaaaataaa ttttgaatag 2820
   gtaaagtaat tttgagtaaa gggaataaag ttgtaggtat cgtattattt gagtttaaaa 2880
   tagattataa agtattatag ttattaaaat agtataatag ttgtataaaa atagaaatag 2940
   agagtaaatg aataggatag aaaatttaga aataaattta attatttata ttaatttgat 3000
   tttttaataa aggttttatg aatatataat tgggaaagaa taaatttttt aataaatgat 3060
   attgggaaaa ttgtaaaatt atatgtagaa gaataaaaat ttttaaatat atataaaaat 3120
   aaatttaaaa tgagttaaat atgtaaatgt aagaattaaa attgtgaaat tattagaaga 3180
   aaatagaggg gaatagtttt ataatattgg tttagataat gatttttgga tatgatagaa 3240
   aaggatagga aataaaagtt aaaatatgta aatgaaatta tattaaatta aaaagttttt 3300
   atatagttaa agaaataatt aaaagattaa agagataatt aatagtatgg tagagaagat 3360
   ttgtaaatta tatatttgat aaagaattaa tatgtaaaat ataaagaata taaataattt 3420
   aatagaagga aaataaattg atttagaaat aagtaaagga ttagaataaa tattttttaa 3480
   aagaagatat atagagaata tgtatatgaa aaatgattaa tttttttagt tattagggaa 3540
   attgaagtta taatgaaata ttattttgta tttgttggaa tggttattat taaaaagata 3600
   aaagataata aatggtggag aggatgtaga aaaaaagaaa tttttgtata ttgttggtaa 3660
   aacgtaaatt aggttttgcg tagtggttta cgattgtaat tttagtattt tgtgaggtta 3720
   aggcgggtag attatttgag gttagtagtt ttagattagg ttggataata tggtaaaatt 3780
   ttgtttttag taaaaatata aaaattagtc gagcgtggtg gtgggcgttt gtaattttag 3840
   ttatttatga ggtagaggta gtagaattgt tggaatttta gaggcggagg ttgtagtgag 3900
   tttagatttt atgattgttt tttagtttag gtaatagagt gagattttgt tttaaaaaaa 3960
   aaaaaaaaaa aaaaaaaaaa aaattaaggg ggtttttttg gaaatttttt ttggaaattt 4020
   ttttaaaatt aaaaaaaaat gttttttttt tcgggttttt aaggggggaa tttttttttt 4080
   aaaaaaaaaa aaaaaaaaaa aagagtaaat tagtatggtt tttatggaaa tttatatgga 4140
   gatatttttg aaagttaaaa atagagttaa agatatgatt aagtaatttt attattaagt 4200
   atatattaaa aattggaaaa ttagtgtttc gaggagatat ttgaattttt atttttattg 4260
   tagtattatt tataatagtt aagatataaa attaatttaa atgtttgtgg atagattaat 4320
   agatgaagaa aatgtggatt atatataaaa tgtaatatta tttagttttt aaatggaagt 4380
   aaattttatc gtttttgata atattgatga atatgaagaa tattatatta agtaaaataa 4440
   gttaggttag gtattaaaat ataaaatatt atattatttt atttatatat agattttaaa 4500
   taggttaaat ttataaaagt agaaggttgt atatagaaaa tatgtataat gtttatttgt 4560
   taaataaata aataagtaaa ttaaaagtat ggtttgaggt ataatttata tattttgatg 4620
   gtatttatta tatgtttaaa agaataaata aattattttt aaatttatgg atttagttta 4680
   gatgtggatg tcggtattga aatttatata tttaatagat aatgagatat tttaattata 4740
   ttttataatt ttatttaatt taatttataa agatgaattg tttatttttt tatttttatt 4800
   ttttattttt ttttttatat attagtaaat aatatttata ttttaagtat aaaagagtag 4860
   taatatttgg tgatatttaa tttttttggt tttttttatg ttttattttt ttttttaatt 4920
   tatattgtat cgattttatt tgttaaatat ttttagaatt tgattatttt ttttaatttt 4980
   aattgtttat tatttttatt cgggttaata tttttttttt tttaagaaat aaatttataa 5040
   gaattttatt aatttatttt tcgttttttt attttatttt ttgtaacgaa gagtgtttta 5100
   atatttagat ttgattatga aaatttttag tttattttaa tttaggtttt tttatttatt 5160
   tattttttga tattgttttt attttgttta ttatatttaa aacgttttgt ttttatttaa 5220
   tttttagaaa gattttattt aggggttttt gtatttttag tttttatttt tttatttatt 5280
   tttgtttttt tattgttgtt tgtttaattt ttaagtttta gattagatgg tatttttttt 5340
   agggaattaa gtttttattt agggatttgt atagttttag tattttcgtt tttaattttt 5400
   tttggttttt gaattgtttt ttgtttattt gtttatttgt ttatttttta agtattagat 5460
   tagatggtat ttttttaagg gaattttttt tgattttttt agaggagata gtattagaga 5520
   tatatgtttt tagggtattt tgtatttttt tagagtagta tttttttttt atttatttat 5580
   ttatgtattt atatattttt gtgagataga gttttatttt gttatttagg ttgaagtgta 5640
   gtggtgttat tttggtttat tgtaattttt gtttttcgga tttaagcgat ttttttgttt 5700
   tagttttttg agtagttagg attataggtg tatataatta cgtttggtta atttttgtat 5760
   ttttagtaga gatggggatt tattatgttg gttaggttga tttcgaattt ttgattttag 5820
   gtgatttatt tattttggtt ttttaaattg ttgttattgg attataggtt tgagttatta 5880
   tatttagttt ttttttttat ttttttattt aattgtgatt tttagtttat ttatttttat 5940
   ttattgttag aatttatgtt ttgtgaggat aggggttatg aattatattt gtattatttt 6000
   tggtattttt tttattagtt atttattgaa tatttattaa ttatttattg aattagtagt 6060
   taagtttatg taggaataaa tgaataaaat ttatgtgtgg tttaattttt aaggaatatg 6120
   aaattatttt tgtaaaatta taattgagga aattataata atgaacgaaa ttagattggg 6180
   aaagtttttg tggttatttg tttgtggttt ggtgttttcg ttttagcgat ggatttagaa 6240
   gttagtttaa gtagtcgttt agtttttttg gattgggggt tggttttggt atttttttta 6300
   ttggtaggta ttgttaattt aatgtgtatg tatttaattg taacggagaa ataattttgg 6360
   agagatgttt tttaattgtg gttttattat agggttttcg tttgtagttg tattgaaggg 6420
   tgtttggatt ggtgagtatt ttaggtgttg ttaatgtttt tttttttttt ttgattggtt 6480
   ttgtggtttt atagtggggt gtttgtttgt agttttattg tagggtgtta ggattggtga 6540
   atttattagg tattgttaat gttttttttt ttttttcgat ttgtttgtag ttttatgatg 6600
   gggtgtttgt agttttattg tagggtgttt gtagttttat tgtagggtgt ttgtttgtag 6660
   ttttattacg gggtatttgt tcggttttat tggggttttg gttggtttta attagtagga 6720
   agagttttgg tttaggagat ttgtttttaa ttaggataat tttgagattt tttagataga 6780
   gaataggagg atagtttgga agggatattt ttggagtttt tgtttaggga ttatttgatt 6840
   tggaaggttt tttgtttgtt ttattttcgt gtgtgtttgt atatgtggag ggaattttag 6900
   aaggaattgt tgaaggaagt ttagtaggtt taatttagag gatttttttt atttggttat 6960
   attcgatgag ttttggagaa agtttaatag gtttgtttta tggtgattat ttgttttttg 7020
   ttttatttag agattttatt gtgaattatt gttttgaggt cgtttttttt tatttggagt 7080
   ggattaaaga taatagggac gaatagaaaa aagtttgagt tttgttaggt tgatattggg 7140
   tgttgaacga ggtgattagt gtttgttttg ttatgtgaat tttgttggga tagaaaatgt 7200
   taaatggttt tttatgtagt tggttgggta gtattttgta ttttaagaat tttgtttatg 7260
   tttttataaa atagaaaatg gtgatttttt ttttgtaaag tggtttgaat tttataatta 7320
   tggtgaggtg agtaggttga ttagaagtcg tttcgttttt ttggaagtta tagagaaaag 7380
   gtatttagaa atttggtatg ttagtaaaaa gtgtaagaaa tttttattag ttaggttttt 7440
   ggtttttttt ttttttttta tttgtgtaaa tagtaaatat tattatatgt ttttttgtaa 7500
   agttttgatt aatagaaaaa agaatttata agattagttt tagtttatag taaatttggt 7560
   gtatttttgt taagaatttg ttttttagtg ttttgtaatg gaaagagggg tattataaga 7620
   tagaatatgg gtttaggatt tttataagtt tgttttttaa gttagtttag taggttggtt 7680
   aattataatg ggtttttgaa attaatattg tatgaaattt ttttgttttg tttggttttt 7740
   ttaagagttt aattttgtga ttatatgtgg atattttttt ttggtttata ttatttagtt 7800
   ttaaaaattt tttttgagta gttaaaagtt ttggtaaggt taaaagtggt ttttttagtt 7860
   ttttttttgg aatagtaata gaaattgttt aatgttgtaa tttagtagtt aagattttgt 7920
   tttttgatat agaggtttag gttttatttt tggtttttgg aacgattttt ttttggtttg 7980
   ttatttgtat aattttgtta tttaatgagg tttttttata attttatgga tagtttttga 8040
   tttttttttt tgaatttttt tttttttgga ttattttggg gaaattttaa attttgtaaa 8100
   aagaaattgt ttattatatt tttgaaatat ttgggaagtt atttttgata aagtttaaaa 8160
   gttagaaatg ttggtcgtat ggttaaagtt gggtaatacg aaatttaaaa ggattttgtt 8220
   aagagtatta aggttaaaag ttagtttaat taaaagtgaa taaataagtt atggatatat 8280
   ttaaaagatt tttatgtttt tttttttttg gatttcgttt ttttgtaaaa gagggttttt 8340
   tttttttagt aaattgaatt attttttttt atttttgttt ttgttatttt taatgtatat 8400
   ataagaggta ttaagataat ttttggtagt ttgagatttt ttgagaaaaa tagaggaagt 8460
   attatagatt ttattttggg aaaaataaat aataagaaat aaataaaaaa ttttttgttt 8520
   tttttatgat aattaaggaa ttaaaagtag atggattatt tttaaaatta aaggttttgt 8580
   tttgttttgt attgtgttat ttgatagttt gaattttggg gatattagaa attattttgt 8640
   attatgagat agttttggta tgtaataatt aggtaggaaa tatattttaa gggatggtta 8700
   atagtatttt tagagggaaa tttggttttt cgtatatttg ggttagagaa gtatgttttg 8760
   ggttattagg aagatacgga aatattttta tgtttgattg agagatgaga tttttatggg 8820
   ggatgggttg attttaaaat gggttgattg gttttaggtt gttttgtaat aaaatgtagg 8880
   ttagaagtat tgtatttttt tttttagtag tatttttttt tttttgggga tttgggattt 8940
   agtataaaat ggtatttttt attttggaga tttgtttttg tttttagttg tgtttgttta 9000
   tttagtttgg aaaaaaaatg tatgtttttt tggttttgtt tttttaaggg ttttattttg 9060
   aagttagtag tttagttaat aattggtaaa taaaaaattt tataagtgtt gaattttttg 9120
   tttgtttgtg tatttatata aatttttata attttgataa aatttattgt atattattag 9180
   taattataat tgttatataa aattgttgga tgttatagaa gtaagtaaaa tttttagtta 9240
   attatagttt taatagtgtt tatagatttt tgttatttat agatattttg ttttgttttg 9300
   gtcgttttta aaaggtagtt tataattagg tataggattt tgagtgtagg ttttagataa 9360
   ttttaaaaat tgtgttattg gaatagagga aaaaaataaa atttttagga tttttatgaa 9420
   gagttgatgt tttaaatgtt attaattttt tagtttttat agttaagata atttattttt 9480
   ttagagatat ttgtaatttt taataagtag gtaaaatata tttttgtgaa aaaattttga 9540
   agtatatttg ttttttttta tttgattttt ttagaatttg gaaatttttt gtaagtattt 9600
   ttaatttatg gtagtatggt taattttata agtataataa aaatttgttt ttttttgtaa 9660
   tatgatataa ttggagaaat tggttatttt attaaggttt tgattggaat ggtatttttt 9720
   ttttaaagaa tcgaagttga tttatagagt taataaaagt tttttgggga atttggtttt 9780
   atattttgtt tatatagagt ttttgtataa ggtttttgat ttgtagtaag taaagaatat 9840
   tattttttaa taggtttatg agttttaagt tattttggga ttttgagagg agaggaattt 9900
   atttaattta taggtattta agggtataaa tgtatggttg ggattggttt ttaaaaagtt 9960
   ttatttgaga ttttttatgg aatagagttt tattaaagtt aatttaaata aaaaatagtt 10020
   taaataaaaa ataattattt ttgttgtatt ttatgtaaat aattaggtta agtatagtaa 10080
   gattaaagtt tattttggta aatagattag ttttattata atttattttt aataaaaata 10140
   gggtttggag agaaaaatta tgttttaaaa gaaaatatat aatatattta ttgttagttt 10200
   tgtttgagtt tttttttata gtttggatta aattttaaat tttttgtgga ttagaagttt 10260
   ttaaattaat gttttttaat ttttgttttt aaaattgaga attgtatttt ttattttagg 10320
   atttgttatt tattttatag gaggttgttg gtttaaatat tatagtaaaa ttatagagga 10380
   gagtattaat gtttttgtat tgtaagtatt ggaagtttag ttagttttgt atgagtatgt 10440
   ttagatagtt gtaaagtggt tatatttttt ttattttggg gtttatttta atttttatta 10500
   tgttttttgt tagtaggaag aagttagagt gatcgatagt ttttttttat ttttatagtt 10560
   tatattttaa gattaagtta ttagaaaatt taaatagagg gattgaaatt gtttttgtaa 10620
   tattataatt gaggtaatta cgatagtgaa agaaataaga tttaattaat tttattttgt 10680
   ttttattttt taagttgttt ttatttattt ttgggtatag gtcgaattga ttttgggaag 10740
   gaatttagtt tatggtttga ttgaaataaa atcggtaata gttttttttc gaaaagattt 10800
   tttttttgtt tggggtttag tttttttttg taggattaat aaattagtta taagattaaa 10860
   aattataatg taagggatat gtagtttttg gtttttagaa ttggaatgtt tttattgtgt 10920
   ttttagggat aatattattt tataaaattt aatattagta tttaagatat tttttagacg 10980
   ttgtatttaa tggattagtt gatattattt aaattagtaa tttaatttaa ttagttttgt 11040
   tattatattt aggaagagaa tatagtaaga aaattttatt ttgatatttt atgattttat 11100
   tttttatttg agtaattagt attttttatt ttttaagttt ttagttgtta aattattttt 11160
   aaacgttttt atttttgaat gttcggggag attgatttaa gtaataataa tattattttt 11220
   tttagaatag tcggatttgc gtgaattatt tttacgttat tgtaattttt ttgttttgat 11280
   aaattggttt tgtttaagta gtgggtaagg tgaatttatt tggtgattat aaatttttaa 11340
   tagtaaatta ggtaagtttg attttattta atagatgata gttgtatgtt ggatatttta 11400
   atagaaggaa ggtatgttta tttttttatt tatttaggta tatttaatga gtataaattt 11460
   ttgaaattta ttttgttgtt attgggggta ttattttggt agtatgtttg tatattttga 11520
   agtttgtagt taagtggaaa aatatatatg taaataagta aaatgaaaaa tattataaga 11580
   atgtaaataa ttagtagaag tttaagaaaa gtataagtaa agtgtgtttt aggtatagtt 11640
   gtttagagaa gggagggttt ggtattgaga tttgaaggat tggtaggaat ttgttttttg 11700
   gaaaattagg agttggtttt ttggtagaga gtagtaagga aaaaagtata aaggtttgag 11760
   ggtgtttcgt gtaattagaa aattgggaga gttgggtttt agaaggaata gaaatgtgag 11820
   gttaggattg aagtggtagt gattagagat agggtaagta tttgaagatg tttgtagaaa 11880
   gttagattaa tggtagagtt tgagagttat gttaatttga gattattttt tttaatagat 11940
   ttttgattta aagtatattt tggaggtatt ttttaagtat tttttttaat tataatatgg 12000
   aaataatagt ataattgtat tgattgatgt taaatattaa attagagaga gtgtatgaat 12060
   tagttattat atggtaagta ttttttaaag aaatggttat tatagattat tttttgttat 12120
   tataataatt attgaatgaa agttgtagtg ataataaaaa tggtaggaat taattagagg 12180
   aaaaaattta ttaggattat agttttggtt ttaggtaaag tgatgtaatg tttaaatata 12240
   ttataggata taatttttta agagtttaag gtaataatag ttgtttatat ttggttgttt 12300
   ataattatta aattacgatt tgtttttgat tagtggtttt taatttattt tatttttatt 12360
   taatgatttt ttgattttag agtttaagtt aaatgttgtg aagttagata taaatttcgg 12420
   agagaattgt tttataggga gaattaaagg agtaaagaaa taataataag gtaatatgag 12480
   tgaagtaaat atagtatttt tttatgaagt tgaaattatg ttttataaag attaatggaa 12540
   aaattggata tatatgtttt aaaaggtaga aaaaattgtt tgaaagttta gatagtaaaa 12600
   aaaagaaaag gtaataggga gttattgtat gtttttgagt aaagttaatt atattaatag 12660
   attattagta gataagtaga gagtgttaat taattggtta atttttttaa tagaataaat 12720
   ttatgttaag ttagttttat aagaattatg tgacgatttt taaaggtaat aaatgtttgt 12780
   agggtattta agattgagtt attcgcggtg aggaaggggg tagttatttg atttttttta 12840
   tatgattata gattgttgcg atatgttata aggtttagaa tttattgatt tagtttaatt 12900
   tttttttttt tttgtgagga gaatgaagtt aaagaagcgt tatgatttgt tggaatatta 12960
   gttgtttaag taattttatt ttttaggttt ttttagtttt tgtgtataac gataggtgat 13020
   tataaaagtt gtgtgtagaa tggattggag agttaagttt tggagggaag gatattagtt 13080
   aaaaataata ataacggtaa aataaaataa ttgtgatatt aatttatgaa atattatatt 13140
   aaagtagtta gtattaggat taacgagaaa gaggaaaaaa ttgaattatt ttaaatagat   13200
   taagaatgta atttataata agagatgtcg aagaagataa taatttagag gtttttagat   13260
   ttgattttgc gaaaaaggtt tgtgatttgg gtaattaatt tggaattaaa agaaagattt   13320
   tagagtgtat ttcgaaattt ttagaattgt tttatataaa taattttttt tggttttttt   13380
   tatttttttt ttgattatta gaagttgaaa gaaaggaatt aatttttttt tggtttaaag   13440
   atatttggaa aatatatgat tttcgttaat atttttaata ttaggtttta tgatttaaat   13500
   tatatataat tagtaagaaa aggaatttaa tatgtgtgtt atcggtttat tttttttttt   13560
   gtgtttattt tagagtaata aattaagtgt tttttaagtt tttattttta agtttttagt   13620
   tagaaaagga tttatttgtt atagggaata gtttttgttt ttttttatag cgttggaaag   13680
   agttttagag ttaaaaatga aagtaaatta tgtatttttt tattgtaaag ggcggaaacg   13740
   tgtaattata gattgtgaaa ttgttgttta gaattttaga tttattatta ggaagtgttt   13800
   tgttattagt aaattggtat ttaattattg gaatttagtt tttgatttgg gggaaaaaaa   13860
   tgaaaatgat aaaatattat tttgtgggtt gatattttag tttgtttttt tttttgtttt   13920
   ttgtggttat ttaaattttt tttatttatt tgtttttttt ttttttgatt ttttagtttt   13980
   tttttttttg attataaaga acgataatga ttgaagaagt ttaatttaat aatttttttt   14040
   tattttttga taagaattaa tttaatagtt tatattttta gttttttggg taggtattga   14100
   gttgtaggag tttttatata gtttagtagt tttcggaatt ttagtgaggt aggagtttcg   14160
   tttattttta tggaaagggg gttgaagtta gggagtaaaa tggttttgta gtaggtttat   14220
   ttttatagaa gtttgtaagt ttagattcgt tggattagaa tttttattag ttagtatagt   14280
   agtttggagt acgttagaga tgatcgagtt ttttagggga ggggcgattg ttattattgt   14340
   agttttagtt agcggttttt ttttattagt gttagggagg ttgggtagat tggattgggt   14400
   agtagttttt atagtgtagt atagtcgttg tggtagatta tggttagatt gtttttttag   14460
   gtgggatcgg gatttatttt ttttttttcg gtaagaaatg tttgtaggaa ttttagtaat   14520
   tttatttaga gtttatagat agagttttta tttttttggt atagagtatt tgcggagagg   14580
   ggtagttgcg gttttaggtt tagttaattt aatttttttt gtttgttggt tttgaagagt   14640
   attagcgatt tagataaggg ggattttttt agtttagtgt attatttttg ttaagggata   14700
   gttagatttt ttttttaagt gagtttttga ttttgggttt tttgattggg ttagattatt   14760
   taataggggt tgttagatat tttatatagg agagttttaa taggcgttag gttagtgatt   14820
   ttttgggaag aagttttcgg aggaaggagg aggtagtatt ttttgttgtt ttgtagtttt   14880
   tattggtgat atataggtga atagtgtttg gagtagattt ttagtatatt gtagtagatt   14940
   tgtagaggaa gggtttgaat gttggaagaa aaataaaaaa aaataaaata ataataatat   15000
   agtattaata aaaaagattt tataaaaatt ttatttaaag gttaatagtt ttaaagatta   15060
   aaggtagata aatttataaa gatgacgaaa aattaacgta aaatcgttga aaattttaaa   15120
   agttggaatg tttttttttt ttaaatgatt ttaatatttt tttagtaagg gtataaaata   15180
   gggttgaagt tgagatggat gaattgatag aagtaggtat tagaaagtgt gtaatgataa   15240
   attttgttga gttaaaggat tatattttaa tttaaggtaa agaagttaag aatcgtgata   15300
   aaagattata ggaattgtta attagaataa ttagtttaga gaggaatata aatgatatga   15360
   tggagttgag aaatatagcg tgagattttt atgatttaaa tataagtatt aatagttaaa   15420
   ttaattaggt agaagaaaga gtattagagt ttgaagatta ttttgttaaa ataaggtagg   15480
   tagaaaagat tatagaaaaa aataaaaagt aatgaataat attttttaga attatgggat   15540
   tatgtaaaaa gattaaattt atgattgatt gaagtatttg aaagagatgt ggagaatgga   15600
   gttaagttaa aaaatatatt ttaggatatt atttaggaga atatttttaa tttagtaaga   15660
   taggttaata tttaaattta ggaaatttag atagttttag taagatattt tataagaaga   15720
   ttaattttaa gatatataat tattagattt tttaaggttg aaatgaaagg aaaaaatgtt   15780
   aagggtagtt agagagagag gttaggttat ttatagaggg aagtttatta gattagtagt   15840
   ggaattttta gtagaaattt tataagttag aaaagattgg agattaatat ttaatattaa   15900
   taaagaaaat aatttttaat ttagaattta atgtttggtt aaattaagtt ttataagtga   15960
   aggagaaata aaattttttt tagataagta aatgttgagg gattttgtta ttattaggtt   16020
   tgttttgaaa gatattttgt aggaaatagt agttatggaa aggaaaattt gttagtagtt   16080
   attataaaaa tatattaaaa tatataggtt tatgatatta tgaagtaatt atattaataa   16140
   gtttgtaaaa ttaattaggt aatattatga tgatagaatt aaatttatat atattaatat   16200
   taattttaaa ataaatgggt taaatttttt aattaaaaga tatataatgg taagttggat   16260
   aaaaagatag gatttattag tgtgttgtat ttaagagata ttgttggtga gaatgtaaat   16320
   taggttaatg attgtggaag agtgtgtggt gagttattta gaatagaaat attatttgat   16380
   taagaaatat tattattggg tatatattta gaagaatata aattatttta ttataaagat   16440
   atattatata tatgtatatg tttattgtag tattatttat aatagtaaag atatgaaatt   16500
   aattaaaatg tttattaatg gtaggttgga tagagaaaat gtgatatata tatattatgg   16560
   aatataatgt agttataaaa atgaatgaga ttatgttttt tgtaaggata agaatgaagt   16620
   tggaagatgt tattcgtagt agattaatgt aggaatagaa aattaaatat tgtatgtttt   16680
   tatttataag tgggagttga ataataagaa tatatggatt tagggaaggg aataatatat   16740
   atggaggtta gataagagga tgggggatgt gagagagtat taaggaaaag agataatgta   16800
   tgtttggttt aataattagg tgatgggttg atttgtgtag taaattatta tggtatacgt   16860
   ttatttatgt aataaattgc gtattttgta tatgtatttt agaatttaaa aaaatattaa   16920
   aataaaatgg tagtatgaag tttaatattg aaggattgta agaggttaga aattcgtttt   16980
   tatttttaat gagtttggga ttagaaaagg tttatgattg taattgaatt tgtagttttt   17040
   ttagtttttt aaaattttta tattattgag tgattgattt aattttaaat tatttttttt   17100
   gtataatata agtgtataat tttgtattta gtgtattgta gtgttttttt agtattttat   17160
   attttgtttg ttattttttt tatgtttgtt gattgttata tatttttaag gaggttgtga   17220
   aatattaggg atatatggtt gaattattta tttttttaaa gttgtgaaaa tataataaag   17280
   aatatatttt ggttatttta ttaaatgata ttaaatagtt taagtttaaa ttattaaata   17340
   gtatttttaa tggtgaaaat ataggtagta tattgttata tttgaatgtt tttttttaga   17400
   aatataattt tagtttttat atatattata agatagtttt aaaggaagaa gtgatatttt   17460
   ttttatagta tttaagatat ttaatataga aatgaaaagc ggtttaatag aaataaagat   17520
   aaagagtggt ttttatttta gaagaaaagt aatggtagat ttaaagtagg tagaaaagat   17580
   aataggattt aggaatttta tagtttgtgg tttaattttt gggttttttt tttttaatgt   17640
   aaatgtgtat aaagatgatt tatttttatt ttatataaat tttggtgttg tgggaagtta   17700
   gggatttcga atagagggat cggttaaagt tatggtagaa gaatataaat tgtgaagatt   17760
   ttatggatat ttattagttt tttaaattaa tatttttata attttttatg tttgttttta   17820
   ttgtaatttt tgaatataaa ttgtggagat tttatggata tttattattt ttttaattaa   17880
   tatttttgtg attttttatg tttgttttta ttttaatttt tttattttat tatttttgta   17940
   agttgagggt gtatgtcgtt ttaggatttt gtgataattg cgttaattgt ataaattgtt   18000
   taaataatat gaaatttggg tattttgaaa aaagaataag ataagagcga cgtttaggga   18060
   ataagggaga taattattag gtttggttgt ttgagagttg ggcggaatag agtcgtattt   18120
   tttttttttt aaaagtaaat aggagaaata ttgttgaatt ttttttttta gtaaggaata   18180
   gaatgcgttt ttagggggag gtttttaaaa tggttgtttt agggatgttt gttttttacg   18240
   gttgttataa ggaatgaaat aagtttcggt tttttatagt gtttttaggt ttattaggat   18300
   gaggaaattt ttatttaata aattttggtt agattggttg tttgttttta aattttgttt   18360
   tatgataaga tgttattaat gataatgcgt gtttaaagtt ttattagtaa ttttaatttt   18420
   attttggttt tgtgatttta ttttgttttt atttgttttg tgatatttta ttattttgtg   18480
   aagtacgtga tttttgtgat ttatatttta ttcgtatatt tttttttttt ttgaaaatta   18540
   ttaataaaaa tttgttggtt ttgtggttta gggggtatta tggaatttgt cgatatgtga   18600
   tgtttatttc ggatatttag ttttaaaatt tttttttttt gtattttttt tttttatttt   18660
   ttagattggt cgatatttag gga                                            18683
<210> 35
   <211> 6641
   <212> DNA
   <213> Artificial Sequence
<220>
   <223> chemically treated genomic DNA (Homo sapiens)
<400> 35
   taaaattaag agtagtggtt agattttgaa aatgtttagt aaataaaatt aatgtagtgt      60
   gaattgagtt ggggaataag atatatagaa ggtagaagaa gtttaatatt attaggtttt     120
   gttgtttttt cgtatttgga agttaggtat tattttttat tatatggagg aggaaatgaa     180
   ggatggaaat agaagaaaat tagtagttta attttaaaag tttaattgaa tgaggttgtg     240
   ggagtatttt aagtgacgtt ttatttttgg acgtatgggt ttttatacgg gatttatatt     300
   tgggttgatt tataaatttg agttatttat ttattatttt aaatttataa ttgatgttat     360
   gagaatttat agattatgaa aagaggtttt taagttaaat gttttgaatt gttttttata     420
   aatattattt tatttattta attggtaaat aaatatttta tgtatttatg tataaaatga     480
   tgtttgaaat atatattgta gaatgtttaa agagttaaat aatatatgta ttattttata     540
   tgtttatttt tttttgtgat tatagtattt aaaatttata ttgttaaata atttttaaga     600
   atttattatt gattatagtt attatgtggt gtaatagatt ttttaaattt attttttcga     660
   tttaattgaa atgttgtttt ttttgattaa tattttttta aattttttgt ttttttttaa     720
   ttattattat tttatttttt gtttttatgt gtttaatttt tgtgagttta tatattagtg     780
   agattatata gtatttgttt tttggtgttt agtttggttt attttattta atgtaatgtt     840
   atttatgtta attaatgttg ttataaatga taagattttt tttttttttt taaagttgaa     900
   tattgttgta ttatgatatt ttttttattt atttatttat ttatagatat ttaggttgat     960
   tttatatttt gtttattgtg aataatgttg taattataat ggaagtgtat atgttttttt    1020
   aatatattga tttaatgttt aaaatatata ttaaatattt gggttgttat attattttta    1080
   atatttttaa tttattttaa tttttaattt tttgaggagt tttttattgt tttttattat    1140
   gattgtatta atttatattt ttattaatag tatataagag tttttttttt tttatatttt    1200
   ttttaatatt tgttattttt ttttttataa tatttatttt aattatttta ataggtgtga    1260
   ggtattattt tattgtggtt ttaatgtttt tggtgattag agatgttgag tattttgtat    1320
   agatttgttg tttatttatg tatttttttt tgaaaaatgt ttattttggt ttttggttta    1380
   ttttgtaata tttgtttttt ttttattgaa ttgtgttttt tatatatttt gaatattaat    1440
   attttattag atatatggat ttaaatattt atttttattt tgttggttat ttttttatat    1500
   tgttgttttt ttggttttgt aaaagttttt tagtttgatg taattttata ttttaatttt    1560
   gttttttttt tttatatttt tagtgttata tttaaaaatt attgtttata ttagtgttat    1620
   taagtttttt tacgtttttg tgtagtagtt ttaaagtttt agttattagg tttatgtttt    1680
   taatttattt tgagttgatt tttgtatatg gtggtagata agggttcgtt tttatttttt    1740
   tgtttttgga tatttaattt ttttaatatt atttatcgaa gaaagtgttt ttagtttatt    1800
   gtttgttttt gaaattttgt taaaaataaa tttattgtaa ataattgggt ttatttttgg    1860
   tttttttgtt ttattttatt gatttttgtg tttgttttta tgttagtatt atgtttgatt    1920
   attataattt tgtagtatgt tttaaagtta ggttgtgtgt tgtttttagt tttgtttttt    1980
   atttaaggat gttttggtta tttgaatttt taaataattt tatatgaatt ttaaggttga    2040
   tttttttatt tttgtaaaaa atgttattgg aggtttaata ggaattgtat tgaatttata 2100
   gattttttgg gtagtataga tataataatg taatattttt taatttatga atacgggata 2160
   ttttttgatt tttgtgaatt ttaggtattt ttttttgtta atgttttata gttatgagta 2220
   tatatatttt taattttttg gttatattta ttttttatta ttttatgaaa tttttttata 2280
   gttattataa atgagatttt tattatttaa ttttttagtt tgttgttata aaaatgatat 2340
   taatttttgt atgttgtttt tgtatttttt aattttattg tatttgtgta ttagttttaa 2400
   tagatttttt atggagtatt tagggttttt ttaatatatg taagattatg ttatttataa 2460
   ataggatata ttaatttttt ttttttttat tttgatgttt ttaaattttt tttgttgttt 2520
   aatttttttg gttaggattt ttagaataga agcggtgaaa gtgggtgtgt ttgttttttt 2580
   tttaattttt gaagaaaagt ttttattttt ttattattga gtatgatgtt aattgtgtgt 2640
   tttttataag tggtatttat tgtgttgtaa atttttttag gttaattgtt ttttatatgt 2700
   ttttatatat ttatttagtt aaaattatat ttatttatta agtatgtaag taagttaaat 2760
   agaattttag aatttaggta gagtagatat tttattaatt aatttgtgta ttttttgtaa 2820
   atttttatat tttttgtttt aatatttata ttgtatttaa aattaacgtt tttgttttgt 2880
   tttttaagaa gtttttgtaa attttttttt tggaattttt ttattttttt tgagtttttt 2940
   aagtatttta tttgtgtttt tttaagaagt tttttaataa ttattatgta atgtagtttt 3000
   atgatttaag agttggggtt ttggattttg attatttgta tttttatttt tgtttcggag 3060
   ttattgtata ttttattttt tttttatagg attatattta attttttaag taatttattt 3120
   gtatatgttt ttttttttat ataaaagagg ttaatatata ttttgtatgg agtttttgtg 3180
   aaattttagt aaagtatttg attttatggg ggtttaatat atattattat tattgaaagt 3240
   ttgttatggt taattttatg gaatgtggtg taaaaatagg atttgaattt taatttatgt 3300
   gatttagggt agtttattat ttaaatgatt tataaaattg ataaaataat aaatatttta 3360
   ttggtttatt gtgagagttt agtatattat tatttgtaaa gtatttagaa gagagatttg 3420
   taaataataa tgattattat tgatatgata taataataat aatgataatt attgatttat 3480
   tatattagta taatgataat tattgattta ttatattagt ataataatga taattattga 3540
   tttattatat gagtataata atgataatta ttgattaaat tattataata atgataatta 3600
   ttgatattgt taataataat gataattatt tatgatataa tgatgattgt tattgtttta 3660
   tgtgaattta ttttttaatt ataaaaaata tataggagat gttatattta ttaaatgtaa 3720
   ttttttgttt tgttttgtaa tttttatgtg tttttttaat aatggttggt ttattattgg 3780
   tatatgaatt taagataatt tggaaaatga gtttggtgtt tttaggttaa aattttgttt 3840
   aatgttttga tatgtttttt ttagaaaatg tattttgtgt gaaagttatt tttatattta 3900
   gataaattag ataatgattt ttatagagtt aatagaagag aggagatatt tggttttttt 3960
   atatataatt tgtttaaatt atattttttg tattgaaata agtgatttat tattttgata 4020
   tatatatgtg tgtgtgtgtg tgtatgtgtg tatatatata tgtgtatata tatgtatata 4080
   tgtgtatata tatgtgtgta tatatatgta tatatatgtg tatatatatg tgtatatata 4140
   tatgtgtata tatatatgtg tgtgtatata tatatacgtg tgtgtatata tatatatata 4200
   cgtgtgtgtg tatatatata tatatatata tatatatata tatatatgtg tgtgtgtgta 4260
   tatatatatt ttttggtaaa aatttgaatt atattaagga gtaatatatt aatttagtat 4320
   tatattaatt tattataata tttgttaaat atttttaatt ttaaattttg gtttgagaaa 4380
   aggcgttgaa gaattaggta tttagatgta tttatttatt taatgattaa gttggatgtt 4440
   tatttatgta tttagtaagt agattaagta tttttaagta ttttgagtag aaaattgtta 4500
   attagtataa ttgggtatag tttgtgtgtt aaaaaatata aatttaaaga tatagttaat 4560
   ttattaagga agttaaatat agagaaaagg aaagattttg tagtttagta ggaataattt 4620
   atagtttaga taagagaatg gattgttttg ggtgtatata ttttaggtag gttattatga 4680
   gtttgagtaa gagagagaaa tttgaatata atggatgatt aggaatttag tattaggttt 4740
   aatgttgtag aaaggaaaaa gataagttat aaaaaataaa tttaagtggt aatgaattgt 4800
   taaatatagt ttggtaggaa tttgatgggt gagagaatgt tataggtata aagaaataat 4860
   gagtatttgt tgaagatatg gtaagataga atgtattttt aagggtgttt tatattatag 4920
   agttttgttt aaagtaaggg taaaattttt taagttgtgt ttagagttga aggagttttt 4980
   atatttttga tagaagatag tggtaaaaaa aaaaggcgta agaatttgga agttatgttt 5040
   aaaggtattt tttagaagtg gatgggataa tttcgtgata aattatatgt taaagaataa 5100
   cgtggttgga taaatattta tttagatttt agtatttaat acggattaaa agtcgattag 5160
   tgtaaaaagt aagattggat attttataat ggatattttt aattttttag aatttgttta 5220
   attataagtt ttatgtagtg atttttgtga tttttttatt tttttttttt tatgaaatta 5280
   ttatttttat taaatttata attttttttt tagggattat tttgtttttt gtggggtggg 5340
   atttagtttt taatcggtta aaaggttgag tttttgggtt tagattgtgt atggaaataa 5400
   ttaatagtag aggtttgtag taagatatat ttaaatttaa atttagattt tgttattaat 5460
   aatttgtgtg gttttttgta tgtattttaa ttttttttgt tttttaatta ttagttttat 5520
   tatttatgaa atggggataa ttttagtatt tttttataag attgtggaag gttagaaata 5580
   ttgtgttggt tggtattaat atgttaataa tttttaagat attagttgtt atttagaatt 5640
   tttttttttg agttttagat ttatttagtt ttagatgtat ttttaataat tttgttttag 5700
   tgttttattg gttttattaa attaatgatt atttaaaaat taaatatttt tttttttaaa 5760
   taaatttgtt ttaattttaa ttttttttta ttttagtaaa agaaattaat atatattaaa 5820
   ttttttttat tagaatgtaa ttggttgttt tattaatgat tggtagtaat tagaagaggg 5880
   ttaggtaaaa aaatttatta ttttgatatt tatatttttt aataaaaatt taaattatgt 5940
   taaggtgtag tgattaattt gatgttatat aaatttatta tgttagagta gttatgatat 6000
   tatttttaat tttaaatttt gattgaggaa aagatgttaa aaaattagga ttttgggtgt 6060
   agttatttat ttgtttatta agttatgtat ttagtaagta tgttaagtat ttttaagtat 6120
   tttgggtata aagatgttaa tgagtataaa gatgtgggta taggatgtgt gttaaaaatg 6180
   taaatttaaa gatatagtgt tttttttaag gaaattaaat atagtaaaga ggtaagattg     6240
   tatagtttag tagtatttat agtttagata agagtatgga ttgttttggg agtgtttatt     6300
   ttaggtaaat tattattatt atgagttgtg agtaagagaa agagaaatat gaaagtagta     6360
   ggtgattagg aaattagtat tatttttaat gttatagaag gaagaagaaa agttataaaa     6420
   ataaatttaa gaggaaatga attgttaagt atagtttggt atgaatttga taggtgagag     6480
   aattttgtag ttgtaaaaaa agatgtgtgt tattcgttgg agatatggta aaatagaatg     6540
   tatttttaga ggagttttat attatagagt tatgttttag gtgaaggtag aattttttaa    6600
   gttgtgttta tagttaaagg agttttttta tttttgatag a                         6641
<210> 36
   <211> 6641
   <212> DNA
   <213> Artificial Sequence
<220>
   <223> chemically treated genomic DNA (Homo sapiens)
<400> 36
   tttgttagaa atagaaagat ttttttaatt atgggtatag tttaaagagt tttgttttta      60
   tttaagatat aattttgtaa tatgaaattt ttttaaaaat atattttatt ttattatgtt     120
   tttagcgaat aatatatatt tttttttgta attgtagaat ttttttattt attaaattta     180
   tattagattg tgtttagtaa tttatttttt tttaaatttg tttttatagt tttttttttt     240
   ttttttgtgg tattgggaat aatgttaatt ttttggttat ttgttgtttt tatatttttt     300
   ttttttttgt ttatagttta tgatgatgat aatttatttg aaatggatat ttttaggata     360
   gtttatgttt ttgtttagat tgtaaatatt attagattat ataattttgt ttttttgttg    420
   tatttagttt ttttggggaa agtattatat ttttgaattt gtatttttag tatatatttt     480
   atatttatat ttttgtgttt attaatattt ttgtgtttaa aatgtttaaa agtatttagt     540
   atgtttgtta aatatatgat ttaatgaata aatggatgat tgtatttaga gttttaattt     600
   tttagtattt tttttttaat taaaatttga aattgaaaat aatattataa ttattttagt      660
   atggtgaatt tatgtgatat taggttaatt attatatttt agtataattt gaatttttat     720
   tggaaaatat agatattaaa gtgataaatt tttttgtttg gttttttttt aattattgtt     780
   agttattggt aaagtaatta gttgtatttt ggtgggagaa atttgatata tattagtttt     840
   ttttattaaa atggaaaggg gttaaagttg gaataaattt gtttagaaga aaaagtgttt     900
   aatttttagg taattattag tttaatgaag ttaatgaaat attggagtag aattgttgaa     960
   aatatattta gagttaaatg agtttggagt ttagaggaga gagttttgga tgatagttaa    1020
   tattttggga gttattggta tgttgatgtt agttaatata atgtttttaa ttttttatag   1080
   ttttgtgaga aagtattgaa attgttttta ttttatagat ggtaagattg atgattaggg    1140
   agtaagagag gttaagatat atgtaggaag ttatatagat tattagtaat agaatttaga    1200
   tttaggtttg aatatgtttt attataagtt tttattatta attattttta tgtataattt    1260
   gaatttaagg atttagtttt ttagtcgatt ggaaattgga ttttatttta taaaagataa    1320
   ggtagttttt gaaggaaaaa ttataggttt ggtgaaaatg atagttttat aaaggaagga    1380
   ggataggggg attatagaag ttattgtatg ggatttataa ttgaatagat tttaaaaaat    1440
   tggaaatatt tattgtaaaa tatttagttt tattttttat attggtcgat ttttgattcg   1500
   tattaggtat tagggtttgg atagatgttt gtttagttac gttgtttttt agtatgtggt    1560
   ttattacgaa gttattttat ttatttttga aggatatttt tggatatagt ttttaaattt    1620
   ttacgttttt tttttttgtt attgtttttt attagaaata taaagatttt tttaattttg    1680
   ggtatagttt aaagagtttt gtttttgttt tagataaagt tttgtaatgt gaaatatttt    1740
   tgaaaatata ttttgtttta ttatgttttt aataaatatt tattattttt ttgtatttgt    1800
   agtatttttt tatttattaa atttttatta ggttgtgttt aataatttat tgttatttaa    1860
   gtttgttttt tatagtttgt tttttttttt tttgtagtat tgggtttaat gttaaatttt    1920
   tggttattta ttgtatttag attttttttt tttgtttaaa tttatgatga tttatttgaa    1980
   gtgtgtgtat ttaggatagt ttattttttt gtttagattg taaattattt ttattagatt    2040
   gtaggatttt tttttttttt tgtatttagt ttttttggtg aattaattat atttttgaat    2100
   ttgtgttttt taatatataa gttatattta attgtgttaa ttaataattt tttgtttaaa    2160
   atgtttaaaa gtgtttaatt tgtttgttga atatataagt aaatatttaa tttaattatt    2220
   aagtggataa atgtatttag atatttaatt ttttagcgtt tttttttaaa ttaaaattta   2280
   gaattgaaaa tatttaataa atattatggt agattaatat aatattgagt taatgtatta    2340
   ttttttggta taatttagat ttttattagg agatatatat atatatatat atatatatat   2400
   atatatatat atatatatat atatatatat atatatatac gtatatatat atatatatat    2460
   atacgtatat atatatatat atatatatat atatatatat atatatatat atatatatat    2520
   atatatatat atatatatat atatatatat atatatatat atatatatat atatatatat    2580
   atatatatat atatatatat atatatatat atatatatat atattaaagt aataaattat    2640
   ttattttaat ataagagata tgatttaagt aggttgtatg tggaaaagtt agatattttt    2700
   ttttttttgt taattttata aagattatta tttagtttat ttaaatataa ggatagtttt    2760
   tatataaaat atatttttta aagaaaatat gttaggatat tgaataaaat tttggtttag    2820
   aagtattaga tttatttttt aggttatttt gagtttatat gttaataatg aattaattat    2880
   tattagaaag atatatggaa gttataaaat aggataaaaa gttatattta ataaatgtaa    2940
   tattttttat gtattttttg tgattaaaaa atgaatttat ataaagtaat gataattatt     3000
   attatattat gagtaattat tattattatt aataatatta gtaattatta ttattataat 3060
   gatttaatta gtaattatta ttattatatt tatataataa attagtaatt attattatta 3120
   tattgatata ataaattagt aattattatt atattgatat aataaattag taattattat 3180
   tattattatt atattatatt agtaataatt attattattt ataagttttt tttttaagta 3240
   ttttataaat agtagtatat tggattttta taataaatta gtagaatatt tattatttta 3300
   ttaattttat aagttattta aataataggt tgttttaaat tatatgaatt agggtttaaa 3360
   ttttattttt atattatatt ttatagagtt aattatgata ggtttttagt aataataata 3420
   tatgttgaat ttttatgagg ttaagtgttt tattgaaatt ttataaaaat tttatgtaaa 3480
   atgtatatta atttttttta tatggaaaag gaaatatata tagataagtt atttaagaaa 3540
   ttgagtgtaa ttttgtgaaa agaaaatgaa gtatatagta atttcggagt aggaatagaa 3600
   gtataagtga ttagaattta gagttttaat ttttaagtta tgaaattata ttatatgata 3660
   gttgttgaag agttttttaa gagaatatag ataagatgtt taaggaattt agaaaagatg 3720
   agaaaatttt aggaggagag tttatagagg ttttttagaa gataagataa aaacgttggt 3780
   tttaaatgta atgtagatgt tggaataggg aatgtgaaga tttgtaaaga gtatatagat 3840
   tagttaatgg aatgtttatt ttgtttgagt tttgaagttt tatttaattt atttgtatgt 3900
   ttagtaaatg aatatagttt tgattgaatg aatatgtgga gatatgtgaa aagtaattaa 3960
   tttaaagagg tttataatat aataaatgtt atttatagaa aatatatagt taatattata 4020
   tttaatggtg aaaagataaa agtttttttt taaagattag gaagaggata aatatattta 4080
   tttttatcgt ttttatttta gaaattttaa ttagaggaat tagataatag aaagaattta 4140
   aaggtattaa aataggagag gaagaagtta atgtgttttg tttatgaatg atatgatttt 4200
   atatatatta agaaaatttt aaatgtttta tgaaaaattt gttggaatta atatataaat 4260
   gtagtaaagt tgaaggatat aaaaataata tgtaaaaatt agtattattt ttataataat 4320
   aaattaaaaa attggatgat aaaaatttta tttataatgg ttataaaaaa attttatgaa 4380
   ataataagga ataaatgtaa ttaaaaagtt aaagatgtat atatttataa ttataaaata 4440
   ttgatagaag aaaatgtttg agatttataa aaattaaaag atatttcgtg tttatgaatt 4500
   ggaaaatatt atattattat gtttatatta tttaagaaat ttatgaattt aatataattt 4560
   ttattaagtt tttaatggta ttttttatag aaataggaaa attaatttta aaatttatat 4620
   ggaattattt aaaaatttaa atagttaaag tatttttgag taaagaataa aattggaggt 4680
   agtatataat ttgattttaa aatatattgt aaagttataa taattaaata tagtattggt 4740
   ataaaagtaa atatagagat taatggaata aaataaaaag gttagaaata aatttagtta 4800
   tttatagtaa atttattttt gataaagttt taagaataga taataggtta aggatatttt 4860
   tttcgataaa tgatgttggg aaaattaaat atttaagagt agaagaatga aaacgggttt 4920
   ttatttgtta ttatatataa aaattaattt aaaatggatt aaaaatataa gtttaataat 4980
   tgaaattttg aaattattat ataaaaacgt ggaaaagttt aatgatattg gtatagataa 5040
   tgatttttgg atataatatt aaaaatatgg gaaaagaaag taaaattaag atatgagatt 5100
   atattaaatt aaaaagtttt tgtaaagtta aggaaataat agtgtgaaga gataattaat 5160
   agaataggag taaatatttg aatttatatg tttgataaga tattaatatt taaaatatat 5220
   gagaaatata atttaatagg aagaaaataa atattataaa atgagttaag gattaaaata 5280
   gatatttttt aaaggaagat gtataaatgg ataataggtt tatataaaat gtttaatatt 5340
   tttgattatt agggatatta aaattataat gagataatgt tttatatttg ttagaatgat 5400
   tagaatgggt attataaaaa aaaagataat aagtgttgga aaggatgtag agaaaaagga 5460
   atttttgtat attgttggtg aaaatataaa ttagtatagt tataatgaaa aataatagga 5520
   aattttttaa aaagttaaaa attaaaatga attaaaaatg ttaaaaatga tgtagtagtt 5580
   taaatattta gtatgtattt taaatattaa gttagtgtgt tgaaaagata tatatatttt 5640
   tattgtgatt gtagtattat ttataatagg taagatataa aattaattta agtatttgta 5700
   gatggatgaa tggatggaga aagtattata atgtaatagt atttagtttt agaaaaaaga 5760
   aagaaatttt gttatttgtg ataatattga ttaatatgga taatattata ttaaataaaa 5820
   taagttaagt tgggtattaa aaaataaata ttatatgatt ttattgatgt gtagatttat 5880
   aaaagttaaa tatataaaag tagagggtag aatggtggtg attaggagga ggtaggaagt 5940
   ttggggagat gttgattaaa ggaaataata ttttaattag atcggagaag taagtttaag 6000
   aaatttattg tattatatag tgattatagt taatgatgaa tttttgaaaa ttgtttgata 6060
   gtgtaaattt taagtgttgt aattataaaa aaagatgagt atatgaggta atgtatatgt 6120
   tatttagttt tttaggtatt ttataatata tattttaaat attattttgt atataagtat 6180
   ataaaatatt tatttgttaa ttaaataaat aaaataatat ttataaaaaa taatttaaga 6240
   tatttggttt gagagttttt ttttataatt tataaatttt tatagtatta attataagtt 6300
   taaaataata aataaatgat ttaaatttat ggattagttt agatgtggat ttcgtatgga 6360
   aatttatacg tttaaaaatg agacgttatt taaaatattt ttataatttt atttaattga 6420
   atttttaaaa ttgaattgtt aatttttttt tatttttatt ttttattttt ttttttatat 6480
   agtaaaaaat aatatttaat ttttaagtgc gggaaggtag taaaatttgg tgatattaaa 6540
   ttttttttgt tttttgtatg ttttattttt taatttaatt tatattgtat tgattttatt 6600
   tattaaatat ttttagaatt tgattattat ttttaatttt a 6641
<210> 37
   <211> 5527
   <212> DNA
   <213> Artificial Sequence
<220>
   <223> chemically treated genomic DNA (Homo sapiens)
<400> 37
   ggttttttgt tgttgttaaa ttaataattt tagtttttgg gtttagttat ttgttgttgt      60
   taaaaatgtt gttgagagtg atggagaggt gttttagttt ttattttata gattagaaaa    120
   ttaaggttta gagaaattaa gtagttttgg agataggagt tatggttttt ttatttgagt    180
   tattattttg aaatattaag gataagttaa ttttattaat agttttaggt ttgtgagaaa    240
   tgggtttggt ttattttgaa gattgtaaat aaataaaaaa atagattgtt ttattattga    300
   tgagaaatat gagttatttt gttaggaatt gaaagtatag ttgtttataa aaaagttatg    360
   tcggggttta gtttttttag gtttttgtgt gtgaaaaatt atttagttaa agtttttttt    420
   ttattttttt ttgtgtttag taggatgttg aaatttttgg aaaagataat ggaaattagg    480
   ggtaggaaga gaatagtgtg gggtgttaaa tagattttag taattttttt gtttacggtt    540
   atttttgcgt agatatttcg attttttatg cgtaggtgga gtagcgtttt ttagtttttt    600
   ttagaggttt ttttttgttt gttttttagt tatatcgttt gagattgata acgttttata    660
   gtaagacgga aatttttttt tatagtatta ttttgcgatt ttgtagttat aaagttgaga    720
   tttttgaacg tggtattttt ttgttttttt attgttttac gtgtatttgt gttatttttt    780
   ttgttttatc gtaaatatat ttttaaaatt tttatttttt ttatttagaa atagaattat    840
   aagcggatgg ttaaataatt taaataaatg gtttatgggg aaaagtgaat tttatattgt    900
   tttttaaatt tttagtgttt tttatggaga taattattgt ttttaatgtt ttgaatattt    960
   ttttagagat attttatttt tatataagta tatgtgtatt tgtgtttttt ttttattttt   1020
   tttttataaa tattagtaga ttgttttgta ttttggttag tttatttaat atattttgga   1080
   gttaggtgcg gtggtttatg tttgtaattt tagtattttg ggaggtcgag gtgggcggat   1140
   tatttgaggt cgggagttcg agattagttt gattaacgtg gagaaatttc gtttttatta   1200
   aaaatataaa attagttagg cgtggtggta tatgtttgta attttagtta ttagggaggt   1260
   tgaggtagga gaatcgtttg aatttgggag gcggaggttg cggtgagtcg agatcgtgtt   1320
   attgtatttt agtttgggta atagaatata tagatttgtt tgattttttt tatgattgtt   1380
   atagttttat tgaatgtatg gattattagt gttttagtta gtattttagt gatgggtatt   1440
   tagggattat aaattttata agggtagaaa taatatttta tttatttttt gtttttgata   1500
   tggtttggat gtgtgttttt gtttaaatta tacgttaagg taatttttta tgttagaggt   1560
   ggggtttggt ggaggtgatt ggattatggg gatggatttt ttatgaatgg gttagtatta   1620
   tttttttggt gttgtttttg tgatagagtt tttatgagat ttggttgttt aaatgtgtgt   1680
   ggtatttttt tatttttttg gttaagtttt tgttatgtaa gatgtttgtt tttgttttgt   1740
   tttttgttat gaataaaagt tttttgaggt ttttttagaa gtagatgtta ttatgttttt   1800
   tgtatagttt gtggaattgt gagttaatta agtttttttt ttttatgaat tatttagttt   1860
   taggtatttt tttgtaatag tgtgaggatg agttaatata gttttttata ttgtaattta   1920
   aggtaatgtt ttgtataaag ggatgaatta gattgtttag taattaaaat ataaatataa   1980
   attataagta tatttattta tgaattgggg gatagttttt atttgatttt gagttttgat   2040
   atttgttgtt ttgttttgtt aaaaatgtgt tagtttttat ttagtttttt attagttata   2100
   ttattaatta atatgtataa tatgtataat ttgttaggtt aggaggaaat ttatggatat   2160
   aagaaatgtt tttttttttt aggaaatgat aatttgtttt gggagagaag gtaggtattt   2220
   tttggtaaaa gtgatataat aaaataatta aaaaaagatg gatttttttt ttttttttga   2280
   gatggagtta cgttttgtta tttaggttgg agtgtagtgg cgcgatttcg gtttattgta   2340
   atattcgttt tttaggttta agcgattttt ttgttttagg ttttcgagta gttgggatta   2400
   taggtattta ttattacgtt cggttaatat tttgtatttt tagtagagac ggggttttat   2460
   tgtgttagtt aggatggttt cgattttttg attttgtgat tcgtttgttt cggtttttta   2520
   aagtgttggg attataaata tgagttattg tattaggcga aaagatggat tttttaaaaa   2580
   aggtgttagg ataattaggt ttttatttgt aagaaagtaa agttagaagt ttattttaga   2640
   ttatgtatat tagtataaaa atgttaaatg gtttaaagat aaaatgttaa aataagttta   2700
   gaaaggtatt aaaagaaaat atagaaaata tagtattagt aaaaattttt ttatataaga   2760
   tgtaaaagtt agaagttata aaggaaaagg ttgataaatt agaatagata aaaatgtaaa   2820
   atttttgtat aatataagat ataaagaatg aatttaaaga tatataatga aggttaggaa   2880
   gatgaagatg ttttggcgat gagtggtggt tatatattag tattaatgta tttaatgtta   2940
   ttgaattgta tatttaaaat gatgaaaatg gtaattttta tgttatgtgt attttattat   3000
   ataaaaaaag atatgaaatg aatcggggaa aatatttgtt atataattaa gaatgaaggt   3060
   ttttaataaa atttgtaaaa ttatatatat ttttaggaat gaattaataa ataattttta   3120
   tgaattagaa aaaagtgata atttaattaa aaaatgaata agggatataa gtaatgtgtt   3180
   ttatagaaaa aataaaaatt gataatgaag ttatgaaaaa atgtttagtt tttttagtaa   3240
   ttgtataaaa taaattaaaa taatgagata ttatttttaa gattagtaaa tgttaaagaa   3300
   aaataataat tggtgagggt gtggggaagt gggtatttat atttatgttt ggaaatataa   3360
   attggtgtaa ttttataggg agagtaattt tataatattt tttaaagatt tatatgtata   3420
   attttatggt agagaaattt attttttttt taggattttt ttttttttaa aaatagtgat   3480
   gtggatgaaa aatatatgtt tattattgta tagggtataa tagttgaaaa ttggaaacga   3540
   taatatttat atttttttta gtaggggaat ggttaaataa attttataag ttatttggta   3600
   gatattaggt atgagttaaa agttagggtt tagttagaga tggaaagtat attagtaatt   3660
   tgaaagggaa aatgtaatat gaagaattat taattagtaa aagaaggtta attgttaaag   3720
   gtataagagt atttaagttg tttgtagtta gtaggtttcg gttggtgagt aggaagttgt   3780
   tcgttgggag gttgttaaag ttttttgaag gtgagtatta ttggttttat aagttgttgg   3840
   tagttatggc gttaagagta ggaagagaag tattagaatt cggaagagaa atttagtttt   3900
   ttgttaggtt ttgtatcgtt tttttggcgt tttttattga taaagttagt aaaattgtgt   3960
   cgttagtaaa ggagattttt ttatgggatg tagtttggtg ttattaaaga gaatagagtg   4020
   gatttggagt ttagatgtaa tataatgatt gatattggta tagtatattt attttgtttt   4080
   tgtaaataaa atggtatatg tgatgttttt ttttgttttt ttgtatataa aataatattt   4140
   gtttttattt attatgtatt tatgttttta ttttgtatgt taggagttaa gtattttgta   4200
   tgtattaatt tattttgttt ttataataat ttttatatgt aggaattatt atagttattt   4260
   tatgaatgag tcgaggaagg tattgagacg ttaagtaatt tgtttaaggt tacgtagtta   4320
   gtaagtggta gagtaagaat tattatggtt ttataagttt aggaaaaagt ttgaaagaat   4380
   taaaatgtta atagcgggga ttttaaggaa gtattgaaga ggttatggga gaagttttta    4440
   ttttgttaaa aaattagttt tttaaataaa taaatatagt gaggtttttt tagaagtaga   4500
   tgttattatg ttttttgtat agtttgtgga attgtgagtt agttaaattt ttttttttta    4560
   taaattattt agttttaggt atttttttat aatagtgtta ggatgagttg atatagtttt   4620
   ttatattgta atttaaggta atgttttgta taaagggatg agttagattg tttagtaatt   4680
   aaaacgtaaa tataaattat aagtatattt atttatgaat tggggggttg ttttgtgtgt   4740
   atagataagg tatatttttt aaaaaaatta tttttttaag aagaaaataa attagttaat    4800
   aaacgataat ttatagtgtt aggaagtgag aaataagtgt gtgataaacg gtggagaatg   4860
   ggagtatttt tcgtagtggg cgggaggaga cgaggagggc gttttttggg gagtggtagt   4920
   ggttggagta aaggtttgga ggaggtaagt tatgtgtttt gagtttttgg tttttgtttt   4980
   attttgtgtt tgagttggtt tgaaggttgg ttgtttagat tgagtttttt gtttgtttgt   5040
   atttcgttaa tagttttaga agaaggtgat tggtggttgt ttgaggaata ttagtgggta   5100
   agagaattag tatttttgga gtatttgttg tttgtgagat taagtattat gtatattgtt   5160
   ttatttattt tttatagtaa ttttattaag tagttttttt ttacgtgaaa agatggaggt   5220
   tgggtggagt aaaaggaggt atttagagtt tttagtaagt gagaggtaga gttgggattt   5280
   gaatttagat ttgtttgata ttgaagttta ggttggtttt attttttcgg attgtttttt   5340
   agggagtaga agatagatat tttattttag ttggttgttt ttagaagttt gattttgttg   5400
   gtttaaaacg attttagttt tttgtttaga ggttgcgggt tgcgggttaa gatattagta   5460
   gaaggagggt ttagttagag aggttgatat gggtttttat ttatcgttat tttttttttt   5520
   ttttagg                                                              5527
<210> 38
   <211> 5527
   <212> DNA
   <213> Artificial Sequence
<220>
   <223> chemically treated genomic DNA (Homo sapiens)
<400> 38
   tttggaagga aggagagatg acggtgagta gaagtttatg ttagtttttt tggttgggtt     60
   ttttttttat tgatgttttg attcgtagtt cgtagttttt gggtaaggaa ttaaagtcgt    120
   tttgagttag tagggttaga tttttagaag tagttagtta aggtaaaata tttgtttttt    180
   attttttgga aagtagttcg gagaggtgga attagtttag attttagtat taggtagatt   240
   tggatttaaa ttttagtttt gttttttatt tgttgaggat tttaaatatt tttttttgtt    300
   ttatttagtt tttatttttt tacgtggaaa agaattgttt ggtaaggttg ttgtggggag    360
   tgaataaagt aatatatata gtgtttaatt ttatagatag tagatgtttt agaaatgtta    420
   atttttttgt ttattggtat tttttaggta gttattagtt attttttttt gaagttgttg   480
   gcggggtata ggtaggtagg aagtttagtt tgaataatta gtttttagat tagtttagat    540
   ataaggtgaa atagaaatta aaaatttaga gtatatgatt tatttttttt aaatttttgt    600
   tttaattatt gttatttttt agggaacgtt tttttcgttt tttttcgttt attgcggaga    660
   gtgtttttat tttttatcgt ttattatata tttgtttttt attttttggt attgtgagtt   720
   gtcgtttatt aattggttta tttttttttt ggaaaaataa tttttttaaa aaatatattt   780
   tatttatgta tataaagtag ttttttaatt tatgaatgga tatgtttgtg gtttgtattt   840
   gcgttttaat tattaagtaa tttggtttat ttttttgtgt aaagtattgt tttaggttat    900
   agtgtaggaa attgtattag tttattttag tattgttata aagaaatatt taagattgga    960
   taatttataa agaaaagagg tttaattggt ttatagtttt ataggttgta taggaagtat   1020
   agtgatattt gtttttgggg aagttttatt gtatttattt atttgaagga ttgatttttt   1080
   aataaagtga aaattttttt tatggttttt ttaatgtttt tttgaggttt tcgttgttaa   1140
   tattttgatt tttttagatt tttttttagg tttataaagt tatagtaatt tttgttttgt  1200
   tatttattag ttgcgtgatt ttgggtaagt tatttaacgt tttagtgttt ttttcggttt   1260
   atttataaag tagttataat gatttttgta tgtgaaggtt attatgagaa taaaatgagt   1320
   taatatatgt aaaatattta gtttttggta tgtagagtaa agatataaat atataataag   1380
   tagaaataaa tattgtttta tatatagaga gataaagaga gatattatat atattatttt   1440
   gtttataaaa gtagggtaag tatattgtgt tagtattaat tattgtgttg tatttgagtt   1500
   ttaagtttat tttgtttttt ttggtgatat taagttatat tttataaaaa gatttttttt   1560
   gttagcggta taattttatt ggttttgtta gtagagggcg ttagagggac ggtgtaaggt   1620
   ttagtagagg attggatttt tttttcgggt tttggtgttt ttttttttgt ttttaacgtt  1680
   atgattgtta gtagtttgta gaattagtgg tgtttatttt tagggaattt tggtagtttt   1740
   ttagcgggta gttttttgtt tattagtcgg ggtttgttga ttgtagatag tttgagtgtt  1800
   tttgtatttt tagtagttag ttttttttta ttagttaata attttttata ttatattttt   1860
   ttttttaaat tattggtgtg ttttttattt ttaattggat tttaattttt agtttatgtt    1920
   tggtatttat tagatggttt gtaaaattta tttaattatt tttttattga agggaatgtg    1980
   agtattatcg tttttagttt ttagttgtta tattttgtgt agtagtgaat atgtgttttt    2040
   tatttatatt attgtttttg aaggaaaaaa aattttggaa gaggaataaa tttttttgtt    2100
   atagggttgt gtatgtaagt ttttggaaaa tgttgtgaga ttgttttttt tataaggttg    2160
   tattaattta tatttttaaa tataggtgta agtgtttatt tttttatatt tttattaatt    2220
   attatttttt tttaatattt attaatttta ggggtaatgt tttattgttt tagtttgttt    2280
   tgtgtaatta ttaaggagat tgaatatttt tttataattt tattgttaat ttttattttt    2340
   tttgtgaaat atattgttta tattttttat ttatttttta gttggattgt tatttttttt    2400
   taatttatag aaattatttg ttgatttatt tttaaaagtg tgtatagttt tatagatttt    2460
   attaagggtt tttattttta gttatgtagt aaatattttt ttcgatttat tttatgtttt    2520
   tttttatgtg atgaaatata tataatataa aaattattat ttttattatt ttaagtgtat    2580
   agtttagtaa tattaagtat attaatattg atgtgtaatt attatttatc gttaaaatat    2640
   ttttattttt ttaattttta ttgtatgttt ttaaatttat tttttgtgtt ttatattata    2700
   taaaagtttt atatttttat ttattttaat ttattaattt ttttttttat gatttttggt    2760
   ttttatattt tgtgtaggga aatttttgtt ggtgttgtat tttttatatt tttttttaat    2820
   atttttttag atttgtttta atattttatt tttaagttat ttgatatttt tgtgttggta    2880
   tgtatggttt gaggtgggtt tttaatttta tttttttgta ggtggggatt tgattatttt    2940
   aatatttttt ttaaaaaatt tattttttcg tttggtgtag tggtttatgt ttgtaatttt    3000
   agtattttgg gaggtcgaag taggcggatt ataaggttag gagatcgaga ttattttggt    3060
   taatatagtg aaatttcgtt tttattaaaa atataaaata ttagtcgggc gtggtggtgg    3120
   gtgtttgtag ttttagttat tcgggagttt gagataggag aatcgtttga atttgggagg    3180
   cggatgttgt agtgagtcga gatcgcgtta ttgtatttta gtttgggtga tagagcgtga    3240
   ttttatttta aaaaagaaaa aaagatttat ttttttttga ttattttatt atgttatttt    3300
   tgttaaaaag tatttgtttt tttttttaaa gtagattatt attttttaga agggagggat    3360
   attttttgta tttataagtt tttttttgat ttggtagatt gtatatgtta tatatgttgg    3420
   ttggtgatat gattgatagg aagttgaatg agaattagta tatttttaat aaagtaaagt    3480
   agtaggtatt agaatttaga attaaataaa agttgttttt taatttatga atggatatgt    3540
   ttgtggtttg tatttgtgtt ttaattatta agtaatttgg tttatttttt tgtgtaaagt    3600
   attgttttag gttatagtgt aggaaattgt attagtttat ttttatattg ttataaagaa    3660
   atatttaaga ttggataatt tataaggaaa agaggtttaa ttggtttata gttttatagg    3720
   ttgtatagga agtatagtgg tatttgtttt tggggaagtt ttagagagtt tttatttatg    3780
   gtagaaggta aagtaagggt aagtatttta tatggtagga atttgattaa gagagtgggg    3840
   aggtgttata tatatttaaa taattagatt ttatgagagt tttattataa gaatagtatt    3900
   aaagggatgg tgttaattta tttatgaagg atttattttt atgatttaat tatttttatt    3960
   aggttttatt tttgatatgg gggattattt tgacgtgtga tttgggtagg gatatatatt    4020
   taaattatat tagaaataga ggatgggtaa gatattgttt ttgtttttat gaagtttgtg    4080
   atttttaaat gtttattatt aaggtgttag ttggagtatt gatgatttat atatttaatg    4140
   gagttgtagt agttatgaaa aggattaagt agatttatat gttttgttgt ttaggttgga    4200
   gtgtaatggt acgatttcgg tttatcgtaa ttttcgtttt ttaggtttaa gcgatttttt    4260
   tgttttagtt tttttagtag ttgagattat aggtatgtgt tattacgttt ggttaatttt    4320
   gtatttttag tagagacggg gtttttttac gttggttagg ttggtttcga attttcgatt    4380
   ttaggtgatt cgtttatttc ggttttttaa agtgttggga ttataggtat gagttatcgt    4440
   atttggtttt aagatatgtt aaatgagtta attaaggtgt aaaatagttt gttagtgttt    4500
   gtggggggaa aatgagggaa ggatataaat atatatatgt ttatataaga ataaaatatt    4560
   tttgaaagaa tatttaaaat attagaaata ataattattt ttataaaagg tattgaaagt    4620
   ttgggggata gtgtgaaatt tatttttttt tatggattat ttgtttaaat tgtttaatta    4680
   ttcgtttgta gttttatttt taaataaaga aaatgggaat tttagaagta tgtttacgat    4740
   gaaataagaa agatgatata ggtatacgtg aaatagtaag ggaatagaag agtgttacgt    4800
   ttagaagttt taattttgtg gttgtagaat cgtaaggtgg tgttgtaaag ggaggttttc    4860
   gttttgttgt agggcgttgt taattttagg cgatgtggtt ggaaggtagg tagaaggaag    4920
   tttttggaaa gaattaggaa acgttgtttt atttgcgtat ggggggtcga ggtgtttgcg    4980
   tagagatggt cgtgggtagg gaggttgttg agatttgttt agtattttat attgtttttt    5040
   ttttgttttt gatttttatt gtttttttta gaaattttag tattttattg agtataaaga    5100
   gaaataaaag gggaattttg attgagtggt tttttatata taaggatttg gagaggttgg    5160
   atttcggtat agttttttta tgggtagttg tatttttaat ttttggtaga atgatttata    5220
   ttttttatta gtaatggagt agtttatttt tttatttgtt tataattttt aagataaatt    5280
   aagtttattt tttataggtt taaaattgtt gataaagttg gtttgttttt gatgttttaa    5340
   gatgataatt taagtggaga ggttatggtt tttattttta agattattta gtttttttgg    5400
   gttttgattt tttgatttgt aaaatgggaa ttaaaatatt tttttattat ttttagtaat    5460
   atttttagta gtagtaggtg gttaaattta agggttggag ttgttaattt ggtagtagta    5520
   agaagtt                                                              5527
<210> 39
   <211> 10696
   <212> DNA
   <213> Artificial sequence
<220>
   <223> chemically treated genomic DNA (Homo sapiens)
<400> 39
   ttaaaaggga gttttagagt aaatataggt tttggatttt atcgttaata attatagaat      60
   atgaatatta gtttttttgc gtaaaatatt ttagatagat atatatattg aatatagaaa      120
   tagagataaa taaaagttag ttataattta gattttattt ttgttaagat tgtttagttt     180
   atgttaaatt tttatttttt tgttttattt attattttcg ggcgagtttt ttataagttt      240
   ttttaatatt agtttgattg gttgttcggt tgatttttag gattttgtat gggttaaaat     300
   ttaacgtaga tagaatttgt aggattagaa ttagaattat taagaattgt taggaaatgt     360
   ttaatttttt tatttttatt tttttttttt tttagagttg aaatgattga agaggtaatg        420
   agttttagtg gtagaaattt ggatagtttt gttttttagg taaaggtata agatatatag      480
   atgaaaaata aaattaataa aaggtatttt gtaggaaaag tgttaagttg ttgaattgta     540
   ttttaaaata aaaatttcgt tattgttttt gaatattata aatgagaatt attatagatg     600
   aaaagatgag taataagaat agtaattttt tagaatattt tgattagaaa agttttagaa     660
   atgaatgaaa atgaatggga tgtagaaata atatataaat gagagaaaat ttaaatagga     720
   taattaggac gattattttt taaatatatg attttattta ttttgataag aataaataaa     780
   ataaatggtt taagaatatt ttattgagta tatgttaaat aaatttatga aattgtaaga     840
   tttattatgt ataaaattat aaaatgagat agttgtggta tttttgggta ttaaataagt     900
   ataaatatgg acgtgaaata tttttaaaaa attgtgtagt taagacgtag tatgttttaa     960
   ttttattttt ttttattata ttatttaaat aagaaaatga gaagttgttt tttaattatt     1020
   agtaatatgg tgtttttttg tttttttttt ttgagacgga gttttattcg ttgtttaggt     1080
   tggagtgtaa tgatgggatt ttagtttatt gtaatttttt ttttttaggt ttaagtaagt      1140
   tttttgtttt agttttttta gtagttggga ttataggtat gcgttatttt gtttagttaa      1200
   ttttttgtat ttttagtaga gatagggttt tattatgttg gttaggttgg ttttgaattt     1260
   ttgattttag gtgatttatt cgtttcggtt ttttaaagtg ttgggattat aggcgtgagt     1320
   tatcgcgtcg gttaatatgg ttattattaa tggaaatttt agatgagaaa aagcggtaag     1380
   aaaaaattta gttttatatg tgaagaagaa ttaacggtaa agattaaata gtaggcgtgg     1440
   tttttaaaag ttttaagttt tatttttggt gagtatagtg atttaattta ttgtgaattt     1500
   tggaaataat tttggttggt taatgaatag ggtagtgttg gaattgtaga tatttatatt     1560
   ttataattat gattgttgtt atattttata atgttttttt ttaaggaata tatttatttt     1620
   gattttttat aagttaatat agttttaagg tttaagtgtt ttttgtgata ttttattgaa     1680
   gaagggtaaa gttataatag tggtggattt tcgtaagtgt aagtttaatg tcgtttggga     1740
   gtttttataa aattgattaa tttgatgatt aacgtttaaa attgtatacg tgttgaataa     1800
   aattttaaaa taaattagtt ttttttttgt taaggtaatt tattatattt tagaaaatac      1860
   gtgaaggtaa tgtagttatt ggttaagtat atgggttgaa gattaggatt tattttttat     1920
   tattttatta tttgggtaaa ttatttaaat ttattaagta ttagcgtttt tatttataaa     1980
   atgggaatga atattattat tttgaaggat tattatgtgg ggattaaatg tatgtaaagg     2040
   gtttattgag tttagagtta atagttaata aatggtcgtt agtgttatta tttttattat     2100
   gtagtacgat cgttgtttta tttaattatg ttagtttttt ttagttagag ggtcgaagtg     2160
   gcggtggttt gggaagtttt ttagttgata tgcgagatag acggcgtcga tttcggagta     2220
   gtaaatattt gtttaatagg taggttgtta gttttttggg tagtatattt aggattagga    2280
   agaagttttt agagataagt tttaggttta agtttcgttt cgcgtttttg tcggaggtcg     2340
   taattttgta ggagatagtt ttttttttcg tcgtttttcg agagtttagg gttcggtttt      2400
   gttggttttc ggtttttttc ggggttagta gtgattggaa acgagaagga attgagggga      2460
   aatttttgag tcggtttggt ttcgtcgcgt tcgcggtttc gcgcgttacg tcgggaagga     2520
   gttatcggga aagtatatcg gtttaatcgg ttgtagtttc gtgttttagt tagttcgtcg     2580
   ggtcgggagt ttttcgttta tattagcgtt cgtttgggtt tggggagggg aaagaggagg     2640
   ggataagttc gaggggagtt acgtgacgtt taaaggttta tcgtattgaa ggcgaggatc     2700
   gaataggtcg agggcgttta ttgggcgaat ttattttgat ttttgttatt cgtcgtttag     2760
   gcgtggattt ggggtggttg ttatagaaac gaggcgcgtt ttttggttgt agttaaaagg     2820
   ttgatcgtga ttaaagaggt taaaacgtgt tttttagcga ggcgaagtcg ttatgtatga     2880
   tgggatatta gaaggatttt ttcgatttag gaaggtgttt tcgtttttat cgggagatgg     2940
   ttttcgttta gtaatagttg ttaggatggg gacgtatttt ttggttttta ttttttttaa      3000
   ttttattcgt agaattttag agcgttagat tggaagtgat tttagggatt ttttaatttt     3060
   ttaatttttg taatattgat agggagatcg aggtttagag aggggtagag atttgtttaa      3120
   ggtttagaat ttagtatttt ttttattttt gtttatttat tttttttttt tttgagagaa     3180
   tttaggattt ttaatttcgt ttatcgaaaa tcgagtttta tttaagaatg tattagtttg     3240
   tgttttaagt acggggaaag ggcggtgggg gtggagagag ggataaggta taggtaagtg     3300
   aaggaaattt tttttgagtt aatgagatag attttttgcg gatataaaga gtttttatat      3360
   ttttttgttt tgtttttatt tttttttttt tgtatttatt attatttaat atagtatgtt      3420
   ttatttattg atatagttta ttatttgatt tttttattgg aatgtaaatt ttaaaagtag     3480
   ggtattttgt tttgtttatt tttatgtttt tagtgtttga ttataggttt aataaatatt     3540
   cgtagaagtg aacgaatggg gttgaggttt tggtgtttaa gagattatat tttaaggtgg     3600
   atttttaaga gtgtggttta tagagttagg ttcgggtttt aattttgttt gtttcggtta     3660
   tttagtattt aagtgatttt aaatattcgt ttttttgaat tttagggttt atatttgtaa      3720
   aatcggcggg gggtgggaat atttttttgg taggattgta gttaaggata agtgaaataa      3780
   gtatgaaaag tggttagtac gatgtttggg atttagtaaa tgtttagtaa atagaaacga     3840
   ttatttttga aatgtgaaaa tgaatcgttt agaacgatgg gagtttatag gattagattt      3900
   ttgtcgtttt tttttttttt tgtgtgggag gtgttagggt gggagggagg gaagagggaa 3960
   tggaagattg tgtaaggtgg gtttggttgg attagatatt cgagtttgga atagttaatt 4020
   gcgatttggg tataagggcg gtgaggggag tttatggttt tatttggggt ggaggagggg 4080
   gttaagggga agttatgttt cggaggaggt tgaatgggcg ggatagatta cggttttatt 4140
   tatggttttt tttttgttgg aagtgtgacg atttttttcg gatttttgta aaagcggggt 4200
   tatagtgaat tttggcgaag ggttcgcggt ggattaggaa ttggtgttag aattgtaaaa 4260
   gggtatttgc gttcggagtg gggaggtgat gcgggggcgg ggcggggttt tgggggcggg 4320
   gcgagtcgcg gaggtgtcgt tttggggcgg ggcggtgata gaaggatgga ttttgggggt 4380
   gaggattgat tttgggggcg gggcgagagt gcgaattgga gtttgaggtt gagcggtgac 4440
   gtaaggacga ttttgagggt ggagcgaggg gaggggattg gtttttggag gcggggcggt 4500
   ggtacgaggt tagtttttgg gggcggggcg gtggcgtaag attgattttg ggggcggggt 4560
   gggggcgtgg gttgaatttt gggggcgggg cggtggcgcg gaatggattt tggggtcggc 4620
   gcgaggcgtt gaggtgcgtt ttgagggcgg ggcgatgacg tagagcgaat tttgggggcg 4680
   gggcgagatt ttttagatgc gtttcgggga gtgggcggtg acgcggtggt aggttttggg 4740
   gataggcggt gattttagaa ttgattttgg ggatagggcg aggcgcggag gcgttttgaa 4800
   ggcgaggcgg tgacgtaaga gcgtcgggtt ttggggattg gggcgggcgg tcgaggttcg 4860
   gaagcggatt gaggcggttt ggagttttag ttggttattc gttatggacg aagcggatcg 4920
   gcggtttttg cggcggtgtc ggttgcggtt ggtggaagag ttgtaggtgg attagttttg 4980
   ggacgttttg ttgagtcgcg agttgtttag gttttatatg atcgaggata tttaggtgcg 5040
   ttttcggttt tttgttttcg cgttgtacgg tcgggttttt acgtttttcg aggaggcgtt 5100
   gtgcgggagt ttagtttttt ttgtggtttt aaatttttta gcggagttaa agaattttaa 5160
   atatttttta gtcgggtgtt cgttaaagcg attttatgtt atatttagtt agcgtatatt 5220
   gttagtgttt gtgttttttg tgtaaaggaa aaatgtgaga tatattttga gaattttgaa 5280
   taagaaataa tttatgaatt ggagaatatt agattgaaag agttttagtg ttgtaatgat 5340
   aaaacgttag aggtaagtat ttattgggaa aatgtggaag taaaataaag aaattatttg 5400
   attggtttgt agttataaaa ttgttttttt tttttaaggt ttgttaattg attgtttttg 5460
   gttggtttta agttttattt ttttttaata taagtattta ttagatatga tttaagttaa 5520
   attttgttta tgtttgtagt ttaagtaaga tttaggttat ttaggatatt tgattggttt 5580
   tgtttgttta ggaatttttt atggttggtt tttattttaa tttacgttaa tatttgcgag 5640
   gaaggaggcg tgtgcgtttt tattttttta gtatagaggt ttcgggaatt tattttggta 5700
   ttaggttgtt tggtagcgtg tttttgggtt agttatttat tttttttgag ttatagtttt 5760
   tgggttgtga aatgggaata agaataatat tgttataagg attaaatgaa ataacggatg 5820
   tgaaattgta gtgttaggta tagagcgggg gattagcgta ttttgttttt tttggttagt 5880
   tttttttgtg tttagtttat gttttgttaa gaggttttgg aaatgagggg atttgatgta 5940
   gaatggagaa taatgaagtt aaatatttga ggttttgttt aaaagtattt ttttattttt 6000
   gttttatttg atttttatta taggtttgtg aggatgtagg agatattttt tattatgagg 6060
   tgtagtaggt gtgagtggtt ggttggatat ttttggttag tgttttggtt tagggaaaga 6120
   gtttttaaat aattgagtat tatatgagta attatttatt aataataaaa taagttttgt 6180
   gttttttgtg tggtttttat tggggaatag taatggtttg aggttatagt ttttgttttt 6240
   aggaatgttt acggggatta taaatttaaa tgtttatgga ggattaatgg atgggcgtta 6300
   tagtggatag tttttgtatt gtttttcgtt tttagttgag tatggttata ttaataaatg 6360
   gtttagtatt gttatatttt atatttattt tgaggtaggg ttttattttt gtttagtttg 6420
   gagtgtaggg atgagattat ggtttattgc gattttgatt ttttgggttt aagtggtttt 6480
   tttgttttag tttttagagt agttgggatt ttaggtatat gttattatgt ttggttaatt 6540
   aaaaaaaatt ttttttttgt atagatagga gttttgttat gttgtttaga tgagttttaa 6600
   atttttggtt ttaagtgatt tttttgtttt agtgttggga ttataggtgt gagttattgt 6660
   attcggttta gtttttatat tttaaaaaag aagttgaaaa ttttgatttt tttgttaaat 6720
   tttttggttt aaaatgttag taataaattt agaaattttt taagaatttg gtgggaatta 6780
   taggttgtta tttgttattt agtatggttg aaaaataggt aggtttggaa ttagtggatt 6840
   tgggttttta ttttggtttg ttatatatta gtaagtgtaa aaaattataa ttaatgagtt 6900
   aaataaatag aaaaatttta tgatttagtt tttttgaaat tttttatttt aaaaagtggg 6960
   aagttttaat ttaattttat aaagttatga gggagaaaaa gatgaaataa tgtatttaat 7020
   ataggttgat tttttggagt gtttagtaaa tgtggagaga aatttaaata ttattattta 7080
   tttgtatgta gttaattgtt ttgttagtga gatttatttt tttagggtat tgtaggtttg 7140
   tgttgttttt aatagtagtt attagttata tgtcgttaaa ggtaaattga agtaattaaa 7200
   attaaataaa attaaaaatg gtagggttaa gttttatagg gtttgtgggt tttttttttc 7260
   gtgtgcggag atgagagatc gtagaaataa agatatagga gaaagagata gaaaaaaaga 7320
   tagttgggtt cgggggatta ttattattta gacgtggaga tagggtagtg gtttcgaatg 7380
   tttggttgcg ttgttattgg atataaggta aggggatagg gtaaggaggg tgaatttttt 7440
   aagtgatgat aaggtgaagt aagttacgtg attataggat aggggttttt tttttttagg 7500
   tagttgaagt agagagagaa ggtagtatat gttagcgttt ttttttatgt atttataaga 7560
   aagattaaag attttaagat ttttattatt ttttttatcg ttatttatga attttaaata 7620
   ggaattagga gtacgggagg aatatgaaag tgggtaagga gcgtgattat ggaagtatag 7680
   tattataggg aggggtttag gttttcggat gattgcgggt aggtatggat aatatttagt 7740
   tttttataag aagttggtgg agtagagtgt tttttgacgt ttttaaggaa aggagatttt 7800
   tttttttttt tttttttttt tgaggtggtg ttttattttt tcgtttaggt tggagtgtaa 7860
   tggtacgatt ttagtttatt gtaattttta tttttcgggt ttaagttatt tttttttttt 7920
   tttttttttt ttttgagacg gagtttcgtt ttgtcgttta ggttggagtg tagtgtcgta 7980
   atttcggttt attgtaagtt tcgtttttcg ggtttatgtt attttttttt tttagttttt 8040
   cgagtagttg ggtttatagg tattcgttat tacgttcggt taattttttg tatttttagt 8100
   agagatgggg ttttatcgtg ttagttagga tggtttcgat tttttgattt cgtgatttgt 8160
   ttattttggt tttttaaagt gttgggatta taggtttgag ttatcgcgtt tagttagggt 8220
   ttaagttgtt ttgttttagt tttttgagta gttggaatta taggtgcgta ttattacgtt 8280
   tagttaattt ttttgtattt ttagtagaga cgggttttat cgtgttggtt aggttggttt 8340
   tgaattttta attttgtgat ttgtttgttt tggtttttta aagtgttggg attataggcg 8400
   tgagttatcg cgtttggtag gagatttttt ttcgcggttt gttaagtaat gggtgttttt 8460
   ttagatattg gtattatcgt ttgattaagg agtttttaag cggtttttat gcgggtttga 8520
   tagagggttt attttttgtt ttttaggtta ttttttataa tgttttttta gtatttgatt 8580
   ttatattcgt cggttatttt taagttatat tagtaatgta ataaagagta atattaaaag 8640
   ttaatgatta ataatgttta taataatgat tgataattgt ttatgattat ttttatattt 8700
   aatttgtatt atgattattt ttattttaat tatttttttt attatattga aatagtttgt 8760
   ggttttagtt ttttgtttcg gtatttgggt aatttttcgt ttataaaaat ttagtttttt 8820
   agttgtatta tttaagtgtt taatggttat gtgatatatg gttattaaat aggataatat 8880
   agattaagaa tatttttatt attacggaag gtttttatag atagtgttgt tgtaggatag 8940
   ttgaggatac ggtgttattt tatttttgtg gattttgaga atttgtttag tgttgggtat 9000
   ttgaggatag gtggtagata attagatttt ttttttttag tgattgattt tattattttg 9060
   atatttttta attttttttt ttgagatagg gttttgtttt gttatttagg ttgaagtgta 9120
   gtggtgttat tatagtttat tgtagtttta ttttttgggt tcgagttatt tttttgggag 9180
   ttttagtttt ttgagtagtt gggattatag gtgcgtgata ttatatttgg tttttttttt 9240
   gttatttttt gtagagatag ggttttattg tgttgtttag gttggtttcg aattgggttt 9300
   aagttatttt tttattttag ttttttaaag tgttggaatt gtaagtatga gttattgtgt 9360
   ttggtttgtt tgttaatttt gatttttgtt ttgtttttat agttttttta gttagtgttt 9420
   atgttttatg gatttttttt ttttgtattt ttgttttgtt tttttatttt tttttatttt 9480
   agttgtatta tttttgttgt tttttgattt ggttatggtt tatttttttt tattttttat 9540
   tttgattttt ggatgatttt ttgaaaggtt atatttgttt ttgttatttt atttttaaaa 9600
   ttttttggtg gtcgggtatg gtagtttacg tttgtaattt tagtattttg agaggttgag 9660
   gttggtggat cgtttgaggt taggagtttt gagattagtt tggttaatat ggtgaaattt 9720
   tgttttaatt aaaaaatata aatattagtt aggtatggtg gtgggtgttt gtagttttag 9780
   ttatttggga ggttgatgtg ggagaattat ttgtatttaa aaggtggagg ttgtagtgag 9840
   ttgagatggt gttattgtat tttagtttgg gtgatagagt aagatttttt aaaaaataaa 9900
   taaaaaaata atcgatggtt ggttacggtg gtttatgttt ataattttag tattttggga 9960
   ggttaaggtt ggaggattat ttgaggttag tttaagatta gtttgggtaa tatggtgaaa 10020
   ttttgttttt attaaaaata aaaaaaaaat tagttaggta tggtggtgtg tgtttgtaat 10080
   tttaattatt tcggaggttg aggtaggata atcgtttgaa ttttggagtt ggaggttgta 10140
   gtgagttgag attgtattag tatattttag tttgggtgat agagtgagat ttcgttttaa 10200
   gaaaaaaaaa aaaaaataaa aataaaatta aaaaattttt tggtggtatt tgataggttt 10260
   taggatgaat tttttggttt ggttatataa aggttttgtt ttttgttatt atttattttt 10320
   tattttataa gattttgaat tcgttattta ttttatgata tttttagttt tatgtgtttt 10380
   tttttgggta tttgtttttt gttgtgttat tgttgatttg tttgattgta ttttttatta 10440
   gaaatttgtt ttttaaggat tgagattgtt tatggtagta tttttagtat ttagtatagt 10500
   gttgggtatt ttttgttttg taaaataatg aatagattag taaaggttgg taaatggtat 10560
   atagtgtatt tgtagtttta ttgttgttta cgtgtttgat tgtttagatt tatagtagtt 10620
   gggaaatggg gagataaggt gagagttata taaaagtacg tggataatta gtattttgtt 10680
   ttttttttta aatagt 10696
<210> 40
   <211> 10696
   <212> DNA
   <213> Artificial sequence
<220>
   <223> chemically treated genomic DNA (Homo sapiens)
<400> 40
   gttgtttgga aagaaaggta ggatattaat tatttacgta tttttatatg gtttttattt      60
   tgttttttta ttttttagtt attgtaagtt taaataatta ggtacgtggg tagtaatgga      120
   gttgtaggtg tattgtgtgt tatttattag tttttgttga tttgtttatt attttgtagg     180
   gtaaaagatg tttagtatta tgttaagtgt tgaaaatgtt gttatggata gttttagttt     240
   ttaaggagta aatttttaat ggggaatgta attagataaa ttagtaatga tataataaga     300
   gataagtgtt tagagggaag tatatggggt tgaggatatt atgaagtgaa tagcgaattt     360
   agagttttat gaggtgggag gtagatagtg gtaagggata aggtttttgt gtggttaggt     420
   taagaaattt attttgagat ttgttaggtg ttattaaaga gttttttggt tttgtttttg     480
   tttttttttt ttttttttga gacggagttt tattttgtta tttaggttag agtgtattgg      540
   tgtaatttta gtttattgta atttttaatt ttaaggttta agcgattatt ttgttttagt     600
   tttcggagta gttggggtta taggtatata ttattatatt tgattaattt tttttttatt      660
   tttagtagaa atagggtttt attatgttgt ttaggttggt tttgaattga ttttaagtga     720
   ttttttagtt ttggtttttt aaagtgttgg gattataggt atgagttatc gtggttagtt     780
   atcggttgtt tttttgtttg ttttttgagg agttttgttt tgttatttag gttggaatgt    840
   agtggtatta ttttagttta ttgtaatttt tattttttgg gtataaatga tttttttata   900
   ttagtttttt aggtagttgg gattataggt atttattatt atgtttggtt aatgtttgta    960
   ttttttggtt aagatagggt tttattatgt tggttaggtt ggttttaaaa tttttgattt  1020
   taagcgattt attaatttta gttttttaaa gtgttaggat tataggcgtg agttattatg  1080
   ttcggttatt aaagagtttt aagaatggag tgatagagat agatatggtt ttttaaaaag  1140
   ttatttaggg gttagggtga aaaatggaaa ggagtgagtt atagttagat taggaggtag  1200
   taaagatggt gtagttagaa tagagagaag tgaaaggata aagtagagat ataaagagag  1260
   agggtttata ggatatgggt attgattggg gggattgtga agatagagta agggttaagg  1320
   ttgataggta ggttaggtat agtggtttat gtttgtaatt ttagtatttt gggaggttga  1380
   agtgggagga tagtttgagt ttagttcgag attagtttgg ataatatagt gagattttgt  1440
   ttttataaaa aataataaaa aaaaagttag gtatggtgtt acgtatttgt agttttagtt  1500
   atttaggagg ttgaggtttt taagaggatg gttcgagttt aggagataag gttgtagtga  1560
   gttgtgatag tattattgta ttttagtttg ggtgatagag taagattttg ttttaaaaaa  1620
   aaaaattgag aggtgttagg atggtggggt tagttattaa gaaggaagag tttggttatt  1680
   tgttatttgt ttttagatat ttagtattgg ataggttttt aggatttata gggataagat  1740
   ggtatcgtgt ttttaattat tttatagtag tattgtttat gagaattttt cgtgatgatg  1800
   gaaatatttt tggtttgtat tgttttattt agtaattata tattatatga ttattaagta  1860
   tttgggtagt ataattgagg aattgaattt ttgtgggcga aagattattt aggtgtcgag  1920
   gtaagagatt gaagttataa attgttttag tataataaag aaaatagtta gaataagaat  1980
   agttataata taaattaggt atagagatga ttatggataa ttattaatta ttattataaa  2040
   tattattaat tattagtttt taatattatt ttttgttgta ttgttaatat aatttaggaa  2100
   taatcggcgg gtatagggtt aggtgttgaa gggatattgt gagaagtgat ttagaaggta  2160
   agaggtgagt tttttgttag gttcgtataa gggtcgtttg agggtttttt ggttaagcgg  2220
   taatgttagt gtttggggag atatttatta tttagtagat cgcgaagggg agttttttgt  2280
   taggcgcggt ggtttacgtt tgtaatttta gtattttggg aggttaaggt aggtagatta  2340
   taaggttagg agtttaagat tagtttgatt aatacggtga aattcgtttt tattaaaaat  2400
   ataaaaaaat tagttgggcg tggtagtgcg tatttgtaat tttagttatt taggaggttg  2460
   aggtagaata gtttgaattt tggttgggcg cggtggttta agtttgtaat tttagtattt  2520
   tgggaggtta aggtgggtag attacgaggt taggagatcg agattatttt ggttaatacg  2580
   gtgaaatttt atttttatta aaaatataaa aaattagtcg ggcgtggtgg cgggtgtttg  2640
   tagatttagt tattcgggag gttgagggag gagaatggta tgaattcggg aggcggagtt  2700
   tgtagtgagt cgagattgcg gtattgtatt ttagtttggg cgatagagcg agatttcgtt  2760
   ttaaaaaaaa aaaaaaaaaa aaggaaaata gtttgaattc ggaaggtgga ggttgtagtg  2820
   agttgagatc gtattattgt attttagttt gggcgaaaaa gtgggatatt attttaaaaa  2880
   aaaaaaaaaa agaaagggag tttttttttt ttggaggcgt tagggagtat tttgttttat  2940
   tagttttttg tgggaggttg gatattattt atgtttgttc gtagttattc ggagatttaa  3000
   attttttttt gtggtgttgt gtttttatgg ttacgttttt tgtttatttt tatgtttttt  3060
   tcgtattttt ggtttttgtt tgaagtttat agatagcggt agaagaaata gtgaaagttt  3120
   taaagttttt gatttttttt ataagtgtat agaagaaaac gttgatatat gttgtttttt  3180
   ttttttgttt tagttattta aaagggaagg gtttttgttt tatgattacg tgatttgttt  3240
   tattttatta ttatttagaa gatttatttt ttttattttg tttttttgtt ttatatttaa  3300
   taatagcgta gttaggtatt cggggttatt attttgtttt tacgtttagg tggtagtggt  3360
   ttttcgggtt tagttgtttt tttttttatt tttttttttt gtgtttttat ttttacgatt  3420
   ttttattttc gtatacgagg agaaaaattt ataggttttg taaggtttga ttttattatt  3480
   tttagtttta tttaatttta attattttaa tttattttta acgatatgtg gttaatggtt  3540
   attgttggga atagtataga tttatagtgt tttgagaagg taagttttat tagtagaata  3600
   gttggttata tgtaaataga tggtggtgtt taggtttttt tttatatttg ttgaatattt  3660
   taggaggtta atttgtgtta aatgtattgt tttatttttt ttttttttat aattttatag  3720
   agttaaatta gaatttttta ttttttaggg taaaaaattt tagggaaatt gagttatagg  3780
   gtttttttgt ttgtttggtt tattggttgt ggttttttat atttgttaat atgtaatagg  3840
   ttaggatgag agtttagatt tattggtttt aaatttgttt gttttttagt tatattgaat  3900
   ggtaaatggt aatttgtggt ttttattagg tttttaaaaa atttttgaat ttgttgttaa  3960
   tattttaagt tagggaattt gatagaggaa ttaagatttt tagttttttt tttaagatgt  4020
   gaaagttggg tcgggtatag tggtttatat ttgtaatttt aatattgagg taggagaatt  4080
   atttgaggtt aagagtttga gatttattta ggtaatatag taagattttt atttgtataa  4140
   aaaaaaaatt ttttttaatt agttaggtat ggtggtatgt gtttaaagtt ttagttgttt  4200
   taaaggttga ggtaggaaga ttatttaagt ttaggaggtt aaggtcgtag tgagttatga  4260
   ttttattttt gtattttagg ttgggtaaga gtgagatttt gttttaaaat aagtgtaaga  4320
   tgtggtaata ttgggttatt tgttgatatg gttatattta gttggagacg gagggtagtg  4380
   taaaggttat ttattatgac gtttatttat tggtttttta tgggtatttg agtttgtgat  4440
   tttcgtgaat atttttgaag gtaaggattg tggttttaaa ttattattgt tttttagtag  4500
   gggttatata gaaggtataa aatttatttt attattaatg aataattatt tatatgatat  4560
   ttaattattt aaagattttt tttttgggtt agggtattgg ttagggatat ttagttagtt  4620
   atttatattt attgtatttt atggtaggaa atattttttg tatttttata ggtttgtgat  4680
   gagggttaaa tgagataaag atgggaaagt gtttttaaat aaaattttaa atgtttgatt  4740
   ttattatttt ttattttgta ttaggttttt ttatttttaa aattttttaa taaaatataa  4800
   attgaatata gaaggggttg gttaaagagg gtagggtacg ttgatttttc gttttgtgtt  4860
   tagtattata gttttatatt cgttatttta tttagttttt atgatagtat tatttttatt  4920
   tttattttat agtttagaaa ttgtggttta aagagggtaa gtgattggtt taagaatacg    4980
   ttgttaggta gtttgatgtt aaagtgggtt ttcgaaattt ttatgttaga aaagtaaaaa    5040
   cgtatacgtt ttttttttcg taagtattaa cgtaaattaa aatggagatt aattatgaag    5100
   aatttttgag tagataaaat taattaggta ttttaagtga tttaaatttt gtttaaattg    5160
   taaatataag taaaatttaa tttgggttat atttgataaa tgtttatgtt agagagaaat    5220
   aaaatttaag attagttaaa agtagttaat taatagattt taaaaaaaaa aggtaatttt    5280
   gtaattgtaa attaattaaa taattttttt attttgtttt tatatttttt taataaatat    5340
   ttgtttttga cgttttgtta ttgtaatatt aaagtttttt tagtttgatg ttttttaatt   5400
   tatgaattgt tttttattta aagtttttaa gatgtgtttt atattttttt tttatataag    5460
   aaatataagt attaatagtg tacgttgatt gagtatggta tggaatcgtt ttagcgaata    5520
   ttcgattaag aggtgtttgg gattttttgg tttcgttgag gggtttgaag ttatagggaa   5580
   ggttaggttt tcgtataacg ttttttcgag gggcgtgggg attcggtcgt gtagcgcggg     5640
   gatagggggt cgggggcgta tttggatgtt ttcgattata tggggtttga atagttcgcg     5700
   gtttagtagg gcgttttaga gttggtttat ttgtagtttt tttattagtc gtagtcggta    5760
   tcgtcgtagg agtcgtcgat tcgtttcgtt tatggcgagt agttaattaa gattttaggt    5820
   cgttttagtt cgttttcggg tttcggtcgt tcgttttagt ttttaggatt cggcgttttt    5880
   gcgttatcgt ttcgttttta ggacgttttc gcgtttcgtt ttgtttttag ggttaatttt    5940
   ggggttatcg tttgttttta gaatttgtta tcgcgttatc gtttattttt cgggacgtat    6000
   ttagaaggtt tcgtttcgtt tttaggattc gttttgcgtt atcgtttcgt ttttaggacg    6060
   tattttagcg tttcgcgtcg attttagaat ttatttcgcg ttatcgtttc gtttttagga    6120
   tttaatttac gtttttattt cgtttttaag gttaattttg cgttatcgtt tcgtttttaa    6180
   gggttagttt cgtgttatcg tttcgttttt aagagttaat tttttttttt cgttttattt     6240
   ttagaatcgt ttttgcgtta tcgtttagtt ttagatttta gttcgtattt tcgtttcgtt    6300
   tttaaagtta atttttattt ttaggattta tttttttgtt atcgtttcgt tttaaggcgg   6360
   tattttcgcg attcgtttcg tttttagggt ttcgtttcgt tttcgtatta tttttttatt    6420
   tcgggcgtag gtgttttttt gtaattttgg tattagtttt tggtttatcg cgggtttttc     6480
   gttagaattt attatagttt cgtttttata gagattcgga gggagtcgtt atatttttag    6540
   taagagggaa attatgagtg gagtcgtgat ttgtttcgtt tatttaattt ttttcggggt    6600
   atggtttttt tttagttttt ttttttattt taagtaaaat tataagtttt ttttatcgtt     6660
   tttgtgttta gatcgtagtt gattgtttta aattcgagtg tttgatttaa ttaagtttat    6720
   tttatatagt tttttatttt tttttttttt ttttttattt taatattttt tatatagaaa      6780
   aaaaaaaaaa cggtagaaat ttagttttgt aaatttttat cgttttgaac ggtttatttt    6840
   tatattttaa aaataatcgt ttttgtttat tgagtatttg ttaagtttta ggtatcgtgt    6900
   tagttatttt ttatgtttat tttatttatt tttaattata attttgttag gaaagtattt    6960
   ttatttttcg tcggttttat agatgtagat tttagggttt agggaaacga atgtttaagg    7020
   ttatttgggt attaagtggt cgaggtaggt aggattgaaa ttcgaatttg attttgtaag    7080
   ttatattttt aagaatttat tttagaatat aattttttga gtattaaaat tttagtttta     7140
   ttcgtttatt tttacgaata tttattgagt ttgtagttaa atattaagaa tatgaaggtg    7200
   aataaaataa aatgttttgt ttttagagtt tatattttag tggaaggatt aggtgataaa    7260
   ttatattaat aaataagata tattatgtta gatagtgata aatgtaaaga gaaaaaaata    7320
   aaaataagat agggagatat gaaagttttt tatattcgta agggatttat tttattggtt    7380
   taaagaaagt tttttttatt tatttgtgtt ttattttttt ttttattttt atcgtttttt    7440
   tttcgtgttt aaggtataga ttggtatatt tttgggtgga gttcggtttt cggtaaacgg    7500
   gattaaaagt tttggatttt tttaaaaaaa aaaaagataa gtaaataaaa ataaaaggaa    7560
   tgttggattt taggttttgg gtaaattttt gttttttttt ggatttcggt ttttttatta  7620
   atattgtagg agttggagag ttaaagaatt tttaaggtta tttttaattt gacgttttaa    7680
   ggttttacga atagggttaa agagaatagg gattagagag tgcgttttta ttttagtaat    7740
   tgttgttaga cgagggttat ttttcggtag gagcggaaat atttttttgg atcgaggagg    7800
   tttttttggt attttattat gtatagcgat ttcgtttcgt taggaggtac gttttggttt     7860
   ttttggttac gattagtttt ttggttgtaa ttaaagagcg cgtttcgttt ttatagtaat    7920
   tattttaggt ttacgtttag acggcgagtg gtaagggtta aaatagattc gtttagtaga    7980
   cgttttcgat ttgttcggtt ttcgttttta gtgcgataga tttttaagcg ttacgtgatt    8040
   tttttcgggt ttgttttttt tttttttttt tttttaggtt taggcgagcg ttggtgtgga      8100
   cgggaagttt tcggttcggc gaattaattg gagtacggag ttgtagtcgg ttgggtcggt    8160
   gtattttttc ggtaattttt tttcggcgtg acgcgcggaa tcgcggacgc ggcggggtta   8220
   ggtcgattta gggatttttt tttagttttt tttcgttttt agttattgtt ggtttcgggg    8280
   ggagtcggga gttaataggg tcgggttttg ggttttcgga gggcggcggg ggagggggtt    8340
   gttttttgta gggttgcgat tttcggtagg agcgcgaggc ggggtttaga tttggagttt     8400
   gtttttggga gttttttttt ggttttggat atgttgttta gagagttggt agtttatttg     8460
   ttgggtaggt gtttgttgtt tcgaggtcgg cgtcgtttat ttcgtatgtt agttaaagaa    8520
   ttttttagat tatcgttatt tcggtttttt agttggaagg aattgatatg gttaaataag      8580
   gtagcgatcg tattgtatgg tagaaataat agtattaacg attatttgtt gattgttgat    8640
   tttgggttta gtaaattttt tatatgtatt tagtttttat atgatgattt tttaaggtgg   8700
   tagtatttat ttttatttta tgaataagga cgttgatgtt tagtaagttt aaataatttg    8760
   tttaggtaat aaagtagtgg agaatagatt ttagttttta atttatatgt ttaattagta    8820
   gttatattgt ttttacgtgt tttttaggat ataataggtt gttttaataa agaagaaatt    8880
   ggtttgtttt aggattttat ttagtacgta tgtaatttta aacgttggtt attagattag    8940
   ttaattttgt gaggattttt aggcggtatt aaatttgtat ttacggaaat ttattattgt    9000
   tatagttttg ttttttttta gtaaggtgtt atagaagata tttgaatttt aaaattgtat    9060
   taatttgtga gaaattaaga taaatgtgtt ttttgaaaaa aaatattgta gggtataata     9120
   gtaattataa ttgtggagta taaatatttg tagttttagt attattttgt ttattaatta     9180
   gttaggatta tttttaaaat ttataatgaa ttaagttatt gtatttatta gagatggagt     9240
   ttgaaatttt taaagattac gtttgttatt tggtttttgt cgttaatttt tttttatatg     9300
   taaaattaaa tttttttttg tcgttttttt ttatttgagg tttttattaa taatgattat     9360
   attggtcggc gcgatggttt acgtttgtaa ttttaatatt ttgggaggtc gaggcgggtg     9420
   gattatttga ggttaggagt ttaaaattag tttggttaat atggtgaaat tttgttttta     9480
   ttaaaaatat aaaaaattag ttaggtaggg tggcgtatgt ttgtaatttt agttattggg     9540
   gaggttgaga taggagattt gtttgaattt gggaggggga ggttgtagtg agttgagatt     9600
   ttattattgt attttagttt gggtaacgag tgaaatttcg ttttaaaaaa aaaaaataaa     9660
   aaaatattat attattaatg attaaagagt agttttttat ttttttgttt gaatggtgtg     9720
   gtgagaaaga atagaattga ggtatattgc gttttaatta tatagttttt tgagagtgtt     9780
   ttacgtttat gtttgtattt atttaatgtt taagaatgtt ataattattt tattttgtag     9840
   ttttgtatat ggtaaatttt atagttttat gagtttgttt aatatgtatt taatgaagta     9900
   tttttaaatt atttatttta tttattttta ttagaataaa tgaaattatg tatttgagag     9960
   gtagtcgttt tgattatttt atttaggttt ttttttattt atgtattatt tttgtatttt    10020
   atttattttt atttattttt aaagtttttt tagttaaagt gttttggaga gttgttattt    10080
   ttattattta tttttttatt tgtaatagtt tttatttgtg atatttaaaa gtagtagcga    10140
   gatttttatt ttaagatata gtttaatagt ttgatatttt ttttgtaaaa tattttttgt    10200
   tggttttgtt ttttatttat atattttata tttttattta aaaagtaaga ttgtttaggt    10260
   ttttgttatt gaaatttatt gtttttttaa ttattttagt tttggaaagg aagagaaatg    10320
   gaagtgagaa agttgagtat tttttggtag tttttgatag ttttggtttt gattttgtaa    10380
   attttgtttg cgttggattt tgatttatat agagttttag gggttagtcg aatagttagt    10440
   taggttgata ttaaaaaggt ttataagaag ttcgttcggg aatggtaggt gaaataaaga    10500
   aatagaggtt taatataagt taaatagttt tagtagggat gaagtttaaa ttatgattag    10560
   tttttatttg tttttgtttt tatgtttaat atatatattt gtttaaaata ttttacgtag    10620
   gagagttaat gtttatgttt tataattgtt agcgataaga tttaaagttt atgtttattt    10680
   tgaaattttt ttttag                                                  10696
<210> 41
   <211> 17959
   <212> DNA
   <213> Artificial Sequence
<220>
   <223> chemically treated genomic DNA (Homo sapiens)
<400> 41
   ttagtgggtg atgtttgata tttattttta gtatttaggg ttggaggggt tggatatttt      60
   tatgtatgta tgtatatacg tgtgtatata tatatgtata tataaagtat ttataagata     120
   aagtaaatgt tagaaaagat tattggttat gtgttattta tatgttatat atatttttta    180
   ttttttgttt agttattatt ggttattttt tatttaggta gtagttgtga gaaattaaat     240
   gtatttatgt tttagaatta tggggagaaa ataattgttg aatgtttttt ttgtgtttat     300
   tatggtttta ggtgttggga atatttagtg ggtaagatgg attaggtttt tttttttatg     360
   gagttgatat tttagttagt ggagaaaaat aataagttag tagatagata aatgaggaat     420
   tttagatgag gatagagtgt tataaagata gtaagataga gtaggttttt tgaaaagttg     480
   attttagaat aagaagaagt taattacgcg aggatttgga agagatagtt ttcggtagtg     540
   gtgttgtagg atttttttgt tagtttagtt aaaaagagcg tttttgttat ataattacga     600
   aagattaggt ttatagatat tttgaagggt ggggaaaatg gaatttatta ggtaaaaagg     660
   aaaaaaagga aatagggatt tttagtaaag taagagtttt gttagtcggt ttttcgcgtt     720
   atagattgaa ttttagattt tattcgggaa gaggttaggt tttttttttt tgtaaagggc     780
   gtgaattttt gtggttttat tttatttttc ggtgtgtagg tagttagagt ttttttgcgg     840
   atttttttat gtttggttgt tttagtggga gtggttaata taaagtttta aggggaagaa     900
   ttggtttgtt ttgttagagg aattttagaa aggttagtgt ggttagagga gttaagaggg     960
   aggagtttat aattgggtag gatttttatt atgtagggtt tggtaggtta tgtgtgttag    1020
   gggttgtatt tggttgttta tgaagatatt aattataagg attttattta atagaaattt    1080
   gttttttata taataaaaaa gtttggacga ggagtttata gttttgaagc gtttttatgg    1140
   atttttagat ttttatatgt ttttgatttt tttgttttgt tatttttagt ttgtgatttt    1200
   tatgtttttg attgttttat agttgcgata tgattgtttt atttttggtt ttttgtttga    1260
   atttaaggtg gtataaatga tgttattagg aggaaggggt gtttttatat taagaatgta    1320
   aatttttttt aaaatatttt agtagatttt tatggttaaa attgtgttat ttggttattt    1380
   ttagttataa gggaagttga aaaataaatg tttttttttt gcgaagttta ttattttttt    1440
   aatagtattt tttttattag aaatgttatt gagtagatag attattttat attgtaatga    1500
   gtagtttgga ttgtatttta gaggagaagt tatttgtcga taggtgggaa attaagagtt    1560
   ttttgaagat gtaaaattaa tttggttgtt atattgttag gagtagaatg tatttgaatt    1620
   acgtagtatt taaatataat acgttagtgg tagtgaattt gtataggttt gtagtaattt    1680
   taatttttgt ttttttagaa ggaagaattt aattgagggg tataaagtag aaagagagat     1740
   tgaggtaatt ttaaaggagg agtgaaagtt tattaaaaag ttttagattt ggaataaaag    1800
   gaaagtatat ttggaagatg gttaagtagg taattttaga gattaagtgt acggtttgat    1860
   ttttgattta gggttttata tattggtata tttttggagt tttgtatttt ttgatttttt    1920
   agggattagt tttatttttt gatttttttt ttggggtagg ttgtttatat gtatagtgat    1980
   ttgttagcgt ttggaaagtg agtaggggta gtgtgtttat tggaagtgta tatatgttta    2040
   tttgaggtaa ttttttttta ttagttttat gtttttagaa ggttatatat tagttaaacg    2100
   ttgttatttt gtttttcgat gtgtggtaaa gagattttat taggaagttg ttgattatta    2160
   tttttaggtt ttttttattt attgggagat tgtttttttt tggtattgat tgtaattaat    2220
   tattatttta gagaaatagt gtaataattt tttgattatt atttgatggt tatataatat    2280
   ttttggtggt gtgggttgtg ggggaagagt ttttttttgt tttgtttagg tcgattagtt    2340
   atttattgta agataattag agttttttaa ttagtataaa tttggagaat aggttatagg    2400
   taaagagata taatttgtta gattttaggg tagtttaggg gttattaggg tttttttgga    2460
   gttttgtttt tttttgtgaa ggtttttttt tttatttttt agggtttttt attaatagta    2520
   tttgttattg agtttaagat gttgtggttc gtaatatgta attttgtttt aggtattatt    2580
   aagggtaaaa atattattaa ttaaatgata ttataatgtg tttttttaat ttaaaatttt    2640
   tattttatat ttttaagaaa taatttttta ggtttaagat atacgatata agtttttaat    2700
   tatatagtat ttttgtatat atataaaaag gaagttattt atgaaattaa ttagttaggt    2760
   atttttaaat ttttaagaac gtgagtgatg tgttattttt ttaaagagta atttagtatt    2820
   aattttagga ttttttttta aattgttggt atttatttgg aagtttggat gtgaatgtaa    2880
   gataatggta agtatattat gattgtagtt tggttaataa ttattttatt atcggtgttg    2940
   aaatgtgtgt atggagtggg gaatgtaaga ttaatgtatg ttatgtgtat ttttatgtgt    3000
   gttttgatgt atagagttgc gtagtattga gttagataat aaatagagaa gttaaggtgg    3060
   cggtggtgag tttggagttt agtggagggg tagaaagaga gaaattaagt tgtagatgtg    3120
   gtatttataa gcgtttgtag gtggtggtgg tggtgagttt ggagtttagg ggaggagtag    3180
   aaatagagaa atgaggttgt agatttggta tttgtaagta tttgtatgat agtcgtttat    3240
   aggttgtatg tgattattta tgggaagttt agatagagaa gaaggttaat gttgagtttt    3300
   gcgatattta aatgtttata aatttagaag agggcgaatt gttaagttga tagagtgagt    3360
   agttagggtg gttagagaat tggagggtaa ggtagtgatg tggtattttc ggagtaaagg    3420
   aggaggggga cggttagttg tattaagtgt tgttaagagt ttaaatagaa tgaaagtaga    3480
   aaatgattat tggatttggt agtatatagg ttataagtga tttttataaa aggatttttt    3540
   atggttgagg gtagtgtagt agaatttgat gaaaattaga taaattggat tggagggagt    3600
   gataagagat gaggagttta gtttttttga atagttttgt tgttaaaggg aaaagtatag    3660
   ttaagcgagg ggtggtgttt ttttcgttaa agagattagg tttgtttgaa tgcgaattta    3720
   gtaaggtatt gcggaagtta atagtagttg taaaatattg gggtaggcgg gaagaatacg    3780
   atttaaggta cgagagttag gggttagtta cggtaatagg aagatgatgg tgaaaattaa    3840
   aagtttttga gagtggtttt aatttaggaa gtttattttg ttaaggttaa ggacgtgtat    3900
   tgatataatt ttaggaggtt ttgatgatat gtgtttaggg tggttaggaa atattttggt    3960
   tttatatatt tgagggagat atgagatatt aattaatacg taagatgtag gttggttttt    4020
   taggaggtaa aatgtgaagt ggaggcgttc gggttatagg tatatttaaa ggttttttga    4080
   ttggtaattg gttgagagtt attatttaaa gatttggatt tagtagaagg gaatgttcgg    4140
   gttgtatgaa ggagttgtgg agattaaggt gttattatgt agatgaagtc gggaatatat    4200
   tgtaaatgtt ttttattaga gttttttatt agttttaggg ttttcgtgcg gatgtttgtg    4260
   aggttatgtt tgattttcgt tttttattaa ggtttgaatt agttttttaa gataatttag    4320
   aaggttatag gcggagggga gggtttattc gttggttgta gggtttagaa ttttattttt    4380
   ggtttataac gaggagggta gggatagaag ttatcgtttt tgttttttag cgttggtttt    4440
   tattcggaaa gtaggttttt ttttcgtatc gttttttaat aaatttttgt tttttcggtt    4500
   ttcgtcgttg ttatttgggt tttttttttc gcgttttatt aggcgttgtc gcggtagggg    4560
   ttgagggttt taggggacgg tcgttttttt gtcgggacgt ttcggttttg gttcgtattg    4620
   tggggcgtag ttatttagtc gtcgtcgcgt agttaggagt tcggttgagc gcgttcgttg    4680
   taggagttat ttttagttgg gttttattta atttttcgta attttagttt tcgtagttag    4740
   tcggggattt cgtttttttt tttttttttt ttttttttat ttttttattt tttttttttt    4800
   tttttttttt tcgttttcgt tttcgttttc gttttcgttt tcggttatag tattcgacgt    4860
   ggcgcggtcg gttgcgtacg tatcgtatgt ttggtttcgc ggtttagtag ggcgggcggg    4920
   tcgcggtgac gttagtgcgt atgtcgtcgg tgggttcggt tgcgttacgt cggcgcgcgg    4980
   cggtcgcgtt tttttaacgg tagtggtatt tattcggttc gcgcggtttc ggcgttttac    5040
   gcgcgcgcgt cgttttttcg ttcgttcgtt tttcggcgtt tatatttgag tttattcgcg    5100
   tacgttcgtt cggttcgaga atcgcgtcgt cgtttcggtt tcgcggaagt ttcgtcgcgt    5160
   tatgttttcg tttttcgaag ggaaattaga gattaaagtt ggatattcgt tcgtcggtat    5220
   tgattttttt tttttttttt tacgcgcggg tgtcgggggc gggttcgaat tcgggggcgt    5280
   cgggcggggg tttcgcgggg agggtttcgg cgagcgttga gacgggtttc gggttttgat    5340
   gttcgttcga ggggcgcggg tcgcggagag gtcgggcgtt taggttcggg aggagttcgt    5400
   cggagtttcg gtttcgtttg tcggcggcgg cgaagcgttt ttttttgagg atcgcgggcg    5460
   gtcgcgtatt tgtgcgattg cgcgtaggtt tcggtgtttt gggcgaattt agttgtataa    5520
   tttataataa agttttggcg gataatattt atttagagga tatagatgtt tagaaagaaa    5580
   gtagtagaag taatgaaaag ttagtggtgt taatattatg gtgatgatta attagaggtt    5640
   gttagcgttt tatatcgatt taagaattaa ggagcggaaa taatttttat tattaattat    5700
   ttaatatgat ttttatcgtt agtttttttt atatttagat tttttttttt ttttaaagtt    5760
   gttttttttg attttgagta aattttaaat ttttgagtta aggaagttaa agttaggaaa    5820
   gagcggtttt ttttagaata tagggattta agtagttttt atgtttttgt tatttttttt    5880
   tttttttggg tatagttttt gtttgtattt tggattttgt ttagacgggt ttgttttcgt    5940
   ttttattttt ttttttagtg gtagtcggtt tatatggagg gatattggtg agaagattaa 6000
   aggttttggg ttttagaagt taagttattt taatgtatgg atgcgtgagg tttttttagg 6060
   ttttaatagt acgttttagt gttataaatt tagttttgta ggaatagaag tttttgagaa 6120
   atatggtttt ttttggtggt atatttgcgt tatttttttg ataataaaga tttttggtta 6180
   ggcgcggtgg tttatgtttg taattttagt agtttgggag gttaaggagg gtggattatt 6240
   tgaggttagg agtttaagat tagtttggat aatatggtga aattttattt ttattaaaag 6300
   tagaaaaatt agttgggtat ggtggcgggc gtttgtaatt ttagttattt aggaggtcga 6360
   ggtaggagaa tggtttgaat tcgggaggta gaagttgtag tgagttaaga ttgagttaaa 6420
   gtattttagt tttggcgata aagcgagcga gattttattt taaaaaaaaa aaaaaaaaaa 6480
   aaaaagattt ttattatttt tttttgttgt ttttcgttat agggcgggaa ttttttgtat 6540
   tagtataatg aataggagtc gttttttaaa tatgttgtag tttagagtag gtaatatttt 6600
   ttagtggtga cggaggtttt ttataataaa attaattggt attgggaaag aaaaatgttt 6660
   aattgatgtt agagaattta agaggtatgg ggttttagaa aattatttta ttgtgatgga 6720
   aaatggtttt gggggaattt gtgttgtatt ggttttaaga aaaaaagtat tttaaaggga 6780
   gttttttttt tgatgggatt ttttttaagg ataattgatt tttattttat tatagcgggt 6840
   ttttgtttat tgtggggagg agcgttttga tttttatttc gggtagggtt gtttttagga 6900
   attgtagtag ttatttgttt tggtttttta ttttttagga tatgggttat attaaagcgt 6960
   atttgttgga atgatataga attaaagtat agtaattaaa ttgtatatag agatatattt 7020
   ataaagaagt tttttgtttt gttttttgaa atagagtttt attttgttat ttgggagtgt 7080
   agtggtacga ttttggttta ttgtaatttt cgttttttag gtttaagaga tttttttgtt 7140
   ttagtttttc gagtagttgg gtttataggt gtttgttatt atatttggtt gatttttgta 7200
   tttttagtag agatcggatt ttgttatgtt ggttaggttg gttttgaatt tttgatttta 7260
   ggtgatttat ttattttagt tttttaaagt gtttatatgg agatttttaa ggttagaggt 7320
   ataaattgta tatatttatt ttttgggaga aattttttgt tggattttgg ttttttttga 7380
   attgtagagt aaatagttag gttttgtgaa ttatagagta agtagtttat ttatttttga 7440
   ggagggaaga ggtagtttat ttgtgttagt gtagtagtga gtttgggttt taagtttgaa 7500
   gatattattt tacgttttat tggggtttag tttttttttt ttatgaaatt cgaggtttgg 7560
   gtttcggggt aagttatagg gtggggttgt gtttgttgta gtgagtttta cgtttcgttt 7620
   tagtattagg gtattagtat tgtttatttt ttaggtaaat ttttttagta attttagggt 7680
   aattttttta gatttacggg gtgagttttt gcgggtggtt gttattattt gttatttgtt 7740
   tttaagatta tagttagagg aggggtttaa tagtagtatt ttttaaatgt tagtagtatt 7800
   ttttaaatgt taatagtatt ttttaaatgt taatagtatt ttttaaatgt taatagtatt 7860
   ttttagatgt taatagtatt ttttaaatgc gtttttttag atgttataga ggattatgtg 7920
   tgaatttttt ttggtaggaa gttttggtgt ttttgtttag tttggtattg ttagagagta 7980
   ggtagttttg aagggtagat agtttttaaa aaaaaaaaaa aaaggcggtt tattatatta 8040
   aaggataatt attaaggata gttaagagta tagtttataa attaaaatta ttgttttaaa 8100
   aatgttgaga atgtttttat taagtgttta aattggataa tagatttgtg ttttaatgtt 8160
   taattagaaa taataaaatt tttttatagt gattatttta atgttttttt gtgttttttt 8220
   tttgttgttg tttggtttat attttttgta tatttttcgt aatttttttg ttttttttcg 8280
   tgtattaatt ttcgtttttt tgtttgtttt tttgttttta gggttgggtt tttttgattt 8340
   ttttattttc ggggtggtat aaaatattta ataagtattt tagagttttt ttagggtttt 8400
   tttttatttt ttattgtttt tatagtaaat cgggtttata ttttttttaa ttttgagttt 8460
   tttgtattgt gcgtagggat gtaggtggtt tttttggtcg gtttaggtag tagattggtg 8520
   gaaatattaa tgaagtttgt tgagtgttat aatgtgttga tatattttta agcgtttggt 8580
   acgtttaatt tatgaaatta ttagttattt taatattgag atatcgagga tagtgtcgtt 8640
   taaaaatttg atttagagtc gggtgtcgtg gtttacgttt ataattttag tattttggga 8700
   gatttaggtg gggagagtat ttgagtttag gagtttaaga ttagtttggt taatatggtg 8760
   aaatttcgtt tttattaaaa atataaaatt agtcgggtgt gttggtgggt gtttgtaatt 8820
   ttagttattt tgtaggttga ggtaggaaaa tcgtttgaat ttgggaggta gaggttgtgg 8880
   tgagttagga ttatattatt ggattttagt ttgggtaaga agagcgaata tttcgtttaa 8940
   aaaaaaaaaa tttgatttaa ttgtttggag tgtttgttgt taattttttt gagttagtga 9000
   tttagtggga gcggtcggaa aggttgttgt tttttggaat tttagtagtt cgatagacga 9060
   ttttattatt ggttaggtgt ttttttttaa ggtgggagac gaatgaggta gtttgtaaag 9120
   gtttttgaag agtgttagag aaaggtgttg tgtaaatata ggtattgtta tttataagtg 9180
   atggatggta ttgtattatg ttttggtaaa tgtgttgtcg tttgtagttt tttgtagaag 9240
   taatttagtt ttaatgattt gagattttat tattgttttt gattataaaa tatttgtaga 9300
   gaaatggggt gagtgtttgt ttgatgagtt attgtaaatg tagattagta gagttagaat 9360
   tagcgagatt ttttttgttt tatttaatag tagttatagt attttatagt agaaaagagg 9420
   atttaattat aagtttcgtt attatttatt tttgttagtt tttgtgagtt tgtggtagtg 9480
   agatgtgtaa aatttttttg ttaaatatat tttgttggtt ttgttttttt ttatttttta 9540
   gtggagttaa aattatgtgt tagtagagtt tttataaatt tgttatatga tagttttgaa 9600
   tgttattaat tttatatgtg gttttttttg aaaagtagat tttatatttt tttttaaagt 9660
   gaattaaatg gaaatataag tacgattata tttattttaa gttatgttat tttttttagt 9720
   aattttagag taatttagtt tttttttata ttaattgata cgttagatat gagttaagga 9780
   tgttttttaa aagtttagtg taattttttt tgaattattt ttttgtagaa agatttttag 9840
   attatttatt gttagaaata gaagattaat ttgtttttta ttaattttgt ggttatagat 9900
   ttgttaagta tagttttagt gttaattagt taggattaga aattgaatgg tttaggtaag 9960
   aggtttgttt ttgtttttaa tatgttaagg aaaataagat tttttagtta ttattttttt 10020
   ttagaagttt tttgttaagg tggtttatgg tattttattt tttttttagt ttttttttgt 10080
   tgtttttgtt tagatagggt tttttttagg tttgagtgta gtggcgtgat tttggtttat 10140
   tgtaattttg gttttttggg tttaagagat tttttcgttt tagtgtgttg agtagttggg 10200
   attataggtg tgtattatta taattagtta atttgtgtat tttgtgtaga tataaggttt 10260
   tattatgttg tttagttagg ttttaaattt ttgggtttaa gcgatttgtt tattttggtt 10320
   ttttaaagtg ttgggatttt aggtatgagt tatcgtattt agtttttttt agttattttt 10380
   ggttatttgt gatttttaac gtttttgtga gtgttttttt tatttcggtt agtgttttgt 10440
   gtatttttat ttggttttgt gattgttttt ttttttttag ttttagaagt tttttttgtt 10500
   tttatagaga atgtttattt ttgtataaag tattatatta ggtatagaga atggtatttt 10560
   ttgttgtaat aaataattta aatttttagt ggttaaatta atttaggttt attttttttt 10620
   tgggtggggt ttttttaata aagagtaatt tagaggttta gatatttttt gttgtataat 10680
   attagttttt tattgtgtgg ttgttgttga ggggataagt gtaggtcgtc gtgttggggt 10740
   tcgtatggtg tggtttataa gtggtatgta ttattatatt agttttttat ggttgttgta 10800
   ttaaattata ataaatttag tggttttaaa taatatagtt ttattatgtt atagttttag 10860
   aggtgagaag tttaagatgg gttttattgg gttaaaatta aagtatttat agggtaattg 10920
   gttttttttg gaggatcggg gagaatttta tttttttgtt ttttttagtt tttagaggtt 10980
   gtttattttt tttggttcgt ggcgtttttt tttataagtt aaggaaagtt tttttaaagg 11040
   tagtaatgat tagttatatt tttatattat atatttagag ttttgatttt ttcgtttttt 11100
   ttttttatat ttagagattt ttgagattat atgggtttat ttgggtaatt tagaataatt 11160
   tttttatttt tagtttatga ttttaagtat agaatagtag tttttttttt ttttttttag 11220
   tttatagtta gttgatgagt aattttaatt ttatttataa tttttatttt ttttggtcgt 11280
   gtagtttagt atatttatag gttttagagt ggggggacgt gatgatatga tgttgtttat 11340
   taaagttatt tttttttata ttattgggta ggatttagtt tgatgttttt aatttaatta 11400
   cggggaagtt gggagtgaga ttggtaaata tttgattagt tttagtttta attaattttt 11460
   aatgatagtt tttttaaagt atttttaggt agtcgatttt attttttttt aaattttttg 11520
   aattagtttt tttgtattta aggatatata tgtagttgtt ttatgtattt tttagtttga 11580
   aaggggttta gttagatgtt cggtaaggtg tttattttgg tttagttagt atttattttg 11640
   ttttttgggt tagaaattat gttaaggatg gaatatatat tgttgtggaa gatggagtgt 11700
   ttggtttagg ggggttttat tatgtttttg aattttattt tttaataggg ttgtttcgtg 11760
   taatttaaga gtttttttat ttttatagtt tatgattttg agtatagaat agtagtatga 11820
   agttaacgag agtgtaaatt taggaaagta tttggggttt tagtttattt tttagtataa 11880
   attattattt ttattttatt tatgtatatt tgaaagtttt tgtaagggtt ttatttggtt 11940
   tgggggtaag gtttttattg gggtttttta taatttatgt ttgttattgg gttagattgt 12000
   tagtatttat tttttgtata cgtttttatc gttttttatt tttttatttt ttttattttt 12060
   tagggttttt atttttaggt gtatgggttt ttatagatgt gagtttttat ttgttagtga 12120
   tttttttttt ttttggtgga gttatttttt ttaattaaga taggttatag ttttttgttt 12180
   tgttcgtatt gggaagtttt atttatttaa ttttattagt ttttttgttt atttgttatt 12240
   atattgtttt atttgagatt ttttggttta attataattt gtagtattgg ttgttttttg 12300
   ttttgttttg ttttttttat tggaatagtt tttagaattt ttcgttattt tttttaggta 12360
   gaagagtttt tttttgaata atatttttat agtttttttt aggtaagaat aaaggtagta 12420
   tatatttgtg agttatattt tagtatttgt agtggatgtt tttgggatat ttgtgtgagt 12480
   taatagtttt ttttagtttt ttgttggaaa ttgttttttg gtttatggaa tttttttagt 12540
   attttttttt tttttttttc gtagtagtat tttttagttt gttatttggt attagtagat 12600
   agtaagttat tagtgattgt attttatagt taggtatttt gatatttgag gacggatgtt 12660
   tggtttaggg ttttatatag ttatttgttt ttttggtgtt tgtttatttt ttagatgtga 12720
   tcgtaggtaa agtttttttt attttttttt ttatttgggt aggtagttgt tttgtggaga 12780
   gttttttatt ttaaatttat ttttttttat ttaggtagtg taataatttg gttttgtttg 12840
   taggtcgttt ttttgagtta tttttagaag ttatggtgtt ttgttttttg gaaggaattt 12900
   gattaaggaa aggtttattt acgattaagg gtggaattta gtttgtcgtt tgttagttgt 12960
   gcgatttggg gtcgttggtt tttttttttt tttgttttag gatttatttc gtggggttgt 13020
   tgtgtagtga aagatgcgtg gattttaagt ggttagagcg gtgtttgtta tagagtggtg 13080
   atgaggagta gaagttggat ttgtgatttt tttaaagttt ttttaggtgg tgatgtaatg 13140
   acggaggttt tggtggtggt tagtattacg atgggtaggt gttttatagg gtatggtttt 13200
   tattttttag taattcgtag ggtagcgttt tttaaggaat tgttgtgaag agagggtgaa 13260
   gagttggaaa agtattaacg atttgaggat tttagaggtt ttttttgagt attaaatgtt 13320
   aatttaaaaa tttatttaat aagattagtt aatagttgag tatattttgt gtgttaggtt 13380
   ttgttggagg tattttatgt gttttaattt atgtaatttt tagttggttt tgggagaggg 13440
   gaatattttt attttttttt tataggtggt agattttgag gtatagagag ttttagtaat 13500
   ttgtttatat atagttatat atataagtat atacgtatgt atatatgtgt atatatttat 13560
   gtataggtat atgtatatat atatatatat aggtagaatt gggatttgat tttaaggttt 13620
   tggttttagt atttgtgttt ttattttttg tatagtattt ttgttaagag gaagtttatt 13680
   ttttatattt gaagttttta tttgaataat aattattacg ataaaaattt aaggtttttt 13740
   tgtttttttt aaaaatattg atttgtaaat aattttgtaa agatttttgt aaaagtaatt 13800
   ttttttttgt agttttataa atgaagagtt ttagagatag tgatataata attataataa 13860
   atatgtgttg ttatattttt tggtagtatt gtaagttaaa atgaatttgt tttagttttg 13920
   agttattatt gttattattg ttatttttta ttgttatatt aattattaaa tataattgag 13980
   agggattaat gaagttaatt gtttttagaa attaaattaa atgtaagtgt taatgttggg 14040
   tgattgagtt ttgatattgt gtgtttattt tagtagagga gcggataggt tgttttgtag 14100
   gagttttaga agatttgatt attttaaatg ttattttaat atttattatt agtttttttt 14160
   atagttagtt gtatagtttt ttttattttt attttatgaa tatatttgtg agttatagtt 14220
   taaaagtttt atgtgtgtta agttggaaag atttagagat tatttttaaa tgggtatttg 14280
   tattgttggt ttggtttgtt ttaataaaat attatagatt gggtggttta aattatagat 14340
   atttattttt tttagtttgg gaggttgaag tttaagatta aggtgttggt agttttgttt 14400
   ttttttgaga tttttttttt gggtttgtag atggtatttt tttgttgttt ttttgtaggt 14460
   tatttttttg tgtgtgtacg attcggtgtt tttttttttt atagagatat tatttttatt 14520
   atattagagt tttattttaa ttttttattt taatttaatt gttttttaag attttttttt 14580
   taaatataat tataatttaa gggtatgtta gttaggattt taatttatga attttggggg 14640
   gatattattt agttcgtaat agtatttttt attaattttt gatgagtatg ggaaagaggt 14700
   gggatagagt agggagggaa atgaattttt aaaaaatatt tagtgggata gtttttttaa 14760
   gttttttttt gagttcgaaa tatttataat tttgagattt tgtgttttaa gagagtggaa 14820
   aatgatttta aggtgtaggt agataattgt atttttagtt tttatttttg attgggaggt 14880
   taattacgta ggttgggaaa tttttttaat tttagattaa aaagttgttt gtaaatggta 14940
   ggtttttttt gttttgtttt tggttttttg tttttttaaa agaatttgat tttgaagtga 15000
   aaggaatttt tggaaattta aaacgaagaa ggaattgaaa tttagatttt tagtatgaga 15060
   ggtgggttta taggaggtag ggagtagtta aggttggatg tttagaggtt atatttttgg 15120
   ggaaaggtgg aagagggaaa tttgtttagt ggttttagga gggttaggaa gtcggattgt 15180
   gttaggttgg gttttatatg gggatagagg tttttttgag aatttgtaaa taaaggtttg 15240
   tttttatatg agtttagttc gtatttgtat tgtttggtaa tttgtttttt gagaaatttg 15300
   ttttgaaaag gatttttaga tatttatatt tttggtttga taaaaatacg tgtagtattt 15360
   ttttagaggg ttgttgtttg tattataggt tttatgagat ttttattggt aaagtttagt 15420
   gaagatagat ttttttttta agttttttag gataagagta agaatttata ggtaaaaata 15480
   gatagtagga ttagatttta gaggatttta gaaaattaaa taattggata tataattaaa 15540
   atgattaaat atataaagga attgaacgta taagaaaaga ataagatttt attaaatatt 15600
   tggtgaattt gagataaaag ttgtaggaat gaaaaataga gtgattaaaa attaattgat 15660
   gggatataga gtagattttt gagaggatta ttgaatagga ttttgttttt ttttgtttta 15720
   agtagaatgt ttttttcgtt gagaagaggt gcgggtagta gggagatttt agttttaaat 15780
   ttaagttaga ggaaatagtc gttatttttt ttgttttttt ttgttttttt ttaatagtta 15840
   gttgttgggg tagtgttggt tttgggagta aacgttatta ttttttttta gtcgggttgg 15900
   ttaagttttt tagggaaatt gaagattagt atatttgaag tatgtaagtt ttaggttgtt 15960
   ttttttattt tttttttcgt ttattagtag tataaagaaa gttggtttgg gttttcgtga 16020
   ttcggtaggg tttatttagt ttaaaatgtt agaggatatg gtgtggtaag ttgattgtat 16080
   cgtgatgtta gtcgttttgt ggtagagatt tagagatttg aagggtaaag tttagtaagg 16140
   tttatattta gagtttgggg ggttaatgat tataaggaaa atgattttgt tttttagtta 16200
   gtattttatt ttttcgaacg tttttttttt tttaatagtt tgaggaggtt tatttttttt 16260
   ttgtagatga agtagggaat ttttgagaat ttgagatatt agtttgagat tatatagttg 16320
   agggcgtgga ggaacgtgtc gattttaaat atatgttggt ggttattatg cgaggttggt 16380
   tttttggatt tagggttagt tagtattttt aaattttgtt tatggaatat tgtagttttg 16440
   atggaggata attggtatta tagttaaaaa aaaaaaaaaa aaaaaagttg tgtggttaga 16500
   taggttgggg agagttggta agttgtatgt tttttgtaag ttttttttat gtaggtagta 16560
   tattaagggt taagaaggtt ttttttgggg aaatttattt aattttgttt aatttggtat 16620
   tttttatttt taatagatgt tagattattc gattttttat aatatagtat taatatatta 16680
   gttataaagg aattgtatat taaattataa taagagatta ttttatattt attaggatgg 16740
   ttataattaa aaagatatat aatagtaagt gtcgggaata tgtggagaat tggaatttat 16800
   atattggtgg gaatgtgaaa tagtgtggag gttttggaag atagtttggt ggtttttaga 16860
   aggataaatt tagaggtatt atgtggtttg gtagttttat ttttcggtat atatttaagg 16920
   aaatgaaaat atatgtttat ataaaaattt acgtgtaagt tttttatagt tgtattgttt 16980
   ataatagtta aaaaagtaga aataacgtaa atgtttaatt gatgagtgga gagatataat 17040
   gtgatatatt tatgtaatag aatatttgat aagaagagga atgagatatt tatacgtgtt 17100
   gtaatatgga tgaattttga aaatacgtta agtgtaagga gttagttatt aaaggttata 17160
   tagtttataa ttttatttat attatatgtt cgaaataagt aagtttaggt gattggtttt 17220
   taggagttgg gaggggtgga atggagggta attgtttatg gttgtggggt ttttgtttgg 17280
   ggtgatgaaa attttttaaa attgattatg gtaatggttg tataattttg aatgtattta 17340
   aaattatcga attatatagt ttaaatgaat gaattgtatt ttatatgaat tatatattta 17400
   taaagttgtt ttttaaaatt aagttaatat ttgttagcgg tagggtttta tagtatagtt 17460
   tgggtaaagt tgtagtagaa gttttgttgt tatttgtttg ggttttgtga aggtatagta 17520
   gttgttttat gttatttatt attatattat tttattaaaa atattttcga tatttgagaa 17580
   agtaaatttt ataggggagg gttgaggtga gtttttgtgt tgagattgtg gagaattgaa 17640
   agtagtagtt attttttttg tgtagtttgt gttttcgtta tattgttgtt ttgataaaat 17700
   atttgaaagg tgattagata gaaaaataag agttttggaa agtttttgag tggatttgta 17760
   gtaagagatt atgttgagtg ttgtattatt ttgaatttat ttcgataatt ttgatttttt 17820
   ttagttaatt atttttattg ttagattttt tagattgatt tgtggttatt gagtaattag 17880
   aattttttta gttggttttt ttgatttata ggtagtttta tttttatatt aattttatat 17940
   tttttgtttt tattttagt 17959
<210> 42
   <211> 17959
   <212> DNA
   <213> Artificial Sequence
<220>
   <223> chemically treated genomic DNA (Homo sapiens)
<400> 42
   attgaaatga aaatagagag tgtgggatta atgtggaaat ggggttgttt gtggattagg     60
   gggattagtt ggaaaggttt tggttgttta gtggttataa gttagtttgg agaatttggt    120
   aatgaaaatg attaattagg gaaggttagg gttgtcgggg taaatttaaa atgatatagt    180
   atttaatata attttttatt atagatttat ttagaaattt tttaaggttt ttgttttttt    240
   atttggttat tttttaagta ttttgttaga gtagtagtgt aacgagggta taaattatat    300
   aaagagagtg gttgttgttt ttagtttttt atagttttaa tataagagtt tattttaatt    360
   tttttttgtg aaatttattt ttttaaatat cgaagatgtt tttgatgggg tgatatggtg    420
   atgggtgata tgggatagtt attgtgtttt tatagagttt aggtaagtgg taataaaatt    480
   tttattgtag ttttgtttaa attgtgttgt gaaattttat cgttggtaga tgttaattta    540
   gttttaaaaa atagttttat agatatgtaa tttatgtaga atataattta tttatttaaa    600
   ttgtataatt cgatggtttt gagtgtattt agaattgtat aattattgtt ataattaatt    660
   ttaggagatt tttattattt taaatagaaa ttttatagtt atgaatagtt attttttatt    720
   ttattttttt tagtttttgg aagttagtta tttggatttg tttatttcgg atatgtgata    780
   taagtggaat tataaattat gtggtttttg gtgattggtt ttttgtattt agcgtgtttt    840
   taaggtttat ttatgttata gtacgtgtaa gtattttatt tttttttttg ttaaatattt    900
   tattgtatgg atgtattata ttgtattttt ttatttatta attagatatt tgcgttgttt    960
   ttattttttt ggttattatg aataatgtag ttgtgaagaa tttatacgta agtttttgta   1020
   tggatatatg tttttatttt tttgagtata tatcgagaaa tggaattgtt aggttatatg   1080
   gtgtttttga atttattttt ttgaaaatta ttaagttgtt ttttaaagtt tttatattat   1140
   tttatatttt tattagtgtg tgggttttaa ttttttatat gttttcgata tttattattg   1200
   tgtgtttttt taattatagt tattttagtg ggtgtgaagt ggttttttat tgtggtttga   1260
   tgtgtaattt ttttatggtt aatatgttaa tattgtgttg taaggggtcg ggtaatttag   1320
   tatttattaa gagtgggaaa tgttaggttg aataaaatta aataggtttt tttaagagag   1380
   atttttttgg tttttagtgt gttatttgta tgaggaagat ttataggagg tatatagttt   1440
   gttagttttt tttagtttat ttgattatat aatttttttt tttttttttt ttttggttat   1500
   aatattagtt attttttatt agggttatag tgttttatga atagaatttg gaaatgttgg   1560
   ttggttttaa atttaaaaag ttagtttcgt atggtagtta ttagtatgtg tttggggtcg   1620
   gtacgttttt ttacgttttt agttatgtgg ttttagatta gtgttttaga tttttaaaga   1680
   ttttttgttt tatttgtaga aaggagataa atttttttag attattaaga agagagaggc   1740
   gttcgggaga gtggggtgtt ggttagaaag tagggttatt ttttttgtag ttattggttt   1800
   tttaggtttt ggatgtgggt tttattgagt tttatttttt aagtttttga gtttttgtta   1860
   taagacggtt aatattacga tatagttaat ttgttatatt atgttttttg gtattttaaa   1920
   ttgagtggat tttgtcgagt tacgaaaatt taggttaatt ttttttatgt tgttgatgaa   1980
   cgggaaggag aatgggagag gtagtttggg gtttatatgt tttaggtgta ttggttttta   2040
   atttttttgg gagatttggt tagttcgatt ggagggagat ggtgacgttt gtttttagga   2100
   ttagtattgt tttagtagtt ggttgttaga aaagggtaaa agagagtaag gaaaatgacg   2160
   attatttttt ttggtttggg tttagaatta aaattttttt gttgttcgta ttttttttta   2220
   acggagagga tattttattt ggggtagaag gaaatagaat tttgtttagt aattttttta   2280
   aaaatttgtt ttatatttta ttaattgatt tttaattatt ttatttttta tttttatagt   2340
   ttttgtttta aatttattag gtatttgatg ggttttattt ttttttttgt gcgtttaatt   2400
   tttttatata tttaattatt ttagttgtat atttaattat ttgatttttt gaagtttttt   2460
   ggagtttaat tttgttgttt gtttttgttt gtggattttt gtttttgttt tgggagattt   2520
   ggaggaggaa tttgttttta ttgggtttta ttagtaagga ttttatgggg tttgtgatgt   2580
   agatagtaat tttttgggga gatgttatac gtgtttttgt taggttagga gtataggtgt   2640
   ttgaaaattt tttttaaggt agatttttta gaagataaat tgttaaatag tataaatacg   2700
   aattgaattt atgtaaaagt aagtttttgt ttataaattt ttaaggagat ttttgttttt   2760
   atgtggagtt tagtttgata tagttcggtt ttttggtttt tttagggtta ttgggtaggt   2820
   tttttttttt tatttttttt taggggtgtg gtttttgagt atttaatttt ggttattttt   2880
   tgttttttgt gggtttattt tttatgttaa aggtttgaat tttagttttt ttttcgtttt   2940
   gggtttttag gaattttttt tattttaagg ttaaattttt ttaaaaaagt aaaaagttaa   3000
   agataaaata gaaaagattt attatttgta ggtagttttt taatttaaga ttaaaaagat   3060
   tttttagttt acgtagttgg ttttttaatt agaaatggag attgaaaata taattattta   3120
   tttatatttt agggttattt tttatttttt tgaaatatag gattttagag ttatgaatgt   3180
   ttcgggttta aggaagagtt tagaagaatt gttttattaa atatttttta aaaatttatt   3240
   tttttttttg ttttatttta tttttttttt atgtttatta gaaattgatg aaagatgtta   3300
   ttacgggttg aatagtgttt ttttaaaatt tataggttga agttttaatt gatatatttt   3360
   taaattgtaa ttgtatttgg agagagggtt ttaaaagata attaagttaa aatgaggaat   3420
   tgaggtaggg ttttaatata atagaaatgg tgtttttata agagaaagag atatcgggtc   3480
   gtgtatatat aaagaaatgg tttgtaagga aatagtaaga aagtgttatt tgtaagttta   3540
   ggagagaggt tttaggagaa agtagagttg ttaatatttt gattttggat tttagttttt   3600
   taagttggga gaaataaatg tttatggttt aagttattta gtttgtggta ttttgttaag   3660
   gtaggttaaa ttaataatat agatgtttat ttaaaaatga tttttgaatt tttttaattt   3720
   ggtatatata aaatttttaa attgtaattt atagatgtgt ttataaagta aaaataaaaa   3780
   gagttgtgta attaattata aagaaaatta atgatgaatg ttaaggtgat atttgaaatg   3840
   attaagtttt ttgaagtttt tgtagggtag tttgttcgtt tttttgttga aataagtata    3900
   tagtgttaag gtttagttat ttagtattag tatttgtatt taatttagtt tttaaaaata    3960
   attaatttta ttaatttttt ttagttatgt ttggtgatta atgtagtaat aaaaagtggt    4020
   aataataata ataataattt aaagttgaga tagatttatt ttaatttata atattgttaa    4080
   aaaatatgat agtatatatt tattgtgatt gttgtattat tgtttttaga attttttatt    4140
   tgtaaaatta taaaagaaga attatttttg tagaaatttt tgtagaatta tttgtaaatt    4200
   agtattttta ggagaaatag aaaaatttta gatttttgtc gtggtggttg ttatttaaat    4260
   gagagtttta aatgtaaaaa ataagttttt ttttagtaga agtgttgtgt agaggataag    4320
   aatatagatg ttggagttag aattttggga ttaaatttta gttttgtttg tgtgtgtgtg    4380
   tgtgtgtatg tgtttgtgta taagtgtgtg tatatgtgtg tatacgtatg tgtttgtgtg    4440
   tgtggttgtg tgtgggtaag ttgttgaagt tttttgtgtt ttagggtttg ttatttatga    4500
   agaaagagtg aaaatgtttt ttttttttag ggttagttga ggattatatg agttaggata    4560
   tatgaaatgt ttttaatagg atttggtata taggatgtgt ttaattgtta gttagtttta    4620
   ttaaatgggt ttttaagtta gtatttagta tttaagagag gtttttggag tttttaaatc    4680
   gttggtgttt ttttagtttt ttattttttt tttatagtag ttttttaaag agcgttgttt    4740
   tacgggttgt tggggaatga aggttatgtt ttgtaaagta tttgtttatc gtggtgttga    4800
   ttattattag gattttcgtt attatattat tatttgagga agttttggga gaattataga    4860
   tttaattttt gttttttatt attattttgt ggtaggtatc gttttaatta tttggagttt    4920
   acgtattttt tattgtatag tagttttacg gagtagattt tgaggtaggg agaaggggaa    4980
   gttagcggtt ttaaatcgta tagttggtaa gcggtaaatt gggttttatt tttaatcgta    5040
   ggtaagtttt tttttgatta gatttttttt aggaagtaga gtattatggt ttttgagagt    5100
   aatttaaagg aacggtttat agatagggtt aggttattat attatttaga taagggaagg    5160
   tgagtttgaa atgaaaagtt ttttatagag tagttgtttg tttaagtggg aaggggagtg    5220
   gaggaagttt tatttacggt tatatttggg aggtaagtaa atattaagga ggtaaatgat    5280
   tatgtgggat tttgggttaa gtattcgttt ttaggtgtta ggatgtttgg ttgtaagatg    5340
   tagttattgg tggtttgtta tttgttagtg ttaaatagta aattagaaaa tgttattgcg    5400
   ggaagaagaa aggggaaata ttggaagagt tttatgaatt agggagtaat ttttagtagg    5460
   gagttaggaa gggttgttaa tttatataaa tgttttaaaa gtatttattg taggtattgg    5520
   agtatgattt ataagtatat gttatttttg tttttgtttg agaagagtta tggaaatgtt    5580
   atttaaaggg aagttttttt atttaaaaga agtgacgaaa ggttttgagg attgttttag    5640
   tggaaggaat aaaataaaat agaaaataat tagtgttgta ggttatgatt aaattaaagg    5700
   attttaggta gagtaatgta gtggtaggta agtaaagggg ttggtgagat taggtgggta    5760
   gaatttttta atgcgaatag ggtaagggat tatggtttat tttagttaag aaaaatgatt    5820
   ttattaggag agaggagaat tattggtaag tggagattta tatttatgga agtttatgta    5880
   tttgaaggtg ggaattttaa aaagtggaga ggataaaggg gtgagaggcg gtaagaacgt    5940
   atgtagagag tgggtattga tagtttggtt taataataga tataagttat agaagatttt    6000
   aatgagaatt ttgtttttag attaagtgga gtttttatag gagtttttag gtgtgtatag    6060
   ataaggtaga agtaatggtt tgtgttaaaa aatgagttgg agttttagat atttttttga    6120
   atttgtattt tcgttggttt tatattgtta ttttgtgttt agaattatag attgtgaagg    6180
   tgagaaggtt tttaaattat acggagtaat tttgttaaag aatgaagttt agagatatag    6240
   tgggattttt ttgggttaaa tattttattt tttatagtag tgtgtgtttt atttttgata    6300
   tagtttttga tttaaaggat aaaatgagta ttgattgggt taggatgggt attttgtcgg    6360
   atatttgatt gggttttttt taagttggga ggtatatgga gtagttatat gtatattttt    6420
   gagtgtaggg aggttgattt aaaaagttta ggggaaagta ggatcggttg tttggaagtg    6480
   ttttggaaaa gttgttattg ggaattggtt gaggttagga ttagttaggt gtttgttagt    6540
   tttattttta gttttttcgt agttggattg ggggtattaa attgggtttt gtttaatagt    6600
   atggggaaaa gtgattttgg tgggtagtat tatgttatta cgttttttta ttttggaatt    6660
   tgtgagtgtg ttgggttata cggttagggg aaatgagggt tgtagatgga attaaggttg    6720
   tttattagtt gattgtaagt tgaggaagaa gaagggaaat tgttattttg tgtttagaat    6780
   tatagattga aggtgaggag attattttgg attatttagg tgggtttatg taattttaag    6840
   ggtttttgaa tgtgaaagag ggaggcggaa gagttagggt tttgagtgtg tgatgtgagg    6900
   atgtgattgg ttattgttgt ttttgaagaa gttttttttg gtttgtggag gaaggcgtta    6960
   cgagttaagg aaagtgggta gtttttagaa gttggaaaag gtaagaaaat gggatttttt    7020
   tcgatttttt agaaggaatt agttgttttg tggatatttt gattttagtt tagtgagatt    7080
   tattttggat tttttatttt tagaattgta atataataaa attgtgttgt ttagaattat    7140
   taagtttatt gtgatttggt atagtagtta taggaaattg atgtgatgat gtatattatt    7200
   tgtgggttat attatacgga ttttagtacg gcgatttata tttgtttttt tagtagtagt    7260
   tatataatga aaagttagtg ttatatagta aggggtgttt gggtttttga attgtttttt    7320
   gttaagaaga ttttatttag gagagaaata aatttggatt agtttggtta ttgagaattt    7380
   aggttgtttg ttataatagg aagtattatt ttttatattt aatatagtat tttatataaa    7440
   agtgaatatt ttttatagaa ataaaaaaaa tttttgaagt tgggaaggga aaagtagtta    7500
   taaaattaag taagaatgta tagggtattg gtcgaaatga aaaggatatt tataaagacg    7560
   ttagaagtta tagataatta aaaatggtta gggaaggttg ggtgcggtgg tttatgtttg    7620
   gagttttaat attttgggag gttaaggtgg gtaggtcgtt tgagtttagg agtttgagat    7680
   ttgattggat aatatagtaa aattttgtgt ttatataaaa tatataaatt agttggttgt    7740
   ggtggtgtat atttgtagtt ttagttattt agtatattga ggcgagaaga ttttttgagt    7800
   ttaggaggtt aaggttgtag tgagttaaga ttacgttatt gtatttaagt ttgagagaga    7860
   ttttgtttag ataagaataa taaaaaaagg ttagggaaga ggtgaaatgt tatgggttat    7920
   tttgatagaa ggtttttgaa aaaagatagt aattaaaggg ttttgttttt tttggtatgt    7980
   tgggaataag ggtagatttt ttatttggat tatttaattt ttggttttga ttggttgata 8040
   ttgggattat gtttagtaaa tttgtgatta tagggttaat agaagataaa ttagtttttt 8100
   gtttttagta atggataatt tgaaagtttt tttgtaggga gataatttag ggagaattat 8160
   attagatttt tgaaaggtat ttttaattta tgtttgacgt attaattagt gtaagagaga 8220
   attaagttat tttgaagtta ttaaaaaaag taatatggtt tgaaataaat ataatcgtgt 8280
   ttatattttt atttaattta ttttgagagg ggatgtagag tttgtttttt aaagaaaatt 8340
   atatgtaaga ttagtaatat ttaaagttat tatgtagtag atttatgaga attttattga 8400
   tatatggttt tggttttatt gggaaatgga ggggaatagg gttagtaaaa tgtatttaat 8460
   aaagagattt tatatatttt attgttatag atttataaag attggtagaa atgagtaata 8520
   gcgaggttta taattaggtt tttttttttg ttgtggaatg ttatgattgt tgttagatga 8580
   aatagaaaga atttcgttaa ttttaatttt gttaatttgt atttgtaata atttattaaa 8640
   taagtattta ttttattttt ttgtaaatat tttgtagtta agagtagtgg taaggtttta 8700
   aattattaag gttggattat ttttatagag gattgtagac gatagtatat ttgttaggat 8760
   atgatgtagt gttatttatt atttgtaaat aataatattt gtatttgtat agtatttttt 8820
   tttaatattt tttagaagtt tttataaatt gttttattcg ttttttattt tgggaggagg 8880
   tatttggtta gtgatggagt cgtttgtcgg gttgttgaag ttttaaaagg taatagtttt 8940
   ttcggtcgtt tttattaggt tattagttta ggggagttgg taatagatat tttaaataat 9000
   tgggttaggt tttttttttt tagacgaaat attcgttttt tttgtttagg ttggagttta 9060
   gtggtgtgat tttggtttat tataattttt gttttttagg tttaagcgat ttttttgttt 9120
   tagtttatag agtagttgag attataggta tttattagta tattcgatta attttgtatt 9180
   tttggtagag acggggtttt attatgttgg ttaggttagt tttgaatttt tgagtttagg 9240
   tgtttttttt atttgggttt tttaaagtgt tgggattata ggcgtgagtt acgatattcg 9300
   gttttgggtt aggtttttaa acggtattgt tttcggtatt ttaatgttaa gatgattggt 9360
   gattttatga gttaaacgtg ttaagcgttt agaagtgtgt tagtatattg tgatatttaa 9420
   taaattttat tagtgttttt attagtttgt tgtttgaatc ggttaagagg attatttgta 9480
   tttttgcgta tagtatagag agtttagagt tgaggaaggt gtgggttcgg tttgttatgg 9540
   gggtagtgag gggtgagaag aggttttggg gaaattttgg agtgtttatt gagtattttg 9600
   tgttatttcg ggaataaaga gattaagaag atttagtttt ggaagtaaga agataggtag 9660
   agaggcgaag gttagtgtac gaaggaaaat aaggggattg cggggaatgt ataggagata 9720
   tgggttagat agtaataaag agaagatata aaagaatatt aagatagtta ttgtaagaga 9780
   attttattat ttttgattga gtattgaggt ataagtttgt tatttaattt aagtatttaa 9840
   tgaaagtatt tttaatattt ttaaggtagt gattttgatt tatgaattgt gtttttggtt 9900
   atttttgatg attatttttt agtgtagtgg gtcgtttttt tttttttttt ttaaaaatta 9960
   tttatttttt agagttgttt attttttggt aatgttagat taaataagaa tattagagtt 10020
   ttttgttaga aaaggtttat atatggtttt ttgtaatatt taggaaagcg tatttgaggg 10080
   gtgttgttgg tatttgaggg gtgttgttgg tatttgaggg gtgttgttgg tatttgaggg 10140
   gtgttgttgg tatttgaggg atgttgttgg tatttgaggg gtgttgttat tgggtttttt 10200
   ttttaattat ggttttggag atagatgata gatggtgata gttattcgta agagtttatt 10260
   tcgtaagttt aaaggggttg ttttgaagtt attagaggag tttatttggg ggatgaatag 10320
   tgttggtatt ttgatgttga agcgggacgt gagatttatt gtagtaaata tagttttatt 10380
   ttgtgatttg tttcgaggtt taaatttcga attttataaa ggagaaaaat tgagttttag 10440
   tgaagcgtga agtgatgttt ttaaatttag aatttaagtt tattgttata ttagtataaa 10500
   taagttgttt tttttttttt taggggtaaa taaattattt attttgtaat ttataaagtt 10560
   tagttgttta ttttgtaatt taaagaaaat tagagtttag taaggaattt tttttaggga 10620
   gtgagtatgt atagtttata tttttgattt taaaaatttt tatatgggta ttttggaagg 10680
   ttgaggtggg tggattattt gaagttagga gtttaagatt agtttggtta atatggtaaa 10740
   attcggtttt tattaaaaat ataaaaatta gttaggtgtg gtggtaggta tttgtaaatt 10800
   tagttattcg ggaggttgag gtaggagaat tttttgaatt tagaaggcgg aggttgtagt 10860
   gagttaagat cgtgttattg tatttttagg tgatagagtg agattttgtt ttaaaaaata 10920
   aaataaaaaa tttttttatg ggtgtgtttt tgtatgtaat ttaattattg tattttggtt 10980
   ttgtattatt ttaatagata cgttttaatg taatttatgt tttggggagt ggagggttag 11040
   agtaggtggt tgttgtagtt tttggaggta gttttgttcg ggtggaaatt taaggcgttt 11100
   ttttttatag tggataggga ttcgttgtgg tggaatggaa gttagttatt tttaaaagaa 11160
   attttattaa aaaaaaaatt ttttttgaag tatttttttt tttaaaatta atgtaatata 11220
   aattttttta aaattatttt ttattatagt ggaatgattt tttaaaattt tatgtttttt 11280
   aagttttttg gtattagtta gatatttttt ttttttagtg ttagttagtt ttgttgtaga 11340
   gagttttcgt tattattgaa aaatattgtt tattttggat tgtaatatat ttaaagaacg 11400
   gtttttattt attgtgttgg tgtagagaat tttcgtttta tagcggagga tagtagagaa 11460
   ggataatggg aatttttttt tttttttttt ttttttttga gatggagttt cgttcgtttt 11520
   gtcgttaagg ttggagtgtt ttggtttaat tttggtttat tataattttt gtttttcggg 11580
   tttaagttat ttttttgttt cggttttttg aatagttggg attataggcg ttcgttatta 11640
   tgtttagtta atttttttat ttttagtaga gatggggttt tattatgtta tttaggttag 11700
   ttttgaattt ttgattttaa gtgatttatt ttttttggtt ttttaaattg ttggggttat 11760
   aggtatgagt tatcgcgttt ggttaggaat ttttattgtt aagaaaatgg cgtaaatgtg 11820
   ttattaaaag aagttatgtt ttttaaagat ttttgttttt ataagattga gtttatgata 11880
   ttaaggcgtg ttattaagat ttaaaagaat tttacgtatt tatatattga agtgatttgg 11940
   tttttgagat ttaaggtttt tgattttttt attagtgttt ttttatatgg gtcgattgtt 12000
   attggaaagg agagtagaaa cggaaataga ttcgtttgga tagaatttaa agtgtaaata 12060
   ggaattgtgt ttagagagag gaggaggtgg tagaagtatg aaggttgttt agatttttat 12120
   gttttaagaa agatcgtttt tttttggttt taattttttt ggtttagaag tttaggattt   12180
   gtttaaaatt agaaaaagta attttggggg aaaaaaaaaa tttggatgtg gaagaaattg   12240
   gcggtgaaag ttatattgag tgattaatga taaaaattat tttcgttttt tggtttttga   12300
   gtcggtatga aacgttggta gtttttaatt aattattatt atgatattaa tattattgat   12360
   tttttattgt ttttgttatt ttttttttag atatttgtat tttttaagta aatattattc    12420
   gttaaaattt tgttataagt tgtgtaattg ggttcgttta aggtatcgga gtttgcgcgt   12480
   aatcgtatag gtacgcggtc gttcgcggtt tttaggaaga aacgtttcgt cgtcgtcggt   12540
   aagcggggtc gaagtttcgg cgggtttttt tcgggtttgg gcgttcgatt ttttcgcggt   12600
   tcgcgttttt cgggcgggta ttaaggttcg gaattcgttt tagcgttcgt cggggttttt   12660
   ttcgcggagt tttcgttcgg cgttttcggg ttcgagttcg ttttcggtat tcgcgcgtgg   12720
   agagagagga aaagagttag tatcggcggg cgggtgttta gttttagttt ttagtttttt   12780
   ttcgggaggc gaagatatga cgcggcgggg ttttcgcggg gtcgaggcgg cggcgcggtt   12840
   ttcgggtcgg gcgggcgtgc gcgagtgagt ttaggtgtga gcgtcgggga gcgagcgggc   12900
   gggagaacga cgcgcgcgcg tggggcgtcg gggtcgcgcg agtcgggtga gtgttattgt   12960
   cgttaagggg gcgcggtcgt cgcgcgtcga cgtggcgtag tcgagtttat cgacggtatg   13020
   cgtattgacg ttatcgcggt tcgttcgttt tgttgggtcg cgaagttaag tatacggtgc   13080
   gtgcgtagtc ggtcgcgtta cgtcggatgt tgtggtcggg ggcgggggcg ggggcggggg   13140
   cgggggcgga ggaagggagg ggagagaagg ggtgaagaag tgagagggag aaggaaggag   13200
   agggaggcgg ggttttcgat tggttgcggg gattggagtt gcggaaagtt aagtagggtt   13260
   tagttgagga tggtttttgt agcgaacgcg tttagtcggg tttttggtta cgcggcgacg   13320
   gttgggtgat tgcgttttat agtgcgggtt agggtcgggg cgtttcggta gggaggcgat   13380
   cgttttttga ggtttttagt ttttgtcgcg gtagcgttta atgaagcgcg gaaaggagag   13440
   tttagatggt agcgacgggg gtcgagagag taggaatttg ttaaaagacg atgcgagggg   13500
   agagtttgtt tttcgagtga gggttagcgt tggggagtag agacggtggt ttttgttttt   13560
   gtttttttcg ttatgaatta aaaataaaat tttaagtttt gtagttaacg gatggatttt   13620
   ttttttcgtt tgtggttttt taagttattt tgaaaaatta gtttaggttt tgatgggaag    13680
   cggaagttaa atatggtttt ataaatattc gtacggagat tttaagatta atagaggatt   13740
   ttgataagaa atatttgtag tgtattttcg gttttatttg tatgataata ttttggtttt   13800
   tataattttt ttatgtaatt cggatatttt tttttattga gtttaggttt ttagatgata   13860
   atttttaatt agttgttaat tagaaaattt ttaaatgtat ttatgattcg gacgttttta   13920
   ttttatattt tattttttaa agagttaatt tatattttac gtattgattg atgttttatg   13980
   ttttttttaa atgtataaaa ttaagatgtt ttttggttat tttgggtata tgttattaga   14040
   attttttgag gttgtgttaa tgtacgtttt taattttggt aaaataaatt ttttaaattg   14100
   agattatttt tagaaatttt tggtttttat tattattttt ttgttgtcgt ggttgatttt   14160
   tggttttcgt gttttggatc gtgttttttt cgtttgtttt agtgttttgt aattgttgtt   14220
   aattttcgta atgttttgtt ggattcgtat ttagataggt ttaatttttt tagcgaagga   14280
   aatattattt ttcgtttggt tgtatttttt tttttgataa taaaattgtt tagggaggtt   14340
   gagtttttta ttttttgtta ttttttttag tttagtttgt ttggttttta ttaagttttg    14400
   ttgtattgtt tttaattatg ggaggttttt ttgtggaagt tatttataat ttatgtgttg   14460
   ttagatttaa tggttatttt ttatttttat tttatttgag tttttagtag tatttgatgt   14520
   agttggtcgt tttttttttt ttttgtttcg gaggtgttat attattgttt tattttttag   14580
   ttttttggtt attttggttg tttattttgt tagtttggta gttcgttttt ttttagattt    14640
   ataaatattt gagtatcgta aggtttagta ttggtttttt tttttattta gattttttat    14700
   agatagttat atgtagtttg tggacgatta ttatgtagat gtttataggt gttaaatttg   14760
   taattttatt tttttatttt tgtttttttt ttgaatttta gatttattat tattattatt   14820
   tgtagacgtt tatgggtgtt atatttgtaa tttaattttt ttttttttgt ttttttattg   14880
   aattttaaat ttattatcgt tattttggtt tttttatttg ttgtttaatt tagtgttgcg   14940
   taattttgtg tattagggta tatatggaga tgtatatagt atatattggt tttgtatttt   15000
   ttattttata tatatatttt aatatcggta ataggataat tgttggttag gttgtagtta   15060
   tgatatgttt gttattgttt tgtatttata tttaaatttt taagtgggtg ttagtagttt   15120
   agaagagaat tttgaaatta atattgggtt attttttaaa gagatagtat attatttacg    15180
   tttttaaagg tttaaggata tttaattgat taattttatg agtaattttt tttttatgta   15240
   tgtgtaaaag tgttatatag ttaaaaattt atgtcgtatg ttttaagttt aaagaattat   15300
   tttttggaag tataaaatga aaattttaag ttgaaagagt atattgtggt attatttaat   15360
   tggtagtgtt tttattttta gtggtattta aaataaagtt gtatgttacg agttatagta   15420
   ttttagattt aataataaat gttattggta aggaattttg gggggtgggg gagaaagttt   15480
   ttataggaag gagtagagtt ttagaggggt tttggtggtt tttggattgt tttgaggttt   15540
   gataggttgt gtttttttgt ttgtgatttg ttttttaggt ttgtattgat tgggaaattt    15600
   tggttgtttt atagtaggta gttagtcggt ttgagtaggg taggagagga tttttttttt    15660
   ataatttata ttattagaaa tgttgtgtga ttattaggtg atggttagga agttgttata   15720
   ttgttttttt aaaataataa ttggttgtag ttagtgttag ggaaaggtag ttttttagta   15780
   gatagaaaaa atttgaaagt ggtggttagt agttttttgg taagattttt ttgttatata   15840
   tcgagaggta aaatggtaac gtttgattgg tatatgattt tttaggaata tgaggttggt   15900
   aagggaagat tgttttaagt gagtatgtgt atatttttaa taaatatatt gtttttgttt   15960
   attttttaag cgttggtaag ttattgtgta tgtggatagt ttattttaag ggaagaatta    16020
   ggggataaga ttgatttttg aagaattaag ggatgtaaga ttttagaagt atgttaatgt    16080
   ataaaatttt aagttaaagg ttaaatcgtg tatttgattt ttaaagttat ttgtttggtt   16140
   attttttaag tgtatttttt ttttgtttta gatttaaagt tttttaataa attttatttt      16200
   tttttttaaa gttgttttag tttttttttt tgttttatat tttttagttg aatttttttt    16260
   tttgaagagg taagaattga ggttgttgta gatttgtata gatttattgt tattaacgta   16320
   ttatatttgg gtgttacgtg atttaggtat attttgtttt tgatagtgtg atagttaagt   16380
   taattttata tttttaaaga atttttgatt ttttatttat cggtaaatag tttttttttt    16440
   agaatataat ttaagttatt tattatagtg tggaataatt tgtttattta atagtatttt   16500
   taatgaagga aatattgtta agaaggtagt gaatttcgta aaaaaaagat atttattttt   16560
   tagttttttt  tgtagttagg ggtagttaag tgatatagtt ttgattatgg aaatttgttg   16620
   gggtgttttg gggaaaattt atatttttaa tatggggata tttttttttt ttggtgatat   16680
   tatttgtatt attttgaatt tagatagaag gttaaaggta gaataattat atcgtaattg   16740
   tgaggtaatt aaaggtatga aaattataag ttaaaggtga tagagtagga aggttaaagg   16800
   tatgtgggaa tttgagggtt tatggaggcg ttttagagtt gtggattttt cgtttagatt   16860
   tttttgttgt gtgggaaata aatttttatt aaataaagtt tttgtggttg gtgtttttat   16920
   gggtagttaa atgtaatttt tagtatatat ggtttattag attttgtatg atggggtttt   16980
   tgtttagttg tgagtttttt ttttttggtt tttttggtta tattggtttt tttgaggttt   17040
   ttttagtagg ataagttaat tttttttttt gggattttgt gttagttatt tttattgaga   17100
   tagttaagta taaaggggtt cgtagagaaa ttttgattat ttgtatatcg gaagatgggg   17160
   tggagttata gaagtttacg ttttttatag gggagaggag tttgattttt tttcgggtgg  17220
   aatttgggat ttaatttgtg acgcgggaaa tcggttagta ggatttttgt tttgttgaaa   17280
   gtttttgttt tttttttttt ttttttgttt aataaatttt atttttttta ttttttaaag  17340
   tgtttgtgag tttaattttt cgtggttatg tgataaggac gtttttttta gttaggttaa   17400
   taagaaagtt ttataatatt attgtcgaga attgtttttt ttagattttc gcgtagttaa   17460
   ttttttttta ttttggaatt agttttttaa ggggtttgtt ttgttttatt gtttttatag  17520
   tattttattt ttatttgaaa ttttttattt atttgtttgt tggtttattg ttttttttta 17580
   ttgattagaa tgttagtttt atgaggggag aaatttaatt tattttgttt attaggtatt   17640
   tttagtattt aaaattatgg tgggtataga agaagtattt aatagttatt ttttttttat   17700
   aattttagaa tatagatata tttaattttt tataattgtt atttaaatga agagtagtta   17760
   atgatgatta aatagagaat gaaaaatgta tatagtatat agatggtata tggttagtga   17820
   ttttttttga tatttgtttt attttgtaaa tattttgtgt gtgtatatgt gtgtgtatac  17880
   gtgtgtgtat gtatgtatgg gagtgtttag tttttttagt tttagatgtt gaggataaat  17940
   gttaggtatt atttattgg                                                  17959
<210> 43
   <211> 6059
   <212> DNA
   <213> Artificial Sequence
<220>
   <223> chemically treated genomic DNA (Homo sapiens)
<400> 43
   acgcgtttgt aattttagtt atttgggaag ttgaggaaag agaattattt gaatttggga     60
   ggtagaggtt gtagtgaggt aagattgtgt tattgtattt tagtttgggc gaggagagcg    120
   aaattttttt ttaaaaataa aaataaaaat aaaaaattat agcgagattt aattttaatt    180
   aaaaaaataa agaaattgta tatatatata tgtatatata tgtatattat atatatatat   240
   tatatgtacg ttttgagaaa aataatatta tgaatttatt attcgttaaa aattagagaa    300
   aatattaaat tttttataaa tttatatagt atttttatat aggggttatg ttaatagaaa    360
   gaaaggatta ttagataatt ttttttaaaa tttttttatt attttattga attttataat   420
   ttgttttatt aaaatatttt tttatatttt ttgttttttt tttttttttt gtgagataga    480
   gtgttgtttt gatgtcgagg ttagagtgta gtggtatgat tttagtttat tgtaattttt    540
   tttttttagt ttaagtaatt tttggtttaa gtaattttcg tgttttagtt tttcgagtag    600
   ttgggattat aggtgtattt taatatatgt agttaatttt ttgtatttta gtagagacgg    660
   ggttttatcg tgttgtttag gttggtttcg attttttgag ttttgataat ttattcgttt   720
   cgttttttta aagtgttagg attataggcg tgagttattt tatttgatta attttttgtt   780
   tttttaaaag tatagtaatt atttttaagt tttagtaata aattaatggg tagtttgtta    840
   aatagttatt ggattttata ttaggagggt tttttatatg tatgtatatt taaaaatgat    900
   attaagattt ttttatataa atttattgta gagatatatt ttaaaataag gtaaggaata    960
   taaatgatgt ttattttttt tttaaattaa tagttttttt atataaaaat attagatgat   1020
   ataaaatata tttttttaaa gtataaagtt ttatatagaa taaaataaat attttatggt  1080
   tatttttaag aataagtagt gataaaaagt ttttgtaatt gggtaataaa attttagttt   1140
   ttaggaatat gttttttttt tttttgtttt tttgagagtt gttattatta tataatttat   1200
   agtatttttt taattataga aaattagcgt ttttttaagt atagagatta tatattaatt   1260
   atttttgtgt tttttgtata ggattaattt agtgtttgtt ataattaagt taaattttaa   1320
   gataggagtt ttaaattatt ttaggtttaa tttaaaattg aaaaaaagac gttttttaga  1380
   gtttgagaat aaaaagatat tgataaagaa taaagttata gatatttgtg cgatattagt   1440
   tttttttttt attaaatata gagttattta ttaatattgt agaaatgttt gtagacgata   1500
   aaaagaataa attaaaaata tggattttgt taatatgttt aaatattaat atagattttt   1560
   ttattttaaa tttatattaa tatttatata ttcgaataat gaaaatgaaa ggtttttaaa   1620
   tgagatttaa atataaattg gttttatttt attatagttt aagtttttaa aattatatat   1680
   ataaattatg tgattgcgtt tatgtttatt ttttaatttg agggtttagt gttttttatt  1740
   cgattttttg aaggataagt gattttaaaa aatttttaag aattattaat ttagtttatt 1800
   aagaaatatt ttatttttta gaagaatata gttaagttgg tttagtttat gtatttttat 1860
   tttatattta tagagtttat agacgggatt tttattatat gaaattagtt tttttttttt 1920
   tttttttttt ttttttggag atggagtttt ttttgtggtt taggttggag tgtagtggcg 1980
   taattttagt ttattgtaag ttttgttttt cgggtttacg ttattttttt gttttagttt 2040
   tttcgagtag ttgggattat aggcgttcgt tattacgttc ggttaatttt ttgtattttt 2100
   agtagagacg gggttttatt ttgttagtta ggatagtttc gattttttga tttcgtgatt 2160
   cgattttttg atttcgtgat tcgtttattt cggtttttta aagtgttggg attataggtg 2220
   tgagttatcg cgttttgtcg aaattagttt tttaaaggat ttttttagtt tagttaagaa 2280
   gttttaatat ttataggtat atatattaat aattattagt atttaaaaat agttttttgg 2340
   ttttaatttt atattgtttt ttttaattta atatttatta tcgttaaaat aatttttttt 2400
   agattgtatt taggttattt atagagatat tttgatgatt tttattttaa ggtatggatt 2460
   atttattgaa tggttatatt tgttgaatga gtgaaaaagg attttttttt ttatagattt 2520
   taagttaagt tgttagtatt tatgtagttg ttttggtata agttaagttg tgaaagtttt 2580
   tttttttacg tatataattt taagtgttag aaatgttttg ttttttttat aaatttaaaa 2640
   taatatagtt atggatatgt attttatata ggttagattc ggtaaggata gttacgtttt 2700
   atatatatat ttgatataat cggttttttt taaatataag ttaattagtt ttatgtgttt 2760
   ttattttttt agtatattta gatttgtcga taaagatttt ttgaggtatt atggtatgtt 2820
   gaatatattg ttgtagtagt agtgaaaata tgggagttat agttttaatt ttgttattaa 2880
   tttaatatgc ggttttggag aagttttttt tttatgttta aattttttat tttgtaaaat 2940
   ggaagaaagt attttattat ttttatagag atttagtatt ataaagaaat tgaaagagtt 3000
   tttttattgt attgtaaatt attattataa attcgcgata ataaataagg ttatttattt 3060
   tttatagttt tgtagtaatg attttgttaa agatattttg gttttttata tttatataat 3120
   ttattttaat taaaggtata aattattttt aagattataa ttggttatgt taagtttttt 3180
   ttttttttga ttttatagat atatgttgta tagaatatta ttattgtcgt ttttttttaa 3240
   taagtaatta ggggttttta atttttaaaa tatatatata tattaatttg taaggaattt 3300
   tttaatattt atttaagaaa taagttaatt tataagggga attttattat atttatataa 3360
   ataaaggtaa atttttacgg aaatatttgt tatttttata tatatatatt aatatagtcg 3420
   ttatatatta atagattatt tgtttgttag attaaaaata ttttttttta tgtataaatt 3480
   aattagtgat atttattaaa cgtataatat tgagattatt ttgtcgtttt tatatatcgt 3540
   ttaacgcggt agggatatta tagattttgt tatataggta aaattttttt ttaagatcgt 3600
   gttttcgatt atttttattt tttaattaaa tatatataaa ttggattatg ggggaggttt 3660
   cgattatgtt tataaatata aattaatttg ttaggggtat agtcgatttc gtttttattc 3720
   gtttattttt tagttttttt cgggagtttg tttgttaggg atattttttt atttcgtttg 3780
   gagggatttt tcggtaggtt taggattttt ttttttgttt tttttttttt tatttttatt 3840
   attgtttttt tagaggttta ggttcgggag atgatagtgg tttttagaaa gtttaggatt 3900
   taatcgttga gagagtgttt aggttcgaat gtcggtttaa atcgttttat ttatcgtgtc 3960
   ggaggtcgag agcgatgaga gtatagggaa gtgaggaaga gggggtggtc gttaggtttt 4020
   ttcgtttttt tgggtttatc gcggattttt ttcgtttgtt aggaggcggt tagcgggtaa 4080
   gtcgattggc ggaaatgcga gagaggagaa gggaaaggtg gagggttaaa ggggtaaatt 4140
   gagaggaggc ggatttcgta atcgatattg ggatcgtttt tttttcgtaa agcgaattta 4200
   aaatggcggc ggtagcggcg gtagtagagt ggcgcggtag tttttttaga gggagggagt 4260
   taagggcgtt tagcgatatt tttaattttt tatttttttt ttttcgcgtt tttttttacg 4320
   gttttcgcgt ttcgttcgat ttcggttatg tagcgcgtac gttagttcgt acgcgtacga 4380
   gtgtttacgg gttcgtcgtt ggttgttttt attaattatt attttcggtc gttttgcgag 4440
   ttagttattt cgtacgcgtt tatttacggg aattttttcg tttagttttt tatttttttt 4500
   tagattttgt taagtcggtt ttagcgtagg tttttacgcg tatttttagc ggcgcgagtt 4560
   taagtttttt ttattttttt tttttttttt tttttttttt ttcgttcgta cggttattaa 4620
   tcgtcgttaa agtagtcgtc gttagtattt ttattttata tttttcgcgc gtgcgttttt 4680
   tatagttttt atcgcgggat tgttttattt ttggcggttg taggggtagg agaggaaggg 4740
   atcggtaaag cgagtttggt ttgtcgtttg attggaattg ttagggtggt cggtcgagtt 4800
   ttatgataat ttattttttt gggttcgtcg tcgcggcgtt gcggttcgtt ttttttttta 4860
   tgggatcgcg gcgaggggat cgagggcggt ttagcgtttt tttgtcggtc ggtggttgga 4920
   ggtcgcggcg gttgcgcgtt gagtcgtttt ttgtcggatg attcgatttt tttttgtagt 4980
   tttaggacgg gcgttttgga gtcggtttta ggtagcgtgt gtcggtcgtt tagtttggag 5040
   aattagtttt cgatttacgg tgagggaatg gatcgatacg ggtattgtat cgttgaggga 5100
   aaggagcggg atttcggatt tttaggaggt agggagtgag gtcggtagat cggcgcgttt 5160
   tcggggggga tttttttcgg gcgttgagtt gttaatttgg gattcgagga agttcggcgt 5220
   ggtggtgtgt tttttgttgt tgttaatttt tttcggattt ttcgaatttt atattattgt 5280
   ttatatgcga tgatgtttgt ttgtttttga cgtatttatt tatggatggt attttagttt 5340
   cgttagatag tttggtgatg gaggatgaag aaattatgtg ttttttattt agttttggat 5400
   ttagtttttt ttgtttttag taagttattt ttgttagttt tttattaaaa agtgtatata 5460
   aaaattaggt aattttaaat atgtttttag ggttggtgtg tgaaataata agatagggtt 5520
   gggcgcggtg gtttacgttt gtaattttag tattttggga ggtcgaggta ggtggatgat 5580
   aaggtgaaga gatcgagata attttggtta atatggtgaa atttcgtttt tattaaaaat 5640
   ataaatatta gtcggtcgtg gtggcgggcg tttgtagttt tagttattcg ggaggttgag 5700
   gtaggagaat tgtttgaatt cgggcggcgg gaagttgtag tgagtcgaga tcgcgttatt 5760
   gtattttagt ttggcgataa agcgagattt cgttatatat atatatatat atacgaaata 5820
   ataatatatg taaagtggaa ttagttttta ggtataggga aggtgtagag tattgtcgtg 5880
   ttgttattta tagttttgtt gatgtcgata acgtttgtgg gtgtaatggg tagtgttttg 5940
   tttttttaag tcgttaataa aataaagtag gtcgggcgtt ttgttaaata gttgtattaa 6000
   tgatttgatt tagattaatg aggatatatt ttttaaattt atgtttttta taaaatttt 6059
<210> 44
   <211> 6059
   <212> DNA
   <213> Artificial Sequence
<220>
   <223> chemically treated genomic DNA (Homo sapiens)
<400> 44
   aaaattttgt aaaaggtata aatttgaaaa atatattttt attaatttaa attaagttat     60
   tgatataatt atttaataag acgttcggtt tgttttgttt tattaacgat ttggagggat    120
   agaatattat ttattatatt tataaacgtt atcgatatta atagaattgt aaatgataat    180
   acggtaatat tttgtatttt ttttatgttt aggagttaat tttattttat atatattatt    240
   atttcgtgtg tgtgtgtgtg tgtgtgacgg agtttcgttt tgtcgttagg ttggagtgta    300
   gtggcgcgat ttcggtttat tgtaattttt cgtcgttcgg gtttaagtaa tttttttgtt    360
   ttagtttttc gagtagttgg ggttataggc gttcgttatt acggtcggtt aatatttgta    420
   tttttagtag agacggggtt ttattatgtt ggttaggatt gtttcgattt ttttattttg    480
   ttatttattt gtttcggttt tttaaagtgt tgggattata ggcgtgagtt atcgcgttta    540
   gttttatttt attattttat atattaattt tggaggtatg tttggagttg tttaattttt    600
   atgtatattt tttgataagg aattaataaa aataatttgt tgaagataag ggaaattgag    660
   tttagaattg aatgagaagt atatggtttt tttatttttt attattaggt tgtttaacga    720
   ggttgaagta ttatttatga gtaagtgcgt taagggtaaa taaatattat cgtatatgga    780
   tagtggtgtg aaattcgaga aattcgagga gggttaataa taataaaaag tatattatta    840
   cgtcgaattt tttcgggttt taggttggta atttaacgtt cgggaggaat ttttttcgga    900
   ggcgcgtcgg tttatcggtt ttatttttta ttttttggag gttcggagtt tcgttttttt    960
   tttttagcgg tataatattc gtgtcggttt atttttttat cgtgagtcga ggattagttt   1020
   tttagattag gcgatcgata tacgttgttt ggggtcggtt ttaaagcgtt cgttttgaga   1080
   ttgtaaaaaa gggtcgggtt attcggtagg aaacgattta acgcgtagtc gtcgcggttt   1140
   ttaattatcg gtcggtagag gggcgttagg tcgttttcga ttttttcgtc gcggttttat   1200
   ggaggaggag gcgagtcgta gcgtcgcggc gacgaattta gggaggtagg ttgttatggg   1260
   attcggtcgg ttattttggt aattttagtt aaacggtaag ttagattcgt tttgtcggtt   1320
   tttttttttt ttgtttttgt agtcgttagg aatggaatag tttcgcggtg gggattgtgg   1380
   gaggcgtacg cgcgaggagt gtagggtggg ggtgttggcg gcggttgttt tggcggcggt   1440
   tggtggtcgt gcgggcgggg agggaggggg aggaaaaaag aggaggtgga gaaggtttgg   1500
   gttcgcgtcg ttgaaggtac gcgtgagggt ttgcgttgga gtcggtttga tagggtttga   1560
   gggggagtgg agaattgggc gaggaggttt tcgtgggtga gcgcgtacgg gatggttggt   1620
   tcgtaggacg gtcgaaggtg gtggttggtg ggagtagtta gcgacgagtt cgtagatatt   1680
   cgtacgcgtg cgggttgacg tgcgcgttat atggtcggag tcgggcgaag cgcggggatc   1740
   gtggggaaga acgcgaggag ggaggagtgg gaggttgggg gtgtcgttag gcgtttttag   1800
   tttttttttt ttggaggagt tgtcgcgtta ttttgttgtc gtcgttgtcg tcgttatttt   1860
   gggttcgttt tgcgaggggg aaacgatttt agtgtcggtt gcgggattcg ttttttttta   1920
   gtttgttttt ttagtttttt attttttttt tttttttttt cgtattttcg ttagtcggtt   1980
   tattcgttgg tcgttttttg ataagcggga gggattcgcg gtggatttag ggaagcggag   2040
   gagtttggcg gttatttttt tttttttatt tttttgtatt tttatcgttt tcggttttcg   2100
   atacggtgag tagaacgatt tgggtcggta ttcgggttta agtatttttt tagcgattga   2160
   attttgggtt ttttgggagt tattgttatt tttcggattt ggatttttgg gagagtagtg   2220
   gtgaagatgg gggaggggag agtagggagg gggattttga gtttatcggg aagttttttt   2280
   aggcggggtg aaggggtgtt tttgataggt agattttcgg agggggttgg gaagtaagcg   2340
   agtggaggcg gagtcggttg tgtttttgat aggttagttt gtgtttgtag gtatgatcgg   2400
   agtttttttt atagtttagt ttgtgtgtgt ttaattaagg gataagagtg gtcgaggata   2460
   cgattttgag aggggatttt atttgtgtga taaggtttgt ggtgtttttg tcgcgttgaa   2520
   cggtatgtgg gaacgatagg atgattttag tgttgtacgt ttaataaatg ttattggtta   2580
   gtttgtatat ggaaagagat gtttttggtt tagtagatag atggtttgtt ggtgtgtagc   2640
   ggttgtgttg gtgtgtgtat gtgaggatgg taagtgtttt cgtaagagtt tgtttttatt   2700
   tgtgtaagtg taatggagtt ttttttataa gttagtttat tttttgaata aatgttggga   2760
   ggttttttgt aggttgatat gtgtgtgtat tttagggatt gggagttttt agttatttgt   2820
   tgggaaagag cggtaatggt agtgttttgt atagtatgtg tttgtgaggt tagggagggg   2880
   agaaatttgg tatagttagt tgtggtttta agaatagttt gtgtttttgg ttagagtgag   2940
   ttgtgtaaat gtgaagggtt aaaatatttt taatagaatt attgttataa agttgtaaag   3000
   aatgggtggt tttatttgtt gtcgcgggtt tatagtaata gtttataata taataagaaa   3060
   gtttttttag tttttttgtg atattgaatt tttgtgaaaa tggtaggatg ttttttttta   3120
   ttttataaaa taggaaattt aggtataagg agagaatttt tttaaggtcg tatgttaaat   3180
   tggtgataaa gttggaattg taatttttat atttttatta ttattgtagt agtatattta   3240
   gtatattatg gtgttttagg aagtttttgt cggtaaattt gagtatgttg gaaagatagg   3300
   agtatataag attggttggt ttgtatttgg gggagatcga ttgtgttaga tatgtatgtg    3360
   gaacgtagtt gtttttgtcg agtttgattt gtatgaaatg tatatttatg gttatattat    3420
   tttggattta tggaggaagt agagtatttt tgatatttaa ggttgtatgc gtgagggaga    3480
   aggtttttat agtttaattt gtattagagt agttgtatgg gtgttgatag tttaatttga    3540
   aatttgtggg aggaaggatt tttttttatt tatttaataa atataattat ttaatgaatg    3600
   gtttatgttt tgaggtgagg attattaagg tgtttttgta aatagtttgg atgtaatttg    3660
   aggggagttg ttttgacggt ggtaggtatt aagttgaggg aggtagtgtg gaattaaaat    3720
   tagagaattg tttttgggta ttggtaattg ttaatatatg tgtttgtgga tgttggaatt    3780
   ttttggttgg gttgaaggaa ttttttgaaa gattgatttc ggtagggcgc ggtggtttat    3840
   atttgtaatt ttagtatttt gggaggtcga ggtgggcgga ttacgaggtt aggagatcga    3900
   attacgaggt taggagatcg agattatttt ggttaatagg atgaaatttc gtttttatta    3960
   aaaatataaa aaattagtcg ggcgtggtgg cgggcgtttg tagttttagt tattcgggga    4020
   ggttgaggta ggagaatggc gtgaattcgg gaggtagagt ttgtagtgag ttgagattgc    4080
   gttattgtat tttagtttgg gttatagagg agattttatt tttaaaaaaa aaaaaaaaaa    4140
   aaaaaaaaag attgatttta tgtagtaagg atttcgttta tgaattttat gaatatgagg    4200
   taagaatgta tgaattaagt taatttagtt gtattttttt ggaaaatggg atgttttttg    4260
   ataggttaga ttagtggttt ttaaaagttt tttggggtta tttatttttt agagaatcgg    4320
   atgaaagata ttgggttttt aagttagaag atgagtataa gcgtaattat atgatttata    4380
   tatatgattt taggaattta aattgtaatg aaatagagtt aatttatatt tagattttat    4440
   ttgaaaattt tttattttta ttgttcgggt gtgtggatgt tagtatgagt ttaaggtgaa    4500
   ggagtttatg ttagtatttg aatatgttga taggatttat atttttaatt tatttttttt    4560
   atcgtttgta aatatttttg tagtattagt aaatgatttt gtatttggta aaaagggaag    4620
   ttaatgtcgt ataagtattt atggttttgt tttttgttag tatttttttg tttttaaatt    4680
   ttgagaagcg tttttttttt agttttggat tgggtttgag gtgatttgaa gtttttgttt    4740
   tggagtttaa tttagttata ataaatattg agttaatttt atgtaggaga tataaagata    4800
   attaatatat ggtttttgtg tttgaagaga cgttaatttt ttgtggttga agagatgttg    4860
   tgaattgtgt gatagtggta gtttttaaaa ggataaggaa gagggaaata tatttttaga    4920
   agttgaaatt ttattattta attgtaaaga ttttttgtta ttatttattt ttaggaataa    4980
   ttataaagtg tttattttat tttgtatgga attttgtatt ttaaaaaaat atgttttgta    5040
   ttatttggtg tttttgtata aaggaattgt tggtttgaaa agaaggtaaa tattatttat    5100
   attttttatt ttgttttggg atgtattttt atagtgagtt tatgtaaaaa ggttttaatg    5160
   ttatttttgg gtatatatat atgtagaaag tttttttgat ataaaattta gtagttattt    5220
   gatagattat ttattaattt attattggaa tttaaaaata attattgtat ttttaaaaga    5280
   ataagaaatt ggttaggtgg ggtggtttac gtttgtaatt ttagtatttt gggaggacga    5340
   ggcggatgga ttgttagagt ttagaagatc gagattagtt tgggtaatac ggtgaaattt    5400
   cgtttttatt aaaatataaa aaattagttg tatgtgttgg agtgtatttg tagttttagt    5460
   tattcgggaa gttgaggtac gagaattgtt tgaattaaga attgtttgaa ttgggagggg    5520
   gaagttgtag tgagttgaaa ttatgttatt gtattttagt ttcggtatta gagtaatatt    5580
   ttgttttata aaaaaaaaaa aaaaagtaag aaatgtaaaa aggtgtttta ataggataga    5640
   ttataggatt tagtgagata atggagaagt tttaggaaaa attgtttgat agtttttttt    5700
   ttttattgat atggtttttg tataagaatg ttatgtaaat ttgtgaaaag tttaatattt    5760
   tttttaattt ttaacgggta gtaggtttat ggtgttattt tttttaaaac gtatatgtga    5820
   tatgtgtgtg tagtgtgtat atatgtgtat gtatgtgtat ataatttttt tattttttta    5880
   attgagatta ggtttcgtta tggttttttg tttttgtttt tgtttttgag agagagtttc    5940
   gtttttttcg tttaggttgg agtataatgg tataattttg ttttattgta atttttgttt    6000
   tttaggttta agtgattttt tttttttagt tttttaaata gttgggatta taggcgcgt     6059
<210> 45
   <211> 9838
   <212> DNA
   <213> Artificial Sequence
<220>
   <223> chemically treated genomic DNA (Homo sapiens)
<400> 45
   atttttgtgt tatttagaga agtagttgtt agttgcgcgg gtttgttgag tatttgaagt      60
   aggattagtg cggtaaggaa ttacgagatg ttattttgaa cgtatttgga tggttttgtg     120
   gagcgagggg ttttggattg aatttcgtag gttttagtaa tttttcgaat tgttgacgag     180
   tttataagtt ttatttgtta ttaggatttt ttgtggttat tttagttttg attagtttat     240
   tagaaattta tttttttttt tttttttttt gagatggagt tttatcgttt gttaggttgg     300
   agtggaatgg tattattttt gtttattgta atttttattt ttcgggttta agtaattttg     360
   tttcgggttt ttgggtagtt gggattatat gcgtgtgtta ttacgtttag ttaattttta     420
   tatttttagt agagatgagg ttttattatg ttggtttgga tgattttgat tattcgattt     480
   tgtgatttat tcgttttggt tttttaaagc gttgggattg tattattgtg ttcggttttg     540
   taaatatttt tttagaggta gagttttgtt gttttttaga tgagtatagt ggtgtagtta     600
   gtttattgta ttttttattt tttggatttt tttgttttag tttgttaggt agttgggatt     660
   ttaggtatgt gttattatgt ttagttaatt tttatttatt ttgagataga attttgtttg     720
   gtttggttgg tttcgaattt ttgattttaa gtgatttgtt cgtttcggtt ttttaaagtg 780
   ttgggaatta taggcgtgag ttattgtgtt gatttaattt ttattttttt tgtagtaatg 840
   gggttttatt atgttgttta ggttggtttt tttttcgggt ttaagcgatt tttttgtttt 900
   agttttttaa agtgttggga ttttaggtgt gagcgttaat agtttagaaa attatttgtt 960
   tttttaaata gtgtttaaga tgtgaggagg tttaattttg ttagtgttga taaggttaat 1020
   atgattattt ttcgtttttt attttgtttt taggaaggta ggtggggatt gtgaagtagg 1080
   gttgtggggt tttcggtaaa ggcgggggta gttttggttt tgtattgtta aggttaagtt 1140
   ttagttgagg ttttgtttat atgtaggtat gtttgaggtt tggtttggtt gtcgtatttg 1200
   aattggggtt gtaattgacg ttgtttttag tagttagggt aacggattat tgtgtttcga 1260
   gaattttatt ttatcgttta ggatggagtg tagtgttgtg attttggttt attatagttt 1320
   ttgttttcgg atttaagtgt ttaagtaatt tttgtgtttt agtttttcga gtagttggaa 1380
   ttataggcgt ttattattag gtttgtgtaa tttttgtata tttaggtgag atggggtttt 1440
   attatgttgg ttaggttggt tttaagttcg atcgtaagag attagttcgt tttagttttt 1500
   taaagtgttg gggttatagg tgtgagttat tatatcgggt tttatttttt tttagtagtg 1560
   taaacgttaa gatggtatgt ggaagttgaa tttttttttt tttttgagat agtttttgtt 1620
   ttgttattta ggttggagtg tagtggttta attttggttt attgtaagtt tcgtttttcg 1680
   ggtttacgtt atttttttgt tttagttttt cgagtagtcg ggattatagg tattcgttat 1740
   tatgttcggt taattttttg tatttattag tagagatagg gttttatcgt gttagttagg 1800
   atggttttga tgttttgatt tcgtgatttg tttatcgtgg ttttttaaaa tgttggaatt 1860
   ataggcgtta gttattatgt tttgttattt tttttttttt gagatggagt tttttttcgt 1920
   tatttaggtt gtagtatagt ggtatgattt cggttcgttg taatttttat tttttgggtt 1980
   taagtaattt ttttgcgtta gtttttcgag tagttgggat tataggtatg tgttattagg 2040
   tttggttaat ttttgtattt ttagtagaga tagggtttta ttatgttgag taggttggtt 2100
   ttgaattttt attttaagtg atttgttcgt tttggttgtt taagttgttg ggattacggg 2160
   cgtgagttat tatatttggt taatttattg tacgttgaaa atgtaaataa aaaatataaa 2220
   ggtcggtttg tggtttacgt ttgtaatcgt agtattttga gaggtcgagg tcggcggatt 2280
   atagatattg agattatttt ggttaatatg gtgaaatttt gcgtttatta aaaatataaa 2340
   aattagttgg gagtggtggc gtgtatttgt agttttagtt attcgggagg ttgaggtagg 2400
   agaatcgttt gaatttagga ggtagagatt gtagtgagtt tagattttat tattgcgttt 2460
   tagtttggac gatagaagga gattgtttta aaaaaaaaaa aaaaaaaaaa aaaaggttgg 2520
   gtaaggtggt ttatgtttgt aatttttgta ttttgggagg tcgaggcggg cggattagga 2580
   ggttaggaga tcgagattat tttggttaat acggtgaaat tttgttttta ttaataaata 2640
   taaaaaatta gttgggcgtg gtgacgggcg tttgtagttt tagttattcg ggaggttgag 2700
   gtaggagaat ggcgtgaatt tgggaggtgg agtttgtagt gagtcgagat tgtgttagtg 2760
   tattttagtt tgggtgatag agcgagattt cgttttaaaa taataataaa aaatgaattt 2820
   aggtcgggcg taatggttta cgtttgtaat tttagtattt tggaaagttg aggtgtcgga 2880
   ttatttgagt ttaggagttc gagattagtt tgggtaatat ggtgaaattt cgtttttatt 2940
   aaaaatataa aaattagtcg ggcgtggtga tgggtatttg taattttagt tatttgggag 3000
   gttgaggtac gagaatcgtt tgaattcggg aggtggaggt tgtagtgagt tggatatcgt 3060
   cgttgtattt ttgtttgggt gatagagtga gattttgttt taaaaaataa aattaaaaaa 3120
   aaaataaatt tgatattttt tagtgttgtt agttggtata tattatgttt taataaaatg 3180
   tttgttgtta tttttatttt tgagacgaag agggattttg tgtatgataa ttttagattg 3240
   attttatttg tattttaagt atttagaatg tgggtaatag tttttagtgg aggaaattgt 3300
   tatataggga ttaaagtagt tttagggttt attttaggag ttgttaggcg cgttcggtta 3360
   taggtgagtt atttattcgt ttaatcggtg ttttaggaag gtaggaagcg ggatgtaggg 3420
   ttttagtttt atttttcgta gttttagtat ttgggttaga ggagaggcgg gcggggcgat 3480
   gaagggatcg gaagtcggtt tttttttcgt tcgtttgatt agcgtcgatt gtagttaatg 3540
   aaattaggga tggcgagtgt ttagttagtg gtgaggttgt attcgggtgt gggtgtcgaa 3600
   aaggtagttg ggggcgggac gtcgaggtgc ggtacgagcg gcgggcgtat cggttaatag 3660
   gcggcggggt tcgtttttcg ggggtgggta ttggcgggtg cggtgacgtc ggcgtaggcg 3720
   cgtcgcggcg gtaggggcgc ggggtttttg gaggcggcgg cggtggttag cgcggcggcg 3780
   tcggtcgtat ttttcgggtt gcggagggta aagagcgggt tcgggtcgag gttggagggt 3840
   tgggtggggt tagagcggcg tttcgggggt tcgcggagga cgagggaggg agagaatttg 3900
   aggagttggt aggtttgggg atttatttgg gtgatcgggt cggggaaagg gttcggtcgg 3960
   gcgtgcgagg ggcgtagcgg ttttagggat gttggagcgg agttttaggg atgggtaagg 4020
   ttttgggggc ggggggaggt tttgtaaagg gagttattgg ggtttgttta ggggtttgtg 4080
   cggagattgc gggcgagaag gggtagagga cggtggagga gatttagatg gggttttttt 4140
   tggcggttcg ttttgggggg taggggagac ggcggttggg ggattttgat atatggagcg 4200
   tggaggaaga ggttaatttt gcgaggatag tggaggttag cgggggaggg aggtaaagtg 4260
   ggtttttagg agcgtggaag gcgtcgttcg gtttttggaa aatttagaat tggagggagt 4320
   tagagatggt tgggggtggg cgggggtgtt ttatcgatga gggttttaag agatttgtcg 4380
   agggtaagga ttaggagggg ttaaggtagt aatttgggaa ttttgatcgg aatggtagta 4440
   ttttgggagg tttgggacga tcgtttgagt tttggagttt taggttgtag tgagttatga 4500
   ttgtattatt gtattttagt tagggcgata gagcgagatt tttttttaaa aaataataat 4560
   attaataata aaagaagtag atttgagaat ttcgttaaga tattggttag ggagtaaggt 4620
   agttttggga tttgaggttg aggagaacgt gtaggaatgg tgggggattt tggttgagta 4680
   ggttgagaga agtttaggag tgagaggtaa aaaaagacgt aggtagcgtc ggggaaatta 4740
   gaattatgtg tttggagaga gttttgcggg gtgaaggtgg tttagtgggg ttgttattaa 4800
   atttttgagt ggttatgggg ttttaagagg tgatatgata ggatttagtt tagtaaatat 4860
   agtatttttt tttgtcgagt ttggagtttt agagtggaga ggtgggaggt ttgtgtttgt 4920
   tggataggtt tgtttattaa tttaggagaa aagggtggtg ttttagtttt ttatttttgt 4980
   ttttttttgt tttttttagg gttgttatta ggggattttt gaggttttat ttttaggttg 5040
   cggtgattgt attttttttg gagtggaagt tgttggaagg cggatcggtc gttatgttta 5100
   cgtttaggta ggaggacgtg gaggattatt atgagatggg ggaggagttg ggtaggtgag 5160
   ttgtttgttc gtacggttgg gttttaggga taggagagag gcgttgtggg gttttttgaa 5220
   tagtagtttt ttttttttgt tttttatttt gtatgttgag aaggaatgag ttagtattat 5280
   gcggtggggg aacgaatttg ggtcgtggag ttagatagtt tcggttgtga atttttgttt 5340
   gggaattttt tgtgtgtttt ttgtggggag aaaagtaatt gagtcgttat agtagttaag 5400
   atcgtgtttg gtatggggta ttagagtaat agatgttagt tgtttttgtt attaaaagat 5460
   gtgttttttg tttgggtaat atagtaagat tttattttta taaaaaataa aaatgaaaaa 5520
   aaattaatta ggtgtggtag tttatgtttg tagttttagt tattagggaa gttgagacgg 5580
   gaggatggtt tgagtttagg agttggagga ggttgtagtg agttatgatc gtgttattgt 5640
   attgtagttt gggtaataga gtgaaatttt atttatttaa aaaaaaaaaa atttttttgt 5700
   gttaattttt ttttgtggga ggtattttag tttttttgtt tagtttttgg atgtttaaag 5760
   tggggggagg gaggggcgtg ttttttagtt gggtttaaat ttcggttaat tgtttaatat 5820
   gtttgtggga aaaagtagag gttttttttt ttagggaggt agttgatttt ttgtttgttt 5880
   gggttttgga gggttttttt ggggttttat tttttttaga ttagttaggt attattaggt 5940
   tttgtttttt tttgtagagt ttttagtttt atttggtgag tttgttattg gttagttttt 6000
   ttggagtttt tttatgatgt tattagaagg gaatgtaaat aagtgagttt tgtgttaggt 6060
   tagggagagt gttgtaggtt tttttgggat tttgaggttt tttttttagg aattttttcg 6120
   ttagtggtag agaaagtatg tttttttttt ttttttttta tgttttagtg ttgatttttt 6180
   ttgttagggg tcgtttggta aaagttagta aaatttagtt aaattttttt taattcgtcg 6240
   attttatttt taggaattaa tttattggaa aattgtatat atatggatgg aatgatttaa 6300
   gattggggta gttgttagaa cgtttattag aataatagtt tttttttttt ttttttgata 6360
   ttattttatt ttgtcgttta ggttggagtg tagtgatgcg atttcgattt attgtagttt 6420
   cggtttttag tttagttttt cgagtagttg ggattatagg agttcgttat tatttttggt 6480
   taatttttgt atttttagta gagataggat tttattatgt tggttaggtt ggtttcgaat 6540
   tattgatttt aggtgattta tttgtttcgg ttttttaaag tgttgggatt ataggcgtga 6600
   gttatcgtat ttcgttggga atagtagttt tttaaatggt tttttttaaa ttattgtatt 6660
   agggttggaa taggatgtag tttgtaaaaa gaattaggta gggttaggtg cggcggtttt 6720
   cgtttgtaat tttagcgttt tgggaggtta aggcgggagg atcgtttgag tttaggattt 6780
   taagattagt ttgggtaata taagttgatt tttatttttt aaataaatag aaatttataa 6840
   ttttaaaaaa agtatttagt tttttgtttt tgtcgtgaag ttttggaggt ttggtgtgta 6900
   ttttatgtcg tagttttttt cgggattggt tatattttac gggtttgata gttttgggtg 6960
   gttagtgttt gttgttttga cggtattgtt ttaggtagtg gtttggaata tttttagaga 7020
   tatatgaagt gtaagatgaa ggtttagaat gtagtaattt ttgtttgttt gtttgagacg 7080
   gtgtttggtt ttgttgtcga gattggagtg tagtggtgtt attttggttt attgtaattt 7140
   tcgtttttcg ggtttaagtg atttttttgt tttagttttt taagtagttg gaattatagg 7200
   cgtttattaa cgtgtttagt taatttttgt atttttagta gagacggggt tttgttatgt 7260
   tggttaggtt ggttttgaat ttttgatttt aaatgatttg ttcgtttttg ttttttaaag 7320
   tgttgggatt ataggtgtga gttattgtgt ttggttttag tttatatttt ttttttgaga 7380
   cggagttttg tagtgtcgtt cgggttggag tgtaatgtcg taattttagt ttattgtaat 7440
   tttcgttttt taggtttaag cgattttttt tgttttagtt tttcgagtag ttgggattat 7500
   agatgtttgt tagtacgttc ggttaatgtt tttgtatttt tagtagagac gtggttttta 7560
   ttatgttggt taggttagtt ttgaattttt gatatcgtga tttgtacgtt tttgtttttt 7620
   aaagtgttgg gattataggc gtgagttatt gtgttcggtt ttttagttcg tatttttaaa 7680
   tattatgaat tttattagta gaaattgtcg gtgtgtaggt tacgtttggg tcgtgtttgg 7740
   gttgtggtag gtatttagga agtgggttgg gagtgatatt tgagattatt attgaggagg 7800
   gtttgttgtg tttttatttg cgttgggggt tggggaagtt atagagaagc gaagagggtg 7860
   ggtttagttt ttggggagtt tttttttcgg ttggggagat gaggtacgtt ttatatttcg 7920
   tagaggaatt gagagtttgt tagtggtttt gtttttaagg agtaagtttg tggttagtga 7980
   gatcgggggt tgtttggagg tgggcggttg tggtttgttg ttgttgtagg aggatttagt 8040
   ggtagaattt gaaatttggt tggtaatagt aaaataggga aaggcgtggt ttatagaatt 8100
   aggttgttta ggtgtgtagc ggtcgtaggt gtgattgggt gttggtgtat atggtgtttt 8160
   gtgttttcgg ggtcgaagtt tattttagta tggaggaggc gttttggtag agtgaatgac 8220
   ggggtttatg tgttattcgt agtgtttggt ggtttttttt ttttggtttg gtttgttttt 8280
   aggtaggttt tttaggtagt tttttacgtg gtggtttttg tcgcgagttt ggatttaagt 8340
   ttagtagagg gagagagaga tttttttttt ttagtaggcg tttagagttt tgtggattgg 8400
   atcggttttt ttgggttgga aggcgtggtt ttttgtttgt ttaggtttag tcgtgttttt 8460
   ttttattttt tttttgggtt attgagtttt atagattgag agtgagcggt aaaggttgtt 8520
   ttgggatagt tgggtttgtt agtaggagat agcgtggagt cgggaggtag gtacgtagga 8580
   attcggtatt tagtagtagt tgtttgtttt ttgttagagt atttgggttt tggagtttgt 8640
   agtatttggt ttgaaggttt gaattttttc gttattgatt gattgtatga ttttgggtta 8700
   gttgtttttt tttttttagt tttggttttt ttaaaatgcg gatattggtt agatgtagcg 8760
   gttttatttg tattgttaat atttggggtg ggggagtggg aggatttttt gaggttagga 8820
   gttttaaagt agtttgggta atacggtaag attttgtttt tataaaaaat aaataaatta 8880
   ggtgtggtag tgtgtttttg tagttttatt tatttgggag ggattatttg agtttaggag 8940
   ttcgagatta gcgtgggtaa tatagtgaga ttttattttt ataaaaagaa aaaaaaatta 9000
   attgggtgtg gtggtatgtg tttgtagttt tagttgtttg agaggttgag gtaggaggat 9060
   tgttcgagtt taggaggtta aggttgtagt gagttatgat tgaattattg tattttagtt 9120
   tgagtaatag ggttagattt tgttttaaga taaaataaaa taaaaataaa aaaagcggat 9180
   ttggtatttg ttttgtattt tttatttttt tgaggtagga ttttgttacg ttgtttaggt 9240
   tggagtatag tggtataatt ttagtttatt gtaatttttg ttttttaggt ttaagcgatt 9300
   tttttgtttt agttttttaa gtagttggga ttataggtat tcgttattat atttagttaa 9360
   ttttttgtat ttttggtaga gatagggttt tattatgttg gttaggttgt ttttgaattt 9420
   tttagtttaa gttatttgtt tttttcgaat ttttaaagtg ttgggattat aggcgtgagt 9480
   tatcgcgttt ggttttttta gttttttttt gatatgtatt gaattgttta tttttttatt 9540
   ttttaaaaat acgtcggtgt tatggtgtta acgtgtggtt ttgtattttt ttttatatat 9600
   ttgttgggta tagttttggg aggggtggga acgtcgcgtt tgatggtgtg gggcgttaag 9660
   gaattgtttt ttgagaaggc gtagagtttg tattttcgta gtggttagtt tatgtttttt 9720
   aagtgtttgg tgtagggtcg cgtttaagag gagtaagtgg aggtgacggg gagttagtta 9780
   tttttattta tttttttcgt tttttttatt tttatttatt tttttttttt tttttagt 9838
<210> 46
   <211> 9838
   <212> DNA
   <213> Artificial Sequence
<220>
   <223> chemically treated genomic DNA (Homo sapiens)
<400> 46
<210> 47
   <211> 5976
   <212> DNA
   <213> Artificial Sequence
<220>
   <223> chemically treated genomic DNA (Homo sapiens)
<400> 47
   tagtattttt aatttttata tttttagtag agatggggat tttattatgt tggttacgtt      60
   ggttttgaat tttttttttt tttttttttt ttgtgataga gttttttgtt gtttaggttg     120
   gagtgtagtg gtagtgattt ttgtttattg tagtttgttt ttatttttta gattttagcg    180
   atttttttgt tttaggtttt taaatagttg ggattatagg tgtgtgttat cgtgtttagt     240
   taatttttgt atttttagta gagataaggt tttattatgt tggtaaggtt ggtttggaat     300
   ttttgatttt aagtgattcg tttattttag ttttttaaag tgttgggatt ataggtatga     360
   gttattgcgt ttagtttatt tattgattta ttgagatgag gttttattat gttgtttagg     420
   ttggtttgta attttgagat taagtaattt atttatttta attttttaaa gtgttaggat 480
   tataggcggt gagttatagt tggttatatt ttattttatt tatttatttt ttgagataga 540
   gttttgtttt gttatttagg ttggagtgta gtggtattat tttggtttat tgaaattttc 600
   gttttttggg tttaagcgat ttttttgttt tagtttttta agtagttggg attaaaggcg 660
   cgagttatta cgtttagtta atttttgtat ttttagtaga gacgggggtg ttattatatt 720
   ggttaggttg gtttcgaatt tttagatttc gtgatttgtt tgtttcggtt ttttaaagtg 780
   ttggaattat agtatgagtt attgtatttg gtttatttat ttattttttt gagatatagt 840
   ttttttgtgt tgtttatgtt ggagtgtagt ggtgcgattt tggttttcgg taatttttgt 900
   ttttcgggtt taagcgattt ttttgtttta gttttttaaa tagttgggat tataggcgtt 960
   cgttattata tttagttaat ttttttgtaa ttttagtaga gacggggttt tattatgttg 1020
   ggtaggttgg tttcgaattt ttgattttag gtgatttgtt tgtttcggtt ttttaaagtg 1080
   ttgggattat aggtttgagt tattacgttc ggtttatttt tttatttttt gagatagagt 1140
   tttgttttat cgtttaggtt ggagtgtagt ggcgtgattt tggtttattg taatttttgt 1200
   tttttaaatt taagggattt ttttgtttta gtttttaaat agttgggatt ataggcgttt 1260
   acgattacgt ttggttattt tttgtatttt tagtagagac gaggtttttt tatgttggtt 1320
   aggttggttt cgaatttttg attttaagtg atttgttcgt ttcggttttt taaagtggta 1380
   ggattatagg tatgagttat tatgtttggt tttatatttt aaaataaaat aattagatta 1440
   ttatttattg tttagtggaa ttgaaatatt atagtgatgg tcgggtacgg tgttttacgt 1500
   ttgtaatttt agtattttgg gaggttgagt tgggtggatt acgaggttag gagtttgaga 1560
   ttagtttggt taatatgtta aaaatttata tttattaaaa aaaaaaaata taaaaaatat 1620
   aaaaaattag ttgggtatgg tgttgtgtgt ttgtaatttt agttgtttag gaggttgagg 1680
   taggagaatc gtttaaattc gggagtggga ggttgtagta agtagagatt gtgttattga 1740
   attttagttt gggcgataga gtaagatttt attttaaaaa aaaaaaaaaa gtataaatta 1800
   gtttttatta gttgggaatt attttttttt ttttttatta tttttttttt ttttgagttt 1860
   ttattattat tttatttttt tataaatttt atgtttttat atttgaaagt tttttattga 1920
   ttaatttttt atattttttt gtatatatat gtatattaat tgagatgaaa aatatttttt 1980
   ttgtgattgt aagattttag ttgttaaaaa ttattttgga ggagttattg ttataattat 2040
   tataagaata gaatgttttt ataaatatgt gatcgtatat tttttttttt ttttttaaga 2100
   cggagtttcg ttttgtcgtt taggttggag ggtagtggcg cgatttcggt ttattgtaag 2160
   tttcgttttt cgggtttacg ttattttttt gttttagttt tcggagtagt tgggtttata 2220
   aggcgtttat tattacgttt agttaatttt tttttttttt tttttttggt atttttagta 2280
   gagatagggt tttatcgtgt tagttagggt ggtttcgatt ttttgttttc gtgatttttt 2340
   tgtttcggtt ttttaaagtg ttgggattat aggcgtgagt tatcgtgttt ggtcgatcgt 2400
   aaatattttt ataattgtta aaaagaaata tttaataaga tagtagtttg ggagtggcgg 2460
   tttacgttta taattttagt attttgagac gttgaggtgg gaagattgtt tgaggtaagg 2520
   agtttaagat ttatttgggt aatatagtaa gattttgtcg ttataaaaaa tttttttttt 2580
   tttttttttg agatagagtt ttgttttgtc gttaggttgg agtgtagtgg ttgattttag 2640
   tttattgcga ttttcgtttt tcgggtttaa gcgatttttt tgttttagtt tttcgagtag 2700
   ttgggattat aggcgcgtgt tattaagttt agataatttt tgtgttttta gtagagacgg 2760
   ggttttatta tgttggttag gttggtttta aattttcgat tttaggtgat tcgttcgttt 2820
   cggtttttta aagttttggg attataggta tgagttatcg cgtttagttt ttataaaaaa 2880
   ttttttttaa aaaaattagt taggtgaggt ggtgtgcgtt tgtagtttta gttatttgag 2940
   gtgggaggat ggtttgagtt taggaatttg aggttatagt gagttatgat cgtattatcg 3000
   tattttagtt tgggtgatag aataagattt tttttttttt tttttttttt tttttttttt 3060
   tttttttttt gagataattt cgttttgtcg tttaggttgg agtgtaatga cgcgatttcg 3120
   gtttattgta attttcgttt ttcggattcg agcggttttt ttgttttagt tttttgagta 3180
   gttggagtta taggcgtttg ttattacgtt tagttaattt ttttgtattt ttagtagaga 3240
   cggggtttta ttatgttggt taggatggtt tcgatttttt gattttatga tacgtttatt 3300
   tcggattttt aaagtgttgg gattataggc gtgaattatc gcgttcggtc gattttgtat 3360
   ttaaaaaaaa aaaagataaa agaaaggaaa aaaaaaaaaa aaaaaaagaa aaggtggtat 3420
   ttatggtgtt ttggtgaaag aaatagggta tatttttttt tattttaaat aggtaagatt 3480
   atttttagga gattttatgg ttgagaatta tatttttgag gggggagggg aaaaaagaga 3540
   attatttttt tttttttttt ttttaagagg gtgattttat tgtaaaagga gaagtgaaga 3600
   atgagaattt gtattgtagg tgttttttta ggaagattaa ttttgggaaa gttgaggttt 3660
   ttgaaattaa attttttata ttttttaagt tttagttttc gcggagagtt tttatgtttt 3720
   ttattttgaa agttgtagat tttgacgatt ggagagaagt gatgtgggat tgtttaacgg 3780
   acgggtgttt gcgattttag gatttagcgt tagggattta ataagttttg tgattttagt 3840
   taagtggtaa gatgtttatt atttgtggaa atgggaattt tagcgattgg gattttgtta 3900
   atttttttgg tttttttata agtttatttt tggatttagt tttgttttta cgagtttttt 3960
   tagttttttt aaatataacg aggtgggtat taaatttgtt tttttatttt ttattttttt 4020
   tttttttttt ttcgaagagg aggaggagga gatttaaagt attttatgag ttttatttcg 4080
   ttttatagta gttttcgtag cggtaatttg tataaatttt tttttaagtt ttattttatt 4140
   atcgtttatt cgtagatttt aggtttttta cgataaagta cggttatttg gttagtatag 4200
   acggtttttc gaggattttt tttggagaag aattatttag gttttaacgg tttggttggt 4260
   tattagtttt gtggtgaagt ttgaaaagta aattagtaat aatagtaata ataataatat 4320
   ttttatggcg cgttgtgttt atttgtacgt atgtgttata acgatcgcgg gacgtagtat 4380
   ttggtagtag aaatagttgt tggatagcgg taaggagcgc gtcgtttttg taggggaagg 4440
   ggttgggtta gtcgaataag tcgtggaaat gggaatttgg gggataggaa agtagggtta 4500
   gatagtttgt taatagttta attgaaagaa aaggttttat ttttttttta agagattttt 4560
   gttttttttt gaaattttgt atagttttgg aaagtttaat ttttaattta ttttgttcgg    4620
   tttttttgtt tttttttgga aacgaggttc ggtttttttt acgcgttatt tttcggcggt    4680
   tgggttttag gtaggaagtt tttgattggc ggagtcgtag aagtatcggg tgaaaagagt    4740
   tttattggtt gcgtatcgga gttcgggcgt agttagtgtc gtacggtaaa atggcggttt    4800
   ttagttgttt tgggacgttt gggttcgggg agataaattt cgatttggaa gagatcgttg    4860
   attatgtggg tcgtattgga ggtgttcgcg gggcgggttt attgttattt tcggttatag    4920
   gaagtttgtg tttcgtacga taatatggcg gcgtttagtt gtatgaaggc ggaaattttg    4980
   tgattttcgg ttcgtagtgg ggtttgcggt gggagtggga aggaaggcgg agggaattat    5040
   gcgaggtttt gagaattgcg gcgagggtcg tttcgagaga cggtttttga ggtgggggtc    5100
   ggacggtgcg gggattagag gcgggggcgg ggatatagag ggagaggtat tggggagagt    5160
   tgggcgttga gtttgagggg aaagttgtgg tatttttttg tttttgaaag ggagtggagc    5220
   ggtcgggggt tgaattgggg gagggggatt tggcgggaag cgtatttagc ggatcggtcg    5280
   gatttacggt tgggatttaa gaggaatcgc gagagcgtag cgcggtgcgg ggggattttt    5340
   gattagttac gggttatcga gagaatgggg aaaattaggg tggggtgaaa gggaggggat    5400
   ttgggtaaaa tatttttaat ttaaagtttt agtttgagga gatttatcgg agggtgaaat    5460
   atggagataa tttaaggagg agaaaagaga acggattgtt gggagggaat tttttttatt    5520
   ttagagagtt taggagttgg ggtgattatt taggataggg tgggtgtggg gttgatttgt    5580
   ttgagagtag agggggtaaa tatatgaggt ggcgtttata ggagtgtgtt gtttgtgtaa    5640
   gggggtgtta ggacgatttg gggtgtagtg tgggaggaag aattaatttg ttaattttaa    5700
   gaaatagggg ggaaatgtaa tagaaagatc ggaaatcgac gtgaggggtg gagtagaagg    5760
   tggtaattta aggtgtgggg gtggtttgtt ttttttttat attatttgaa gggttttaaa    5820
   aagtatatag aattaatggt tattgaaatt aggtggtgaa gatagtgttg gttagggttg    5880
   gatgttattg aaatttaagg agtttagaaa atgtgtgagg tgaagggatg gggttatgtt    5940
   ggaggggttt attttgaggt tttatttttt tttagt                              5976
<210> 48
   <211> 5976
   <212> DNA
   <213> Artificial Sequence
<220>
   <223> chemically treated genomic DNA (Homo sapiens)
<400> 48
   gttggaaggg gatggggttt tagaatgagt ttttttagta tagttttatt tttttatttt     60
   atatattttt tgaatttttt gggttttagt aatatttagt tttgattaat attgttttta    120
   ttatttgatt ttaataatta ttagttttat atgttttttg gggtttttta agtggtgtgg   180
   ggggggggta aattattttt atattttaag ttgttatttt ttgttttatt ttttacgtcg     240
   attttcggtt tttttgttgt attttttttt tattttttag agttggtaag ttgatttttt    300
   tttttatatt gtattttaaa tcgttttgat atttttttgt atagataata tatttttgtg    360
   ggcgttattt tatgtgtttg ttttttttgt ttttaaataa gttaatttta tatttatttt    420
   attttgaatg attattttaa tttttagatt ttttgaggtg aaagaggttt ttttttaata   480
   gttcgttttt tttttttttt tttgaattat ttttatattt tatttttcga tgagtttttt    540
   taaattgggg ttttaggttg aagatatttt atttaagttt tttttttttt attttatttt    600
   aatttttttt attttttcgg tgattcgtaa ttgattaaaa gttttttcgt atcgcgttac  660
   gttttcgcga ttttttttag attttaatcg tgggttcggt cggttcgtta gatgcgtttt   720
   tcgttaaatt tttttttttt agtttagttt tcggtcgttt tatttttttt tagggatagg    780
   aaggtattat agtttttttt ttagatttag cgtttagttt tttttaatat tttttttttt    840
   atattttcgt tttcgttttt gattttcgta tcgttcggtt tttattttag aaatcgtttt    900
   tcgaggcgat tttcgtcgta atttttagaa tttcgtatgg tttttttcgt tttttttttt    960
   atttttatcg taggttttat tacggatcgg aagttataga gttttcgttt ttatgtaatt   1020
   aagcgtcgtt atattgtcgt acggaatata ggttttttgt ggtcggaggt gatagtaggt   1080
   tcgtttcgcg aatattttta gtgcggttta tatagttagc ggtttttttt aggtcggagt   1140
   ttgttttttc gaatttaggc gttttaaagt agttgggggt cgttattttg tcgtacggta  1200
   ttggttacgt tcggatttcg gtgcgtagtt agtggagttt tttttattcg gtgtttttac   1260
   gatttcgtta attagaaatt ttttgtttgg ggtttaatcg tcggagagta gcgcgtaggg   1320
   aggatcgagt ttcgttttta aggaggggta ggggaatcga atagggtgga ttaggaattg  1380
   ggttttttaa agttgtgtag agttttaggg aagggtagaa gttttttaaa agggaagtaa   1440
   aatttttttt tttagttggg ttattggtaa gttatttggt tttgtttttt tgttttttag   1500
   gtttttattt ttacggttta ttcggttgat ttagtttttt tttttgtaag ggcggcgcgt   1560
   tttttgtcgt tgtttagtag ttgtttttat tgttaggtgt tgcgtttcgc gatcgttata   1620
   atatatgcgt ataaatgagt ataacgcgtt ataaaagtgt tattgttatt attattgttg   1680
   ttgatttgtt ttttaagttt tattatagaa ttaatgatta gttagatcgt tgggatttga   1740
   atggtttttt tttagagggg gttttcgaag ggtcgtttgt gttgattagg tggtcgtgtt   1800
   ttgtcgtggg aggtttaggg tttgcggatg ggcgatgatg gggtggggtt tggaggagag   1860
   tttgtgtaaa ttgtcgttgc gagggttgtt gtgaggcgaa atgaggttta taaaatattt   1920
   tgggtttttt tttttttttt ttcggggaga ggggaaaaaa gaatgaagga tgaagaaata   1980
   gatttgatat ttatttcgtt gtgtttaggg aggttggagg gattcgtgga ggtaaggttg   2040
   ggtttagagg tgagtttatg aggaaattag aaagattaat agggttttaa tcgttaagat    2100
   ttttattttt ataagtggtg ggtattttgt tatttaatta gggttataga gtttattaga    2160
   tttttaacgt tgagttttag aatcgtaaat attcgttcgt tgaataattt tatattattt    2220
   ttttttagtc gttaaagttt atagttttta aaatggggaa tataaaaatt tttcgcgagg    2280
   attgggattt ggaagatgta agggatttaa ttttaagaat tttaattttt ttaaaattgg    2340
   tttttttaag gaaatatttg tagtataggt ttttattttt tatttttttt tttataatag    2400
   agttattttt ttaagaaaaa aaaaaaaaag aatagttttt tttttttttt ttttttttta    2460
   aaagtatagt ttttaattat gaaatttttt ggggtaattt ttatttgttt aaaataaagg    2520
   gaaatgtatt ttgttttttt tattaaggta ttatgaatat tatttttttt tttttttttt    2580
   tttttttttt tttttttttg tttttttttt ttttaaatgt agggtcggtc gggcgcgatg    2640
   gtttacgttt gtaattttag tattttgggg gttcgaggtg ggcgtattat ggggttaaga    2700
   gatcgagatt attttggtta atatggtgaa atttcgtttt tattaaaaat ataaaaaaat    2760
   tagttgggcg tggtggtagg cgtttgtagt tttagttatt taggaggttg aggtaggaga    2820
   atcgttcgaa ttcgggaggc ggaggttgta gtaagtcgag atcgcgttat tgtattttag    2880
   tttgggcgat agagcgagat tattttaaaa aaaaaaaaaa aaaaaaaaaa aaaaaggaaa    2940
   aaaaaagggt tttgttttat tatttaggtt ggaatgcggt ggtacggtta tagtttattg    3000
   taattttaaa tttttgggtt taagttattt ttttatttta agtagttagg attataggcg    3060
   tatattattt tatttggtta atttttttaa aaaaagtttt ttgtggaggt tgggcgcggt    3120
   ggtttatgtt tgtaatttta gaattttggg aggtcgaggc gggcggatta tttgaggtcg    3180
   gaagtttgag attagtttgg ttaatatggt gaaatttcgt ttttattaaa aatataaaaa    3240
   ttatttgggt ttggtggtac gcgtttgtag ttttagttat tcgggaggtt gaggtaggag    3300
   aatcgtttga attcgggagg cggaggtcgt agtgagttga gattagttat tgtattttag    3360
   tttggcgata gagtaagatt ttgttttaaa aaaaaaaaaa aaaaggtttt ttatagcgat    3420
   agggttttat tgtgttgttt agatggattt tgagtttttt gttttaagta atttttttat    3480
   tttagcgttt taaagtgttg ggattatggg cgtgaatcgt tatttttaga ttattatttt    3540
   attaaatatt tttttttaat agttataaaa atgtttacga tcggttaggt acggtggttt    3600
   acgtttgtaa ttttagtatt ttgggaggtc gaggtaggag gattacgagg gtaggaaatc    3660
   gagattattt tggttaatac ggtgaaattt tgtttttatt aaaaatatta aaaaaaaaaa    3720
   aaaaaaaaaa ttagttgggc gtggtggtgg gcgttttgta gatttagtta tttcggaggt    3780
   tgaggtagga gaatggcgtg aattcgggag gcggagtttg tagtgagtcg agatcgcgtt    3840
   attgtttttt agtttgggcg atagagcgag atttcgtttt aaaaaaaaaa aaaaaagtgt    3900
   acgattatat gtttataaga atattttgtt tttataataa ttgtaatagt aattttttta    3960
   gaataatttt taataattag agttttataa ttataagaaa gatatttttt attttaatta    4020
   atatatatgt atatgtagga agatgtaaaa gattggttaa taaaggattt ttaagtataa    4080
   aagtataaag tttgtgggga agtggagtag tagtaggagt ttaaggaaaa aagaggtgat    4140
   gaaagaaaaa aggaatgatt tttaattggt aagaattaat ttatattttt tttttttttt    4200
   tgagatggag ttttgttttg tcgtttaggt tggagtttaa tggtataatt tttgtttatt    4260
   gtaatttttt attttcgggt ttaagcgatt tttttgtttt aattttttga gtagttggga    4320
   ttataggtat ataatattat gtttagttaa ttttttgtat tttttgtatt tttttttttt    4380
   agtggatatg ggtttttggt atgttggtta ggttggtttt aaatttttga tttcgtgatt    4440
   tatttaattt agttttttaa agtgttggga ttataggcgt gaagtatcgt gttcggttat    4500
   tattatgata ttttaatttt attggataat aaatggtgat ttaattgttt tattttaaaa    4560
   tgtggggtta ggtatggtga tttatgtttg taattttatt attttgggag gtcgaggcgg    4620
   gtagattatt tgaggttagg agttcgagat tagtttgatt aatatgagga aatttcgttt    4680
   ttattagaga tataaaaaat agttaggcgt ggtcgtgggc gtttgtaatt ttagttattt    4740
   gggagttgag gtaggggaat tttttgaatt tgggaggtaa aagttgtagt gagttaagat    4800
   tacgttattg tattttagtt tgggcgatag agtaagattt tgttttaaaa aataaaaaaa    4860
   taggtcgggc gtggtggttt aaatttgtaa ttttagtatt ttgggaggtc gaggtaggta    4920
   gattatttga ggttaggagt tcgagattag tttgtttaat atgatgaaat ttcgttttta    4980
   ttaaaattat aaaaaaatta gttgggtgta gtggcgggcg tttgtagttt tagttatttg    5040
   ggaggttgag gtaggagaat cgtttgaatt cgggaggtag aggttgtcgg aagttaagat    5100
   cgtattatta tattttagta tgggtaatat agagagattg tgttttaaaa aaataaataa    5160
   ataggttagg tgtagtggtt tatgttgtaa ttttagtatt ttgggaggtc gaggtaggta    5220
   gattacgaag tttaggagtt cgagattagt ttggttaata tggtgatatt ttcgttttta    5280
   ttaaaaatat aaaaattagt tgggcgtggt ggttcgcgtt tttagtttta gttatttggg    5340
   aagttgaggt agaagaatcg tttgaattta ggaggcggag attttagtga gttaagatgg    5400
   tgttattgta ttttagtttg ggtgatagag taagattttg ttttaaaaaa taaataaata    5460
   aaataaaatg tggttagttg tggtttatcg tttataattt tagtattttg ggaagttgag    5520
   gtgggtggat tgtttgattt taggattata gattagttta ggtaatatag tgagatttta    5580
   ttttaataaa ttaataaata ggttgggcgt agtggtttat gtttgtaatt ttagtatttt    5640
   gggaggttga ggtgggcgga ttatttgagg ttaggagttt tagattagtt ttgttaatat    5700
   gatgaaattt tgtttttatt aaaaatataa aaattagttg ggtacggtgg tatatatttg    5760
   tagttttagt tatttggggg tttgaggtag gagaatcgtt agaatttggg aagtggaggt    5820
   aggttgtagt gagtagagat tattgttatt gtattttagt ttgggtaata ggagattttg    5880
   ttataaaaaa aaaaaaaaaa aaaagagttt aagattagcg tggttaatat ggtgaaattt    5940
   ttatttttat taaaaatata aaaattaggg gtgttg                              5976
<210> 49
   <211> 6192
   <212> DNA
   <213> Artificial Sequence
<220>
   <223> chemically treated genomic DNA (Homo sapiens)
<400> 49
   gttttaatcg gagaaatggg aaattggatt gattaagatt tgaaaatgaa ataggtaatt     60
   gttgaagagt attggtttat ttaaaaagcg tgtatttgtt ttttgttaga tagttataga    120
   gtttgttatg aaaagtttaa gttttggtta ggtattgtag tttacgtttg taattttagt    180
   attttgggag gttgaggtgg gcggattatg aggttaggag attaagatta ttttggttaa    240
   tacggtgaaa ttttattttt attaaaaata taaaaaatta gttgggtatg gtggcgggcg    300
   tttgtagttt tagttattag ggaggttgag gtaggagaat ggcgtgaatt taggaggcgg    360
   aggttgtagt gagtagagat cgcgttattg tattttagtt tgggtgatat agcgagattt    420
   tgttttaaaa aaaaaaaaag aaaagtttaa gttttttttt taggttgtta gtatgttttg    480
   tggtaatttt atttttatta gttttggaaa ggagggtaaa ggagaagtag agttgaaaat    540
   taaataagtg atggttatgt ttttttaaag gattttagat ttttttttat taaatatata     600
   tatagagagg gtaaatttgt aaaattaaaa gaatttttgg gtataatagt ggttttaagt    660
   ggtaagattt ttagtattgt ttatagtatt ggtaaattaa atgttttata agaattagga    720
   atagagtcgg gtacggtggt ttacgtatat aattttagta ttttgggagg ttgaggcggg    780
   tagattattt gaagttagga gtttaagatt agtttggtta atatggtgaa atttatcgtt    840
   attaaaatta taaaaattag ttaggtgtgg tggtaggtat tcgtagtttt agttatttgg    900
   gaggttgagg taggagaatt gttcgaattc gggaggtagt gagtcgagat tgcgttaatg    960
   tattttagta tgggcgatag agagagattt cgttttaaga aagaaaaaag aattaggaat   1020
   tgaggatatt ttttaatttt agtttgtggg tatttttaat ataaaataat ttagagaaat   1080
   ttagatttta agatgtttta gtattttagt aaattaaatg tggtaaggta agaaaggaga   1140
   ggtttttttt tcgagatatt tgttaaattt tagaatattt gatagtgaga aggttagaat   1200
   tagtttttgt tattttttat gttttataat taagatattt tttttttgaa ttatttatat   1260
   tgttaataga aattggtatg ggggaatata ttaatttttt taagtttaat ttttgtaata   1320
   gacgtgtaga ataagatagt aagaaatatt aagcggtggt tttttgtttt tgaaggaagt   1380
   gaaatattag taagttgttg aaaattttta gaggtgtttt aaggagatgg gagaggagga   1440
   atgaggtttt aagttgaaat tatttttttt tttttatttt taattttaat taaatttaaa   1500
   aatgtatgga tgattagtgg agttatatag gtttaatata ggtttaatag agtatacgat   1560
   ttggggtatg ttataaattt ttagtattta tttttgtgaa atggaggtta gtagtttgta   1620
   ttttataaag gttgtagtga atattaaacg agataataga tgtaatttgt gtttaatatg   1680
   atattatata ggtagttaaa tgtaagatgt tttttagttt tttttttttt attttatttt   1740
   attttatttt tggattataa attttttcgg agattttatt atttattgtt tttcggtcgt    1800
   agaggtatag gcgttaacgt tggtttttat ggttttttta ttggagtggg atttgtttga   1860
   atttgtaggt gaaaagttag agcggagttt tcggttaata aaattaaatt tagggaagcg   1920
   aggatgggtt tttagttaaa agtttggtgg cgtttgttag ttatagtgcg tttttttttt   1980
   attaacgtat tgagagggtt cgattagatt atttttgatt atttaatttg acgagagaag   2040
   cgtaaaggtg attatagatg aagaggttgg tagtcgtttt aggaggttga ggtcgtgagg   2100
   gagtgggaat tttaatggaa ataggttttt tttcggaggg gatagtcggg aacgatcgat   2160
   ttattttttt ttttttaagt tttcgtagtt taaggttggg gattttttaa gggtttcgtt   2220
   atttttttta ataagtttaa gtgggattag aggtgttggt aggcgggaag tggggattcg    2280
   ggggtatagt taaatattag agttcgggtt tcggaatgtg gttaaagagg ggttatcggg   2340
   gggaggggtc gggtggggag gatttgggga ggggcggggt ttaggagggg gtaagaggtc   2400
   ggggttttta gtttcggaga ttcggggttt ttattcggtt tttagtcgta tgttgagcgt   2460
   tttttttagt cggggttttc gtcgtatagc ggggtagttt tatttttttt atttggttcg   2520
   gttgttttgt ttgtttcgag gacgttattt ttttgaggtt tgattaatta atcgtcgttg   2580
   ggcgaggttt tttttaggcg ttttcgtttt cgttttttcg tttagattaa ggtagtcgga  2640
   atagagtttg tcgatgtagg ggattggggt agaggggtgg agttcgggtg ttcgggttat   2700
   tttgtatagt tttcgttaag attttgtttt ttaggaagat tttaagttta tatttttttt   2760
   gcgttgttat atgtgtatta ttttggtttt gttttttttt tttgagacgg agtttcgtat   2820
   ttgttgttta ggttggagtg taatggcgcg atatcggttt atcgtaattt tagtttttcg   2880
   gttttaagtg atttttttgt tttagttttt cgagtagttg ggattgtagg tatgcgttat  2940
   tatgttcggt taatattgta tttttagtag agatggagtt tttttatgtt ggttaggttg   3000
   ttttcgaatt ttcgatttta ggtgatttgt tcgtttcggt tttttaaagt gttgggatta   3060
   tagaagtgag ttatagcgtt cggtttatat tatttttatt gagttaattt ttgtttattt   3120
   ttatagtgtt atgggtatat ttagggattt gttaatggat gaatgcgaag aaatttggaa   3180
   attaattttt agatattagt ttagttttat ttattgagtg tttattatat gttagggatt   3240
   ttggtaggtt ttgaaataaa aatatttaag atttaatttt tatttttacg tttaggttaa   3300
   atatatttat aattatagta ttatggggat aggattcgga taagtgtgta ggtttattgg   3360
   gagaatataa aagggggtat tttgaagaga agaggttttt ataaaacgtt attattttat    3420
   attggttgaa ttgttagatt tttttaattg ttagatttta aattaaatat tggacgggag   3480
   tagtggttta tatttgtaat tttaatattt taggaggtta aggtaggagg attttttgag  3540
   tttaggaggt tgaggttgta gtgaattatg attatgttat tgtattttag tttgggcgat   3600
   agagcgagat tttgtttaaa aataagataa gaaaataaaa aatgaaatta tatgaaaaag   3660
   gatatatata ttagagttaa tcgttagttt ttattatagt tgtgttttga ttaatataga    3720
   ttgtttaaaa gggatgattt aatgaataat tttttttatt aaacgttgtt ttaaatgttt    3780
   aaattttttt tgagataata tttaattttt ttttttacgt tttttttgtt tgtttttgtt    3840
   tttgtttttg tttttgtttg agacgaagtt ttgtttttga cgtttaggtt ggagtgtaat    3900
   ggtgcgattt gggtcgattg taattttcgt ttttcgggtt taagcgattt atttgtttta    3960
   gttttttaag agttgggatt ataggcgtgc gttattacgt tcggttaatt tttgtatttt    4020
   tagtagagat agggttttat tatgttggtt aggttggttt taaattattg attttagatt    4080
   atttgttcgt ttcggttttt taaagtgttg agattatagg ggtgagttat cgttttcggg    4140
   gtttttgttt acgtttttat aaataaaata tgaggtggga ggattgtttg agtttaggag    4200
   attaaaattg tagtgagtta ttatcgcgtt attgtatttt agtttgggag atagagcgaa    4260
   acgttgtttt aaaaaaagaa aaaagaaata aggaaggaaa gtgtttttcg aatgaataaa    4320
   tatgagtaga taattatttt tagtttatat aaaatgtttt tttttaagag aggaattttt    4380
   tgattatttt tattattatt tttttaattt tagatgttta ttttttagga ggaaaaaaat    4440
   aaattttttt atagtagtag tattaaaata aagatgaata tgttgttaag aggtatttga    4500
   ataattttta atagtgtcga gtcgtatttt tttttttttt ttcgatgtat ttgagatttt    4560
   aatgtttatt atatttattt aagcgttttg ttttgagatt ttaaattaat ttgtatagtt    4620
   gaagtaggaa atagaatggg tggaaatatg cgggtgtttg tattagtatt atgtatttta    4680
   tatgcgaata ggaatgtttt aaaaatttaa tatagtatat atgcgtgtgt atcgttatgt    4740
   aaaataagtt tttattggtt ttatatttga acgcgcgaat gtagttttta aaagttaaaa    4800
   ataattttta ggttgaatcg cgcgatagaa cgaaagcggt tttaaattta gagattatag    4860
   ttttcggtat gtttcgacgt tatacgtgat tagcggtaat tagaattata tttttcggcg    4920
   tgttttggtt ttacgtgtga tttgttgcgg aacgaattat atttttcggt aggttgcgga    4980
   agggggtcga gtagaaggat cgtcgtttcg gtttttcgcg atttttcgaa ggtgggtagg    5040
   ttttattttg tggaggatgg aggtgatcgg ggacgtcggg gtattagaat ttggcgagat    5100
   tcggatttta aagtcgtgtt tgttgcgtcg taattatagt cgcgaatagt acggcgtggt    5160
   cgttttttgt ttcgaagatt tgaggagtaa gggttggttc gggatttcgg gcgagggtgt    5220
   gggggagtta gggtcgggtg aggacgttcg gcgggggcga ggcggggtta ggagaggcga    5280
   tttggggatt tttgtatttc gggtggaggt ttgggataga aacggttcga gtgggtatag    5340
   cgatttatag atttaaggcg tttgagcgta attaatttcg tgtgagattt tacggtggtg    5400
   ggaagatagt taggaagtaa tgtgtaaatt aagttagatt cggtttaggt tttttgtttg    5460
   aaattatttc gatgttggat taaacggatt ttattgggtt tttgttattt cgttatattt    5520
   agatggtata agaattttta gttgagttta cggcggtgtg gggaggggtt tttatcgttc    5580
   gttttggtag gaatttatgt aggaaagagg gagttgttat tgattatttt tggaggttta    5640
   gataaggaac gtgagttgta agtgacggag tcgacggaag tttcgagttc ggttttatat    5700
   ttttattagt tgtttggttt tgtatttaat tagtttttaa tttttagttt ttttattagt    5760
   aaattgggta taatatttat agttttgttt taaagggttg ttggtttttt taaataagat    5820
   gatatattta agtgatttat aagttttatt agttagtagt aataaatcgt ttatttaaag    5880
   aagttttata ggttatatgt gtgagttagt tttaggtatg tagttatagt gtgtgttaga    5940
   gggtatagtt gttttttttt agttattaat agaaattata tggaaatttt tagagattta    6000
   tgaatagtta aagtattata aatgttttgg taatatggat tttgtatata agtatttatg    6060
   tatttttttt tttgatatta gattatttta tttttaattt ttttttagtg tcgtggttgg    6120
   atgtgcgtgg tatagaggtt ggtgtttttt gtagtttagc gtatatatgt tttgttgttt    6180
   tattgggtgg ta                                                        6192
<210> 50
   <211> 6192
   <212> DNA
   <213> Artificial Sequence
<220>
   <223> chemically treated genomic DNA (Homo sapiens)
<400> 50
<210> 51
   <211> 5989
   <212> DNA
   <213> Artificial Sequence
<220>
   <223> chemically treated genomic DNA (Homo sapiens)
<400> 51
   ttttaggtat aaataatttt tttgttttag ttttttgagt agttggtatt ataggtgttt       60
   gttattatgt ttagttaatt tttttgtatt ttagtagaga tgggttttat tatgttggtt      120
   aggttggttt taaatttttg attttaggtg atttgtttat tatagttttt taaagtgttg      180
   ggattatagg cgtgagttat tgtatttcgt ttatgataaa gttttattaa tatttttagg      240
   gtggtaaggg agttatggaa agttaagtat attgtaattt tgaaaaaaaa aaaatggggt      300
   tttgttagaa aggaagaaag ggaggataaa tgttggataa gacggttggg cgtggtggtt      360
   tatatttgta attttagtat tttgggaggt taaggtgggt ggattatgag gttaagagtt      420
   taagattagt ttggttaagt tggtgaaatt tcgtttttat taaaaatata aaaattaggt      480
   tatgtatggt ggtttatgtt tgtaatttta gtattttggg agggtaaggt gggtagatta      540
   taaggttaag agttcgagat tagtttggtt aatatggtga aattttattt ttattaaaaa      600
   tgtaaaaatt agttgggtgt ggtggcgggt gtttgtaatc gtagttattt gggaggatga      660
   ggtagagaat tgtttgaatt taggaggcgg agtttgtggt gagtcgagat cgcgttattg      720
   ttttttagtt tgggcgatag agtgagattt cgttttaaaa aattaaaaaa taaatattgg      780
   ttaagacgag tagtaatgtt tgttatattt tttgagagat tattgaattt tagttatatg      840
   ttagtgttat ttggggagat ttgggtttga attttttttt ttttttgggg gattttttgg      900
   ttaaaaatgt atttgaaatt gggtgtggtg gtttatgttt ataattttag ttatttaaga      960
   ggtttaggtg ggaggatttt ttgaggatag gagttgtaga ttattttgga taatatagta     1020
   agattttgtt attttttttt tttttttttt tttgagatag agtttcgttt tgttgtttag     1080
   gttggagtgt agtggtacga atttcggttt attgaaagtt ttgtttttcg gatttatgtt     1140
   atttttttgt tttagttttt tgagtagttg ggattatagg tattcgttat tatgtttagt     1200
   taatttttcg tatttttttt ttagtagaga cggggtttta tcgtgttggt taggatggtt     1260
   ttgatttttt gatttcgtga tttgttcgtt ttagtttttt aaagtgttgg gattataggc     1320
   gtgatatcgt gttcgattaa gatttgtttt ttaataaata gggttagttg taatggttta     1380
   tgtttataat tttagtattt tgggaggtta aggagggtag atggtttgag gttaggagtt     1440
   cgagattggt ttggataata tggtgaaatt ttatttttat aaaaaaatat aaaaattagt     1500
   taggtatggt ggtgttggtt tgttgtttta gttatttggg aagttgaggt aggagtatta     1560
   ttttagttta ggaggttaag gttgtagtga gtcgagattg tattattgta ttttagtttg     1620
   agtaatatgg tgatattcgt tttaaaaaat aataataata aataatgaat aaatgtaatt     1680
   tattttaaag tgaaatttgt attttttttt ttagtttttg tataaggaaa aattattgtt     1740
   tttttatttt ttttaatttt tttttatttt attatttgat aaaattttat tttataaagt     1800
   atgagagtaa agttattttt tttataatat ttttttttag tttttttagt ttaaagtgaa     1860
   ttttttaatt ttttaatttt aaaatgaagt tgttaatgtt ttgtgtagag tatatatttt     1920
   attttatatt atggttagtt gttgtataag attagatatt ttttaaatag agaaattatt     1980
   ttttatttat tataattata aaatgtttta tttttgttat ttatattata aatttttatg     2040
   gttttaggtt ttgtttaaaa tataaatggg tggtatggac gtcgtattat tttattaaat     2100
   tgtgatattt tggggattag gaatttttgg ttaagaggga gatttacgtt tataatttta     2160
   atattttatt tatttattta tttttgagat ggcgtttcgt ttttgttgtt taggttggag     2220
   tgtaatggcg tatttttagt ttatcgtaat tttcgttttt taggtttaag cgattttttc     2280
   gttttagttt tttaagtagt tgggattata ggtacgtgtt attacggttc ggttaatttt 2340
   gtatttttag tagagatgga gtttttttat gttggttggg ttggttttaa atttttgatt 2400
   ttagatgatt cgttcgtttt ggttttttaa agtgttggga ttgtaggtgt gagttattgc 2460
   gttaggtttt attattatta ttattttttt tgagattaag ttttgttttg ttgtttaggt 2520
   tggagtgtag tggtattatt ttggtttatc gtaattttcg ttttttgggt ttaagtagtt 2580
   tttttgtttt agtttttaga gtagttggta ttatagatgc gtattattat atttatttaa 2640
   tttttgtgtt tttagtagag atagggtttc gttatgtttt ttaggttggt tttaaatttt 2700
   tgggtttaag cgatttattt attttagttt tttaaagtgt tgggattatt ggtatgaggt 2760
   atagagttcg gtttgtaatt ttaatatttt gggaggttaa ggtaggagga ttatttgagt 2820
   ttaggagttt aagatcggtt tgggtaaaat agtgatattt tatttttata agaaataaaa 2880
   aaattagtta agtatagggg tatgtatttg tgtttttcgt tattcgcgag gttgtggtgg 2940
   gaggattatt ttagtttagg aggttgaggt agtaatgagt attgatggtg ttattgtatt 3000
   ttagtttggg tgatagggta agattttatt ttaaaaaaat aaataaaaag tgagtttgtt 3060
   tatttttttt atgtttttta gtattttgtt tttgaatttt agttattatt tttatagatt 3120
   ttttaataaa aaggttgttt taattaacgt taattaatat atatggtata taagaagatt 3180
   tttgttttta agggttttat aaatttttag agttaaatgt gttttattat tagtataaaa 3240
   ataaatggtg ttagttgggt gtagtgattt atatttgtaa ttttagtatt ttaagaggtt 3300
   gaggtaggtg gattatttga ggttaggagt ttgagattag tttggttaat atggtgaaat 3360
   tatattgtta ggtttttgag tttaagttaa gttatcgtat tttttgtgat ttgtacgtat 3420
   atatttagat ggtttgaagt aattgaagat ttataaaaga agtaaaaata gttttaattg 3480
   atgatatttt attattgtga tttgtttttg ttttattcga attgattaat gtattttgta 3540
   atttttttta tttttaagaa ggttttttgt aatttttttt atttttgaga atgtattttg 3600
   tgagatttat ttttgtttat aaaatattgt ttttaatttt attatttatt ttaaaatttg 3660
   taagaattaa tgataattta ttattttttg ttgatttttt tttcggattt agttcgtttg 3720
   tatttaggtg aaataaatag ttatgttgtt tatataaagt ttgtttggtg gtgtttttat 3780
   atagacgcgt atgaaatata tttttattaa aaatataata attagttggg cgaggtggtt 3840
   tatagttgta attttagtat tttgggaggt cgagataggt aggttatttg aggttatgag 3900
   ttcgagatta gtttggttaa tatcgtgaaa attttatttt tattaaaaat ataaaaatta 3960
   gttagatgtg gtggcgtacg tttgtaattt tagttattcg ggaggttgag gtatcgaatc 4020
   gtttgaacgt gggaagtgga gtttgtagtg agtcgagatc gttttattgt attttagttt 4080
   gggtaataga gttagattgt tttaaaataa ataaaaaatg gtgttaagat ttttagacga 4140
   gattttaatg gattaaggtt tatatgtaaa tagtattaaa gattatggaa tagagatggg 4200
   agaagtaagt agggaggtag gaatagttta gttgtggtag ttttagttta gtttatttat 4260
   ataaatggtt ttttagggta gtacgtggag tatttttatt tttaaatatt ggattgagag 4320
   tagagagttg tgtaaaataa ttataagttt ttaattatgt ttttttaatt atttttatta 4380
   tttaagattg ttgtttttat ttattattga attttttcgt tttttttttt taatttttgt 4440
   gttagttaat ggttgaaatt ttgatttggt aaaaaatttt tggcgaaaat tagtaaaaag 4500
   ggtttataaa ttaggtttta gggaagtata gaggtagtta cgagaaggtt cgaggtgttt 4560
   atggaaagag ttcgagttta ggagttttgg gaggatttta ggcgttcgga gtcgtcgtta 4620
   cgtaatcggt atttagagtt ttcgaagatc ggaaggtttc gtttaggttt cggtttaggt 4680
   ttcggtttcg gtttcggttt cggtttcgtt tcggttcggt cgggtagttg gtaggtgtcg 4740
   tgcgtaattt ttcggaagtt gtcgtttttt ttttttttta tgggaatatt ttttttaaaa 4800
   aaaaagagtt cgttggcgtt atttcgtagg attggtcgtt ttaaaatcgt gataaaggag 4860
   ttgttcgtta tttttatttt tcgttttttt ttagtaagga gtcggggttt tttttagttt 4920
   ttttagttag gcggcggcgg cgattggtaa tgtttggttt taaaagaaac gcggtaatcg 4980
   gatttaattt ttattgtggg ggggtcggtt tgggggtcgg tagcggcggc gttattcgtt 5040
   cgggagggcg atttttggtt acggagaagg aggtttcggt tcggcgagag atagggggag 5100
   gggaggtcgg cgcggtgatt ggcggaagcg tcggcgtaag tttttcgttt atttttacgt 5160
   tagattttcg gagggtcgcg cggtcgtcgt ttattggcgt cgaggttttc gacgttatcg 5220
   cgattttcgc gaggttgttt tttttcgcgt ttattcgtcg cgcggcgtcg tttgaggaga 5280
   tggaagtttc ggtcgttgac gttattatgt cgttcgaaga ggagttggac gggtacgagt 5340
   cggagttttt cgggaagcgg tcggttgttt tgtcgttgtt ggagttggtc ggggaatttg 5400
   gtaataatat tagtacggac gggttattat tttcgacgtc gtcgttagta gaggaggagg 5460
   aggacgagtt gtatcggtag tcgttggaga ttatttttcg gtattttcgg gagtaggtta 5520
   tcggcgttaa ggatataaag ttaatgggta ggtttggggt tattagtagg aaggcgttgg 5580
   agattttacg acgggttggg gatggcgtgt agcgtaatta cgagacggtt ttttaaggta 5640
   agggggttta ttaatcgtta aggttttatt tttttttttt tatttttttt cggatttatt 5700
   cgttaagggt gggttggaaa tcgaaacgag ttagtgttga aacgtgtttt attttatttt 5760
   tgaagttaga atattttggt tatgagttat tgttttcgtt tattttgatt tttttggaaa 5820
   tggtagtttt tgtttaaaga tcggaaaggg tgggatgtta attttaagtg ggttaatttt 5880
   gagttttgta aattttagta gcgatttttt cgtcgcgggt gggtaggcga attttgcgtc 5940
   ggtttagata aaggaggtcg tgaggatttg tatgtttttt ttttttagg 5989
<210> 52
   <211> 5989
   <212> DNA
   <213> Artificial Sequence
<220>
   <223> chemically treated genomic DNA (Homo sapiens)
<400> 52
   tttgagaaag aaaagtatgt aggtttttac ggtttttttt gtttaaatcg gcgtaagatt      60
   cgtttgttta ttcgcggcgg gaaaatcgtt attgggattt atagaattta ggttgatttt     120
   atttgaaatt gatattttat ttttttcggt ttttgaataa gagttgttat ttttaaaaga     180
   attaagatgg gcggaaataa tgatttatgg ttagaatatt ttggttttag gaataggatg     240
   agatacgttt taatattgat tcgtttcggt ttttaattta tttttggcgg gtgagttcgg     300
   ggagagatgg aaaaaaggga gtgaggtttt ggcgattaat gaattttttt attttggaag     360
   gtcgtttcgt ggttgcgttg tacgttattt ttaattcgtc gtaaggtttt tagcgttttt     420
   ttgttggtgg ttttagattt gtttattggt tttgtgtttt tggcgtcggt ggtttgtttt     480
   cgaaggtatc gagagataat ttttagcgat tgtcggtata attcgttttt tttttttttt     540
   gttggcggcg gcgtcgaggg tagtgattcg ttcgtattgg tgttattatt agatttttcg     600
   attaatttta gtagcggtag gatagtcggt cgtttttcga gaggtttcgg ttcgtattcg     660
   tttagttttt tttcgggcga tatgatggcg ttagcggtcg gggtttttat ttttttaagc     720
   ggcgtcgcgc ggcgggtggg cgcgaagaaa agtagtttcg cgggggtcgc ggtgacgtcg     780
   gggatttcgg cgttaatggg cggcggtcgc gcgatttttc gggagtttgg cgtgagggtg     840
   gacggggggt ttgcgtcggc gttttcgtta attatcgcgt cggttttttt tttttttatt     900
   tttcgtcggg tcgaggtttt ttttttcgta gttaaaagtc gttttttcgg gcgggtggcg      960
   tcgtcgttgt cggtttttaa gtcggttttt ttatagtaga ggttgagttc gattatcgcg    1020
   ttttttttga ggttaaatat tgttagtcgt cgtcgtcgtt tggttgagaa aattggggaa    1080
   gatttcgatt ttttattgga aggaagcgga agtgagaagt ggcgagtagt ttttttatta    1140
   cggttttagg gcggttagtt ttacggggtg gcgttagcga attttttttt tttaaaaaaa    1200
   gtatttttat aaaaggggaa aggggcggta gttttcggag ggttgcgtac ggtatttatt    1260
   agttgttcgg tcgggtcggg gcggggtcgg ggtcggggtc ggggtcgggg tttgagtcgg    1320
   ggtttgagcg gggtttttcg gttttcggag gttttgagtg tcggttacgt aacggcggtt    1380
   tcgagcgttt ggggtttttt tagagttttt gggttcgagt tttttttatg agtatttcgg     1440
   gttttttcgt ggttattttt gtgttttttt gagatttgat ttgtgagttt tttttgttgg     1500
   ttttcgttag aggtttttta ttaaattaaa attttaatta ttgattaata taggggttga    1560
   aggaagagga cggggaagtt tagtgatgga tgggaataat agttttagat gatagggata    1620
   attaagaggg tatagttggg gatttgtggt tattttgtat agttttttat ttttagttta    1680
   atgtttggaa atgaggatgt tttacgtgtt attttaaaga attatttatg taagtggatt    1740
   aagttaaaat tgttatagtt aaattatttt tgtttttttg tttgtttttt ttatttttgt   1800
   tttatagttt ttggtgttat ttatatatag gttttaattt attagaattt cgtttgagag    1860
   ttttgatatt attttttgtt tgttttgaga tagtttagtt ttgttgttta ggttggagtg    1920
   tagtggggcg atttcggttt attataagtt ttatttttta cgtttagacg attcggtatt   1980
   ttagtttttc gagtagttgg gattataggc gtgcgttatt atatttggtt agtttttgta    2040
   tttttggtag agatggggtt tttacgatgt tggttaggtt ggtttcgaat ttatggtttt    2100
   aagtgatttg tttgtttcgg ttttttaaag tgttgagatt atagttgtga gttatttcgt     2160
   ttagttgatt attgtatttt tagtagagat gtgttttatg cgcgtttgtg tgaagatatt    2220
   attaaatagg ttttgtgtga gtaatatggt tgtttatttt atttgggtgt aggcgggttg    2280
   agttcgaaaa gagagttagt aaagggtgat ggattattat tagtttttat aggttttggg    2340
   ataggtggtg aagttgagag taatgttttg tgggtagggg tggattttat aaagtatatt    2400
   tttaagggtg gggagaatta taaagaattt ttttaagggt gggggagatt ataaagtata    2460
   ttgattagtt cgggtggggt agaaataaat tataatggtg gaatgttatt agttaaggtt    2520
   atttttattt tttttgtgga tttttagtta ttttaggtta tttggatgta tacgtgtaag    2580
   ttatagggga tgcgatggtt tggtttgggt ttagaggttt gataatgtgg ttttattatg    2640
   ttggttaggt tggttttaaa tttttggttt taggtgattt atttgtttta gttttttaaa    2700
   gtgttgggat tataggtgtg agttattgta tttagttgat attatttatt tttgtattga    2760
   taataaggta tatttgattt tagaggtttg taaagttttt gagaataagg atttttttat   2820
   atgttatgta tgttagttaa cgttaattaa agtagttttt ttgttaaaag atttgtaaaa    2880
   ataatagtta aaatttaaaa gtaaggtgtt gagagatata ggaaaggtga gtaagtttat    2940
   tttttattta tttttttgag atgaggtttt gttttgttat ttaggttgga gtgtagtggt     3000
   attattagtg tttattgttg ttttaatttt ttgggttgaa gtgatttttt tattatagtt    3060
   tcgcgagtag cgaggaatat aggtgtatgt ttttatattt ggttaatttt tttatttttt    3120
   gtagagatgg ggtattatta ttttgtttag gtcggttttg aatttttgga tttaggtgat    3180
   ttttttattt tggtttttta aagtgttggg attatagatc gggttttgtg ttttatgtta    3240
   ataattttag tattttggga ggttgaggtg ggtggatcgt ttgagtttag gagtttgaga    3300
   ttagtttggg aaatatggcg aaattttgtt tttattaaaa atataaaaat tagatgggtg    3360
   tggtggtgcg tatttgtaat attagttatt ttggaggttg agataggaga attgtttgaa    3420
   tttaagagac ggaggttgcg gtgagttaag atagtgttat tgtattttag tttgggtaat    3480
   agagtaagat ttggttttaa aaaaaataat aataataatg aggtttggcg tagtggttta    3540
   tatttgtaat tttagtattt tgggaggtta aggcgggcga attatttgag gttaggagtt    3600
   tgagattagt ttaattaata tggagaaatt ttatttttat taaaaatata aaattagtcg    3660
   ggtcgtggtg gtacgtgttt gtaattttag ttatttggaa ggttgaggcg ggagaatcgt    3720
   ttgaatttgg gaggcggagg ttgcggtgag ttgagagtgc gttattgtat tttagtttgg    3780
   ataataagag cgaaacgtta ttttaaaaat aaataaataa ataaagtgtt ggaattatag    3840
   gcgtgagttt tttttttggt taaaagtttt taatttttaa aatgttatag tttagtaagg     3900
   tgatacggcg tttatattat ttatttatgt tttgggtagg atttagaatt ataggggttt     3960
   atagtatgag tggtaaggat agagtatttt gtggttatag tgaataagaa atagtttttt     4020
   tatttaagga atgtttaatt ttgtatagta attaattata atgtgagatg gaatgtatgt     4080
   tttatataag gtattaataa ttttattttg gagttaagag gttgggaaat ttattttggg     4140
   ttgagagagt tagaggaaag tgttatggag gaggtagttt tgtttttatg ttttataaag     4200
   taaaatttta ttaggtggta aagtggaaag agattgagga aataggagga gtaatgattt     4260
   ttttttgtat agaggttaaa aaaggaaatg taagttttat tttaaaataa attgtattta     4320
   tttattattt gttattatta ttttttgaga cgggtattat tatgttgttt aggttggagt     4380
   gtagtggtgt agtttcggtt tattgtaatt ttgatttttt gagttaaagt gatattttta     4440
   ttttagtttt ttaagtagtt gggataatag gttagtatta ttatgtttgg ttaatttttg     4500
   tgtttttttg tagagatggg gttttattat gttgtttagg ttaatttcga atttttggtt     4560
   ttaagttatt tgtttttttt ggttttttaa agtgttagga ttataggtat gagttattgt     4620
   aattggtttt gtttgttagg aaataagttt tggtcgggta cggtgttacg tttgtaattt     4680
   tagtattttg ggaggttgag gcgggtagat tacgaggtta ggagattaag attattttgg     4740
   ttaatacggt gaaatttcgt ttttattaaa aaaaaaatac gaaaaattag ttgggtatgg     4800
   tggcgggtgt ttatagtttt agttatttag gaggttgagg taggagaatg gtatgaattc     4860
   gggaggtaga gtttttagtg agtcgagatt cgtgttattg tattttagtt tgggtaatag     4920
   aacgagattt tgttttaaaa aaaaaaaaag aaagaaagta ataaagtttt gttatgttat     4980
   ttaggatggt ttataatttt tgtttttaag ggattttttt atttgagttt tttgaatagt     5040
   tgggattata agtatgagtt attatattta gttttaaata tatttttgat tagaggattt     5100
   tttaagaaaa aaaaaaaatt taggtttaag tttttttagg taatattagt atataattag     5160
   agtttagtag ttttttagga ggtgtgatag atattgttat tcgttttagt tagtatttat     5220
   tttttaattt tttgagacgg agttttattt tgtcgtttag gttggagggt agtggcgcga     5280
   tttcggttta ttataaattt cgttttttgg atttaagtaa ttttttgttt tattttttta     5340
   agtagttgcg attataggta ttcgttatta tatttagtta atttttgtat ttttagtaga     5400
   gatggggttt tattatgttg gttaggttgg tttcgaattt ttgattttgt gatttgttta     5460
   ttttgttttt ttaaagtgtt gggattatag gtatgagtta ttatgtatgg tttaattttt     5520
   gtatttttag tagagacggg gttttattag tttggttagg ttggttttga atttttaatt     5580
   ttatgattta tttattttgg ttttttaaag tgttgagatt ataggtgtga gttattacgt     5640
   ttagtcgttt tatttaatat ttattttttt tttttttttt tttgatagaa ttttattttt     5700
   ttttttttag aattataatg tgtttagttt tttatgattt ttttgttatt ttgaaaatgt     5760
   taataaaatt ttgttatggg cgggatgtag tggtttacgt ttgtaatttt agtattttag     5820
   gaggttgtgg tgggtagatt atttgaggtt aggagtttga gattaatttg gttaatatgg     5880
   tgaaatttat ttttattaaa atataaaaaa attagttggg tatagtggta ggtatttgta     5940
   atgttagtta tttaggaggt tgaggtagga gaattgtttg tatttggga                 5989
<210> 53
   <211> 9881
   <212> DNA
   <213> Artificial sequence
<220>
   <223> chemically treated genomic DNA (Homo sapiens)
<400> 53
   tatttggtaa ggtagatttt atagtaattt tgaatatgat tttttttagt ttttagttat       60
   gtattattaa gtgttttttg ggtagaggta gagttaagta ttgagtgtta gttttgacgt      120
   tcgtatgttt tagttttttt tatatataaa aagtagtata tatatttatt tttttttatt      180
   ttattatttg ttgtagggat taaattcggg agagtaattt tgaagtttat aaattttttt      240
   gaagagattt aagaatttat tatgtttttg gtgtattaag ttttggggta tatatttaga      300
   atattttatg ttttggaagt tgagtattag ttttttttta ttgtttgttt ggtagagttt      360
   gtttgattat tgtaggtttt tttatttatg tttttagttt aggtattttt tttttgatat      420
   gaggtttttg attagaaaag agtttttttt ttaggtgttt tgagagaagt ttgtaaattt      480
   ggatagtata ttttattttg ataaaattat tttgttgtgg ttttgatgta taaaaaaaaa      540
   tttttttttt gagatagagt tttattttgt tatttaggtt ggaatgtagt ggcgtaattt      600
   tggtttattg taattttcgt tttttgggtt taagcgattt ttttgtttta attttttaag      660
   tagttgggat tataggcgtg tattattata tttggttaat tttgtatttt tagtagagat      720
   agggttttat tatgttggtt aggttggttt tgaatttttg attttaggcg atttgttcgt      780
   tttggttttt taattatata tagtttgatg ttatttataa aaattttaaa agtataaata      840
   gatttttaag gaatatatat atatataaat tttaaaagtt taaagaaatg tatggtaggg      900
   gtatatatta aatttaagat agtggttttt agaggatgaa gaaaggagat attatagagg      960
   tgattatagg gaattttttt gtatgtgtat tgtttttagg aagaggaatg ggaaaaggga     1020
   tattatattt tttttttttt tttttttgag atagagtttt attatgtttt ttagggtgga     1080
   gtgtaatggc gttattttag tttattgtaa ttttcgtttt tagggttgga gcgatttttt     1140
   tgttttagtt tttcgagtag ttgggattat aggtatgtat tattatattc ggttaattgt     1200
   tttgtatttt tagtagagat ggggttttgt tatgttgggt aggttggttt tgaattttcg     1260
   attttaggtg attcgttcgt tttagtattt taaagtgttg ggattatagg tttgagttat     1320
   tgcgtttagt tgggagatta tattttttat attttttgat atgtataaag ttattaatta     1380
   tatttttttt ttagaaaggt tatagattaa taatattgat ttttaatttt tttaaaatta 1440
   gaagggttat aagaaatggt tgaatttagt tattttgttt tatagattag ggaattgaag 1500
   atcggaaatt tgtgtaattt tttttgtttt ttataaatag ttagattaga atttagattt 1560
   tttgtttttt tattcgaatt aatggtttgt gagtatagtt tttaaagaag gaagtggtga 1620
   ttatttagtt gagtttttaa gttaattttt aattttattt ttttttttta tttttttttt 1680
   tttttttttt tgagatggag tttttttttg tcgtttacgt tggagcgtag tggtacgcgt 1740
   ttgtaatttt agtaatttgg gagggtgagg taggagaatc gtttgaattt gggaggcgga 1800
   gaatgtagcg agttgagatc gtgttattgt attttagttt ggtaatagaa gggaatttcg 1860
   ttttaaaaat aaaataaaat aaaataaaat aaaataatta tatatataga attagttggg 1920
   cgtgttggcg ggtgttattt gtaggattat aagattatat ttgtaatttt aatattttgg 1980
   gaggttaagg tgtgggggag gattatttga ggttaggagt ttaagattag attgggcgat 2040
   atagtaagat tttgtgttta tttttttaaa aaaattagaa gatgagtaat tatttataaa 2100
   ttagtgttaa ttacgatggg aaaaatttta ttttttatta tggtttcgtt aatatattta 2160
   ttaacgtttt gttttttttg attgattgag atagagtttg gttttattat ttaggttaga 2220
   gtgtagtgga gtaatttcga tttattgtaa tttttttttt cgggtttaag taattttttt 2280
   tttttagttt ttcgagtagt tgggattata ggcgtttatt attgtattcg gttaattttt 2340
   tttgtatttt tagatagtgt tttattatgt tggttaggtt agtttcgaat ttttgatttt 2400
   acgtgattta tttgtttcga ttttttataa gtatgagaat gataatagta ttgtttgtaa 2460
   cggtaaacga ttgaataatg tttaagtatt tattaaagga ggagatgaat aaaaattgtg 2520
   gtgtattcgt tattattatg ggttgaatgt gtttttttta aatattatgt tgaatattat 2580
   ttttagtatt ttagaatggg aatgtatttg gagatagggt ttttaaagag gtgattaagg 2640
   taaaatgagg ttatatggat gggtttaata tgattagtgt ttttataaga agaggggatt 2700
   aggatataga taatatatag ataatgggta attatataag gatatagtga aaagataggt 2760
   tgtattttag tttggtaata gaggggaatt ttattttaaa aataaaataa aataaaataa 2820
   agtaaaataa ttataaataa aaaattagtt gggtaatttg gtgggtgtta tttgtaggat 2880
   tataagatta tatttataat tttaatattt tgggaggtta agtggggagg attatttgag 2940
   gttaggagtt taagattaga ttgggtaata tagtaagatt ttgtgtttat ttttttaaaa 3000
   aaattagaag atgagtaatt atttataaat tagtgttaat tatgatagga aaaattttat 3060
   tttttattat ggtttagtta atatatttat taagttaagg aaagagggta ttaggagaaa 3120
   tttaatttgt taataatttg atttttaatt tttagttttt agaattatga gaaaataaat 3180
   ttttgtttaa gttatttagt ggtattttgt tatggtaatt ttagaaaatt aatataatta 3240
   ttatataata atttattata tatttatata atttagtatg ttattgtaga ataacgttaa 3300
   aatattaaaa atgaattaat tagggttagg tgcggtggtt tatatttgtt atgttagtat 3360
   tttgggaggt tgaggcgggt ggattacgag gttaagagtt tgagattagt ttggttaata 3420
   tggtgaaatt ttgtttttat taagaatata aaatatagtt tggcgtggtg gcgcgtgttt 3480
   gtaattttat ttatttcgga ggttgaggta ggagaattgt ttgaatttaa tattatatta 3540
   tttagttatt tgaagatagt ttttaatttt tatgtttaat tttttttttt tttttttttt 3600
   tttttttttt tgagatgtag tttcgttttt gtcgtttagg ttggagtgta atggcgttat 3660
   ttcggtttat tgtaattttc gttattcggg tttaaacgat ttttttgttt cggtttttta 3720
   agtagttggg attataggtg atcgttatta tgtttcgtta attttttgta tttttagtaa 3780
   aaacgggggt ttcgttatgt tgggtaggat ggtttcgaat tgttgatttt aggtgattcg 3840
   ttcgtttcgg ttttttaaag tgcgttggga ttatagggga gagttattgc gttcggtttt 3900
   tttcgtttat atgtaggtta aaaatttttt tgagttttta attatttttt tttttttttt 3960
   tttttttttt ttgaggagtt tcgtttttgt tgtttaggtt ggaatggcgt tatttcggtt 4020
   tattgtaatt ttcgtttttt aagtttaaac gatttttttg agtagttggg attataggta 4080
   tgcgttatta tattcgatta attttgtatt tttagtagaa atagggtttt attatattgg 4140
   ttagattggt ttcgaatttt tgattttagg tgattcgttt gttttaggtt ttttaaagtg 4200
   ttgggattat aggcgtgagt tatagcggtt ggtgttttta attatttatt tgatatggtt 4260
   ttaagttttt ttgttatttt agtgtcgtta tcgtttcgtt tttttttttt attatttttg 4320
   gggagtaagg gtgaggattg gggaatggta gaggaagcgt agttttttcg aagagttgaa 4380
   ggtagtttgt gattatgatt ttagtttttt tttttttttg ttttttgatt ttttaatata 4440
   cgtttgtaaa tattataaga ttttgtaagt tttttattta ttattttttt aaatttgacg 4500
   atttttaatc gttcgggttt tacgattttt ttttcggatt aggatttatt ttatggtagt 4560
   gagtttagtt tttttagttt tttagttttt ttattgtttt atttatggtt ttcggaggtt 4620
   ttatcggaag ttttcgtgtg aggtcgtgtg ggaggtcgga agttgcggtt ttattattcg 4680
   ttattttaaa atgttgtcga ggttttagga tttgtgattg ttattttttt ttttattcgg 4740
   gttcggcggg ggagcgattt atggagttgt cgtaaggtgt ggtttttggt tttcgtcggc 4800
   ggggtgtcgg gagcgggagt tggagagtga ggagtaggcg gtcgtatcgt tagttgtgcg 4860
   ggtagcgggc gggaggagtt tgagaggtgg gtcgggcggc gtggggaagg tcgcgttagg 4920
   cggaagaagg gttcgggggg aggggaagag ttgtgggatg agggttcgcg gaagataaaa 4980
   gtggaggtgg ggtgacgtga agtaattttg gttaataggt gataaattta aagtcgacgt 5040
   agttggattg agtttaattg gatataatat ttgaggattg ttgaggtaat ttggagaaag 5100
   gaggtgggag agagggaatc gggtagaatg atattgacgt gttttaagtt tttaagttag 5160
   tttttaggtt tgcggatagt ttattttaga ggtatttgag tgtttgtttg aaaatgtttg 5220
   ttttggtttg gcgcggtggt ttacgtttgt aattttagta ttttaggagg tcgaggtggg 5280
   tggatttatt tgaggatagg agttcgagat taatttggtt aatttggtga aatttcgttt 5340
   ttagtaaaaa tataaaaata ttagatgtgt gtggtggcgt atgtttgtaa ttttagttat 5400
   tcgggaggtt gaggtaggag aattatttga attcgtgggc gggggttgta gtgagtcgag 5460
   atcgcgttat tgtattttag tttgggttat agagggagat ttcgttttaa aaaaaaaaaa 5520
   agaaaaaatg ttcgttttgg ggttatattg aaatttatag atagtatcga gatggggttt 5580
   agagaaatat attttaatga gtatattttt tagtgatttt tgtatatttt aaagtttgaa 5640
   aattataggt ttttttggta ttgtttttgg tttaaatatt ttttttatat atattgagta 5700
   gtggtataaa atgtattgtt aatatataga aaatatgaaa ttttttaaat attttcgatt 5760
   agagaattat aagagtagta aatgtggttt tattaagtgg gaagaaagta gtaatttaaa 5820
   ataatttttt gggagattga attgagtaat aataaaattt tagtttttcg ttaataataa 5880
   taataataat aataataata ataatttatt gaatgtggtt agtttattag atgaggaaag 5940
   aggaaggtat tttttgtatt tttgtttagt tttttttata agtattatta agttaatagt 6000
   tttgtttttt tggtgtttgt attatgtaat gtttttaata ttttttaatt gtgttttttt 6060
   atgtattaaa tgtttttttt tttgttattg tgtgttgtta ttatttttga aataggtaat 6120
   tttgtgtgta tatagttaaa ttttattgtt attggatttt ttggtttgat agattaaaga 6180
   tataatagaa aattattttt gttatttttt tggggaaatt aatggattta aaattagtag 6240
   tattggatat ttttttttag ttatttttta gagtaattgt tatattttat ttttagggtt 6300
   ttaattttga agaaatttat taaattttat agagtatttt ttaatattga gttcgttgtg 6360
   gttttgaatt ggggttagta cgagaggtat tgagggtttg ttatattata tgtattaatt 6420
   agttggtatg tttagtattt gttagggttt ttaatggcgg tttttaaatt ttggggatta 6480
   tagttttttt tgaaaagtta atgaatttta tgaaattttt ttttataagt agtataatat 6540
   taaatattaa ttttagggtt ttattgaaat tattgattta tgatttttag gttttgtttt 6600
   ttggtaggat gttaaagaag agtgaatggt ttataaagag tttggtagta tatacgtttt 6660
   ttaaaaggtt atgtatttat agttaaagtt attaaggtga gtgaaatatt tggttagttg 6720
   ttttgagttt gggtaggggt aggattatta tatagttggt gtagattgtt gtgaattgta 6780
   taatttttgg ggtattgttt ataggttgtt atataaatga ttttttagag atgtgtaata 6840
   tatatttgag gaggttttgg gggtggggtg gggtgataat ggatatatta tcgtaatttt 6900
   tttttttgat atagggtttt gttttgttat ttaggttgaa gtgtagtggc gagaggttta 6960
   cggtagtttt gattttttgg ttgaagcgat ttagttattt tagtttttcg agtagttgag 7020
   attataggta tgtatattac gtttggttga attttttgtg tttttgaaga gatggcgttt 7080
   tgttatgttg tttaggttgg ttttgaattt ttgggtttaa gtaattattt attttagttt 7140
   tttaaagtgt tgggtttata ggtaggagtt attatattgg gtatttaggg taatattttt 7200
   taaatgaaaa taaaataagg agtattagaa ataaaaatgg ggttagtttt tttgtttatt 7260
   taagtttttt atattttgta tgtgatttgt ataaggtatt ttaatgtaat tttaatggta 7320
   taacgtgttt tttggggttt tttattttgt ttattttgag tttttggtgt tgtagatgta 7380
   ttatttttgt tgaatgtatt tttgatatta gaagaagaag gtaagttgaa attggtaatt 7440
   aagtttatag agtattgtag ttttttttgg aaatagggtt tcgttttgtt tttttaggtt 7500
   ggagtgtagt gatgtaatta taatttatta tagttttagt ttttaaggtt taagtgatcg 7560
   ttttttttag ttttttgagt agttgggatt ataagtttat gttattatat ttggttaatt 7620
   taaaatattt tttttgtgag gaggtgtggt tttattgtgt tgtttatgtt tgtttagaat 7680
   ttttgggttt aagcgatttt tttagtttag ttttttaaaa tgttgttttt gtttggtata 7740
   attgttagta ggtaaatttt atattattgt ttgttttgag ggtttggggg ttttttgttt 7800
   gtttttggtg gagttatata agtttattat ataaaggtat gaaaagagtt attgttatta 7860
   ggaaaaattt aagttattat aggtttgggt atatagggtt aattttgtga tgtatatttt 7920
   tagttttgaa tttatagggg tatagttttg gattattgta atttttattt tgttagttta 7980
   ggtgattttt atgttttagt ttttttagta gttgggattg taggttcgtg ttatgtttag 8040
   ttagttgttg tatttttagt agagataggg ttttgttttg ttgtttagtt tggtttttgt 8100
   taaaaataaa ttttcgaagt tgtaaatgaa tattatttaa atataaattt aattttttta 8160
   gtaaggtaat tttattttta tggaagggtg tattttacgg atggagtaat ggtgagagta 8220
   tatttgaata agggagggga aggggttttt atttttgacg taggtagttt ttatttttgt 8280
   gttgttttcg tattggttag ggttggatcg tatagtttaa gttaatattg atgggttatt 8340
   ttaaagagag taggggtatg agttggagtg gttgggcgag tagtatggcg ggaagaatag 8400
   ttatagaata ggtgatttag gatgatttag gttaagggag gtgattagga gtgatttagg 8460
   atggagtagg tgatagaagt taggaggggg ttgtttattg aaacgagggg taaggagatg 8520
   aagagaatga ggaagttaaa tttttaaagt tttaaatgaa gaataaagaa taggggagtt 8580
   gaatatatag atatattggt tttttggaga gtattttaga atttattgta tttaataatt 8640
   tataggttaa aatttttgaa gaggaattta ttaaatttta tattttaaat ttttgatttt 8700
   aggtgatttg tttattttag ttttttaaat tgttaggatt ataggcgtga gttattgtat 8760
   ttggtttatt ttagtcgtta tttatttttt ttttgatgtg gtttgttagt tattagtttt 8820
   aataattgtt gttttttgtt agaaaaaggt ataaatgaag gttaatttaa gtttatttaa 8880
   aaatttattt ttcggttggg tatggtggtt tacgtttgta attttagtat tttgggaggt 8940
   tgaggtgggc ggattacgag gttaggaatt tgagattagt ttgattaata gggtgaaatt 9000
   tcgtttttat taaaaatata ataatgagtt gggcgtggtg gcgtgtgttt gtaattttag 9060
   ttatttagga ggttgaggta ggagaatcgt ttgaatttgg gaagtagagg ttatagtgag 9120
   tcgagattgc gttattgcgt tttagtttgg gggatagcga gattgtgttt taaaataaat 9180
   aaataaataa ataatttatt tttttttttt gtagtttttg tttttttaag gataaaaggt 9240
   aggtgaaaga tttttattga attttatttt gtattttatt ttatgttata tttttttttt 9300
   gatggtatat gtttatttcg gtttaattta ttttgtaggt ttgtaattgt tgtgtttttt 9360
   tttggggttg gtatatttat aagttgtttt aattggtatt taaaatgttg atttttagtt 9420
   aattttttat ggttttttgt taagatatgt tttaatatgt tgtagaggtt tttttagaag 9480
   ttttgttgga attttaaaat ttgtgattta ttttggtaaa tttttagttt tgtatttaga 9540
   gagtgtttta tttgataatt attggggaga taatgatttg atttttagtt aaaatagttt 9600
   attgattaat attagttgga ggttggagcg tgtttgaaat tgttattgtt ttagttttta 9660
   tttgagagaa gtttggacga tttttatttt taattaaatt agaattagaa agtagttaga    9720
   agatatgtag aattttagta agggtatata gtatttgtgt acggtgtttt attatatgtg    9780
   ttatatgtgt tttttatttt tgttggttgt ttttgtgtga atgttaaata gggaattttt    9840
   tgtattagaa tagatgttat aaattttttt ttttatttag t                        9881
<210> 54
   <211> 9881
   <212> DNA
   <213> Artificial Sequence
<220>
   <223> chemically treated genomic DNA (Homo sapiens)
<400> 54
   attaaataga aaaaaaaatt tataatattt attttaatgt agaaaatttt ttatttagta      60
   tttatataaa ggtaattagt agaagtaaaa gatatatatg atatatataa tggagtatcg     120
   tatataaatg ttgtatattt ttgttgaggt tttgtatatt ttttgattat tttttagttt     180
   tgatttaatt aagagtaaga atcgtttaag ttttttttag gtgaaggttg aaatagtaat      240
   agttttaggt acgttttaat ttttaattga tgttaattag taaattgttt tagttgaaag     300
   ttaaattatt atttttttaa tggttattag gtagagtatt ttttagatgt aagattgggg      360
   atttattaag gtaagttata ggttttaaga ttttaatagg atttttagga aaatttttat     420
   aatatgttgg aatatgtttt gataaaggat tatagaaagt taattaagag ttaatatttt     480
   aagtattagt taaaatagtt tgtaaatgta ttaattttag aaaaaaatat agtaattata     540
   aatttataaa atgggttaaa tcgagatgga tatatattat taaagaaaag atgtagtatg     600
   aagtgaaata tagagtaaag tttaatgaaa attttttatt tattttttat ttttaaaaaa      660
   ataaaagtta tagagaagga agatagattg tttatttatt tatttatttt gagatatagt     720
   ttcgttgttt tttaggttga agcgtaatgg cgtaatttcg gtttattgta atttttgttt     780
   tttaggttta agcgattttt ttgttttagt tttttgagta gttgggatta taggtatacg     840
   ttattacgtt tagtttatta ttgtattttt agtagagacg ggttttattt ttgttggtta     900
   ggttggtttt aaatttttga tttcgtgatt cgtttatttt agttttttaa agtgttggga     960
   ttataggcgt gagttattat gtttagtcgg aaaatagatt tttaaataaa tttaagttaa    1020
   tttttattta tgtttttttt taataaaagg taataattat taaaattgat gattaataag     1080
   ttatattagg gaaagggtaa atggcgatta gaataggtta ggtgtagtgg tttacgtttg    1140
   taattttaat agtttgggag attaagatgg gtagattatt tgaggttagg agtttgagat    1200
   gtaggattta ataaattttt ttttaaaggt tttagtttgt aaattgttaa gtataatgag    1260
   ttttgagatg ttttttaaaa aattaatgta tttgtatgtt tagttttttt gttttttgtt    1320
   ttttatttaa aattttaaaa gtttaatttt ttttattttt ttattttttt gtttttcgtt     1380
   ttagtaaata attttttttt agtttttatt atttgtttta ttttgagtta tttttggtta    1440
   ttttttttga tttgagttat tttgagttat ttgttttgta attgtttttt tcgttatatt     1500
   attcgtttag ttattttagt ttatattttt gtttttttta aaatagttta ttagtattag    1560
   tttagattgt gcggtttaat tttagttaat acgggaataa tatagaagta ggggttattt    1620
   gcgttaggga taagaatttt tttttttttt ttgtttaggt gtgtttttat tattgtttta    1680
   ttcgtgagat gtattttttt atagaagtaa aattgttttg ttgagaaaat taaatttatg    1740
   tttgagtgat atttatttgt agtttcgaaa atttattttt aatagaggtt agattaggta    1800
   atagggtaaa attttgtttt tattaaaaat ataataatta gttgggtatg gtacgagttt    1860
   gtagttttaa ttattaagga agttgaggta tgagaattat ttgaattggt aaggtggagg    1920
   ttgtagtgat ttaagattgt gtttttataa atttagggtt ggggatgtat attataaggt    1980
   taattttgta tgtttaaatt tataatgatt tgaatttttt ttaatagtaa taattttttt     2040
   tatattttta tatgatggat ttgtgtaatt ttattaaaaa taaataaaaa atttttaaat    2100
   ttttaaaata aatagtgatg tgaaatttat ttattaataa ttgtgttagg taaaagtaat    2160
   attttgggag gttgagttag gaggatcgtt tgagtttagg agttttagat aaatatgagt    2220
   aatatagtaa gattatattt ttttataaaa aaaatatttt aaattagtta ggtgtagtgg    2280
   tatgagtttg tggttttaat tatttaggag gttgaggagg acgattattt gagttttaaa    2340
   ggttgaggtt gtggtgagtt gtgattgtat tattgtattt tagtttgggg ggataaagcg    2400
   agattttatt tttaaaaaaa attgtaatat tttgtaagtt tagttattag ttttagtttg    2460
   tttttttttt ttagtattaa gaatatattt aataaaaatg atatatttat agtattagga    2520
   atttaaggtg agtaggatgg aaaattttag gaaatacgtt atgttattga aattgtatta    2580
   gagtatttta tataggttat atataaaatg tgaaaggttt aagtagataa agaagttggt    2640
   tttatttttg tttttaatgt tttttgtttt gtttttattt aaaaagtgtt attttgggtg     2700
   tttagtatgg tggtttttgt ttgtaaattt agtattttgg gaggttgagg tgggtgatta    2760
   tttgggttta ggagtttaag attagtttgg gtaatatggt aaaacgttat ttttttaaaa    2820
   atataaaaaa tttagttaga cgtggtgtat atgtttgtgg ttttagttat tcggggggtt    2880
   gagatggttg gatcgtttta gttaggaggt taaggttgtc gtgaattttt cgttattgta    2940
   ttttagtttg ggtgatagaa taagattttg tattaaaaaa aaaagttacg atggtatgtt    3000
   tattattatt ttattttatt tttaaggttt ttttagatat atattatata tttttaaagg    3060
   attatttata tggtagttta tgaatagtgt tttaagagtt atatagttta tagtaatttg    3120
   tattaattat atggtaattt tgtttttatt taagtttagg ataattagtt agatgtttta    3180
   tttattttaa tggttttagt tgtaagtgta tgattttttg aaaagcgtat gtgttattag    3240
   gttttttgtg ggttatttat ttttttttgg tattttgtta aagaatagaa tttgagggtt 3300
   atggattagt ggttttaatg aagttttgaa attgatgttt aatattgtgt tatttataag 3360
   aagaggtttt atgaggttta ttagtttttt aaggaggatt ataattttta aagtttaaga 3420
   atcgttatta gagattttag tagatgttag gtatattaat tgattaatgt atatggtgtg 3480
   ataaattttt agtatttttc gtattagttt taatttaaaa ttataacgag tttaatgtta 3540
   gaaaatattt tgtaaaattt gatggatttt tttaggattg ggattttgaa aataaaatat 3600
   agtaattgtt ttaaaaggta attgggggaa aatatttaat gttgttgatt ttaagtttat 3660
   taattttttt aaagaagtaa tagaagtaat tttttgttgt atttttggtt tattagatta 3720
   aaagatttaa tagtaatgag atttaattgt atatatataa aattatttat tttaaaaata 3780
   atgataatat ataatggtaa aaggaaaaat atttaatata taaaaaagta taattaaaaa 3840
   gtattaaaag tattatatag tgtaaatatt aaaaagatag agttattaat ttagtggtgt 3900
   ttataaggaa aattaggtaa gaatgtagaa aatgtttttt ttttttttta tttagtgagt 3960
   tggttatatt taataagttg ttgttattat tattattatt attattatta gcgaaagatt 4020
   gaagttttat tattatttaa tttagttttt taaaaagtta ttttaaattg ttgttttttt 4080
   tttatttgat gaaattatat ttgttgtttt tgtgattttt tgatcggaaa tatttggaaa 4140
   attttatgtt ttttatatat tgatagtgta ttttatgtta ttatttagtg tatgtagaaa 4200
   ggatgtttgg gttaggagta gtgttaaaaa aatttgtggt ttttaagttt taaggtatat 4260
   aaaaattatt gggaggtgtg tttattaaaa tatatttttt tgagttttat ttcgatattg 4320
   tttgtaggtt ttagtgtggt tttaaaacga atattttttt tttttttttt tttgagacgg 4380
   aatttttttt tgtagtttaa gttggagtgt agtggcgcga tttcgattta ttgtaatttt 4440
   cgtttacgga tttaagtgat ttttttgttt tagtttttcg agtagttagg attataggta 4500
   tgcgttatta tatatattta atatttttgt atttttatta gagacggggt tttattaggt 4560
   tggttaggtt ggtttcgaat ttttgttttt aggtagattt atttatttcg gttttttgaa 4620
   gtgttgggat tataggcgtg agttatcgcg ttaagttaaa ataagtattt ttaaataagt 4680
   atttaagtgt ttttaaggta gattgttcgt agatttaaaa attaatttaa aaatttaaga 4740
   tacgttaatg ttattttatt cggttttttt ttttttattt ttttttttta aattgtttta 4800
   atagttttta aatgttgtgt ttagttaaat ttaatttaat tacgtcgatt ttaggtttgt 4860
   tatttgttgg ttaaagttat tttacgttat tttattttta tttttatttt tcgcgagttt 4920
   ttattttata attttttttt ttttttcgag tttttttttc gtttggcgcg gtttttttta 4980
   cgtcgttcga tttatttttt aagttttttt cgttcgttat tcgtataatt ggcggtacgg 5040
   tcgtttgttt tttatttttt agttttcgtt ttcggtattt cgtcgacgaa ggttaagagt 5100
   tatattttac ggtagtttta tgagtcgttt tttcgtcgag ttcgggtggg gggaggggtg 5160
   gtagttatag attttagggt ttcggtagta ttttgaaatg gcgagtaatg aagtcgtaat 5220
   tttcggtttt ttatacggtt ttatacgagg gttttcggtg aggttttcgg aaattataga 5280
   tggagtaatg ggaggattag agagttagaa aggttgagtt tattattata gagtagattt 5340
   tagttcgggg agagagtcgt ggggttcgaa cggttgaaag tcgttagatt tgaagagata 5400
   ataagtgagg gatttgtagg gttttgtgat atttatagac gtgtattagg agattagaaa 5460
   atagagaaga ggaagagtta aggttataat tataggttgt ttttagtttt tcggaaaaat 5520
   tacgtttttt ttgttatttt ttagttttta tttttatttt ttaagggtgg tagggaaggg 5580
   aaacggagcg atggcggtat tagggtggta aggaagtttg aagttatgtt aaataaatag 5640
   ttgaaagtat tagtcgttgt ggtttacgtt tgtaatttta gtattttggg aggtttaagg 5700
   taggcggatt atttgaggtt aggagttcga gattagtttg attaatatgg tgaaattttg 5760
   tttttattaa aaatataaaa ttagtcgggt gtggtggcgt atgtttgtaa ttttagttat 5820
   ttaggagaat cgtttgaatt tgggaggcgg aggttgtagt gagtcgagat agcgttattt 5880
   tagtttgggt aataagagcg aaatttttta aaaaaaaaaa aaaaaaagaa aaagaaatag 5940
   ttgaaggttt aagggagttt ttaatttgta tataaacgag ggaggtcggg cgtagtggtt 6000
   tttttttgta attttagcgt attttgggag gtcgaggcgg gcggattatt tgaggttagt 6060
   agttcgagat tattttgttt aatatggcga aattttcgtt tttattaaaa atataaaaaa 6120
   ttagcggggt atggtggcgg ttatttgtaa ttttagttat ttaggaggtc gaggtaggag 6180
   aatcgtttga attcgggtgg cggaggttgt agtgagtcga gatagcgtta ttgtatttta 6240
   gtttgggcga taagagcgaa attgtatttt aaaaaaaaaa aaaaaaaaaa agagggaaag 6300
   ggttgagtat agaaattgga agttgttttt aaataattga atagtataat attgggttta 6360
   agtagttttt ttgttttagt tttcggagta gatgggatta taggtacgcg ttattacgtt 6420
   aagttatatt ttgtattttt agtagagata gggttttatt atgttggtta ggttggtttt 6480
   aaatttttga tttcgtgatt tattcgtttt agttttttaa agtgttggta tgataggtgt 6540
   gagttatcgt atttggtttt ggttaattta tttttaatgt tttaacgttg ttttatagta 6600
   atatattgaa ttgtataaat gtataatgaa ttgttgtata gtaattgtat tagtttttta 6660
   gagttgttat aataaagtat tattgaatgg tttaaataga aatttatttt tttatagttt 6720
   tggaagttgg aagttgaaga ttaagttgtt agtaagttag gttttttttg atgttttttt 6780
   tttttggttt gataaatatg ttgattaggt tatggtgaag aatagagttt tttttattat 6840
   ggttgatatt gatttgtgag taattgttta ttttttaatt tttttaaaaa aatagatata 6900
   gggttttatt atgttgttta atttggtttt gaatttttgg ttttaagtga ttttttttat 6960
   ttggtttttt aaagtgttag gattataggt ataattttgt aattttatag gtggtattta 7020
   ttaggttgtt tagttaattt tttgtttgtg gttgttttgt tttgttttgt tttgttttgt 7080
   ttttgaggtg gagttttttt ttgttgttag gttggagtgt agtttgtttt tttattgtgt 7140
   ttttgtgtgg ttgtttattg tttgtatgtt gtttatattt taattttttt tttttatgag 7200
   gatattaatt atattgggtt tatttatgtg attttatttt attttaatta tttttttaaa 7260
   ggttttgttt ttaaatatat ttttattttg aagtattggg gatggtgttt aatatgatgt 7320
   ttgagaggga tatatttagt ttataatagt aacggatgta ttataatttt tatttatttt 7380
   tttttttaat aaatatttag gtattattta atcgtttgtc gttgtaaata atgttgttat     7440
   tatttttatg tttatgggag gtcgaggtag gtggattacg tgaggttaga agttcgagat     7500
   tagtttggtt aatatggtaa aatattgttt aaagatataa aaaagattag tcgggtgtag     7560
   tggtaggcgt ttataatttt agttattcgg gaggttgagg gaggagaatt gtttgaattc     7620
   ggaggaggag attgtagtga gtcgagattg ttttattgta ttttagtttg ggtgatggag     7680
   ttagatttta ttttagttaa ttaaaaaaaa taaaacgttg ataaatatgt tgacgaggtt     7740
   atggtgaaga atagagtttt ttttatcgtg gttgatattg atttgtgagt aattgtttat     7800
   tttttaattt ttttaaaaaa atagatatag ggttttatta tgtcgtttag tttggttttg     7860
   aatttttggt tttaagtgat ttttttttat attttggttt tttaaagtgt taggattata     7920
   ggtgtaattt tataatttta taggtggtat tcgttagtac gtttagttaa ttttatgtgt     7980
   gtggttattt tgttttgttt tgttttgttt tgtttttgag acggagtttt tttttgttgt     8040
   taggttggag tgtagtggta cgattttagt tcgttgtatt tttcgttttt tagatttaaa     8100
   cgattttttt gttttatttt tttaaattgt tgggattata ggcgcgtgtt attacgtttt     8160
   agcgtgggcg atagaggaag attttatttt aaaaaaaaaa aaaagaagaa atgaaagaaa     8220
   gaaatgaaat tggaaattgg tttaaaaatt taattgagtg attattattt ttttttttaa     8280
   gaattatgtt tataaattat tgattcgaat aaaaagatag gaaatttggg ttttagttta     8340
   gttatttgta gaaaatagaa gaagttatat aggttttcgg tttttagttt tttaatttgt     8400
   aaaataaaat ggttgaattt agttattttt tatggttttt ttagttttaa aaaaattaaa     8460
   agttagtatt attggtttat agtttttttg aaaaaaaaat ataattaatg attttatata     8520
   tattaaaagg tataaagaat atgatttttt agttgggcgt agtggtttag gtttgtaatt     8580
   ttagtatttt gggatgttga ggcggacgga ttatttgggg tcgggagttt aagattagtt     8640
   tgtttaatat ggtaaaattt tatttttatt aaaaatataa aataattagt cgggtgtggt     8700
   ggtgtatgtt tgtaatttta gttattcggg aggttgaggt aggagaatcg ttttaatttt     8760
   ggaggcggag gttgtagtga gttgagatgg cgttattgta ttttattttg gaaaatatag     8820
   tgagattttg ttttaaaaaa aaaaaaaaaa agaatatgat gttttttttt ttattttttt     8880
   ttttaagaat aatatatata taaagaagtt ttttgtagtt atttttatgg tatttttttt     8940
   ttttattttt tgggaattat tattttgaat ttggtatgta tttttgttat gtattttttt     9000
   agatttttag agtttatatg tgtatatatt ttttaaaaat ttgtttatgt ttttaaaatt    9060
   tttataaatg gtattaaatt atatgtggtt gggaggttaa ggcgggtaga tcgtttgagg     9120
   ttaggagttt aagattagtt tggttaatat ggtgaaattt tgtttttatt aaaaatataa     9180
   aattagttag gtgtggtggt gtacgtttgt agttttagtt atttgagagg ttgaggtagg     9240
   aggatcgttt gaatttagga ggcgggggtt gtagtgaatt aagattgcgt tattgtattt     9300
   tagtttgggt gatagagtaa gattttgttt taaaaaaaaa attttttttt gtatattaag     9360
   attatagtaa ggtggtttta ttaggataga atgtattatt taaatttata aatttttttt     9420
   agaatattta gagaaagaat ttttttttgg ttaagagttt tatattaaag aaagaatatt     9480
   taaattagaa gtatgggtaa aagggtttgt agtaattaaa taggttttgt taggtaggta     9540
   ataaagggga gttagtgttt agtttttaga atatgaggta ttttgaatat atattttaga     9600
   gtttggtata ttaagagtat aataggtttt tagatttttt taaggaaatt tataggtttt     9660
   agaattgttt tttcggattt aatttttata ataaatgatg aggtagaaaa gggtaggtat     9720
   gtatattgtt ttttatatat gagaaaaatt gaggtatacg gacgttaaga ttggtattta     9780
   atatttaatt ttatttttat ttaagaggta tttaatagtg tatagttaga gattaaagaa     9840
   agttatattt aaggttgtta tgaggtttat tttgttaagt g                        9881
<210> 55
   <211> 6224
   <212> DNA
   <213> Artificial Sequence
<220>
   <223> chemically treated genomic DNA (Homo sapiens)
<220>
   <221> unsure
   <222> (2227)
<400> 55
   gtatttttat ttgattattt atttgataaa tgaggataga gtttatattt ataaaaggta       60
   aatgataatt tggttttggt taaaaattaa gttatagaga atttttgttt tagttttaaa      120
   ggttttgtgt atcgtggttt agtattttaa agtaggggag aggttgagtt tataagagta      180
   gggagttaag gattttcgtt gttttatttt ggtgtttaat tttatttttt ttaatttgta      240
   tattgagttt tttggggttt gggtttagta aagtgttaaa atggttaatt tatggttaga      300
   attagaaaag tttattttag aaattgggcg attcgggtta tgattgtttc ggttttaggt      360
   tgtagtatat tttttgtgga taataaattt ttatttagtg ttaggcggtg gtgttttggg      420
   tattaaagta tagttgtgaa aattagagat gaggtatttt agattttttt tcgggatatt      480
   agtttttggt aggtttattt gtaattttgg aatattaggg tttggggtag ggagagttag      540
   ttatataata gtttgttttg gttatttatt agtttttgtt tttgagggtt ttacgtttat      600
   ttgtttatgt atttagtttt tttaaattag tatagatggg taggttgttt gtttttaaaa      660
   gtgaaatggg agtttttgaa aatgtatatt tttttaggta tttttttgtg agttttgttg      720
   ttattttaga gaggtggaat agtgattgtc gatttagggg tggtattcgt tggataagat 780
   ttttgttata ttatattgtt gtattttaag gtgaggggtg gaatagaggg ttggattgaa 840
   gattgtgggg aaaagaaaga ttagattgtt atcgtgtgta tgtagaaaag gaagatataa 900
   gaaattttat gttgatttgt ataaagaaaa attgtttttg ttttgagatg ttgttaattt 960
   gtaattttag ttttaatttt gtgtttataa aaatatgtgt tatattgaat taaggtttaa 1020
   gggatttagg gttgtgtagg atgtgttttg ttaatatatg tttgtagata gtatgtttgg 1080
   taaaagttat cgttattttt tattttttat taattaggga tatgatgtat tgtggaaagt 1140
   cgtagggatt tttgtttaag aaagtttggg tatttgttta aggttttttt ttattgagat 1200
   agtttgagat atggtttcgt gggaagggaa agattttatt gttttttagt tcgatatttg 1260
   taaagggttt gtgttgagga ggattagtaa aagaggaagg tttttatttt tcgtatattt 1320
   ttgggaacgg aatgtttcgg tgtaaagtcg attattggtt ttatttattg agataggaga 1380
   aaatcgtttt gtggttggaa gcgaaatatg ttggtagtaa tattgttttg ttattttttg 1440
   ttatattgag atgtttgggt aaagagaaat aaaaatttag tttatatgta tattttagta 1500
   tagtattttt ttttgaattt atttatgatg tagatttttt tatttatatg tttttttgtt 1560
   gatttttttt ttattattat tttgtttatt tcgttttgtt tgaagagggt ttgttttttt 1620
   atatttgtgg gtatttttcg taaggtggag acgagaaatt gagaaaagaa atgatataga 1680
   gataaagtat agaggaagaa aagtgggttt aggggattgg tgtttagtaa ttgaggattt 1740
   atattagtat tggtttttga gtttttttag tatttattga ttattatttt tattattttg 1800
   gtaagaggga tgtggtatta tatattataa ttataaggta atggtgggga gggggttaat 1860
   aggaaaaaat gtgagtaaag gatttagtgt tataagtaag tttaaggaaa ggtgttgtgt 1920
   ttggatgtgt atataggtta gatttatgtt tgattttata taaatatttt agtgtagtaa 1980
   agagtagtat tgtcgttagt atgttttatt tgtagttata aggcggtttt tttttatttt 2040
   agtaaataga atgtatggtt gggttttata tttaagatat tttattttta gggataagta 2100
   ggagatagat gttttttttt tattttaatt gtaaagaggt tttttttttt tattaatatt 2160
   ttttagtatc gattttttac gggtgtttgt aaagaggttt ttttttttta ttaatatttt 2220
   ttagtanaga ttttttacgg gtgtcgggtt gggggattgt aaggtttttt tttttttatt 2280
   aggttatatt ttagataatt ttagtggggg gaaatttgga taatatttag gtttttttgg 2340
   gtagaggttt ttgcggtttt tcgtattgta ttgtgtattt ggttaatcga gaatggagaa 2400
   tggcgatgat ttttattaag tatattgttt gtaaataaat ggttaataag ttatattttg 2460
   yatagtttta aattttttaa attttgattt aatatagtat atgtttttgt gagtataggg 2520
   ttggggttaa ggttatagat taatagtatt ttaaggtaga aaaatttttt ttagtataga 2580
   ttaaaatgga gttttttttg tttttttttt ttatatagat atagtaatag tttgattttt 2640
   tttttttttt tttatattgt cgtatttttt ttgtcgagat agtgaaaata gtaattaata 2700
   aatattgagg gaattcggag atcgatgtcg gttatttggg tttattttat attttgtttt 2760
   tgcgttttat tttttttttt agtttttttt tttatttgac gagaaatatt tataggtgtg 2820
   aaggggtagg atttttttaa aggttaaggt tatggaggtt tttttagtga aaattcgggg 2880
   cggggaggtg aggagagttt tggacgaacg gaagaacggg gaaggttagc gtagggtttg 2940
   agtggttcgt ttatcgatat agatgttttt tagttgtttc ggttttagat ttacgttttt 3000
   gagaatcgcg gttatgattg tcgagagaag ttcgtcgggg gtggtgtttt gtagtagaaa 3060
   gagtagtcgt agtgattttt atttttttat gtttatttta gggagataaa gcgattatag 3120
   ttgggtgcgg agttgttttc ggcgtttagg ataattaaat atacgaatcg gattttagtt 3180
   cgcgtcggcg tttttttata ttcggcgttt agattttcgg gttttatttt gaagtcgtcg 3240
   cggttcgttc ggtagatggt cgtgcgtcgt tcgtgtatta ttattacgtt cgcggtcgag 3300
   gtttgcgggg tatcgtttag gtaaggcgcg gtttgcggta tttagtcgga atcggtcgga 3360
   ttttttaggt ggtttagtat tatttgtagt ttttgtattg cgtaggttaa ttttgtagat 3420
   tagttattag ttcgggaatt gatcgcggag ttaatagata gtcgttcgta tacgcgtaga 3480
   attatatttt agtttttaag gttttaattt cgtttatagt tttgcgaata gacgcgagcg 3540
   attttttaaa agtttcgttt atttttagtt ttagttaatt ataaggagag tcggtttggg 3600
   aattcgtttt ttttcgttat cgtttattaa gttttttata ggagggttta taggtatcgt 3660
   ttagtcgagt tttttattta atgtttcgtt tattttggtt tatcgtagtt aattagaagg 3720
   tgtattttta cgaattttcg tttagaaatg tagatcggaa gtttttttat tttttgtagt 3780
   taaattcgag atttgatttt taaatttttg ttttttgttc gtagttaaac ggaaggttgg 3840
   gtagttagga tcgtttattg tttgggggtt ggagatggaa gtcgaggttt taattttttt 3900
   atttattttt taggaggttt tgttattttt tcggttttag tattcgagtt gggggattta 3960
   aatattttcg agacgttcga gttttgtttc gacgtggttg ggttttttta ttttttcgag 4020
   aagataggga atttgggttt tcgggttggt cgggagaata gattttatag atatgcgggg 4080
   tgtgataagt tttttaattg gagaatgaat attttatatt tttattagtt ttatatttaa 4140
   ttggaatgat tttattagtg tttatttttc gtgttaggtt ggggttttta ttaaggtaga 4200
   gaggtattat gtattcgggg agtttgcgat atttttgata tttagtagat gattaataag 4260
   tatttgtcga atgagtaaat gaatgaatta gggtttttta ggttaggatt gtgtttttaa 4320
   atttaaaggg taaggttacg tgtttttttg atttttaata gtagggttat gtttttattt 4380
   gagattttgg agagtagtgt tgggtttttt atagatatta aaggataggg tgttttgtgt 4440
   tttttaattt agtgttatga cgtttttaat tatttcgagg gtagggttac ggtatgtttt 4500
   aggttttaga aggtagttgt tcgtttttta tttttatttc ggggagtata gtaggggttt 4560
   ttttatattt ttattggtag gattgcgtgt tttttagaga tagtggaggg tagagttggt 4620
   gtttttttat ttgttttttt ttcgttattt tcgagggtag gatcgtatga attttttaga 4680
   ttttttcgtg gaagaattgt ggttttatta gattttaaaa agtaaggttc gttttttata 4740
   attttcgaag gagggtcgtt tttatttcgt cgttaattta ttagtattat tattagattt 4800
   tcgagggcgg tgtcgtggat ttttttagat tttaaaaggt agatttttat tttttacgtt 4860
   ttttatatta ttcgtttcga gattttaagg gtagaggtgg gtattttcgt tttcgtattt    4920
   ttgttcgggg ttttagattg tagggtargg cggcgttttt cggaaagcga aagcggcggg    4980
   gcghggcggg tgttaggaga aagaggaagc gttggtagat aatgcgattc gatcgcgttg    5040
   aggttttagg atcgttcgtt atggttgtag gaggtttcgg cgcggggttt gcggtttcgg    5100
   ttttttttat attttttttt tttttggttg tttttaatat gcgagtgcgg cgtcgtttgt    5160
   ttttgttttt gaacgtgcgg atataggtgg cggtcgattg gatcgcgttg gcggaggaga    5220
   tggattttga gtatttggag attcggtaat tggagatata agcggatttt attggtaggt    5280
   tgttggacgt ttggtaggga cgttttggcg tttttgtagg tcgattgttc gagttgttta    5340
   ttaagttggg ttgcgacgac gtgttgttgg agttgggatt tagtattggt gaggacgttt    5400
   tttttttggt ttcgtatttg gggggtgagg aggttgattt ttcgcggttt tagtattttg    5460
   ttttttatgg agagatttta ttttttgttt cgggggttcg aagaagtttg tagagggaga    5520
   attatgcggg tttcgttttt ttttaataat cggtcgcggt tattaagaag gattggagaa    5580
   aggttcggat aggcggagat gggaaggaag tagtttaggt agaggttttt aggtagggtt    5640
   aggagttaga attaggtttt tgtgggggta tttgggttgt tttaagtaga gtaataggat    5700
   aggtggggcg attgatagtg gattgtttta gaaattttaa gttttgggga aatgtagttt    5760
   ttttttttat ttattggtat ttatataata tatatgtata ggcgttggat atagtcgttt    5820
   atagataggt atattttgtt gagttggaat tattgtatta taattagtgg gttttttgag    5880
   tttttttggt atgtttagtt ttttgtttat atttgttttg gattttagaa gaagtagatt    5940
   tattttggta ttatttttag gatttttagg aagggataga gatataaatt tgattttgat    6000
   ggttttttag aaagttagaa tattattgat atttttttgg gttagttaga gttagtggga    6060
   gtttaatttt ttagagtcgt tgagtttcgc gtggtattag tgaattgggg aagtttttta    6120
   gaataattta gttagaggag gtgggatagc ggttggattt tgattgtggg taaagaggta    6180
   ggtattttta gggaggttgt tttattttgt ttttttttta taga                     6224
<210> 56
   <211> 6224
   <212> DNA
   <213> Artificial Sequence
<220>
   <223> chemically treated genomic DNA (Homo sapiens)
<220>
   <221> unsure
   <222> (3998)
<400> 56
   tttgtgggga agagatagag taaagtagtt tttttgggag tgtttatttt tttatttata      60
   gttaggattt agtcgttgtt ttattttttt tggttgggtt gttttagagg gtttttttag    120
   tttattggtg ttacgcgaag tttaacggtt ttgagaagtt gagtttttat tggttttaat     180
   tgatttaaag ggatgttagt ggtgttttgg ttttttggaa ggttattaaa gttaggtttg     240
   tatttttgtt tttttttagg gattttaaga atggtattaa ggtaggtttg ttttttttgg    300
   gatttagggt agatgtgagt aaggggttgg gtatgttaga aaggtttagg agatttattg     360
   gttatggtgt agtgatttta atttagtaag gtgtgtttgt ttgtgggcgg ttgtatttaa     420
   cgtttatgta tgtatattat gtaagtgtta gtgagtagaa agaagggttg tattttttta     480
   ggatttgagg tttttaagat agtttattgt taatcgtttt atttgttttg ttgttttatt     540
   tgaaatagtt tagatgtttt tatagaagtt tgattttgat ttttggtttt atttgaaagt     600
   ttttgtttaa gttgtttttt ttttattttc gtttattcgg attttttttt agtttttttt    660
   aataatcgcg atcggttatt aagaaggaac gggattcgta tggttttttt tttgtaggtt    720
   ttttcgggtt ttcgaggtag gaaatggggt ttttttatgg gagataggat gttgaggtcg     780
   cggaaagtta gtttttttat tttttaggta cgaggttagg aaggggacgt ttttattaat     840
   gttgggtttt agttttagta gtacgtcgtc gtagtttagt ttggtaagta gttcgagtag     900
   tcggtttata gaggcgttag ggcgtttttg ttaggcgttt agtagtttgt tagtggggtt     960
   cgtttgtgtt tttagttgtc ggatttttaa gtatttaaag tttatttttt tcgttagcgc    1020
   ggtttagtcg gtcgttattt gtgttcgtac gtttaagaat agagataggc ggcgtcgtat    1080
   tcgtatgttg agagtagtta ggggaaggga ggatgtggag gagatcgggg tcgtagattt    1140
   cgcgtcggga ttttttgtag ttatggcggg cggttttgga gttttagcgc ggtcgggtcg   1200
   tattgtttgt tagcgttttt tttttttttt ggtattcgtt dcgtttcgtc gttttcgttt    1260
   ttcgagaagc gtcgttytgt tttataattt ggagtttcga gtaaaagtgc ggaggcgggg    1320
   gtgtttattt ttatttttga ggtttcgagg cgggtgatgt ggggggcgta agaagtagga    1380
   atttgttttt tgagatttgg agaggtttac ggtatcgttt tcgagggttt ggtgatggtg    1440
   ttagtaggtt ggcggcggag tgaggacgat tttttttcgg gggttgtagg aaacgggttt   1500
   tgttttttgg gatttggtgg agttatagtt tttttacgga ggaatttgag gggtttatac    1560
   ggttttgttt tcgaggatgg cgaggagggg ataagtggga aggtattagt tttgtttttt    1620
   attgtttttg gggaatacgt aattttgtta gtaggggtat gggaagattt ttgttgtgtt     1680
   tttcgaggtg ggagtgggaa acggatagtt gttttttgga gtttgaggta tgtcgtggtt    1740
   ttgttttcga aatggttggg gacgttatga tattaggttg gggagtataa ggtattttgt    1800
   tttttggtgt ttgtggaaag tttagtatta ttttttaggg ttttaggtga agatatggtt    1860
   ttgttattgg gagttaaggg ggtacgtggt tttgtttttt aggtttaggg atatagtttt 1920
   gatttgggga gttttggttt atttatttat ttattcgata agtatttatt gattatttat 1980
   taagtgttaa gggtgtcgta ggtttttcgg atatatggta tttttttgtt ttggtggaga 2040
   ttttagttta gtacgggaaa tgggtattaa taaaattatt ttaattgggt gtaaaattgg 2100
   taaaagtgtg aaatgtttat tttttagtta gagagtttgt tatatttcgt atgtttgtaa 2160
   aatttgtttt ttcggttagt tcggaagttt aaattttttg ttttttcgaa agaatgggaa 2220
   agtttaatta cgtcggagta ggattcgggc gtttcggaag tatttaggtt ttttagttcg 2280
   ggtattggga tcggagggat agtagagttt tttaggaagt gggtagagga attagagttt 2340
   cggtttttat ttttagtttt taagtagtaa gcggttttgg ttgtttagtt tttcgtttgg 2400
   ttgcgggtag gagataggga tttgggggtt aggtttcggg tttggttata aaaggtggaa 2460
   agattttcgg tttgtatttt tgggcgggga ttcgtaaaga tatatttttt ggttggttgc 2520
   ggtaggttaa ggtaggcggg gtattgagtg gaaagttcgg ttgggcggtg tttgtgggtt 2580
   tttttataga gggtttggta ggcggtggcg gggaggggcg ggtttttagg tcgatttttt 2640
   ttgtggttgg ttgaggttgg aggtggacgg gatttttgga gggtcgttcg cgtttgttcg 2700
   tagagttgtg ggcggagttg aggttttgga ggttgagatg tggttttgcg cgtgtgcgga 2760
   cggttgtttg ttaatttcgc ggttagtttt cggattggtg gttggtttgt agggttgatt 2820
   tgcgtaatgt agaggttgta ggtagtgttg ggttatttga ggggttcggt cgatttcggt 2880
   tggatgtcgt aggtcgcgtt ttgtttgagc ggtgtttcgt aggtttcggt cgcggacgtg 2940
   gtggtggtgt acgggcggcg tacggttatt tgtcgggcgg gtcgcggcgg ttttaaggtg 3000
   aggttcgagg gtttgggcgt cgagtgtggg aagacgtcgg cgcgggttgg ggttcggttc 3060
   gtatgtttgg ttattttgga cgtcggaggt agtttcgtat ttagttgtgg tcgttttgtt 3120
   tttttggggt gggtatgggg gagtgggggt tattgcggtt gttttttttg ttgtaggata 3180
   ttattttcga cgagtttttt tcggtagtta tgatcgcggt ttttaaggac gtgaatttga 3240
   ggtcggaata gttgggggat atttgtgtcg gtgagcgaat tatttaggtt ttgcgttggt 3300
   ttttttcgtt ttttcgttcg tttagagttt tttttatttt ttcgtttcga atttttattg 3360
   ggggaatttt tatggttttg atttttgaag gggttttgtt tttttatatt tgtgggtatt 3420
   tttcgttaga tgggaggaga gattgagaaa agaaataaga cgtagagata aagtgtagag 3480
   tgggtttagg tgatcggtat cggttttcga gtttttttag tatttattga ttattatttt 3540
   tattatttcg gtaaggggag tgcggtagtg tgggggaaag gaagagagat tagattgtta 3600
   ttgtgtttgt gtagaaaagg aagatagaag aaattttatt ttgatttgta ttaagaaaaa 3660
   tttttttgtt ttgagatgtt gttaatttgt agttttagtt ttaattttgt gtttatagaa 3720
   atatgtgttg tattgaatta aggtttgagg gatttagggt tgtrtaggat gtggtttgtt 3780
   aattatttgt ttgtaggtag tatgtttggt aaaagttatc gttatttttt attttcgatt 3840
   aattaggtat atagtgtaat gcgaaaggtc gtagggattt ttgtttaaga aagtttgggt 3900
   attgtttagg ttttttttta ttgagattgt ttgagatatg gtttagtggg aagggaaaga 3960
   ttttatagtt ttttagttcg atattcgtaa agggtttntg ttgaggagta ttagtaaaag 4020
   aggaaggttt ttttgtagat attcgtaaag ggtcggtgtt gaggagtatt agtaaaagag 4080
   gaaggttttt ttgtagttga gataagagga aagtatttgt tttttgtttg tttttgggaa 4140
   tggaatgttt tgggtgtaaa atttaattat atattttatt tattgagata ggagaaaatc 4200
   gttttatggt tgtaggtgag atatgttggc ggtaatattg ttttttattg tattgagatg 4260
   tttgtgtaaa gttaaatata aatttggttt atgtgtatat ttaggtatag tatttttttt 4320
   taaatttatt tatgatattg agttttttgt ttatattttt ttttgttgat ttttttttta 4380
   ttattatttt atagttatgg tatataatgt tatatttttt ttgttaagat agtagagata 4440
   gtgattaata aatattgagg gaatttagag attagtgttg gtgtaggttt ttaattgttg 4500
   agtattagtt ttttgggttt attttttttt ttttatattt tgtttttgtg ttattttttt 4560
   ttttagtttt tcgtttttat tttgcgagaa atatttatag gtgtggaggg gtaggttttt 4620
   tttaggtaaa acggggtgaa tagggtgatg gtggggagaa ggttagtagg aaaatatgtg 4680
   agtaaaggaa tttgtattat aaataagttt aagggaaggt attgtgttga gatgtgtatg 4740
   taggttagat ttttgttttt ttttatttag atattttagt gtagtaaaga gtaatagagt 4800
   agtattgttg ttagtatatt tcgtttttag ttatagggcg gttttttttt attttagtaa 4860
   atagaattaa tagtcggttt tatatcgaga tatttcgttt ttagggatgt gcgggaaata 4920
   gaggtttttt ttttttatta atttttttta gtatagattt tttataggtg tcgggttggg 4980
   ggatagtaag gttttttttt ttttacgagg ttatatttta ggttgtttta gtggggggaa 5040
   attttggata aatatttagg tttttttggg tagaggtttt tgcggttttt tatagtgtat 5100
   tatgtttttg gttaatagag aatggagaat gacgatgatt tttattaagt atattgtttg 5160
   taaatatatg ttgataaggt atattttgta tagttttaaa ttttttaaat tttgatttaa 5220
   tatagtatat gtttttgtga gtatagagtt ggggttaaag ttatagatta atagtatttt 5280
   aaagtagaaa taattttttt ttgtatagat taatatggag ttttttatgt tttttttttt 5340
   tatatatata cggtaataat ttgatttttt ttttttttat aatttttagt ttagtttttt 5400
   attttatttt ttattttgaa atatagtaat atgatatggt aagagtttta tttagcggat 5460
   gttattttta ggtcggtaat tattgtttta tttttttggg gtgatagtag ggtttatagg 5520
   aggatatttg gggagatgtg tatttttaga aatttttatt ttatttttaa gggtagataa 5580
   tttgtttatt tgtgttgatt tggaaaagtt gggtgtatgg gtaagtgagc gtgggatttt 5640
   tagggatagg agttggtgga tgattaggat aagttgttgt atggttggtt ttttttgttt 5700
   taaattttga tgttttagag ttgtaagtga gtttgttaag ggttagtgtt tcgaagaaag 5760
   atttggaatg ttttattttt ggtttttata gttatatttt agtgtttaga atattatcgt 5820
   ttggtattga atgaaagttt gttgtttata ggaaatgtgt tgtagtttgg ggtcggggta 5880
   attatggttc gaatcgttta gtttttgagg taaatttttt tagttttagt tatgggttgg 5940
   ttattttgat attttattgg gtttaggttt taggaaattt agtgtgtagg ttggggagga 6000
   taaggttgga tattagaatg aggtaacgag agtttttggt tttttgtttt tgtggattta 6060
   attttttttt tattttgaag tgttgagtta cgatatataa aatttttggg attggagtag 6120
   aagttttttg tagtttgatt tttagttaaa gttaggttgt tatttgtttt ttgtgagtgt 6180
   ggattttgtt tttatttgtt aggtgagtaa ttaagtaaga atgt 6224
<210> 57
   <211> 5291
   <212> DNA
   <213> Artificial Sequence
<220>
   <223> chemically treated genomic DNA (Homo sapiens)
<400> 57
   aagtgattta tttattttgg ttttttaaag tgttgggatt ataggtgtga gttatcgtgg 60
   ttagttttag gggataattt aggttttttg tgtttttgtt ttaaatttaa atttttattt 120
   ttttgaggta gtattatttt gttttttagg ttggagtgta atggtataat taatttattg 180
   tagttttgaa tttttgggta taagtgattt ttttaattta gttttttaag tagttaggat 240
   tataggtgtg tattattatg tttggttgaa tttttttttt aagagatgga tttttgttgt 300
   gttgtttagg ttcgtgttaa tttttggttt taaatgattt ttttttttag gttttttaaa 360
   gtgttgggat tatagacgtt agttattgta tttagtttag tttaagtttt aagtattaag 420
   aattgatttt ttggaggaat agggattttt taaggatttt tatagttagt gattttaaat 480
   gtagtgtttg gattagtaag tagtattagt attatttaag aagttattag aaatgtaaat 540
   ttttggttag gcgcggtgtt ttatgtttgt aattttagta ttttgggagg ttgaggtggg 600
   tagattacga ggttacgagt tcgagattag tttgattaat atggtgaaat tttgttttta 660
   ttaaaaatat aaaaattagt cggtcgtggt gaagggtatt tgtaatttta gttatttagg 720
   aggatgaggt aggagaatta tttgaattta ggaggcggag gttgttgtga gttaagatcg 780
   tattattgta ttgtagtttg ggtaatagaa tgagatttta ttttaaaaaa aaaaaaaaaa 840
   gtaaattttt gagttttatt ataggtttgt tgaattagaa attttgagga tgggtttagt 900
   agttcgtttt aataaatttt ttaggtggtt attataaatg ttgaagtgtg agagttattg 960
   tttgtaattt tatttagaga ggtatatagg taatttaaaa ataggttttg ataaaagaat 1020
   tttattaaat ttaaaaaata aatttaggta attttataaa taaataaatt tcggtcgggc 1080
   gcggtggttt acgtttgtaa ttttagtatt ttgggaggtc gaggcgggcg gattacgagg 1140
   ttaggagatc gagattattt tggttaatac ggtgaaattt cgtttttatt aaaaatataa 1200
   aaaattagtc gggcgtggtg gcgggcgttt gtagttttag ttattcggga ggttgaggta 1260
   ggagaatggc gtgaattcgg gaggtagaaa ttttagtgag tcgagatcgc gttattgtat 1320
   tttagtttgg gtgatagagt aagatttcgt ttgaaaataa ataaataaaa aaaataaatt 1380
   ttaaatatgg aattattttt ttattttttt tttttgtttt gttttttttt ttttttgaga 1440
   tagagtttcg ttttgttatt taggttggag tgtagtggcg cgatttcggt ttatattgta 1500
   atttttgttt ttcgggttta tattattttt ttgttttagt ttttcgagta gttgggatta 1560
   taggtgttcg ttattacgtt cggttaattg tttttttgta tttttagtag agacggggtt 1620
   ttatcgtgtt agttaggatg gtttcgattt tttgatttcg tgattcgttc gtttcggttt 1680
   tttaaagtgt tgggattata ggcgtgagtt attgcgttcg atttttttta ttttttttaa 1740
   tgttttgtaa atattaatgt atttatatat gtatttagaa ttttaaaaaa ttaattttat 1800
   tgagttataa ttaatatata gtaaaaatgt ttttattttg agtaatttta tgttttttga 1860
   taagtgtttt gtatttatgt tacgattatt ataatcgaga gagaatattt ttattatttt 1920
   agaagggttt ttttgtagtg agtatttttt aggagtttta gcggtcggtg atattgattt 1980
   gttttttgtt attgtagatg agatttgttt gttatatata atttttaaaa attaaatgat 2040
   atgtatggtt ttttttgttt agtataatgt ttttgagttt atttatttgt tgtatatatt 2100
   aatattttgt ttttttttat tatttgtatt ttatttatgg atacgttgtt tatttatgtg 2160
   tttattttta atggatattg ggttgttttt gattttttgg ttattattat gaataaagtt 2220
   gttatgaata ttattgtata aatttttgtg tgtttatgtg tttttatttt ttttgggtaa 2280
   atatgtagga atggaattgt tagattgtat ggtaagagta tatttagttt tttatgatat 2340
   tagtgaattg tttttttaaa gatattgtat taatattatt ttatattttt ataattaatg 2400
   tatgagtttt tagttgtttt atatttttaa ttaatagttg atattgtata atataaatgt 2460
   taatttttag aatattttta taattttaga gtagatagtg attttttagg atataaaaat 2520
   tattaattat aataaaaatt aaaattggat tttattaaaa ttaaaaattt ttttttgaaa 2580
   gatattatta agaaaatgaa aagatggtta atagattgag agaatatatg tgtaatgtaa 2640
   atatttaata aaggattagt atgtaaaata tataaagaat ttttataatt tagtaataaa 2700
   aaggaaaata cgtatttttt aaatgggtta agaatttaat tacggttagg tgtagtggtt 2760
   tatatttgta attttagtat tttgggaggt cgaggtgggt ggattacgag gttaggggtt 2820
   cgagattagt ttggttaata tggtgaaatt ttgtttttat taaaaatata aaaattagtt 2880
   gggcgtggtg gtgggcgttt gtaattttag ttattcggga ggttgaggta ggggaatcgt 2940
   ttgaatttag gaggcggagg ttgtagtgag ttgagattgt gttattgtat tttagtttgg 3000
   gcgatagagc gagattttgt tttttaaaaa aaaagaaaaa aatttaatta ttttattaaa 3060
   gaagatatat gaatgattaa taagttatta aattaaaatt ttagtgagta tatatatatg 3120
   ttttgaagta gttaaatttt aaaatattgt tttatgtgtg tgtatttttt agttaaaaaa 3180
   gtgtttgaaa atagtattat tttgaaattt aaaattttaa attttttttt taaattaggt 3240
   agtagtgtta ttggaagtaa aagagaagtt attttaattg ttaaagagta tttattaaga   3300
   atttataata aatattatgt ttaatgttta aattttagaa gaatttatat taaaagttaa   3360
   gaataagata ggatatttat ttttattatt aagtaatatt atattagagg atttaattaa   3420
   tgtaataagg taataaataa aagataaaat tgttataatt agatattata aatatttaag   3480
   aaaatttagg gggatttata gttatatatt aaatagttta gaaggagttt tatatattat   3540
   taatgaatat tgagaataaa aaatagtttt taaaattttt ttaatttata agggtaataa   3600
   aatgtataag gtattaaaat aaatgtaatg tgtatttggg tataaatata gtaatataat   3660
   gaaaaggttt attggggaaa aaattatagg gagaagggtt aataacgtga agaggttttt   3720
   ttgttatttg gatttttgga aaaagatttg ttggtttagg tatagttgga tgtattatgg   3780
   aaagtggttt taataaatat tggtattgta ataattgtgt atgttgatat aaaaagaggg   3840
   agttaatgaa tatttggtcg attatttaat aattgttttt ttgttgaaga aatggttttg   3900
   ttggattaga gtttaattgt ggtttatata ttaatgttgt tataatagtt agaagtttta   3960
   ttaagaaatt tgttaaatta ttaaaaagag gaaatgaatt ttttaggata tttggattgt   4020
   ttttttttaa aaatttagtt ttggttgggt gttgtggttt acgtttgtaa ttttagtatt   4080
   tggtgaggtc gaagtgggcg gattacgagg ttaagagatc gagattattt tggttaatat   4140
   ggtgaaattt tgtttttatt gaaaatataa aaattagttg ggcgaggtgg tatgtgtttg   4200
   tagttttagt tgttggggag gttgaggaag gagaattatt tgaattcggg aggcggggtt   4260
   tgtagtgagt tgagattacg ttattgtatt ttagtttggt aatagagtaa gatttagttt   4320
   aaaaaaaaaa aaaaaaaatt tagtttgtat ggtagtataa tattaggtaa aagtatagat   4380
   tagttagatt attggttaat tttaattttt atgtgtttga gtttttttat ttgtaatatg   4440
   gagatgatat agtaatagtt gattttggat tgttaaagga attaagtgga tatatgtaaa   4500
   gtgtttagaa ttgtgtttgg taagtagtag gttgtaatat tgtgatataa taaatatata   4560
   tatttgattt ttatttagtt tttggtatag atttttagaa atttttgtaa ttttttgagt   4620
   gataggggtg atagaaatat tttttattag aatatttggt tttggttttt gatataagag   4680
   tttttaagat ttttggaatt tttaagtgat aagagtgtat gatagtgagt taattggtgg   4740
   ttgggatttt ttagataatt ttaggatggg ggttattttt tgaaagatta aggtatgatt   4800
   agaggtttgg gatttgtagt tttacgtttc gatttttaga gagggtaaaa gggttggcga   4860
   ttgattaatt attaattgtt agtgatttag ttaattatgt ttaagtgatg gtatttttat   4920
   taaaaaataa attataggtt tggagagttt tcggtttggt taattttaat tatatattaa   4980
   gaaggcgatg tattttaaat tgtatgaaga taaaaggttt tgtgtttatt tgggattttt   5040
   ttggacgttg ttttgtgtat tttttcgatt gtttgtttat ttgtattttt tataataaag   5100
   tagtaaatat aagtaaagtt tttgagtttt gtgagttatt ataagaaacg atcgaatttg   5160
   ggattttttt ttcggaagtc gttttttttt ataagggaga gagttgtttt tttttttttt   5220
   ttgcgtgtta aattttcgtt tttaaattta tttttcgtgt gtatcgtgtt tttaattttg   5280
   ttggtgcgag a                                                         5291
<210> 58
   <211> 5291
   <212> DNA
   <213> Artificial Sequence
<220>
   <223> chemically treated genomic DNA (Homo sapiens)
<400> 58
   tttcgtatta ataaggttaa ggatacgata tatacgaaga gtgggtttag gagcggaggt     60
   ttaatacgta aaagaaaaag gagaatagtt tttttttttg tgagagagag cggttttcga    120
   aaggaaaatt ttaggttcga tcgtttttta taatggttta tagaatttag aaattttatt    180
   tatgtttatt gttttattat aaaggatata gatgaatagg tagtcgaaga ggtatatagg    240
   gtaacgttta gaagggtttt aggtgagtat aggatttttt gtttttatgt agtttgaggt    300
   gtatcgtttt tttggtatgt ggttggggtt aattaaatcg aaagtttttt aaatttgtgg    360
   tttatttttt aatggaggtg ttattattta ggtatgattg gttaaattat tggtaattgg    420
   tggttaatta atcgttagtt tttttatttt ttttggaggt cgaggcgtgg ggttgtaaat    480
   tttagatttt taattatgtt ttagtttttt aggggatagt ttttattttg aagttgttta    540
   aaggatttta gttattagtt agtttattgt tatatatttt tattatttgg agattttaaa    600
   ggttttagaa gtttttgtgt taggaattaa gattaagtat tttaataaaa gatgttttta    660
   ttatttttgt tatttaggaa attataaggg tttttggaga tttgtgttag gaattggatg    720
   aagattaaat atatatattt attatattat aatattgtag tttattattt gttaggtata    780
   attttaagta ttttatatgt gtttatttaa tttttttaat agtttaaaat tagttattgt    840
   tatattattt ttatattata gataaggaaa tttaagtata taggggttaa ggttaattag    900
   tgatttggtt agtttgtatt tttatttgat gttgtgttgt tatatagatt agattttttt    960
   tttttttttt tagattgagt tttgttttgt tgttaggttg gagtgtaatg gcgtgatttt   1020
   agtttattgt aaatttcgtt tttcgggttt aagtgatttt ttttttttag tttttttagt  1080
   agttgggatt ataggtatat gttatttcgt ttagttaatt tttgtatttt tagtagagat   1140
   agggttttat tatgttgatt aggatggttt cgattttttg atttcgtgat tcgtttattt   1200
   cggttttatt aaatgttggg attataggcg tgaattatag tatttagtta agattagatt   1260
   tttaagaaaa ggtaatttag atgttttaga aggtttattt tttttttttg gtggtttagt   1320
   aaatttttta gtaaggtttt tagttgttat agtagtatta gtatgtgaat tatagttaag   1380
   ttttagttta ataaggttat ttttttagta gagaagtaat tgttaaataa tcgattaagt     1440
   atttattggt tttttttttt tgtattaata tatataattg ttatagtgtt agtgtttatt     1500
   ggaattattt tttatggtgt atttagttgt gtttagatta gtaggttttt ttttagaagt     1560
   ttagatggta ggggaatttt tttacgttgt tgattttttt ttttgtaatt ttttttttaa     1620
   taaatttttt tattatgttg ttgtatttat atttaaatat atattatatt tattttagta     1680
   ttttgtatat tttgttgttt ttataaattg gaggggtttt aaaaattatt ttttattttt     1740
   aatatttatt gatgatgtat aggatttttt ttggattgtt taatatatga ttgtaaattt     1800
   ttttgaattt ttttagatgt ttgtagtatt tgattatgat agttttgttt tttatttatt     1860
   gttttgttgt attgattagg ttttttaata taatgttgtt taataatgaa gatagatatt     1920
   ttattttatt tttgattttt ggtataaatt tttttaaagt ttgagtatta aatatgatgt     1980
   ttgttgtaga tttttagtgg atatttttta gtagttaaag taattttttt tttattttta     2040
   gtgatattat tatttaattt gaagagagga tttggaattt tgagttttaa aataatattg     2100
   tttttaaata tttttttagt taaaaagtat atatatatag ggtagtgttt taaagtttag     2160
   ttattttagg gtatatgtgt atatttattg aagttttaat ttaatggttt attggttatt     2220
   tatgtatttt ttttggtaag gtaattaaat tttttttttt ttttttaaaa gatagagttt     2280
   cgttttgtcg tttaggttgg agtgtagtgg tataatttta gtttattgta attttcgttt     2340
   tttgggttta agcgattttt ttgttttagt ttttcgagta gttgggatta taggcgttta     2400
   ttattacgtt tagttaattt ttgtattttt agtagagata gggttttatt atgttagtta     2460
   ggttggtttc gaatttttga tttcgtgatt tatttatttc ggttttttaa agtgttgaga     2520
   ttataggtgt gagttattgt atttggtcgt aattaaattt ttgatttatt taaaaagtgc     2580
   gtgttttttt ttttattatt aaattataag gattttttat atattttgta tattagtttt     2640
   ttgttagata tttatattgt atatatattt ttttagtttg ttaattattt ttttattttt     2700
   ttaatggtgt tttttaaaaa gaagttttta attttgatga agtttaattt taatttttgt     2760
   tatgattgat agtttttgtg ttttaagaaa ttattattta ttttaaagtt gtgaagatat     2820
   tttaaaagtt aatatttgta ttgtataata ttaattgtta gttgaggata tggagtaatt     2880
   ggaagtttat atattagttg tgggaatgta aaatggtatt aatataatgt ttttggaaaa     2940
   atagtttatt ggtgttatgg aaagttaagt atatttttgt tatataattt agtaatttta     3000
   tttttatata tttatttaag aaaaatgaaa atatatgaat atataaagat ttatataata     3060
   atgtttatag taattttatt tataataata attaaaaaat tagaaataat ttaatgttta     3120
   ttggggataa atatatggat aaataacgta tttataaatg aagtataggt ggtaaaaaga     3180
   agtaaagtat tgatatatgt aataaatgaa taagtttaaa aatattatgt taagtaaaag     3240
   aagttatata tattatttaa tttttaaaaa ttgtatataa tagataaatt ttatttatag     3300
   tgatagaaaa tagattaatg ttatcggtcg ttggaatttt tagggagtat ttattgtaaa     3360
   ggagtttttt tggagtgatg aagatgtttt ttttcgattg tggtggtcgt ggtatgggta     3420
   tagaatattt gttaaaaggt atggaattat ttaagatggg agtattttta ttgtatgtta     3480
   attataattt aataaaattg atttttaaaa attttaagta tatatataaa tatattaatg     3540
   tttatagagt attaagagaa gtgaaaaagg tcgggcgtag tggtttacgt ttgtaatttt     3600
   agtattttgg gaggtcgagg cgggcggatt acgaggttag gagatcgaga ttattttggt     3660
   taatacggtg aaatttcgtt tttattaaaa atataaaaaa ataattagtc gggcgtggtg     3720
   gcgggtattt gtagttttag ttattcggga ggttgaggta ggagaatggt gtgaattcgg     3780
   gaggtagaaa ttgtagtgtg agtcgagatc gcgttattgt attttagttt gggtgataga     3840
   gcgagatttt gttttaaaaa aaaaaaaaaa taaaataaaa gagagaagtg aaaaaataat     3900
   tttatgtttg agatttgttt tttttgtttg tttgttttta gacggagttt tgttttgtta     3960
   tttaggttgg agtgtagtgg cgcgatttcg gtttattgaa atttttgttt ttcgggttta     4020
   cgttattttt ttgttttagt ttttcgagta gttgggatta taggcgttcg ttattacgtt     4080
   cggttaattt tttgtatttt tagtagagac ggggttttat cgtgttagtt aggatggttt     4140
   cgattttttg atttcgtgat tcgttcgttt cggtttttta aagtgttggg attataggcg     4200
   tgagttatcg cgttcggtcg agatttgttt gtttataaag ttatttgaat ttgtttttta     4260
   agtttagtag aattttttta ttaggatttg tttttaagtt atttatgtat ttttttaaat     4320
   gggattatag gtagtagttt ttatatttta atatttatgg taattatttg gaggatttgt     4380
   taaagcggat tgttagattt atttttagag tttttgattt aataggtttg tgatggggtt     4440
   taagaatttg tttttttttt tttttttgag atggagtttt attttgttgt ttaggttgta     4500
   gtgtagtggt gcgattttgg tttatagtaa ttttcgtttt ttggatttaa gtgatttttt     4560
   tgttttattt ttttgagtag ttgggattat agatgttttt tattacggtc ggttaatttt     4620
   tgtattttta gtagagatag ggttttatta tgttggttaa gttggtttcg aattcgtgat     4680
   ttcgtgattt atttatttta gttttttaaa gtgttgggat tataggtatg aggtatcgcg     4740
   tttggttaag aatttgtatt tttaatgatt ttttgggtga tgttaatgtt atttattggt     4800
   ttaaatatta tatttagagt tattgattgt agagattttt aaaagatttt tattttttta     4860
   aagagttaat ttttaatgtt taaaatttga attaggttgg gtatagtggt taacgtttgt     4920
   aattttagta ttttggaagg tttaggagag aggattattt gaggttagga gttgatacga     4980
   gtttggatag tatagtaaga gtttattttt taaaaaaaaa atttagttag gtatggtggt     5040
   gtatatttgt agttttagtt atttgagaag ttgaattggg aggattattt gtgtttagga     5100
   atttaaggtt gtagtgagtt gattgtgtta ttgtatttta gtttgagaga tagagtgata     5160
   ttgttttaaa aaaataaaaa tttaaattta aaatagaaat ataaaaaatt taaattattt     5220
   tttaaggttg gttacggtgg tttatatttg taattttagt attttggaag gttaaggtgg     5280
   gtggattatt t                                                          5291
<210> 59
   <211> 5572
   <212> DNA
   <213> Artificial Sequence
<220>
   <223> chemically treated genomic DNA (Homo sapiens)
<400> 59
   agaaagtaga aggaaagttt tataaaagaa agagaatagg ttaggtacgg tcgttacgtt     60
   tttaatttta gtattttggg aggttgaggt aggtggatta tgaggttagg agtttaagat    120
   taatttgatt aatatggtga agtttcgttt ttattaaaaa tataaaaatt aattaggcgt    180
   gtgtgtttgt aattttagtt atttaggagg ttgaggtagg agaatcgttt gaattcgaaa    240
   ggtgaaggtt gttgtgagtt tagattaggt tattgtattt tgatttgggc gatagagcga    300
   gattttatgt taaagaaaaa gaaagaggat aagaaaattt tttaattgga aggtagatag    360
   ttgattaaaa gggtttattg attgtataat ataataatga taaaagatta aattagagta    420
   tatttttaag atattttaga ggatttaagt aagaagattt aaaaattttg agatagaaaa    480
   tataatgtaa ttagaatgtt attggttttt taaatagtaa ttttggaaat tagatgataa    540
   taaagtaatg tttttaaaat tatgaaggaa aatgtttttt aatttagagt tttatgtttt    600
   attaaattat taattaagta tgaagataaa tttaaaatat tttttaatat gtaaggtttt    660
   taagaatgag ttatattatt tttagaatat attgaggata tattttgtta aaatgaaggg    720
   gagaaataaa aagagaaaag tatgtaattt aggaaataag aagtttatag agaaaatgat    780
   ttttaaggtg ttagaggagt ataattttag gatgattata agtaacgagt tttaaaatta    840
   gtttagatta ggttaggtgc ggtggtttat atttgtaatt ttagtatttt gggaggttaa    900
   agtaggttgg ttgtttgagt ttagaagttc gagattagtt tgggtaatat ggtgaaattt    960
   tcgtttttat taaaatataa aaaattagtt gggcgtggtg gtatgtgttt gtatttttag   1020
   ttattttgga ggttgatgta ggagaattgt ttgaatttag gaggcggagg ttgtagtgag   1080
   ttaagattgc gttattgtat tatagtttta ttaatagagc gagatttcgt ttttaaataa   1140
   ataaataaaa ttaatttata ttaaagtagg ggatggaggg ttttagaata gatgttttta   1200
   aaaagagaat agaattgata gtttatttaa tgtgattaac gttattgaga ggaggaaaat   1260
   ttgagtatat atttgtgatt ggtatataaa aaaataagtc gatgattaaa gaaaaaaaaa   1320
   gaggtaagtt ttaattgtag aaaaatggta aagataaaag gtatagttgt gtaataagga   1380
   aaaatagttg taaaaaaaaa gaaatgtaat tatatatatt atatgattta gttatgaata   1440
   gtatttgtat agttataata ttacgggcgt gtaggagtat gaaaagtata tgtgtggtcg   1500
   ggtatggtgg tttatgtttg taattttaga attttgggag gtcgaggcgg gtggattacg   1560
   aggttaggag atcgagatta ttttggttaa tatagtgaaa tttcgttttt attaaaaatg   1620
   taaaaaaaaa aaaaaaaaaa aaaaaaaatt agtcgggcgt ggtggtagtt atttgtagtt   1680
   ttagttattt aggaggttga ggtaggagaa tggcgtgaat tcgggaggcg gagtttgtag   1740
   tgagtcgaga tcgcgttatt gtattttagt ttgggagata gagcgagatt ttattttaaa   1800
   aaaaaagaaa aaaaaaagaa aaagaaaaga aaagtatatg tgttattagt gtattagagt   1860
   taaatttttt tttatattta aaaatggaaa aattaagatg tataatagta gatatgtata   1920
   taaaaaataa atatgaagat ttttattaat ggaaatagtt aaaaagttta aagttttggg   1980
   tagggttttt agaatggata aggtagagag gggattgttg tttttgttta taatttttgt   2040
   agaattaaag tatgtaattt ttttatttta tgtatatata atattttgat gttagaggat   2100
   gaattgtata tgttttagaa atatttgtat tgaaggtaaa atggttattt tttaatatat   2160
   tagttattta tatatataat aatatatttt tttaaaatta ttaagattaa tatttaggtt   2220
   ttattttgat atatttaaat gaatttgttt ttgttagtat tattattata ttttatttta   2280
   ttattaaggg taagtgagtc gttaaaaatt ggttatttta ggttaattta gaggtgttta   2340
   atcggggaag aattttattt tagggattat gtggtaatgt tataatatag gtgggggttt   2400
   tttattggta tttaataggt agaagttaac gatgttgtta aataatttat aatgggtagg   2460
   atagtaaaga attatttagt tttaaatgta atagtgttga ggttgagaaa ttaagtttta   2520
   agtttttgag gattatttta ttagaacgtt atatataaag attgatgata tgtaaatatt   2580
   ttgtaattta ggattgattt agttaaatat ttcggtgtaa tgttggaagt agtttggttg   2640
   tgaaatatat tttcgggaat attgagaatg gtaaagataa aaggtataat aaatgataat   2700
   aataataaaa tatagagttt tgtattttaa taattttttt tatttatggt ttttagggta   2760
   ttttatagat taataatatt tattttggta tttatatatt attttttatt taaggattgt   2820
   aggtattttt ataatatttt tacgatgtag gaagtattat tatttttatt ttatatgtaa   2880
   gtaaatagag gtataaaagt taagtaattt gtttaaagtt atataagtta gtagtagtta   2940
   aaatttttga tttagaattt attaatatta agagaattgg tttaagttat gtagtgataa   3000
   atttatgtgg ggtgttattt tagtttattt aaagtttatc gtttttaggt tgatattgta   3060
   tatttaatat tttatttgtt ataatttttt ttttttttat atatttttta gagattaagg   3120
   attttaagtt tttagaaggg attatgttta ttgagtgttt ttttcgaatt aagtatattt   3180
   tatgtgtagt gttagttttt aagatagttt aaatataatg taattgggag gttgagagta   3240
   ggagaattgt ttgaatttag gaggcggagg ttgtagtgag ttgagatttc gttattgtat   3300
   tttagtttgg cgatagagcg agatttcgtt ttaaaaaaaa aaaaaatgta atttttgttg   3360
   attttatagt atagaaagtt gagtattaga taatgtaaat atgtttaaga ttttttagtt   3420
   agttgattat attttttttt tattatattt tgtagttttt ttaggagaaa agttttttga   3480
   taagttataa agtatatgaa tgtgaatacg tttaatgttt tagttttttt tatttttttt   3540
   aaaatttaga aaataaatta atgaatatgt aattgagaaa ttttaggtgg tatattgggg   3600
   ttggtattag tttaggtaaa tagtcgttta gttttttaga tttattttta ataaaagttt   3660
   ttaatttttt aaggattttt ttagagtttt cgttatacgt tttttataat agttagatta     3720
   aagattaaaa ttgttttttt ttgagaaata tagagtatgt gaattatttt tttttgtttt     3780
   tagttttgtg gtaggtaaat attgattgtt tatttattat gtgttatttg ggtaaaatag     3840
   gaatattaag taggaagaaa ggtttatgtt aggtaagagc gtgatttagg gtttttttat     3900
   ttttttataa aatggagatt tggtatttgt agttttttat aatgatgtgt tttgatatta     3960
   tttggatata gaaaggttag ttttaggtgc gttagtgata gtttatttcg ttttgattta     4020
   gaaattttag atgatttgta ttagaggata agtttttggt atgttaataa tgaaaaaata     4080
   gagataatta ttgttttagt ttattttaaa ttagtattag tgtagcgtta gtattttggt     4140
   agggagtttt gttgttaaat ttattttttg taaagaggag aggtagagat agggttaagg     4200
   ggaaaatttt aagattggaa tcgttaatat aataaattgt cgaattgagt tttttttttc     4260
   gtaattttaa gatattagta agtttttttt tttagttaat tttttttatt agggtatcgt     4320
   agttttttta gaaggagggt gttgggtttg ttttaggttt ttttattttt ttgttcgttg     4380
   ttttagtata tttgaaaagg gagtagcgat taggaaaaga gatacgtggg tattttttta     4440
   ttttgtttag ttattttttg aattattata agttatcgta tttttttttt tagttagtag     4500
   cgttcgagat ttttttttaa ataatacggt tttgtattta aaaggaagag tggtgggaga     4560
   agagagaggc ggagaagata agtaagaagg gcgtggagtg tcgttttcgt ttcggagtcg     4620
   gaggcgtcgg gaggtcggac gtcgcgaagt tgttagttta ggaatgtgtc gtttaattcg     4680
   taggtcgcgg gcgatcgcgg cgggcgcgtt gtggtttgcg gcgggagcgg ggtagaggac     4740
   ggttggcgta gggtaggttg tagcggcggg tcggacgcga cgtcgcgtat ttgagcgttc     4800
   gggggcgggg cgttagcggt tacgattttt tttatcgcgc gacgcgtttt tcgcgcgtcg     4860
   tttcggtttt tttcgttcgt gtttcggcgc gttcggtttt tttcgcgttt ttggtttttt     4920
   tttttttttt tttttttttt tttttttttt ttttttgttt tgggattgtt tggatttcgt     4980
   atcgcgagtt tgtcgcggta ttttcgcgcg gcggaagagc gcgcgttagt ttcggtatat     5040
   ttgggagtcg gattttagtt ttacgtttcg ttttttataa gtttttttaa ggtaaggcgt     5100
   tcgtttatat tcggtttttt ttacgttcgg cgggatagtt gggttttcgt tttttttgcg     5160
   aatcggttag gagtttcgcg tttcgttttg ggagtggggt tgtagttgac ggggatttcg     5220
   gatcggcggt ggttagagcg cggagtaggc gatacgacga gtcgataggt ggcgggttta     5280
   gttttagtat ttcgatcgtc gtcggcgcgg attttggtgg ggatggggcg ggcgggtcga     5340
   tttgggggtg gggttagttt tttttttttt tttttagggg cggcgatcgt cggggttcgt     5400
   attgtaggtg cgtgggagaa attttgtagg gtggggattc ggcggttgtt ggtcggtagt     5460
   gattggtggg cgcgttcgag gattttaagg ggcgtagttc gggggtagat ttttgggtcg     5520
   ggcggggatt ttacgttttt tttattcgtt tttttttgtt ttttatttta gt             5572
<210> 60
   <211> 5572
   <212> DNA
   <213> Artificial Sequence
<220>
   <223> chemically treated genomic DNA (Homo sapiens)
<400> 60
   gttgaggtga aagataaaag ggggcgggta agggaagcgt aagattttcg ttcgatttaa       60
   gggtttgttt tcgggttgcg ttttttggag ttttcgagcg cgtttattag ttattatcgg      120
   ttagtagtcg tcgggttttt attttgtaaa gtttttttta cgtatttata gtacggattt      180
   cgacgatcgt cgtttttaga agggaggaga gaggattgat tttattttta agtcggttcg      240
   ttcgttttat ttttattaag gttcgcgtcg gcggcggtcg agatattagg gttagattcg      300
   ttatttgtcg gttcgtcgta tcgtttgttt cgcgttttag ttatcgtcgg ttcgaggttt      360
   tcgttagtta taattttatt tttaaggcga ggcgcggagt ttttagtcgg ttcgtagagg      420
   aggcggggat ttagttgttt cgtcgagcgt ggaaaggatc gggtgtgagc gagcgtttta      480
   ttttggagga gtttgtaggg gacgaggcgt agggttggga ttcggttttt aggtgtgtcg      540
   aagttggcgc gcgttttttc gtcgcgcgaa agtgtcgcgg taaattcgcg gtgcggagtt      600
   taggtaattt taggatagga gggagggaga agaaggaggg agggagggag ggagggagtt      660
   aggggcgcga agggagtcga gcgcgtcgaa gtacgagcgg aaaaggtcga ggcggcgcgc      720
   gaggggcgcg tcgcgcggtg ggaagggtcg tggtcgttga cgtttcgttt tcgggcgttt      780
   aggtgcgcgg cgtcgcgttc ggttcgtcgt tgtagtttgt tttgcgttag tcgttttttg      840
   tttcgttttc gtcgtagatt atagcgcgtt cgtcgcgatc gttcgcggtt tgcgagttag      900
   acggtatatt tttgggttag tagtttcgcg gcgttcggtt tttcggcgtt ttcgatttcg      960
   gggcgggaac ggtattttac gttttttttg tttgtttttt tcgttttttt ttttttttat     1020
   tatttttttt tttaagtata agatcgtatt atttgagaga aagtttcgaa cgttgttggt     1080
   taaggggaaa agtgcgataa tttgtgatga tttagggaat gattagatag gatgggaaaa     1140
   tatttacgtg tttttttttt tagtcgttgt ttttttttta gatgtattag ggtagcgggt     1200
   aggagaatag aaagatttga gataaattta gtattttttt tttaagaaaa ttgcggtgtt     1260
   ttggtgaaaa gggttggtta agaggaagga tttattagta ttttagggtt gcgggagaaa     1320
   aaatttagtt cgatagttta ttgtattggc gattttagtt ttggagtttt ttttttaatt     1380
   ttgtttttgt tttttttttt tatagagaat gaatttagta gtaaagtttt ttattaaagt     1440
   attaacgttg tattgatgtt aatttgagat gagttggggt agtgattgtt tttatttttt     1500
   tattattaat atgttagagg tttatttttt gatgtaagtt atttgaaatt tttggattag     1560
   agcggggtgg gttgttattg acgtatttaa gattgatttt tttatattta agtaatgtta 1620
   gggtatatta ttgtgggagg ttataaatgt taggttttta ttttgtaaaa aagtaggaga 1680
   gttttaagtt acgtttttat ttaatataaa tttttttttt tatttaatat ttttgttttg 1740
   tttaggtagt atatgatgag tgagtaatta gtgtttgttt gttatagaat tgggaataga 1800
   agaaagtgat ttatatgttt tatatttttt agggaaagat agttttggtt tttggtttgg 1860
   ttgttgtggg aaacgtatgg cgagagtttt ggaaaaattt ttagaaaatt aaaagttttt 1920
   gttgggaata ggtttaaaag gttgagcggt tgtttattta agttagtatt aattttagtg 1980
   tgttatttga agttttttaa ttatatattt attagtttgt tttttgagtt ttaaggagag 2040
   taagggaggt tgggatatta aacgtattta tatttatatg ttttgtaatt tattagagga 2100
   tttttttttt ggaagggttg taggatatag tggaaagagg gtatagttag ttagttgaga 2160
   gattttgggt atgtttatat tatttggtat ttagtttttt gtattataaa attagtaaaa 2220
   attatatttt tttttttttt gaggcggagt ttcgttttgt cgttaggttg gagtgtagtg 2280
   gcgggatttt agtttattgt aattttcgtt ttttgagttt aagtaatttt tttgttttta 2340
   gttttttaat tatattatat ttaagttgtt ttaagaattg atattgtata taaaatgtgt 2400
   ttgattcgag gaaggtattt agtaaatata gtttttttta ggggtttaaa gtttttggtt 2460
   tttaagaagt gtatgggaga agaggaaatt ataatagatg gggtattgaa tatataatat 2520
   tagtttaaaa acgatagatt ttgaatgaat taggataata ttttatataa atttattatt 2580
   gtatggttta gattagtttt tttagtgtta ataggttttg agttaggaat tttggttgtt 2640
   attgatttgt gtggttttgg gtaagttgtt taatttttgt gtttttgttt atttatatat 2700
   aaaatgggga taataatatt ttttgtatcg tgagagtgtt gtgaaagtgt ttgtagtttt 2760
   tagataaaag gtggtatgtg agtattagag taggtattat tagtttataa agtgttttag 2820
   gaattatgga tgaaagagat tattgaggta taaagttttg tgttttgtta ttattattat 2880
   ttattatatt ttttgttttt attattttta atattttcga agatatattt tatagttaga 2940
   ttgtttttaa tattgtatcg aggtatttag ttgagttagt tttaaattgt aagatgtttg 3000
   tatattatta atttttatgt atagcgtttt gatgaaataa tttttaaaga tttggagttt 3060
   ggttttttaa ttttagtatt gttatatttg gggttggata attttttgtt gttttgttta 3120
   ttgtaggttg tttagtagta tcgttggttt ttatttatta gatattaata agaaattttt 3180
   atttgtattg tgatattgtt atatggtttt tgggataaaa ttttttttcg gttgagtatt 3240
   tttgagttag tttaaaataa ttaattttta gcgatttatt tgtttttaat aataaaatga 3300
   aatatgatga taatgttgat aaaaatagat ttatttaaat atgttagagt gaaatttaga 3360
   tgttagtttt aatgattttg aggaagtgta ttattatatg tatggatagt tagtgtattg 3420
   ggaagtagtt attttgtttt taatgtaggt atttttggaa tatatgtaat ttatttttta 3480
   atattaaaat attatatgtg tataggataa aaaaattata tattttagtt ttataaggat 3540
   tataataaaa aatagtaatt ttttttttat tttatttatt ttgaaaattt tatttaaaat 3600
   tttgaatttt ttaattgttt ttattaatag agatttttat atttattttt tatgtgtata 3660
   tttgttattg tatattttga tttttttatt tttagatata gaagaggatt tagttttaat 3720
   atattaataa tatatatatt tttttttttt tttttttttt tttttttttt tttttgagat 3780
   ggagtttcgt tttgtttttt aggttggagt gtagtggcgc gatttcggtt tattgtaagt 3840
   ttcgtttttc gggtttacgt tatttttttg ttttagtttt ttgagtagtt gggattatag 3900
   gtggttgtta ttacgttcgg ttaatttttt tttttttttt tttttttttt tgtattttta 3960
   gtagagacgg ggttttattg tgttagttag gatgatttcg attttttgat ttcgtgattt 4020
   attcgtttcg gttttttaaa gttttgggat tataggtatg agttattatg ttcggttata 4080
   tatatatttt ttatattttt atacgttcgt agtattatga ttatataaat attgtttata 4140
   gttgagttat gtggtgtata tgattgtatt tttttttttt tataattgtt tttttttgtt 4200
   gtataattat attttttgtt tttattattt ttttgtagtt aaaatttgtt tttttttttt 4260
   tttttaatta tcggtttatt tttttatata ttagttataa gtatatattt aaattttttt 4320
   ttttttaatg acgttaatta tattgggtaa gttattagtt ttattttttt tttggaaata 4380
   tttgttttgg agttttttat tttttgtttt aatatggatt gattttgttt gtttgtttgg 4440
   agacggagtt tcgttttgtt ggtgaggttg tagtgtagtg gcgtagtttt ggtttattgt 4500
   aattttcgtt ttttgggttt aagtaatttt tttgtattag tttttagagt agttgggaat 4560
   ataggtatat gttattacgt ttagttaatt ttttgtattt tagtagagac gggggtttta 4620
   ttatgttgtt tagattggtt tcgaattttt gagtttaggt aattagtttg ttttggtttt 4680
   ttaaagtgtt gggattatag gtgtgagtta tcgtatttgg tttaatttgg attgatttta 4740
   aggttcgttg tttgtggtta ttttgggatt atgttttttt aatattttga gaattatttt 4800
   ttttgtagat tttttgtttt ttgaattgta tatttttttt tttttgtttt ttttttttat 4860
   tttagtagag tatattttta gtatattttg aagatagtat aatttatttt tagagatttt 4920
   gtatattgga aaatgtttta aatttatttt tatatttgat taatagtttg gtggagtata 4980
   gaattttagg ttagaaagta ttttttttta taattttgaa ggtattgttt tattattatt 5040
   tagtttttag agttgttgtt tagaagatta gtggtatttt gattgtattg tattttttgt 5100
   tttaaaattt ttggattttt ttatttagat tttttgaaat attttgaaga tatgttttga 5160
   tttggttttt tattattatt atgttatgta gttagtggat ttttttaatt agttatttgt 5220
   tttttagtta ggaaattttt ttattttttt tttttttttt tgatatggag tttcgttttg 5280
   tcgtttaggt tagagtgtag tggtttgatt taggtttata gtaattttta tttttcgggt 5340
   ttaagcgatt tttttgtttt agttttttga gtagttggga ttataggtat atacgtttgg 5400
   ttaatttttg tatttttagt agagacgggg ttttattatg ttggttaggt tggttttgaa 5460
   tttttgattt tatgatttat ttgttttagt tttttaaaat gttgggatta gaggcgtgac 5520
   gatcgtgttt ggtttatttt ttttttttta taagattttt tttttatttt tt 5572
<210> 61
   <211> 5864
   <212> DNA
   <213> Artificial Sequence
<220>
   <223> chemically treated genomic DNA (Homo sapiens)
<400> 61
   tttttatttt taaaaattgt ggtaaaatat atataatata aaatttatta ttttaattat      60
   ttttatgtgt ataatttagt attattaagt ttagtaagtt tttatttatt atggatagat    120
   ttttggaaat tgtgatttta agtaatatta ttggtaataa aattaatttg ttatgggatg    180
   attgatataa ataagagtta agtttttgta atatattttt ggttataaaa atattaataa    240
   acgtttaaat aaagatttta aatatttttt attttaaata ttgaaataaa tgtgagttat    300
   atatttaaga aagattgata aaaataagat aatttttttt ggagatggag ttttgttttg    360
   ttgtttaggt tggagtgtag cggtgcgatt tcggtttatt gtaattttta ttttttaagt    420
   ttaagtgatt tttttgtttt agttttttaa gtagttggga gtataggtgc gtattattat    480
   gtttggttat tttttgtatt tttagtagag atagggtttt attatgttgg ttaggttatt    540
   tgtagttttt aataattgag taatatatat ttttatgaat aaaatttgga gtatatttgg    600
   ttttttttat tcgatttttt tagaatttgg aaattgtaag tatttttaat ttatgataat    660
   atagttattt gtataagtgt attaataatt tgtttttatt tgtaatagga tataattggg    720
   gaaattggtt gtttattaat gtttttattt gatttacgga gttaattaaa tttttttgga    780
   aaaatcgttt ttataatttt gtttatatag tttttgtata gggtttttga tttgtggtaa    840
   gtaaagaatg ttattttttg ataggtttag gagttttaag tttattttgg aattttaaga    900
   ggagaggatt attaaattta taggtatttg atggtataaa tttatggttg ggtttagttt    960
   ttaaaaagtt ttatttgaaa tattttttat ggaataaagt tttattaaag ttaatttaaa   1020
   agttaatgtt aaaaaataat tatttttgtt gtattgtata taaataattc ggttaaatat   1080
   aataaggtaa atttattatg atttgttttt agtataaatg ggaaattaga gattggaaaa   1140
   ttatagttta ttagttgtta gattttagtt ttgtttaatg ttttttaatt tttattattt   1200
   tttatagttt ggattaaatt ttaatttttg tgggttataa gtttttaaaa taatgttttt    1260
   aatttttttt tttttttttt tttttatttt ttttaatttg gagttattta aaattaagtt   1320
   gttattaaat taagttattt aaaattaatt ttcgtgaagt tttgtgaatt gaagtagata   1380
   atttaaattt tagaagaaaa taatgggaat ttatttatat atataagtta tttttatatt   1440
   tgtttattga tgtatggatt ttagagtaat gtggtttaga ttaatttttt aagattgttt   1500
   ttattgtttt tttttttttt ttaaattttt ttttgatagg atattagatt ttataatttt   1560
   ttaaaaatga gtttttttaa taatttagga tttatttttt taggaaaaaa ttattttagt   1620
   tatgagagag tagataaaat ttgggaatag aaatttattt tttttaaaaa tgtttttttt   1680
   aaaatatttt aaaaaagaaa aggggggaaa tgtgaaagga aattattttg gaattttata   1740
   attattaagt taatgggaaa agttaagttg ggaattgttt agggtaaatt tgttttttat   1800
   tttatttagt tatttttttg tttattgaga taaatgtatg tttgattgtt ttttttagaa    1860
   aggtgaatta gaaatttaaa agagtgtaat tatttgtttt ttatttattt gtgatttgga   1920
   agtttttttt tatttcgagt tgttttattt tttttttgag ttgttttatt ttttttgatt     1980
   aatatttatt ttatatatgt tgattgatgt tttatatttt tttaaaatgt ataaaattat   2040
   tttgtgtttt gattatttta ggtatatgtc gttaggattt tttgaggttg ttataatcgt   2100
   gtgtttttga ttttcgtaag ataaattttt taaattaatt gagatttgtt tttaattttt   2160
   ggggtttata taggttaaat tgtaaaagat ttgtgttaga ggaggaagtt tatttagagg   2220
   gttggggggt tttattttat ttttggttta taggattttg atttttttta ttattttaag   2280
   ttggtaatga aaaaaatagt aaaagtatat aggattaagg attttaggtt gggtatagta   2340
   atttatattt ataattttag tattttggga ggttaaggtg ggaggattgt ttaagtttag   2400
   gagtttgagg ttaatcgggg taatatatga gatttcgttt taaagaaaaa aaaaatatgt   2460
   agaaaattag agaggataaa ttaaaatttt aataagaaaa atatattaat ttggaaattt   2520
   ataggaaatg ggtgattgtt taataaaaat atatatgaat aaaaggaatt ttaaaataag   2580
   aaatatataa ataggttagt tagtatagaa aagtttggaa tgatgtttag atatttttta  2640
   ttggaaaaga ttttagggat atatagattt agagttaagt tttaattgaa tttggttaaa   2700
   tttgttgatt tttagtaaat tttgatttta tttaaaatgg ttaaggttta gttgagggaa  2760
   ggggaaagaa gtgatttttt ttaattattt ttacggtata atttttgtga aaggaaaata   2820
   aaatttagga ttataaaatt attaaattaa agggtaaagt taagttggaa attatgttag   2880
   gtaaatttgt tttttatttt atttttaaat aagatagttg ttaagataag aggttacgta   2940
   ttttttttaa aatttgttta taaggaaatt ttttgtggat aaaggataga tagaatttaa    3000
   agttattttt ttaaggtgcg tttgagataa atgtatattt aattgttttt ttttttttat   3060
   tgtttacgta aaaatgataa tttattgagt tagattaaat tgtgtattta gtggaaggtt   3120
   gattaaggat ttaaaaaaat gcgatttttt gtttttattt tatttatgat ttggaagttt  3180
   ttattttaag tttcgtttta tttgattaaa gtatatttta tatatattga ttgatgtttt   3240
   atgttttttt aatatgtata aaagtaagtt gtattttgat tattaagggt atatggttat   3300
   tagggttttt tgaggttgtg ttacgggtgt aattttaatt ttggtgaaat aaatttttta   3360
   aattgatgga tgtcggatgt ggtggtttat gtttgtaatt ttagtatttt gggaagttga   3420
   gacggataga ttaattgagg ttaggaattc gagataagtt tggttaatat ggtgaaattt   3480
   tgtttttatt aaagatacga aaattagttg gttgtattgg tatatattta taattttagt   3540
   tgtttaggag gttgaggtaa gaggatcgtt tgtttgggag gcgaaggttg tagtgagtcg   3600
   agattatgtt attgtatttt tttagtttgg gtgatagagt aagatgttat tttaaaaaag   3660
   aaaaaaaaaa attgatggag agttaatttt tggtttataa tgtaaatgtt tatttaaaaa   3720
   tagatattat tttatgtatt attataaata tattgttatt tagttgttcg ggaggttgag   3780
   gtaggagaat gtttgaattc gggaggcgga ggttgtagtg agtcgagatc gtattattgt   3840
   tttttagttt gggcgataga gcgagatttt attttaaata aaataaaaat aaataaatat   3900
   attgttaatt aaattgtgat tttgttaaaa ataaatgttg ttgatgattt tatttttatt   3960
   aaagttattg tagtagttaa gggaaagttt tttttttgtt ttttgaaggt ttattgaaaa   4020
   gttaattgat aaaaggtata ttaattggag aaagggtata taaatttatt aatatgtata   4080
   tgggggagaa ttagagtgat tttgttgaag ttttaaatgg ggtttagaaa tttatatatt   4140
   attttgaggt tatagaatgg gggtttggat tgtggtataa taggttatgc gggtggagat   4200
   ttggttgata attacggttt tgttatgtaa atgaaatttt ttaagtagaa ttttttaaag   4260
   agaataggtg gtatatgttt tttttagatt tttgtaaatt aatagtaaaa ttttaaattt   4320
   tttaatcgat tgatggattt tttgtttgtt taagggtatt ttgaagttaa tttgaaaaat   4380
   tagtttaggt aatgatggga agtgggggtt ggttatgttt tattatatat tttttttttg   4440
   gaatttaggt atagtaagtt agtattaata ttaaaataga gattttaaga ttgatagaaa   4500
   agattttttt gtagaggttt ttttattatt tttaggtata taggattttt ggttttcgtt   4560
   gtagggtttt tattttattt gtttaatttt tatttgtttt ttattgttta tttttggtta   4620
   gtattcggtt agcgtatagg ttttttatat tttgaggtgg atattagtag ggaaggtttg   4680
   cgggagggtt ttgttttttt ggcggttatt cgtaggttta tagtaagata tgtgtagagg   4740
   tgagcggagc gtcgttatta gttgttattt ttttaaagat tagaattatt ttagagtatt   4800
   cggaggtatt aggaaagcgg ttcggaggta gaggcgttag gggcggggtt gatgtcgaga   4860
   ggtttttttt gtttcgtttt ttcgtattgg ttttgtaatc gttttcgtta gacgcgtttt   4920
   cgttttttgt ttttcggaaa ggtgttcgtt ttttgttttt cggttcggcg ttcgattttc   4980
   gtttttcgat ttagttgtgg gttgcgtttt acgttagttc gcgtttcgta tggttgtcgt   5040
   cggggttagg tttgtggagt tgggtttcgt cgagtcggcg tcggcgtggc gattgcgtag   5100
   cgagtagttt tttagtaagg tgggcgggcg gtcggtatgg ttgggcgcgg tcgggttgtc   5160
   ggggttttag gttttggttt gcgagttgtg cggtcgttcg tttttttttt tgttgtaggt   5220
   gtatgcgtcg ttgtttggtc gttcggacgt tttttatcgt tgtatttttt ttttttgttg   5280
   tcgcgagtag tcgtgttgtg tcggtttgcg aggtgagtcg ttaaacgggg tcgggacggg   5340
   gttatgtttt cgacgcgtgc gcggcgttaa tttcgggagc gattaggggg acggcggtaa   5400
   gtatcgttgt cgttttagtt ttagagtttt tttttcgcgt tgttttagga gtattttttt   5460
   tcgggttttt ttaggatgtg tttatttttg ttatataagt ttttaattgt gtttacgcgt   5520
   attggtaatt tttagttttt aattagtggg tattaggtat ggtgttaatt tagataatag   5580
   aaattgtttt ggaaagatgg attttagtag ttttttcgta tttttggtag tcgtttttaa   5640
   cgattagttt tattaatttg attaatttaa attattttta ggaggtaatt tagttgcgtg   5700
   tatcggacga aatttagttt ttggatttta aaagtttatt tttgaaagta aaaattttta   5760
   tttttttttt gttgttgtta tttttagttt tacgtgtaga ggataaaata tgtgaattgt   5820
   tgttttgttt gaagataaaa tttttttttg tatttttttt tagt                   5864
<210> 62
   <211> 5864
   <212> DNA
   <213> Artificial Sequence
<220>
   <223> chemically treated genomic DNA (Homo sapiens)
<400> 62
   attaaaaaag aatatagaga aaagttttat ttttaaataa aatagtaatt tatatatttt     60
   attttttgta cgtaaaattg aaaataataa taataaaaaa gaaatgaaag tttttgtttt    120
   taggaataag tttttaaaat ttagaaatta gatttcgttc ggtatacgta attgagttgt    180
   tttttagagg tggtttgagt taattaaatt aataagattg atcgttaaga acgattgtta    240
   aaaatacgaa aaagttattg ggatttattt ttttaagata atttttatta tttgaattaa    300
   tattatattt ggtatttatt gattaaaagt tgggggttat taatgcgcgt gggtatagtt    360
   agaagtttat gtagtaaaaa tgagtatatt ttggaagggt tcgggagaag gtgtttttgg    420
   ggtagcgcgg agagggagtt ttgaggttgg ggcggtagcg gtgtttgtcg tcgttttttt    480
   ggtcgttttc ggaattaacg tcgcgtacgc gtcggaggta tggtttcgtt tcgatttcgt    540
   ttggcggttt atttcgtagg tcggtatagt acggttgttc gcggtagtag aagaggaaga    600
   tgtagcggtg gaaggcgttc gggcggttag gtagcggcgt atatatttgt agtaggaagg    660
   agagcgggcg gtcgtatagt tcgtaggtta gggtttgggg tttcggtagt tcggtcgcgt    720
   ttagttatgt cggtcgttcg tttattttgt tggggaattg ttcgttgcgt agtcgttacg    780
   tcggcgtcga ttcggcgaag tttagtttta taggtttggt ttcggcggta gttatgcggg    840
   gcgcgggttg gcgtggggcg tagtttatag ttgggtcgga aggcggaaat cgggcgtcgg    900
   gtcggaaggt aagaggcggg tattttttcg gaggatagga ggcggaaacg cgtttgacgg    960
   gagcggttgt aggattaatg cgagggaacg gggtagagga aatttttcgg tattagtttc   1020
   gtttttggcg tttttgtttt cgagtcgttt ttttggtgtt ttcgggtgtt ttgggatggt   1080
   tttggttttt gggagagtgg tagttggtga cggcgtttcg tttatttttg tatatgtttt   1140
   gttgtgggtt tgcgggtggt cgttagggag gtagagtttt ttcgtaaatt ttttttgttg    1200
   gtgtttattt tagggtgtgg gaaatttgtg cgttggtcga gtgttaatta agagtaggta 1260
   gtgaaagata aatgaaggtt gaataggtaa agtgaggatt ttatagcgga aattaagaat 1320
   tttgtgtgtt tgagagtaat gaagaagttt ttgtagaaga gttttttttg ttagttttaa 1380
   ggtttttgtt ttaatgttag tgttggtttg ttgtatttga attttaaggg aggagtgtat 1440
   aatgaggtat ggttaatttt tattttttat tattgtttga attagttttt taggttaatt 1500
   ttagaatgtt tttgggtaag tagagggttt attagtcggt tggagggttt agaattttat 1560
   tgttggtttg taaaggtttg aaagaaatat gtattatttg ttttttttaa ggagttttat 1620
   ttaggaggtt ttatttatat aataagatcg tggttgttag ttaggttttt attcgtataa 1680
   tttgttatgt tataatttaa atttttattt tgtaatttta agatggtata taagtttttg 1740
   aattttattt ggggttttag taaaattatt ttggtttttt tttatgtgta tgttaataaa 1800
   tttgtatgtt ttttttttaa ttaatgtgtt ttttgttagt tgatttttta gtgaattttt 1860
   agaggataaa aaggaagttt ttttttggtt attatagtgg ttttaatgga agtaaagtta 1920
   ttaataatat ttatttttga taaaattata gtttagttgg tagtatattt gtttgttttt 1980
   gttttatttg agatggagtt tcgttttgtc gtttaggttg gagagtaatg gtgcgatttc 2040
   ggtttattgt aattttcgtt tttcgagttt aagtattttt ttgttttagt ttttcgagta 2100
   gttgggtagt agtatatttg taatggtata taaaataatg tttgttttta aataaatatt 2160
   tatattgtaa attaaaagtt aattttttat taattttttt tttttttttt tgagatggta 2220
   ttttgttttg ttatttaggt tggaggagtg tagtggtatg atttcggttt attgtaattt 2280
   tcgtttttta ggtaagcgat ttttttgttt tagttttttg agtagttgga attataggtg 2340
   tgtgttaata taattagtta attttcgtat ttttggtaga gatagagttt tattatgttg 2400
   gttaggtttg tttcgaattt ttgattttag ttgatttgtt cgttttagtt ttttaaagtg 2460
   ttgggattat aggtatgagt tattatattc ggtatttatt aatttagaaa gtttatttta 2520
   ttaagattaa ggttgtattc gtgatatagt tttagaaggt tttgatgatt atgtgttttt 2580
   ggtggttagg gtatagtttg tttttatata tgttagggag atatgagata ttaattagta 2640
   tgtgtaagat gtattttagt taggtaaagc gggatttgag gtgagggttt ttagattatg 2700
   agtagataag aggtaaaaga tcgtattttt ttgagttttt gattagtttt ttattgaata 2760
   tataatttag tttggtttag tgaattatta tttttacgta aataataggg gaggggaagt 2820
   aattagatat gtatttgttt taggcgtatt ttaaagggat aattttgagt tttgtttgtt 2880
   ttttatttat aaggaatttt tttgtgggta aattttaagg gaggtacgta gttttttatt 2940
   ttggtagtta ttttatttag gaatagaatg ggaggtaggt ttgtttgata tagtttttag 3000
   tttgatttta ttttttggtt tagtgatttt gtggttttga gttttatttt ttttttatag 3060
   aaattatatc gtaaaagtaa ttgaagaaaa ttattttttt tttttttttt taattaggtt 3120
   ttgattattt taaataaaat taggatttgt tgaaggttaa taaatttaat taagtttagt 3180
   taaaatttaa ttttgaattt gtatgttttt ggggtttttt ttagtgagag atgtttaagt 3240
   attattttaa gtttttttat attaattggt ttatttgtat gttttttatt ttaggatttt 3300
   ttttgtttat gtgtattttt attaggtaat tatttatttt ttgtaggttt ttaggttaat 3360
   atattttttt tattggaatt ttaatttatt ttttttagtt ttttatatat tttttttttt 3420
   tttgagacga ggttttatat gttgtttcgg ttggttttaa atttttgggt ttaagtaatt 3480
   tttttatttt ggttttttaa agtgttggga ttataggtgt gaattattgt atttagttta 3540
   gaatttttag ttttatatat ttttgttgtt ttttttattg ttaatttgaa ataataaaaa 3600
   gggttagaat tttataaatt aaaaataaaa taaggttttt taattttttg aatggatttt 3660
   tttttttgat atagattttt tataatttaa tttgtatgaa ttttaaaaat tggagatagg 3720
   ttttagttaa tttagaaaat ttattttgcg aaggttaagg atatacgatt atgatagttt 3780
   taggaggttt tgacgatatg tatttaagat agttagagta tagggtggtt ttatatattt 3840
   tagggagata taagatatta attaatatat gtaaaatgaa tattggttag gaaaggtggg 3900
   ataatttaaa gaaaaaatgg gataattcga agtggggagg ggtttttagg ttataggtag 3960
   gtgagagata aatggttgta tttttttgag tttttgattt atttttttaa aagaggtagt 4020
   tagatatgta tttattttag tgagtagagg gatgattgaa tggaatggga agtaggtttg 4080
   ttttaagtag tttttagttt gatttttttt attagtttag tgattatggg attttaaggt 4140
   aatttttttt tatatttttt tttttttttt ttttaaaata ttttggagaa agtatttttg 4200
   aagaaaataa gtttttgttt ttaggtttta tttgtttttt tatggttagg atggtttttt 4260
   tttagaaggg taggttttga gttattagga aagtttattt ttagaaggtt gtgaagttta 4320
   atattttatt aagagaaaat ttggggagga agggagaata ataagaataa ttttggaaaa 4380
   ttgatttagg ttatattatt ttgaagttta tatattagta agtaggtatg aaagtggttt 4440
   atgtatgtaa ataggttttt attatttttt tttgaagttt aagttgttta ttttagttta 4500
   tagggtttta cgaaagttag ttttaagtga tttagtttag tgatagttta gttttaagtg 4560
   attttaaatt aggaaaaatg ggaaaaaaaa gaaggaaaaa aattgaaaat attattttga 4620
   agatttgtag tttataaaaa ttagaatttg gtttaaattg tagaaaatga taaaaattga 4680
   aaaatattag gtaagattag aatttaataa ttggtggatt atagtttttt agtttttagt 4740
   tttttattta tattaaagat aaattatgat aggtttgttt tattatattt ggtcgaatta 4800
   tttgtatata gtgtagtaag aataattatt ttttaatatt ggtttttaaa ttggttttga 4860
   tggaattttg ttttatagag ggtattttag ataagatttt ttaaaagttg agtttagtta 4920
   tggatttgtg ttattaaata tttgtgagtt tggtgatttt ttttttttga ggttttaaga 4980
   taaatttgag gtttttgggt ttgttagaaa gtgatatttt ttatttatta taggttagga 5040
   attttgtata ggagttgtgt agataaagtt atgaggacgg tttttttaag ggggtttaat 5100
   tggtttcgta agttaagtaa aagtattggt aaataattag tttttttaat tgtgttttgt 5160
   tataaatgaa aatagattat tagtgtattt atgtaaataa ttgtattgtt ataagttaag 5220
   aatatttata gtttttaaat tttggagaaa tcgggtagag agaattaaat gtgttttaaa 5280
   ttttgtttat aggagtatgt gttatttaat tgttaaaagt tgtagatagt ttgattaata 5340
   tggtgaaatt ttgtttttat taaaaatata aaaaatagtt aggtatggtg gtgcgtattt    5400
   gtatttttag ttatttggga ggttgaggta ggagaattat ttgaatttgg gaggtggagg    5460
   ttgtagtgag tcgagatcgt atcgttgtat tttagtttgg gtaatagagt aagattttat    5520
   ttttaaaaaa aattattttg tttttattaa ttttttttaa atgtatagtt tatatttatt     5580
   ttaatgttta aaatgagaaa tatttggggt ttttatttag acgtttgttg atgtttttgt    5640
   gattagaaat atgttataag aatttaattt ttgtttatat taattatttt atggtaaatt    5700
   ggttttatta ttagtagtgt tgtttaaagt tatagttttt aagaatttat ttatgataaa    5760
   tgaggattta ttggatttaa tagtattgaa ttgtatatat aaaaatggtt aagatgataa    5820
   attttatgtt atgtgtattt tattataatt tttaaaaatg aaaa                      5864
<210> 63
   <211> 13123
   <212> DNA
   <213> Artificial Sequence
<220>
   <223> chemically treated genomic DNA (Homo sapiens)
<220>
   <221> unsure
   <222> (4849..)
<400> 63
   aggtagggtt gtttgatgtt atatatttaa ttatatatat attttttttt ttttttttat     60
   agttttttac gaggtagaat tttagagttt ggtgtgtttg tggttggagt atagtagttt    120
   ttttataatt ttattgtagt ttttattgaa tattttggtt tgtaaatata aaggattttt    180
   tgaagtataa ttttaaataa cgtgtggtag atatttggga gaggaaaatt gtagatagaa    240
   aaatgtggtc ggatgttttg tgggcgggcg gtagcgatgt tcggttaggt ggttttaagg    300
   agaggaagta gtgatgtatt gaggtaagag agttgataga tgaaattata tggattttaa    360
   gatagagtgg aaggagggtg ttttaaatat tgtagggtgt gtttttatta ggttatgaga    420
   atgtagttgg gatataggtt tgtttttttt tgtttgtatt atcggagtga tgatttagat    480
   aaatttaaaa attattttat tttgaagtat gtttgatgtg aagaggttta aataatattt    540
   taggattaag agttaggaga tttaaattat tgtttagata tgtattatcg aatttaggtg    600
   tgtgattttt tgtaagtttt ttaatttttt ttattgtagg tttttttttt tttaaaataa    660
   gggagttggg ttattgaatt gtaagaattt ttttagtttt agattgtttt gattgttttt    720
   agtattggaa atttagatat aattagaagg tacgttttta gttttttaga tttatattgt    780
   ttaatttagt agttatatgt ggttatgaaa tatttgggat gtggttaata tgataaaatt    840
   ttttatttta ttttattgtt attaaattta tattaagaaa ttgatattgt atttagttat    900
   tgaaaaattt tatttatttt tgagatagag tttcgttttg tcgtttaagt tggagtgtag    960
   tggtatataa tttcggttaa ttgtaatttt tgttttttgc gtttaagtga tttttttgtt   1020
   ttaatttatt gagttgttgg gattatagga atttgttatt atgtttggtt aatttttgta   1080
   tttttagtag agacggggtt ttattatgtt ggttaggttg gttttgaatt tttgatttta   1140
   aattatttgt tcgttttggt tttttaaagt gttaggatta tagatatgag ttattatatt   1200
   tggcgtgaaa aattttagtt tgtttaaaat aatttaggaa tgtgaatttg ttttttgtaa   1260
   tagtaaattt tgtgaaattt aaatatagat taagtgtttt cgatgaaaat ttggtatttg   1320
   aattgggata tgttgtaagt ataaaatatt agatttaaga taatattaaa aaaggaagta   1380
   aaatgtttta ataatttttg ttgattatat gttgaaatga taatgtttta gaaatattgg   1440
   gttaaataaa ataaattgtt aaaattaatt ttatttgtgt ttatttttta aaatatgatt   1500
   agtagaatat ttgttaatgt gcgtggtatt ttttttggat agtttttata ggataggtta   1560
   tattttataa attgtagtga gtggttggat tggtatattg gttttagttg attatggatg   1620
   ttttggagag gaaagttgtg ttatttttta aggaagatat ttgtattagt ttgagttatt   1680
   atttttatta gataaaatta gttttaatta gttttgttaa tagtgtttta agagagttag   1740
   gggatgtgag gttttttaga gtttttttgg tttttagtgt ttttaaaatt gtaatgtgtt   1800
   gtaggtatta tgagtaaatg tattgcgata tttttatttg agtatattta ttatttttat   1860
   ttttagagag gggatttgtt tggaggagtt tgtttttatt tagtgagtat ttttgttggg   1920
   tggggggttg agttacgttt ttttagaggt tgttggtttt gtttgcgttt ggttattttt   1980
   agtttagtta taggtttttg gtatatttag ttttagatag gtagtagagt ttgttttgtt   2040
   ttttttatat gaatttttta gaaggagggt gtgttagtgg taagagtttt gagtttgagg   2100
   ttttttgttt tgtgtaaaat tttttttttt tgtatttata gggagtaaat atggaatcgg   2160
   tgagaattat tttttttgat tattatgtgg ttagtatgcg gtgattgatt tgatgtgatt   2220
   ggggaaggtt ttttttttgg atagaatatg gtgagtgtcg tgttattatc ggagaggatt   2280
   attttttttg gttatatgtg gtaagtagtg attgggaacg attatcgttt ttagttagtg   2340
   tgtgattatt aatttggagt gatttaggag gattattgta tttgtttgtt tggattgtta   2400
   taataaatat tatagagcgg atagtttaaa taatagatag ttttggaggt tagaggttgt   2460
   tgattaagat attagtaggt tttgtttttt ttgaaatttt tttgtttgtt ttgtaaatgg   2520
   ttattttttt gttatgtttt tatagggtgg ttttttagtt agtgttgtat gtgttttaat   2580
   ttttttttat taggatatta gttattttgg attaggggtt atttatatta ttttatttta   2640
   ttttaattat ttttttaaag gttttatttt tatatacggt tatattttga gtttttgagg   2700
   gttagaattt taacgtatga attggaaaag aatataattg agtttataat aatcgttatt 2760
   tttggtcgga atgtgttatt taaggatggt taatttaatg aattgtgtgg ttagaagtaa 2820
   tgattataga atgattatta ggttatttgg agatttttta tttaagggaa tgagagtagg 2880
   aatatatatt tttgatacgg tgatgaatga gtttaggtaa atgaggtgga tggaggaatg 2940
   tattgatttt tgaatttttt tgtaaggtta tagatgtatt gttttttttt tagtattagg 3000
   aattttttgt tatttgaatt atagtgtttg agattaagta agtgtagttg aatgtttttg 3060
   ggatagtgtg gtaagtttta gttaataaat ttgatattag gtataagggt taataattta 3120
   tttagggtta taaaagaaat ataaattatt agttttgtat taaagaatat aaggtagagg 3180
   tgattaggtt gggaatagaa agtaggaatt agtagagttt tgatttttga agagggtaat 3240
   ttttttaaag ttagaaattg atgaataggt atggatgagg aggatggtga taagttggag 3300
   ttaaggtaga agtaggaaaa tgtaagatat gataatattt ttaggaggtt ggttggttat 3360
   agtagaggta ttgtgattaa gaggattggt ttatagtggt gttagagaga gaaaagtttg 3420
   tgtttaggtg tgaagtgatt tgggattgga ttagtgacgg ggagtatggg gtgagtgtga 3480
   gaagattgtt aaaagttggt aattgatgaa atgttgaaat taaggaggtt taaagttaaa 3540
   tatataattt taaaattatt ataatgatgt aattttgtga tttttaagag gttattagtt 3600
   ttggtattgg tgttgagatt tttggaatgt aatatttatg tttatgaaag aaaagatggg 3660
   aatagtttta ttgtttacgg atttttatat tttagaaaag ttaattttag tgtgtggtat 3720
   tttattttta ttttaatttt aggttttttt agttagttta attgttagta ataagaaatt 3780
   agatagatgg aagtaatatt ttttatttga taaggtttag gggtaagata ttattaattt 3840
   gggaggagga gttaatttaa gtaataataa atagatttta taaaagggtt gtgatgtatt 3900
   ttatattttt attttgggga aaatgtagta ggaaagggaa atagttagtt ttataggaat 3960
   ttgtttggtt tattgggata gaataagaaa atttgttttt tatatgttaa ttgaatatta 4020
   agttttttga aagtaagagg acgattttat ttttagtatt tttttttacg gaagggtttt 4080
   ttgtaattat cgtaaatgtt ttttgaaagt aattttagat tatataatta attttatata 4140
   tgtatgagta tgtgtgtatt ttatatatat aaattaggat tatatattta tgtaaaatat 4200
   ttgtgttttg tatagaattt tgaaaatttt tattgttttt aaaggataat agattttgag 4260
   taggttgttg agggttttta tttttttttt tagtattata aatgtaaaat cggttattta 4320
   gtttattggg atttttttta tattgttttt agttagtgtt ttagaaggga tattattcgc 4380
   gatagcggac gttagtagtg gggtttaggt tttataaatt tttaattgat gttgatattt 4440
   cgaggaattt ttgttaagtt gtagtaaatt gggtttttta tttatagggt acgtttcggt 4500
   tttaaggaag atttttaaga agagttagaa aataggtttt tggtaggtat tgaagtttgg 4560
   taattagtcg taggtagttg ggacgaattt tgcggttttt tttttttcgg ttttcggata 4620
   tttatatttt tcgaattgat ttttttgaag gtgggaagga gtatatagag tagtagtttt 4680
   agtttcgcgc gttttttttg tagcgtcgtt tttttcgttt ttttttcgcg gtagttcgag 4740
   tgcgttttat ttgcgtcgtt acgtcgtcgc gttttttcgt ttcgtttcgg gttagttagg 4800
   cgtcgtattt ttttatcggt aggtttcgtt tcgtatacgc gttttcggnn cggtgtttcg 4860
   tttttttcgt tacgtgatag atttattttt cgtattcgtt tcgattggtt ttttcgattt 4920
   agggggaggg atttggcgaa cggcgatgtt ttagacgcgg ttgtagtttt taaatcgcga 4980
   ttgtaagttt cggtagtttt tttcgttgtt gtcgttagga gttattttac gagaagttag 5040
   gttataatcg tcggtttttg tttggaaaag taaaagtgga ttttgttacg ttcggagttt 5100
   tttggcgttt cgttcggttg gagttagaga attcgttttg tggcggtttt cggcgtgggg 5160
   cgggatagcg gttttttgga gggggtagtt tcgggagaat ttgcggcggt cggagcggtt 5220
   agtgttttgc gggattggcg ggttggttcg gggttgcgat ggaattagtc gtttcggtat 5280
   agagatttta tacgtgagga aggaggattc gaggagggtt taggcggtac ggttcggttg 5340
   agaggggttt ggatttcgtc gtttttgagg tggttgattg ttgttggcgt tttttcgttt 5400
   tgcggttgga gttcgggttt tttggggttt cgcgcggggg gcggtcggcg gtttttttat 5460
   tatttttttt agttcggggc gcgtgtttgt gtttgtgcgt gttcgtgtgt tttcgtgcgt 5520
   tttttattcg gtcggtttcg gtttttttta ggtcgcgtta ggtagggttt ttttcgcggc 5580
   gggttcgcgc gggttttttt tatagtaagg ttacgcggtt tttttagttt ttttcgagtt 5640
   ttagttgcgg cgtgggcgcg tttttatcgg gattcgtgga cgcgttttgt ttttagttgt 5700
   ttttcgttta gagaaagttc gtgtttattt tgtgggacgt ttttagttat tgtttgggaa 5760
   tagtgtttta aaattagcga gagattaaga cgggttatag ttgttttcgt gatttttagc 5820
   gatttgattt ttgttttgat gttttgtgat ttatttagtg tgtacgattt atttttaaat 5880
   tatattatta ttggatgtta acgatttttg atatttattt aggttttttg ttttattgta 5940
   gggaaaagcg ttttatttga atttttttcg agggagaagt agagataaag ttgaaagagg 6000
   ttttatagta gttggtagtt ggtattagtt tttgtttgga ttagaggtat tttgatatta 6060
   atttggaaat tggaattaag aaaatacgtt tttaaaatcg taatatttat tagattttat 6120
   taatatttaa atatatgagg attgtgtatt atatttatcg attgttttgt cgacgtaatg 6180
   tttatatttg tggtgttaat gataagtaga attttgttag ggacgtttga cgtggtgtgg 6240
   ttattttacg tttttaagtt tatgagaatg tttgcgcgga gatagtatag ttttgtagaa 6300
   atgagtggta gcgtatgtaa tttggtattt tgaatttagg agtataattt tattaaagga 6360
   aaataaattt atttttttat tgataatatt gttttttagt tttatggtga attgttcgga 6420
   agtaattttt aataagtgtt tattttataa atattagatc gtgtattttt aggattgtgt 6480
   attttttaag aaaattggtt tatagaagcg gtgtaaaagt tttaaattta tttgtttcgg 6540
   attttttttt tttgagtaga tgtttaatta aatttttttt agtattttaa taattggagg 6600
   tattaataga tgttttattt ttgagatata tattgtatat tttagatttt tttttttttt 6660
   taaagtaggg atttaaaatt gaggtgaaat atatttgttt atatggtaag attttttaaa 6720
   agtagaattt ttgtaattga agattatttt tttttgtgtg tgaatagaat ggttgcggtt 6780
   ttttttggga ttattgatta gtgaattacg atttggttaa gatagaatgc gtttttagga 6840
   agttggaggt ttgattaatc gttgtgttag tatatgagta ataaatttga agaagatata 6900
   agtattttta tggttgacgt ttttaattag ataattttat ttttaagttt gttttgtttt 6960
   atttttgtta agtgaatatt ttaatatgtt ttaaagtttt ttgataatta taagggagaa 7020
   tttttttatg aaaatatttt tgttttaatt ttagagatat aagaagttaa ggaatttagt 7080
   taataatatt tttatttaat cgttaaattg ttttaaaatt gtatgtatag tatattgtaa 7140
   tttatatttt ttgtatgtaa gattggtagt taagaatagt attttggtcg ggcgtagtgg 7200
   tttaagtttg taattttagt attttgggag gtcgaggcgg gcggattata aggttaggcg 7260
   ttcgagatta atatggtgaa atttcgtgtt tattaaaaat ataaaaatta gtcgggtatt 7320
   gtggcgcgcg tttgtagttt tagttattcg ggaggttgag gtagaagagt tgtttgattc 7380
   ggaaggtcga ggttgtagtg agtcgagatt acgttattgt attttagttt gggtaataga 7440
   gggagatttc gttttaaaaa aaaaaaaaaa aaaaaaaaaa atagtatttt taggtttatt 7500
   atttttaaag gatattgatt gatttttttt tttataaaaa aattttttgt attgtatggg 7560
   tatttgtttt gttagaaata ttagtagtta tttatttatt atttttttat ttaggaaaat 7620
   tttaaatgtt agttgtttta gttttttata atattgatga taaaatttag tgattttatg 7680
   tttaagtatt gtagtttgta ttttatgttg ggttgttatt aaaggttatt tttttgaata 7740
   ttttagttaa gaaatgtttt tataaagtta taagtatgta tttttattta aatatgttgt 7800
   aatgttgtaa tttttagggt atattttatt aagtttttta ttattttttt tttagaatat 7860
   ttaattttaa attttagtgt tatttaaaat atatatatta gtttttttta atatttatat 7920
   gtattgtatt ttggtttatt tttgattttt tgtaattatt ttaaaagagg gtgtagtgat 7980
   attgtaagta ttgaaaaatt ttaatagtga gatggtaatt ttttttttaa tttttaaagg 8040
   tagattttat tttgtattgt aatagttatt gaataaatag tatttaagcg gtgttattaa 8100
   gtaaataata ttttataata gaaatttaat ttttcgtttt gtgagtgata agtattatgt 8160
   atttgttgtt tatttcggaa ttttttattt tattttcgtt ttttaatttt ttattttggt 8220
   ttataattaa taggtttaat tttttttttg gaggtgggaa gatatagtat taggttttag 8280
   ggttttggta tagagttagg tttaggagtt attgtttgat attttagttt ttggatagta 8340
   tatattagaa tggttggatt agagttagtt tagttgattt tttattgtga tttttagagg 8400
   attaagttta tgaattatat tttttgttta aaatatttta aaaaataaaa tttgttggta 8460
   gaatatatat gaggaatata attttttttt tgaaattgaa aattattatt atttttattt 8520
   tgatattaat atttttaatt aatgattaat taaaaatata tatttttgtt atattaggag 8580
   aaagtttttt ttttattttt gatggacgtt aaattttttt tttagttttt tagggaaagt 8640
   ttgttagtag taaatgtttt aaatgtttat gtagaaagga gttgtttttt ttagaatttt 8700
   cgggtttatt ttgttttttg ttgttttttt aatgtatttt atgatgattt agatttatta 8760
   gtgataaatt ttgataggat atttatttga attttttgat ttttggaggt ttttaaaaga 8820
   gtgatgtttt taattgtagt aggtatttgt attagtagtt tatattaaaa ataagtagtt 8880
   attttgtttg gaaatagtgg taaaaataaa aggtttattg gtattatatt atagtatatg 8940
   ataaaataat tttatttgtg tttttagatt ttgtggttat ttgtttagaa ttgatatttt 9000
   gtaagtgtaa tttaaaatag ttattttatt tgtttaaatg ttgaattaag tagagattag 9060
   ttgtatttag agttttagta ttgtaaattt aagatggatt ttaagttttt ttaatttttc 9120
   gaagatatga gatatattta taaatttttt tttttatatt ttataatttt tataatttag 9180
   tttattaaat atttttaaaa tttttatcgg gtggtagata ttatgttggt tgtttaggta 9240
   taaaagtaaa aggatgttgt tttttaaata ttgaatagaa ttgtaattta atatgttata 9300
   ttttgaggta aagaagtatg ttaaggattt ttttatttga agtataaagg agaagttttt 9360
   aaattagttt gtggttttta ggtaaagttt tttgggggag aggttatttt gtgttgagtt 9420
   ttgaaggatg tgtaagaaga gttagttatt taaggaaagt agaaaggttt ttttaggtaa 9480
   agaagtataa taaagatgtg gtggtaaaat atgaaatatg agtgcggtag gaaatgtagt 9540
   tggtgtatat ttggttttag aggtttttat gtgttatgtt gtaagtattt gaaagtttgt 9600
   agatattggg gagttaatga aggaattaaa atagataatt tgatttttta aatgttttta 9660
   ttattgtata ttttattttt taaaaatttt tttatttgat aattttgaaa taaatattat 9720
   gataaatatt ttttaaaata gagttagagg tttatagaga tagttttgtt tttgtttata 9780
   ttatatttgt tttttgtatt taagtaagtg aaggtttaga ttaagtttaa tatttttttt 9840
   tttttttttt tttattgatt tatttttaaa tttttttggt aattattata atgagaatta 9900
   atgaaaaaaa tttttaaaaa taagtgagat attattattt gtagtagtat tgtgtgatta 9960
   aaagtttaat tgtgttttat tttttattat attttttata ggatttaatc gtgtatatat 10020
   atgtatataa atttatttat taggtttttt aagatttttt ttaaatgtta taatttgata 10080
   aaatgttatg aaatgaattt taaatggagt gagatatata ggtaatttag aatcggtaat 10140
   atgaagtgtg gtagaagata agaaaaggtt tgggaattgt tggagttaaa gagaatttgg 10200
   tgggaaattt aggaagatat aagaaggagc gatagagagt tgatttataa tttgtatgta 10260
   ttttatatga agggtaatta gatgtttgag gagggtttgg tggataaagg tgatatttat 10320
   gttttattat atgtaaaatt ggttaaagga gaggaaatgg aaaaatattt tggttttgat 10380
   aattggttta gatgtagtaa ataaaaggaa aatgaattta agaaatgttt aatggatgtt 10440
   taaagtagtt tgtgatgtag gtatattttt aataaataga tgaatttatg aggaaattgg 10500
   tatttatatt gattttttag aataatggaa aaaaattagt gaaattgagc gttattagaa 10560
   ttaagaagat atataagtag ttaaaataga aaataaaaat atttttaaga atgaagttta 10620
   gattaagttg ggatgagaat tttgtatatg gaaatagtat agttgtttaa taatggtagg 10680
   gaattgtagg gagagtattt ttgggtagaa tgttaatttt gttttgtgga ataatagcga 10740
   tatgtttttg tgatttgtat tgtatttgtg ttagtggata tgagtttagt atattttaat 10800
   ttttaatttt tgtagtattg gaagattaat ttttttatta tttatatttt agaggtagta 10860
   aaatcgaagt tttttttatt agtaaaaggg aatgcgaagt aaattataat aaaattttat 10920
   tttattttta ttatattaga atagttaaat ttaaaagttt gaattgggta agtattgtta 10980
   aagtagtgat aaatcgtttt atttagtgtt agtgggagtg taaattgggg gtggaaagaa    11040
   ggggttatgt ttaatatgtt ttatttttaa tttaaatgaa atatgattaa gtaataagat    11100
   tggaaagtaa atgtttgtat atagtgtttt ttattttata tttgaaatat tatatatttt    11160
   gttttttttt taaataattg tgttgtcgta gattaggcga tttaatagaa gtatattttt    11220
   ttaacgattt tggaggttag aaatttggga ttagggtgtt tgcggggttg gttttttttg    11280
   aggtttgttt ttttggtttg tagatggttg ttttttttta gtgtgtttat atagtttttt    11340
   ttttgtgtgt gtttgtgttt taattggttt attttaatga ttttatttta atttaattat    11400
   ttttttaaag gttttgtttt taaagatagt tatattatga gatatagggg ttagggtttt    11460
   ttaatttagg aatttggggg aggatataat ttagtttata ataatagtaa agttaaataa    11520
   tttaattaag gttttatagt tggtatgatg gatgaatgga atttagattt aggtgttttt    11580
   tagtttaaag gttattttta gaatgttata ttataatttt ttgattttta gagatgtatt    11640
   ttaaaattag ttagagttgt ttttttaaag attaagagat gtagagaaaa taattttatt    11700
   atttagttat gtgaaagtta taattttaat ttaatttttt gagataaggt ttttatttat    11760
   cgtttaggtt gaattgtagt gacgcgttta taatttgaga ataatattaa gaagagaaaa    11820
   tgtagaaatt gaaaacgttg aattttaatg atattgtttt ttggttaagt agattatagt    11880
   tggtatttga atttaagaag ataaagaata gtatatataa gtagaaaata ggaaaaaaag    11940
   atgttagagg aggaataaat gtttagcggg ttattatagg agggtgttaa aagaataata    12000
   attgatattt tattaagtag gtatagtaat aagttataag tgttttgaaa tatagtattt    12060
   gttattgttt tatgaatgta attaatataa tagttttgat aatattgaat gtatataaat    12120
   attataaatt tttagatata tatatgagtt ttagatatag aatttcgaaa taaagtttta    12180
   tgtataggtt agttattttt ttaataaaat tattgagtat tttatatata gtaggttttg    12240
   tgttaggttt tgttaaagga gggattttta agatttttga gagtttttga gtttagtatt    12300
   gaaatatagt taattcgtgg aagaagtttt attttttgta gttttttacg ttagtaatat    12360
   aatttataat atgtgtattt attattaaat ttttttagtt atgtgaaaat aagtttaaaa    12420
   tgaataatta ttattttgtc gtattaaatt atttattatg tgaaatttat aattttattt    12480
   atttatttat ttatttattt atttatttat tgagataaga tttcgtttta ttgtttaaat    12540
   tgaattgtag tagtatattt atagtttatt gtaattttaa atttttgggt ttaagtgatg    12600
   tttttgtttt agttttttaa gtagttggga ttataggtgc gagttattat atttagttaa    12660
   tttttaaatt tttttataga gatgggattt cgttatgttg ttaggattgg ttttgaattt    12720
   ttggttttaa gtgatttttt tgttttagtt ttttaaagtg ttgggattat aggcgtatat    12780
   tattatgttt agttgaaatt tttataattt taaagaatgg agttttatga tgaaattaag    12840
   tgattgtttg tattataatt ttattataat tatttttttt aaattgagag gatttttatc    12900
   gtagttttat tttgaaatga agttagtaat ttcgtttaag tgttatagtg ttattatggt    12960
   ttatgtgtat gttttttgtt tttttttttt tgtggataga atgtgttttt taaatttttt    13020
   ttttttttag ttgtatttta tagttatatt tggagttgtg taatgtaatg tatattttgt    13080
   ttgagataat attttttttt tgtatttttt ttttttgtat agg                      13123
<210> 64
   <211> 13123
   <212> DNA
   <213> Artificial Sequence
<220>
   <223> chemically treated genomic DNA (Homo sapiens)
<220>
   <221> unsure
   <222> (8274..)
<400> 64
   tttatgtaaa aaaagagaat atagagaaaa gatgttattt taggtaggat gtgtattata      60
   ttatatagtt ttaaatatag ttataaaata taattaaaaa aaaaaaagat ttaaagagta     120
   tattttattt ataaggagga aaaaataaaa agtatatata taaattataa taatattatg     180
   gtatttgaac gaagttattg attttatttt agaataaaat tacggtaaaa atttttttaa     240
   tttaaaaaaa atagttatga taaaattata atgtaaataa ttatttagtt ttattataaa     300
   attttatttt ttaaagttat agaggtttta gttggatatg gtggtatacg tttataattt     360
   tagtattttg ggaggttgag gtaggaggat tatttgaggt tagaagttta aaattagttt     420
   tggtaatata gcgagatttt atttttatga aaaaatttaa aaattaattg ggtgtagtgg     480
   ttcgtatttg tagttttagt tgtttgggag gttgaggtag gagtattatt tgagtttaga     540
   agtttgaggt tgtagtgagt tatgaatgtg ttattgtagt ttagtttggg taataaagcg     600
   agattttatt ttaataaata aataaataaa taaataaata aataaataaa gttataggtt     660
   ttatataata ggtaatttaa tacgataaga taataattat ttattttaaa tttattttta     720
   tatggttaaa gaaatttggt ggtaaatgta tatattgtga gttatattat tgacgtgaag     780
   gattataaga aatagaattt tttttacgga ttggttatgt tttagtatta aatttagaaa     840
   tttttaaagg ttttaagagt tttttttttg ataggattta gtatagagtt tgttatgtat     900
   gaagtgttta ataattttgt tggaagagtg attaatttat atataaagtt ttatttcgaa     960
   gttttgtatt tgaagtttat atatgtattt ggaaatttgt gatgtttata tgtatttagt    1020
   gttattaaaa ttattatatt aattgtattt ataaaataat aatagatatt atattttaag    1080
   atatttatga tttattgtta tgtttattta ataaaatatt aattattatt tttttagtat 1140
   ttttttgtag taattcgtta agtatttgtt ttttttttga tatttttttt ttttattttt 1200
   tatttatatg tattgttttt tattttttta aatttaaatg ttaattatag tttatttagt 1260
   taaaaagtag tgttattaga gtttagcgtt tttagttttt atattttttt tttttggtgt 1320
   tatttttaaa ttatgaacgc gttattgtag tttagtttgg gcgatagatg gagattttgt 1380
   tttaaaaaat taaattaaaa ttatagtttt tatataatta gatagtaaaa ttgttttttt 1440
   tatatttttt agtttttggg gaaatagttt taattagttt tggggtatat ttttaggagt 1500
   taaagggttg tagtgtagta ttttgagaat aatttttaga ttgaaagata tttgggtttg 1560
   aattttattt atttattata ttagttatga gattttaatt aaattattta attttgttat 1620
   tgttatgggt tgaattgtgt ttttttttaa atttttaagt tgaaaaattt taatttttgt 1680
   attttataat gtgattgttt ttggagatag ggtttttaaa aaggtgatta agttaaaatg 1740
   aggttattaa agtgggttaa ttaggatata gatatatata gaggaaagat tatgtgaata 1800
   tattaggaga aggtagttat ttataagtta agagggtagg ttttagaaga aattaatttc 1860
   gtagatattt tgattttaga tttttagttt ttagaatcgt tgagaaaata tatttttgtt 1920
   aagtcgtttg atttgcggta atatagttat ttaaaaagaa aataaaatat ataatatttt 1980
   aaatgtaaga taaggaatat tatatataaa tatttatttt ttaattttat tatttggtta 2040
   tattttattt aaattaaaaa taaaatatat tagatatagt tttttttttt tatttttagt 2100
   ttatattttt attagtattg gatgggacgg tttattatta ttttgataat atttgtttaa 2160
   tttagatttt taagtttggt tattttagtg tgatgaaggt gaaatggaat tttgttgtga 2220
   tttatttcgt attttttttt attggtgaag aaagtttcga ttttgttgtt tttaaagtat 2280
   ggatgataaa agaattgatt ttttagtgtt gtaaggatta aaaattaaaa tatgttgaat 2340
   ttatgtttat tgatataggt atagtgtaag ttataagggt atatcgttat tgttttataa 2400
   aataaaatta atattttatt taaaaatatt ttttttgtag ttttttgtta ttgttgaata 2460
   gttgtattgt ttttatgtat aaaattttta ttttaattta gtttaaattt tatttttaaa 2520
   agtatttttg ttttttgttt taattattta tgtatttttt tagttttgat agcgtttagt 2580
   tttattgatt tttttttatt gttttgaaag attaatatgg gtattaattt ttttataagt 2640
   ttatttattt attaaaaata tatttatatt ataagttgtt ttaggtattt attaagtatt 2700
   ttttaagttt attttttttt tatttattgt atttgaatta attgttagag ttaagatatt 2760
   tttttatttt tttttttttg gttagttttg tatatagtgg agtatgaatg ttatttttgt 2820
   ttattaagtt ttttttaaat atttggttgt tttttatata aggtatatgt aggttatgag 2880
   ttagtttttt gtcgtttttt tttgtatttt tttaaatttt ttattaaatt ttttttaatt 2940
   ttaataattt ttaggttttt ttttgttttt tattatattt tatattatcg attttaaatt 3000
   atttatatgt tttattttat ttgaaattta ttttataata ttttattagg ttatagtatt 3060
   taaaaaaaat tttaagggat ttggtaagta aatttatatg tatgtgtata tacgattaga 3120
   ttttataaaa agtgtaatag aaggtaagat ataattaagt ttttaattat atagtattat 3180
   tataaataat ggtattttat ttatttttaa agattttttt tattaatttt tattataata 3240
   attattaaaa gaatttggaa gtagattagt gaaggaagga aaagaaggaa atgttaggtt 3300
   tgatttgggt ttttatttat ttgaatgtag aaagtaagtg tgatgtgaat aaaagtaaga 3360
   ttatttttat aaatttttag ttttatttta aaaagtattt attatgatat ttattttaaa 3420
   attattaaat aagaaagttt ttaaaaaata gaatatataa taataaagat atttagaaaa 3480
   ttaaattgtt tgttttgatt tttttattag ttttttagta tttataagtt tttaagtatt 3540
   tatagtatga tatataaagg tttttgaggt taagtatata ttagttatat tttttgtcgt 3600
   atttatattt tatattttgt tattatattt ttgttatatt tttttgtttg aaaaagtttt 3660
   tttatttttt ttaaatggtt aatttttttt atatattttt taaaatttaa tatagaatgg 3720
   tttttttttt aggaagtttt gtttaaaaat tataggttga tttaaaggtt ttttttttat 3780
   gttttaaata agaggatttt tggtatattt ttttatttta aaatgtaata tgttagattg 3840
   taattttgtt tagtgtttaa agggtagtat ttttttattt ttgtgtttaa gtaattagta 3900
   tggtatttgt tattcggtag gaattttaaa aatgtttgat gaattgaatt gtaagaatta 3960
   tgagatatag aaaaaagaat ttgtaggtat attttatgtt ttcgaggggt taggagaatt 4020
   tgagatttat tttgaattta tagtattgga gttttgaatg tagttagttt ttgtttaatt 4080
   tagtatttgg ataaataaag taattatttt aaattgtatt tataaaatgt tagttttaaa 4140
   taggtagtta taaagtttgg aaatatagat aaaattattt tgttatatat tataatgtaa 4200
   tgttaataga ttttttgttt ttattattgt ttttagataa ggtggttatt tgtttttaat 4260
   ataagttatt aatataaata tttattgtag ttaagaatat tattttttta agagttttta 4320
   aaaattagga gatttaggta agtgttttat taagatttat tattgataag tttagattat 4380
   tatgaagtat attggaaagg taataggaag taaagtaagt tcgaagattt taagggaagt 4440
   aatttttttt tatataggta tttaaggtat ttattgttag taagtttttt ttaggaaatt 4500
   gaaaaagaga tttaacgttt attaaaagta aaggaagaat ttttttttgg tgtggtagaa 4560
   gtatgtgttt ttaattagtt attgattaga agtattggta ttaagatgga aatgatgatg 4620
   atttttaatt ttaaggaaga gattatattt tttatatgta ttttgttaat aggttttgtt 4680
   ttttaaaata ttttgaatag aaaatataat ttataaattt aattttttaa aaattataat 4740
   aggaagttag ttaaattaat tttaatttaa ttattttagt atatattatt taagggttga 4800
   agtgttaggt aatgattttt aggtttgatt ttgtgttaag attttaaggt ttaatgttgt 4860
   gtttttttat ttttagaaaa agagttaaat ttgttaattg tgaattagga taaaaggttg 4920
   aaaaacggaa atagagtggg ggatttcggg gtgggtaata aatgtatggt gtttgttatt 4980
   tataagacgg aaaattgggt ttttgttgta aaatattgtt tatttaatag tatcgtttaa 5040
   gtattattta tttagtaatt attataatgt aaaatggaat ttgtttttaa ggattaaaga 5100
   ggaaattatt attttattgt taaaattttt taatgtttat agtattatta tatttttttt 5160
   tggagtaatt gtaaagggtt agggatgaat taaaatataa tgtatataaa tattaaaaga 5220
   aattaatata tatgttttaa atggtattga agtttgaggt taaatgtttt ggaaaaagga 5280
   taataaaaaa tttaataaag tatgttttga aaattgtaat attataatat atttaaataa 5340
   aaatatatgt ttgtaatttt ataagaatat tttttaattg aaatatttaa agaaatagtt 5400
   tttgataata gtttagtata gaatataaat tatagtattt aaatataaaa ttattgaatt 5460
   ttgttattaa tattatgaaa aattaaagta attgatattt gaagtttttt taagtggaaa 5520
   ggtagtagat aaatgattgt taatgttttt gataaaataa atatttatat agtgtagaag 5580
   atttttttat gaaaagaaga attaattagt attttttaaa agtagtaagt ttaggaatat 5640
   tatttttttt tttttttttt ttttttttga gacggagttt ttttttgttg tttaggttgg 5700
   agtgtagtgg cgtgatttcg gtttattgta atttcggttt ttcgggttaa gtaatttttt 5760
   tgttttagtt tttcgagtag ttgggattat aggcgcgcgt tataatgttc ggttaatttt 5820
   tgtattttta gtagatacgg ggttttatta tattggtttc gaacgtttga ttttatgatt 5880
   cgttcgtttc ggttttttaa agtgttggga ttataggttt gagttattgc gttcggttag 5940
   aatattattt ttaattgtta attttatata tagaaaatgt gaattataat atattatgta 6000
   tgtaatttta aggtaattta acgattaagt gaaagtgtta ttaattgaat tttttgattt 6060
   tttgtgtttt taaaattaaa gtaaaaatat ttttataagg aaattttttt ttataattat 6120
   taaagagttt taaaatatgt taaaatgttt atttaataaa agtaaaatag agtaagttta 6180
   aaaataaaat tatttgatta gaaacgttag ttataaaaat gtttgtattt tttttaaatt 6240
   tgttatttat atgttaatat agcgattagt taaattttta attttttaaa aacgtatttt 6300
   attttaatta aatcgtaatt tattaattaa tgattttaag agaaatcgta attattttat 6360
   ttatatataa aaaaggatgg tttttaatta tagaaatttt atttttaaaa agttttgtta 6420
   tgtaagtaaa tatattttat tttaattttg gatttttatt ttagaaaaaa aaaaagattt 6480
   aaaatgtata atatgtattt taaaaataaa atatttatta atatttttaa ttattaagat 6540
   attagaaaaa gtttaattga gtatttgttt agaggaggag gattcgaggt agatgagttt 6600
   aaaatttttg tatcgttttt atggattagt ttttttaaaa aatatataat tttaggggta 6660
   tacggtttaa tatttataaa ataagtattt gttgaaaatt attttcgaat agtttattat 6720
   aaaattaaaa aatagtgtta ttaatgagaa agtaggttta ttttttttta ataaaattgt 6780
   gtttttgggt ttaaaatgtt aggttatata cgttgttatt tatttttata gagttatgtt 6840
   gttttcgcgt agatattttt atagatttga aaacgtaaag tggttatatt acgttaaacg 6900
   tttttggtaa ggttttgttt attattagta ttatagatgt aaatattacg tcgataaaat 6960
   aatcggtgaa tgtgatatat agtttttatg tgtttaaata ttagtgaaat ttgataagta 7020
   ttacgatttt aaaaacgtat ttttttaatt ttaattttta aattgatgtt agagtgtttt 7080
   tagtttagat agaaattaat gttagttatt agttgttata aagttttttt taattttgtt 7140
   tttatttttt tttcggagga aatttaaatg aaacgttttt ttttataata aaataaagag 7200
   tttgggtaaa tgttaaaaat cgttggtatt tagtagtggt atagtttgag gatgagtcgt 7260
   gtatattaag tgggttataa ggtattaaag taaaaattag atcgttgaaa attacggaaa 7320
   tagttgtagt tcgttttgat ttttcgttgg ttttaaaata ttgtttttaa gtagtaattg 7380
   aaaacgtttt ataagatgaa tacgaatttt ttttagacga aaggtagttg ggagtaggac 7440
   gcgtttacga gtttcgatgg gaacgcgttt acgtcgtagt tgggattcgg gagaggttag 7500
   ggggatcgcg tggttttgtt gtggaaggga ttcgcgcggg ttcgtcgcga agggggtttt 7560
   gtttggcgcg atttggggag agtcggggtc ggtcgggtgg agggcgtacg gggatatacg 7620
   gatacgtata gatatagata cgcgtttcgg gttgaggagg gtggtgggag gatcgtcggt 7680
   cgtttttcgc gcggagtttt agggaattcg gattttagtc gtaaagcgga gaggcgttag 7740
   tagtaattag ttattttagg ggcggcggag tttaagtttt ttttagtcgg atcgtatcgt 7800
   ttaaattttt ttcgggtttt ttttttttac gtatgaggtt tttgtatcga ggcggttggt 7860
   tttatcgtag tttcgagtta attcgttaat ttcgtaggat attaatcgtt tcggtcgtcg 7920
   taggtttttt cgggattgtt ttttttaggg ggtcgttgtt tcgttttacg tcgggggtcg 7980
   ttataggacg agttttttag ttttagtcgg gcgaggcgtt agggagtttc gaacgtggta 8040
   ggatttattt ttattttttt agataagggt cgacggttgt gatttggttt ttcgtgaagt 8100
   gatttttggc gatagtagcg gagaggatta tcgaagtttg tagtcgcggt ttgaaaattg 8160
   tagtcgcgtt tggggtatcg tcgttcgtta gatttttttt tttaggtcgg gagagttaat 8220
   cggaacgggt gcggagggtg ggtttgttac gtgacgggag gggcggggta tcgnntcggg 8280
   ggcgcgtgtg cggggcgggg tttgtcggtg agagggtgcg gcgtttggtt ggttcgaggc 8340
   ggggcgggga ggcgcggcgg cgtggcggcg taggtggggc gtattcgggt tgtcgcgagg 8400
   gaggggcgga gggggcggcg ttgtagaggg ggcgcgcggg gttggaatta ttgttttgtg 8460
   tatttttttt tatttttaga gaggttagtt cgagaggtgt gaatattcgg agatcgggga 8520
   gaaggaagtc gtaaaattcg ttttagttgt ttgcgattag ttattaaatt ttagtgttta 8580
   ttaaagattt attttttgat tttttttgag agtttttttt aggatcggaa cgtattttgt 8640
   aagtggagaa tttagtttgt tataatttgg tagaggtttt tcggagtgtt aatattagtt 8700
   ggggatttat ggaatttaga ttttattgtt aacgttcgtt gtcgcgaatg gtgttttttt 8760
   tgaggtattg attgaaggta gtgtagggag ggttttaata ggttgaatag tcgattttgt 8820
   atttatagtg ttaaggaaaa agatgaaagt ttttagtaat ttatttaaga tttgttattt 8880
   tttgaagata gtggaaattt ttaaagtttt gtgtaagata taaatgtttt atatagatat 8940
   ataattttag tttatatgta taggatatat atatatttat atatatatgg gattagttat 9000
   gtaatttagg attgttttta gaggatattt acgataattg tagaaaattt tttcgtggga 9060
   aaggatattg ggagtggagt cgtttttttg tttttaaaag atttaatgtt tagttagtat 9120
   ataaaggata ggtttttttg ttttatttta gtaagttaag taaatttttg tgaagttagt 9180
   tgtttttttt ttttattgta ttttttttaa gataaaggta tagaatgtat tatagttttt 9240
   ttatgaagtt tgtttgttgt tatttaggtt aatttttttt tttaggttaa taatgttttg 9300
   tttttaaatt ttgttaaatg gagaatattg tttttattta tttaattttt tgttgttaat 9360
   aattgggttg attagagaag tttaaagtta ggatgaaaat aaaatgttat atattggaat 9420
   taattttttt ggggtataag aattcgtaag taatgagatt gtttttattt ttttttttat 9480
   gagtataggt gttgtatttt agaggtttta gtattagtat taaaattagt aattttttgg 9540
   gggttatagg attatattat tatggtggtt ttggggttgt gtgtttaatt ttggattttt 9600
   ttaattttaa tattttatta attgttagtt tttagtaatt tttttatatt tattttatat 9660
   ttttcgttat taatttagtt ttagattatt ttatatttag atatagattt tttttttttt 9720
   ggtattattg taagttagtt tttttggtta taatattttt gttgtaatta gttagttttt 9780
   tagggatatt gttatgtttt gtattttttt gtttttgttt tagttttagt ttattattat 9840
   tttttttatt tatatttgtt tattagtttt tgattttaag aaggttattt tttttaggaa 9900
   ttagaatttt gttgattttt attttttgtt tttagtttga ttatttttgt tttatgtttt 9960
   ttggtgtagg gttgatagtt tgtgtttttt ttatgatttt aaatagattg ttaatttttg 10020
   tgtttgatgt tagatttgtt aattaggatt tattatatta ttttaaggat atttaattat 10080
   atttatttgg ttttaggtat tgtggtttaa gtaataagga atttttggtg ttgaggagga 10140
   ggtagtatat ttgtggtttt gtaggaaaat ttagaagtta atatattttt ttatttattt 10200
   tatttattta aatttattta ttatcgtgtt aaagatatat gtttttgttt ttattttttt 10260
   gagtaggaaa tttttaggtg atttgatggt tattttgtgg ttattgtttt tgattatata 10320
   gtttattaag ttgattattt ttgaatgata tatttcgatt agggatggcg gttgttatgg 10380
   gtttagttgt gttttttttt agtttatacg ttgaaatttt aatttttagg aatttagagt 10440
   atgatcgtat gtggagatag ggtttttaaa gaggtaatta aggtaaaatg agataatatg 10500
   agtggttttt gatttaagat gattggtgtt ttgataagaa gaaattagga tatatataat 10560
   attgattaag ggattatttt gtgaggatat aataagaagg tggttatttg taaagtaagt 10620
   agagaggttt tagaagaaat aagatttgtt gatattttga ttaatagttt ttagttttta 10680
   gaattgtttg ttgtttaagt tgttcgtttt gtggtatttg ttatggtagt ttaagtaaat 10740
   aaatatagtg gtttttttgg attattttag gttagtaatt atatattgat tgggagcgat 10800
   gatcgttttt agttattgtt tattatatgt ggttagggaa ggtgattttt ttcggtaatg 10860
   gtacgatatt tattatattt tgtttaggga agggattttt tttagttata ttaagttagt 10920
   tatcgtatat tgattatata atgattaagg aaagtgattt ttatcgattt tatgtttatt 10980
   ttttgtagat gtagaaaagg agggttttgt atagagtaga aggttttaaa tttaaaattt 11040
   ttgttattga tatatttttt ttttgaagaa tttatgtgga aaaggtaaaa taagttttat 11100
   tatttgttta gaattgaata tattagaaat ttgtggttag gttaaaggta gttagacgta 11160
   ggtaaggtta atagtttttg aaggggcgtg gtttaatttt ttatttaata ggggtgttta 11220
   ttgaatggag ataggttttt ttaaatagat tttttttttg gaggtaggaa tagtgagtgt 11280
   atttagatgg agatgtcgta gtgtatttat ttatggtgtt tatagtatat tgtaattttg 11340
   aaagtattga gaattagaga gattttaaga gattttatat tttttgattt ttttgaaata 11400
   ttattagtaa agttaattgg ggttagtttt gtttgatgag agtaataatt tagattggta 11460
   taagtgtttt ttttaaaggg tgatatagtt ttttttttta aaatatttat gattaattag 11520
   ggttaatatg ttaatttaat tatttattgt agtttataga gtataatttg ttttgtaaaa 11580
   gttgtttaaa agaaatgtta cgtatattgg taaatgtttt attagttata ttttaaaaag 11640
   taaatataga taaaattaat tttaataatt tattttattt aatttaatat ttttaaaata 11700
   ttgttatttt aatatgtaat taataaaaat tattgagata ttttattttt ttttttaata 11760
   ttgttttaaa tttagtattt tatatttata gtatgtttta atttagatgt taaattttta 11820
   tcggaaatat ttgatttgta tttagatttt ataaaattta ttgttgtaaa aagtagattt 11880
   atatttttaa gttgttttga ataaattaaa gttttttacg ttaggtgtgg tggtttatat 11940
   ttgtaatttt agtattttgg gaggttaagg cgggtagatg atttgagatt aggagtttaa 12000
   gattagtttg gttaatatgg tgaaatttcg tttttattaa aaatataaaa attagttagg 12060
   tatggtggta ggtttttgtg attttagtaa tttagtaggt tgaggtagga gaattatttg 12120
   aacgtaggag gtagaggttg tagttagtcg agattgtgta ttattgtatt ttagtttggg 12180
   cgataaagcg agattttgtt ttaaaaataa ataaagtttt ttaataatta aatatagtat 12240
   tagtttttta atataaattt aatgataatg aaataaaata gaaaattttg ttatgttagt 12300
   tatattttaa gtgttttatg gttatatgtg gttattaagt taggtagtgt aggtttgaag 12360
   ggttagaaac gtgtttttta attgtgttta aatttttagt gttagagata attagagtag 12420
   tttggagtta gaaaaatttt tgtagtttag taatttaatt tttttgtttt agaagggaag 12480
   aaatttatag tgagagaagt tgagggattt gtaaaagatt atatatttaa attcggtggt 12540
   atatatttga ataataattt aggttttttg atttttaatt ttaagatatt atttaagttt 12600
   ttttatatta gatatatttt agaataaagt aatttttaaa tttatttgaa ttattatttc 12660
   gatgatatag gtaagagaaa gtaaatttgt attttaattg tatttttata atttgataag 12720
   agtatatttt ataatattta aggtattttt tttttatttt gttttagggt ttatgtggtt 12780
   ttatttgtta gtttttttat tttaatgtat tattgttttt tttttttgga gttatttggt 12840
   cgaatatcgt tgtcgttcgt ttatagagta ttcggttata tttttttgtt tgtagttttt 12900
   tttttttagg tgtttattat acgttattta aggttgtgtt ttagagaatt ttttatattt 12960
   gtaaattaaa atatttagta gaaattatag taaagttgta gaagagttgt tatgttttag 13020
   ttataaatat attaggtttt aaagttttat ttcgtgagaa gttgtgagag agaaagagaa 13080
   gatatatgtg tggttaagtg tgtgatatta ggtagttttg ttt 13123
<210> 65
   <211> 8770
   <212> DNA
   <213> Artificial Sequence
<220>
   <223> chemically treated genomic DNA (Homo sapiens)
<400> 65
   ttagtttaga ggaattataa aattttaaga agttttcgaa ttaaatatga atggtgatag      60
   tgaagaagaa agagaaatgt tattatatta ataaatatag gttaagtgag taaaggaaga     120
   aacgtgtgta tattttttat aataaggttg tttttttttt aattttgtta ttaaatttat     180
   agtgtgtttt ataaatatgg atttttagaa ttatagaaat attatagttt tttttttagt     240
   ttgttaaagt gtttggaatt tttattatta agttttagga agttttgtaa gaatatttta     300
   ttaagaaata taatatagta atatattata atttgtttga aaaatgagta tgatttattt     360
   tattagttag tttcgggaaa taggtttttg aaagaatgga agttttttaa attttttttt     420
   tatggagatg gttggtagtt aaaggagtag gattatagag attttaaatt agattgaagg     480
   gattgaggga taaatgagaa aggggaatgt taaggtaaat ttttataatg gtttgtatta     540
   ttttaagtaa taggaattaa tttattttag aaatatcggg tttattgtta aaattaatat     600
   tgtttttagt tcgtttttaa taatatttat tttggggttt tttaatattt tatttggtta     660
   ttaatagtag tttttataga aatataaatg aaattaagaa ttgaatattt atatttatga     720
   tgtaaaataa aattattttt ttaagtgttt tagttaagtg ttgataggaa ataaaataga     780
   atttataggg atagtggatt ttttgataag tttgagatgt tagtatcgta cgtttttagg     840
   taaagtgtaa tagaaggaat tgtatttgtt attatattaa ttgttttttt tttagtggag     900
   ggtttttgat tttggtatta tgatttattg ggttattaag gattttagtt gtttttttat     960
   tatggtgggt tttgggtttt tttatttagt ttagataata tatgtaaagg atttgaggtt    1020
   tttagataag aagtaatatt tagtagaatt tggtatttat aggatgatga aatttatgat    1080
   attattattt tagtttttac ggttataatt tatagaaagt ttttttttat ttttagaatt    1140
   agtttttgta gtggttttga atgtgtaaac gttatttttt agtaatgttt gaggtagaat    1200
   ttgaaaattt gaaatatgat ttaaagttat tttataataa ttaagttgtt atagttattt    1260
   aaggttgttt tttaatatgt tttttatgaa tttgtttttt taatagagta tagtgtaggg    1320
   aaaaggggag ggggtaattg tataaaagat ataaaaggtt tagttttagt ttggtttatg    1380
   attgttatgt tttggaattt tgtatagttt tagaaagaga gtgttattga ttattaaaaa    1440
   tatataattt tttttttttt atttttttaa tttttagaaa tatgggtttt attatgttgt    1500
   ttaggataga tttaaatttt cggatttaaa ggattttttt tttttagttt ttcgagtagt    1560
   tgggattgta gacgtatgtt aattaataat taataattag gggttaggcg cggtggttta    1620
   cgtttgtaat tttagtattt tgggaggtta aggcgggtgg attacgaggt taggagaatc    1680
   gagattattt tggttaatac ggcgaaattt cgtttttatt aaaaatataa aaaattagtc    1740
   gggcgtagtg gcgggcgttt gtagttttag ttattcggga ggttgaggta ggagaatggc    1800
   gtgaattcgg gaggcggagt ttgtagtgag tcgagatcgc gttattgtat tttagtttgg    1860
   gtgatagagt aagacgacgt tttaaaaaaa aaaaaaaaaa aaaaagaatt aataattaat    1920
   taattgttgt tgatttttga ttaagaaagg tttatgaaat atgtttgagt ttagtagggt    1980
   ttttttttat ttgttatacg tggtgttttt ttttattagg ttttttttgt tttttttatt    2040
   tgggtaagta tatttttttt attgtatggt atagggatat aggttgtaag tatagtttta    2100
   cgggaatcgt ttaattttaa gaggttatgt gtgtttattt tatatagttt atgatttata    2160
   aattttagta ttggtgttaa aatggtacgt aatttaggaa ttttttaaaa atattttata    2220
   taattagaat ttttttttta tttaataagg agatatattt ttttttttaa gtgtttatta    2280
   aatgttaata aatatttttt tttttaaaga gttttgttta ttttgataag gtttatagtt    2340
   taaattaaat tttttttaag gttttttagt attttatatt tttttggttt taatttattt    2400
   aataaatttt tttaaattgt attttttttt ttttttataa gaaggtattt tatttatatt    2460
   tttttttttt tttttttttt ttttttttga tttattaaga aaattgttat tggttttgtt    2520
   tatgtgttat ataatagaag gggaaaaggt aagggtatag tttaatatag cgttgattta    2580
   gaaggtggaa aggatagtaa agatcgtttg gtggtgggag tggtaattta taagtttagt    2640
   agtagaatta tggaaagtgg agttcgaata taatttaggg ttatagatta tttttgtatt    2700
   tgattgttat tattttgtta taaattttgg taggtaagat gataaagtag ttatattagg    2760
   atagttggta gtggtaggta tgaacgtggt tttttaggtt tgttattttt ttttattgta    2820
   tttttagttt atgatattag atagtaagat atgagttaga taggggtagt ggaagttgta    2880
   ggaggattga agttatggag ttagagatga gaggaaatag gttaattttg tttataaagg    2940
   agtattagta ggagagtggt gtgaggatta atattttatg tagggttttt tagatatatg    3000
   attttttttt gttagtttaa ttgaaattta ggaggttttt tgaaggtgaa attaagattt    3060
   ttttattttt gaagtcgtat tttgtttaag gttcgtgttt ggttgttttg agaggttggg    3120
   agataggata ttaaaagagt atttgtttta taggattttt tttgagagga atggtttagt    3180
   aagattggtt attatgggtt ttggagtaat ataggtttaa tgttattggc gattttgttt    3240
   tttttggttg tggaaattgg gtaaatgttt taagtttttt aatttttata taaaaagtat    3300
   atataagtat atagtaagta tttgataaat gttattttag attttgttat ttttgttagt    3360
   tttggatttt agtaaggttt atttataatg gatagttgat tatttattgt ttgaatgttg    3420
   atagtatatt tgtgaataag aagagaggat aaataataag aggtttggaa ttggaagaaa    3480
   ttttagttat cggttttaaa agacgaggat tagtttttag agaggttatt agtgatattt    3540
   tgttttaaat ttttttttgt taaaaaagtg ttatttttta tttagttttt atttttattt    3600
   ttgtaagttt ttattggcga gaggaggata tttttttaat taaaaatttg tgatatatgg    3660
   tttgataagt atttttaaat gtaattaagg ggattatagt atagaatatt tttattcggt    3720
   tttagtttag ttaatttagt aaatgattta aagggtttcg tgtttagagg gtagtaagat    3780
   agtaaaaacg ttttaagatt tttttttgta tagtatttgt atttttattg tttagggggt    3840
   tgtatttatt tttatatgat ttattttttt tgtgtaacgt atgggggtag ggagggaagc   3900
   gttagtatta ttagttatta gtattttggg gtgaattgat tattaggata attaaaaata   3960
   tatttaataa agttttggag atatatggta agtatcgtga tggattagaa gcgattttta   4020
   agttgaaatg ttgttttgaa tgtaatgtta gttggatgtt tggtgagtag aaatagataa   4080
   ggggaaatgg agtaagcgcg gtataggggt gagtgttgtt ttgattttta agtttattta   4140
   agattggggt tacggggtcg ggtgtttttt gggatttacg ttttatgtag tttggggttt   4200
   agagttggta ggaataatgg agatggggat taaaggttgt tatcgttgtt ttttttcgaa   4260
   atgttcgttc gggttttgtt tggggttttc gtttacgcgt ttattacgtg tatagtttaa   4320
   attttttaga gtcggttcga ggtttgcggg ttcgggcgtt agggtggttt ttagggtttt   4380
   tttttttttt tatttttttt tttttttttt atagttgatg attttttgta gaaaaataat   4440
   tgcgaaaaag aatttatttt atttttagtt tttattatcg cgatttttat atggatgtga   4500
   cgtgatttta gttttcgaaa tgtttaggtg atttacgcgg ggatatttcg gggcggggcg   4560
   agggagtttt ttcgggcgtt tgtagggatc gggcggggcg gggtagcggg gcggggtagg   4620
   gaaagggggc ggggcggggt tcgtaggttc ggtcgggcgg aggtcgcgcg cgtttttcgt   4680
   tttttgcgtt ttttggcggt cggttggacg tatttcgtag ttcgatttag agagagttac   4740
   gtgattttgg aaagtttcgt ggaaattttc gggtttataa ttcgtatata attgaaacgg   4800
   ggtaaagtag attgcgtagt ttgtagtttt cgtggcgggt taagcgagtt tggagttcgc   4860
   gggggcggag cggtgagagc ggtcgttaag agagattata tttttagtcg attttgttag   4920
   cgagcgagtt cgattttagg cgtttatgga gcgtcgtttt cgttcggttt ttgtttcgat   4980
   tttcgtttgc ggcgcgtttt tgttttgatt aggatttggg attttgcgaa aggatcgcgg   5040
   ggttcggaga ggtaatcgtc gcgtttttcg gagaggtaat tcgtcgcgtt tttcggagag   5100
   gtaatcgtcg cgttttttcg tgtcgtttgt atcgggttta tcgtagttaa gggatgcggg   5160
   tgtatgttgg gtagcgattt ttcgggttgg tattatatat atggcgttgg ttttgtagcg   5220
   attatggaat gggagtatga tggacgaata tgtatgggaa tatgaagtga tgggagatga   5280
   ggttattgat gtagaggatg gagatgatag ttatggagat atttagttag ttgttttttt   5340
   tagataatgt acggaattta agttgtagtt gattggtgaa gaggcgtttt tcgggttttt   5400
   cgatcgggtg gtcgttggcg ttttgttttt ttcgtgtttt ttttggagtt aagcgttgta   5460
   tgtttatggt tacgtagatt ttgtagaagc gttgcggttt ttttttaaat tgtgtaggat   5520
   tagttcgacg gggcgtaggg tatcgtagtg gtcgttagtt cggcggaggt ggggggtttt   5580
   agttttagag atagtttttt agacgtttac gggtttttgt agggggttgg ggttttggcg   5640
   cggggtttag tttttttagt agtagtagtg taagagaagt atgtatgtag acgtgggtcg   5700
   ttggtttttg ttttagtttt tattttacgg ttagattgtt tttgttagtt ggagtcgttt   5760
   tatattttgt tatttggttt tttagaaaag tagtggtatt aagtaaggaa ggagtttaat   5820
   tttttttgag ttcgttttgt ttttcggttt tttcgtttta gatttttttg ttttggaaag   5880
   ttagtttggg ttagtgtttt tatggtagtt ttttggtaga attattggtt attaaaggga   5940
   tttttggatc gtaattttaa tgtattagat tattgagtag ttaatgaatg ttttattttt   6000
   attgtttaag gtgttgtttt gatttttttt ttaatttttt gtattatttt tgattttttg   6060
   gagaggtata tttttaaatt tggatgaggt atttgttgat aaataattta ttaattttta   6120
   taatgtagat aaaaatgttt gtttagagtg gaaaaataaa ataaggggga gaagagtttg   6180
   agtaacggag aagttttgtg gaattttagt gataaaagtt gagaaattat ttttaaataa   6240
   gatagtgagg taataaatgt taaaaaaaaa aaaagttaag ggtaatagtg tattgtttgt   6300
   tatttataat gtatttgaaa tatttgtttt ttaattttaa gcgtagagtg gtttaagatt   6360
   tggaaatttg ggtagtgggt tttgattttt aaggaggtag ttttataggt tgtgtaaggt   6420
   taattttatt aatgagatta tattggcgta ttcgatattg atatttgtat gttttgttta   6480
   tggtttatag tttttattat tttagatttt tggtgaaaaa tttttagatt tagaagagaa   6540
   aaataagagt taggtaagta aaaagagata atattttatt tgtttaaaat ttttttattg   6600
   tttatttttt tttatttgat tgtggtaggt attgtagtta taggtttggt gttgtgggat   6660
   tgtaaggggt aggtattgaa agatttggtt tttaggtaaa attttgtttt tgaatggttg   6720
   tgggatttga gtagtatttg taattttatt gttttagttg tagattaagg tggtagtgta   6780
   attttaggga agggaataag gttatttttg atttttagtt ttattattaa gtatggtgat   6840
   tggtttttag taggtggtta ggtagtgttt gttgggtgga tgggtggatg gatggataga   6900
   tggatggatg gatggatgga tggatggatg gatggatgga cggacggacg gacggataga   6960
   tgatattgtg aaagagattt ttgttgtgtt ataaaggaaa ggtatatgtg ttatttttat   7020
   tttgttatga ttttgtttaa ttttatttgt agaggacgtt tgatattaaa agagagtatt   7080
   atttgattta gagaatgatt tttattatgt tttttttagt aattagatta agaattttaa   7140
   tttaaaatta ttattagaaa atttttgagg agaaattatg ggttatttag tttaattttt   7200
   tttttttgag tgggttataa gaattatttt aatattttat taatggtgtt gagagtttag   7260
   atttatatag aagtagttat tttaaaattt aggttttttt tttttttttt tttataggtt   7320
   taatattttt attaattgga aagttgtttt ggtaaataag atgttgtagg aatggaatta   7380
   ataattattg tgaaattttt tagggaatgt ttttgtgttt tgaaaatttt tttatttttt   7440
   tggaaattag gaagtgtggt tagatatttt attaagggtt agtaaattat agttggtttt   7500
   ttatagtgtt ttatattgga gagattttat attatgttaa tttgtattat tttgcgttgt   7560
   tggtatgggt tgtagatgtt ggtttttgtt attttttttt ttttatttgt tagagtggtt   7620
   ttttggtgga aatttttttt tttgtttatt tttagatttt tatttttttt attagttgat   7680
   tatgtttaaa gattttaggg tgaaaagtta ggtagatttg tttttgtgtt ggtatttgga   7740
   gggtgatgat aaaattttat tggtagttga gttaaaatga ttgagtttta atatttgttt   7800
   ttagaagtat agaatttatt gttttattat tattttgcgg ttttttattt tttgttttcg   7860
   tagagattta ggttttatcg tgttgtttag gttagtttta aatttttgag tttaagtgat   7920
   tttttttttt cgatttttta aagtgttagg attaaaggta tgagttatta tgtttagttt   7980
   attgttagtt tgttttttat tttgatatga agttcggtgc ggaggttttg aaggaatgtt     8040
   gattgttttt aaaattgtaa agtattttgt tggaaatgga tttttttatt gattagaaaa     8100
   tattcggttt taatttttta taaatattag aataaatttt atgattaaat attttataaa     8160
   tatttttttt tggggttaaa gaggaaatat taattagtag tttaagtgat agtataaaat     8220
   tgtaagattt tttataaagt attttattgt ttttttagta tagggaggga gtgataattt     8280
   aagagtttgt ttaagagtag gagtgtttgg tggtggtttg aggggaggag ttatagattt     8340
   gttagggtta ttttgagttt ttaattggta tttagtattt tttaaattta gtgttgttta     8400
   gggtggtttt agaatgaaac gggagtttaa gttggttttg aggagtagta tttgttgttg     8460
   atagataggg ttggtttttt gataaatatt attgaaaata cgggttattt tttttttttt     8520
   tagtatttat tttttgtttt tttttttttg tttaggttaa ttttttaaag tgatgtaatt     8580
   taaaatagtt tagtgtgagt taatttttgg ggttagttag tatttcggga gtagaggtgt     8640
   taagattttt tgtttataga ttagggtaat gataagatta aatattgggg ttttttgtag     8700
   gtagttatag aggagtcgta ttaaagttag gttaatagaa tggttttttt tttttttttt     8760
   tttttttagg                                                                8770
<210> 66
   <211> 8770
   <212> DNA
   <213> Artificial Sequence
<220>
   <223> chemically treated genomic DNA (Homo sapiens)
<400> 66
   tttgaaaagg aaaggaaaaa aaaaaagtta ttttattagt ttgattttaa tacgattttt       60
   ttatagttgt ttgtaggaaa ttttagtgtt tgattttgtt attattttga tttgtaggta      120
   aaagatttta gtatttttat tttcgggata ttagttggtt ttagagatta atttatattg      180
   gattgtttta agttatatta ttttaaaaga ttagtttaaa taagagaagg aaggtagaga      240
   gtgggtattg aggaggaggg gaataattcg tgtttttagt aatgtttgtt agggagttaa      300
   ttttgtttgt tagtaatagg tattgttttt taaaattaat ttaagttttc gttttatttt      360
   gaggttattt tgaatagtat tggatttggg gagtattgga tgttagttaa gggtttaaag      420
   tagttttgat aagtttgtag tttttttttt tagattatta ttaagtattt ttgtttttgg      480
   gtaaattttt gagttattat ttttttttta tgttggaagg atagtaaaat attttatgaa      540
   aaattttgta attttatgtt gttatttagg ttgttggttg gtgttttttt tttagtttta      600
   aagagaagta tttataaagt gtttaattat gaagtttatt ttgatgttta taaagagtta      660
   aaatcgaatg ttttttaatt aataaaaaag tttattttta gtaagatgtt ttgtaatttt      720
   aagagtagtt agtatttttt tagaattttc gtatcggatt ttatattaga ataagaaata      780
   gattaataat aggttaggta tggtggttta tgtttttaat tttggtattt tgggaggtcg      840
   agggaggagg attatttaag tttaggagtt tgagattagt ttgggtaata cggtgagatt      900
   taggttttta cgaaaataaa aaatgaaaaa tcgtaaaata ataatagaat aataaatttt      960
   atatttttaa gaatagatat tggagtttaa ttattttaat ttaattgtta atgaaatttt     1020
   attattattt tttaggtatt aatatagagg taagtttgtt tggtttttta ttttgaaatt     1080
   tttaagtata attaattagt agaaggaatg aaaatttaga agtagataag gaaagaagtt     1140
   tttattagga ggttattttg gtaaatgaag gaggaaaggt ggtaaagatt agtatttata     1200
   atttatgtta atagcgtagg atgatgtaaa ttaatatgat gtaggatttt tttagtgtgg     1260
   aatattgtaa aaagttagtt gtgatttatt agtttttagt agggtgtttg gttatatttt     1320
   ttaattttta agaagatgaa gaagttttta gagtataaag atatttttta ggaaatttta     1380
   tagtgattgt taattttatt tttgtaatat tttatttgtt agggtaattt tttagttaat     1440
   aaaaatgtta aatttatgga agagggagga gaagaaattt agattttagg gtagttattt     1500
   ttgtgtgggt ttagattttt aatattattg ataaggtgtt gagataattt ttgtggttta     1560
   tttaaaggag agaggttgga ttagatgatt tatggttttt ttttaggagt tttttggtga     1620
   tagttttaga ttgggatttt taatttgatt gttgggaaag gtatagtggg agttattttt     1680
   tggattagat ggtgtttttt tttgatgtta gacgtttttt ataggtggaa ttgggtaaaa     1740
   ttatagtagg ataaaaataa tatatatatt ttttttttgt ggtatagtaa aagttttttt     1800
   tataatatta tttattcgtt cgttcgttcg tttatttatt tatttattta tttatttatt     1860
   tatttattta tttatttatt tatttattta tttatttaat aagtattatt tgattattta     1920
   ttgaaggtta attattatgt ttgatgatgg gattaaaagt taaagatgat tttatttttt     1980
   tttttgaaat tatattatta ttttagtttg tagttaaggt aatggagtta taggtattgt     2040
   ttagatttta tagttattta ggggtaaagt tttgtttagg agttaggttt tttaatattt     2100
   gttttttgta attttataat attaaatttg tggttatagt gtttgttata attagatgga     2160
   aagagatggg tagtaggaag attttaaata aatgaggtgt tatttttttt tgtttgtttg     2220
   atttttattt tttttttttg aatttaaaga ttttttatta aagatttaaa atagtgagag     2280
   ttgtaaatta taggtaagat atgtaaatgt taatatcgga tacgttagtg tgattttatt     2340
   ggtagggtta gttttgtata gtttgtaaaa ttgttttttt agaaattaaa atttattgtt     2400
   tagattttta ggttttggat tattttacgt ttaagattaa agaataaatg ttttagatat     2460
   attataagtg gtaaataatg tattattatt tttaattttt ttttttttta atatttgtta     2520
   ttttattgtt ttatttaaag gtagtttttt aatttttgtt attaggattt tatagaattt     2580
   tttcgttatt taaatttttt ttttttttgt tttatttttt tattttgggt agatattttt     2640
   gtttgtattg tggaaattga tgaattattt attaataaat attttattta aatttgggga 2700
   tgtgtttttt taaaaaatta aaaatagtat aaagaattga aaaaaaaatt aaagtagtat 2760
   tttaaataat gagaatgaag tatttattgg ttgtttaata atttggtata ttaaggttgc 2820
   ggtttagaag tttttttaat aattaatggt tttgttaaaa ggttattata agaatattga 2880
   tttagattgg ttttttaagg taagaagatt tgaggcgagg agatcgaaaa ataaagcggg 2940
   tttaaaaaga gttaagtttt ttttttattt aatgttattg tttttttgag aggttaggtg 3000
   gtaggatgtg ggacgatttt agttgataaa gatagtttaa tcgtggggta ggggttggag 3060
   taggggttag cgatttacgt ttatatgtat atttttttta tattgttgtt attggaaaag 3120
   ttgaatttcg cgttaggatt ttagtttttt gtaaggattc gtgagcgttt gggaagttgt 3180
   ttttgggatt gaagtttttt attttcgtcg ggttggcggt tattgcggta ttttacgttt 3240
   cgtcgggttg gttttgtata atttgggaaa aagtcgtagc gtttttgtaa ggtttacgtg 3300
   gttatgagta tgtaacgttt ggttttaaaa aagatacgaa aggagtaaag cgttaacgat 3360
   tattcgatcg gagggttcga ggggcgtttt tttattagtt agttgtagtt taagtttcgt 3420
   gtattatttg aaaggaatag ttggttggat gtttttatgg ttgttatttt tattttttgt 3480
   attagtgatt ttatttttta ttattttata tttttatata tattcgttta ttatattttt 3540
   attttatgat cgttgtaaaa ttaacgttat gtatatagtg ttagttcggg gggtcgttgt 3600
   ttaatatgta ttcgtatttt ttgattgcgg tgagttcgat gtagacggta cggggaggcg 3660
   cggcggttat tttttcggga ggcgcggcgg gttatttttt cgggaggcgc ggcggttatt 3720
   ttttcgggtt tcgcgatttt ttcgtaaagt tttaagtttt ggttaaggta ggagcgcgtc 3780
   gtaggcgggg gtcggggtag ggatcgggcg gaggcgacgt tttatggacg tttggggtcg 3840
   ggttcgttcg ttggtagggt cggttggggg tgtgattttt tttggcggtc gtttttatcg 3900
   tttcgttttc gcgggtttta agttcgtttg gttcgttacg aagattgtag attgcgtagt 3960
   ttgttttgtt tcgttttagt tgtatgcggg ttgtaggttc ggggattttt acgggatttt 4020
   ttaaagttac gtgatttttt ttgggtcggg ttgcgaagtg cgtttagtcg gtcgttagag 4080
   ggcgtagggg gcgaggggcg cgcgcggttt tcgttcgatc gggtttgcgg gtttcgtttc 4140
   gttttttttt tttgtttcgt ttcgttgttt cgtttcgttc ggtttttgta ggcgttcgga 4200
   gaaatttttt cgtttcgttt cggggtgttt tcgcgtgagt tatttgggta tttcggaagt 4260
   tggggttacg ttatatttat gtggaaatcg cggtgatggg aattggaaat gaaataggtt 4320
   ttttttcgta gttgtttttt tgtagaaaat tattaattgt ggagaagaag aagggaaata 4380
   agaaagaaag aaaattttaa aaattatttt ggcgttcggg ttcgtaggtt tcgggtcggt 4440
   tttgaaaagt ttgggttgtg tacgtgatga gcgcgtaggc gggagtttta gataggattc 4500
   gggcgggtat ttcgagaaaa agtagcggtg atagtttttg gtttttattt ttattgtttt 4560
   tgttagtttt ggattttagg ttgtatgaga cgtaggtttt aggggatatt cgatttcgtg 4620
   gttttagttt taggtaaatt taggaattag aataatattt atttttatgt cgcgtttgtt 4680
   ttattttttt ttgtttattt ttgtttatta agtatttagt tgatattgta tttaaagtag 4740
   tattttagtt tagaaatcgt ttttagttta ttacggtgtt tgttatgtgt ttttaaggtt 4800
   ttattaggtg tgtttttagt tgttttgata attagtttat tttagggtgt tgatggttga 4860
   taatattggc gttttttttt ttatttttat gcgttgtata gggagggtga gttatgtgga 4920
   gatggatgta gttttttggg tagtgggggt gtaggtgttg tgtagggagg agttttaggg 4980
   cgtttttatt gttttattgt tttttaagta cgggattttt tggattattt attagattgg 5040
   ttaaattaga atcggatgga aatattttgt attatggttt ttttaattat atttaagaat 5100
   atttgttagg ttatatatta taggttttta attagaaaag tatttttttt tcgttagtaa 5160
   ggatttgtag agatgggggt gggggttgga tggagggtgg tatttttttg gtaaaaagaa 5220
   gtttggagta gagtgttatt agtggttttt ttgaagattg gttttcgttt tttaaaatcg 5280
   atggttggaa ttttttttag ttttaaattt tttgttattt gttttttttt tttgtttata 5340
   aatgtgttat taatatttaa ataatggata gttagttatt tattgtaaat ggattttgtt 5400
   ggagtttagg gttagtagaa atgatagggt ttaaaatgat atttattaag tatttgttat 5460
   atgtttatat atgtttttta tatgaaggtt aagaagtttg aggtatttgt ttagttttta 5520
   taattaaaag gggtagagtc gttagtggta ttaagtttgt gttattttaa agtttataat 5580
   gattagtttt attagattat tttttttagg aaagattttg tgagataggt atttttttgg 5640
   tgttttgttt tttagttttt taaaatagtt aaatacggat tttagataga atgcggtttt 5700
   agaggtagaa gggttttggt tttattttta ggaaattttt tgggttttag ttgagttggt 5760
   agaagaagat tatgtatttg aaagattttg tatggaatgt taatttttat attatttttt 5820
   tgttggtgtt tttttgtaaa tagagttggt ttgttttttt ttatttttgg ttttatgatt 5880
   ttaatttttt tgtagttttt attgttttta tttggtttat gttttgttgt ttggtgttat 5940
   gaattgggag tgtagtaaag aggagtgata agtttgaggg gttacgttta tatttgttat 6000
   tgttaattgt tttgatgtaa ttgttttgtt attttgtttg ttaggatttg tgataaggta 6060
   atagtagtta gatgtaggag tggtttatag ttttggatta tgttcgaatt ttatttttta 6120
   tgattttatt gttgggtttg tgaattgtta tttttattat taggcggttt ttattgtttt 6180
   ttttattttt taaattagcg ttgtgttggg ttgtattttt gttttttttt tttttattat 6240
   gtaatatatg gataagatta atgataattt ttttggtgaa ttagaaggaa agaaaagaag 6300
   agaaggagag agtatagatg gaatgttttt ttatgaaaaa aaggagaaat ataatttggg 6360
   gagatttgtt gaataagttg aaattaaaga gatatagaat gttgaaaagt tttgaaggag 6420
   atttaatttg agttataggt tttgttaaga tggatagaat tttttaaaga agagaatatt 6480
   tattaatatt tagtagatat ttaggagaaa aaatatgttt ttttattaga tgaaaaagga 6540
   attttgatta tgtgaaatat ttttggagag tttttaagtt acgtgttatt ttgatattaa 6600
   tgttggggtt tatgggttat gaattatgta aaatgagtat atatgatttt ttggaattaa 6660
   acggttttcg tgagattgtg tttgtagttt gtatttttgt attatgtagt aaaaaagatg 6720
   tgtttgttta gatgggggga gtaggaagaa tttgatgaaa gagagtatta cgtgtgataa 6780
   gtgagaggag attttgttga gtttaaatat attttatgag ttttttttaa ttaagaatta     6840
   gtaatagttg attggttgtt gatttttttt tttttttttt tttttttgag acgtcgtttt     6900
   gttttgttat ttaggttgga gtgtagtggc gcgatttcgg tttattgtaa gtttcgtttt     6960
   tcgggtttac gttatttttt tgttttagtt tttcgagtag ttgggattat aggcgttcgt     7020
   tattacgttc ggttaatttt ttgtattttt agtagagacg gggtttcgtc gtgttagtta      7080
   ggatggtttc gatttttttg atttcgtgat ttattcgttt tggtttttta aagtgttggg     7140
   attataggcg tgagttatcg cgtttggttt ttggttgttg attgttaatt agtatgcgtt     7200
   tgtagtttta gttattcggg aggttgagag ggaagggttt tttgagttcg ggagtttgag     7260
   tttattttgg gtaatatagt aagatttatg tttttaaaaa ttaaaaaaat aaaaaagaag     7320
   aaattatgta tttttaataa ttagtgatat tttttttttg agattgtata gaattttagg     7380
   gtatggtagt tatgggttaa attaaaattg gattttttgt gttttttgta taattgtttt   7440
   tttttttttt ttttgtattg tgttttattg gaaaggtaga tttatagaaa atatattggg     7500
   aagtagtttt aggtaattat ggtagtttag ttattgtagg gtagttttga attatgtttt     7560
   agatttttaa attttgtttt aaatattatt aaagggtaac gtttgtatat ttaaggttat    7620
   tataagggtt agttttgaga ataaagagaa gttttttgtg gattataatc gtgagaattg    7680
   ggataatgat gttataaatt ttattatttt atgaatgtta aattttattg aatgttgttt     7740
   tttatttagg gattttagat tttttatatg tgttatttgg attgaatgga gaaatttaag     7800
   atttattatg gtggagggat aattgaagtt tttagtgatt tagtgagtta tagtattagg     7860
   attaaagatt ttttattggg aaggaggtag ttggtgtaat gataagtata gttttttttg    7920
   ttgtattttg tttgaaagcg tgcgatgttg atattttaaa tttgttagaa aatttattat     7980
   ttttgtgaat tttgttttat tttttgttaa tatttggttg aaatatttgg gaaagtaatt     8040
   ttattttata ttataaatat aaatatttaa tttttaattt tatttatgtt tttgtgaagg     8100
   ttgttattaa tgattaaata ggatgttaga aagttttaga atgggtgtta ttaggaacga     8160
   gttagaagta gtgttggttt tagtagtaag ttcggtgttt ttgagataaa ttagtttttg     8220
   ttatttggaa tagtgtaagt tattgtgaaa atttgttttg atattttttt tttttatttg     8280
   ttttttaatt tttttagttt agtttggggt ttttataatt ttgttttttt ggttgttagt     8340
   tatttttata aaaaaagaat ttgaaaggtt tttatttttt tagagatttg tttttcggga     8400
   ttggttggta agataaatta tgtttatttt ttaaatagat tgtgatgtgt tattgtgtta     8460
   tattttttga taaaatgttt ttatagaatt ttttagagtt taatggtgga aattttaagt     8520
   attttagtag gttggagaaa gagttgtggt atttttatga ttttgagagt ttatgtttgt     8580
   aagatatatt atggatttaa taataagatt gggaggggaa taattttatt gtaggaaatg     8640
   tatatacgtt ttttttttta tttatttagt ttatgtttat tgatgtgatg atattttttt    8700
   ttttttttta ttgttattat ttatgtttga ttcgaaggtt ttttggagtt ttatgatttt    8760
   tttgagttga                                                            8770
<210> 67
   <211> 6264
   <212> DNA
   <213> Artificial Sequence
<220>
   <223> chemically treated genomic DNA (Homo sapiens)
<400> 67
   gggtaatata gtgagatatt atttttataa aatatttaaa aattagttag gtatggtgat      60
   gtatgtttgt aattatagtt atttgggagg ttgaggtggg aggatggttt gattttagta     120
   gtttgaggtt atagtgagtt ataattatgt tattgtattt taattttagt aatagtgtaa     180
   gattttgttt tttaaaaaaa aaaaaagata tttaggtatt taggtaggaa tttgaaaggc     240
   gaataaaagt ttattatata gggtaaggtg gagggagggg aaggttttta aagtagaggg     300
   aaaagtataa atggatgtaa agaggtttga tatataagtt tttgttttat ttttttttta     360
   gattattgtt aatttgaggg gaatgtagga gataattgaa taaattcgta ggttaaggaa     420
   attgtaaaaa tggagtattt ttggttttaa gagggaggat aattggtgga atattaaatg     480
   attttatatg atttgggtat tggttatatt ttttattttt tttattattt ttttttttgt     540
   ttattttgtt ttgggtatat tgattttttt gttatgtttt tgagatgtta agtataaggt     600
   tgttttaagt tttttgtttt tgttgttttt attgtttaga gtttgatttt tttttttttt     660
   ttatttttta gaggtttttt ttggttagtt tatttaaaat aggtgttatt tttttagatt     720
   tatttagttg ttattttttt cgtagtattt attattattt tatttatgtg tttgttattt     780
   agtttttttt aattagaata taagttttat gaaggtaagt tttttttttg ttatttttaa     840
   agtttagaat agggtttggt aaaatatagt tatacgagta ttagtaaaag agataaataa     900
   aaggagtggg gagagggtag agaagaattt agatgagttg gaggtgaagg gaataggtat     960
   tgtaggagtg taattttaat agtttttgtt ttattggttg agtaagatgg gaatttattt    1020
   tttgagagga gagagggatt taaagtttgg gatatttgtg gaagatgaga acgagtaata    1080
   tagggagaaa gttgggaatt taaatatttg aatttaaatt aaatagataa ttaattgtat    1140
   tgtgttaagg ggtggggtga gagttaaaag agaagatagt tttttttcgt ttagaatgtg    1200
   gtattttgat ttattttttt tggagttgag tagagaatta tagggtgggg agaattaaat    1260
   agaggttttt gaaagaataa gtaggtgagg gtgggagcgg agagaaattg aaatttggta    1320
   gaaaataatt tttttgagag ttatgtttga gtggtataga atttgagaga atgaaattaa    1380
   agtatagata agggtttttt ggttttacgt ttttatgatt aaaaaaaaaa aagtttttaa    1440
   tatatttgtt atttggtagt tgggaatttt taattggtta gaagtgagtt ttaggatata 1500
   ttgttaattg ttgttttttt tttttttttt ttttttgggt agggtatttg tgtaaataga 1560
   tagtaacgaa atagaattgg attgttttat tttaattttt ttttttttgt ttttattttt 1620
   attgtaatta ttttattaat aagttagggt tgttagtatt tatattaagt ataatttatt 1680
   attgtgtata attagtttta gggtggtaat gagatacggt tttttggtga gaattggaag 1740
   gttagtgatt aatatttaga attttttttt tggatgattg tggattataa tatttttagt 1800
   tgttttgggg atttttttgt ttgtaaaaaa ataaaaaaaa attttagtgg aatatgaagt 1860
   aggttttttg gtgtaaaata gtttttttat gaattgtagt ttattaaaaa gaattgttgt 1920
   tttttagaat tattatttaa tttaggaatt tcgttattga gtgtatattt agaggaatat 1980
   aaattatttt attataaaga tatatgtatg tgaatgttta ttgtagtatt atttataata 2040
   gtaaagatat tgagttaatt taaatattta ttaataatag attggataaa gaaaatgtgt 2100
   tatatatata ttatggaata ttatgtagtt ataaaaaata ttaagattat gttttttgtg 2160
   ggaatatgaa tggaagtgga ggttattatt tttagtaagt taacgtagga atagaaaatt 2220
   taatattgta tgtttttatt tagaagaggg agttaaatga tgagaattta tgaatgtaaa 2280
   gaagggaata atagatagta gggtttattt gagggtggag ggtgagcgga aggagtggag 2340
   tagaaaagat aattattggg tattgggttt tatatttggg tgatgaaata atttgtataa 2400
   taaatttttg tgatacgagt ttgtttatgt aataaatttg tatatgtagt tttaaattta 2460
   aaagttaaaa gaaaaaagaa ttattgtttt ttataaacgt gttaaatttt agtattaggg 2520
   ttttttttat tattaagatt ttgaaagtta atatgttttt aattgatttt tttttttttg 2580
   taatttggtt tatttatggt tttttttatt ttggtttgtg gtaattttta ttttttggga 2640
   taaaaatttg ggtttttttt ttttatattt tatatttaat ttgttaggaa attttgtggg 2700
   tttggatttg aaaatatatt taaaatttga ttattatttg ttatagacgt tgttatcgtt 2760
   tttgagtaag ttatttttat tttttgtttg gtttattata agagtttttt attgtttttt 2820
   gttgattttt agttttttat agtagttttt aaaatatgtt agttgatatt atttttttgt 2880
   ttaaaatttt tttataattt tttattttat ttagagtaaa ggtaaagttt ttagaatagt 2940
   ttataaggtt ttttttgttt ggttttttat attattttat tttatttttt attatttttt 3000
   ttttatcgtt tgttttagtt attttagttt ttttgatggt tttagaattt gttaggtagg 3060
   tttttatatt aggttttagc gttggttgtt ttttttgttt ggaaattttt ttttatattt 3120
   gtggagttta tatttttatt ttttttaaga ttgtgtttat atatcgtttt tttagtgagg 3180
   tttattttga ttattatatt taaagttgta tttgtatatt ttattttttt tttttatttt 3240
   attttatttt ttttataata attatatttg ttttttgaat tttttgttta ttgatgtatt 3300
   tttaggattt agaagaatat ttattaagtg gtaggtattt aataaatatt tattgaatga 3360
   gggatgaatt tattttgttt aatttagtat aattttttta agaaaataag attataaatt 3420
   agattttttg agaacgttaa attttttttt ttttttttag atggagtttt attttcgttg 3480
   tttaggttga agtgtagtgg ggtgattttt gtttattgta attttcgttt tttaggttta 3540
   agagattttt ttgttttagt ttttcgagta gttggtatta taggtatgcg ttattatgtt 3600
   cggttaattt ttatattttt agtagagata agggttttat tacgtggtta ggttggtttc 3660
   gaatttttga ttttaggtga tttgttcgtt tcggtttttt aaagtgttgg gattataggc 3720
   gtgagttatt gcgttcgggc gagaacgtta aattttagtt gaattaatcg taatttagta 3780
   tttttagata tagtatttgg gatggtgtaa tagggagttc gatggagatt ttggtaaggg 3840
   atatggtggg ttaagaaaga attgtttgtt tttgatatta gataaattta ggggatttat 3900
   tttattttag gttttagaat tatgtataaa ggtagagaaa agtgattttg agtgaatttg 3960
   tattgataat ttattagaat aaagtttttt ggagttaggg ttttcgtagt atgtgttttt 4020
   ttggaaggtg gaataaaagt ttgaattcgt tttgttattt tttggaaggt agttttgtta 4080
   tttttttagg ttttggtttt tttttttgga aaaggatggt atttatcggt ttttcgtatc 4140
   ggaggttgtt tgtagggtgt ttttagtatt ttttagggaa gttttttaga atgttgtttg 4200
   atatattttt ataaaattta tttttattgt ggtaaaatat atataatata aaagttattt 4260
   ttttatttag tttgcggtgt acgtttgtta attttatagt aagtaatata tgagttttaa 4320
   attagaattt ggataacggt aaatgagatg aaaggcgaat aattcgaaag tcggattatt 4380
   gaggagtttt gggaacgcgt cgcgagttga agttatacgg ggaattttat tttgtcggga 4440
   tagggtcggt tttggtcgtt tttagggagt cgggtagttt tcgcggcgtg gttcgggttt 4500
   tttttgaagt tcgttcgttt tttttcgttt ggtttagcgg cgcggtcgtc ggaggatttt 4560
   gtttacgcgt ttaacgtcgg gcgttttgtt ttgaaggtaa agggtttttt gtttcgtacg 4620
   attcggagtc gtcgaggagg tgacggcggt agttttgggg cggttcggtg ttatttatta 4680
   ttattttgtt tttttgaggc gcggtttttt aattgtttcg gtttcgttag gaggcgcgcg 4740
   ttttagtcgt ttcgtcgcgc ggtttcgatc gtattttagt tcggggcgaa gaaagaaaag 4800
   gcggtcggtt ttcgtcgttt ttcgtttatt tagtcgggtt cgcgtaggcg cgcgtagttt 4860
   tcgttttcgc ggtcgttttt tttttaggtc gtcgggcgcg tcgcgggcgg ggtcggcgcg 4920
   gggggcggga gtcggtacgg gttcggttgg ggttcgattc gggattagtt ggtttcggag 4980
   cggaggaggg agtttcgatc gttacgagcg tcgaagagat aaagtcgcgt tagggggttc 5040
   ggtcggggcg ggggagttcg gggtttgttg gtgttttagt tcgcgcggag ggtttttcgg 5100
   attcgcgcgt cgtcgttgtc gtcgtcgtcg tttcgtaata ggttcgggcg gtttcgtttt 5160
   tcgttttttt ttttcgtatt cgcgattttt cggggtattt tagttcggtc ggggtcgtag 5220
   tttggttatt cgtttttatg cggttcgggt ttaagatgat gtcggtaagt gggcgagcgg 5280
   ttagggcgtc gggttcggaa gtgtgcgggg tcgcgtgtcg ggggttggtt cgcgttttcg 5340
   ggcgcgtcgg ggaaggagtt tgggggtcgt agttcgcgcg gaggggtcgg ggtttttcga 5400
   gtttcgagcg ggttttagga aggggaggtt ttattttttg ggttttgcgt ttatagtcgt 5460
   cgtgggggcg cggcgagatt ttaatttcga ggcgggtttt tttagttgtt tcggagttag 5520
   gcgagtcgtt cgcgagttta gtagttattt aaatttttcg ttttttattg ggttttgatt 5580
   tttttgtagt ttttgaaggt taagaatacg ggaatcgggg attttgtttt ttgttgtgtt    5640
   tttttatttt ttattggagt gaaatatacg ttattggaac gggtattttt gttttgtttt    5700
   taattgaatt tcgattttga tatttatagt agtagttaag ttatttttat ttttttgtgg     5760
   aaaggggttt tggtaaatgt aatttcggaa gttttagatt gtaggaaagc gtacgtgttt    5820
   ggtttttttc gttttttata gtttttagta agattttcgg agaggattag tgtttttttt     5880
   aggagtttcg taggtttttg atacggttat taggttatag atgaatagta ggtttgagat    5940
   attttagaaa tgtttttgtt atttagcgat cgcggaatgg aggtttgcgg tgtgttgcgg    6000
   tggtttttgt agattttagt agcgagtttt ttcgtggaag ttgtatttgt atttttttta     6060
   ttgaagtggg gatattttgt aggtatttta tgtttgtggt tttatttgtt tagataaaga    6120
   aataaaaagt ttttattttt atgaagtttg tggaatggag tgatgataaa gtttgtagtt    6180
   atatatatag agtataatat gttgtttttt ggttttttgt ttgtgttatg gagggcgttt     6240
   taaattttta ttgggtcgat ttaa                                           6264
<210> 68
   <211> 6264
   <212> DNA
   <213> Artificial Sequence
<220>
   <223> chemically treated genomic DNA (Homo sapiens)
<400> 68
   ttaagtcgat ttagtgagag tttgggacgt tttttatgat ataagtaggg ggttagggaa     60
   tagtatatta tattttgtat gtatgattgt aaattttgtt attattttat tttatagatt    120
   ttataaaagt aaaagttttt tgtttttttg tttgaataaa tggagttata agtataggat    180
   gtttgtaaga tgtttttatt ttagtgggag aggtgtaaat ataattttta cgaagaaatt    240
   cgttattgaa gtttgtagaa gttatcgtaa tatatcgtaa atttttattt cgcgatcgtt    300
   gaatgataga gatatttttg aaatgtttta aatttgttgt ttatttgtgg tttggtggtc    360
   gtgttaaaag tttgcggagt ttttaaagaa ggtattaatt tttttcggag gttttgttaa    420
   aagttgtggg aaacgaaggg agttaggtac gtacgttttt ttgtaattta aaattttcga    480
   gattgtattt attaaaattt ttttttataa agaggtaagg gtgatttagt tgttattgtg    540
   gatattaaga tcgaaattta attaaaaata aagtaaaggt attcgtttta gtaacgtatg    600
   ttttatttta gtaaaaagta aaaagatata gtaaagaata aggttttcgg ttttcgtatt    660
   tttgattttt aagaattgta aaaaagttaa gatttagtga aagacgaaaa gtttgagtgg    720
   ttgttgggtt cgcgggcggt tcgtttgatt tcggagtagt tggaggaatt cgtttcgagg    780
   ttagggtttc gtcgcgtttt tacgacggtt gtgggcgtag agtttagagg atggagtttt    840
   ttttttttga aattcgttcg aagttcggaa agtttcggtt ttttcgcgcg ggttgcggtt    900
   tttaggtttt tttttcgacg cgttcgaggg cgcggattag ttttcggtac gcgatttcgt    960
   atattttcgg gttcgacgtt ttggtcgttc gtttatttat cggtattatt ttggattcga   1020
   atcgtatgga agcgggtggt tagattgcgg tttcggtcga gttgaggtgt ttcggagggt   1080
   cgcggatgcg ggggagggga gcggagagcg aggtcgttcg gatttgttgc gaggcggcgg   1140
   cggcggtagc ggcggcgcgc gggttcgaag ggtttttcgc gcgggttggg gtattaataa   1200
   gtttcgggtt ttttcgtttc ggtcgggttt tttgacgcgg ttttgttttt tcgacgttcg   1260
   tgacggtcgg ggtttttttt ttcgtttcga aattaattaa tttcgggtcg ggttttagtc   1320
   gagttcgtgt cggttttcgt ttttcgcgtc gatttcgttc gcggcgcgtt cggcggtttg   1380
   ggggaggggc ggtcgcggga acgggagttg cgcgcgtttg cgcgggttcg gttgggtaga   1440
   cggggagcgg cgggagtcgg tcgttttttt ttttttcgtt tcggattgaa gtgcggtcgg   1500
   ggtcgcgcgg cggagcggtt ggagcgcgcg ttttttagcg gagtcggggt aattggaagg   1560
   tcgcgtttta ggaaaatagg atggtagtga atggtatcga gtcgttttag ggttgtcgtc   1620
   gttatttttt cggcggtttc gagtcgtgcg aggtagggga ttttttgttt ttagaatagg   1680
   gcgttcgacg ttgggcgcgt ggatagagtt tttcggcggt cgcgtcgttg ggttaggcgg   1740
   agagaggcgg acggatttta ggggaggttc gggttacgtc gcggaaatta ttcgattttt   1800
   tggaggcggt taggatcgat tttgtttcga taaaatggag tttttcgtgt ggttttagtt   1860
   cgcggcgcgt ttttagagtt ttttagtgat tcggttttcg gattgttcgt tttttatttt   1920
   atttgtcgtt gtttaaattt taatttaaaa tttatgtgtt atttgttgta aggttaataa   1980
   acgtatatcg taaattggat aaagggataa tttttatgtt gtgtatgttt tattataata   2040
   aaaataaatt ttgtaaaaat gtgttaggta gtattttgag gagttttttt ggggggtgtt   2100
   gggaatattt tgtaaatagt tttcgatacg gggagtcggt ggatattatt tttttttagg   2160
   gagggaggtt aaggtttagg gaaatggtag ggttgttttt taaagagtag tagagcgggt   2220
   ttaggttttt attttatttt ttagagagat atatattgcg ggaattttaa ttttaggaag   2280
   ttttgtttta gtaagttgtt agtgtaagtt tatttagaat tatttttttt tgtttttata   2340
   tatgatttta gaatttgaag tggggtaaat tttttgggtt tatttagtat taagaataga   2400
   taattttttt ttagtttatt atatttttta ttagaatttt tatcgaattt tttattgtat   2460
   tattttagat attgtatttg gaaatattaa attacggtta atttaattag aatttaacgt   2520
   tttcgttcgg gcgtagtggt ttacgtttgt aattttagta ttttgggagg tcgaggcggg   2580
   tagattattt gaggttagga gttcgagatt agtttggtta cgtggtgaaa tttttgtttt   2640
   tattaaaaat ataaaaatta gtcgggtatg gtggcgtatg tttgtaatgt tagttattcg   2700
   ggaggttgag gtaggagaat tttttgaatt tgggaagcgg aggttgtaat gagtagagat   2760
   tattttattg tattttagtt tgggtaacga gagtgaaatt ttatttgaaa aaaaaaaaaa   2820
   gaatttaacg tttttaaaaa atttggttta tagttttgtt tttttaagag ggttatattg   2880
   agttaagtaa aataaattta ttttttattt agtaaatatt tattaaatat ttgttatttg   2940
   gtagatattt ttttaagttt tagggatata ttaatgaata aaaagtttaa aagataagtg   3000
   tagttattgt gaaaaaaata gaataaggta agagaagggg ataaggtgta taggtgtaat   3060
   tttaaatatg gtggttaagg taagttttat tgaaaaggcg gtgtgtgagt atagttttga   3120
   aggaaatgag ggtgtgaatt ttataaatat gaaggaagat ttttaggtag agggaatagt   3180
   taacgttaag gtttgatatg ggagtttgtt tggtaaattt tgggattatt aaggaggtta   3240
   agatagttgg agtagacgat gaggggagag tagtaggaga tgagatggag tgatatgaag   3300
   ggttagataa ggagggtttt gtaggttatt ttaaggattt tatttttgtt ttgagtgaaa   3360
   tggggagtta tggaaaggtt ttgaatagaa gagtgatgtt agttgatata ttttaagagt   3420
   tgttgtggaa aattgaaagt taatagagaa tagtaagaaa tttttgtgat aagttaagta   3480
   aaagatggaa gtggtttgtt taaaggcgat aatagcgttt atggtaaata gtggttaggt   3540
   tttggatata tttttaagtt taaatttata gaattttttg atagattaga tgtggaatat   3600
   gagagaaaga agtttaaatt tttgttttaa aagatggaag ttgttataaa ttaaaatgga   3660
   gaagattatg agtggattaa gttatagaaa agaggagatt aattgggaat atgttaattt   3720
   ttagaatttt aataataaaa gaaattttaa tattgaaatt tagtacgttt gtgggaagta   3780
   ataatttttt ttttttttaa tttttaggtt tggggttata tatgtaggtt tgttatatag   3840
   gtaaattcgt gttatagggg tttgttgtat agattatttt attatttagg tatgaagttt   3900
   agtatttaat agttattttt tttgttttat ttttttcgtt tattttttat ttttaagtag   3960
   gttttattgt ttgttgtttt tttttttgta tttatgagtt tttattattt agtttttttt   4020
   tttaagtgag aatatgtagt attgggtttt ttgtttttac gttagtttgt taaggataat   4080
   agtttttatt tttatttatg tttttataaa agatatgatt ttgatatttt ttatggttgt   4140
   atagtatttt atggtgtata tgtagtatat tttttttatt tagtttgtta ttgatggata   4200
   tttaggttga tttaatgttt ttgttattgt gaatagtgtt ataatgaata tttatatgta   4260
   tgtgttttta tggtagaatg atttatattt ttttgggtat gtatttaata acgggatttt   4320
   tgggttgaat ggtagttttg ggaagtaata attttttttg gtaaattgta gtttataaag   4380
   ggattatttt atattaaaaa atttatttta tattttattg gggttttttt ttattttttt   4440
   gtagataaag aagtttttaa aatagttgaa gatattatga tttatagtta tttaggaaaa   4500
   gaattttgga tattggttat taatttttta gtttttatta aaaaatcgtg ttttattatt   4560
   attttaaaat tggttgtata taatagtaaa ttatatttag tatgagtgtt ggtagtttta   4620
   gtttattaat aaaataatta taatagaaat ggaagtaagg agagaagaat tggaataaga   4680
   tagtttagtt ttatttcgtt attgtttgtt tatataaatg ttttgtttaa gggaagggag   4740
   gagaaaaggg atagtaattg gtaatatgtt ttgaaattta tttttagtta attgagaatt   4800
   tttagttgtt aaatagtagg tatgttgagg attttttttt ttttaattat agaagcgtga   4860
   aattaaagaa tttttgtttg tgttttggtt ttattttttt agattttgta ttatttaaat   4920
   atggttttta gaagggttgt tttttattaa gttttaattt tttttcgttt ttatttttat   4980
   ttgtttattt ttttaagagt ttttgtttgg ttttttttat tttataattt tttatttaat   5040
   tttaagaaag ataagttaaa atgttatatt ttgagcgggg aggagttatt tttttttttg   5100
   gtttttattt tattttttgg tatagtataa ttaattattt atttggtttg aatttaaatg   5160
   tttaagtttt taattttttt tttgtgttat tcgtttttat tttttataga tgttttaaat   5220
   tttgagtttt tttttttttt tagaagatga atttttattt tatttagtta atgaaataga   5280
   gattattgag attgtatttt tgtaatattt atttttttta tttttaattt atttgggttt   5340
   ttttttattt tttttttatt ttttttgttt atttttttta ttaatattcg tgtggttgta   5400
   ttttgttagg ttttgtttta gattttagag gtaatagaga agagatttgt ttttatggag   5460
   tttatatttt agttgggagg agttagataa taaatatatg aataaggtaa taataaatgt   5520
   tacgaaaaaa atagtaatta ggtaaattta agagaatagt atttgtttta gataggttgg   5580
   ttagagaaag tttttgagga atgaggggag ggagggaatt agattttggg tagtgggaat   5640
   agtaaggata aagagtttga ggtagttttg tgtttggtat tttaaagata tggtaaaaag   5700
   attagtgtgt ttagggtaga gtgaataagg aggggagtgg taagagaggt ggaagatgta   5760
   gttagtgttt aggttatgta gaattattta atattttatt aattattttt ttttttgaaa   5820
   ttagagatat tttattttta tagttttttt aatttacgga tttatttagt tattttttat   5880
   atttttttta agttagtagt aatttgaaag aaaaatgggg tagaagtttg tgtattaagt   5940
   ttttttgtat ttatttatat tttttttttt gttttaaaaa tttttttttt tttttatttt   6000
   attttatatg gtaaattttt attcgttttt taagttttta tttaaatgtt taaatgtttt   6060
   tttttttttt taagaaataa ggttttatat tgttattgag gttggagtgt agtggtatga   6120
   ttatagttta ttgtaatttt aaattgttga gattaagtta tttttttatt ttagtttttt   6180
   aagtagttgt gattataagt atgtattatt atgtttggtt aatttttaaa tgttttgtag   6240
   agatggtgtt ttattgtgtt gttt                                          6264
<210> 69
   <211> 9106
   <212> DNA
   <213> Artificial Sequence
<220>
   <223> chemically treated genomic DNA (Homo sapiens)
<400> 69
   tttatgttat tttttcgatt tagttttttg agtagttggg attataggcg tttgttatta     60
   tattcggttt tttttttttt ttttttagta gagatggggt tttatcgtgt tagttaggat    120
   ggttttaatt ttttgatttc gtgatttgtt ggttttaatt ttttgatttc gtgatttgtt    180
   cgttttagtt ttttaaagtg ttgggattat aggtttgagt tatcgtgttt ggttttattg    240
   atttaatttt ttattggatt taattttttt atttttttta gtttgtttag atttattttt    300
   ttttgagtta gttttggtta tttgtgtata gttgtttata gtatttattt aaaatgtttt    360
   tttttttttt ttttgtaagg tttatagtga tgtttttatt tttatgtttg attttagtta    420
   tttgtatttt tattttttat tttttagttt agttaaagat ttgttagtgt tgtttatttt    480
   tttaaaattt ttattttagt tattgtattt tttagtttta gaattttgat ttggtttatt    540
   ttttataatt tgtttttttg ttgatatttt gtttatatat ttattgttta tttggttttt    600
   tttagttttt tgtttatggt tttttttagt tagttaagta tatttaagat tatttaaata    660
   aacgtttaag taaattttta aatatttaaa taaaagtttt tgtttagtaa gtttaatgtt    720
   taggtttttt tagggatggt ttttattaat tttttttttt tgtgaatggg ttgtattttt    780
   tttttttttt ggtatgtttt ttattttatt tatttattta ttttttggtt aaaaattggg    840
   tatttcgggt ttggtatggt ggtttacgtt tgtaatttta gtattttggg atgtcgaggt    900
   gggtgggtta taaggttagg tattcgagat tattttggtt aatataatga aattttgttt    960
   ttattaaaaa tataaaaaat tagttgggtg tggtggtggg tgtttgtaat tttagtaatt   1020
   cggaaggttg aggtaggaga atcgtttgaa tttgggaggt ggaggttgta ggttgttgag   1080
   atcgcgttat tgtattttag ttttggtgat agtgggagtt tttgttttaa aaaaaaaaaa   1140
   aaaaataaaa attgggtatt ttaatttgaa aaagttgatt gaaagaaaaa aaaatttggg   1200
   tattttgaat attataatgt ggtaattttg gaaattagat tttgttaatg tttattaagg   1260
   gttgtagttt tttgtttgtt tagattatgt tttagttttt ttggttatta gtttttattt   1320
   tgaagttagt taggggtttt tagttattag ttattttatt agtatattaa taatatttat   1380
   tatatttgag attttaaagg ttttagaagt ttgtgttagg aacggggata aagattaaat   1440
   atttattttt attatattat aattttttaa tttatttatt gagttattat gttttatttt   1500
   agggtatatg tagttttttt atattagggg aaatttagtt tattaagtgt aatattgaaa   1560
   gttaaataat aataatataa taataattaa ttagggatgg gcgcggtggt ttatgtttgt   1620
   aatttaattt tagtatttta ggagatcgaa gcgggagggt tattttaggt taggaattcg   1680
   agattatttt ggttaatatg gtaaaaattt tttttttatt aaaattataa aaattagttg   1740
   ggtagggtgg tatatatttg taattttagt taatttggga ggttgaggta agagaattat   1800
   ttgaatttgg gaggtagagg ttgtagtgat ttgagatcgt gttattgtat tttagtttgg   1860
   gcgaaaaagt aagattttgt tttaaaataa taataataat aaattaggtt ggggagttat   1920
   tttttaggaa ttttgtagta agggtttata tgtattagtt agagttggat tttgtgattt   1980
   ttaggtgttt tttttttttt aagaatcgat tatttttata attttgttaa atgtaattaa   2040
   ttttttaaat ttattaaaga ttattatttg gattattatt tttttagtta aaatgttggt   2100
   ttgttttagt tttatttagg gttaatttgt atttaaatag ttttttgaaa atatttggtt   2160
   ttttttattt atttatggtt aattgatttt tgataagaag ttaagataat ttaataggaa   2220
   aaggataatt tttttaacga atggtgttgg gataattaga agtttatatg taaaataatg   2280
   aatttggatt ttaattttat attatatata aaaattattt taaaatggat taataattta   2340
   aatataggag ttaaaattat aaaattttta gaagaaaata taggagtaaa tttttataat   2400
   tttggatttg ataatgatat tttatatagg atattaaatg tataaataat aaaagaaaaa   2460
   atagttaagt tgaagtttat taaaattaaa gttttatgtg tatgaaagga tattagaaag   2520
   tgaaaaagat aattatagaa tgggagagaa atatttgtaa agtatatatt tgataagggt   2580
   ttagtgttta gaatatataa agaattttta taatttaata ataaaaagat aatttaattt   2640
   taaaatgggt aaaggatttg aataggtatg tttttaaaga agatatatag atggttaata   2700
   aatatatgaa aaggagaatg tttaatagta ttagttatta ggaaaatgta aattaaaatt   2760
   agaatgaggt atcgttgtat atttattagg ataataataa aatggaaaat aataagtgtt   2820
   ggtgaggatg tggagaaatt gggattttgt atattgttgg taggaatata aaatggttta   2880
   gttatcgtgg aaattatttg gggtttttta aatagttaaa tattgaaggt tgggtacggt   2940
   ggtttatgtt tataatttag tattgtggga ggttaaggta ggtagataat ttgagtttag   3000
   gattaagatt agtttggtta atatggggaa attttatttt tattaaaaat ataaaaatta   3060
   gttagatgtg gtggtatata tttgtaattt tagttattta ggaggtcgag gtaagataat   3120
   tgttcgaatt tgggaggtag aggttgtagt gagttaagat tgcggttatt gtattttagg   3180
   ttgggtaata gagtaaggtt ttgttttaaa aaaaaaaaaa aattgaatta ttatgcgatt   3240
   taataattta atttttatgt atgtattttt tttaaataaa aatgggtttt taaataaata   3300
   tatgtatatg tatgtttatg gtagtattat ttataaagtt aaaagatgta aatagtttta   3360
   atgtttatag atgaataaat tgtggtatat atgatatata tatatatacg tatatatata   3420
   tatatatata tatataaata ttatttagtt ataaaaagga ataaagtttg ttatatgtta   3480
   tttgaggatg aatttcgaaa atatgttaag tgaaagatat aaaagtttat atattgtgat   3540
   ttcgtttata tgaagtattt aaagtaagta aatatagaga tagaagtaga ttggtaattg   3600
   ttaggggttg ggggggaaga gggtattggg aagaaatttt ttaatgggta tggggttttt   3660
   ttttcggaat gaggaaaatg ttttggaatt agatagaggt ggtggttgta tagtattgtg   3720
   aatttattaa atgttattga aatgtttatt ttaaaatggt aaattttatg ttatgtgaat   3780
   tttattttaa gttaaaaaaa aaaaagtaaa attatttaga taacgttaaa ttattgataa   3840
   ttttaattta ttttatagat tttaaatttt ggaattataa ttttttaggt taatttttag   3900
   gttaggtagt aatttatttt tgtttaattt tatgtagagt ttttggtggt tttgaagcgt   3960
   tttagggaat agattaggaa tatttttatg gtgttagttt atattgggtt attagtgttt   4020
   ttttaatgtg agaataatcg ggaggaggag gggatgtgga tttaaaatta taagaaagag   4080
   tgttttcgaa gtttatgttt tatcgtttta cgttgttgtt aggttcgtag gaagatgata    4140
   ggttcggttt ttattttttt taaggtcgtc gtttagtttc gacgtcggga tgattttgtt    4200
   atttacgcgg cgtgaaggtt atttttttcg cgcgttcggg attttaggtg gggttttagt     4260
   ggacgaggga acgcggcgtc gtttatcggg gtgtgtttgg gcgggcgtgg ggcggggttt    4320
   gggtcggcgt cgggttcggt cgggcgttta gttagttgcg taaatttcgt tggagcgcgg    4380
   cggtagagta ggtgagcggg cggtgtcggg gggtgtttag gttagggttt tgtcgtttgc    4440
   ggcgttgagg gttcggggtg gggttgcgtt ttgagggttt tgttttgttt ttcgtacgtt    4500
   tttggttacg gttttttttt cggttgtggg tttgcggttt ttgcgtgcgt agcgtttttg     4560
   gtttttgcgg ttagcgcggg ggcggagaga ggagagtgtt cggtaggcgg cggttgggtc    4620
   ggttcggaat tgggtcgtgg aaggatcgcg gggagcggtt tttaggtttt cggttttatt    4680
   gcgtttttat ttttttgcgt tcgtttgtcg tcgagtttcg gttgggggtg ggcgcggcgc    4740
   gagcggttaa agggtcggtg tatttaaagg agcggtgtac gtgggttttt gaggcgtgta    4800
   gtaggcgggg gcgttttgtt tttttttttt ttcgtcggag attttcgttg cgtcgagttt     4860
   attcggtttt tagtatcggg ttttgggtat gtttttttcg ggaaggaggc gcgcgggggt     4920
   tttgttcgta cgtgaggggt agggtcgtag gtttaagttt agagtcggtt tttgttagta    4980
   gcggtgtttg gttgttttat taggtatttt tagtagtgag gagatagtta gaagtaagtt    5040
   tttggagttg aaggaatttg agatagaagt tagttttttt tttgaatttt attgatgaag     5100
   aaattgaggt tatagagtta aagtgatttt ttttaaggtc gtttaggtac gatatagtag    5160
   agttaggttt cgattttagt gttttggttt ttagatttgt tgtttatttt ttcgagtaga    5220
   ttttattttt gtttattgtt ttaataagta ttttttttga aaggtatgaa cggtgttgag     5280
   tgaagtaatt gtatttttat ttataaatgg agaatttgag agtattttat agagacgatt    5340
   gtagattaat ttaatttaga agcgatagtt tggggttgtt aaggttgttt acgaagtaat    5400
   ttgattagga tcgattttag tttttagtaa ggaagttttt gatgtttttg tagttaattt     5460
   tgtagatatt tgagttttta aggtttttag ttaagatttt tggcggtaat tggagtttcg    5520
   ggataagttg ttttaggtgt gtgagtttta ggtttttttt ttttgaatgt ggttgtgggt    5580
   agtcggtgat tggcgtaggt gtagaagggg tttggttttt ggttttattt tagagttgcg    5640
   tttttacgac gtttacgttg agttttgggt tttagggtag agattggagt gagggtttgg    5700
   gggtatgttg ttttgaagtg ggatggatgt attaggtttt tggggaaaat tttgtatttt    5760
   ttggtgttga agtgtttatg tgttaagttt tgagtttagt atgtttatat gtggggagtg    5820
   agtggtttgt ttttgtttat ttgaaagagt agtaaggagg aggaggagta agggttaggg    5880
   gttgttgttg gggtgtttgg agttgtggtg tataatgtta tatttgtttt tttttcgtag    5940
   ttgtttatcg tttttttaag gggggtttgt tttttgttta ttttggtttt tttttgttat     6000
   cgatgttaat aatgatatat atttcgttta tgagttggtt ataataaaaa gttattttgt    6060
   atagaatatt agaatttaag attttaagaa ttttaatgat attgaaatag tttattatta    6120
   tttttttata gaagaggaaa taagtttagg gagttaagta gttagtttag ttatgtggtt     6180
   ttagtgtttt aagtattagg atttgaatat agttggttat attgttttta ttttttgagt      6240
   ttttgcgtag gaggtttttg tatttaattt ttattcgtgt ttttttatta ttgttgggaa     6300
   agttttgtgg agtttttatg agtaattttt tgataaataa ataaaatttt tttaaagaaa    6360
   ttaaagtagt gtgtgtaggt tatatgtagt gtgtttaatg aaaatatttt tggcgggttt    6420
   ttagttgttg ttttgatttt cggatattgt ttagttgggg attgataaga tagtaaatat    6480
   ttttgtaagt atttttattt gttttatttt tagttgttat agttgcggaa aggcgggggt    6540
   gaggttgaga ggtttcgaga ggaatatttt ttattgggtt ttaattttgg agatgggatg    6600
   attattatgt taatgtttgg agaaaagaat gattttaggt tgggtgttgt ggtttatgtt    6660
   tgtaatttta gtattttggg aggtcgaggt gggtggatta tttgaggtcg gaagtttgag    6720
   attagtttga ttaatatgga gaaattttat ttttattaaa aatataaaat tagtcgggcg    6780
   tggtggtata tgtttgtaat tttagttatt taggaggttg aggtaggaga atcgtttgaa    6840
   tttaggaggc ggaggttgta gtgagtcgag atcgggttat ggtattttag tttgggtaat    6900
   aagagtaaaa ttttatttta aaaaaaaaaa aatggtttta tattagtttt taggaaagat     6960
   tagatgttag tgagggagat tattttttga gagttttttt attgagtggg agaatgggtt     7020
   gtttgtttat ttttgtgaaa attttagaac gggaagaata atttaaaggg tgtttattat    7080
   tttgttgtat tttaattaga aatttattgg atttttttta aaataaaagt aatttatgtt    7140
   tattttagaa aattagggaa aaaaaatttt ttttgagatg gagttttatt tttgttgttt    7200
   aggttggagt gcgatggtat gattttagtt aattgtaatt tttgtttgtc gggttttagc    7260
   gatttttttg ttttagtttt ttgggtagtt gggattatag gtgtttgtta ttatttttag    7320
   ttaatttttg tatttttatt agaggcgggg ttttattatg ttggttaggt tggttttaaa     7380
   tttttgattt taggtgattc gttaattttg gttttttaaa atgttggtat tataagtata      7440
   agttattgcg ttgggttgag ataattattt ttaatatgta ttttttttta tattgtttat    7500
   atatgtatat ttatttatta aaataataga gggagggaat cgtagtgaag tttgtgtatt    7560
   ttgttttttt tgttatgttt ttagaatttt ttttttattg agtatttatg gaaaagtaga      7620
   tttgatggtt gtgtagagta tttgaattat ttattttatt agttttatag taggatattt    7680
   ttttattttt ttttatgttt tagagaagta gtgttttttg ttttttttat gtttatatgt     7740
   gggtattttt tattatggag ttaaatttat ttgggtaagt agtagaggga gagtagagtg    7800
   agttttgggg gtaggagaga gatttgagag ttttgaggtg atagatgagt tggtgagtga    7860
   gtgattaggg agtatttttt gatatatatt tgttcgtggt gaaggtatgt gttttgtgag    7920
   tgtgttttta gaaagtttgt gtagtgtgtg gtgggtttgt ttgtgtgatt aaattttggt    7980
   tattgggtac gtgattttta taagtgttga ttgggttgag aagagttttt tgatgggtag    8040
   tttggagatt tgagttgtaa ttgtggtttt tggttatggg atattaattg attatttttg     8100
   tttttttagg ttttagttgt ttttaattat aatataggga gttgattagg tggcgttgat    8160
   ggtttttacg gtttttgtta gttttgatat tgttttatgg atatgttttt ttatttgata    8220
   atatgtttac gtggttatag tgtttggggt tgggtcggga atggaaattt gatttttggg     8280
   gtttggtttt tgaagttagt ttatgtgttt ggtggtttag tagattcgta attttttaag     8340
   aggtatattt ataggttatc gtgttttttt ttattgtgtt ttttttttat tttatttttt     8400
   ttataattac gatttattga atatttattg tgtgttggat attttatagg ttatttttag     8460
   gttttacgat aattttgtaa ggtatgtttg ttttgttttt tatagtagag gaaatgagtt     8520
   gtgttagatt agattgtttg aggttttttg gttagggagt atgtggttta aattatatag     8580
   gtaggcgatt tgaattttgt tagtttttaa agtttttgtt tttgatcgtt gatttgttgt     8640
   tgtttgttta aaaataaatg tgtttttgga gtttatttta gaggggcgtg ttaggggttt     8700
   ttttttttat ttttttataa attatttttt tttttggttg ttttaggaaa tgagagaatt     8760
   ttgtttgggt tttaggtatt tttgagtggg atagggttgt tagaggtaag tttagagttt     8820
   ggtttaaaat ttaggaggtt ttattagagg gtttttgggg tttgtggttc gggagggtgg     8880
   tagggtagta ttttattttt ttttgagatt taggttttag ttttggttta ggttagggag     8940
   aattattttt aagtggtatg tgttattata tgagttgaga tggatggtta gttggattaa     9000
   atatatagtc gggtatttag ggttaggggg aggaaggtga gtagggaagt tgtgggtaat     9060
   tgtttgggta ttatttgatt ttagtaaatt tttttttgtt ttaagt                    9106
<210> 70
   <211> 9106
   <212> DNA
   <213> Artificial Sequence
<220>
   <223> chemically treated genomic DNA (Homo sapiens)
<400> 70
   gtttaaaata aggaagagtt tgttaaggtt aggtgatatt tagataattg tttatagttt      60
   ttttgtttat tttttttttt ttggttttgg gtattcgatt atgtatttgg tttagttgat     120
   tatttatttt agtttatata gtaatatata ttatttgggg atggtttttt ttggtttaag     180
   ttagagttgg ggtttgagtt ttaaagggaa gtgaggtatt gttttattat tttttcggat     240
   tataggtttt aggggttttt tgatggggtt ttttgaattt tgggttaggt tttagattta     300
   tttttaatag ttttgtttta tttagaagtg tttggggttt aggtagagtt tttttatttt     360
   ttgaagtagt tagggaaaag ggtggtttgt ggggaagtgg gagagggagt ttttagtacg     420
   tttttttgga gtaggtttta gaaatatatt tatttttaaa taagtagtag taagttagcg     480
   gttaaaagta gaggttttgg agattgatag ggtttagatc gtttgtttat gtgatttgaa     540
   ttatatattt tttggttaag aggttttaga tagtttaatt tgatatagtt tatttttttt     600
   gttgtaaaaa gtagaatagg tatattttgt aagattatcg taaaatttag agataatttg     660
   taaagtgttt agtatatagt agatgtttaa taagtcgtag ttataaagaa gatgaaatgg     720
   aggagggata tagtgagaaa ggatacggta gtttatggat gtgttttttg gaaagttacg     780
   gatttgttga attattagat atatgaattg gttttaaagg ttaggtttta gagattaagt     840
   ttttattttc ggtttagttt taggtattat ggttacgtaa atatattatt aaatgaaagg     900
   atatatttat aggataatgt tagagttggt aaggatcgta aaggttatta acgttattta     960
   gttagttttt tgtattataa ttaaggataa ttgaagttta gaggggtaaa agtaattagt    1020
   taatgtttta tggttaaaag ttatagttat aatttaagtt tttaaattgt ttattaagga    1080
   gtttttttta gtttagttag tatttgtgag ggttacgtat ttagtggtta gggtttggtt    1140
   atataggtag gtttattata tattatatag gttttttggg agtatattta taagatatat    1200
   gtttttatta cgggtaggta tgtgttaaga aatgtttttt aattatttat ttattagttt    1260
   atttgttatt ttaaaatttt taagtttttt ttttgttttt agggtttatt ttgttttttt    1320
   tttgttattt gtttaggtag gtttggtttt atggtgggga gtgtttatat atgggtatgg    1380
   ggaggataga gggtattgtt tttttggggt ataagggagg gtgggaaagt gttttgttgt    1440
   gggattggta ggataaataa tttaaatgtt ttatatagtt attaaatttg tttttttatg    1500
   agtatttaat aaaaggaaaa ttttgaaggt ataatagaaa aaataggata tataaatttt    1560
   attacggttt ttttttttta ttgttttaat agataaatat gtatatgtga atagtgtgga    1620
   aggaaatgta tgttaagagt ggttatttta gtttagcgta atggtttatg tttgtaatat    1680
   tagtattttg ggaggttaag gttggcggat tatttgaggt taggagtttg agattagttt    1740
   ggttaatatg gtgaaatttc gtttttaata aaaatataaa aattagttgg gagtggtggt    1800
   aggtatttgt aattttagtt atttaggagg ttgaggtagg agagtcgtta gaattcggta    1860
   agtagaggtt gtagttagtt gagattatat tatcgtattt tagtttgggt aataagagtg    1920
   aaattttatt ttaaaaaaaa tttttttttt ttaatttttt agaataaata tgaattattt    1980
   ttattttaaa aagagtttag taagtttttg gttaaggtat agtagaatgg tggatatttt    2040
   ttgaattgtt tttttcgttt tagaattttt ataaagatga ataagtagtt tattttttta    2100
   tttagtgaag aggtttttaa gaaatgattt tttttattga tatttgattt tttttgagga    2160
   ttgatgtgaa attatttttt tttttttgag atggagtttt gtttttgttg tttaggttgg    2220
   agtgttatgg ttcgatttcg gtttattgta attttcgttt tttgggttta agcgattttt    2280
   ttgttttagt tttttgagta gttgggatta taggtatgtg ttattacgtt cggttaattt    2340
   tgtattttta gtaaagatgg ggttttttta tgttggttag gttggtttta aattttcgat    2400
   tttaggtgat ttatttattt cggtttttta aagtgttggg attatagata tgagttatag    2460
   tatttagttt gaaattattt ttttttttag atattaatat gatggttatt ttatttttag    2520
   gattggagtt tagtggaaaa tgtttttttc ggggtttttt agttttattt tcgttttttc    2580
   gtagttgtgg tagttgggaa tagaataggt gggaatattt gtagaaatat ttgttgtttt 2640
   attagttttt aattaaatag tgttcgaaag ttaaagtaat agttgaaaat tcgttagaga 2700
   tgtttttatt agatatattg tatgtgattt atatatattg ttttggtttt tttaaaaaaa 2760
   ttttgtttgt ttgttaggaa gttgtttatg gggattttat aaaatttttt tagtagtagt 2820
   ggagagatac gggtaggagt tgaatatagg aattttttgc gtaaaaattt aagagataaa 2880
   gatagtgtag ttagttatgt ttaaattttg gtgtttaaag tattgaagtt atataattgg 2940
   attagttgtt taattttttg aatttgtttt tttttttgta aaaaggtaat aataaattat 3000
   tttagtgtta ttgaaatttt taagatttta aattttaata ttttgtataa gatagttttt 3060
   tattatgatt aatttataag cgagatatat gttattatta atatcgataa tagagaaagg 3120
   ttaaagtagg taagaggtaa attttttttg gggggacggt gagtagttac ggaggggaga 3180
   taggtgtgat attatgtatt atagttttag gtattttagt agtagttttt agtttttgtt 3240
   tttttttttt tttgttgttt ttttaaatag ataggaataa gttatttatt ttttatatgt 3300
   gaatatgttg gatttaagat ttggtatatg ggtattttaa tattaaaggg tatagagttt 3360
   tttttaaaaa tttgatatat ttattttatt ttaaagtaat atgtttttaa gtttttattt 3420
   tagtttttgt tttggaattt aaggtttaac gtgggcgtcg tgaggacgta gttttgaggt 3480
   ggggttaaga attaggtttt ttttgtattt gcgttagtta tcggttgttt ataattatat 3540
   ttaggagaga agaatttgag gtttatatat ttgaagtagt ttatttcgag attttaatta 3600
   tcgttaaagg ttttggttga aggttttgga ggtttaggtg tttgtagaat tggttataga 3660
   ggtattagag gtttttttat tggaagttgg ggtcggtttt aattaagtta tttcgtagat 3720
   agttttggta attttaggtt gtcgtttttg agttaagtta gtttataatc gtttttatgg 3780
   aatgttttta ggttttttat ttgtaaatag ggatgtagtt attttattta atatcgttta 3840
   tattttttaa agggaatatt tattaaggta ataaataggg gtgaggtttg ttcggaggga 3900
   tggatagtag atttagaagt taggatattg gggtcgaagt ttggttttgt tatatcgtat 3960
   ttgggcgatt ttgggaaaag ttattttagt tttgtggttt tagttttttt attagtaaaa 4020
   tttagagggg ggattagttt ttgttttagg tttttttagt tttaaaagtt tgtttttggt 4080
   tgttttttta ttgttggaaa tgtttgataa aatagttaaa tatcgttgtt aatagaaatc 4140
   ggttttaggt ttgagtttgc ggttttgttt tttacgtgcg ggtagagttt tcgcgcgttt 4200
   ttttttcggg gaaagtatgt ttaggattcg gtgttagagg tcgaatggat tcggcgtaac 4260
   ggaggttttc ggcgagagag aagaagaata aaacgttttc gtttgttata cgttttagag 4320
   atttacgtgt atcgtttttt taaatgtatc ggttttttaa tcgttcgcgt cgcgtttatt 4380
   tttagtcggg gttcggcggt aggcgggcgt agggaagtgg ggacgtagtg aggtcgaagg 4440
   tttgagggtc gtttttcgcg atttttttac gatttagttt cgggtcggtt tagtcgtcgt 4500
   ttgtcgggta tttttttttt ttcgttttcg cgttggtcgt agaggttagg agcgttgcgt 4560
   acgtaggggt cgtagattta tagtcgggga agggatcgtg gttagaggcg tgcggagggt 4620
   agggtagggt ttttagggcg tagttttatt tcgggttttt agcgtcgtag gcgatagggt 4680
   tttggtttgg gtatttttcg gtatcgttcg tttatttgtt ttgtcgtcgc gttttagcgg 4740
   agtttacgta gttggttgag cgttcggtcg gattcggcgt cggtttaggt ttcgttttac 4800
   gttcgtttag gtatatttcg gtgggcgacg tcgcgttttt tcgtttattg gggttttatt 4860
   tggagtttcg ggcgcgcggg gagggtggtt tttacgtcgc gtggatgata gggttatttc 4920
   gacgtcggaa ttaagcgacg attttagaag gagtggaggt cgggtttgtt atttttttgc 4980
   gggtttggta gtagcgtggg gcggtaggat ataggtttcg aggatatttt tttttgtagt 5040
   tttgggttta tatttttttt ttttttcggt tgtttttata ttaaaggagt attaatggtt 5100
   taatgtgagt taatattatg agaatgtttt tggtttgttt tttagggcgt tttagagtta 5160
   ttaggagttt tgtataaaat tgaataggaa taaattgttg tttgatttga gaattggttt 5220
   agggggttgt ggttttagga tttggggttt gtggggtagg ttgagattgt taataatttg 5280
   gcgttgtttg agtagtttta tttttttttt tttaatttaa ggtgaaattt atataatata 5340
   aaatttatta ttttaaagtg ggtattttag tggtatttag tgaatttata atgttgtgta 5400
   attattattt ttgtttagtt ttaaaatatt ttttttattt cgaaagaaaa attttatatt 5460
   tattgagaag ttttttttta atgttttttt tttttttagt ttttggtaat tattagttta 5520
   tttttgtttt tatatttatt tattttagat attttatata aacggaatta tagtgtgtgg 5580
   atttttgtgt tttttattta gtatgttttc gaggtttatt tttaggtagt atgtaataga 5640
   ttttattttt ttttatgatt gaatagtgtt tgtgtgtgta tatgtatatg tgtgtgcgtg 5700
   tgtgtgtgtg tattatgtat gttatagttt atttatttat ggatattggg gttgtttgta 5760
   ttttttggtt ttgtgaataa tgttattatg aatatgtatg tatatgtatt tgtttgagag 5820
   tttattttta tttggggagg gtatatatat aggggttgaa ttgttggatc gtatggtaat 5880
   ttaatttttt tttttttttg agatagagtt ttgttttgtt gtttagttta gagtgtagtg 5940
   gtcgtaattt tggtttattg taatttttgt tttttaggtt cgagtaatta ttttgtttcg 6000
   gttttttgag tagttggagt tataggtgtg tgttattata tttggttaat ttttgtattt 6060
   ttagtagaga tggggttttt ttatgttggt taggttggtt ttgattttga atttaagtta 6120
   tttgtttgtt ttggtttttt atagtgttgg attataggta tgagttatcg tgtttagttt 6180
   ttagtgttta attatttggg gaattttaag tggtttttac gatggttgaa ttattttata 6240
   tttttattag taatgtatag gattttaatt tttttatatt tttattaata tttgttattt 6300
   tttattttat tgttatttta gtaggtgtgt agcggtattt tattttggtt ttggtttata 6360
   tttttttaat aattaatgtt attaagtatt ttttttttta tgtgtttgtt ggttatttgt 6420
   atattttttt tggagatatg tttatttaag ttttttgttt attttaaaat tgggttgttt 6480
   ttttgttgtt gggttgtaag agttttttat atattttgga tattagattt ttattagata 6540
   tatgttttgt aaatattttt tttttatttt gtaattgttt tttttatttt ttgatgtttt 6600
   tttatgtata taaaatttta attttgatga attttagttt aattgttttt ttttttgttg 6660
   tttgtgtatt tggtgttttg tgtgagatat tattgttaaa tttaaggttg tgaagattta 6720
   tttttgtgtt tttttttaag agttttatag ttttagtttt tatatttagg ttgttgattt 6780
   attttaagat aatttttgta tgtagtgtga ggttagggtt taagtttatt gttttgtatg 6840
   tggattttta gttgttttag tattattcgt tgaagagatt gttttttttt tattgaattg 6900
   ttttggtttt ttgttaaaaa ttaattggtt atagatggat ggggaaaatt aaatattttt 6960
   aaagagttgt ttaaatgtag attaattttg gatggaattg gggtaggtta gtattttggt 7020
   taagaaaata atggtttaaa taatgatttt tgataaattt gagaaattaa ttatatttaa 7080
   tagaattata aaaataatcg atttttggaa aaggaaggat atttgaaggt tataaagttt 7140
   aattttaatt gatgtatatg agtttttgtt atagaatttt tgggaaataa ttttttaatt 7200
   tggtttgttg ttgttgttgt tttgaaatag ggttttgttt tttcgtttag gttggagtgt 7260
   agtggtacga ttttagatta ttgtaatttt tgttttttaa gtttaagtga tttttttgtt 7320
   ttagtttttt aagttagttg gaattatagg tatgtgttat tttgtttagt taatttttgt 7380
   aattttagta gagagggggt ttttgttatg ttgattaggg tggtttcgaa tttttgattt 7440
   gaagtgattt tttcgtttcg gttttttgaa gtgttgggat tagattatag gtatgagtta 7500
   tcgcgtttat ttttgattgg ttgttgttgt gttgttgtta tttagttttt agtgttgtat 7560
   ttgataggtt gaattttttt taatgtgaag aggttgtata tgttttaagg tgaagtataa 7620
   tgatttaata aatgagttag gaagttgtga tataataaaa ataaatattt ggtttttatt 7680
   ttcgtttttg gtataagttt ttaaaatttt tggaatttta ggtgtgatga atgttgttgg 7740
   tatgttaatg aaatgattgg tggttgggga tttttgatta gttttaggat aggggttggt 7800
   ggttagaaag attaaggtat gatttaaata aatagaagat tgtagttttt aataagtatt 7860
   aataggattt gatttttaga gttattatat tataatattt aaaatgttta ggtttttttt 7920
   ttttttaatt agttttttta ggttgaagtg tttagttttt gttttttttt ttttttttga 7980
   gatagagatt tttattgtta ttagggttgg agtgtagtgg cgcgatttta gtagtttgta 8040
   atttttattt tttaggttta agcgattttt ttgttttagt ttttcgagtt gttgggatta 8100
   taggtattta ttattatatt tagttaattt tttgtatttt tagtagagat agggttttat 8160
   tatgttggtt aggatggttt cgaatgtttg attttgtgat ttatttattt cggtatttta 8220
   aagtgttggg attataggcg tgagttatta tgttaggttc gaagtgttta gtttttaatt 8280
   aaaaagtaaa taaataaata aaataggaag tatattaaga aaaaagaaaa gtataattta 8340
   tttataggaa aaagaaattg atagaaatta tttttgggga ggtttagata ttgaatttgt 8400
   tagataaaga tttttattta aatgtttaaa agtttattta aacgtttatt taaatggttt 8460
   taaatatgtt taattaatta aaggaaatta tggataaaga attaaaggaa gttaggtaaa 8520
   taataaatgt ataaatagaa tattagtaaa gagataaatt atgaaaaata aattaaatta 8580
   aaattttgga gttgaaaagt ataatagttg aaatgaaaat tttgaaaaga tgaataatat 8640
   tgataaattt ttagttagat tgagaaataa agagtaaaga tgtaaataat taaaattaga 8700
   tatgaaagta ggggtattat tatggatttt ataggaaaaa aaaaaaaaag tattttaagt 8760
   gaatattgtg aataattgta tataaatgat taaaattgat ttaggaagaa ataaatttga 8820
   ataagttagg aaaagtaaag agattgaatt taataagagg ttgaattagt aaggttaggt 8880
   acgatggttt aagtttgtaa ttttagtatt ttgggaggtt gagacgggta gattacgagg 8940
   ttaggagatt gagattagta gattacgagg ttaggagatt gagattattt tggttaatac 9000
   ggtgaaattt tatttttatt aaaaaaaaaa aaaaaaaagt cgggtgtggt ggtaggcgtt 9060
   tgtagtttta gttatttagg aggttgagtc gggagaatgg tatgaa 9106
<210> 71
   <211> 10132
   <212> DNA
   <213> Artificial Sequence
<220>
   <223> chemically treated genomic DNA (Homo sapiens)
<400> 71
   tatatgtaag gtaattgggg taggtaatga ggaaattaaa aaatgttatt gttgttttta     60
   acgagtataa taagatggta ataattaata tttattgagt tttttatatg gtaggtagta    120
   ttttgagttt tttatagtta tttaatttaa ttattataat tattatataa gggaaatatg    180
   gaaattttat gtttttgttg taggataagt tattaagata gtgatttttg aaatatttta    240
   tgtatataat gaatagtaaa tggtttgagt aaaaattttt ttttgatttc gtattttaat    300
   gatattaaga tttgaattat aggtatatgt agaagttatt aaattagatt tttttttttt    360
   taaataatag tatgagatga gttttttata tttatttttt aagtatttat tatatttatt    420
   ttaatatatt agaagtggtg ttttaatata ttttaatttt ttttttattt taaggttgtt    480
   tatttttaaa attataaggt ttagaggaat tataaaagat aaagttatcg ttttattaaa    540
   tttgaaagta ttgttaaagt aaatttttta taatagttaa ttatttaagg aggagttaaa    600
   ttaaatttaa tattgagtat tttatggtat tttgttggta tattgtttgg ttgttatttt    660
   tggggtagga taatatgttt tttttaggaa atattattat ttagagattt ttattatttt    720
   gtgaattttt gtatggtttt gtttttgaat tagttttatt tatgaaagtt agatttgaaa    780
   aatttaggag aagttaggtg tcgtggttta tgttcgtaat tttaatattt tgggaggtta    840
   aggtgggtag atattaggtc gggagtttga tattagtttg attaatatgg agaaatttta    900
   tttttattaa aaatataaaa ttagttaggt atggtagtgt acgtttgtaa ttttagttat    960
   ttgggaggtt aaggtaggag aatcgtttga attcgggcgg tagaggttgc ggtgagttga   1020
   gattatgtta ttgtatttta gtttgggtaa taatagttaa attgttttaa aaaaaaaaaa   1080
   aaaaaattta ggagttattt ttagtttatt ttggtttaag ttttatatat aaagaatatt    1140
   taaaataagt tattgtaagt tttataaagt aatttgtatt ggggaaagga ttatatatga    1200
   aataggaata tatatgttat tttttatggg atattttaga gggatttcga gtttggtttt    1260
   agattattaa taaaagagtt atataaattt tttggttttt tagtgtatat gaaagttttt    1320
   tatattaaag tgtagtttgt taagtgtgta ataatatgtt taaaaaaata atgtatatat    1380
   tttaatttta aaatattgtt aaaagatgtt agggattatt tgagttttta gcgagttata    1440
   aatgttggtg tggggtattg ttgagggttg ttgattgatt agggtggtag ttgttgaagg    1500
   ttgggatgat tggtaatttt ttaaaataag gtaataagga agtttgtttt atttttttgt    1560
   ttgttttgag atggagtttc gttttgttat ttaggttgga gtgtagtggt atgattttgg    1620
   tttattgtaa tttttgtttt ttggtttaag tgatttttgt gttttagttt tttgagtagt    1680
   tgggattata ggtgtttgtt atcgtgttcg gttaattttt gttattttta gtagagatag    1740
   ggttttgtta tgttggttag tttgggttcg aatttttggt tttttaaagt tttgggatta    1800
   taggtatgag ttattgtatt tggttattta ttgattgttt tatttattaa agattttttt    1860
   gtagtatgtg atgttgtttg gtagtatttt atttatagat ttttttttaa aattggggtt    1920
   aattttttta aattttgtta ttgttttgtt aattaatatt ttgaattttt tgttgttatt    1980
   taaatataat ttatagtata tttattagga gtagatttta ttttaataaa ttattttttt    2040
   tgtttattcg taaaaagtat ttttttattt attaaagttt tattatgaga tggtaataat    2100
   gtagttatat ttttaggttt tatttttaat tttatttgtt ttgttatttt tgttatattt    2160
   atagtgattt tttttattga agttttgaat tttttaatat tagtgatgag gattggaatt    2220
   aatttttttt aaatttttgt taatgtggag atatatgtat atatttatgt ataatgtatg    2280
   tgtgtgtgta tatgtgtgtg tgtatatata tatatatata tatatatata tatatatata    2340
   taaaatggat aggtatgttg ttttgtttgt aaaatattat ttagtgtgtt aattgaaata    2400
   tatataattt ttaaatattt atatatgtat tttatatata ttatatatat atatatatat    2460
   atatatatat attttttttt tttttttttt ttttttttta aagagtgttt tattttgtta    2520
   tttagattgg agtgtagtgg tatagttagt tgattgtagt tttaaatttg ggtttaaata    2580
   attttgttat tttagtgttt tgagttgttg ggaatatagg tatgtgttat tatttttggt    2640
   taattaaaaa aaaaaaaaaa ttgtagagat agggttttgt attgttgttt aggttggttt    2700
   taaaattttt ggttttaagt aatttatttt ggttttttaa agtgttgggt ttataggtat    2760
   gagttattgt ggttgatata tgtattttta atttttcgtt tttttttttt ttttttttta    2820
   gtttttagtt atatatcgta agtggatatt ttgatttttt atgaattata aatgttttta    2880
   atggtatttc gaatgatgaa ttttttttag aagattttta atttattttg tttagaatta    2940
   ttagaggaat tatgatgata gttatagttt tatgaaatgt attttgtaaa taagatttga    3000
   aagttaaaat ttttttttga tttttgggtt ttagaatgga tgttgtgtta gtaagtacga    3060
   taatagtatt agtttttgta tatttttatt agaatttttg gatgattagg tgtattgtta    3120
   atgaggagta atattttgaa aggaattttt tttttgagta ttagagttta atagtgggtt    3180
   ttaaatattt tttaagttat gttgtaaata gatgtgttgt tattaattag tttttgttgg    3240
   tttatttata gattatattt agtataattt ttaagggtat taggattttt agaatggtaa    3300
   aggaatgttg gttttaaagt tattagttgt attagttttt aataagagta ttagtttgtt    3360
   ttttgaagtt ttgaagatag gtattgattt ttttttagtt atgaaagttt tagatggaat    3420
   ttttttttaa tagaaggtcg ttttatatat atttaagatt tgttgttttg gttgggtacg    3480
   gtggtttata tttgtaattt tagtattttg ggagatcgag gcgggtagat tgtttaaggt    3540
   taggagttta agattagttt ggttaaatat ggtgaaattt tgtttttatt aaaaatataa    3600
   aattaatttg gatatggtgg tatattttgt aattttagtt atttgggagg ttgaggtaga    3660
   agaatcgttt gaatttagga ggtagaggtt gtaaaagatc gtattattgt attttagttt    3720
   tggtgataaa gtaagattgt tttaaaaata aaagattgtt gtttagtgta gttgttttta    3780
   ttaatgattt tagttagatt tggataattt gatgtagttt ttacgttagt atttgttgtt    3840
   ttattttgta tttttatgtt atgaagatag tttttttttt taaattttag gaattatttt    3900
   ttttgttagt ttttagtttt ttttttgtaa tttttttatt ttttttagtt tttatagaat    3960
   tgaagagtta gggttttgtt ttggattagg ttttggttta agggaatgtt gtggttgttt    4020
   ttatttttta tttagattaa ttagaatttt ttttatatta gtaataaggt tgtttagttt    4080
   tattgttttt tgtgtgttta ttggagtagt atttttaatt ttttttaaaa tttttttttt    4140
   tgtatttatg atttggtttt ttttttttgt atttataatt tggttaattg gagtaagaag    4200
   tttagttttt tgtttattaa tattattttt ttattaattt aattatgttt gtttttgatt    4260
   taaagtgata ggtttttttt tttatttgga tatttagagg tttttgtagg attattagtt    4320
   ggtttaattt taaaattgtg tttttaggaa tagggaggtt ttaggagggg gagagagaag    4380
   ggaaagttcg gtggaatagt ttaaatatat atagggttta tggattaatt tttttttttt    4440
   ttatgagcgc ggtttatggt gttttaaaat aattgtaata atattaaaga ttattgatta    4500
   tagattataa tagtagatat aataataatg aaaaattttg aaatattgcg agagtattaa    4560
   aatgtgatat agagatataa agcgagttaa tgttgaaata taggtagtat ttgttaagcg    4620
   gtataaaata atttatgttt gtatttttag aaagtatagg tatagtaaag taaggaatag    4680
   taaatgtttg gggtagttgg gattaaatga gttcgagttt atataagtga tgtgaaattg    4740
   tttcgggtta ggcggtttgt gtgttttttc gggtggtaat tggtatgttt ttagggtacg    4800
   gttagtttta gcgggtttgg aggcgaagat cgcgtatttt tttttgtttt cgcgtttttg    4860
   gttttcgcgg gtttcggagc gattgggagg agttttaatt ttgggaagga ttagttcgat    4920
   ttacgggagt tcgggcggcg cgtaattaga attaattggt tttacgttcg ggggtagggg    4980
   gcggggtcgt cgaggaaggg tcgagggcgg ggtcgggttc gggagtttgt ggttttagga    5040
   agaggagggt aaggtgtttg gttgcgcgtt tggttgtaat gagttcggtt tcggggtttc    5100
   gtagggggta ttcggtaggt aggtaggggg cgtcgattgg gtatcgagta ggtttgcgtt    5160
   ttgtttcggt tggaggggaa acgagggtta gtggatcggg cgcgcggatc ggagtggggt    5220
   cgcgggtttt ttcggttttg tagggcggtt tggagaagtt gtatttttgg attgaagcgt    5280
   ttagggcggg tggggacggt ttcgcgtttt agtttaagaa atattcgggg atttggttgt    5340
   cgttttttgg cggtttcgag tagcgacgtc ggcgtcgggg cgtgtttttg tcgtttttgt   5400
   ttttcggggc gatgagcgtt ttcggggcgt ttcggttgtt taaattttaa aaggttgttt    5460
   ttacgcgggt ttggcgtttt tttatatttg gtcgtttgaa atgttggggg ttggattttt    5520
   tgtttgttta tttcgaaaag tgaaaaggag gggttgtttc gtcgagttat aatgaaaata    5580
   taataggttc gcggggtgtt cgggttaaga ttttgggttt tttcgggttt tagttggagg    5640
   cggttagttt ttggcggtta gtatatttcg gtattttttt tttttattta ggaaatttgt    5700
   tgttgtttgt cggtttgagt taaaattata ataatttttt tttgtaaagt aattaattgt    5760
   taagagcgta cgttttgtta tttattattg gtaaagttta gaaattttaa gtttggttag    5820
   agtgaaataa ataaaaaggt cgaaacgttt gtagttggtc ggtcgtggtg gtttacgttt    5880
   tgtaatttta atattttggg aggtcgaggc gggcggatta ttagaggtcg ggagttttcg    5940
   attagtttaa ttaatacggt gaaatttcgt ttttattaaa aatataaaaa aattagttag    6000
   gtttgggcgt aagttattgg ggaggttgag ataagagaat cgtttgaatt ttggaggtgg    6060
   aggttgtagt gagtcgagat cgttttattg tattttaatt tcggcgatag agtgagattt    6120
   cgaattaaga agaaaaaaaa aaaaaaagaa cgaaacgttt gtagttggtt agagtgtttt    6180
   tgtttaagaa ttttttatat tttgttgtat aaaggaaatg gtgttttaga ggtaagttgt   6240
   aaaagttata tggtcgtaga ggaggtagat aggtagtttt gttttggagg tatagtgtag    6300
   ggtttagttg tagtttttag gggcgggaag tcggggtttt tttttattta tttgtttttt    6360
   tttttttttt tttttatcgt attttttagt gtatgtttta ggtttgatgg aggttatttt     6420
   atatagggtt attaaatata ttgttgatgg agtcgatggg tagattttta tagagagtgt    6480
   agtgaatgaa gacgatgata gtagaatgat cgaagtcgtt tgggaagttg aataaagttt    6540
   agaaaggtag tcggtttcgg gtgggagggg ttgcgtgggg atgggtttat agagattttg    6600
   gaatttgtag gtgggttggt ttttaatttt agttttattt tgtcgatttt ggtacgtttg    6660
   gtttattttt tttgtttttg tttttgagtt gtggagttgt gagataaggt atttaaggta    6720
   ttaggttatt aataaatggt agttggtagg attatttgga gggattatta aattagagtg    6780
   aggtgaaaaa aggaatgatt agttttgtaa aatttaagtt gatttaagga ataaggaggt    6840
   ggtagttata ttattttgta aaggaagaga agtgaaaaga gggttagata tagtggtggg    6900
   cgtttttagt tttagttatt ggggagattg aggtaggaga atcgtttgaa atttggaggt    6960
   ggatttgtag tgagttaaga ttgcgttatt gtattgtagt ttgggcgata gagggaggtt    7020
   ttgttttaaa aaaaaaaagt gaaaagaggt taagaattag gtttaaagaa gggtatattt    7080
   ttgataagta gattgtagat gaaaaggaat ttttatattt gttatatagg gtagtttttt    7140
   tattaaaatt tatttttttt atattaataa agttattttt tattttattt aggtttttaa    7200
   atattaacgt aggtcgggcg tggtggttta tgtttgtaat tttagtattt ttaggaggtt    7260
   gaggtaggtg gattatttga ggttaggagt ttaagattag ttaggataat atagtaaaat    7320
   tttgttttta ttgaaaatat aaaaaaaaag tagttgggtg tggtggcgta tgtttgtaat    7380
   tttagttatt taggaggttg aggcgggaga atcgtttgaa ttcggaggcg gaggttgtgg    7440
   tgagtcgaga ttacgttatt gtattttagt ttaggtaata gagtaagatt ttattttaaa    7500
   aagatagtaa taaaaaataa atattaatgc ggtataatga tatttaagtg tttttttttg    7560
   gaaagtttgt ttttttggga tatatagttt aaggtggtta tgatgtgtta ttttttttta  7620
   atttgtgggt aaagtttatt aattttagaa aagtattgga atttaggttt tggtatttgt    7680
   ttttatagtt ttttgttttg atttagtgtt tgttagagtg gttattggat ttggttatta    7740
   attggtattg taggatgtag ggtgtatttt gtgagagtga aggtaagtta tagttggtta    7800
   gtattagaat tgggatgttt agagtttagg ttttttttta tattcgtagt attaataaat    7860
   aataataata ataaaaaatt taaaaaaaaa attttttatt gaggttgtta aatttgttaa   7920
   ttttgaaatt gaatgtatta agagatgggg tttagttttg ttatttaggt tggagtatgg    7980
   tgattattta taggggtagt tatagtatat tatgttttag aatttttggg tttaagagat    8040
   ttttttgttt tggtttttta ggtaatcggg attataggtt tgttattatg ttcggttaga   8100
   acgttatttt aaatagattt ttttgagtag ttatttaata ttagttagtt atttttaaat    8160
   ttgtaggttt tgattaatgt gagggtatat ttgagataat ttaaatttat attttaagtt    8220
   attaatttta atgtgttagt taataattgg gaagttattt ttttggtaat atgagaaata    8280
   agagttttaa agatttaagt aagaatatat ttttagtgtt tttattttga gggatatgag    8340
   ttagtttgtt gagtagttat ttggtatagt ttttatgagt taggttgtga gtcgttttcg    8400
   gtttagtagt tagtatggag tagaagttat aggaggtgag tcgttggttt gttgttatcg    8460
   aggaggatat tgagatatga gtgtttttta agttgtgtag aattttggta tgtgaggagg    8520
   atggttgtga tatatggatt taattttttt gaagttattg tttttttata ttttaattgg    8580
   ttaaaagata agtaggtgag gttattaaaa ggtagtagat ttcgatagtt tttgaaaatg    8640
   ggaagagtga aatttttgtg ttgtttttat agagtagtgt gtggagtagg aagtatttcg    8700
   ttattgtgtt tatttagggt aaaggtcgga ttaaatagtt tttttatttg ttatgtagcg    8760
   gggaggatta gttggagagg ttgtttttgg tttttatatt ttagtgtaaa aattattttg    8820
   tgaaaaatgt agttgttatt tttttttttt aagattttga ttttgtttta gttaggattt    8880
   aggaatttgt attttaatat gtatattagg aattttgatg tttaaggttg agtaaaattg    8940
   atttagggcg tatagataga ggatgtttta gtgtggggtt ttagttgggt tttattgttt    9000
   ggggtagttt taggaggaat aggttaaaag ggatttgaat tgaatttttt attttgtatt    9060
   ttttttgagg agaagtgagg ttagattaat taggtgatgg gggttagtaa tggggagatt    9120
   tttaaggaga ggggaattaa tttttttggt taggagatga tgggtaagag tttatgtttt    9180
   agggttattt ttttttaatt gaagggtgta ttttgttata ttgttaatat tttagtttat    9240
   ttattgttgt ttttttttta agttgttgag ttattaggta aaaatagatg tatgagtgga    9300
   tggggttttt attgtagaag atgttatatt ttgtattgtt tagtgtagaa gttattggtt    9360
   aatttattta aattgataat aaaatcgagt tttttagttg tattagttgt atttaaatgt     9420
   ttagtagata tatgtggtta gtggttattt tatgagatag tgtaaatacg gaataatttt     9480
   attattatag atatttttat gatatagtat tgttttagag agtagagagg aatttagtgg     9540
   aaggttgttt agggagtagg gagtttagtt gagaatggag agtagagggt tttttttata     9600
   gggttggaaa tgtagatgtg ttatgtagga gatttaaggt ttaagttttt gtttttgttt     9660
   tttttttttt ttatttaaat ttttattttt tttaggttta tattattgtt aatttagtat     9720
   taattataaa ttatatttta gtttttttgt aggatttaat ttttttttag aaaatgtgta     9780
   aaaatatttt ttgtataata aagtaatttt atattttaat ttcgtaatta tagaattgtt     9840
   attttaggta tattttgtag tgaatgtatt tgtgtaatat tttttttatt tttgtttata     9900
   agattgttat tttgtaggtt aagttggggg tgagaaagta tagagttgtt atagtttgtt     9960
   tattagtaag tagttttttt ttattttttt ttttttatat attttggttt ttaaattaat     10020
   taaaatagaa gggttttaag agtttgggag ttagaagggg gatgggagtt tttattatat     10080
   tttattgtta taagtaaaaa ataattaata aaaattaatt ttttttaata gg             10132
<210> 72
   <211> 10132
   <212> DNA
   <213> Artificial Sequence
<220>
   <223> chemically treated genomic DNA (Homo sapiens)
<400> 72
   tttattaaaa aaagttgatt tttgttaatt attttttatt tatggtagta aaatatagta      60
   ggaattttta tttttttttt gatttttaaa tttttaagat ttttttgttt tggttagttt    120
   aaaagttaaa gtatgtgaag aaaaggaaat gaagggaaat tgtttattaa taaataaatt     180
   ataatagttt tatgtttttt tatttttaat ttgatttgta aaataataat tttataaata     240
   gaaatggaga aaatattata taaatgtatt tattgtaaaa tatgtttaga gtaatagttt     300
   tgtgattacg gagttgaaat atgaagttgt tttgttatgt agaaaatgtt tttgtatatt     360
   ttttgaaaaa gaattaagtt ttataggaaa attgaaatat agtttgtggt taatgttgga     420
   ttagtagtgg tgtaagtttg gaagaggtag aagtttgggt ggaggaaggg aagggtaagg     480
   gtaaagattt ggattttgga ttttttgtat gatatatttg tatttttaat tttgtaaggg     540
   gagttttttg ttttttattt ttaattggat tttttatttt ttggatagtt ttttattgaa     600
   ttttttttta ttttttagag tagtgttatg ttatggaagt gtttgtgatg gtggaattgt     660
   ttcgtatttg tattgtttta taaggtagtt attggttata tgtgtttatt gagtatttaa     720
   atgtagttaa tgtaattgag gaattcgatt ttattgttaa tttaaatgaa ttggttagtg     780
   gtttttatat tggatagtgt aaagtgtggt attttttata ataaaggttt tatttattta      840
   tgtatttgtt tttatttgat agtttaatag tttagagggg agatagtaat gaataaattg     900
   aaatattagt agtatagtaa gatgtatttt ttagttgagg aagaatgatt ttaaagtatg     960
   gatttttatt tattattttt tagttaaaag ggttggtttt ttttttttta gagatttttt     1020
   tattattagt ttttattatt tgattgattt aattttattt ttttttaaaa aaaatataag     1080
   gtggggaatt tagtttaagt tttttttagt ttattttttt tggaattgtt ttaagtaatg    1140
   agatttaatt aagattttat attaaggtat tttttgtttg tacgttttaa gttagttttg    1200
   tttaattttg ggtattagaa tttttggtgt gtatgttaaa atgtagattt ttggatttta    1260
   attgaggtag aattagaatt ttggaggaag gaggtgatag ttgtattttt tataaagtga    1320
   tttttatatt aaggtgtgag agttaaaagt aattttttta attggttttt ttcgttgtat    1380
   ggtagatgag aaagttgttt aattcgattt ttgttttagg tgagtatagt aacgaggtat    1440
   tttttatttt atatattgtt ttatgaagat agtataggga ttttattttt tttattttta    1500
   aaagttgtcg gaatttattg ttttttgata attttatttg tttatttttt aattaattag    1560
   agtataaaga aataataatt ttaagaaaat taaatttata tgttataatt atttttttta     1620
   tatgttaaaa ttttgtataa tttggaaagt atttatgttt tagtgttttt ttcggtggta    1680
   gtaggttagc ggtttatttt ttgtggtttt tgttttatat tgattgttga gtcgaaggcg    1740
   gtttataatt taatttatgg aagttgtgtt aaatggttgt ttagtaaatt aatttatatt    1800
   ttttagggtg aaaatattgg agatgtattt ttgtttgggt ttttaaaatt tttgtttttt    1860
   atattgttaa agaagtagtt ttttagttgt tagttgatat attaaaatta ataatttaag    1920
   atgtgagttt gggttgtttt aaatatgttt ttatattagt taagatttat aggtttagga    1980
   atggttaatt agtattaagt agttgtttaa gaaaatttgt ttaaaatgac gttttggtcg    2040
   ggtatagtgg taagtttata gtttcggtta tttggaaggt taaggtagga ggattttttg    2100
   agtttaagag ttttaggata tagtgtgtta tgattgtttt tgtgaatagt tattatattt    2160
   tagtttgggt aatagagtta gattttattt tttaatatat ttagttttaa agttaataga   2220
   tttggtaatt ttagtgaggg attttttttt taagtttttt gttgttgttg ttgtttgtta    2280
   atgttacgga tgtagaaaaa aatttaggtt ttggatattt taattttgat gttggttagt    2340
   tgtaatttat ttttattttt ataaagtgta ttttgtattt tgtagtatta gttaatgatt    2400
   aggtttagtg gttattttag taaatattga attagaataa aaagttatgg agatagatgt    2460
   taggatttaa gttttaatat ttttttggag ttgataaatt ttatttatag attgagagaa    2520
   aataatatat tatgattatt ttgagttgtg tattttaaga agataagttt tttagaaggg    2580
   ggtatttaag tattattatg tcgtattaat atttattttt tattattatt tttttgggat    2640
   ggagttttgt tttgttgttt aggttggagt gtagtggcgt ggtttcggtt tattataatt    2700
   ttcgttttcg ggtttaagcg attttttcgt tttagttttt tgagtagttg ggattatagg 2760
   tatgcgttat tatatttagt tatttttttt ttgtattttt agtagagata gggttttgtt 2820
   atgttgtttt ggttggtttt gaatttttgg ttttaagtga tttatttgtt ttagtttttt 2880
   ggaagtgttg ggattataag tatgagttat tacgttcggt ttgcgttaat atttaaaaat 2940
   ttggataaag taagaaatag ttttattgat atggagaaaa taaattttaa tgaaggagtt 3000
   attttgtata atagatatag aaattttttt ttatttgtaa tttatttatt aaaaatgtgt 3060
   ttttttttaa atttgatttt tgattttttt ttattttttt tttttgagat agagtttttt 3120
   tttgtcgttt aggttgtagt gtagtggcgt aattttggtt tattgtaagt ttatttttag 3180
   gttttaagcg atttttttgt tttagttttt ttagtagttg ggattagagg cgtttattat 3240
   tgtgtttggt ttttttttta tttttttttt tttgtaggat aatgtaattg ttattttttt 3300
   attttttaaa ttagtttagg ttttataaaa ttaattattt ttttttttat tttattttaa 3360
   tttgatagtt tttttagata attttgttag ttattatttg ttgatggttt gatgttttag 3420
   atattttatt ttatagtttt atagtttagg aatagggata gagaaagtgg gttaggcgtg 3480
   ttaaggtcgg taaagtgaag ttgggattga aagttagttt atttatagat tttaaagttt 3540
   ttgtaggttt atttttacgt agtttttttt attcgagatc ggttattttt ttaaattttg 3600
   tttaattttt taaacgattt cggttatttt attgttatcg tttttattta ttgtattttt 3660
   tatgaaaatt tatttatcgg ttttattaat aatgtattta gtaattttat gtagagtaat 3720
   ttttattagg tttagaatat gtattgggga gtgcggtgga ggaagaagag gaagggagta 3780
   ggtgagtgaa ggaaagtttc ggtttttcgt ttttagagat tgtaattgga ttttgtattg 3840
   tgtttttagg ataaggttat ttatttattt tttttacgat tatgtgattt ttatagtttg 3900
   tttttggagt attatttttt ttgtgtagta aaatataaag agtttttaaa taaaggtatt 3960
   ttaattaatt gtaggcgttt cgtttttttt tttttttttt ttttttgatt cgaagtttta 4020
   ttttgtcgtc gaggttggag tgtagtgggg cgatttcggt ttattgtaat ttttattttt 4080
   agggtttaaa cgattttttt gttttagttt ttttagtagt ttgcgtttag gtttggttaa 4140
   tttttttgta tttttagtag agacggggtt ttatcgtgtt agttaggttg gtcgagaatt 4200
   ttcgattttt ggtgattcgt tcgtttcggt tttttaaagt gttgggatta taaggcgtga 4260
   gttattacgg tcggttaatt gtaggcgttt cgattttttt gtttgtttta ttttggttag 4320
   gtttgaagtt tttaaatttt attagtaata agtgatagaa cgtacgtttt tagtagttga 4380
   ttattttgta gaaaaaagtt attgtggttt tggtttaaat cgatagatag tagtaggttt 4440
   tttggatgaa gaagagagat atcggggtgt gttggtcgtt aggaattggt cgtttttagt 4500
   tgaagttcgg gaggatttag ggttttggtt cgggtatttc gcgggtttgt tatgttttta 4560
   ttatgattcg gcggaataat tttttttttt tatttttcga agtgagtaaa taaaagattt 4620
   aatttttaat attttaaacg gttaggtgtg ggggaacgtt aggttcgcgt ggggatagtt 4680
   ttttaaggtt taaatagtcg gggcgtttcg aaagcgttta tcgtttcgga ggataaaggc 4740
   ggtaggggta cgtttcggcg tcggcgtcgt tattcgggat cgttaggagg cggtagttag 4800
   gttttcgagt gttttttaga ttgaagcgcg aaatcgtttt tattcgtttt gagcgtttta 4860
   atttaagagt gtaatttttt taggtcgttt tgtaaagtcg aaggaattcg cggttttatt 4920
   tcggttcgcg cgttcggttt attaattttc gttttttttt tagtcggagt aagacgtaga 4980
   tttgttcggt gtttagtcga cgttttttgt ttatttgtcg ggtgtttttt gcggagtttc 5040
   gaggtcgagt ttattgtagt taaacgcgta gttagatatt ttgttttttt ttttttgaag 5100
   ttataggttt tcgggttcgg tttcgttttc ggtttttttt cggcggtttc gttttttgtt 5160
   ttcgagcgtg gggttagttg gttttgattg cgcgtcgttc gggttttcgt ggatcgggtt 5220
   ggtttttttt agagttaaag ttttttttag tcgtttcgga gttcgcgggg attaagagcg 5280
   cgggggtagg gagaggtacg cggttttcgt ttttaggttc gttgggatta atcgtgtttt 5340
   ggggatatgt tagttgttat tcggggggat atatagatcg tttagttcga ggtaatttta 5400
   tattatttgt gtgagttcgg atttatttaa ttttagttgt tttaaatatt tgttattttt 5460
   tattttgtta tgtttgtatt ttttagaggt ataggtatag attgttttat atcgtttagt 5520
   agatattgtt tgtattttaa tattggttcg ttttgtgttt ttgtgttata ttttggtatt 5580
   ttcgtaatat tttaaaattt tttattgtta ttatgtttgt tgttgtgatt tgtgattagt 5640
   aatttttaat attattgtaa ttgttttggg gtattatgaa tcgcgtttat aaaggaaggg 5700
   gaaattaatt tataagtttt gtatgtgttt ggattgtttt atcgaatttt tttttttttt 5760
   tttttttttt ggggtttttt tatttttgga aatataattt tgaaattagg ttaattaata 5820
   attttataaa ggtttttaag tgtttaaatg aaaggaagag tttgttattt taaattaaaa 5880
   gtaaatatga ttaagttagt gaggaagtaa tgttggtagg tagaaagtta ggttttttgt 5940
   tttagttagt taagttgtga gtgtaaagga gaaaagttaa gttatgaatg taaaggaaaa 6000
   aattttgaag gaaattaaaa atgttatttt agtaaatata taaaagataa taaagttaaa 6060
   tagttttatt gttgatatgg agaaagtttt agttggtttg gatagaagat aaaaatagtt 6120
   ataatatttt tttaagttaa agtttaattt agagtaaggt tttaattttt taattttatg 6180
   gaggttgaga gaggtgaggg aattgtaaaa gaaaagttgg aagttagtag agaggatggt 6240
   ttttgaggtt taaggaaaga aattattttt atgatataaa agtgtaaggt gaagtagtag 6300
   gtgttaacgt agaagttgta ttaggttatt tagatttagt taaaattatt gataaaggta 6360
   gttatattaa ataataattt tttgtttttg agatagtttt gttttgttat taaggttgga 6420
   gtatagtggt gcgatttttt gtaatttttg ttttttgggt ttaaacgatt tttttgtttt 6480
   agttttttaa gtaattggga ttataggatg tgttattatg tttaggttaa ttttgtattt 6540
   ttagtagaga tagagtttta ttatgtttgg ttaggttggt tttgaatttt tgattttgag 6600
   taatttgttc gtttcggttt tttaaagtgt tgggattata ggtgtgagtt atcgtgttta 6660
   gttaaaataa tagattttaa atgtatatga aacggttttt tattggaaga agattttatt 6720
   taagattttt atagttagag agaagttaat gtttgttttt aaagttttaa agaataggtt 6780
   gatgtttttg ttaggggtta atataattga tgattttaaa gttaatattt ttttattatt 6840
   ttgaaaattt tagtgttttt aagaattatg ttaaatatga tttataaatg gattaataaa 6900
   ggttagttag tgatagtata tttatttata gtatggttta gagaatattt aaagtttatt 6960
   gttgagtttt agtgtttaga agaaaggttt tttttaaaat attatttttt attgataata 7020
   tatttggtta tttaagagtt ttgatagaga tgtatagaga ttgatgttgt tatcgtgttt 7080
   gttaatataa tatttatttt gaagtttagg gattaaggag aaattttgat ttttaagttt 7140
   tatttataaa atatatttta taaggttata gttgttatta tgattttttt gatagttttg 7200
   agtaaagtaa attgaaaatt ttttggaaag gatttattat tcgagatgtt attaagaata 7260
   tttgtgattt atgggaggtt aaaatattta tttacggtat gtaattagag attagagggg 7320
   gaaaaaaaga aaaacgaaaa attaaaagta tatgtattag ttatagtggt ttatgtttgt 7380
   aaatttagta ttttgggagg ttaaggtgga ttgtttgagg ttaggagttt tgagattagt 7440
   ttaggtaata atgtaagatt ttgtttttat aatttttttt tttttttaat tagttaagag 7500
   tggtggtata tgtttgtatt tttagtaatt taggatatta agatggtagg attatttgag 7560
   tttaagtttg aggttgtagt tagttgattg tgttattgta ttttagtttg ggtgatagag 7620
   taagatattt tttaaaaaaa aaaaaaaaaa aaaaaaaaaa atatatatat atatatatat 7680
   atatatatat aatatatata aaatatatat ataaatattt aaaaattata tatattttaa 7740
   ttggtatatt gggtggtatt ttataggtag gataatatat ttatttattt tatatatata 7800
   tatatatata tatatatata tatatatata tatatatata tatatatata tatatatatt 7860
   atatataggt atatgtatat atttttatat taataggagt ttgaaagaag ttaattttaa 7920
   tttttattat tgatgttgag gggtttaaga ttttagtgag ggaagttatt gtagatgtgg 7980
   tagaagtagt aagataaata gaattggaag tggggtttga aaatgtggtt gtattgttgt 8040
   tattttatga taaaattttg atagatgagg agatgttttt tacggatgag taaagaaagt 8100
   ggtttattga gatagaattt atttttggtg aatatgttgt gaattgtgtt taaatgataa 8160
   taaaggattt agggtattag ttgataaagt agtggtaggg tttgggggga ttgattttaa 8220
   ttttgaaaga aggtttgtgg ataaaatgtt attaaatagt attatatgtt atagggaaat 8280
   ttttagtgaa tggaataatt aatggatggt taggtgtagt ggtttatgtt tgtaatttta 8340
   ggattttggg aggttaggag ttcgagttta gattggttaa tatggtaaaa ttttgttttt 8400
   attaaaaata ataaaaatta gtcgggtacg gtggtaggta tttgtaattt tagttattta 8460
   ggaggttgag gtataagaat tatttgaatt aggaggtaga ggttgtagtg agttaagatt 8520
   atgttattgt attttagttt gagtgataga acgagatttt attttaaaat aaataaaaaa 8580
   atgaagtaaa ttttttattt gttttatttt aagagattgt tagttatttt agtttttagt 8640
   aattattatt ttaattagtt agtagttttt aataatattt tatattaata tttataattc 8700
   gttgaaggtt tagatgattt ttagtatttt ttagtaatat tttaaaatta aggtatatat 8760
   attatttttt tagatatgtt attgtatatt taatagatta tattttagta tgaaaaattt 8820
   ttatatgtat tgggaaatta gaaaatttgt gtgatttttt tattggtggt ttggaattaa 8880
   attcgaaatt tttttgaggt attttatagg gggtggtata tatgtttttg ttttatgtgt 8940
   agtttttttt ttagtgtaag ttattttgta ggatttgtag taatttattt taaatgtttt 9000
   ttgtatatga aatttgggtt aaagtaaatt ggaggtaatt tttggatttt tttttttttt 9060
   ttttgagata gtttagttgt tgttgtttag gttggagtgt aatggtatga ttttagttta 9120
   tcgtaatttt tgtcgttcgg gtttaagcga tttttttgtt ttggtttttt aagtagttag 9180
   gattataggc gtgtattatt atgtttggtt aattttgtat ttttagtaga gatggggttt 9240
   ttttatgttg gttaggttgg tattaaattt tcgatttggt atttgtttat tttggttttt 9300
   tagaatgttg ggattacggg tatgagttac gatatttggt tttttttgga ttttttaaat 9360
   ttggttttta taggtagagt taatttaggg atagaattat gtagaaattt atagaatgat 9420
   gagggttttt agataatggt gttttttggg gagagtatgt tgttttattt taaaaatagt 9480
   agttaaatag tgtgttaata aggtgttatg gggtgtttaa tgttggattt aatttagttt 9540
   ttttttgggt ggttggttat tgtaaaaaat ttattttgat aatgttttta gatttgatga 9600
   aacggtggtt ttattttttg taattttttt ggattttgta attttaaaag taagtaattt 9660
   tagagtggga aaaaaattaa aatgtgttaa ggtattattt ttaatatatt aaaataagta 9720
   taatgaatat ttaagaaata agtataagga atttatttta tattattatt tgggagagaa 9780
   aaaatttaat ttggtaattt ttatatgtgt ttgtggttta agttttgatg ttattagagt 9840
   acgaaattaa agaagggttt ttatttaaat tatttattgt ttattgtatg tataaaatat 9900
   tttagaaatt attgttttag taatttattt tgtaataaag atatggaatt tttatatttt 9960
   ttttgtatgg tggttgtgat gattgggtta agtgattgtg aaggatttag aatattgttt 10020
   gttatgtgaa gagtttaatg aatattggtt attattattt tgttgtattc gttggaaata 10080
   gtagtggtat tttttggttt ttttattatt tattttagtt gttttgtatg tg 10132
<210> 73
   <211> 5001
   <212> DNA
   <213> Artificial sequence
<220>
   <223> chemically treated genomic DNA (Homo sapiens)
<400> 73
   aagtttaaaa gataattgtt ttagtattta atttaaaaaa tataaattag ttagatatag      60
   tggtgtgtat ttgtagtcgg agttatttag gagattgaga taagaggatt atttgagttt     120
   aagggtttga gattagtttg ggtaatagcg agatttagtt taaaaaaaga aaaaaaaacg     180
   ttaataataa taaaaaaatt tattcgttta ttaaattatt tgtaaggcgt ttgaatgaaa     240
   ggtgtttttt ttattgtggt tttgtttagg gtatttatag ttgtaggtga gttaatttgt     300
   ttggttttgt tttttttagg tttgtttttt tatgatttat ttgtgtgaga aaattatttt     360
   tacgttgttt ttttggagga agtttaatgg atttaattag ttgatgtcgt tgtagtagtt     420
   tgtgtacgac gtgtaggatt agttgaattt tttggttagc gaggaggatt ttaggcgtat     480
   tgtttagtag ttttatagta taggcgaggt gagtttttgg gagtttaggt agggggtcgt     540
   gagaggtgtt agggatttag tttgttttaa attttagttt ttggaagtgg tgtgagtttg     600
   gggaagtagt gtggaaatgg tttagttaat ttataatatt tttatacgtg tgataaatat     660
   agttttaagt gacgttttga attgtagttt tcggtttatt ttaggagttg ttattttgtt     720
   ttgtcgtgtt ttgtttttat ttttagagtt tgttagtttg ggagtgtagt gtttattgtt     780
   ttgggtgatt gggttttttt ttattttttt tgggtattag gaatttaatt tgttattttt     840
   ggtagttttt aggatggttt ttttgtatta gcgtggagtt attagtttta tatagatttt     900
   ttgggtggga aaatagttga gaaaaggtag tgaggtgttt ttgtggaggt aatgtttgga     960
   gatataaaga tagagaattt tggtgtatag atatatagag taattataat taaagatgta    1020
   tattaaaaaa ttttagtaag taatgtttag tagaaatgat tgagaatggg aggttttgtt    1080
   ttggttttgt gatattgagt attagggcgc ggtttggaaa ttttgtttta atggtattat    1140
   atgtttgttt taggaacggg ggtagtgttt tcgtgttttt ttattatgtg aaattgggag    1200
   tttatatgaa ttagtgtggg ttaagaagag cggttttgaa tattgtaatt aaaagtagtt    1260
   ttgagattat taaaagttag atgtttattt ttttaatttt aggtgttagg tttggtttta    1320
   tagttagttt gtatgagtta tataaatttt ttgaaattaa atttgaattt ataagttagt    1380
   gattttgaaa attggagtcg tcgggtagta attttattag tttttaaatt tttagttaaa    1440
   tggttagaga atttgttaat ttatttagga atattttaaa gtgattgaat ttaaggaaga    1500
   atataggtga aggattatat tttagatgat aagatattgt tggatgtaaa aaatgtgtta    1560
   attttaattt taaatttttt tggtatagga gtttttgttt ttttagtagt agattagtaa    1620
   ttttttagat tatattatat agtttttttt atttgttatt atatttttgt ttgtttttga     1680
   gagtttagta agtaaatgaa gatgtaaaga aggagggtaa ttaggtagta ggaattatat    1740
   tgtgtggttc gattttgatt ggtttcgggt tttttttaga tttgtttttc gtatttaatt    1800
   tgtagatttt tagaattttt tagtggtttt tttgattttg ttttttggtt tagggtttta  1860
   tattacgttt agttgattgt tttagttttt tgtttttttt tattatggga tagtttcgtt    1920
   ttttttgagt ttgaggcgat gagtagtgtt taggattaaa ttgtaaatgg tattttagta     1980
   aatgttgtat taggaattag attaatattg gttttgtttt ttagaatttt taatttaggt    2040
   ttaattttga atagattttt agataggggg tattttttag gtttaagtta gattgtggta    2100
   ggaaattatt gtgtggtttc ggtggggtta gagtttttaa gtgagggagt ttagggcgat    2160
   ggttattttt taagaaagga agtagaattt ttttataaag ggaaagtttt ttttgttttt    2220
   tgttattttt tttttttttt tttttatttt tttaagaaat aaatataatt aaaaagttaa    2280
   agtttttttg ggttattatt tttggtttta tttttttttt tttttttcgg aggttaatta    2340
   ttattatggt agtatatttt tttaggtttt ttttatgtgt gtgtgttaaa tatttatata    2400
   atggtataat aatagaatgt tttgattttt ttttgaaaat gtttttaagt ttttagagaa    2460
   gttgtaagga tagtattatg aatttttata taggttatat ttatttattt ttttaattaa    2520
   ttaattaatt tttttttttt tttttttgag atagagtttt attttgttgt taaggttgga   2580
   gtgtagtggt ataatttcgg tttattgtag ttttcgtttt ttgggtttaa gtgattttgt    2640
   tttagtttat agatataagt tattatattt aattaaattt tgtattttta gaagagatag    2700
   ggttttatta tgttggttag gttggtttta aatttttgat tttaagtgat ttattcgttt    2760
   cggtttttta aagtgttggg attataggta tgagttacgg tgtttagttt attatattta    2820
   agttgattag ttgttgatat tttgttatat ttgttcgatt tttttttttt ttatttattt    2880
   atttatttat tttgtagata tgatgatatt ttttttttaa ataatttagt ttatattttt    2940
   taaggagaag gatatttttt tatataattt aatataattg atgattttag aaatataata    3000
   ttaatatagt attatgattg aatatattat ttttatataa gggtttgtta ttggttttaa    3060
   taattttttt taaagttatt tttatttatg tatttatttt gggatttaga atttagttta    3120
   aaattattta ttgtatttgg ttgttatttt ttattttttt tttttttttt atttcgttgg    3180
   tttttttgaa gattttaggg gagttgtttt ttagaatttt tattttgata tttttaagtt  3240
   gttttttttt attcggtgtt tggtttttga gacgtattta cgtggaatgg ttttgggtta  3300
   ttttttagag tatttgttta gtttttgttg tcggtattag gttttttagt gttttaagtg     3360
   gttggtgtat ttggttaggg ttttgttttt aagcgtgatt ttttgttgtt ttttcgtaga    3420
   ttaatattat gtaaagtgaa atagtttagg acgtgttgtt tttggatttt cgttggtttt    3480
   gtataaacgt tttggggaag ttgttgttcg tggagatttt acgggcgttg tattattatc    3540
   ggggtcgtta tatcgtggag gatatttagc gtttggtgtt cgatagcgac gtggaggagt    3600
   tgttgtagat tttcgatgtt atggatattt gcgttcggga tttgagtagc gggattatgg    3660
   tggacgtttt agttttgatt aagatagata atttgtatcg tttttgggtt gatgaggagg    3720
   acgaggtgat ggtgtatggt ggcgtgcgta tcgtgttcgt ggaatatttt attttttttt    3780
   tatgtggtat tttttataag gtttaggtga atttgtgtcg gtggatttat tagtaaagta    3840
   tagagggcga cgcggatatt cgtttgtggg tgaatggttg taagttggtt aatcgtgggg    3900
   tcgagttgtt ggtgttgttg gttaattacg gttagggtat tgaggtttag gttcgcggtt    3960
   tggagacgga gaagattaag tgttgtttgt tgttggattc ggtgtgtagt attattgaga    4020
   acgttatggt tattacgttg ttagggtttt tgatcgtgaa gtattatttg agtttttagt    4080
   agttgcggga gtattatgag ttcgttatga tttattagtt acgggatttt tttcgggtat    4140
   agattttgaa ggaaatttta ttgattaata ttatgggggg gtataaggaa agttttagta    4200
   gtattatgtg tttcgggtgt tacgacgttt atttataggt tagtttcggt atggatattt    4260
   atgtattaga tttgaatttt tttattcgga ggaaattgag tcgtttgttg gattcgttcg    4320
   attttttggg gaaggattgg tgttttttcg ttatgaattt aggttttttt gatttcgtgg    4380
   taaagtataa tattagtaac ggggttttta aggatttttt ttttagtttt ttttacgttt    4440
   tgttgcggga atggattatt tattttgaga gtatagtggg tatttttatg tttaaattga    4500
   gggagttggg tcgtcgggat gtcgtagatt ttttgttgaa ggtatttttt gtgtttaaaa    4560
   ttaatttgga tggtaatggt taggaggttt atgtttcgag ttgtaatagc ggtatttttt    4620
   ataattttat tagttttgtt gtatttcggt gagggtagtt tttggtttgg gtagggtttg    4680
   tttggattgt agaagtaagg gggtgatgta gtttattttt ttttttggag atgttgaggg    4740
   tgtttttttt tgtatttata gttaggggga tgttattttt tttttcggtt tgatttgttt    4800
   ttttgtcgtt attttttttt tcgttttatt tcgttgtttg tggatggtta ttgtagttta    4860
   agagtagaat agatttttta ttttggtcgt ttgaaaagtt agtgtatttt tttttagtgt    4920
   tttggatttt attttttatt ttttagtatt ttgtttttta ttgataattt tgttggaatt    4980
   tttaattttt taatgatatt t                                              5001
<210> 74
   <211> 5001
   <212> DNA
   <213> Artificial Sequence
<220>
   <223> chemically treated genomic DNA (Homo sapiens)
<400> 74
   aaatgttatt gaaaagttag gaattttagt aaaattatta gtaagaagta aggtattgga      60
   ggatgagaga tggagtttaa aatattgaga ggaggtatat tagtttttta agcggttaaa     120
   gtaaaagatt tgttttgttt ttaaattgta atgattattt atagataacg gaatgagacg     180
   gggagggagg tagcggtaga gaaataggtt aagtcggagg gaggagtggt atttttttgg     240
   ttgtgggtgt aggaagaaat atttttagta tttttaaagg gaaggatggg ttatattatt     300
   tttttgtttt tgtagtttaa atagattttg tttaagttag aggttgtttt tatcgggata     360
   taatagagtt aatggaattg taagaggtgt cgttgttgta gttcgaggta taggtttttt     420
   ggttattgtt atttaggttg attttgaata tagaggatgt ttttagtaaa aagtttgcgg     480
   tatttcggcg atttagtttt tttagtttgg atatgagggt gtttattgtg tttttagggt     540
   aggtggttta ttttcgtagt agggcgtgga gggggttggg gaggaaattt ttgggagttt     600
   cgttattggt gttgtatttt gttacgaggt tagggaggtt taagtttatg gcgagaaggt     660
   attagttttt ttttaggggg tcgggcgggt ttagtaggcg atttagtttt tttcgagtga     720
   ggaggtttag gtttgatgta tggatgttta tgtcgaggtt ggtttgtgag tagacgtcgt     780
   gatattcgaa gtatatgatg ttgttgaagt tttttttgta tttttttatg gtgttggtta     840
   gtgaggtttt ttttagagtt tgtgttcgga agaagtttcg tggttggtag attatgacgg     900
   gtttatggtg ttttcgtagt tgttgggggt ttaggtaatg ttttacggtt aggagttttg     960
   gtagcgtggt ggttatgacg tttttaatgg tgttgtatat cgagtttagt agtaggtagt    1020
   atttgatttt tttcgttttt aggtcgcgga tttggatttt aatgttttgg tcgtggttga    1080
   ttagtagtat tagtagttcg gttttacggt tggttagttt gtagttattt atttataggc    1140
   ggatgttcgc gtcgtttttt gtgttttgtt ggtggattta tcggtatagg tttatttgga    1200
   ttttgtgaaa gatgttatat gggaaggggg tgaggtgttt tacgggtacg atgcgtacgt    1260
   tattatatat tattatttcg ttttttttat tagtttagga gcggtgtagg ttgtttgttt    1320
   tgattagggt tgggacgttt attatggttt cgttgtttag gtttcgggcg tagatgttta    1380
   tggtatcgag gatttgtagt agttttttta cgtcgttgtc gggtattagg cgttggatgt    1440
   tttttacggt gtagcggttt cggtagtggt gtagcgttcg tggggttttt acggatagta    1500
   atttttttag gacgtttgtg tagagttagc gggggtttag gagtagtacg ttttgaattg    1560
   ttttattttg tatgatgttg atttgcgggg ggataataga aagttacgtt tagggataga    1620
   gttttggtta ggtgtattag ttatttagag tattaaaggg tttgatgtcg gtaatagagg    1680
   ttgagtagat gttttaagga gtgatttaag gttattttac gtggatgcgt tttaaaaatt    1740
   aaatatcgag tggagaaaag tagtttgaaa atgttagaat agagattttg aaaaataatt    1800
   tttttgaagt ttttaaaaag gttagcgaaa tgaaagaaga aaaaaaaatg ggagatggta    1860
   attaaatgta atgagtgatt ttagattgga ttttgaattt taaaataagt atataaataa    1920
   aaatagtttt aaaagaaatt attgggatta gtggtaaatt tttgtatgag gatgatatat    1980
   ttaattatag tattatatta atgttatgtt tttggaatta ttaattatat tgggttatgt    2040
   gggaaaatat tttttttttt aggagatata ggttaaatta tttagggaag aaatgttatt    2100
   atgtttgtaa aataaataag taaataaata gaaaggaaag gaatcgagta aatgtagtaa    2160
   aatgttaata attgattagt ttaaatgtag taggttgggt atcgtgattt atgtttgtaa    2220
   ttttagtatt ttgggaggtc gaggcgggtg gattatttga ggttaggagt ttgagattag    2280
   tttggttaat atggtaaaat tttgtttttt ttaaaaatat aaaatttagt tgggtgtgat    2340
   ggtttgtgtt tgtaggttga ggtagaatta tttgaattta ggaggcggag gttgtagtga    2400
   gtcgagattg tgttattgta ttttagtttt ggtaatagag tgagatttta ttttaaaaaa    2460
   aaaaaaaaaa aaattaatta attaattaaa aaaataaata aatgtagttt atatgagagt    2520
   ttatggtatt atttttgtaa tttttttaaa ggtttgagaa tatttttaaa aaagagttag    2580
   aatattttat tattatatta ttatgtgaat gtttaatata tatatataga aaaggtttga    2640
   aaggatatgt tgttatgata atagttggtt ttcgagaagg ggagagagga gtgagattag    2700
   agatgatggt ttaaggggat tttagttttt tggttgtgtt tattttttaa aaaaataaaa    2760
   agaaaggaaa aaaaagtgat aaaaaataga aggaattttt tttttatgga agagttttgt     2820
   tttttttttt ggagagtggt tatcgttttg gattttttta tttggaggtt ttgattttat     2880
   cgggattata tagtaatttt ttgttatagt ttggtttaaa tttaaaagat gttttttgtt   2940
   taagagttta tttagaattg aatttaggtt gaaaattttg gagggtagag ttaatattga    3000
   tttagttttt gatatagtat ttgttggaat attatttgta gtttggtttt aaatattgtt    3060
   tatcgtttta ggtttaggaa agacgaaatt gttttatagt ggaggagaat agggagttaa    3120
   gatagttagt taaacgtagt gtgggatttt gggttagaaa atagagttag aagggttatt    3180
   ggggagtttt gaagatttgt aaattagata cggaaggtag gtttggagga ggttcggggt    3240
   taattaaagt cgggttatat aatgtagttt ttgttgttta attatttttt ttttttatat   3300
   ttttatttat ttattaaatt tttaagggta agtagggatg taatgataaa taaaggagat    3360
   tatatgatat gatttagaaa gttgttggtt tattattaga aagtaaggga tttttgtgtt    3420
   aaaggagttt agaattagaa ttgatatatt ttttatattt agtagtattt tgttatttaa    3480
   gatgtggttt tttatttgtg tttttttttg gatttaatta ttttaaagta tttttggatg   3540
   agttggtaga ttttttggtt atttaattgg gagtttaaag gttgatgaaa ttattgttcg    3600
   gcgattttag tttttaaggt tattggtttg tggatttagg tttagtttta agaaatttgt    3660
   atgatttata taaattggtt atagggttag atttggtatt tgagattggg gaggtaagta    3720
   tttggttttt aataatttta gagttgtttt tagttgtaat atttagaatc gttttttttg    3780
   gtttatatta gtttatgtag gtttttaatt ttatatggtg gagaaatacg aaggtattgt    3840
   tttcgttttt gggataggta tatgatgtta ttagaatagg gtttttaaat cgcgttttgg    3900
   tgtttaatgt tatagaatta gagtaaagtt ttttattttt aattattttt gttgggtatt    3960
   gtttgttaga gtttttaaat gtgtattttt aattatgatt attttgtgta tttgtgtatt   4020
   agagtttttt gtttttgtat ttttagatat tgtttttata aggatatttt attgtttttt    4080
   tttaattgtt tttttattta ggagatttgt gtggagttga tgattttacg ttgatataaa    4140
   gaagttattt tgaaggttgt taggagtagt agattaagtt tttggtgttt agggggagtg    4200
   aagagagatt tagttattta gggtagtggg tattgtattt ttaaattaat agattttgga    4260
   aataaaagta aaatacgata agataaaatg atagttttta aggtgagtcg agaattgtag    4320
   tttagagcgt tatttgaggt tgtgtttgtt atacgtgtgg aaatgttgta ggttagttgg    4380
   attattttta tattgttttt ttaggtttat attattttta agagttggaa tttgggatag    4440
   gttgagtttt tggtattttt tacggttttt tgtttgggtt tttaggggtt tatttcgttt    4500
   gtgttgtgga gttgttgagt aatgcgtttg aggttttttt cgttggttag ggggtttagt    4560
   tggttttgta cgtcgtatat aaattgttgt agcgatatta gttggttggg tttattgagt    4620
   tttttttagg aaggtagcgt ggagatgatt tttttatata ggtgagttat gggaggatag   4680
   atttagaggg ggtagggtta gataggttaa tttatttgta gttgtgggtg ttttgagtag    4740
   agttatagtg gaaaggatat tttttattta ggcgttttgt aggtgatttg atgaacgagt    4800
   gggttttttt gttgttgttg acgttttttt tttttttttt agattgggtt tcgttattgt   4860
   ttaggttggt tttaaatttt tgggtttaag taattttttt gttttagttt tttgagtagt    4920
   ttcgattata agtatatatt attgtgtttg gttagtttgt gttttttggg ttgagtattg   4980
   gggtagttgt tttttagatt t                                              5001
<210> 75
   <211> 20
   <212> DNA
   <213> Artificial Sequence
<220>
   <223> DAPK1 primer
<400> 75
   attaatatta tgtaaagtga 20
<210> 76
   <211> 20
   <212> DNA
   <213> Artificial Sequence
<220>
   <223> DAPK1 primer
<400> 76
   cttacaacca ttcacccaca 20
<210> 77
   <211> 18
   <212> DNA
   <213> Artificial Sequence
<220>
   <223> DAPK1 detection oligo
<400> 77
   gttatatcgt ggaggata 18
<210> 78
   <211> 18
   <212> DNA
   <213> Artificial Sequence
<220>
   <223> DAPK1 detection oligo
<400> 78
   gttatattgt ggaggata 18